# EUROPEAN PATENT APPLICATION

(11) **EP 1 849 465 A1**
(43) Date of publication of application: **31.10.2007**
(21) Application number: 06714496.4
(22) Date of filing: 17.02.2006
(51) Int. Cl.: A61K 31/192, A61K 31/19, A61K 31/198, A61K 31/235, A61K 31/27, A61K 31/341, A61K 31/343, A61K 31/38, A61K 31/381, A61K 31/382, A61K 31/4025, A61K 31/404, A61K 31/41

(54) **AGENT FOR CONTROLLING FUNCTION OF GPR34 RECEPTOR**

(30) Priority: 18.02.2005 JP 2005041775; 28.10.2005 JP 2005315146
(71) Applicant: Takeda Pharmaceutical Company Limited, Osaka-shi, Osaka 541-0045 (JP)
(72) Inventor: KOHARA, Yasuhisa TAKEDA PHARMACEUTICAL CO. LTD., Osaka-shi, Osaka, 5328686 (JP); ARAMAKI, Yoshio TAKEDA PHARMACEUTICAL CO. LTD., Osaka-shi, Osaka, 5328686 (JP); MORI, Masaaki TAKEDA PHARMACEUTICAL CO. LTD., baraki, 3004247 (JP); KIMURA, Eiji TAKEDA PHARMACEUTICAL CO. LTD.,, Ibaraki, 3004247 (JP); IMAI, Tomomi TAKEDA PHARMACEUTICAL CO. LTD., Osaka, 5328686 (JP); ITO, Fumio TAKEDA PHARMACEUTICAL CO. LTD., Ibaraki, 3004247 (JP); OGI, Kazuhiro TAKEDA PHARMACEUTICAL CO. LTD., Ibaraki, 3004247 (JP); SUGO, Tsukasa TAKEDA PHARMACEUTICAL CO. LTD., Ibaraki, 3004247 (JP); KOBAYASHI, Hiromi TAKEDA PHARMACEUTICAL CO. LTD., Ibaraki, 3004247 (JP); HAYASE, Yoji TAKEDA PHARMACEUTICAL CO. LTD., Ibararki, 3004247 (JP)
(74) Representative: Jones, Nicholas Andrew
(86) International application number: PCT/JP2006/303357
(87) International publication number: WO 2006/088246

(57) **Abstract**

The present invention provides a GPR receptor function regulator comprising the compound represented by the formula: 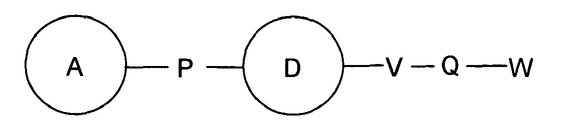 [wherein ring A is an optionally substituted isocyclic or heterocyclic ring, P is a bond or spacer, ring D is an optionally substituted monocyclic aromatic ring which may be condensed with a 5-to 7-membered ring, V is a bond or the group represented by the formula -CR¹⁴=CR¹⁵ - or - N=CR¹⁶- (wherein R¹⁴, R¹⁵ and R¹⁶ each represents a hydrogen atom or optionally substituted hydrocarbon group), Q is a bond or spacer, and W is a carboxyl or a group biologically equivalent to a carboxyl] or its salt or a prodrug thereof

SEQUENCE LISTING

<110> Takeda Pharmaceutical Company Limited

<120> Regulating agent of GPR34 receptor function

<130> G07-0047

<140> PCT/JP2006/303357
<141> 2006-02-17

<150> JP2005-41775
<151> 2005-02-18

<150> JP2005-315146
<151> 2005-10-28

<160> 12

<210> 1
<211> 381
<212> PRT
<213> Homo sapiens

<400> 1

Met Arg Ser His Thr Ile Thr Met Thr Thr Thr Ser Val Ser Ser Trp
                5                   10                  15
Pro Tyr Ser Ser His Arg Met Arg Phe Ile Thr Asn His Ser Asp Gln
            20                  25                  30
Pro Pro Gln Asn Phe Ser Ala Thr Pro Asn Val Thr Thr Cys Pro Met
        35                  40                  45
Asp Glu Lys Leu Leu Ser Thr Val Leu Thr Thr Ser Tyr Ser Val Ile
    50                  55                  60
Phe Ile Val Gly Leu Val Gly Asn Ile Ile Ala Leu Tyr Val Phe Leu
65                  70                  75                  80

Gly Ile His Arg Lys Arg Asn Ser Ile Gln Ile Tyr Leu Leu Asn Val
                85                  90                  95
Ala Ile Ala Asp Leu Leu Leu Ile Phe Cys Leu Pro Phe Arg Ile Met
            100                 105                 110
Tyr His Ile Asn Gln Asn Lys Trp Thr Leu Gly Val Ile Leu Cys Lys
        115                 120                 125
Val Val Gly Thr Leu Phe Tyr Met Asn Met Tyr Ile Ser Ile Ile Leu
    130                 135                 140
Leu Gly Phe Ile Ser Leu Asp Arg Tyr Ile Lys Ile Asn Arg Ser Ile
145                 150                 155                 160
Gln Gln Arg Lys Ala Ile Thr Thr Lys Gln Ser Ile Tyr Val Cys Cys
                165                 170                 175
Ile Val Trp Met Leu Ala Leu Gly Gly Phe Leu Thr Met Ile Ile Leu
            180                 185                 190
Thr Leu Lys Lys Gly Gly His Asn Ser Thr Met Cys Phe His Tyr Arg
        195                 200                 205
Asp Lys His Asn Ala Lys Gly Glu Ala Ile Phe Asn Phe Ile Leu Val
    210                 215                 220
Val Met Phe Trp Leu Ile Phe Leu Leu Ile Ile Leu Ser Tyr Ile Lys
225                 230                 235                 240
Ile Gly Lys Asn Leu Leu Arg Ile Ser Lys Arg Arg Ser Lys Phe Pro
                245                 250                 255
Asn Ser Gly Lys Tyr Ala Thr Thr Ala Arg Asn Ser Phe Ile Val Leu
            260                 265                 270
Ile Ile Phe Thr Ile Cys Phe Val Pro Tyr His Ala Phe Arg Phe Ile
        275                 280                 285
Tyr Ile Ser Ser Gln Leu Asn Val Ser Ser Cys Tyr Trp Lys Glu Ile
    290                 295                 300
Val His Lys Thr Asn Glu Ile Met Leu Val Leu Ser Ser Phe Asn Ser
305                 310                 315                 320
Cys Leu Asp Pro Val Met Tyr Phe Leu Met Ser Ser Asn Ile Arg Lys
                325                 330                 335
Ile Met Cys Gln Leu Leu Phe Arg Arg Phe Gln Gly Glu Pro Ser Arg
            340                 345                 350
Ser Glu Ser Thr Ser Glu Phe Lys Pro Gly Tyr Ser Leu His Asp Thr
        355                 360                 365

Ser Val Ala Val Lys Ile Gln Ser Ser Ser Lys Ser Thr
    370                 375                 380

<210> 2
<211> 1143
<212> DNA
<213> Homo sapiens

<400> 2

atgagaagtc ataccataac aatgacgaca acttcagtca gcagctggcc ttactcctcc      60
cacagaatgc gctttataac caatcatagc gaccaaccgc cacaaaactt ctcagcaaca     120
ccaaatgtta ctacctgtcc catggatgaa aaattgctat ctactgtgtt aaccacatcc     180
tactctgtta ttttcatcgt gggactggtt gggaacataa tcgccctcta tgtatttctg     240
ggtattcacc gtaaaagaaa ttccattcaa atttatctac ttaacgtagc cattgcagac     300
ctcctactca tcttctgcct ccctttccga ataatgtatc atattaacca aaacaagtgg     360
acactaggtg tgattctgtg caaggttgtg ggaacactgt tttatatgaa catgtacatt     420
agcattattt tgcttggatt catcagtttg gatcgctata taaaaattaa tcggtctata     480
cagcaacgga aggcaataac aaccaaacaa agtatttatg tctgttgtat agtatggatg     540
cttgctcttg gtggattcct aactatgatt attttaacac ttaagaaagg agggcataat     600
tccacaatgt gtttccatta cagagataag cataacgcaa aaggagaagc catttttaac     660
ttcattcttg tggtaatgtt ctggctaatt ttcttactaa taatcctttc atatattaag     720
attgggaaga atctattgag gatttctaaa aggaggtcaa aatttcctaa ttctggtaaa     780
tatgccacta cagctcgtaa ctcctttatt gtacttatca tttttactat atgttttgtt     840
ccctatcatg cctttcgatt catctacatt tcttcacagc taaatgtatc atcttgctac     900
tggaaagaaa ttgttcacaa aaccaatgag atcatgctgg ttctctcatc tttcaatagt     960
tgcttagatc cagtcatgta tttcctgatg tccagtaaca ttcgcaaaat aatgtgccaa    1020
cttcttttta gacgatttca aggtgaacca agtaggagtg aaagcacttc agaatttaaa    1080
ccaggatact ccctgcatga tacatctgtg gcagtgaaaa tacagtctag ttctaaaagt    1140
act                                                                  1143

<210> 3
<211> 27
<212> DNA
<213> Artificial Sequence

<220>
<223> Designed oligonucleotide primer to amplify DNA encoding GPR34

<400> 3
atctgtcgac atgagaagtc ataccat 27

<210> 4
<211> 30
<212> DNA
<213> Artificial Sequence

<220>
<223> Designed oligonucleotide primer to amplify DNA encoding GPR34

<400> 4
tatgactagt tcaagtactt ttagaactag 30

<210> 5
<211> 31
<212> DNA
<213> Artificial Sequence

<220>
<223> Primer

<400> 5
agtcgacccg actgaggcca ccgcaccatg g 31

<210> 6
<211> 31
<212> DNA
<213> Artificial Sequence

<220>
<223> Primer

<400> 6
tactagtcct gggtcacagc aggttgatct c                                    31

<210> 7
<211> 1163
<212> DNA
<213> Homo sapiens

<400> 7
gtcgacccga ctgaggccac cgcaccatgg cccgctcgct gacctggcgc tgctgcccct      60
ggtgcctgac ggaggatgag aaggccgccg cccgggtgga ccaggagatc aacaggatcc     120
tcttggagca gaagaagcag gaccgcgggg agctgaagct gctgcttttg ggcccaggcg     180
agagcgggaa gagcaccttc atcaagcaga tgcggatcat ccacggcgcc ggctactcgg     240
aggaggagcg caagggcttc cggcccctgg tctaccagaa catcttcgtg tccatgcggg     300
ccatgatcga ggccatggag cggctgcaga ttccattcag caggcccgag agcaagcacc     360
acgctagcct ggtcatgagc caggacccct ataaagtgac cacgtttgag aagcgctacg     420
ctgcggccat gcagtggctg tggagggatg ccggcatccg ggcctgctat gagcgtcggc     480
gggaattcca cctgctcgat tcagccgtgt actacctgtc ccacctggag cgcatcaccg     540
aggagggcta cgtccccaca gctcaggacg tgctccgcag ccgcatgccc accactggca     600
tcaacgagta ctgcttctcc gtgcagaaaa ccaacctgcg gatcgtggac gtcgggggcc     660
agaagtcaga gcgtaagaaa tggatccatt gtttcgagaa cgtgatcgcc ctcatctacc     720
tggcctcact gagtgaatac gaccagtgcc tggaggagaa caaccaggag aaccgcatga     780
aggagagcct cgcattgttt gggactatcc tggaactacc ctggttcaaa agcacatccg     840
tcatcctctt tctcaacaaa accgacatcc tggaggagaa aatccccacc tcccacctgg     900
ctacctattt ccccagtttc cagggcccta agcaggatgc tgaggcagcc aagaggttca     960
tcctggacat gtacacgagg atgtacaccg ggtgcgtgga cggccccgag ggcagcaaga    1020
agggcgcacg atcccgacgc ctcttcagcc actacacatg tgccacagac acacagaaca    1080
tccgcaaggt cttcaaggac gtgcgggact cggtgctcgc ccgctacctg gacgagatca    1140
acctgctgtg acccaggact agt                                            1163

<210> 8
<211> 30
<212> DNA
<213> Artificial Sequence

<220>

<223> Primer

<400> 8
gtcgacggcc accgcaccat ggcccgctcg                                        30

<210> 9
<211> 1155
<212> DNA
<213> Homo sapiens

<400> 9
gtcgacggcc accgcaccat ggcccgctcg ctgacctggc gctgctgccc ctggtgcctg      60
acggaggatg agaaggccgc cgcccgggtg gaccaggaga tcaacaggat cctcttggag     120
cagaagaagc aggaccgcgg ggagctgaag ctgctgcttt tgggcccagg cgagagcggg     180
aagagcacct tcatcaagca gatgcggatc atccacggcg ccggctactc ggaggaggag     240
cgcaagggct tccggcccct ggtctaccag aacatcttcg tgtccatgcg ggccatgatc     300
gaggccatgg agcggctgca gattccattc agcaggcccg agagcaagca ccacgctagc     360
ctggtcatga gccaggaccc ctataaagtg accacgtttg agaagcgcta cgctgcggcc     420
atgcagtggc tgtggaggga tgccggcatc cgggcctgct atgagcgtcg gcgggaattc     480
cacctgctcg attcagccgt gtactacctg tcccacctgg agcgcatcac cgaggagggc     540
tacgtcccca cagctcagga cgtgctccgc agccgcatgc ccaccactgg catcaacgag     600
tactgcttct ccgtgcagaa aaccaacctg cggatcgtgg acgtcggggg ccagaagtca     660
gagcgtaaga aatggatcca ttgtttcgag aacgtgatcg ccctcatcta cctggcctca     720
ctgagtgaat acgaccagtg cctggaggag aacaaccagg agaaccgcat gaaggagagc     780
ctcgcattgt ttgggactat cctggaacta ccctggttca aaagcacatc cgtcatcctc     840
tttctcaaca aaaccgacat cctggaggag aaaatcccca cctcccacct ggctacctat     900
ttccccagtt tccagggccc taagcaggat gctgaggcag ccaagaggtt catcctggac     960
atgtacacga ggatgtacac cgggtgcgtg gacggccccg agggcagcaa gaagggcgca    1020
cgatcccgac gcctcttcag ccactacaca tgtgccacag acacacagaa catccgcaag    1080
gtcttcaagg acgtgcggga ctcggtgctc gcccgctacc tggacgagat caacctgctg    1140
tgacccagga ctagt                                                     1155

<210> 10
<211> 19
<212> DNA
<213> Artificial Sequence

<220>
<223> Primer

<400> 10
cggctgcaga ttccattca                                                    19

<210> 11
<211> 20
<212> DNA
<213> Artificial Sequence

<220>
<223> Primer

<400> 11
tggctcatga ccaggctagc                                                   20

<210> 12
<211> 21
<212> DNA
<213> Artificial Sequence

<220>
<223> Probe

<400> 12
caggcccgag agcaagcacc a                                                 21

## Description

### TECHNICAL FIELD

The present invention relates to a compound that exerts a preventive/therapeutic effect against immune disease, inflammatory disease, allergic disease, respiratory disease, urinary tract disease, cardiovascular disease and the like by regulating the function and particularly by inhibiting the function of the GPR34 receptor.

### BACKGROUND OF INVENTION

Endogenous ligands of the G protein-coupled receptor GPR34 are lisophosphatidylserine and other lipids which have histamine release activity in rat mast cells stimulated with antigen or concanavalin A and also act to release histamine in cooperation with nerve growth factors in rat mast cells (WO 03/52414). GPR34 antagonists are useful for example as histamine release inhibitors and as preventive/treatment agents for immune disorders including inflammatory disorders (e.g., pituitary abscess, thyroiditis, peritonitis, Crohn's disease, ulcerative colitis, erythema nodosum, chronic rheumatoid arthritis, systemic lupus erythematosus and the like), allergies, asthma, exudative otitis media, Ménière's disease, allergic conjunctivitis, contact dermatitis, allergic rhinitis, anaphylaxis, hives, myasthenia gravis, glomerulonephritis, Sjögren's syndrome, insulin resistant diabetes, atopic dermatitis, leukocyte abnormalities and the like, as well as edema, acid indigestion and the like (WO 03/52414).

The following bicyclic compounds are also known.
(1) The following compound is described in Bioorganic & Medicinal Chemistry Vol. 10, No. 7, pp. 2291-2295, 2002, but its action and use have not been described. 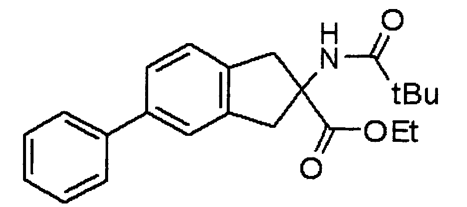
(2) The following compound is described in Synthesis, pp. 339-342, 2002, but its action and use have not been described. 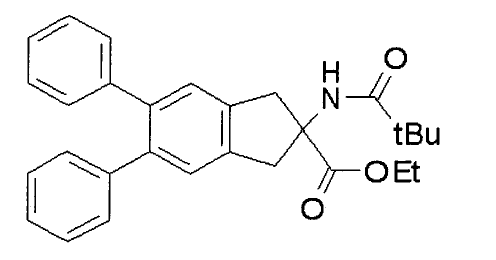
(3) The following compound having an antimicrobial effect is described in WO 2004/18428: 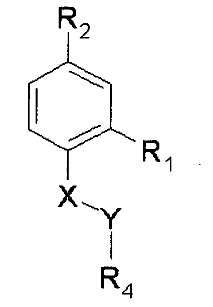 (wherein X is =NH and Y is =CO, CS or -C(=N-CN) or X and Y together form an alkene or C₃₋₅ cycloalkyl; R₁ is -COOH; R₂ is an electron attractive group; and R₄ is an optional substituted heterocyclic group (HET), but the HET may not simultaneously be substituted with a sulfonamide and a urea or thiourea).
(4) The following compound, which participates in 2-phenoxyacetoanilide solubility improvement, is described in IP.com Journal Vol. 3, No. 10, p. 15, 2003. 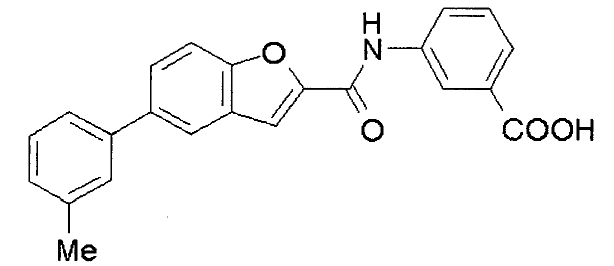
(5) The following compounds, which are fibrinogen receptor antagonists, are described in WO 94/08962: 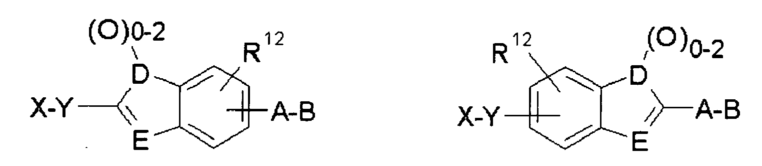 (wherein D and E are selected independently from C, N, O and S; X is selected from 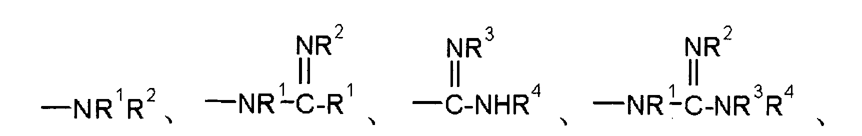 and the 5- and 6-membered monocyclic or bicyclic aromatic or non-aromatic ring which may be substituted with R¹, R², R³ or R⁴, and which contain 0, 1 or 2 hetero groups selected from N, O and S;
   Y and A are independently selected from (CH₂)m, (CH₂)mCONR₃(CH₂)n, (CH₂)mNR₃CO(CH₂)n, (CH₂)mO(CH₂)n, (CH₂)mCO(CH₂)n, (CH₂)mCS(CH₂)n, (CH₂)mSO₂(CH₂)n, (CH₂)mS(CH₂)n, (CH₂)mSO(CH₂)n, (CH₂)mSO₂NR₃(CH₂)n, (CH₂)_{M}NR₃SO₂(CH₂)n, (CH₂)mCR₃=CR₄(CH₂)n, (CH₂)mC=C(CH₂)n, (CH₂)mCH(CH₂)n, (CH₂)m aryl(CH₂)n and (CH₂)mNR₃(CH₂)n and the like;
   m and n are independently integers selected from 0 to 6;
   B is selected from 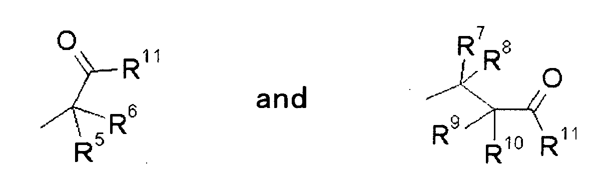 R⁵, R⁶, R⁷, R⁸, R⁹ and R¹⁰ are independently selected from hydrogen, fluorine, and the C₁-₈ alkyl, hydroxyl and hydroxy C₁₋₆ alkyl group and the like; and
   R¹¹ is selected from hydrogen, the C₁₋₈ alkyloxy, aryl C₀₋₆ alkyloxy, C₁₋₈ alkylcarbonyloxy C₁₋₄ alkyloxy, aryl C₁₋₈ alkylcarbonyloxy and C₁₋₄ alkyloxy and the amide bound L- or D-amino acids (wherein the carboxylic acid group is free or esterified with a C₁₋₆ alkyl)).
(6) WO 2005/30773 describes the following compound, which has PAR2 inhibitory activity and is useful for the treatment and prevention of inflammatory disorders, allergic disorders, respiratory disorders, cardiovascular disorders, neural disorders, neuroinflammatory disorders, skin disorders and the like, and which also has prostaglandin E2 production inhibitory activity and is useful in the treatment and prevention of chronic rheumatoid arthritis and the like and as an anti-itching medicine: 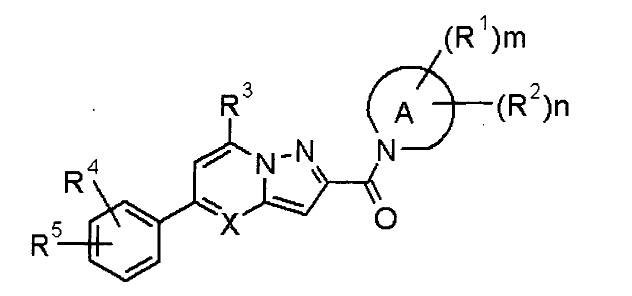 (wherein R¹ is a hydroxyl, carboxyl or the like, m is an integer from 1 to 3, R² is an alkyl, alkoxy or the like, n is an integer from 0 to 4, ring A is a 5- to 8-membered saturated or unsaturated nitrogen-containing heterocyclic ring, R³ is a haloalkyl or the like, R⁴ and R⁵ are each independently hydrogen atoms, halogen atom or alkyl or the like, and X is a methyl, nitrogen atom or the like).
(7) Japanese Patent Application laid-open No. H7-252254 and US Patent Publication 5614531 describes the following compound, which inhibits binding of fibrinogen to the corresponding receptor, and which is useful for the treatment of thrombosis, osteoporosis, tumors, seizures, myocardial infarction, inflammation, arteriosclerosis and osteolytic disorders: 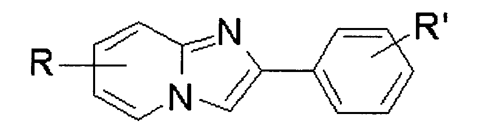 (wherein R is Q or COX;
   R' is Q when R is COX and COX when R is Q;
   Q is NO₂, NH₂ CN, CSNH₂, C(=NH)S-A, C(=NH)NHOH, C(=NH)-NH₂, CH₂-NH₂, CH₂NH-C(=NH)-NH, NH-C(=NH)-NH₂, CH₂NHCO-alk-NH₂, CH₂NHCO-Ar-C(=NH)NH₂, CH₂NHCO-Ar-CH₂-NH₂ or D;
   D is 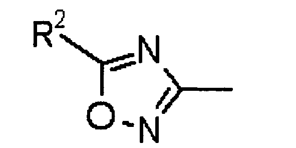 X is OH, OA, AA, AA-AA', 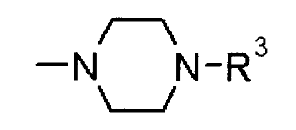 or 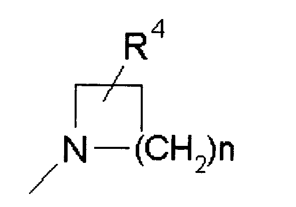 AA or AA' is independently an amino acid residue selected from group consisting of Ala, β-Ala, Arg, Asn, Asp, Gln, Glu, Gly, Leu, Lys, Orn, Phe, Pro, Sar, Ser, Thr, Tyr, Val, C-allyl-Gly, N-phenethyl-Gly, N-benzyl- β-Ala, N-methyl- β-Ala and N-phenethyl- β-Ala, wherein a free amino or carboxyl may also be provided together with a known protective group,
   R² is OH, A or Ar; R³ is -(CH₂)m-COOR⁵; R⁴ is -(CH₂)p-COOR⁵ or -(CH₂)q-O-(CH₂)r-COOR⁵ R⁵ is H or A; m is 1, 2 or 3; n is 1, 2, 3 or 4; p is 0, 1 or 2; r is 1 or 2; A is a C₁₋₆ alkyl; - alk- is a C₁₋₆ alkylene; and Ar is a phenyl).

### DISCLOSURE OF THE INVENTION

Because conventionally used preventive/therapeutic agents for immune disease, inflammatory disease, respiratory disease, urinary tract disease, cardiovascular disease and the like have not been sufficiently effective, there is demand for safe and superior preventive/therapeutic agents of this kind.

As a result of exhaustive research to solve these problems, the inventors discovered that a compound which is characterised by the chemical structure that the ring D of the chemical backbone of the formula: 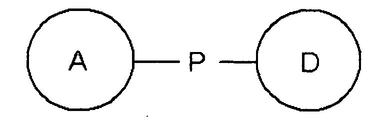 [wherein ring A is an optionally substituted isocyclic or heterocyclic ring, P is a bond or spacer, and ring D is an optionally substituted monocyclic aromatic ring which may be condensed with a 5- to 7-membered ring], has a group represented by the formula -V-Q-W (wherein V is a bond or the group represented by the formula -CR¹⁴ =CR¹⁵- or -N=CR¹⁶ - (wherein R¹⁴ , R¹⁵ and R¹⁶ are each hydrogen atoms or optionally substituted hydrocarbon group), Q is a bond or spacer, and W is a carboxyl or a group biologically equivalent to a carboxyl), which is represented by the formula: 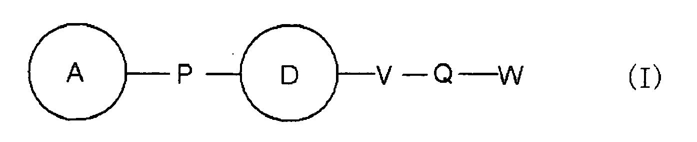 [wherein ring A is an optionally substituted isocyclic or heterocyclic ring,
P is a bond or spacer,
ring D is an optionally substituted monocyclic aromatic ring, and the ring D may be condensed with a 5- to 7-membered ring,
V is a bond or the group represented by the formula -CR¹⁴=CR¹⁵- or -N=CR¹⁶- (wherein R¹⁴, R¹⁵ and R¹⁶ are each hydrogen atom or optionally substituted hydrocarbon group),
Q is a bond or spacer, and
W is a carboxyl or a group biologically equivalent to a carboxyl, and
the group represented by the formula -V-Q-W is substituted at any position on ring D, and
V is the group represented by the formula -CR¹⁴=CR¹⁵- or -N=CR¹⁶- when the group represented by -V-Q-W and the group represented by the formula: 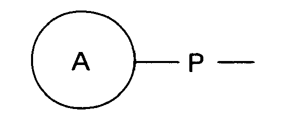 are substituted on the same ring], or a salt thereof (hereunder sometimes abbreviated as "Compound (I)"), has unexpectedly good GPR34 antagonist activity derived from its unique chemical structure, and of the Compounds (I), the inventors for the first time synthesized the compound represented by the formula: 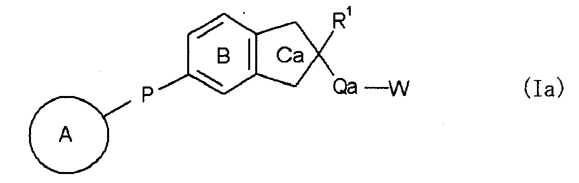 [wherein A is an optionally substituted isocyclic or heterocyclic ring,
ring B is an optionally substituted benzene ring,
ring Ca is a cyclopentene ring which may also include a substituent other than R¹ and the group represented by -Qa-W (wherein Qa is a bond or -CO-Qa'- (with Qa' being a spacer having a terminal carbon atom to which a carboxyl or a group biologically equivalent to a carboxyl is bound), and W is a carboxyl or a group biologically equivalent to a carboxyl),
P is a bond or spacer, and
R¹ is an optionally substituted amino], or a salt thereof (hereunder sometimes abbreviated as "Compound (Ia)", the compound represented by the formula: 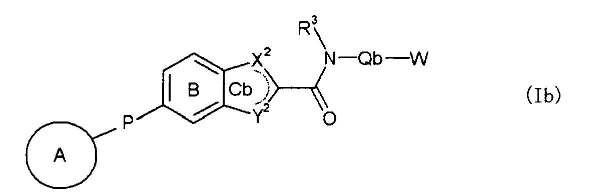 [wherein ring A is an optionally substituted isocyclic or heterocyclic ring,
ring B is an optionally substituted benzene ring,
ring Cb is an optionally substituted 5- or 6-membered ring,
one of X² and Y² is -O-, -S-, -NR⁴-, -O-CH₂-, -S-CH₂- or -NR⁴-CH₂- (wherein R⁴ is a hydrogen atom, optionally substituted hydrocarbon group or substituted sulfonyl) while the other is -N= or -CR⁵= (wherein R⁵ is a hydrogen atom or substituent),
R³ is a hydrogen atom or optionally substituted hydrocarbon group, or R³ and Qb may be bound together to form a ring,
Qb is an optionally substituted chain spacer,
W is a carboxyl or a group biologically equivalent to a carboxyl
P is a bond or spacer, and
any one of the broken lines represents a double bond], or a salt thereof (hereunder sometimes abbreviated as "Compound (Ib)"), the compound represented by the formula: 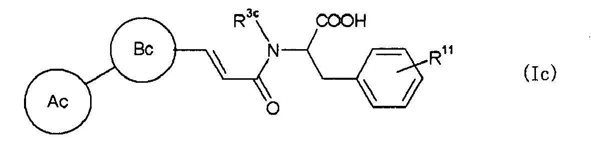 (wherein ring Ac is an optionally substituted 5- or 6-membered aromatic ring,
ring Bc is an optionally substituted 5- or 6-membered aromatic ring,
R^{3c} is a hydrogen atom or optionally substituted hydrocarbon group, and
R¹¹ is a substituent), or a salt thereof (hereunder sometimes abbreviated as "Compound (Ic)") and the compound represented by the formula: 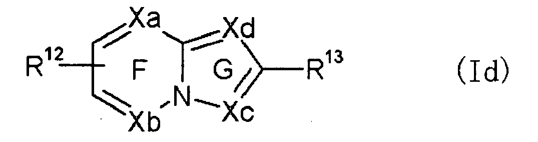 [wherein ring F and ring G form an aromatic condensed azole ring which may also have substituents other than R¹² and R¹³,
Xa, Xb, Xc and Xd are each CH or N,
one of R¹² and R¹³ is the group represented by the formula: 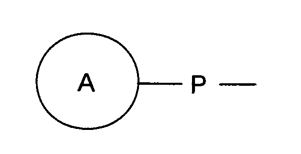 (wherein ring A is an optionally substituted isocyclic or heterocyclic ring and P is a bond or spacer) while the other is the group represented by the formula: 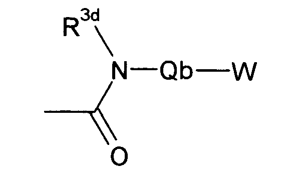 (wherein R^{3d} is a hydrogen atom or optionally substituted hydrocarbon group,
Qb is an optionally substituted chain spacer and
W is a carboxyl or a group biologically equivalent to a carboxyl)], or a salt thereof, with the proviso that R¹³ is not a phenyl having a substituent selected from NO₂ NH₂, CN, CSNH₂, C(=NH)S-C₁₋₆ alkyl, C(=NH)NHOH, C(=NH)-NH₂, CH₂-NH₂, CH₂NH-C(=NH)-NH₂, NH-C(=NH)-NH₂, CH₂NHCO-C₁₋₆ alkylene-NH₂, CH₂NHCO-phenylene-C(=NH)NH₂, CH₂NHCO-phenylene-CH₂-NH₂, 5-hydroxy-1,2,4-oxadiazole-3-yl, 5-C₁₋₆ alkyl-1,2,4-oxadiazole-3-yl and 5-phenyl-1,2,4-oxadiazole-3-yl, in the case where Xa, Xb and Xc are CH and Xd is N, (hereunder sometimes abbreviated as "Compound (Id)"), discovered that these are useful as a pharmaceutical such as safe and superior mast cell degranulation inhibitor, histamine release inhibitor, eicosanoid production inhibitor, mast cell proliferation and differentiation inhibitor, IL-13 production inhibitor and other drugs and as preventive/therapeutic drugs for immune disease, inflammatory disease, allergic disease, respiratory disease, urinary tract disease, cardiovascular disease and the like, and accomplished the present invention based on these findings.

That is, the present invention relates to a pharmaceutical comprising:
[1] A GPR34 receptor function regulator comprising Compound (I) or a prodrug thereof,
[2] The regulator according to [1] above, wherein ring D is an optionally substituted condensed ring formed from a 5- to 7-membered ring and a monocyclic aromatic ring,
[3] The regulator according to [1] above, containing the compound represented by the formula: 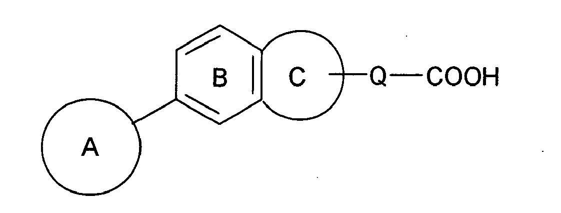 (wherein ring A and Q are as defined above,
   ring B is an optionally substituted benzene ring,
   ring C is a 5- to 7-membered isocyclic or heterocyclic ring which may also include a substituent other than the group represented by the formula -Q-COOH, and
   the group represented by the formula -Q-COOH is substituted at any position on ring C) or its salt or a prodrug thereof,
[4] The regulator according to [1] above, which is a GPR34 receptor antagonist,
[5] The regulator according to [1] above, which is a mast cell degranulation inhibitor, histamine release inhibitor, eicosanoid production inhibitor, mast cell proliferation and differentiation inhibitor or IL-13 production inhibitor,
[6] The regulator according to [1] above, which is a preventive/therapeutic agent for immune disease, inflammatory disease, allergic disease, respiratory disease, urinary disease, central disease or cardiovascular disease,
[7] Compound (Ia),
[8] The compound according to [7] above, wherein P is a bond or a spacer having two atoms in the main chain,
[9] The compound according to [7] above, wherein P is a bond and W is a carboxyl,
[10] The compound according to [7] above, wherein ring A is an optionally substituted aromatic ring,
[11] The compound according to [7] above, wherein R¹ is a carbonylamino having a substituent or a sulfonylamino having a substituent,
[12] The compound according to [11] above, wherein the substituent is a group having an optionally substituted aromatic group,
[13] The compound according to [7] above, wherein R¹ is a group represented by the formula: 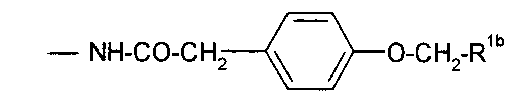 (wherein R^{1b} is an optionally substituted aromatic group),
[14] The compound according to [7] above, wherein either Qa is a bond or Qa'-W is an amino acid,
[15] The compound according to [7] above, represented by the formula: 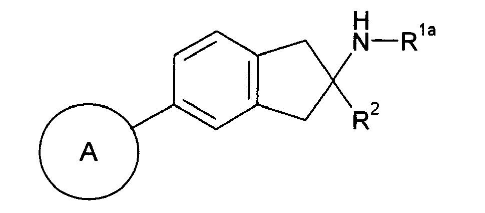 (wherein R^{1a} is either
   (i) a carbonyl having a substituent selected from (1) alkyl which have optionally substituted aromatic group and the alkyl may be further substituted, (2) alkyl which have optionally substituted non-aromatic heterocyclic group and the alkyl may be further substituted, (3) alkyl which have optionally substituted mercapto and which may themselves be further substituted, (4) optionally substituted aromatic group, (5) amino having optionally substituted aromatic group, (6) optionally substituted cycloalkyl, (7) optionally substituted nitrogen-containing non-aromatic heterocyclic group, (8) optionally substituted alkyl and (9) optionally substituted alkenyl, or
   (ii) an alkylsulfonyl which has an optionally substituted aromatic group and the alkylsulfonyl may itself be further substituted,
      R² is a carboxyl, a group biological equivalent to a carboxyl or the group represented by the formula CO-Z-OH (wherein Z-OH is an amino acid), and
      ring A is as defined above),
[16] The compound according to [7] above, wherein
   ring A is a 5- or 6-membered aromatic ring optionally substituted with 1 to 3 group selected from halogen atom, optionally halogenated alkyl and an optionally halogenated alkoxy,
   ring B is a benzene ring having 1 to 3 substituents selected from halogen atom, alkyl and alkoxy,
   ring Ca is an unsubstituted cyclopentene ring,
   P is a bond, ethylene, ethenylene or ethynylene,
   R¹ is an acylamino having an aromatic group, and
   Qa-W is a carboxyl or the group represented by CO-Z-OH (wherein Z-OH is an amino acid),
[16a] The compound according to [16] above, wherein P is a bond,
[17] The compound according to [15] above, wherein
   ring A is a 5- or 6-membered aromatic ring which may have 1 to 3 substituents selected from (a) halogen atom, (b) optionally halogenated C₁₋₆ alkyl, (c) optionally halogenated C₁₋₆ alkoxy and (d) amino optionally substituted with 1 or 2 groups selected from C₁₋₆ alkyl-carbonyl and C₁₋₆ alkyl,
   R^{1a} is:
   (i) a carbonyl having a substituent selected from
      (1) C₁₋₆ alkyl having 5- to 10-membered aromatic group which may have 1 to 3 substituents selected from (a) halogen atom, (b) C₁₋₆ alkylsulfonyl, (c) C₁₋₆ alkoxy optionally substituted with 5- or 6-membered aromatic heterocyclic group which may have C₁₋₆ alkyl, (d) C₇₋₁₃ aralkyloxy which may have 1 to 3 substituents selected from halogen atom, C₁₋₆ alkoxy and optionally halogenated C₁₋₆ alkyl, (e) 5- to 10-membered non-aromatic isocyclic ring-oxy, (f) optionally halogenated C₆₋₁₂ aryloxy, (g) 5- or 6-membered aromatic heterocyclic ring-oxy, (h) optionally halogenated C₆₋₁₂ aryl-carbonylamino, (i) optionally halogenated C₇₋₁₃ aralkylamino and (j) C₆₋₁₂ aryl, and optionally having 1 to 3 substituents selected from amino which may have hydroxyl and C₁₋₆ alkoxy-carbonyl,
      (2) C₁₋₆ alkyl having 6-membered nitrogen-containing non-aromatic heterocyclic group which may have 1 or 2 substituents selected from (a) C₁₋₆ alkyl which may have 1 or 2 optionally halogenated C₆₋₁₂ aryl (b) C₆₋₁₂ aryl, (c) optionally halogenated C₁₋₁₂ aryl-carbonyl, and (d) optionally halogenated C₇₋₁₃ aralkyl-carbonyl,
      (3) C₁₋₆ alkyl having 5- to 10-membered aromatic mercapto,
      (4) 5- to 10-membered aromatic group which may have 1 to 3 substituents selected from halogen atom, the C₁₋₆ alkyl which may have 5- to 10-membered aromatic heterocyclic group optionally substituted with C₁₋₆ alkyl, and the optionally halogenated C₆₋₁₂ aryl,
      (5) amino having optionally halogenated C₆₋₁₂ aryl,
      (6) C₃₋₆ cycloalkyl,
      (7) optionally oxonated 5-membered nitrogen-containing non-aromatic heterocyclic group,
      (8) C₁₋₆ alkyl which may have 1 or 2 substituents selected from optionally oxonated 5-membered nitrogen-containing non-aromatic heterocyclic group and C₇₋₁₃ aralkyloxy, C₁₋₆ alkoxy, carboxy, C₁₋₆ alkoxy-carbonyl and amino group, and
      (9) C₂₋₆ alkenyl, or
   (ii) an optionally halogenated C₇₋₁₃ aralkylsulfonyl,
      and R² is a carboxy-C₁₋₆ alkyl-carbamoyl, carboxyl, 5-tetrazolyl or 5-tetrazolylaminocarbonyl,
[17a] The compound according to [15] above, wherein
   ring A is a 5- or 6-membered aromatic ring which may have 1 to 3 substituents selected from (a) halogen atom, (b) optionally halogenated C₁₋₆ alkyl, (c) optionally halogenated C₁₋₆ alkoxy and (d) amino optionally substituted with 1 or 2 groups selected from C₁₋₆ alkyl-carbonyl and C₁₋₆ alkyl (and is preferably a benzene ring or thiophene ring),
   R^{1a} is:
   (i) a carbonyl having a substituent selected from
      (1) C₁₋₆ alkyl having 5- to 10-membered aromatic group which may have 1 to 3 substituents selected from (a) halogen atom, (b) C₁₋₆ alkylsulfonyl, (c) C₁₋₆ alkoxy optionally substituted with 5- or 6-membered aromatic heterocyclic group which may have C₁₋₆ alkyl, (d) C₇₋₁₃ aralkyloxy which may have 1 to 3 substituents selected from halogen atom, C₁₋₆ alkoxy and optionally halogenated C₁₋₆ alkyl, (e) 5- to 10-membered non-aromatic isocyclic ring-oxy, (f) optionally halogenated C₆₋₁₂ aryloxy, (g) 5- or 6-membered aromatic heterocyclic ring-oxy, (h) optionally halogenated C₆₋₁₂ aryl-carbonylamino, (i) optionally halogenated C₇₋₁₃ aralkylamino and (j) C₆₋₁₂ aryl, and optionally having 1 to 3 substituents selected from amino which may have hydroxyl and C₁₋₆ alkoxy-carbonyl,
      (2) C₁₋₆ alkyl having 6-membered nitrogen-containing non-aromatic heterocyclic group which may have 1 or 2 substituents selected from (a) C₁₋₆ alkyl which may have 1 or 2 optionally halogenated C₆₋₁₂ aryl (b) C₆₋₁₂ aryl, (c) optionally halogenated C₁₋₁₂ aryl-carbonyl, and (d) optionally halogenated C₇₋₁₃ aralkyl-carbonyl,
      (3) C₁₋₆ alkyl having 5- to 10-membered aromatic mercapto,
      (4) 5- to 10-membered aromatic group which may have 1 to 3 substituents selected from halogen atom, the C₁₋₆ alkyl which may have 5- to 10-membered aromatic heterocyclic group optionally substituted with C₁₋₆ alkyl, and the optionally halogenated C₆₋₁₂ aryl,
      (5) amino having optionally halogenated C₆₋₁₂ aryl,
      (6) C₃₋₆ cycloalkyl,
      (7) optionally oxonated 5-membered nitrogen-containing non-aromatic heterocyclic group,
      (8) C₁₋₆ alkyl which may have 1 or 2 substituents selected from optionally oxonated 5-membered nitrogen-containing non-aromatic heterocyclic group and C₇₋₁₃ aralkyloxy, C₁₋₆ alkoxy, carboxy, C₁₋₆ alkoxy-carbonyl and amino group, and
      (9) C₂₋₆ alkenyl, or
   (ii) an optionally halogenated C₇₋₁₃ aralkylsulfonyl,
      and R² is a carboxy-C₁₋₆ alkyl-carbamoyl or carboxyl,
[18] The compound according to [7] above, which is 5-(4-chlorophenyl)-2-[({4-[(4-fluorobenzyl)oxy]phenyl}acetyl)amino]indan-2-carboxylic acid,
[19] A prodrug of the compound according to [7] above,
[20] A pharmaceutical comprising the compound according to [7] above or a prodrug thereof
[21] Compound (Ib),
[22] The compound according to [21] above, wherein ring A is an optionally substituted aromatic ring,
[23] The compound according to [21] above, wherein ring A is an optionally substituted 5- or 6-membered aromatic ring,
[24] The compound according to [21] above, wherein one of X² and Y² is -O-, -S-, - NR⁴- or -S-CH₂-, while the other is -N= or -CR⁵=,
[25] The compound according to [21] above, wherein R³ is a hydrogen atom or C₁₋₆ alkyl,
[26] The compound according to [21] above, wherein Qb is an optionally substituted C₁₋₃ alkylene,
[27] The compound according to [26] above, wherein the substituent is a group having an optionally substituted aromatic group,
[28] The compound according to [26] above wherein the optionally substituted C₁₋₃ alkylene is a methylene having an optionally substituted benzyl,
[29] The compound according to [21] above, wherein Qb is the group represented by the formula: 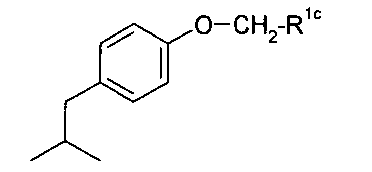 (wherein R^{1c} is an optionally substituted aromatic group),
[30] The compound according to [21] above, wherein P is a bond or a spacer having two atoms in the main chain,
[31] The compound according to [30] above, wherein the spacer having two atoms in the main chain is -CH₂CH₂-, -CH=CH-, -C≡C-, -CH₂O-, -CH₂S-, -CH₂SO- or -CH₂SO₂-,
[32] The compound according to [21] above, wherein W is a carboxyl, 5-tetrazolyl or 5-tetrazolylaminocarbonyl,
[33] The compound according to [20] above, represented by the formula: 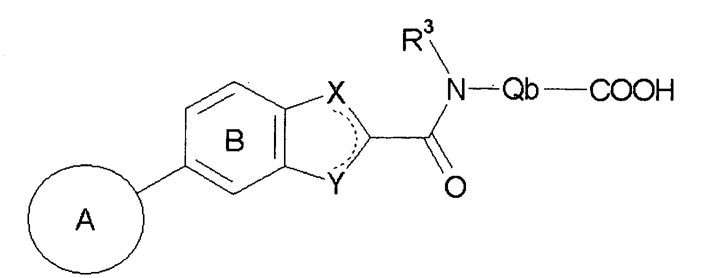 (wherein one of X and Y is -O-, -S- or -NR⁴- while the other is -N= or -CR⁵=,
   ring A, ring B, R³, R⁴, R⁵ and Qb are as defined above, and
   any one of the broken lines represents a double bond),
[34] The compound according to [33] above, wherein one of X and Y is -O- or -Sand the other is -CR⁵=,
[35] The compound according to [34] above, wherein R⁵ is a hydrogen atom or hydrocarbon group,
[36] The compound according to [21] above, wherein:
   ring A is a 5- or 6-membered aromatic ring optionally substituted with 1 to 3 groups selected from halogen atom, optionally halogenated C₁₋₆ alkyl and optionally halogenated C₁₋₆ alkoxy,
   ring B is a benzene ring optionally substituted with 1 to 3 groups selected from halogen atom, C₁₋₆ alkyl and C₁₋₆ alkoxy,
   one of X² and Y² is -O-, -S-, -NR^{4a}- or -S-CH₂-, while the other is -N= or -CR^{5a}=,
      R^{4a} is a hydrogen atom or C₁₋₆ alkyl,
      R^{5a} is hydrogen atom, halogen atom, amino, C₁₋₆ alkyl-carbonylamino, C₁₋₆ alkoxy-carbonylamino or C₁₋₆ alkyl,
      R³ is a hydrogen atom or C₁₋₆ alkyl, or R³ and Qb may be bound together to form a 5- to 7-membered ring,
      Qb is an optionally substituted C₁₋₃ alkylene or NR³-Qb-W is an amino acid,
      P is a bond, ethylene, ethenylene or ethynylene, and
      W is a carboxyl, 5-tetrazolyl or 5-tetrazolylaminocarbonyl,
[37] The compound according to [21] above, represented by the formula: 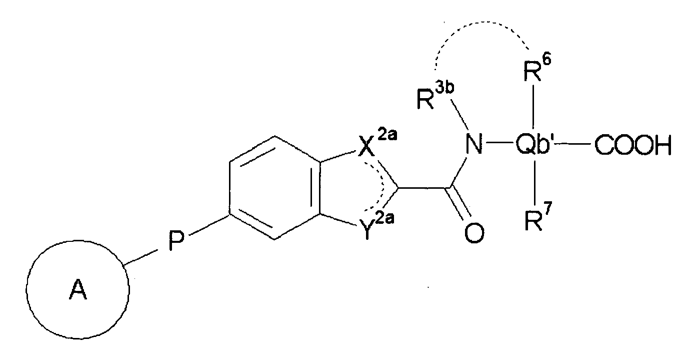 (wherein P is a bond, ethenylene or ethynylene,
   ring A is a 5- or 6-membered aromatic ring optionally substituted with 1 to 3 groups selected from (1) halogen atom, (2) C₁₋₆ alkyl optionally substituted with 1 to 3 groups selected from (a) 5- or 6-membered aromatic heterocyclic group which may have C₁₋₆ alkyl and may be condensed with benzene rings and (b) halogens, and (3) amino optionally substituted with 1 or 2 groups selected from C₁₋₆ alkyl and C₁₋₆ alkyl-carbonyl,
   one of X^{2a} and Y^{2a} is -O-, -S-, -NH-, -NH-CO- or -S-CH₂- while the other is -N= or -CR^{5b}=,
   R^{5b} is a hydrogen atom, amino or C₁₋₆ alkyl,
   one of the broken lines in the ring represents a double bond,
   R^{3b} is a hydrogen atom or C₁₋₆ alkyl,
   Qb' is a C₁₋₃ alkylene,
   R⁶ and R⁷ are located on the same or different carbon atoms and are each
   (1) hydrogen atoms,
   (2) C₁₋₆ alkyl optionally substituted with 1 to 3 groups selected from C₁₋₆ alkoxy-carbonyl, C₇₁₃ aralkyloxy-carbonyl, hydroxyl, carboxyl and C₁₋₆ alkylthio group and the 5- or 6-membered aromatic heterocyclic group optionally condensed with benzene rings,
   (3) C₇₋₁₃ aralkyl which may have 1 to 3 substituents selected from (a) the C₇₋₁₃ aralkyloxy optionally substituted with 1 or 2 groups selected from halogen atom, cyano, optionally halogenated C₁₋₆ alkyl and C₁₋₆ alkoxy and (b) the 5- or 6-membered aromatic heterocyclic ring-C₁₋₃ alkoxy,
   (4) C₆₋₁₂ aryl or
   (5) 5- or 6- member aromatic heterocyclic group optionally condensed with benzene rings, or alternatively
      R⁶ and R⁷ are bound together to form, together with an adjacent carbon atom, a C₃₋₇ cycloalkane optionally condensed with a C₆₋₁₂ aryl which may have 1 or 2 substituents selected from optionally halogenated C₆₋₁₂ aryl and halogens, or 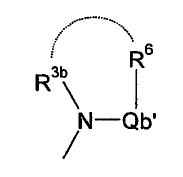 represents a 5- or 6-membered saturated cyclic amino),
[37a] The compound according to [21] above, represented by the formula: 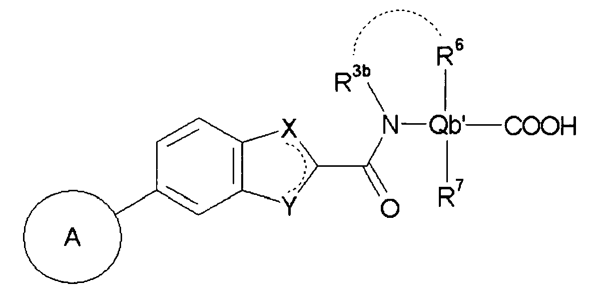 (wherein ring A is a 5- or 6-membered aromatic ring optionally substituted with 1 to 3 groups selected from (1) halogen atom, (2) C₁₋₆ alkyl optionally substituted with 1 to 3 groups selected from (a) 5- or 6-membered aromatic heterocyclic group which may have C₁₋₆ alkyl and may be condensed with benzene rings and (b) halogens, and (3) amino optionally substituted with 1 or 2 groups selected from C₁₋₆ alkyl and C₁₋₆ alkyl-carbonyl,
   one of X and Y is -O- or -S-, while the other is -CR^{5b}= (wherein R^{5b} is a hydrogen atom or C₁₋₆ alkyl)
   one of the broken lines in the ring represents a double bond,
   R^{3b} is a hydrogen atom,
   Qb' is a C₁₋₃ alkylene,
   R⁶ and R⁷ are located on the same or different carbon atoms and each represents a
   (1) hydrogen atom,
   (2) C₁₋₆ alkyl optionally substituted with 1 to 3 groups selected from C₁₋₆ alkoxy-carbonyl, C₇₁₃ aralkyloxy-carbonyl, hydroxyl, carboxyl and C₁₋₆ alkylthio group and the 5- or 6-membered aromatic heterocyclic group optionally condensed with benzene ring,
   (3) C₇₋₁₃ aralkyl which may have 1 to 3 substituents selected from optionally halogenated C₇₋₁₃ aralkyloxy and the 5- or 6-membered aromatic heterocyclic ring-C₁₋₃ alkoxy,
   (4) C₆₋₁₂ aryl or
   (5) 5- or 6- member aromatic heterocyclic group optionally condensed with benzene rings, or alternatively
      R⁶ and R⁷ are bound together to form, together with an adjacent carbon atom, a C₃₋₇ cycloalkane optionally condensed with a C₆₋₁₂ aryl which may have 1 or 2 substituents selected from optionally halogenated C₆₋₁₂ aryl and halogens, or 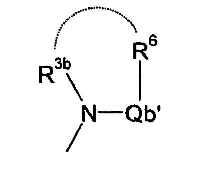 represents a 5- or 6-membered saturated cyclic amino),
[38] The compound according to [21] above, which is N-{[5-(4-chlorophenyl)-1-benzothiophen-2-yl]carbonyl}-O-(4-fluorobenzyl)tyrosine or (2S)-[4-(benzyloxy)phenyl]({[6-(4-chlorophenyl)-3-methyl-1-benzofuran-2-yl]carbonyl}amino) acetic acid,
[39] A prodrug of the compound according to [21] above,
[40] A pharmaceutical comprising the compound according to [21] above or a prodrug thereof,
[41] Compound (Ic),
[42] The compound according to [41] above, wherein 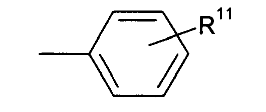 is the group represented by the formula: 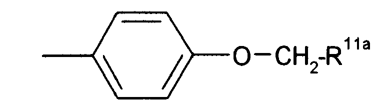 (wherein R^{11a} is an optionally substituted aromatic group),
[43] The compound according to [41] above, wherein ring Ac is an optionally substituted benzene ring,
[44] The compound according to [41] above, wherein ring Ac is an optionally halogenated benzene ring,
   ring Bc is a 5- or 6-membered aromatic ring,
   R^{3c} is a hydrogen atom, and
   R¹¹ is an optionally halogenated C₇₋₁₃ aralkyloxy,
[45] The compound according to [41] above, which is O-benzyl-N-[(2E)-3-(4'-chlorobiphenyl-4-yl)propa-2-enoyl]tyrosine,
[46] A prodrug of the compound according to [41] above,
[47] A pharmaceutical comprising the compound according to [41] above or a prodrug thereof,
[48] Compound (Id),
[49] The compound according to [48] above, wherein the aromatic condensed azole ring is 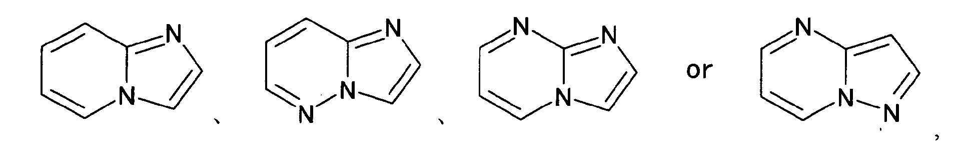
[50] The compound according to [48] above, wherein ring A is a 5- or 6-membered aromatic ring optionally substituted with 1 to 3 groups selected from halogen atom, optionally halogenated alkyl and optionally halogenated alkoxy,
[51] The compound according to [48] above, wherein P is a bond or a spacer having two atoms in the main chain,
[52] The compound according to [51] above, wherein the spacer having two atoms in the main chain is -CH₂CH₂-, -CH=CH-, -C≡C-, -CH₂O-, -CH₂S-, -CH₂SO- or -CH₂SO₂-,
[53] The compound according to [48] above, wherein NR^{3d}-Qb-W is an amino acid,
[54] The compound according to [48] above, wherein Qb is a methylene having a substituent having an aromatic,
[55] The compound according to [48] above, wherein Qb is the group represented by the formula: 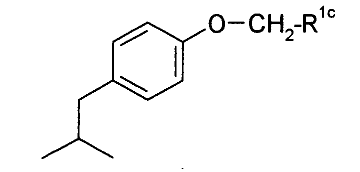 (wherein R^{1c} is an optionally substituted aromatic group),
[56] The compound according to [48] above, wherein W is a carboxyl, 5-tetrazolyl or 5-tetrazolylaminocarbonyl,
[57] The compound according to [48] above, represented by the formula: 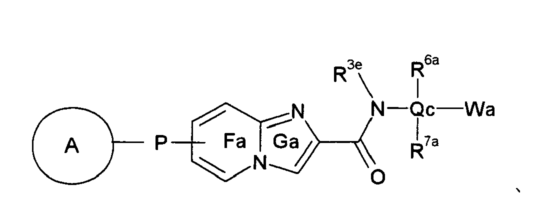 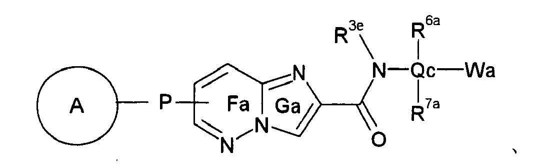 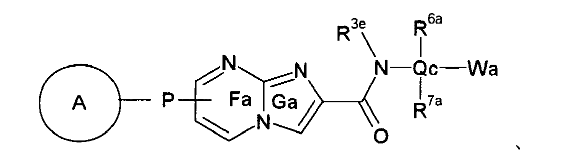 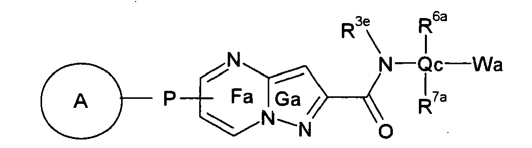 or 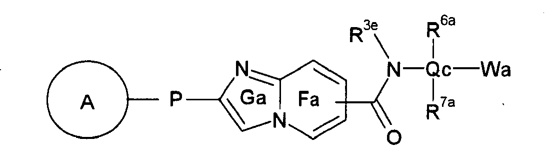 [wherein ring A is a 5- or 6-membered aromatic ring optionally substituted with 1 or 2 groups selected from halogen atom, optionally halogenated C₁₋₆ alkyl, optionally halogenated C₁₋₆ alkoxy and cyano,
   P is a bond, ethylene, ethenylene, ethynylene, -CH₂-O- or -CH₂-S-,
   ring Fa and ring Ga is a ring which may each have 1 or 2 substituents selected from halogen atom and optionally halogenated C₁₋₆ alkyl,
   R^{3e} is a hydrogen atom or C₁₋₆ alkyl or a group represented by the formula: 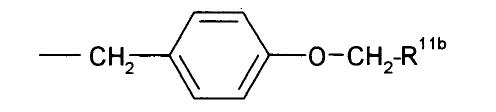 (wherein R^{11b} is a 5- or 6-membered aromatic group optionally substituted with 1 to 3 groups selected from halogen atom, optionally halogenated C₁₋₆ alkyl, optionally halogenated C₁₋₆ alkoxy and cyano)
   Qc is a C₁₋₃ alkylene,
   R^{6a} and R^{7a} are located on the same or different carbon atoms and are each hydrogen atoms or group represented by the formula: 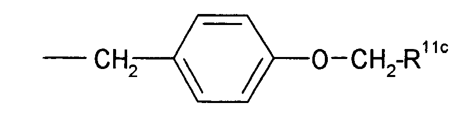 (wherein R^{11c} is a 5- or 6-membered aromatic group which may have 1 to 3 substituents selected from halogen atom, cyano, optionally halogenated C₁₋₆ alkyl and optionally halogenated C₁₋₆ alkoxy), or
   R^{6a} and R^{7a} are bound together to form, together with an adjacent carbon atom, a C₃₋₇ cycloalkane optionally condensed with a C₆₋₁₂ aryl which may have 1 or 2 halogen atoms, and
   Wa is a carboxyl, C₁₋₆ alkylsulfonylaminocarbonyl, 5-tetrazolyl, 5-tetrazolylaminocarbonyl or 5-oxo-1,2,4-oxadiazole-3-yl),
[58] The compound according to [57] above, wherein R^{3e} is the group represented by the formula: 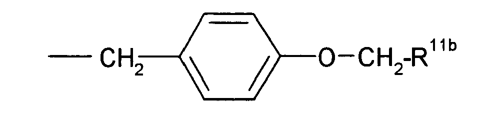 (wherein all symbols are as defined in [57] above), and/or
   at least one of R^{6a} and R^{7a} is the group represented by the formula: 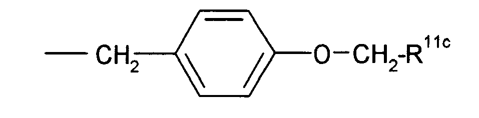 (wherein all symbols are as defined in [57] above),
[59] The compound according to [48] above, which is
   O-benzyl-N-{[6-(4-chlorophenyl)imidazo[1,2-b] pyridazine-2-yl]carbonyl}tyrosine,
   O-benzyl-N-{[6-(phenylethynyl)imidazo[1,2-b] pyridazine-2-yl]carbonyl} tyrosine,
   O-benzyl-N-{[6-[(E)-2-(4-chlorophenyl)ethenyl]imidazo[1,2-b] pyridazine-2-yl}carbonyl) tyrosine,
   O-benzyl-N-{[7-(4-chlorophenyl)imidazo[1,2-a] pyridine-2-yl]carbonyl}-N-methyltyrosine,
   N-{[7-(4-chlorophenyl)imidazo[1,2-a]pyridine-2-yl]carbonyl}-O-(4-methylbenzyl) tyrosine, or
   O-(4-chlorobenzyl)-N-{[7-(4-chlorophenyl)imidazo[1,2-a] pyridine-2-yl]carbonyl} tyrosine,
[60] A prodrug of the compound according to [48] above, and
[61] A pharmaceutical comprising the compound according to [48] above or a prodrug thereof.

Due to its unique chemical structure, Compound (I) of the present invention has excellent GPR34 function regulating activity such as GPR34 antagonist activity, mast cell degranulation-inhibiting action, histamine release-inhibiting action, leukotriene production-inhibiting action, prostaglandin production-inhibiting action, IL-13 production-inhibiting action, tryptase secretion-inhibiting action, antigen-antibody reaction-inhibiting action and the like, and has low toxicity and few side-effects. Consequently, Compound (I) is useful as a safe medicament, such as for example a GPR34 receptor antagonist (including inverse agonist or partial agonist), mast cell degranulation inhibitor, histamine release inhibitor, eicosanoid production inhibitor, mast cell proliferation inhibitor, IL-13 production inhibitor, tryptase secretion inhibitor or antigen-antibody reaction inhibitor; or a preventive/therapeutic agent for immune disorders [for example, inflammatory disease (e.g., pituitary abscess, thyroiditis, peritonitis, Crohn's disease, ulcerative colitis, erythema nodosum, chronic rheumatoid arthritis, systemic lupus erythematosus and the like), allergies (e.g., allergic conjunctivitis, allergic rhinitis, hay fever, metal allergies and the like), asthma, exudative otitis media, Ménière's disease, contact dermatitis, anaphylaxis, hives, myasthenia gravis, glomerulonephritis, Sjögren's syndrome, Basedow's disease, insulin resistant diabetes, atopic dermatitis, leukocyte abnormalities and the like], respiratory disease [for example, chronic obstructive pulmonary disease (e.g., chronic bronchitis or pulmonary edema), diffuse panbronchiolitis, cystic fibrosis, hypersensitivity pneumonitis, idiopathic interstitial pneumonia, pulmonary fibrosis and the like], urinary tract disease (e.g., renal tubulointerstitial disease (fibrosis), interstitial cystitis, allergic cystitis and the like), cardiovascular disease (e.g., arteriosclerosis, acute coronary syndrome, atherosclerotic aortic aneurysm, cardiac anaphylaxis, heart failure, myocardial infarction, angina, arrhythmia, deep phlebothrombosis, post-PTCA restenosis and the like), ophthalmological disease (e.g., pterygium, vernal conjunctivitis, dry eye and the like), cancer (e.g., papillary thyroid carcinoma, non-small cell lung cancer, endometrial cancer, cervical cancer, stomach cancer, pancreatic cancer, lung cancer, kidney cancer, liver cancer, ovarian cancer, prostate cancer, bladder cancer, breast cancer, colon cancer, rectal cancer, Kaposi's sarcoma, mastocytoma and the like), digestive disease [including chronic liver disease, food allergies, allergic enteritis, milk protein-induced proctitis, digestive ulcers (e.g., stomach ulcer, duodenal ulcer, stomal ulcer, Zollinger-Ellison syndrome and the like), gastritis, reflux esophagitis, NUD (non-ulcer dyspepsia), gastric MALT lymphoma, ulcers caused by non-steroidal anti-inflammatory drugs, hyperacidity, ulcers and hyperacidity caused by post-surgical stress and the like], cerebral infarction, hyperlipidemia, acute renal failure, diabetes, obesity, edema, granuloma, atopic myelitis, neurofibroma, nasal mucosal hypersensitivity, Hodgkin's disease, endometrial hyperplasia, central disease [for example, neurodegenerative disease (e.g., Alzheimer's disease (familial Alzheimer's disease, early-onset Alzheimer's disease, sporadic Alzheimer's disease, etc.), Parkinson's disease, Down's syndrome, amyotrophic lateral sclerosis, prion disease (Creutzfeldt-Jakob disease), Huntington's chorea, diabetic neuropathy, multiple sclerosis and the like), psychological disease (e.g., schizophrenia, depression, bipolar disorder, anxiety disorder, attention deficit hyperactivity disorder, panic disorder and the like), and cerebrovascular disease (e.g., cerebral thrombosis, cerebral infarction, transient ischemic attack, etc.) and the like] and others.

### BRIEF DESCRIPTION OF THE DRAWINGS

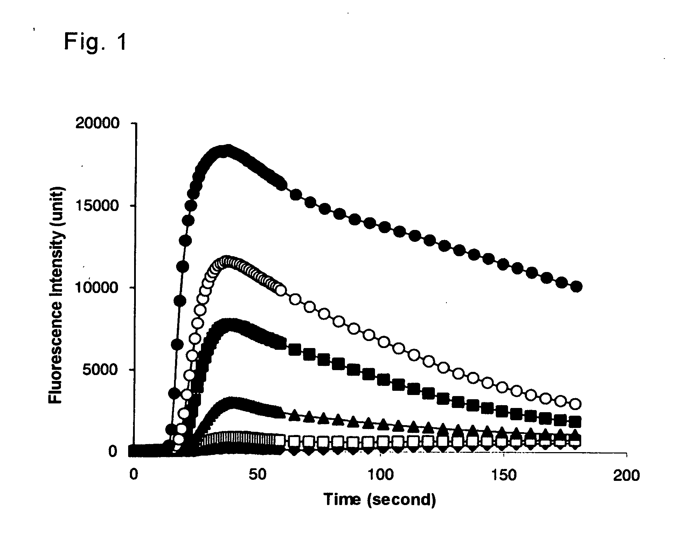

Figure 1 shows changes in calcium levels caused by lisophosphatidylserine stimulus in human GPR34 CHO cells stably expressing Gα16, In the figure, a black circle indicates a lysoPS concentration of 10 µM, a white circle indicates a lysoPS concentration of 1 µM, a black square indicates a lysoPS concentration of 0.6 µM, a black triangle indicates a lysoPS concentration of 0.3 µM, a white square indicates a lysoPS concentration of 0.1 µM, and a black rhomboid indicates a lysoPS concentration of 0 µM.

### DESCRIPTION OF THE PREFERRED EMBODIMENTS

In the present specification, a "hydrocarbon group" includes, for example, a chain or cyclic hydrocarbon group (e.g., an alkyl, alkenyl, alkynyl, cycloalkyl, cycloalkenyl, aryl, aralkyl or polycyclic hydrocarbon group) or the like. Among these, a chain or cyclic hydrocarbon group with 1 to 19 carbon atoms is preferred.

The aforementioned "alkyl" includes, for example, a C₁₋₆ alkyl (e.g., a methyl, ethyl, propyl, isopropyl, butyl, isobutyl, sec-butyl, tert-butyl, pentyl, neopentyl or hexyl) or the like.

The aforementioned "alkenyl" includes, for example, a C₂₋₆ alkenyl (e.g., a vinyl, allyl, isopropenyl, 1-butenyl, 2-butenyl, 3-butenyl, 2-methyl-2-propenyl, 1-methyl-2-propenyl or 2-methyl-1-propenyl) or the like.

The aforementioned "alkynyl" includes, for example, a C₂₋₆ alkynyl (e.g., an ethynyl, propargyl, 1-butynyl, 2-butynyl, 3-butynyl or 1-hexynyl) or the like.

The aforementioned "cycloalkyl" includes, for example, a C₃₋₆ cycloalkyl (e.g., a cyclopropyl, cyclobutyl, cyclopentyl or cyclohexyl) or the like.

The aforementioned "cycloalkenyl" includes, for example, a C₅₋₆ cycloalkenyl (e.g., a 1-cyclopentenyl, 3-cyclopentenyl, 4-cyclopentenyl, 1-cyclohexenyl, 3-cyclohexenyl or 4-cyclohexenyl) or the like.

The aforementioned "aryl" includes, for example, a C₆₋₁₄ aryl (e.g., a phenyl, 1-naphthyl, 2-naphthyl, 2-biphenylyl, 3-biphenylyl, 4-biphenylyl, 2-anthryl or 3-indenyl) or the like.

The aforementioned "aralkyl" includes, for example, a C₇₋₁₉ aralkyl (e.g., a benzyl, phenethyl, diphenylmethyl, trityl, 1-naphthylmethyl, 2-naphthylmethyl, 2,2-diphenylethyl, 3-phenylpropyl, 4-phenylbutyl, 5-phenylpentyl or 9-fluorenyl) or the like.

The aforementioned "polycyclic hydrocarbon group" includes, for example, a bi- to tetracyclic non-aromatic hydrocarbon group (e.g., a 1-adamantyl, 2-adamantyl, decalin-1-yl, tetralin-1-yl, indan-1-yl, androstan-3-yl or 5-androsten-3-yl) or the like.

In the present specification, a "heterocyclic group" includes, for example, a 3- to 14-membered (monocyclic, bicyclic or tricyclic) heterocyclic ring containing 1 to 4 hetero atoms of 1 or 2 elements selected from nitrogen, sulfur and oxygen in addition to carbon atoms, and is preferably a univalent group obtained by removing any one hydrogen atom from a (i) 5- to 14-member (preferably 5- to 10-membered) aromatic heterocyclic ring, (ii) 3- to 14-membered non-aromatic heterocyclic ring or (iii) 7- to 10-membered heterocyclic bridge ring or the like.

Examples of the aforementioned "5- to 14-membered (preferably 5- to 10-membered) aromatic heterocyclic ring" include for example thiophene, benzo[b]thiophene, benzo[b]furan, benzimidazole, benzoxazole, benzothiazole, benzisothiazole, 1H-benzotriazole, naphtho[2,3-b]thiophene, furan, pyrrole, imidazole, pyrazole, oxazole, 1,2,3-triazole, 1,2,4-triazole, tetrazole, 1,3,4-thiadiazole, 1,3,4-oxadiazole, pyridine, pyrazine, pyrimidine, pyridazine, indole, isoindole, 1H-indazole, purine, 4H-quinolidine, isoquinoline, quinoline, phthalazine, naphthyridine, quinoxaline, quinazoline, cinnoline, carbazole, beta-carboline, phenanthridine, acridine, phenazine, thiazole, isothiazole, phenothiazine, isoxazole, furazan, phenoxazine and other aromatic heterocyclic rings, and rings formed by condensing these rings (preferably monocyclic) with 1 or more (preferably 1 or 2) aromatic rings (e.g., benzene ring, pyridine ring or imidazole ring, etc.) and the like.

Examples of the aforementioned "3- to 14-membered non-aromatic heterocyclic ring" include oxirane, oxetane, tetrahydrofuran, dihydrofuran, pyrane, dioxolane, dioxane, azetidine, pyrrolidine, imidazoline, pyrazolidine, pyrazoline, piperidine, piperazine, morpholine, thiomorpholine, thiazolidine, oxazolidine, oxadiazoline, thiadiazoline, triazoline, 1,4-diazepan, 1,4-oxazepan, 1,4-thiazepan and reduced and partially reduced forms of the aforementioned aromatic heterocyclic rings (e.g., 1,2,3,4-tetrahydroquinoline, 1,2,3,4-tetrahydroisoquinoline and indoline, etc.) and the like.

The aforementioned "7- to 10-membered heterocyclic bridge group" includes, for example, a quinuclidine or 7-azebicyclo[2.2.1]heptane or the like.

Said "heterocyclic group" is preferably a 5- to 14-membered (preferably 5- to 10-membered) (monocyclic or bicyclic) heterocyclic group having preferably 1 to 4 hetero atoms of 1 or 2 elements selected from nitrogen, sulfur and oxygen in addition to carbon atoms. Specific examples include 2-thienyl, 3-thienyl, 2-furyl, 3-furyl, 2-pyridyl, 3-pyridyl, , 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, pyrazinyl, 2-pyrimidinyl, 4-pyrimidinyl, 1-pyrrolyl, 3-pyrrolyl, 1-imidazolyl, 2-imidazolyl, 3-pyridazinyl, 2-thiazolyl, 2-oxazolyl, 3-isothiazolyl, 3-isoxazolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl and other aromatic heterocyclic groups,for example, 1-pyrrolidinyl, 2-pyrrolidinyl, 3-pyrrolidinyl, 2-imidazolinyl, 4-imidazolinyl, 2-pyrazolidinyl, 3-pyrazolidinyl, 4-pyrazolidinyl, piperidino, 2-piperidyl, 3-piperidyl, 4-piperidyl, 1-piperadinyl, 2-piperadinyl, morpholino, thiomorpholino and other non-aromatic heterocyclic group and the like.

In the present specification, an "optionally halogenated C₁₋₆ alkyl" includes, for example an alkyl (e.g., a methyl, ethyl, propyl, isopropyl, butyl, isobutyl, sec-butyl, tert-butyl, pentyl, hexyl or other C₁₋₆ alkyl, etc.) optionally having 1 to 5 or preferably 1 to 3 halogen atoms (for example, fluorine, chlorine, bromine, iodine, etc.) or the like. Specific examples include methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl, 6,6,6-trifluorohexyl and the like.

In the present specification, an "optionally halogenated C₂₋₆ alkenyl" includes, for example, a C₂₋₆ alkenyl (e.g., a vinyl, propenyl, isopropenyl, 2-buten-1-yl, 4-penten-1-yl, 5-hexen-1-yl, etc.) optionally having 1 to 5 or preferably 1 to 3 halogen atoms (for example, fluorine, chlorine, bromine, iodine, etc.) or the like.

In the present specification, an "optionally halogenated C₂₋₆ alkynyl" includes, for example, a C₂₋₆ alkynyl (e.g., a propargyl, 2-butyn-1-yl, 4-pentyn-1-yl or 5-hexyn-1-yl, etc.) optionally having 1 to 5 or preferably 1 to 3 halogen atoms (for example, fluorine, chlorine, bromine, iodine, etc.) or the like.

In the present specification, the "optionally halogenated C₃₋₈ cycloalkyl" of an "optionally halogenated, optionally condensed C₃₋₈ cycloalkyl" includes, for example, a C₃₋₆ cycloalkyl (e.g., a cyclopropyl, cyclobutyl, cyclopentyl or cyclohexyl, etc.) optionally having 1 to 5 or preferably 1 to 3 halogen atoms (for example, fluorine, chlorine, bromine, iodine, etc.) or the like. Specific examples include cyclopropyl, cyclobutyl, cyclopentyl, cyclohexyl, 4,4-dichlorocyclohexyl, 2,2,3,3-tetrafluorocyclopentyl, 4-chlorocyclohexyl and the like.

In the present specification, the "condensed C₃₋₈ cycloalkyl" of an "optionally halogenated, optionally condensed C₃₋₈ cycloalkyl" includes, for example a 8- to 14-membered bicyclic or tricyclic C₃₋₈ cycloalkyl (e.g., a 1-adamantyl, 2-adamantyl, decalin-1-yl, tetralin-1-yl, 9-fluorenyl, 1-indanyl or 1,2,3,4-tetrahyddro-1-naphthyl, etc.) or the like. Said "condensed C₃₋₈ cycloalkyl" may also itself be halogenated.

In the present specification, an "optionally halogenated C₁₋₈ alkoxy" includes, for example, a C₁₋₈ alkoxy (e.g., a methoxy, ethoxy, propoxy, isopropoxy, butoxy, isobutoxy, sec-butoxy, pentyloxy or hexyloxy, etc.) optionally having 1 to 5 or preferably 1 to 3 halogen atoms (for example, fluorine, chlorine, bromine, iodine, etc.) or the like. Specific examples include methoxy, difluoromethoxy, trifluoromethoxy, ethoxy, 2,2,2-trifluoroethoxy, propoxy, isopropoxy, butoxy, 4,4,4-trifluorobutoxy, isobutoxy, sec-butoxy, pentyloxy, hexyloxy and the like.

In the present specification, an "optionally halogenated C₁₋₆ alkylthio" includes, for example, a C₁₋₆ alkylthio (e.g., a methylthio, ethylthio, propylthio, isopropylthio, butylthio, sec-butylthio or tert-butylthio, etc.) optionally having 1 to 5 or preferably 1 to 3 halogen atoms (for example, fluorine, chlorine, bromine, iodine, etc.) or the like. Specific examples include methylthio, difluoromethylthio, trifluoromethylthio, ethylthio, propylthio, isopropylthio, butylthio, 4,4,4-trifluorobutylthio, pentylthio, hexylthio and the like.

The "isocyclic ring" in the "optionally substituted isocyclic or heterocyclic ring" given for ring A in Compound (I) may be a cycloalkyl, aryl or the like.

A "cycloalkyl" is preferably a C₃₋₆ cycloalkyl (e.g., a cyclopropyl, cyclobutyl, cyclopentyl or cyclohexyl, etc.) or the like.

An "aryl" is preferably a C₆₋₁₄ aryl (e.g., a phenyl, 1-naphthyl, 2-naphthyl, 2-biphenylyl, 3-biphenylyl, 4-biphenylyl or 2-anthryl, etc.) or the like.

The "heterocyclic ring" of the "optionally substituted isocyclic or heterocyclic ring" given for ring A in Compound (I) may be a 3- to 14-membered (monocyclic, bicyclic or tricyclic) heterocyclic ring containing 1 to 4 hetero atoms of 1 or 2 elements selected from nitrogen, sulfur and oxygen in addition to carbon atoms for example, and specifically may be a (i) 5- to 14- member (preferably 5- to 10-membered) aromatic heterocyclic ring, (ii) 3- to 14-membered non-aromatic heterocyclic ring or (iii) a 7- to 10-membered heterocyclic bridge ring.

Examples of the aforementioned "5- to 14-membered (preferably 5- to 10-membered) aromatic heterocyclic ring" include thiophene, benzo[b]thiophene, benzo[b]furan, benzimidazole, benzoxazole, benzothiazole, benzisothiazole, naphtho[2,3-b]thiophene, furan, pyrrole, imidazole, pyrazole, pyridine, pyrazine, pyrimidine, pyridazine, indole, isoindole, 1H-indazole, purine, 4H-quinolidine, isoquinoline, quinoline, phthalazine, naphthyridine, quinoxaline, quinazoline, cinnoline, carbazole, betacarboline, phenanthridine, acridine, phenazine, thiazole, isothiazole, oxazole, 1,2,3-triazole, 1,2,4-triazole, tetrazole, 1,3,4-thiadiazole, 1,3,4-oxadiazole, 1H-benzotriazole, phenothiazine, isoxazole, furazan, phenoxazine and other aromatic heterocyclic rings and rings formed by condensing these rings (preferably monocyclic) with 1 or more (preferably 1 or 2) aromatic rings (e.g., benzene or the like).

Examples of the aforementioned "3- to 14-membered non-aromatic heterocyclic ring" include oxirane, oxetane, tetrahydrofuran, dihydrofuran, pyrane, dioxolane, dioxane, azetidine, pyrrolidine, imidazoline, pyrazolidine, pyrazoline, piperidine, piperazine, morpholine, thiomorpholine, thiazolidine, oxazolidine, oxadiazoline, thiadiazoline, triazoline, 1,4-diazepan, 1,4-oxazepan, 1,4-thiazepan and reduced and partially reduced forms of the aforementioned aromatic heterocyclic rings (e.g., 1,2,3,4-tetrahydroquinoline, 1,2,3,4-tetrahydroisoquinoline and indoline, etc.) and the like.

The aforementioned "7- to 10-membered heterocyclic bridge ring" includes, for example, quinuclidine, 7-azabicyclo[2.2.1] heptane or the like.

The "heterocyclic ring" given for ring A is preferably a 3- to 14-membered (preferably 5-to 10-membered) (monocyclic or bicyclic) heterocyclic ring having preferably 1 to 4 hetero atoms of 1 or 2 elements selected from nitrogen, sulfur and oxygen in addition to carbon atoms or the like. More preferably, it is a 5- or 6-membered heterocyclic ring containing 1 to 3 hetero atoms selected from nitrogen, sulfur and oxygen atoms in addition to carbon atoms. Specific examples include thiophene, furan, pyrrole, imidazole, pyrazole, pyridine, pyrazine, pyrimidine, pyridazine and the like.

A substituent in the "optionally substituted isocyclic or heterocyclic ring" given for ring A may be for example a halogen atom (e.g., fluorine, chlorine, bromine, iodine or the like), a C₁-₃ alkylenedioxy (e.g., a methylenedioxy, ethylenedioxy or the like), nitro, cyano, optionally halogenated C₁₋₆ alkyl, optionally halogenated C₂₋₆ alkenyl, carboxy C₂₋₆ alkenyl (2-carboxyethenyl, 2-carboxy-2-methylethenyl or the like), optionally halogenated C₂₋₆ alkynyl, optionally halogenated optionally condensed C₃₋₈ cycloalkyl, C₆₋₁₄ aryl (phenyl, 1-naphthyl, 2-naphthyl, 2-biphenylyl, 3-biphenylyl, 4-biphenylyl, 2-anthryl or the like), optionally halogenated C₁₋₈ alkoxy, C₁₋₆ alkoxy-carbonyl-C₁₋₆ alkoxy (ethoxycarbonylmethyloxy or the like), C₃₋₈ cycloalkyl-oxy (cyclopropyloxy, cyclopentyloxy, cyclohexyloxy or the like), hydroxyl, C₆₋₁₄ aryloxy (phenyloxy, 1-naphthyloxy, 2-naphthyloxy or the like), C₇₋₁₆ aralkyloxy (benzyloxy, phenethyloxy or the like), mercapto, optionally halogenated C₁₋₆ alkylthio, C₆₋₁₄ arylthio (phenylthio, 1-naphthylthio, 2-naphthylthio or the like), C₇₋₁₆ aralkylthio (benzylthio, phenylthio or the like), amino, hydroxyamino, mono-C₁₋₆ aralkylamino (methylamino, ethylamino or the like), mono-C₆₋₁₄ arylamino (phenylamino, 1-naphthylamino, 2-naphthylamino or the like), di-C₁₋₆ alkylamino (dimethylamino, diethylamino, ethylmethylamino or the like), di-C₆₋₁₄ arylamino (diphenylamino or the like), formyl, carboxyl, C₁₋₆ alkyl-carbonyl (acetyl, propionyl or the like), C₃₋₈ cycloalkyl-carbonyl (cyclopropylcarbonyl, cyclopentylcarbonyl, cyclohexylcarbonyl or the like), C₁₋₆ alkoxy-carbonyl (methoxycarbonyl, ethoxycarbonyl, propoxycarbonyl, tert-butoxycarbonyl or the like), C₆₋₁₄ aryl-carbonyl (benzoyl, 1-naphthoyl, 2-naphthoyl or the like), C₇₋₁₆ aralkyl-carbonyl (phenylacetyl, 3-phenylproponyl or the like), C₆₋₁₄ aryloxy-carbonyl (phenoxycarbonyl or the like), C₇₋₁₆ aralkyloxy-carbonyl (benzyloxycarbonyl, phenethyloxycarbonyl or the like), 5- or 6-membered heterocyclic carbonyl (nicotinoyl, isonicotinoyl, thenoyl, furoyl, morpholinocarbonyl, thiomorpholinocarbonyl, piperazine-1-ylcarbonyl, pyrrolidine-1-ylcarbonyl or the like), carbamoyl, mono-C₁₋₆ alkyl-carbamoyl (methylcarbamoyl, ethylcarbamoyl or the like), di-C₁₋₆ alkyl-carbamoyl (dimethylcarbamoyl, diethylcarbamoyl, ethyl methyl carbamoyl or the like), C₆₋₁₄ aryl-carbamoyl (phenylcarbamoyl, 1-naphthylcarbamoyl, 2-naphthylcarbamoyl or the like), C₁₋₆ alkoxy-carbamoyl (methoxycarbamoyl, ethoxycarbamoyl or the like), 5- or 6-membered heterocyclic carbamoyl (2-pyridylcarbamoyl, 3-pyridylcarbamoyl, 4-pyridylcarbamoyl, 2-thienylcarbamoyl, 3-thienylcarbamoyl or the like), sulfo, C₁₋₆ alkylsulfonyl (methylsulfonyl, ethylsulfonyl or the like), C₆₋₁₄ arylsulfonyl (phenylsulfonyl, 1-naphthylsulfonyl, 2-naphthylsulfonyl or the like), formylamino, C₁₋₆ alkyl-carbonylamino (acetylamino or the like), C₆₋₁₄ aryl-carbonylamino (benzoylamino, naphthoylamino or the like), C₁₋₆ alkoxy-carbonylamino (methoxycarbonylamino, ethoxycarbonylamino, propoxycarbonylamino, butoxycarbonylamino or the like), C₁₋₆ alkylsulfonylamino (methylsulfonylamino, ethylsulfonylamino or the like), C₆₋₁₄ arylsulfonylamino (phenylsulfonylamino, 2-naphthylsulfonylamino, 1-naphthylsulfonylamino or the like), C₁₋₆ alkyl-carbonyloxy (acetoxy, propionyloxy or the like), C₆₋₁₄ aryl-carbonyloxy (benzoyloxy, naphthylcarbonyloxy or the like), C₁₋₆ alkoxy-carbonyloxy (methoxycarbonyloxy, ethoxycarbonyloxy, propoxycarbonyloxy, butoxycarbonyloxy or the like), mono-C₁₋₆ alkyl-carbamoyloxy (methylcarbamoyloxy, ethylcarbamoyloxy or the like), di-C₁₋₆ alkyl-carbamoyloxy (dimethylcarbamoyloxy, diethylcarbamoyloxy or the like), C₆₋₁₄ aryl-carbamoyloxy (phenylcarbamoyloxy, naphthylcarbamoyloxy or the like), 5- or 6-membered heterocyclic carbonyloxy (nicotinoyloxy, isonicotinoyloxy or the like), optionally substituted 5-to 7-membered saturated cyclic amino, optionally substituted 5- to 10-membered aromatic heterocyclic group (e.g., a 2-thienyl, 3-thienyl, 2-furyl, 3-furyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl or the like), optionally substituted 3- to 10-membered non-aromatic heterocyclic group (e.g., 1-azetidinyl, 2-azetidinyl, 3-azetidinyl, 1-pyrrolidinyl, 2-pyrrolidinyl, 3-pyrrolidinyl, 2-imidazolinyl, 4-imidazolinyl, 2-pyrazolidinyl, 3-pyrazolidinyl, 4-pyrazolidinyl, 2-piperidyl, 3-piperidyl, 4-piperidyl, 1-piperazinyl, 2-piperazinyl, morpholino, thiomorpholino, 2-oxiranyl, 2-oxetanyl, 3-oxetanyl, 2-tetrahydrofuranyl or 4-tetrahydropyranyl, etc.), oxo or the like.

Ring A may have 1 to 5 or preferably 1 to 3 of these substituents at a substitutable position, and when there are two or more substituents, they may be the same or different.

The "5- to 7-membered saturated cyclic amino" in the "optionally substituted 5- to 7-membered cyclic amino" may be a 5- to 7-membered saturated cyclic amino optionally containing 1 to 4 hetero atoms of 1 or 2 elements selected from nitrogen, sulfur and oxygen in addition to 1 nitrogen atom and carbon atoms, and specific examples include pyrrolidine-1-yl, pyperidino, piperazine-1-yl; morpholino, thiomorpholino, tetrahydroazepine-1-yl, homopiperazine-1-yl and the like.

A substituent in the aforementioned "optionally substituted 5- to 7-membered saturated cyclic amino" includes, for example a C₁₋₆ alkyl (methyl, ethyl, propyl, isopropyl, butyl, isobutyl, sec-butyl, tert-butyl, pentyl, hexyl or the like), C₁₋₆ alkoxy (methoxy, ethoxy, propoxy, isopropoxy, butoxy or the like), halogen atom (for example, fluorine, chlorine, bromine, iodine or the like), hydroxyl, cyano, amino, carboxyl, carbamoyl, C₁₋₆ alkoxy-carbonyl (methoxycarbonyl, ethoxycarbonyl, tert-butoxycarbonyl or the like), C₇₋₁₄ aralkyloxy-carbonyl (benzyloxycarbonyl or the like), C₆₋₁₄ aryl (phenyl, 1-naphthyl, 2-naphthyl, 2-biphenylyl, 3-biphenylyl, 4-biphenylyl, 2-anthryl or the like), C₁₋₆ alkyl-carbonyl (acetyl, propionyl or the like), C₁₋₆ alkyl-sulfonyl (methanesulfonyl, ethanesulfonyl or the like), 5- to 10-membered aromatic heterocyclic group (2-thienyl, 3-thienyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl or the like), oxo or the like. Said "5- to 7-membered saturated cyclic amino" may have 1 to 5 or preferably 1 to 3 such substituents at substitutable positions, and when there are 2 or more substituents they may be the same or different.

A substituent in the aforementioned "optionally substituted 5- to 10-membered aromatic heterocyclic group" and "optionally substituted 3- to 10-membered non-aromatic heterocyclic group" includes, for example a C₁₋₆ alkyl (methyl, ethyl, propyl, isopropyl, butyl, isobutyl, sec-butyl, tert-butyl, pentyl, hexyl or the like), C₁₋₆ alkoxy (methoxy, ethoxy, propoxy, isopropoxy, butoxy or the like), halogen atom (for example, fluorine, chlorine, bromine, iodine or the like), hydroxyl, cyano, amino, carboxyl, carbamoyl, C₁₋₆ alkoxy-carbonyl (methoxycarbonyl, ethoxycarbonyl, tert-butoxycarbonyl or the like), C₇₋₁₄ aralkyloxy-carbonyl (benzyloxycarbonyl or the like), C₆₋₁₄ aryl (phenyl, 1-naphthyl, 2-naphthyl, 2-biphenylyl, 3-biphenylyl, 4-biphenylyl, 2-anthryl or the like), C₁₋₆ alkyl-carbonyl (acetyl, propionyl or the like), C₁₋₆ alkyl-sulfonyl (methanesulfonyl, ethanesulfonyl or the like), 5- to 10-membered aromatic heterocyclic group (2-thienyl, 3-thienyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl or the like), oxo or the like. The "optionally substituted 5- to 10-membered aromatic heterocyclic group" and "optionally substituted 3- to 10-membered non-aromatic heterocyclic group" may have 1 to 5 or preferably 1 to 3 such substituents at substitutable positions, and when there are 2 or more substituents they may be the same or different.

The "spacer" represented by P or Q in Compound (I) may be a bivalent chain hydrocarbon (for example, a bivalent C₁₋₅ chain hydrocarbon such as an alkylene, alkenylene, alkynylene or the like) or a bivalent 3- to 8-membered cyclic group (for example, a bivalent 6-membered cyclic group such as 1,2-phenylene, 1,3-phenylene, 1,4-phenylene, cycloxyane-1,4-diyl, pyridine-2,5-diyl, pyridine-2,4-diyl or piperidine-1,4-diyl, etc.) or the like optionally separated, respectively, by 1 or 2 groups selected from -O-, -S-, -SO-, SO₂- -NQ'-, - CONQ'-, -NQ'CO-, -SO₂NQ'-, -NQ'SO₂-, -NQ'CONQ"-, -CO- and the bivalent heterocyclic group (for example, thiazole-2, 5-diyl, thiophene-2,5-diyl, furan-2,5-diyl, pyridine-2,5-diyl, pyridine-2,4-diyl, pyrimidine-1,3-diyl, pyrrolidine-1,3-diyl, piperidine-1,4-diyl, piperazine-1,4-diyl and the like).

Each of Q' and Q" may be a hydrogen atom, optionally substituted hydrocarbon group, optionally substituted heterocyclic group (for example, a 5- to 14-membered (preferably 5- to 10-membered) (monocyclic or bicyclic) aromatic heterocyclic group (e.g., a 2-thienyl, 3-thienyl, 2-furyl, 3-furyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, pyrazinyl, 2-pyrimidinyl, 4-pyrimidinyl, 3-pyrrolyl, 2-imidazolyl, 3-pyridazinyl, 3-isothiazolyl, 3-isoxazolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl, etc.) or the like).

Examples of the substituents in the "optionally substituted hydrocarbon group" and "optionally substituted heterocyclic group" include those given as substituents for the "optionally substituted isocyclic or heterocyclic ring" represented by ring A above. The "hydrocarbon group" and "heterocyclic group" may have 1 to 3 or preferably 1 or 2 substituents in substitutable positions, and when there are two or more substituents, they may be the same or different.

The "spacer" represented by Q is preferably a spacer with 1 to 5 atoms in the main chain. More preferably, it is the group represented by the formula: 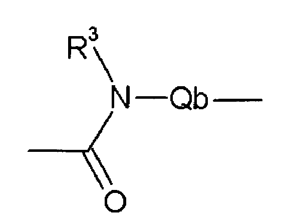 (wherein R³ is a hydrogen atom or optionally substituted hydrocarbon group, or R³ and Qb are bound together to form a ring, and

Qb is an optionally substituted chain spacer) or the like.

"NR³-Qb-W" may also be an amino acid (for example glycine, alanine, beta-alanine, valine, leucine, isoleucine, serine, threonine, aspartic acid, glutamic acid, asparagine, glutamine, lysine, arginine, cysteine, methionine, phenylalanine, tyrosine, tryptophan, histidine, proline or the like) in which the functional group (amino, hydroxyl, thiol, carboxyl, phenolic hydroxyl or the like) may be protected or modified (for example, alkylated, aralkylated, esterified, acylated, amidated or the like). Said amino acid may be an L-amino acid, D-amino acid or DL-amino acid.

Examples of substituents in the "optionally substituted hydrocarbon group" represented by R³ include those given as substituents for the "optionally substituted isocyclic or heterocyclic ring" represented by Ring A above. The hydrocarbon group may have 1 to 3 or preferably 1 or 2 substituents in substitutable positions, and when there are two or more substituents, they may be the same or different.

When R³ and Qb are bound together to form a ring, the ring may be an optionally substituted 5- to 7-membered saturated cyclic amino [for example, a 5- to 7-membered saturated cyclic amino optionally containing 1 to 4 hetero atoms of 1 or 2 elements selected from nitrogen, sulfur and oxygen in addition to nitrogen and carbon atoms (e.g., pyrrolidine-1-yl, piperidino, piperazine-1-yl, morpholino, thiomorpholino or tetrahydroazepine-1-yl, etc.)] or the like. Examples of substituents include C₁₋₆alkyl, C₆₋₁₄aryl, C₁₋₆ alkyl-carbonyl, 5- to 10-membered aromatic heterocyclic (e.g., 2-thienyl, 3-thienyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo[b]thienyl, 3-benzo[b]thienyl, 2-benzo[b]furanyl or 3-benzo[b]furanyl) and oxo group and the like. Said "5-to 7-membered saturated cyclic amino" may have 1 to 5 or preferably 1 to 3 substituents in substitutable positions for example, and when there are two or more substituents, they may be the same or different.

The "optionally substituted chain spacer" represented by Qb may be an optionally substituted spacer with 1 to 3 atoms in the main chain.

A "spacer with 1 to 3 atoms in the main chain" includes, for example, a C₁₋₃ alkylene (e.g., a methylene, ethylene or trimethylene, etc.) or a C₁₋₃ alkenylene (e.g., a vinylene or propenylene, etc.) or the like.

A substituent in an "optionally substituted spacer with 1 to 3 atoms in the main chain" includes, for example an optionally substituted hydrocarbon group (preferably an aryl) or an optionally substituted heterocyclic group [(preferably a 5- to 14-membered (preferably 5- to 10-membered) (monocyclic or bicyclic) aromatic heterocyclic group (e.g., a 2-thienyl, 3-thienyl, 2-furyl, 3-furyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, pyrazinyl, 1-pyrimidinyl, 4-pyrimidinyl, 3-pyrrolyl, 2-imidazolyl, 3-pyridazinyl, 3-isothiazolyl, 3-isoxazolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl, etc.)] or the like.

In the "optionally substituted hydrocarbon group" and "optionally substituted heterocyclic group", examples of substituents include those given as substituents for the "optionally substituted isocyclic or heterocyclic ring" represented by Ring A above. The "hydrocarbon group" and "heterocyclic group" may for example have 1 to 3 or preferably 1 or 2 substituents in substitutable positions for example, and when there are two or more substituents, they may be the same or different.

R³ may preferably be a hydrogen atom or C₁₋₆ alkyl. More preferably, it is a hydrogen atom.

Qb may preferably be an optionally substituted C₁₋₃ alkylene. More preferably, it may be a C₁₋₃ alkylene (e.g., a methylene or ethylene) optionally having an optionally substituted aromatic group (e.g., a C₆₋₁₂ aryl or 5- to 10-membered aromatic heterocyclic group, etc.) or the like. Still more preferably, it may be a methylene optionally having an optionally substituted aromatic group (e.g., a C₆₋₁₂ aryl or 5- to 10-membered aromatic heterocyclic group, etc.) or the like. More preferably still, it may be a methylene optionally having an optionally substituted C₆₋₁₂ aryl, or a methylene optionally having an optionally substituted 5- to 10-membered aromatic heterocyclic group.

When R³ and Qb are bound together to form a ring, the ring may preferably be a 5- to 6-membered saturated cyclic amino or the like.

A more desirable example of "-CO-NR³-Qb-" is the group represented by the formula: 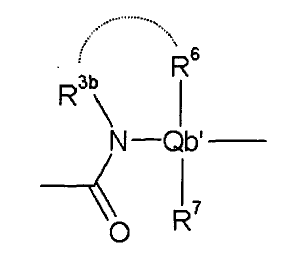 (wherein R^{3b} is a hydrogen atom,
Qb' is a C₁₋₃ alkylene,
R⁶ and R⁷ are located on the same or different carbon atoms and are each
(1) hydrogen atoms,
(2) C₁₋₆ alkyl optionally substituted with 1 to 3 groups selected from C₁₋₆ alkoxy-carbonyl, C₇₋₁₃ aralkyloxy-carbonyl, hydroxyl, carboxyl and C₁₋₆ alkylthio group and the 5- or 6-membered aromatic heterocyclic group optionally condensed with benzene rings,
(3) C₇₋₁₃ aralkyl optionally having 1 to 3 substituents selected from optionally halogenated C₇₋₁₃ aralkyloxy and 5- or 6-membered aromatic heterocyclic-C₁₋₃ alkoxy.
(4)C₆₋₁₂aryl or
(5) 5- or 6- member aromatic heterocyclic group optionally condensed with benzene rings, or alternatively
   R⁶ and R⁷ are bound together to form, together with an adjacent carbon atom, a C₃₋₇ cycloalkane optionally condensed with a C₆₋₁₂ aryl which may have 1 or 2 substituents selected from optionally halogenated C₆₋₁₂ aryl and halogens, or 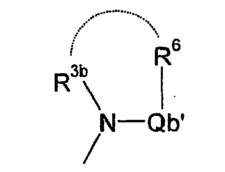 represents a 5- to 7-membered saturated cyclic amino).

In the "optionally substituted monocyclic aromatic ring which may be condensed with a 5- to 7-membered ring" represented by ring D in Compound (I), examples of the "monocyclic aromatic ring" include for example benzene and 5- or 6-membered aromatic heterocyclic rings (e.g., thiophene, furan, pyrrole, imidazole, pyrazole, thiazole, oxazole, 1,2,3-triazole, 1,2,4-triazole, tetrazole, 1,3,4-thiadiazole, 1,3,4-oxadiazole, pyridine, pyrazine, pyrimidine, pyridazine and the like).

In the "optionally substituted monocyclic aromatic ring which may be condensed with a 5- to 7-membered ring" represented by ring D, the "5- to 7-membered ring" includes, for example, a 5- to 7-membered isocyclic ring or 5- to 7-membered heterocyclic ring, etc.

The "5- to 7-membered isocyclic ring" includes, for example, a C₅₋₇ cycloalkene (e.g., a cyclopentene, cyclopentadiene, cyclohexane, 1,3-cyclohexadiene, 1,4-cyclohexadiene, cycloheptene, 1,3-cycloheptadiene or the like) or a benzene or the like.

The "5- to 7-membered heterocyclic ring" includes, for example a 5- to 7-membered heterocyclic ring containing 1 to 4 hetero atoms of 1 or 2 elements selected from nitrogen, sulfur and oxygen in addition to carbon atoms, and is preferably a 5- to 7-membered aromatic heterocyclic ring (e.g., a thiophene, furan, pyrrole, imidazole, pyrazole, thiazole, oxazole, isoxazole, pyridine, pyrazine, pyrimidine, pyridazine or the like) or a 5- to 7-membered non-aromatic heterocyclic ring (e.g., a dihydropyrrole, dihydroimidazole, dihydropyrazole, tetrahydropyridine, dihydropyridine, tetrahydropyrazine, dihydropyrazine, dihydro-1,4-oxazine, dihydro-1,4-thioxazine, dihydrofuran, dihydrothiophene, pyrane, dihydropyrane, thiopyrane, dihydrothiopyrane, dihydrooxepin, dihydrothiepin, dihydroazepin, dihydro-1,4-diazepin, dihydro-1,4-oxazepin or dihydro-1,4-thiazepin, etc.) or the like.

In the "optionally substituted monocyclic aromatic ring which may be condensed with a 5- to 7-membered ring" represented by ring D, a substituent may be an optionally substituted amino, optionally substituted hydrocarbon group, substituted sulfonyl, sulfo, halogen atom (e.g., fluorine, chlorine, bromine, iodine or the like), nitro, cyano, optionally halogenated C₁₋₈ alkoxy, C₁₋₆ alkoxy-carbonyl-C₁₋₆ alkoxy (ethoxycarbonylmethyloxy or the like), hydroxyl, C₆₋₁₄ aryloxy (phenyloxy, 1-naphthyloxy, 2-naphthyloxy or the like), C₇₋₁₆ aralkyloxy (benzyloxy, phenethyloxy or the like), mercapto, optionally halogenated C₁₋₆ alkylthio, C₆₋₁₄ arylthio (phenylthio, 1-naphthylthio, 2-naphthylthio or the like), C₇₋₁₆ aralkylthio (benzylthio, phenylthio or the like), formyl, carboxyl, C₁₋₆ alkyl-carbonyl (acetyl, propionyl or the like), C₃₋₆ cycloalkyl-carbonyl (cyclopropylcarbonyl, cyclopentylcarbonyl, cyclohexylcarbonyl or the like), C₁₋₆ alkoxy-carbonyl (methoxycarbonyl, ethoxycarbonyl, propoxycarbonyl, tert-butoxycarbonyl or the like), C₆₋₁₄ aryl-carbonyl (benzoyl, 1-naphthoyl, 2-naphthoyl or the like), C₇₋₁₆ aralkyl-carbonyl (phenylacetyl, 3-phenylproponyl or the like), C₆₋₁₄ aryloxy-carbonyl (phenoxycarbonyl or the like), C₇₋₁₆ aralkyloxy-carbonyl (benzyloxycarbonyl, phenethyloxycarbonyl or the like), 5-or 6-membered heterocyclic carbonyl (nicotinoyl, isonicotinoyl, thenoyl, furoyl, morpholinocarbonyl, thiomorpholinocarbonyl, piperazine-1-ylcarbonyl, pyrrolidine-1-ylcarbonyl or the like), carbamoyl, mono-C₁₋₆ alkyl-carbamoyl (methylcarbamoyl, ethylcarbamoyl or the like), di-C₁₋₆ alkyl-carbamoyl (dimethylcarbamoyl, diethylcarbamoyl, ethylmethylcarbamoyl or the like), C₆₋₁₄ aryl-carbamoyl (phenylcarbamoyl, 1-naphthylcarbamoyl, 2-naphthylcarbamoyl or the like), C₁₋₆ alkoxy-carbamoyl (methoxycarbamoyl, ethoxycarbamoyl or the like), 5- or 6-membered heterocyclic carbamoyl (2-pyridylcarbamoyl, 3-pyridylcarbamoyl, 4-pyridylcarbamoyl, 2-thienylcarbamoyl, 3-thienylcarbamoyl or the like), C₁₋₆ alkyl-carbonyloxy (acetoxy, propionyloxy or the like), C₆₋₁₄ aryl-carbonyloxy (benzoyloxy, naphthylcarbonyloxy or the like), C₁₋₆ alkoxy-carbonyloxy (methoxycarbonyloxy, ethoxycarbonyloxy, propoxycarbonyloxy, butoxycarbonyloxy or the like), mono-C₁₋₆ alkyl-carbamoyloxy (methylcarbamoyloxy, ethylcarbamoyloxy or the like), di-C₁₋₆ alkyl-carbamoyloxy (dimethylcarbamoyloxy, diethylcarbamoyloxy or the like), C₆₋₁₄ aryl-carbamoyloxy (phenylcarbamoyloxy, naphthylcarbamoyloxy or the like), 5- or 6-membered heterocyclic carbonyloxy (nicotinoyloxy, isonicotinoyloxy or the like), optionally substituted 5- to 7-membered saturated cyclic amino, 5- to 10-membered aromatic heterocyclic group (e.g., a 2-thienyl, 3-thienyl, 2-furyl, 3-furyl, , 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl or the like), a 3- to 10-membered non-aromatic heterocyclic group (e.g., 1-pyrrolidinyl, 2-pyrrolidinyl, 3-pyrrolidinyl, 2-imidazolinyl, 4-imidazolinyl, 2-pyrazolidinyl, 3-pyrazolidinyl, 4-pyrazolidinyl, 2-piperidyl, 3-piperidyl, 4-piperidyl, 1-piperazinyl, 2-piperazinyl, morpholino, thiomorpholino or the like), oxo, thioxo or the like. In the case of a sulfur atom, a sulfoxide or sulfone is expressed, respectively by substitution with 1 or 2 oxo groups. Examples of the "optionally substituted 5- to 7-membered saturated cyclic amino" include those described above.

A substituent in the aforementioned "optionally substituted amino" includes, for example, an acyl, optionally substituted hydrocarbon group or optionally substituted heterocyclic group, etc. Preferably it is an acyl and more preferably a carbonyl or sulfonyl substituted with a group having an optionally substituted aromatic group.

The aforementioned "acyl" includes, for example, the group represented by the formula - (C=O)-R⁹, -(C=O)-OR⁸, -(C=O)-NR⁹R¹⁰, -(C=S)-NR⁹R¹⁰, -SO-R⁸, -SO₂-R⁸ or-SO₂-NR⁹R¹⁰ (wherein R⁸ is an optionally substituted hydrocarbon group or optionally substituted heterocyclic group; R⁹ is a hydrogen atom, optionally substituted hydrocarbon group or optionally substituted heterocyclic group; R¹⁰ is a hydrogen atom, optionally substituted amino, optionally substituted hydroxyl, optionally substituted hydrocarbon group or optionally substituted heterocyclic group; and NR⁹R¹⁰ may be a cyclic amino).

A substituent in the "optionally substitution hydrocarbon group" and a substituent in the "optionally substituted heterocyclic group" represented by R⁸, R⁹ or R¹⁰ may be an optionally substituted aryl [for example, a C₆₋₁₄ aryl (e.g., a phenyl, 1-naphthyl, 2-naphthyl, 2-biphenylyl, 3-biphenylyl, 4-biphenylyl or 2-anthryl) or the like], optionally substituted aromatic heterocyclic group [for example, a 5- to 14-membered (preferably 5- to 10-membered) (monocyclic or bicyclic) aromatic heterocyclic group (e.g., a 2-thienyl, 3-thienyl, 2-furyl, 3-furyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, pyrazinyl, 2-pyrimidinyl, 4-pyrimidinyl, 3-pyrrolyl, 2-imidazolyl, 3-pyridazinyl, 3-isothiazolyl, 3-isoxazolyl, 1-indolyl, 2-indolyl, 3-indolyl, 2-benzothiazolyl, 2-benzo[b]thienyl, 3-benzo[b]thienyl, 2-benzo[b]furanyl or 3-benzo[b]furanyl, etc.) or the like], optionally substituted non-aromatic heterocyclic group [for example, a 3- to 14-membered (preferably 5- to 10-membered) (monocyclic or bicyclic) non-aromatic heterocyclic group (e.g., 1-pyrrolidinyl, 2-pyrrolidinyl, 3-pyrrolidinyl, 2-imidazolinyl, 4-imidazolinyl, 2-pyrazolidinyl, 3-pyrazolidinyl, 4-pyrazolidinyl, piperidino, 2-piperidyl, 3-piperidyl, 4-piperidyl, 1-piperazinyl, 2-piperazinyl, morpholino, or thiomorpholino, etc.) or the like], optionally substituted mercapto, halogen atom (for example, fluorine, chlorine, bromine, iodine or the like), C₁₋₃ alkylenedioxy (methylenedioxy, ethylenedioxy or the like), nitro, cyano, optionally halogenated C₁₋₆ alkyl, optionally halogenated C₂₋₆ alkenyl, carboxy C₂₋₆ alkenyl (2-carboxyethenyl, 2-carboxy-2-methylethenyl or the like), optionally halogenated C₂₋₆ alkynyl, optionally halogenated optionally condensed C₃₋₈ cycloalkyl, optionally halogenated C₁₋₈ alkoxy, C₁₋₆ alkoxy-carbonyl-C₁₋₆ alkoxy (ethoxycarbonylmethyloxy or the like), hydroxyl, C₆₋₁₄ aryloxy (phenyloxy, 1-naphthyloxy, 2-naphthyloxy or the like), C₇₋₁₆ aralkyloxy (benzyloxy, phenethyloxy or the like), amino, hydroxyamino, mono-C₁₋₆ alkylamino (methylamino, ethylamino or the like), mono-C₆₋₁₄ arylamino (phenylamino, 1-naphthylamino, 2-naphthylamino or the like), di-C₁₋₆ alkylamino (dimethylamino, diethylamino, ethylmethylamino or the like), di-C₆₋₁₄ arylamino (diphenylamino or the like), formyl, carboxyl, C₁₋₆ alkyl-carbonyl (acetyl, propionyl or the like), C₃₋₆ cycloalkyl-carbonyl (cyclopropylcarbonyl, cyclopentylcarbonyl, cyclohexylcarbonyl or the like), C₁₋₆ alkoxy-carbonyl (methoxycarbonyl, ethoxycarbonyl, propoxycarbonyl, tert-butoxycarbonyl or the like), C₆₋₁₄ aryl-carbonyl (benzoyl, 1-naphthoyl, 2-naphthoyl or the like), C₇₋₁₆ aralkyl-carbonyl (phenylacetyl, 3-phenylpropionyl or the like), C₆₋₁₄ aryloxy-carbonyl (phenoxycarbonyl or the like), C₇₋₁₆ aralkyloxy-carbonyl (benzyloxycarbonyl, phenethyloxycarbonyl or the like), 5- or 6-membered heterocyclic carbonyl (nicotinoyl, isonicotinoyl, thenoyl, furoyl, morpholinocarbonyl, thiomorpholinocarbonyl, piperazine-1-ylcarbonyl, pyrrolidine-1-ylcarbonyl or the like), carbamoyl, mono-C₁₋₆ alkyl-carbamoyl (methylcarbamoyl, ethylcarbamoyl or the like), di-C₁₋₆ alkyl-carbamoyl (dimethylcarbamoyl, diethylcarbamoyl, ethylmethylcarbamoyl or the like), C₆₋₁₄ aryl-carbamoyl (phenylcarbamoyl, 1-naphthylcarbamoyl, 2-naphthylcarbamoyl or the like), C₁₋₆ alkoxy-carbamoyl (methoxycarbamoyl, ethoxycarbamoyl or the like), 5- or 6-membered heterocyclic carbamoyl (2-pyridylcarbamoyl, 3-pyridylcarbamoyl, 4-pyridylcarbamoyl, 2-thienylcarbamoyl, 3-thienylcarbamoyl or the like), sulfo, C₁₋₆ alkylsulfonyl (methylsulfonyl, ethylsulfonyl or the like), C₆₋₁₄ arylsulfonyl (phenylsulfonyl, 1-naphthylsulfonyl, 2-naphthylsulfonyl or the like), formylamino, C₁₋₆ alkyl-carbonylamino (acetylamino or the like), C₆₋₁₄ aryl-carbonylamino (benzoylamino, naphthoylamino or the like); C₁₋₆ alkoxy-carbonylamino (methoxycarbonylamino, ethoxycarbonylamino, propoxycarbonylamino, butoxycarbonylamino or the like), C₁₋₆ alkylsulfonylamino (methylsulfonylamino, ethylsulfonylamino or the like), C₆₋₁₄ arylsulfonylamino (phenylsulfonylamino, 2-naphthylsulfonylamino, 1-naphthylsulfonylamino or the like), C₁₋₆ alkyl-carbonyloxy (acetoxy, propionyloxy or the like), C₆₋₁₄ aryl-carbonyloxy (benzoyloxy, naphthylcarbonyloxy or the like), C₁₋₆ alkoxy-carbonyloxy (methoxycarbonyloxy, ethoxycarbonyloxy, propoxycarbonyloxy, butoxycarbonyloxy or the like), mono-C₁₋₆ alkyl-carbamoyloxy (methylcarbamoyloxy, ethylcarbamoyloxy or the like), di-C₁₋₆ alkyl-carbamoyloxy (dimethylcarbamoyloxy, diethylcarbamoyloxy or the like), C₆₋₁₄ aryl-carbamoyloxy (phenylcarbamoyloxy, naphthylcarbamoyloxy or the like), 5- or 6-membered heterocyclic carbonyloxy (e.g., a nicotinoyloxy or isonicotinoyloxy, etc.) or the like.

A substituent in the aforementioned "optionally substituted aryl", "optionally substituted aromatic heterocyclic group" and "optionally substituted non-aromatic heterocyclic group" includes, for example a (a) halogen atom, (b) C₁₋₆ alkyl which may have an optionally halogenated C₆₋₁₂ aryl, (c) optionally halogenated C₆₋₁₂ aryl, (d) C₁₋₆ alkoxy optionally substituted with a 5- to 6-membered aromatic heterocyclic group which may have a C₁₋₆ alkyl, (e) C₇₋₁₃ aralkyloxy optionally having 1 to 3 substituents selected from halogen atom, C₁₋₆ alkoxy and optionally halogenated C₁₋₆ alkyl, (f) 3- to 10-membered non-aromatic isocyclic ring-oxy, (g) optionally halogenated C₆₋₁₂ aryloxy, (h) 5- to 6-membered aromatic heterocyclic ring-oxy, (i) optionally halogenated C₆₋₁₂ aryl-carbonylamino, (j) optionally halogenated C₇₋₁₃ aralkylamino, (k) optionally halogenated C₆₋₁₂ aryl-carbonyl, (1) optionally halogenated C₇₋₁₃ aralkyl-carbonyl, (m) C₁₋₆ alkylsulfonyl, (n) 5- to 10-membered aromatic group, (o) hydroxyl, (p) cyano or the like.

A substituent in the aforementioned "optionally substituted mercapto" includes, for example a hydrocarbon group or heterocyclic group either of which may have 1 to 5 substituents selected from halogen atom and C₁₋₃ alkylenedioxy, nitro, cyano, optionally halogenated C₁₋₆ alkyl, optionally halogenated C₂₋₆ alkenyl, carboxy C₂₋₆ alkenyl, optionally halogenated C₂₋₆ alkynyl, optionally halogenated optionally condensed C₃₋₈ cycloalkyl, C₆₋₁₄ aryl, optionally halogenated C₁₋₈ alkoxy, C₁₋₆ alkoxy-carbonyl-C₁₋₆ alkoxy, hydroxyl, C₆₋₁₄ aryloxy, C₇₋₁₆ aralkyloxy, mercapto, optionally halogenated C₁₋₆ alkylthio, C₆₋₁₄ arylthio, C₇₋₁₆ aralkylthio, amino, hydroxyamino, mono-C₁₋₆ alkylamino, mono-C₆₋₁₄ arylamino, di-C₁₋₆ alkylamino, di-C₆₋₁₄ arylamino, formyl, carboxyl, C₁₋₆ alkyl-carbonyl, C₃₋₆ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5-or 6-membered heterocyclic carbonyl, carbamoyl, mono-C₁₋₆ alkyl-carbamoyl, di-C₁₋₆ alkyl-carbamoyl, C₆₋₁₄ aryl-carbamoyl, C₁₋₆ alkoxy-carbamoyl, 5- or 6-membered heterocyclic carbamoyl, sulfo, C₁₋₆ alkylsulfonyl, C₆₋₁₄ arylsulfonyl, formylamino, C₁₋₆ alkyl-carbonylamino, C₆₋₁₄ aryl-carbonylamino, C₁₋₆ alkoxy-carbonylamino, C₁₋₆ alkylsulfonylamino, C₆₋₁₄ arylsulfonylamino, C₁₋₆ alkyl-carbonyloxy, C₆₋₁₄ aryl-carbonyloxy, C₁₋₆ alkoxy-carbonyloxy, mono-C₁₋₆ alkyl-carbamoyloxy, di-C₁₋₆ alkyl-carbamoyloxy, C₆₋₁₄ aryl-carbamoyloxy, 5- or 6-membered heterocyclic carbonyloxy, 5- to 7-membered saturated cyclic amino and 5- to 10-membered aromatic heterocyclic group and 5- to 10-membered non-aromatic heterocyclic group, etc.

A substituent in the "optionally substituted amino" represented by R¹⁰ may be for example (i) a hydrocarbon group or heterocyclic group either of which may have 1 to 5 substituents selected from halogen atom and C₁₋₃ alkylenedioxy, nitro, cyano, optionally halogenated C₁₋₆ alkyl, optionally halogenated C₂₋₆ alkenyl, carboxy C₂₋₆ alkenyl, optionally halogenated C₂₋₆ alkynyl, optionally halogenated optionally condensed C₃₋₈ cycloalkyl, C₆₋₁₄ aryl, optionally halogenated C₁₋₈ alkoxy, C₁₋₆ alkoxy-carbonyl-C₁₋₆ alkoxy, hydroxyl, C₆₋₁₄ aryloxy, C₇₋₁₆ aralkyloxy, mercapto, optionally halogenated C₁₋₆ alkylthio, C₆₋₁₄ arylthio, C₇₋₁₆ aralkylthio, amino, hydroxyamino, mono-C₁₋₆ alkylamino, mono-C₆₋₁₄ arylamino, di-C₁₋₆ alkylamino, di-C₆₋₁₄ arylamino, formyl, carboxyl, C₁₋₆ alkyl-carbonyl, C₃₋₆ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5-or 6-membered heterocyclic carbonyl, carbamoyl, mono-C₁₋₆ alkyl-carbamoyl, di-C₁₋₆ alkyl-carbamoyl, C₆₋₁₄ aryl-carbamoyl, C₁₋₆ alkoxy-carbamoyl, 5- or 6-membered heterocyclic carbamoyl, sulfo, C₁₋₆ alkylsulfonyl, C₆₋₁₄ arylsulfonyl, formylamino, C₁₋₆ alkyl-carbonylamino, C₆₋₁₄ aryl-carbonylamino, C₁₋₆ alkoxy-carbonylamino, C₁₋₆ alkylsulfonylamino, C₆₋₁₄ arylsulfonylamino, C₁₋₆ alkyl-carbonyloxy, C₆₋₁₄ aryl-carbonyloxy, C₁₋₆ alkoxy-carbonyloxy, mono-C₁₋₆ alkyl-carbamoyloxy, di-C₁₋₆ alkyl-carbamoyloxy, C₆₋₁₄ aryl-carbamoyloxy, 5- or 6-membered heterocyclic carbonyloxy, 5- to 7-membered saturated cyclic amino and 5- to 10-membered aromatic heterocyclic group, 5- to 10-membered non-aromatic heterocyclic group and oxos for example or (ii) a sulfonyl having a substituent selected from optionally halogenated C₁₋₆ alkyl, optionally halogenated C₂₋₆ alkenyl, carboxy C₂₋₆ alkenyl, optionally halogenated C₂₋₆ alkyriyls, optionally halogenated optionally condensed C₃₋₈ cycloalkyl and C₆₋₁₄ aryl.

Examples of substituents in the "optionally substituted hydroxyl" represented by R¹⁰ include for example substituents such as those given above for the aforementioned "optionally substituted mercapto".

Examples of the "cyclic amino" represented by NR⁹R¹⁰ include 5- to 7-membered saturated cyclic amino containing 1 to 4 hetero atoms of 1 or 2 elements selected from nitrogen, sulfur and oxygen in addition to 1 nitrogen atom and carbon atoms, and specific examples include pyrrolidine-1-yl, piperidino, piperazine-1-yl, morpholino, thiomorpholino, tetrahydroazepine-1-yl and homopiperazine-1-yl group and the like.

Examples of substituents in the "optionally substituted hydrocarbon group" given as an example of a substituent in the aforementioned "optionally substituted amino" (in the explanation of ring D) include groups such as those given as substituents for the "optionally substituted isocyclic or heterocyclic ring" represented by ring A above. The hydrocarbon group may for example have 1 to 5 or preferably 1 to 3 such substituents at substitutable positions, and when there are 2 or more substituents they may be the same or different.

Examples of substituents in the "optionally substituted heterocyclic group" given as an example of a substituent in the aforementioned "optionally substituted amino" include for example groups such as those given as substituents for the "optionally substituted isocyclic or heterocyclic ring" represented by ring A above. The heterocyclic group may for example have 1 to 3 or preferably 1 or 2 such substituents at substitutable positions, and when there are 2 or more substituents they may be the same or different.

Examples of substituents in the "optionally substituted hydrocarbon group" given as an example of a substituent in the aforementioned "optionally substituted monocyclic aromatic ring which may be condensed with a 5- to 7-membered ring" given for ring D include for example groups such as those given as substituents for the "optionally substituted isocyclic or heterocyclic ring" represented by ring A above. The hydrocarbon group may for example have 1 to 4 or preferably 1 or 2 such substituents at substitutable positions, and when there are 2 or more substituents they may be the same or different.

Examples of substituents in the "optionally substituted sulfonyl" given as an example of a substituent in the aforementioned "optionally substituted monocyclic aromatic ring which may be condensed with a 5- to 7-membered ring" given for ring D include for example groups such as those given as substituents for the "optionally substituted isocyclic or heterocyclic ring" represented by ring A above.

Ring D may for example have 1 to 4 or preferably 1 or 2 of such substituents at substitutable positions, and when there are 2 or more substituents they may be the same or different.

A desirable example of ring D is an "optionally substituted condensed ring formed from a 5- to 7-membered ring and a monocyclic aromatic ring", and an example of this condensed ring is the ring represented by the formula: 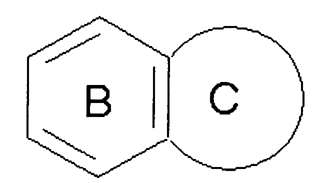 (wherein ring B is an optionally substituted benzene ring and ring C is an optionally substituted 5- to 7-membered isocyclic or heterocyclic ring).

Examples of substituents in the "optionally substituted benzene ring" given for ring B include for example groups such as those given as substituents for the "optionally substituted isocyclic or heterocyclic ring" represented ring A above. Ring B may have 1 to 3 or preferably 1 or 2 such substituents at substitutable positions, and when there are 2 or more substituents they may be the same or different.

The "optionally substituted 5- to 7-membered isocyclic or heterocyclic ring" given for ring C may be similar to the "optionally substituted 5- to 7-membered ring" given for ring D. Ring C may for example have 1 to 4 or preferably 1 to 2 substituents at substitutable positions, and when there are 2 or more substituents they may be the same or different. Such a substituent may be substituted on an atom of ring C simultaneously with the group -V-Q-W.

Examples of substituents in the "optionally substituted hydrocarbon group" represented by R¹⁴, R¹⁵ or R¹⁶ in Compound (I) include for example groups such as those given as substituents for the "optionally substituted isocyclic or heterocyclic ring" represented ring A above. The "hydrocarbon group" may for example have 1 to 3 or preferably 1 or 2 such substituents at substitutable positions, and when there are 2 or more substituents they may be the same or different.

Examples of the "group biologically equivalent to a carboxyl" given for W in Compound W include for example 5-tetrazolyl, 5-tetrazolylaminocarbonyl, C₁₋₆ alkylsulfonylaminocarbonyl (methylsulfonylaminocarbonyl, ethylsulfonylaminocarbonyl and the like), 5-oxo-1,2,4-oxadiazole-3-yl, 5-oxo-1,5-dihydro-4H-1,2,4-triazole-4-yl, 5-oxo-2,5-dihydro-1H-pyrazole-3-yl, 2-oxido-3H-1,2,3,5-oxathiadiazole-4-yl, 1,3-thiazolidine-2,4-dione-5-yl, 2,4-dioxo-1-imidazolidinyl, 2,5-dioxo-4-imidazolidinyl, 5-oxo-2,5-dihydro-1H-1,2,4-triazole-3-yl, 4-methyl-5-oxo-4,5-dihydro-1H-1,2,4-triazole-3-yl, 1-methyl-5-oxo-2,5-dihydro-1H-1,2,4-triazole-3-yl, 4-methyl-3,5-dioxo-1,2,4-triazolidine-1-yl, 5-oxo-2,5-dihydro-1,2,4-thiadiazole-3-yl, 5-thioxo-2,5-dihydro-1,2,4-oxadiazole-3-yl, 3,5-dioxo-1,2,4-triazolidine-1-yl, C₁₋₆ alkoxy-carbonylaminosulfonyl (methoxycarbonylaminosulfonyl, ethoxycarbonylaminosulfonyl and the like), C₁₋₆ alkyl-carbonylaminosulfonyl (methylcarbonylaminosulfonyl, ethylcarbonylaminosulfonyl and the like), C₁₋₆ alkylaminosulfonyl (methylaminosulfonyl, ethylaminosulfonyl and the like), sulfo, aminophosphoryl, O-ethylphosphoryl, N-cyanocarbamoyl, 3-hydroxy-4-isoxazolyl, 3-hydroxy-5-isoxazolyl , 3-hydroxy-4-oxo-4H-pyran-5-yl and the like.

Compound (I) may be for example the compound represented by the formula: 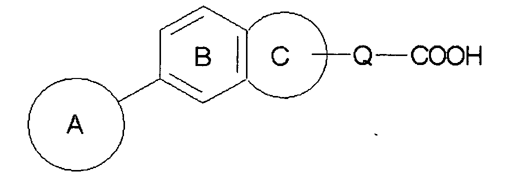 (wherein all symbols are as defined above, and the group represented by the formula -Q-COOH is substituted at any position on ring C).

Of the Compounds (I), Compound (Ia), Compound (Ib), Compound (Ic), Compound (Id) and the like are preferred.

The "optionally substituted isocyclic ring or heterocyclic ring" represented by ring A in Compound (Ia) may be similar to the "optionally substituted isocyclic ring or heterocyclic ring" represented by ring A in Compound (I).

An optionally substituted aromatic ring (e.g., a C₆₋₁₄ aryl or 5- to 14-membered (preferably 5- to 10-membered) aromatic heterocyclic ring) or the like is preferred as ring A. An optionally substituted 5- or 6-membered aromatic ring (e.g., a benzene, thiophene, furan, pyrrole, imidazole, pyrazole, pyridine, pyrazine, pyrimidine or pyridazine) or the like is still more desirable.

Examples of the "optionally substituted benzene ring" represented by ring B in Compound (Ia) include those such as the "optionally substituted benzene ring" given for ring B above.

The "substituent" in the "cyclopentene ring which may also include a substituent other than R¹ and -Qa-W" given for ring Ca in Compound (Ia) may for example be a group such as such as those given as substituents for the "optionally substituted isocyclic or heterocyclic ring" represented by ring A. Ring Ca may have 1 to 4 or preferably 1 to 2 such substituents at substitutable positions, and if there are 2 or more substituents they may be the same or different.

W and P in Compound (Ia) may be similar to the W and P in Compound (I) above.

A bond or a spacer with 2 atoms in the main chain (e.g., -CH₂CH₂-, -CH=CH-, -C≡C-, -CH₂O-, -CH₂S-, -CH₂SO- or -CH₂SO₂-, etc) is preferred as P.

A carboxyl is preferred as W.

A substituent in the "optionally substituted amino" given for R¹ in Compound (Ia) may be for example an acyl, optionally substituted hydrocarbon group, optionally substituted heterocyclic group or the like.

The aforementioned "acyl" includes, for example, the group represented by the formula - (C=O)-R⁹; -(C=O)-OR⁸, -(C=O)-NR⁹R¹⁰, -(C=S)-NR⁹R¹⁰, -SO-R⁸, -SO₂-R⁸ or -SO₂-NR⁹R¹⁰ (wherein R⁸, R⁹ and R¹⁰ are as defined above) or the like.

Examples of substituents in the "optionally substituted hydrocarbon group" given as an example of a substituent in the "optionally substituted amino" above include for example groups such as those given as substituents for the "optionally substituted isocyclic or heterocyclic ring" represented by ring A above. The amino [sic] may have 1 or 2 such substituents at substitutable positions, and if there are 2 or more substituents they may be the same or different.

Examples of substituents in the "optionally substituted heterocyclic group" given as an example of a substituent in the "optionally substituted amino" above include for example groups such as those given as substituents for the "optionally substituted isocyclic or heterocyclic ring" represented by ring A above. The amino may have 1 to 3 or preferably 1 or 2 such substituents at substitutable positions, and if there are 2 or more substituents they may be the same or different.

A substituent in the "optionally substituted amino" represented by R¹ is preferably an acyl or more preferably a group represented by the formula -(C=O)-R⁸, -(C=O)-OR⁸, -(C=O)-NR⁹R¹⁰, -(C=S)-NR⁹R¹⁰, -SO₂-R⁸ or -SO₂-NR⁹R¹⁰ (wherein R⁸, R⁹ and R¹⁰ are as defined above), or still more preferably a carbonyl or sulfonyl either of which has a group having an optionally substituted aromatic group (for example, a C₆₋₁₄ aryl, 5- to 10-membered aromatic heterocyclic group or the like) or the like.

R⁸ and R⁹ are preferably (i) hydrocarbon group (C₁₋₆ alkyl or the like) which may have optionally substituted aryl (C₆₋₁₄ aryl or the like), (ii) hydrocarbon group (C₁₋₆ alkyl or the like) which may have optionally substituted aromatic heterocyclic group (for example, 5- to 14-membered (preferably 5- to 10-membered) (monocyclic or bicyclic) aromatic heterocyclic group)), or (iii) hydrocarbon group (C₁₋₆ alkyl or the like) which may have optionally substituted mercapto or the like.

A substituent in the aforementioned "optionally substituted aryl" and "optionally substituted aromatic heterocyclic group" includes, for example a (a) halogen atom, (b) C₁₋₆ alkyl which may have an optionally halogenated C₆₋₁₂ aryl, (c) optionally halogenated C₆₋₁₂ aryl, (d) C₁₋₆ alkoxy optionally substituted with a 5- to 6-membered aromatic heterocyclic group which may have a C₁₋₆ alkyl, (e) C₇₋₁₃ aralkyloxy optionally having 1 to 3 substituents selected from halogen atom, C₁₋₆ alkoxy and optionally halogenated C₁₋₆ alkyl, (f) 3- to 10-membered non-aromatic isocyclic ring-oxy, (g) optionally halogenated C₆₋₁₂ aryloxy, (h) 5- to 6-membered aromatic heterocyclic ring-oxy, (i) optionally halogenated C₆₋₁₂ aryl-carbonylamino, (j) optionally halogenated C₇₋₁₃ aralkylamino, (k) optionally halogenated C₆₋₁₂ aryl-carbonyl, (1) optionally halogenated C₇₋₁₃ aralkyl-carbonyl, (m) C₁₋₆ arkylsulfonyl, (n) 5- to 10-membered aromatic group, (o) hydroxyl, (p) cyano or the like.

A substituent in the aforementioned "optionally substituted mercapto" includes, for example a hydrocarbon group or heterocyclic group either of which may have 1 to 5 substituents selected from halogen atom and C₁₋₃ alkylenedioxy, nitro, cyano, optionally halogenated C₁₋₆ alkyl, optionally halogenated C₁₋₆ alkenyl, carboxy C₂₋₆ alkenyl, optionally halogenated C₂₋₆ alkynyl, optionally halogenated optionally condensed C₃₋₈ cycloalkyl, C₆₋₁₄ aryl, optionally halogenated C₁₋₈ alkoxy, C₁₋₆ alkoxy-carbonyl-C₁₋₆ alkoxy, hydroxyl, C₆₋₁₄ aryloxy, C₇₋₁₆ aralkyloxy, mercapto, optionally halogenated C₁₋₆ alkylthio, C₆₋₁₄ arylthio, C₇₋₁₆ aralkylthio, amino, hydroxyamino, mono-C₁₋₆ alkylamino, mono-C₆₋₁₄ arylamino, di-C₁₋₆ alkylamino, di-C₆₋₁₄ arylamino, formyl, carboxyl, C₁₋₆ alkyl-carbonyl, C₃₋₆ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5-or 6-membered heterocyclic carbonyl, carbamoyl, mono-C₁₋₆ alkyl-carbamoyl, di-C₁₋₆ alkyl-carbamoyl, C₆₋₁₄ aryl-carbamoyl, C₁₋₆ alkoxy-carbamoyl, 5- or 6-membered heterocyclic carbamoyl, sulfo, C₁₋₆ alkylsulfonyl, C₆₋₁₄ arylsulfonyl, formylamino, C₁₋₆ alkyl-carbonylamino, C₆₋₁₄ aryl-carbonylamino, C₁₋₆ alkoxycarbonylamino, C₁₋₆ alkylsulfonylamino, C₆₋₁₄ arylsulfonylamino, C₁₋₆ alkyl-carbonyloxy, C₆₋₁₄ aryl-carbonyloxy, C₁₋₆ alkoxy-carbonyloxy, mono-C₁₋₆ alkyl-carbamoyloxy, di-C₁₋₆ alkyl-carbamoyloxy, C₆₋₁₄ aryl-carbamoyloxy, 5- or 6-membered heterocyclic carbonyloxy, 5- to 7-membered saturated cyclic amino and 5- to 10-membered aromatic heterocyclic groups and 5- to 10-membered non-aromatic heterocyclic group and the like. Preferably, it is a hydrocarbon group or heterocyclic group either of which may have 1 to 5 substituents selected from halogen atom and cyano, optionally halogenated C₁₋₆ alkyl, optionally halogenated C₂₋₆ alkenyl, optionally halogenated optionally condensed C₃₋₈ cycloalkyl, C₆₋₁₄ aryl, optionally halogenated C₁₋₈ alkoxy, C₆₋₁₄ aryloxy, C₇₋₁₆ aralkyloxy, optionally halogenated C₁₋₆ alkylthio, C₆₋₁₄ arylthio, C₇₋₁₆ aralkylthio, formyl, C₁₋₆ alkyl-carbonyl, C₃₋₆ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5- or 6-membered heterocyclic carbonyl, C₁₋₆ alkylsulfonyl, C₆₋₁₄ arylsulfonyl, formylamino, C₁₋₆ alkyl-carbonylamino, C₆₋₁₄ aryl-carbonylamino, C₁₋₆ alkoxy-carbonylamino, C₁₋₆ alkylsulfonylamino, C₆₋₁₄ arylsulfonylamino, 5-to 10-membered aromatic heterocyclic and 5- to 10-membered non-aromatic heterocyclic group.

The group represented by the formula -NHR^{1a} (wherein R^{1a} is -(C=O)-R⁸, -(C=O)-OR⁸, -(C=O)-NR⁹R¹⁰, -SO₂-R⁸ or -SO₂-NR⁹R¹⁰ (with R⁸, R⁹ and R¹⁰ being as defined above)) is preferred for R¹.

More preferably, R¹ is the group represented by the formula: 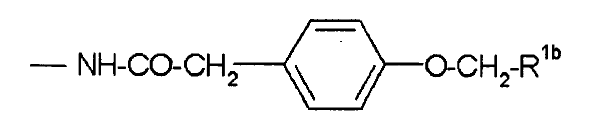 (wherein R^{1b} is an optionally substituted aromatic group).

Examples of the "aromatic group" in the "optionally substituted aromatic group" represented by R^{1b} include C₆₋₁₄ aryl (e.g., phenyl, 1-naphthyl, 2-naphthyl, 2-biphenyl, 3-biphenyl, 4-biphenyl, 2-anthryl, 3-indenyl and the like), 5- to 10-membered aromatic heterocyclic group (e.g., 2-thienyl, 3-thienyl, 2-furyl, 3-furyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo[b]thienyl, 3-benzo[b]thienyl, 2-benzo[b]furanyl and 3-benzo[b]furanyl, etc.) and the like.

A substituent in the "optionally substituted aromatic group" includes, for example a group such as those given as substituents for the "optionally substituted isocyclic or heterocyclic ring" represented by ring A above. The aromatic group may have for example 1 to 4 or preferably 1 or 2 such substituents at substitutable positions, and when there are 2 or more substituents they may be the same or different.

The "spacer having a terminal carbon atom that is bound to by a carboxyl or a group biologically equivalent to a carboxyl" represented by Qa' in Compound (Ia) may be a bivalent chain hydrocarbon (for example, a bivalent C₁₋₅ chain hydrocarbon such as an alkylene, alkenylene, alkynylene or the like) optionally separated, respectively, by 1 or 2 groups selected from -O-, -S-, -NQa"- and the bivalent heterocyclic group (for example, pyrrolidine-1,2-diyl, pyrrolidine-1,3-diyl, piperidine-1,4-diyl, piperidine-1,3-diyl, piperidine-1,2-diyl and the like), or a bivalent cyclic group (for example, a bivalent 6-membered cyclic group such as 2-phenylene, 3-phenylene, 4-phenylene, cyclohexane-1,4-diyl, pyridine-2,5-diyl, pyridine-2,4-diyl or piperidine-1,4-diyl, etc.) or the like.

Qa" may be similar to Q' above.

The "spacer having a terminal carbon atom that is bound to by a carboxyl or a group biologically equivalent to a carboxyl" represented by Qa' is preferably a spacer with 1 to 4 atoms in the main chain.

Qa is preferably a bond, or Qa'-W is an amino acid in which the functional group is optionally protected or modified. An "amino acid in which the functional group is optionally protected or modified" includes, for example, an amino acid (for example, glycine, alanine, beta-alanine, valine, leucine, isoleucine, serine, threonine, aspartic acid, glutamic acid, asparagine, glutamine, lysine, arginine, cysteine, methionine, phenylalanine, tyrosine, tryptophan, histidine, proline or the like) in which the functional group (amino, hydroxyl, thiol, carboxyl, phenolic hydroxyl or the like) is optionally protected or modified (for example, alkylated, aralkylated, esterified, acylated or amidated) or the like.

A bond or -CONH-C₁₋₆ alkylene is preferred as Qa.

Compound (Ia) is preferably the compound represented by the formula: 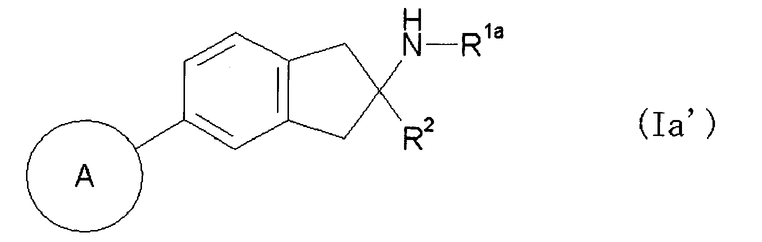 wherein R^{1a} is:
(i) a carbonyl having a substituent selected from (1) alkyl which have optionally substituted aromatic group and which may themselves be further substituted, (2) alkyl which have optionally substituted non-aromatic heterocyclic group and which may themselves be further substituted, (3) alkyl which have optionally substituted mercapto and which may themselves be further substituted, (4) optionally substituted aromatic group, (5) amino having optionally substituted aromatic group, (6) optionally substituted cycloalkyl, (7) optionally substituted nitrogen-containing non-aromatic heterocyclic group, (8) optionally substituted alkyl and (9) optionally substituted alkenyl, or
(ii) an alkylsulfonyl which has an optionally substituted aromatic group and which may itself be further substituted,
   R² is a carboxyl, a group biological equivalent to a carboxyl or the group represented by the formula CO-Z-OH (wherein Z-OH is an amino acid), and
   ring A is as defined above).

The amino acid represented by Z-OH may be an amino acid (for example glycine, alanine, beta-alanine, valine, leucine, isoleucine, serine, threonine, aspartic acid, glutamic acid, asparagine, glutamine, lysine, arginine, cysteine, methionine, phenylalanine, tyrosine, tryptophan, histidine, proline or the like) in which the functional group (amino, hydroxyl, thiol, carboxyl, phenolic hydroxyl or the like) may be protected or modified (alkylated, aralkylated, esterified, acylated, amidated or halogenated, etc.) or the like. Said amino acid may be an L-amino acid, D-amino acid or DL-amino acid.

Of the Compounds (Ia'), a compound in which ring A is a 5- or 6-membered aromatic ring which may have 1 to 3 substituents selected from (a) halogen atom, (b) optionally halogenated C₁₋₆ alkyl, (c) optionally halogenated C₁₋₆ alkoxy and (d) amino optionally substituted with 1 or 2 groups selected from C₁₋₆ alkyl-carbonyl and C₁₋₆ alkyl,
R^{1a} is:
(i) a carbonyl having a substituent selected from
   (1) C₁₋₆ alkyl having 5- to 10-membered aromatic group which may have 1 to 3 substituents selected from (a) halogen atom, (b) C₁₋₆ alkylsulfonyl, (c) C₁₋₆ alkoxy optionally having C₁₋₆ alkyl and optionally substituted with 5- or 6-membered aromatic heterocyclic group, (d) C₇₋₁₃ aralkyloxy optionally having 1 to 3 substituents selected from halogen atom, C₁₋₆ alkoxy and optionally halogenated C₁₋₆ alkyl, (e) 5- to 10-membered non-aromatic isocyclic ring-oxy, (f) optionally halogenated C₆₋₁₂ aryloxy, (g) 5- or 6-membered aromatic heterocyclic ring-oxy, (h) optionally halogenated C₆₋₁₂ aryl-carbonylamino, (i) optionally halogenated C₇₋₁₃ aralkylamino and (j) C₆₋₁₂ aryl, and optionally having 1 to 3 substituents selected from amino which may have hydroxyl and C₁₋₆ alkoxy-carbonyl,
   (2) C₁₋₆ alkyl having 6-membered nitrogen-containing non-aromatic heterocyclic group which may have 1 or 2 substituents selected from (a) C₁₋₆ alkyl which may have 1 or 2 optionally halogenated C₆₋₁₂ aryl (b) C₆₋₁₂ aryl, (c) optionally halogenated C₆₋₁₂ aryl-carbonyl, and (d) optionally halogenated C₇₋₁₃ aralkyl-carbonyl,
   (3) C₁₋₆ alkyl having 5- to 10-membered aromatic mercapto,
   (4) 5- to 10-membered aromatic group which may have 1 to 3 substituents selected from halogen atom, the C₁₋₆ alkyl which may have 5- to 10-membered aromatic heterocyclic group optionally substituted with C₁₋₆ alkyl, and the optionally halogenated C₆₋₁₂ aryl,
   (5) amino having optionally halogenated C₆₋₁₂ aryl,
   (6) C₃₋₆ cycloalkyl,
   (7) optionally oxonated 5-membered nitrogen-containing non-aromatic heterocyclic group,
   (8) C₁₋₆ alkyl which may have 1 or 2 substituents selected from optionally oxonated 5-membered nitrogen-containing non-aromatic heterocyclic group and C₇₋₁₃ aralkyloxy, C₁₋₆ alkoxy, carboxyl, C₁₋₆ alkoxy-carbonyl and amino group, and
   (9) C₂₋₆ alkenyl, or
(ii) an optionally halogenated C₇₋₁₃ aralkylsulfonyl,
   and R² is a carboxy-C₁₋₆ alkyl-carbamoyl, carboxyl, 5-tetrazolyl or 5-tetrazolylaminocarbonyl, or the like is desirable.

More specifically, 5-(4-chlorophenyl)-2-[({4-[(4-fluorobenzyl)oxy]phenyl}acetyl)amino]indan-2-carboxylic acid or a salt thereof or the like can be used by preference as Compound (Ia).

The "optionally substituted isocyclic ring or heterocyclic ring" represented by ring A in Compound (Ib) may be similar to the "optionally substituted isocyclic ring or heterocyclic ring" represented by ring A in Compound (I).

Ring A is preferably an optionally substituted aromatic ring (for example, a C₆₋₁₄ aryl or 5- to 14-membered (preferably 5- to 10-membered) aromatic heterocyclic ring) or the like. More preferably, it is an optionally substituted 5- or 6-membered aromatic ring (e.g., a benzene, thiophene, furan, pyrrole, imidazole, pyrazole, thiazole, oxazole, pyridine, pyrazine, pyrimidine, pyridazine or the like) or the like. Still more preferably, it is a 5- or 6-membered aromatic ring optionally having 1 to 3 substituents selected from halogen atom, optionally halogenated C₁₋₆ alkyl and optionally halogenated C₁₋₆ alkoxy.

The "optionally substituted benzene ring" represented by ring B in Compound (Ib) may be similar to the the "optionally substituted benzene ring" represented by ring B above.

Ring B is preferably a benzene ring optionally having 1 to 3 substituents selected from halogen atom, C₁₋₆ alkyl and C₁₋₆ alkoxy.

A substituent in the "optionally substituted 5- or 6-membered ring" represented by ring Cb in Compound (Ib) may be a substituent such as those given for the "optionally substituted isocyclic or heterocyclic ring" represented by ring A above. The hydrocarbon group [sic] may have 1 to 3 or preferably 1 or 2 such substituents at substitutable positions, and when there are 2 or more substituents they may be the same or different.

A substituent in the "optionally substituted hydrocarbon group" represented by R⁴ in Compound (Ib) may be a substituent such as those given for the "optionally substituted isocyclic or heterocyclic ring" represented by ring A above. The hydrocarbon group may have 1 to 5 or preferably 1 to 3 such substituents at substitutable positions, and when there are 2 or more substituents they may be the same or different.

A substituent in the "optionally substituted sulfonyl" given for R⁴ in Compound (Ib) may be a substituent such as those given for the "optionally substituted isocyclic or heterocyclic ring" represented by ring A above.

R⁴ is preferably a hydrogen atom or optionally halogenated C₁₋₆ alkyl.

The "substituent" represented by R⁵ in Compound (Ib) may be a substituent such as those given for the "5- to 7-membered isocyclic or heterocyclic ring which may also include a substituent other than the group represented by the formula -Q-COOH" represented by ring C above, provided that oxo is excluded.

A hydrogen atom, optionally substituted hydrocarbon group (e.g., an optionally halogenated C₁₋₆ alkyl, etc.) or the like is preferred as R⁵.

Of X² and Y² in Compound (Ib), one is -O-, -S-, -NR⁴- or -S-CH₂- (wherein R⁴ is as defined above), while the other is -N= or -CR⁵= (wherein R⁵ is as defined above). More preferably, one is -O- or -S- while the other is -CR⁵ (wherein R⁵ is as defined above).

R³ and Qb in Compound (Ib) are as defined above.

The "optionally substituted C₁₋₃ alkylene" given as a desirable example of Qb may be a methylene having an optionally substituted benzyl or the like. More preferably, it may be the group represented by the formula: 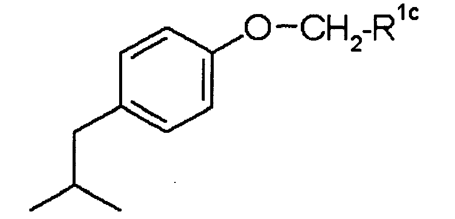 (wherein R^{1c} is an optionally substituted aromatic group).

The "optionally substituted aromatic group" represented by R^{1c} may a group such as those given for the "optionally substituted aromatic group" represented by R^{1b} above.

W and P in Compound (Ib) may be similar to the W and P in Compound (I) above.

A carboxyl, C₁₋₆ alkylsulfonylaminocarbonyl, 5-oxo-1,2,4-oxadiazole-3-yl, 5-tetrazolyl or 5-tetrazolylaminocarbonyl is preferred as W.

P is preferably a bond or a spacer with 2 atoms in the main chain (e.g., -CH₂CH₂-, - CH=CH-, -C≡C-, -CH₂O-, -CH₂S-, -CH₂SO-, -CH₂SO₂- or the like).

The compound represented by the formula: 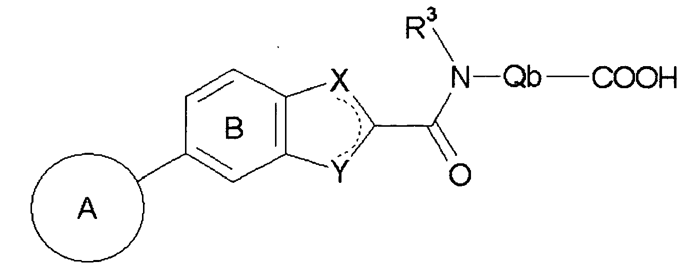 (wherein one of X and Y is -O-, -S- or -NR⁴- while the other is -N= or -CR⁵=, ring A, ring B, R³, R⁴, R⁵ and Qb are as defined above, and any one of the broken lines represents a double bond) or the like is preferred as Compound (Ib).

It is desirable that one of X and Y be -O- or -S- and the other be -CR⁵= herein.

It is also desirable that R⁵ be a hydrogen atom or hydrocarbon group.

Preferably, in Compound (Ib) for example ring A is 5- to 6-membered aromatic ring optionally substituted with 1 to 3 groups selected from halogen atom, optionally halogenated C₁₋₆ alkyl and optionally halogenated C₁₋₆ alkoxy,
ring B is a benzene ring optionally substituted with 1 to 3 groups selected from halogen atom, C₁₋₆ alkyl and C₁₋₆ alkoxy,
one of X² and Y² is -O-, -S-, -NR^{4a}- or -S-CH₂-, while the other is -N= or -CR^{5a}= (wherein R^{4a} is a hydrogen atom or C₁₋₆ alkyl and R^{5a} is a hydrogen atom, halogen atom, amino, C₁₋₆ alkyl-carbonylamino, C₁₋₆ alkoxy-carbonylamino or C₁₋₆ alkyl),
R³ is a hydrogen atom or C₁₋₆ alkyl, or R³ and Qb may be bound together to form a 5- to 7-membered ring,
Qb is an optionally substituted C₁₋₃ alkylene or NR³-Qb-W is an amino acid,
P is a bond, ethylene, ethenylene or ethynylene, and

W is a carboxyl, C₁₋₆ alkylsulfonylaminocarbonyl, 5-oxo-1,2,4-oxadiazole-3-yl, 5-tetrazolyl or 5-tetrazolylaminocarbonyl.

A specific example of Compound (Ib) is the compound represented by the formula: 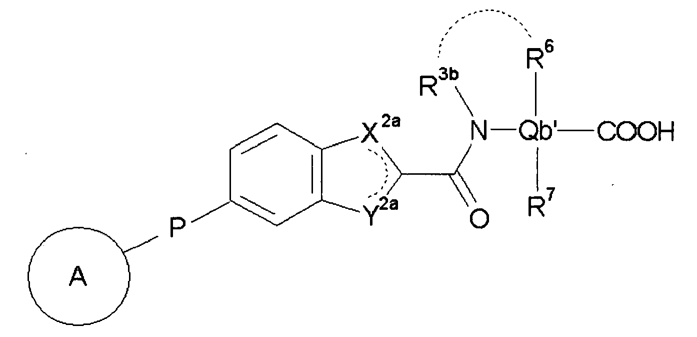 (wherein P is a bond, ethenylene or ethynylene,
ring A is a 5- or 6-membered aromatic ring optionally substituted with 1 to 3 groups selected from (1) halogen atom, (2) C₁₋₆ alkyl optionally substituted with 1 to 3 groups selected from (a) 5- or 6-membered aromatic heterocyclic group which may have C₁₋₆ alkyl and may be condensed with benzene rings and (b) halogens, and (3) amino optionally substituted with 1 or 2 groups selected from C₁₋₆ alkyl and C₁₋₆ alkyl-carbonyl,
one of X^{2a} and Y^{2a} is -O-, -S-, -NH-, -NH-CO- or -S-CH₂- while the other is -N= or -CR^{5b}=,
R^{5b} is a hydrogen atom, amino or C₁₋₆ alkyl,
one of the broken lines in the ring represents a double bond,
R^{3b} is a hydrogen atom or C₁₋₆ alkyl,
Qb' is a C₁₋₃ alkylene,
R⁶ and R⁷ are located on the same or different carbon atoms and are each
(1) hydrogen atoms,
(2) C₁₋₆ alkyl optionally substituted with 1 to 3 groups selected from C₁₋₆ alkoxy-carbonyl, C₇₋₁₃ aralkyloxy-carbonyl, hydroxyl, carboxyl and C₁₋₆ alkylthio group and the 5- or 6-membered aromatic heterocyclic group optionally condensed with benzene rings,
(3) C₇₋₁₃ aralkyl which may have 1 to 3 substituents selected from (a) the C₇₋₁₃ aralkyloxy optionally substituted with 1 or 2 groups selected from halogen atom, cyano, optionally halogenated C₁₋₆ alkyl and C₁₋₆ alkoxy and (b) the 5- or 6-membered aromatic heterocyclic ring-C₁₋₃ alkoxy,
(4) C₆₋₁₂ aryl or
(5) 5- or 6- member aromatic heterocyclic group optionally condensed with benzene rings, or alternatively
   R⁶ and R⁷ are bound together to form, together with an adjacent carbon atom, a C₃₋₇ cycloalkane optionally condensed with a C₆₋₁₂ aryl which may have 1 or 2 substituents selected from optionally halogenated C₆₋₁₂ aryl and halogens, or 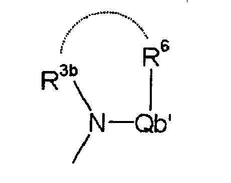 represents a 5- or 6-membered saturated cyclic amino).

More specifically, N-{[5-(4-chlorophenyl)-1-benzothiophen-2-yl]carbonyl}-O-(4-fluorobenzyl)tyrosine or (2S)-[4-(benzyloxy)phenyl]({[6-(4-chlorophenyl)-3-methyl-1-benzofuran-2-yl]carbonyl}amino) acetic acid or a salt thereof or the like can be used by preference as Compound (Ib).

The "5- or 6-membered aromatic ring" of the "optionally substituted 5- or 6-membered aromatic ring" given for ring Ac in Compound (Ic) may be a benzene, thiophene, furan, pyrrole, imidazole, pyrazole, pyridine, pyrazine, pyrimidine, pyridazine or the like. Preferably it is a benzene.

A substituent in the "optionally substituted 5- or 6-membered aromatic ring" given for ring Ac may be for example a group such as those given as substituents for the "optionally substituted isocyclic or heterocyclic ring" represented by ring A above. Ring Ac may have for example 1 to 4 or preferably 1 or 2 such substituents at substitutable positions, and when there are 2 or more substituents they may be the same or different.

An optionally substituted benzene or optionally substituted thiophene is preferred as ring Ac.

The "5- or 6-membered aromatic ring" of the "optionally substituted 5- or 6-membered aromatic ring" given for ring Bc in Compound (Ic) may be a benzene, thiophene, furan, pyrrole, imidazole, pyrazole, pyridine, pyrazine, pyrimidine, pyridazine or the like.

A substituent in the "optionally substituted 5- or 6-membered aromatic ring" given for ring Bc may be for example a group such as those given as substituents for the "optionally substituted isocyclic or heterocyclic ring" represented by ring A above. Ring Bc may have for example 1 to 4 or preferably 1 or 2 such substituents at substitutable positions, and when there are 2 or more substituents they may be the same or different.

Ring Bc is preferably a 5- or 6-membered aromatic ring (e.g., a benzene, thiophene, furan, pyrrole, imidazole, pyrazole, pyridine, pyrazine, pyrimidine or pyridazine) optionally having 1 to 3 substituents selected from halogen atom and optionally halogenated C₁₋₆ alkyl.

The "optionally substituted hydrocarbon group" represented by R^{3c} in Compound (Ic) may be similar to the "optionally substituted hydrocarbon group" represented by R³ above.

R^{3c} is preferably a hydrogen atom.

The "substituent" represented by R¹¹ in Compound (Ic) may preferably be a substituent having an optionally substituted aromatic group or the like. A "substituent having an optionally substituted aromatic group" includes, for example an optionally substituted aromatic group-C₁₋₆ alkoxy, optionally substituted aromatic group-C₁₋₆ alkylthio, optionally substituted aromatic group -C₁₋₆ alkylamino, optionally substituted aromatic group-C₁₋₆ alkyl, optionally substituted aromatic group-oxy, optionally substituted aromatic group-amino, optionally substituted aromatic group-thio, optionally substituted aromatic group-carbonyl, optionally substituted aromatic group-sulfonyl, optionally substituted aromatic group or the like.

Said "optionally substituted aromatic group" includes, for example, similar to the "optionally substituted aromatic group" represented by R^{1b} above. An optionally substituted phenyl, furyl or thienyl is preferred.

R¹¹ is preferably a C₇₋₁₃ aralkyoxy optionally having a substitutent group selected from halogen atom, optionally halogenated C₁₋₆ alkyl, cyano, optionally halogenated C₁₋₆ alkoxy, optionally halogenated C₁₋₆ alkoxycarbonyl and optionally halogenated C₁₋₆ alkylcarbonyl or the like. More preferably, it is a C₇₋₁₃ aralkyoxy optionally having an halogen atom.

The compound represented by the formula: 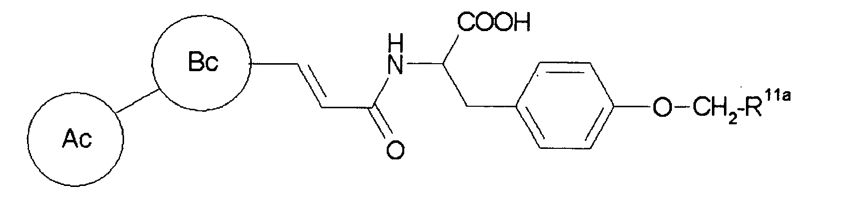 (wherein R^{11a} is an optionally substituted aromatic group and the other symbols are as defined previously) is preferred as Compound (Ic).

The "optionally substituted aromatic group" represented by R^{11a} may be similar to the "optionally substituted aromatic group" represented by R^{1b} above. It is preferably a C₇₌₁₃ aralkyloxy optionally having 1 to 3 halogen atom, and more preferably a benzyl optionally having 1 to 3 halogen atom.

A compound in which:
ring Ac is a benzene ring optionally having a halogen,
ring Bc is a 5- or 6-membered aromatic ring, and
R^{11a} is a C₇₋₁₃ aralkyloxy optionally having a halogen atom is preferred as Compound (Ic).

More specifically, O-benzyl-N-[(2E)-3-(4'-chlorobiphenyl-4-yl)propa-2-enoyl]tyrosine or a salt thereof can be used favorably as Compound (Ic).

The "aromatic condensed azole ring" of the "aromatic condensed azole ring which may also have substituents other than R¹² and R¹³" formed by ring F and ring G in compound (Id) may be for example a ring represented by the formula: 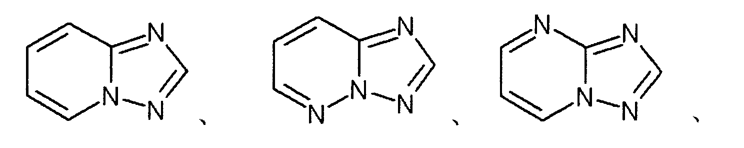 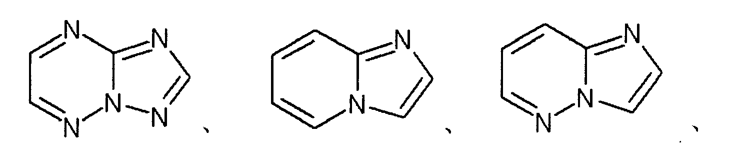 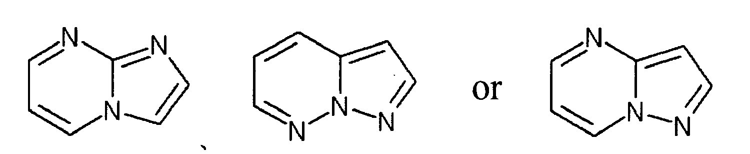 , etc. Preferably it is a ring represented by the formula: 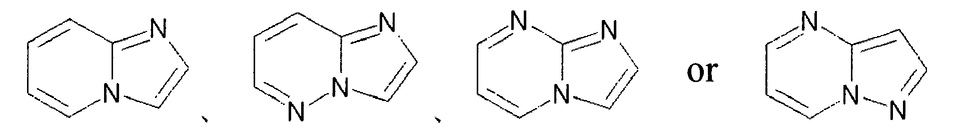 or the like.

Compound (Id) may be for example a compound represented by the formula: 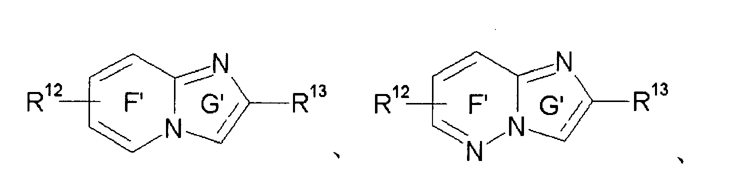 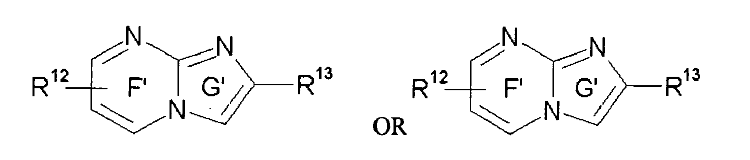 (wherein F' is the same as F, G' is the same as G, and the other symbols are as defined above)

A substituent in the "aromatic condensed azole ring which may also have substituents other than R¹² and R¹³" formed by ring F and ring G may for example be a group such as those given as substituents for the "optionally substituted isocyclic or heterocyclic ring" represented by ring A above. The aromatic condensed azole ring may have 1 to 4 or preferably 1 or 2 such substituents in substitutable positions, and when there are 2 or more substituents they may be the same or different.

Ring A, P, Qb and W in Compound (Id) may be similar to the ring A, P, Qb and W described above.

Ring A is preferably a 5- to 6-membered aromatic ring optionally having 1 to 3 substituents selected from halogen atom, optionally halogenated alkyl and optionally halogenated alkoxy.

P is preferably a bond or a spacer with 2 atoms in the main chain (e.g., -CH₂CH₂-, - CH=CH-, -C≡C-, -CH₂O-, -CH₂S-, -CH₂SO- or -CH₂SO₂-) or the like.

Qb is preferably a C₁₋₃ alkylene (e.g., a methylene) having a substituent (e.g., a C₁₋₆ alkoxy-C₇.₁₇ aralkyl or the like) with an optionally substituted aromatic group (e.g., a C₆₋₁₂ aryl, 5- to 10-membered aromatic heterocyclic group or the like).

More preferably, it is the group represented by the formula: 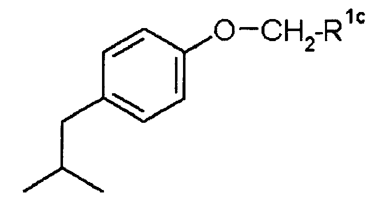 (wherein R^{1c} is an optionally substituted aromatic group).

The "optionally substituted aromatic group" represented by R^{1c} may be similar to the "optionally substituted aromatic group" given for R^{1b} above.

R^{1c} is preferably a 5- or 6-membered aromatic group optionally having 1 to 3 substituents selected from halogen atom, cyano, optionally halogenated C₁₋₆ alkyl and optionally halogenated C₁₋₆ alkoxy.

W is preferably a carboxyl, C₁₋₆ alkylsulfonylaminocarbonyl, 5-oxo-1,2,4-oxadiazole-3-yl, 5-tetrazolyl or 5-tetrazolylaminocarbonyl.

The "optionally substituted hydrocarbon group" represented by R^{3d} in Compound (Id) may be similar to the the "optionally substituted hydrocarbon group" represented by R³ above.

NR^{3d}-Qb-W is preferably an amino acid (for example glycine, alanine, beta-alanine, valine, leucine, isoleucine, serine, threonine, aspartic acid, glutamic acid, asparagine, glutamine, lysine, arginine, cysteine, methionine, phenylalanine, tyrosine, tryptophan, histidine, proline or the like) in which the functional group (amino, hydroxyl, thiol, carboxyl, phenolic hydroxyl or the like) may be protected or modified (for example, alkylated, aralkylated, esterified, acylated, amidated, halogenated or the like). Said amino acid may be an L-amino acid, D-amino acid or DL-amino acid.

Preferably Compound (Id) is the compound represented by the formula: 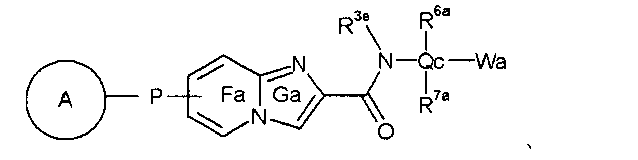 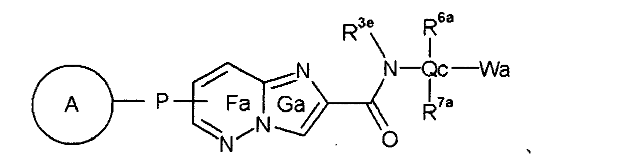 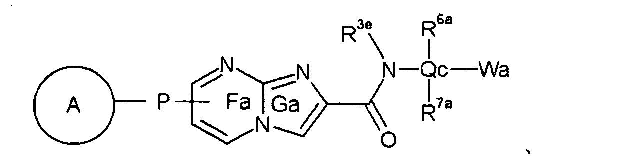 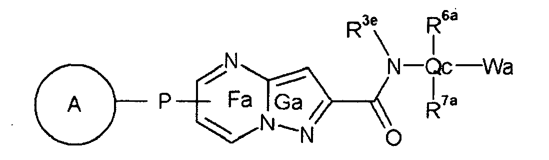 or 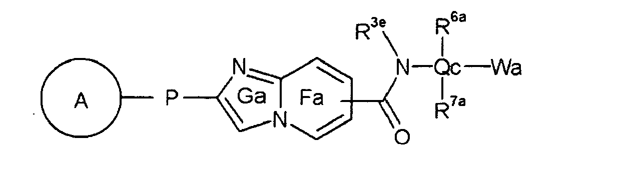 (wherein ring A is a 5- or 6-membered aromatic ring optionally substituted with 1 to 3 groups selected from halogen atom, optionally halogenated alkyl, optionally halogenated alkoxides and cyano,
P is a bond, ethylene, ethynylene, -CH₂-O- or -CH₂-S-,
ring Fa and ring Ga each optionally have 1 or 2 substituents selected from halogen atom and C₁₋₆ alkyl,
R^{3e} is a hydrogen atom or C₁₋₆ alkyl or the group represented by the formula: 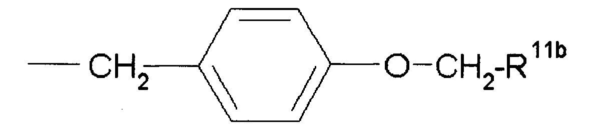 (wherein R^{11b} is a 5- or 6-membered aromatic group optionally substituted with 1 to 3 groups selected from halogen atom, optionally halogenated C₁₋₆ alkyl, optionally halogenated C₁₋₆ alkoxy and cyano),
Qc is a C₁₋₃ alkylene,
R^{6a} and R^{7a} are located on the same or different carbon atoms and are each hydrogen atom or group represented by the formula: 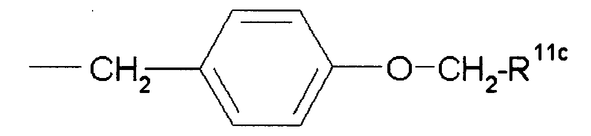 (wherein R^{11c} is a 5- or 6-membered aromatic group or C₃₋₆ cycloalkyl either of which is optionally substituted with 1 to 3 groups selected from halogen atom, cyano, optionally halogenated C₁₋₆ alkyl and optionally halogenated C₁₋₆ alkoxy), or alternatively R⁶ and R⁷ are bound together to form, together with an adjacent carbon atom, a C₃₋₇ cycloalkane optionally condensed with a C₆₋₁₂ aryl which may have 1 or 2 halogen atoms, and

Wa is a carboxyl, C₁₋₆ alkylsulfonylaminocarbonyl, 5-tetrazolyl, 5-tetrazolylaminocarbonyl or 5-oxo-1,2,4-oxadiazole-3-yl) or the like.

Specific examples of Compound (Id) include compounds in which, in the aforementioned formula,
ring A is a 6-membered aromatic ring optionally substituted with 1 to 3 groups selected from halogen atom, optionally halogenated C₁₋₆ alkyl and optionally halogenated C₁₋₆ alkoxy,
P is a bond, ethylene, ethenylene, ethynylene, -CH₂-O- or -CH₂-S-,
ring Fa and ring Ga may each have 1 or 2 substituents selected from halogen atom and C₁₋₆ alkyl,
R^{3e} is a hydrogen atom or C₁₋₆ alkyl or a group represented by the formula: 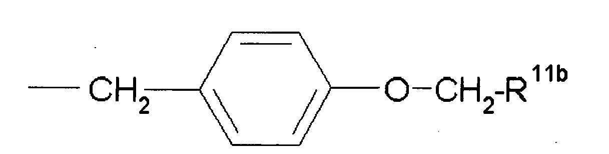 (wherein R^{11b} is a 5- or 6-membered aromatic group optionally having 1 to 3 halogen atom),
Qc is a C₁₋₃ alkylene,
R^{6a} and R^{7a} are located on the same or different carbon atoms and are each hydrogen atoms or group represented by the formula: 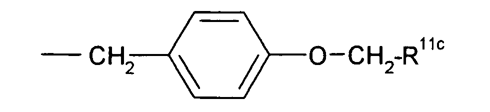 (wherein R^{11c} is a 5- or 6-membered aromatic group or C₃₋₆ cycloalkyl either of which is optionally substituted with 1 to 3 groups selected from halogen atom, cyano, optionally halogenated C₁₋₆ alkyl and optionally halogenated C₁₋₆ alkoxy), or R^{6a} and R^{7a} are bound together to form, together with an adjacent carbon atom, a C₃₋₇ cycloalkane optionally condensed with a benzene ring which may have 1 or 2 halogen atoms, and

Wa is a carboxyl, C₁₋₆ alkylsulfonylaminocarbonyl, 5-tetrazolyl or 5-oxo-1,2,4-oxadiazole-3-yl) or the like.

More specifically, O-benzyl-N-{[6-(4-chlorophenyl)imidazo[1,2-b] pyridazine-2-yl]carbonyl}tyrosine, O-benzyl-N-{[6-(phenylethynyl)imidazo[1,2-b] pyridazine-2-yl]carbonyl} tyrosine, O-benzyl-N-{[6-[(E)-2-(4-chlorophenyl)ethenyl]imidazo[1,2-b] pyridazine-2-yl}carbonyl) tyrosine, O-benzyl-N-{[7-(4-chlorophenyl)imidazo[1,2-a] pyridine-2-yl]carbonyl}-N-methyltyrosine, N-{[7-(4-chlorophenyl)imidazo[1,2-a]pyridine-2-yl]carbonyl}-O-(4-methylbenzyl) tyrosine, or O-(4-chlorobenzyl)-N-{[7-(4-chlorophenyl)imidazo[1,2-a] pyridine-2-yl]carbonyl} tyrosine or a salt thereof may be used by preference as Compound (Id).

Salts of Compound (I) (including Compound (Ia), Compound (Ib), Compound (Ic) and Compound (Id) and its intermediates include for example metal salts, ammonium salts, salts with organic bases, salts with inorganic acids, salts with organic acids and salts with basic or acidic amino acids and the like. Desirable examples of metal salts include sodium salts, potassium salts and other alkali metal salts; calcium salts, magnesium salts, barium salts and other alkali earth metal salts; and aluminum salts and the like. Desirable examples of salts with organic bases include salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine and the like. Desirable examples of salts with inorganic acids include salts with hydrochloric acid, hydrobromic acid, nitric acid, sulfuric acid, phosphoric acid and the like. Desirable examples of salts with organic acids include salts with formic acid, acetic acid, trifluoracetic acid, phthalic acid, fumaric acid, oxalic acid, tartaric acid, maleic acid, citric acid, succinic acid, malic acid methanesulfonic acid, benzenesulfonic acid, p-toluenesulfonic acid and the like. Desirable examples of salts with basic amino acids include salts with arginine, lysine, ornithine and the like, while desirable examples of salts with acid amino acids include salts with aspartic acid, glutamic acid and the like.

Of these, a pharmacologically acceptable salt is preferred. Examples include alkali metal salts (sodium salts, potassium salts and the like), alkali earth metal salts (calcium salts, magnesium salts, barium salts and the like) and other inorganic salts and ammonium salts and the like when the compound contains acidic functional groups and salts with hydrobromic acid, nitric acid, sulfuric acid, phosphoric acid and other inorganic acids or salts with acetic acid, phthalic acid, fumaric acid, oxalic acid, tartaric acid, maleic acid, citric acid, succinic acid, methanesulfonic acid, p-toluenesulfonic acid and other organic acids when the compound contains basic functional groups.

Compound (I) may be either a hydrate or non-hydrate. If a hydrate, it may be an 0.5 hydrate, 1 hydrate, 1.5 hydrate or 2 hydrate.

Compound (I) can be as necessary obtained in the desired R form or S form by using well known methods such as asymmetric synthesis, optical resolution and the like.

A prodrug of Compound (I) is a compound that is changed into Compound (I) by a reaction caused by an enzyme or stomach acid or the like under physiological conditions in the body, or in other words a compound that undergoes enzymatic oxidation, reduction, hydrolysis or the like that converts it to Compound (I) or a compound that undergoes hydrolysis caused by stomach acid or the like that converts it to Compound (I). Examples of prodrugs of Compound
(I) include compounds in which an amino of Compound (I) is acylated, alkylated or phosphorylated (for example, compounds in which an amino of Compound (I) is eicosanoylated, alanylated, pentylaminocarbonylated, (5-methyl-2-oxo-1,3-dioxolene-4-yl)methoxycarbonylated, tetrahydrofuranylated, pyrrolidylmethylated, pivaloyloxymethylated, tert-butylated or the like), compounds in which a hydroxyl of Compound (I) is acylated, alkylated, phosphorylated or borated (for example, compounds in which a hydroxyl of Compound (I) is acetylated, palmitoylated, propanoylated, pivaloylated, succinylated, fumarylated alanylated, dimethylaminomethylcarbonylated or the like), and compounds in which a carboxyl of Compound (I) is esterified or amidated (for example, compounds in which a carboxyl of Compound (I) is ethylesterified, phenylesterified, carboxymethylesterified, dimethylaminomethylesterified, pivaloyloxymethylesterified, ethoxycarbonyloxyethylesterified, phthalidylesterified, (5-methyl-2-oxo-1,3-dioxolene-4-yl) methylesterified, cyclohexyloxycarbonylethylesterified, methylamidated or the like) and the like. These compounds can be manufactured from Compound (I) by known methods.

A prodrug of Compound (I) may also be one that changes into Compound (I) under physiological conditions as described in Igakuhin no Kaihatsu Vol. 7, Bunshisekkei, pp. 163-198, Hirokawa Shoten, 1990.

Methods for manufacturing Compound (I) are described below.

When alkylation reactions, amidation reactions (condensation reactions), esterification reactions, reduction reactions, reductive amination reactions and the like are performed in the manufacturing methods below, they may be performed by known methods. Examples of such methods include those described in Organic Functional Group Preparations, Vol. 2, Academic Press Inc., 1989 and Comprehensive Organic Transformations, VCH Publishers Inc., 1989 and the like.

When a raw material compound is capable of forming a salt in the manufacturing methods below, the compound may be used in the form of a salt. Those salts given as examples of salts of Compound (I) may be used as such salts.

The target compound obtained by the following manufacturing methods may be isolated and purified by known methods of isolation and purification, such as concentration, vacuum concentration, solvent extraction, crystallization, recrystallization, solvent transfer, chromatography and the like. When the manufacturing method comprises multiple steps, a synthesis intermediate may be isolated and purified by known isolation and purification means, or may be used in the following step as a reaction mixture without isolation and purification.

Those solvents used in the following reactions that are described by general terms are explained below.

Methanol, ethanol, 1-propanol, 2-propanol, tert-butyl alcohol and the like are used as "alcohols".

Diethyl ether, diisopropyl ether, diphenyl ether, tetrahydrofuran, 1,4-dioxane, 1,2-dimethoxyethane and the like are used as "ethers".

Ethyl acetate, methyl acetate, tert-butyl acetate and the like are used as "esters".

Benzene, toluene, xylene, cyclohexane, hexane, pentane and the like are used as "hydrocarbons".

N,N-dimethylformamide, N,N-dimethylacetamide, hexamethylphosphoric triamide and the like are used as "amides".

Dichloromethane, chloroform, carbon tetrachloride, 1,2-dichloroethane, tetrachloroethylene, chlorobenzene and the like are used as "halogenated hydrocarbons".

Acetonitrile, propionitrile and the like are used as "nitriles".

Acetone, 2-butanone and the like are used as "ketones".

Formic acid, acetic acid, propionic acid, trifluoracetic acid, methanesulfonic acid and the like are used as "organic acids".

Pyridine, 2,6-lutidine, quinoline and the like are used as "aromatic amines".

Dimethylsulfoxide and the like are used as "sulfoxides".

### [Method A]

Compounds (I-a), (I'), (Ia-a), (Ib-a), (Ic), (Id-a) and (Ie-a) in which the W of Compound (I) is a carboxyl group can be manufactured for example by converting the esters of Compounds (I-1), (I'-1), (Ia-1), (Ib-1), (Ic-1), (Id-1) and (Ie-1), respectively, into carboxylic acids. 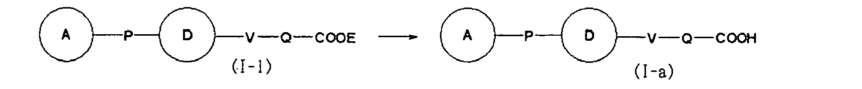 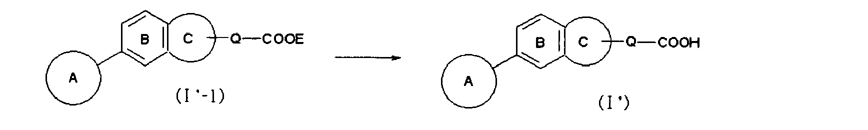 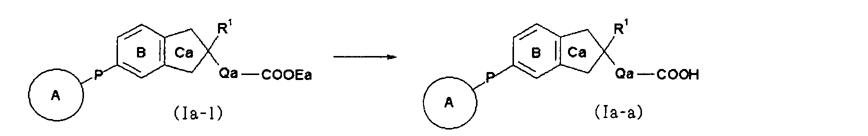 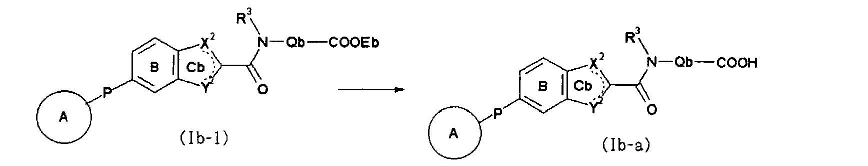 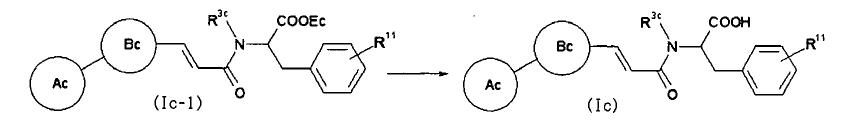 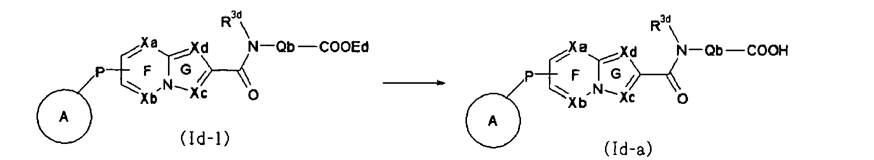 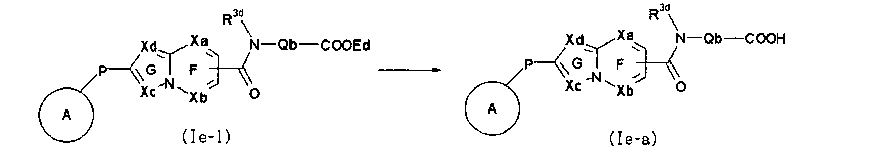 (wherein E, Ea, Eb, Ec and Ed are each ester residues, and the other symbols are as defined above).

A known carboxylic acid ester protective group can be used as the ester residue represented by E, Ea, Eb, Ec or Ed, and examples include hydrocarbon groups optionally having substituents selected from halogen atom and C₁₋₆ alkyl, C₁₋₆ alkoxy and nitro group (for example, C₁₋₆ alkyl such as methyl, ethyl, propyl, butyl, tert-butyl, 2,2,2-trichloroethyl, methoxymethyl and the like, C₂₋₆ alkenyl such as vinyl, allyl, methacryl and the like, C₇₋₁₄ aralkyl such as benzyl, 4-methoxybenzyl, 2-nitrobenzyl and the like) and C₆₋₁₄ aryl such as phenyl and the like) and heterocyclic group and tri-substituted silyl group (e.g., trimethylsilyl, triethylsilyl, tertbutyldimethylsilyl and tert-butyldiphenylsilyl, etc.) and the like.

This reaction can be accomplished by a well-known method selected according to the type of ester residue from among the hydrolysis reactions using acids or bases, hydrogenolysis reactions using catalysts and hydrogen sources, ultraviolet irradiation reactions, de-allylation reactions using catalysts, de-silylation reactions using fluorine anions and the like (e.g., the methods described in Protective Groups in Organic Synthesis, John Wiley and Sons, 1980) or by an equivalent method in solvents that do not have an adverse effect.

Examples of solvents that do not have adverse effects include hydrocarbons, alcohols, ether solvents, halogenated hydrocarbons, nitriles, amides, water and the like. A mixture of two or more of these in suitable proportions may also be used. Of these, an alcohol or ether or water is preferred.

Inorganic acids (e.g., nitric acid, hydrochloric acid, hydrobromic acid, iodic acid, sulfuric acid or the like) and organic acids (e.g., trifluoracetic acid, trichloroacetic acid or the like) can be used as acids.

Aqueous solutions of hydroxides of alkali metals or alkali earth metals (e.g., sodium hydroxide, potassium hydroxide, barium hydroxide, lithium hydroxide and the like) can be used as bases.

The strength of acids and bases may be for example about 1 to 10 N or preferably about 4 to 10 N.

Palladium (for example, palladium carbon, palladium black, active charcoal or the like), nickel (for example, Raney nickel or the like), platinum (e.g., platinum oxide, etc.) or the like can be used as the catalyst in hydrogenolysis.

Hydrogen gas, 1,4-cyclohexadiene, hydrazine, formic acid (e.g., formic acid or ammonium formate, etc.) or the like can be used as the hydrogen source in hydrogenolysis.

Zero-valent or bivalent palladium (e.g., palladium acetate, palladium chloride, tetrakis triphenylphosphine palladium or active charcoal, etc.) or the like can be used as the catalyst in a de-allylation reaction.

Hydrogen fluoride, potassium fluoride, cesium fluoride, tetrabutyl ammonium fluoride or the like can be used for the fluorine anions in a de-silylation reaction.

The reaction temperature is normally 0 to 150°C or preferably 20 to 80°C.

The reaction time is 1 to 24 hours for example or preferably about 2 to 10 hours.

### [Method B]

Compound (I'-1), Compound (Ia-1a) and Compound (Ib-1a) can be manufactured for example by reacting Compound (I'-2), Compound (Ia-2) and Compound (Ib-2), respectively, with Compound (I-3). 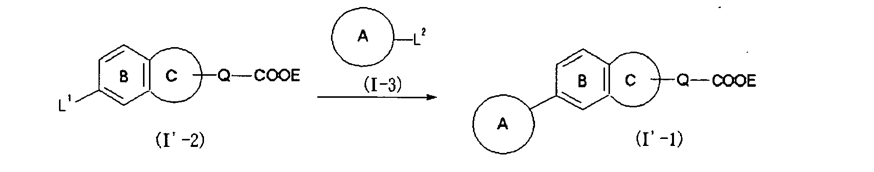 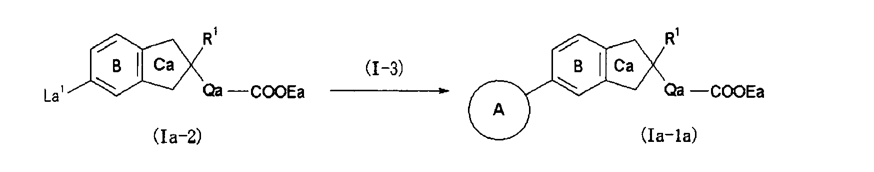 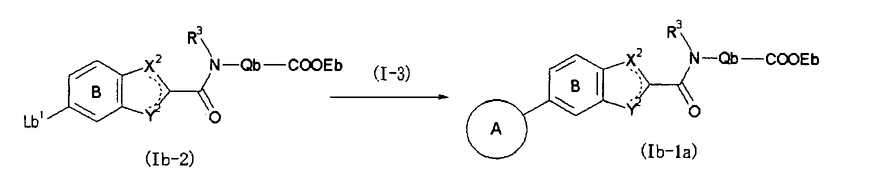 (wherein L', La¹ and Lb¹ are each leaving groups or functional groups capable of cross-coupling reactions with Compound (I-3), L² is a functional group capable of cross-coupling reactions with Compound (I'-2), Compound (Ia-2) and Compound (Ib-2) or L² is a hydrogen atom when ring A is a nitrogen-containing heterocyclic ring and L² is on a nitrogen atom, and the other symbols are as defined above).

When Compound (I-3) is commercially available the commercial product can be used as is, or Compound (I-3) can be manufactured by known methods or their equivalents.

Compound (I'-2) can be manufactured by known methods or their equivalents.

A cross-coupling reaction is a coupling reaction using a metal catalyst, and may be for example a Suzuki reaction, Heck reaction, Stille reaction, Buchwald amination reaction or other known coupling reaction.

When using a Suzuki reaction, Stille reaction or other cross-coupling reaction, the functional groups capable of cross-coupling reactions (L¹, La¹, Lb¹ and L²) form pairs with one another, and when a halogen atom (e.g., a chlorine, bromine or iodine) or trifluoromethylsulfonyloxy for example is used for one, boron (such a boric acid, boric acid ester, diethylboron or the like), tin (e.g., trimethyl tin or tributyl tin), zinc (e.g., zinc chloride or the like), magnesium (e.g., magnesium chloride or magnesium bromide) or the like is used for the other.

A halogen atom (e.g., chlorine, bromine or iodine) or trifluoromethylsulfonyloxy or the like may be used as the leaving group represented by L¹, La¹ or Lb¹.

Palladium (e.g., tetrakis triphenylphosphine palladium, dichlorobis(triphenylphosphine) palladium, palladium acetate, di-µ-chlorobis(η-allyl) palladium (II), palladium carbon or the like), nickel (e.g., nickel chloride or the like) or copper (e.g., copper acetate) or the like can be used for the metal catalyst.

This reaction can be performed in solvents that do not adversely affect the reaction. Examples of such solvents include hydrocarbons, alcohols, ethers, amides and water and the like. A combination of two or more such solvents in suitable proportions can also be used. Of these, an alcohol, hydrocarbon or ether or water is preferred.

This reaction may also be performed in the presence of a base if necessary. Examples of such bases include carbonates of alkali metals or alkali earth metals (e.g., sodium carbonate, potassium carbonate or cesium carbonate, etc.), alkoxides of alkali metals or alkali earth metals (e.g., sodium methoxide or sodium tert-butoxide, etc.) and the like.

The base is used in the amount of normally 0.1 to 10 mole equivalents or preferably 0.5 to 2 mole equivalents per 1 mole of Compounds (I'-2), (Ia-2) and (Ib-2).

The metal catalyst is used in the amount of normally 0.01 to 1 mole equivalent or preferably 0.03 to 0.1 mole equivalent per 1 mole of Compound (I'-2), (Ia-2) or (Ib-2).

Compound (I-3) is used in the amount of 0.5 to 10 mole equivalents or preferably 1 to 2 mole equivalents per 1 mole of Compound (I'-2), (Ia-2) or (Ib-2).

The reaction temperature is normally 0 to 200°C or preferably 50 to 150°C.

The reaction time is normally 0.5 to 48 hours or preferably 1 to 24 hours.

A microwave reactor may be used for this reaction. In this case, the reaction temperature is normally 20 to 200°C or preferably 100 to 150°C and the reaction time is normally 1 minute to 4 hours or preferably 1 minute to 30 minutes.

### [Method C]

Compound (Ib-1a) can be manufactured by means of a condensation reaction between Compound (Ib-4) and Compound (Ib-5) for example: 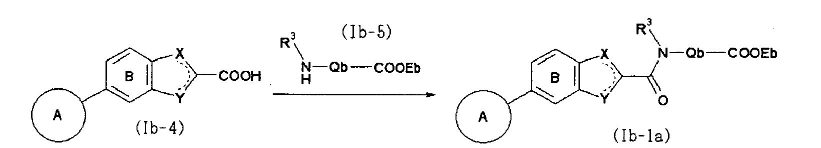 (wherein all symbols are as defined previously).

The condensation reaction between Compound (Ib-4) and Compound (Ib-5) can be performed using a condensing agent in solvents that do not adversely affect the reaction.

When Compound (Ib-5) is commercially available the commercial product can be used as is, or the compound can be manufactured by known methods or their equivalents.

Examples of solvents that do not adversely affect the reaction include ethers, alcohols, hydrocarbons, halogenated hydrocarbons, nitriles, amides, esters and the like. A combination of two or more such solvents in suitable proportions can also be used.

The condensing agent may be a condensing agent used in peptide synthesis for example, and specific examples include carbodiimide derivatives (e.g., dicyclohexylcarbodiimide, 1-ethyl-3-(3-dimethylaminopropyl)carbodiimide and diisopropylcarbodiimide, etc.), phosphor reagents (e.g., cyanodiethyl phosphate or BOP-Cl, etc.), triazine condensing agents (e.g., 4-(4,6-dimethoxy-1,3,5-triazine-2-yl)-4-methylmorpholinium hydrochloride, etc.), carbodiimidazole and the like.

This reaction may be performed in the presence of a base if necessary, and this base may be for example triethylamine, diisopropylethylamine, 4-dimethylaminopyridine, triethylenediamine, tetramethylethylenediamine, N-methylmorpholine or the like.

Compound (Ib-5) is used in the amount of normally 0.5 to 5 mole equivalents or preferably 0.8 to 1.5 mole equivalents per 1 mole of Compound (Ib-4).

The condensing agent is used in the amount of normally 0.5 to 10 mole equivalents or preferably 1 to 2 mole equivalents per 1 mole of Compound (Ib-4).

The base is used in the amount of normally 1 to 10 mole equivalents or preferably 1 to 5 mole equivalents per 1 mole of Compound (Ib-4).

The reaction temperature is normally 0 to 100°C or preferably 20 to 50°C. The reaction time is normally 0.5 to 100 hours or preferably 1 to 48 hours.

After the carboxylic acid of Compound (Ib-4) has been converted to a reactive derivative, this reaction can be performed in solvents that do not adversely affect the reaction, and in the presence of a base if necessary.

Examples of reactive derivatives include acid halides (acid chlorides, acid bromides and the like), acid imidazolides, acid azides, acid anhydrides, mixed acid anhydrides, esters, active esters (for example, 1-hydroxybenzotriazole ester, N-hydroxysuccinimide ester or N-hydroxy-5-norbornen-2,3-dicarboxyimide ester) and the like. This reactive derivative can be manufactured by known methods from the carboxylic acid.

Examples of solvents that do not adversely affect the reaction include ethers, alcohols, hydrocarbons, halogenated hydrocarbons, ketones, nitriles, amides, esters, pyridine, water and the like. A mixture of two or more such solvents in suitable proportions can also be used.

The base may be for example a tertiary amine (e.g., triethylamine, diisopropylethylamine, pyridine, 4-dimethylaminopyridine, triethylenediamine, tetramethylethylenediamine, N-methylmorpholine or the like), inorganic base (e.g., sodium hydrogencarbonate, sodium carbonate, potassium carbonate, sodium hydroxide or potassium hydroxide, etc.) or the like.

Compound (Ib-5) is used in the amount of 0.5 to 5 mole equivalents or preferably 0.8 to 1.5 mole equivalents per 1 mole of Compound (Ib-4).

The base is used in the amount of 1 to 10 mole equivalents or preferably 1 to 5 mole equivalents per 1 mole of Compound (Ib-4).

The reaction temperature is normally -20 to 100°C or preferably 0 to 50°C.

The reaction time is normally 0.5 to 100 hours or preferably 1 to 24 hours.

The Compound (Ia-2) used as a raw material compound in [Method B] above can be manufactured by the following [Method D] or [Method E], or by equivalents methods.

### [Method D]

A Compound (Ia-2a) in which the Qa of Compound (Ia-2) is a bond can be manufactured for example by subjecting the amino group of Compound (Ia-3) to an alkylation or acylation reaction.

### [Method E]

Compounds (Ia-2b) in which the Qa of Compound (Ia-2) is -CO-Qa'- can be manufactured for example by converting the ester of Compound (Ia-2a) to a carboxylic acid to obtain Compound (Ia-4), which is then subjected to a condensation reaction with various amino acid esters, hydroxy acid esters and mercapto acid esters (Ia-5). 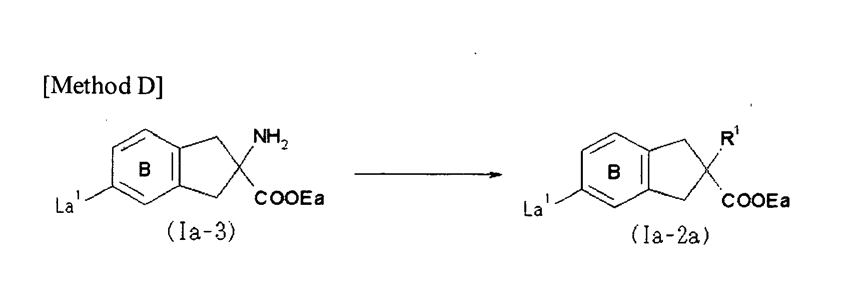 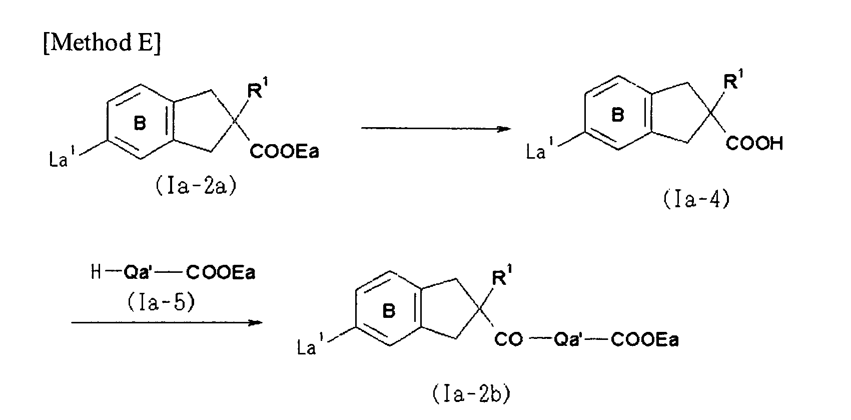 (wherein all symbols are as defined previously).

When Compound (Ia-5) is commercially available the commercial product can be used as is, or the compound can be manufactured by known methods or their equivalents.

Alkylation or acylation reaction of an amino group is used to convert Compound (Ia-3) into Compound (Ia-2a), and may be performed by known methods or their equivalents in solvents that do not adversely affect the reaction.

Alkylation reaction of an amino group is for example a nucleophilic substitution reaction with an alkyl halide or a reductive alkylation reaction with an aldehyde or ketone.

Such a nucleophilic substitution reaction with an alkyl halide is performed in the presence of a base in solvents that do not adversely affect the reaction. Examples of solvents that do not adversely affect the reaction include ethers, alcohols, hydrocarbons, halogenated hydrocarbons, nitriles, amides, esters and the like. A combination of two or more such solvents in suitable proportions can also be used.

The base may be for example a tertiary amine (e.g., triethylamine, diisopropylethylamine, pyridine, 4-dimethylaminopyridine, triethylenediamine, tetramethylethylenediamine, N-methylmorpholine or the like), inorganic base (e.g., sodium hydrogencarbonate, sodium carbonate, potassium carbonate, cesium carbonate, sodium hydroxide or potassium hydroxide) or the like.

The alkyl halide is used in the amount of normally 1 to 5 or preferably 1 to 3 mole equivalents per 1 mole of Compound (Ia-3).

The base is used in the amount of normally 1 to 10 or preferably 1 to 5 mole equivalents per 1 mole of Compound (Ia-3).

The reaction temperature is normally 0 to 150°C or preferably 0 to 80°C. The reaction time is normally 0.5 to 100 hours or preferably 1 to 24 hours.

The reductive alkylation reaction with the aldehyde or ketone can be performed using a reducing agent in solvents that do not adversely affect the reaction. Examples of solvents that do not adversely affect the reaction include ethers, alcohols, hydrocarbons, halogenated hydrocarbons , nitriles, amides, esters, acetic acid and the like. A mixture of two or more such solvents in suitable proportions can also be used.

The reducing agent may be for example a borohydride (e.g., sodium borohydride, lithium borohydride, sodium cyanoborohydride, sodium triacetoxyborohydride or the like), or a hydrogen for hydrogenetion or the like.

This reaction may also be performed in the presence of an acid. Acetic acid, trifluoracetic acid, hydrochloric acid or the like can be used as the acid.

The aldehyde or ketone is used in the amount of normally 1 to 5 or preferably 1 to 3 mole equivalents per 1 mole of Compound (Ia-3).

The reducing agent is used in the amount of normally 0.25 to 5 or preferably 0.5 to 2 mole equivalents per 1 mole of Compound (Ia-3).

The acid is used in the amount of normally 1 to 10 or preferably 1 to 5 mole equivalents per 1 mole of Compound (Ia-3).

The reaction temperature is normally 0 to 150°C or preferably 0 to 50°C

The reaction time is normally 0.5 to 100 hours or preferably 1 to 24 hours.

An acylation reaction of the amino group of Compound (Ia-3) may be for example a) an amidation reaction with a carboxylic acid or reactive derivative thereof, b) a sulfonamidation reaction with a sulfonyl halide, c) a ureido- or thioureido-producing reaction with an isocyanate, carbamoyl halide or thioisocyanate, or d) a carbamation reaction with alkoxycarbonyl chloride.
a) An amidation reaction with a carboxylic acid or reactive derivative thereof can be performed by methods similar to those described for the reaction in [Method C].
b) A sulfonamidation reaction with a sulfonyl halide can be performed in the presence of a base in solvents that do not adversely affect the reaction. Examples of solvents that do not adversely affect the reaction include ethers, hydrocarbons, halogenated hydrocarbons, nitriles, amides, esters, water, pyridine and the like. A mixture of two or more such solvents in suitable proportions can also be used.
   The base may be for example a tertiary amine (e.g., triethylamine, diisopropylethylamine, pyridine, 4-dimethylaminopyridine, triethylenediamine, tetramethylethylenediamine, N-methylmorpholine or the like), inorganic base (e.g., sodium hydrogencarbonate, sodium carbonate, potassium carbonate, cesium carbonate, sodium hydroxide or potassium hydroxide, etc.) or the like.
   The base is used in the amount of 1 to 10 or preferably 1 to 5 mole equivalents per 1 mole of Compound (Ia-3).
   The sulfonyl halide is used in the amount of 1 to 10 or preferably 1 to 2 mole equivalents per 1 mole of Compound (Ia-3).
   The reaction temperature is normally 0 to 150°C or preferably 0 to 50°C.
   The reaction time is normally 0.5 to 100 hours or preferably 1 to 24 hours.
c) A ureido- or thioureido-producing reaction with an isocyanate, carbamoyl halide or thioisocyanate and d) a carbamation reaction with alkoxycarbonyl chloride can be performed by methods similar to those used for the aforementioned sulfonylation reaction.

A reaction that converts an ester to a carboxylic acid is used as the reaction that converts Compound (Ia-2a) to Compound (Ia-4), and can be performed by methods similar to those used in [Method C] above.

The condensation reaction between Compound (Ia-4) and Compound (Ia5) is performed using a condensing agent in solvents that do not adversely affect the reaction.

Examples of solvents that do not adversely affect the reaction include ethers, alcohols, hydrocarbons, halogenated hydrocarbons, nitriles, amides, esters and the like. A combination of two or more such solvents in suitable proportions can also be used.

The condensing agent may be a condensing agent used in peptide synthesis for example, and specific examples include carbodiimide derivatives (e.g., dicyclohexylcarbodiimide, 1-ethyl-3-(3-dimethylaminopropyl)carbodiimide, diisopropylcarbodiimide, etc.), phosphor reagents (e.g., cyanodiethyl phosphate and BOP-Cl, etc.), triazine condensing agents (e.g., 4-(4,6-dimethoxy-1,3,5-triazine-2-yl)-4-methylmorpholinium hydrochloride, etc.), carbodiimidazole and the like.

This reaction may be performed in the presence of a base if necessary, and examples of such bases include triethylamine, diisopropylethylamine, 4-dimethylaminopyridine, triethylenediamine, tetramethylethylenediamine, N-methylmorpholine and the like.

Compound (Ia-5) is used in the amount of 0.5 to 5 mole equivalents or preferably 0.8 to 1.5 mole equivalents per 1 mole of Compound (Ia-4).

The condensing agent is used in the amount of 0.5 to 10 moles or preferably 1 to 2 moles per 1 mole of Compound (Ia-4).

The base is used in the amount of 1 to 10 or preferably 1 to 5 mole equivalents per 1 mole of Compound (Ia-4).

The reaction temperature is normally 0 to 100°C or preferably 20 to 50°C.

The reaction time is normally 0.5 to 100 hours or preferably 1 to 48 hours.

This reaction can be performed in the presence of a base if necessary in solvents that do not adversely affect the reaction after the carboxylic acid of Compound (Ia-4) has been converted to a reactive derivative.

Examples of such reactive derivatives include acid halides (e.g., acid chlorides and acid bromides, etc.), acid imidazolides, acid azides, acid anhydrides, mixed acid anhydrides, esters, active esters (e.g., 1-hydroxybenzotriazole ester, N-hydroxysuccinimide ester and N-hydroxy-5-norbornen-2,3-dicarboxyimide ester, etc.) and the like. This reactive derivative can be manufactured by known methods from the carboxylic acid.

Examples of solvents that do not adversely affect the reaction include ethers, alcohols, hydrocarbons, halogenated hydrocarbons, ketones, nitriles, amides, esters, pyridine, water and the like. A mixture of two or more such solvents in suitable proportions can also be used.

The base may be for example a tertiary amine (e.g., triethylamine, diisopropylethylamine, pyridine, 4-dimethylaminopyridine, triethylenediamine, tetramethylethylenediamine, N-methylmorpholine or the like), inorganic base (e.g., sodium hydrogencarbonate, sodium carbonate, potassium carbonate, sodium hydroxide or potassium hydroxide, etc.) or the like.

Compound (Ia-5) is used in the amount of normally 0.5 to 5 or preferably 0.8 to 1.5 mole equivalents per 1 mole of Compound (Ia-4).

The base is used in the amount of normally 1 to 10 or preferably 1 to 5 mole equivalents per 1 mole of Compound (Ia-5).

The reaction temperature is normally -20 to 100°C or preferably 0 to 50°C.

The reaction time is normally 0.5 to 100 hours or preferably 1 to 24 hours.

### [Method F]

Compound (Ia-3) can be manufactured according to the following scheme.

Compound (Ia-7) or Compound (Ia-8) obtained by a reduction reaction or the like of Compound (Ia-6) is brominated to obtain Compound (Ia-9), which is then formed into a ring by an alkylation reaction with Compound (Ia-10) to derive Compound (Ia-11), and the isocyanate group of this Compound (Ia-11) is converted into an amino group by acid treatment to thereby manufacture Compound (Ia-3). 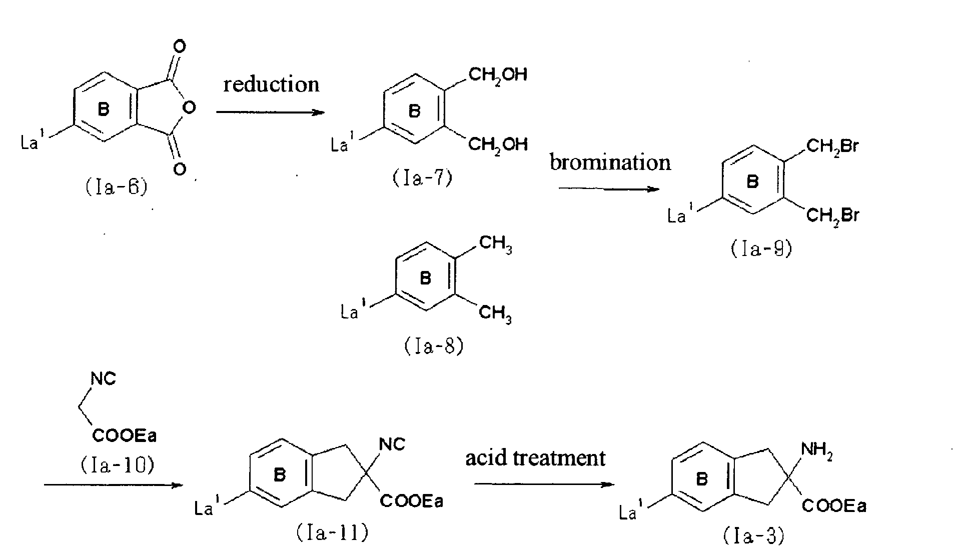 (wherein all symbols are as defined as above).

When Compound (Ia-6) and Compound (Ia-10) are commercially available the commercial products may be used as is, or the compounds may be manufactured by known methods or their equivalents.

Compound (Ia-7) is manufactured by subjecting Compound (Ia-6) to a reduction reaction. This reaction is performed using a reducing agent in solvents that do not adversely affect the reaction.

Examples of solvents that do not adversely affect the reaction include hydrocarbons, ethers and the like. A mixture of two or more such solvents in suitable proportions can also be used. Of these, an ether is preffered.

The reducing agent may be for example an aluminum hydride (aluminum lithium hydride, diisobutyl aluminum hydride or the like) a borane (e.g., diborane, or borane-dimethylsulfide complex, etc.) or the like.

The reaction temperature is normally -100 to 100°C or preferably -20 to 80°C. The reaction time is 1 to 24 hours for example or preferably about 1 to 10 hours.

Compound (Ia-9) is manufactured by subjecting Compound (Ia-7) or Compound (Ia-8) to a bromination reaction. This reaction is performed using a brominating agent in solvents that do not adversely affect the reaction.

The reaction that converts Compound (Ia-7) into Compound (Ia-9) is a reaction that converts hydroxyl groups into bromo groups, while the reaction that converts Compound (Ia-8) into Compound (Ia-9) is a bromination reaction of benzyl position carbon atoms, and known bromination reactions can be used for each.

The solvent used for the reaction that converts Compound (Ia-7) into Compound (Ia-9) may be a hydrocarbon, ether, halogenated hydrocarbon, nitrile, amide, ester or acetic acid or the like. A combination of two or more such solvents in suitable proportions can also be used. Of these, a halogenated hydrocarbon, ether, hydrocarbon or the like is preferred, and of these, a halogenated hydrocarbon or hydrocarbon is especially preferred.

The solvent used for the reaction that converts Compound (Ia-8) into Compound (Ia-9) may be a hydrocarbon, ether, halogenated hydrocarbon, ester or the like. A combination of two or more such solvents in suitable proportions can also be used. Of these, a halogenated hydrocarbon, hydrocarbon or ester is preferred.

Thionyl bromide, phosphorus tribromide, phosphorus oxybromide, hydrobromic acid or the like can be used as the brominating agent in the reaction that converts Compound (Ia-7) into Compound (Ia-9). Of these, thionyl bromide or phosphorus tribromide is preferred.

N-bromosuccinimide, bromine, hydrobromic acid or the like can be used as the brominating agent in the reaction that converts Compound (Ia-8) into Compound (Ia-9). Of these, N-bromosuccinimide is preferred.

When N-bromosuccinimide for example is used as the brominating agent in the reaction that converts Compound (Ia-8) into Compound (Ia-9), the reaction can be made more efficient through the use of a radical initiator. The radical initiator can be for example an azobis radical initiator (e.g., dimethyl 2,2'-azobis isobutyrate, azobis cyanovaleric acid, 1,1-azobis (cyclohexane-1-carbonitrile), 2,2'-azobis(2,4-dimethylvaleronitrile), azobisisobutyronitrile, 2,2'-azobis(4-methoxy-2,4-dimethylvaleronitrile) or the like) or triethylborane, tributyltin hydride or the like. Of these, azobisisobutyronitrile or 2,2'-azobis(4-methoxy-2,4-dimethylvaleronitrile) is preferred. The radical initiator is used in the amount of normally 0.01 to 1 mole equivalents or preferably 0.05 to 0.2 mole equivalents per 1 mole of Compound (Ia-8).

The brominating agent is used in the amount of normally 1.8 to 3 mole equivalents or preferably 2 to 2.5 mole equivalents per 1 mole of Compound (Ia-7) or Compound (Ia-8).

The reaction temperature is normally -100 to 200°C or preferably -20 to 120°C. The reaction time is for example 0.1 to 100 hours.

Compound (Ia-11) can be manufactured by reacting Compound (Ia-9) with Compound (Ia-10). This reaction is performed using a base in the presence of solvents that do not adversely affect the reaction.

Examples of solvents that do not adversely affect the reaction include hydrocarbons, alcohols, ethers, halogenated hydrocarbons, nitriles, amides, ketones, sulfoxides, esters, water and the like. A mixture of two or more such solvents in suitable proportions can also be used. Of these, a nitrile, alcohol, ether, hydrocarbon or amide for example is preferred, and a nitrile or hydrocarbon is especially preferred.

### Examples of bases for use in this reaction include

1) Hydrides of alkali metals or alkali earth metals (for example, lithium hydride, sodium hydride, potassium hydride, calcium hydride and the like), amides of alkali metals or alkali earth metals (for example, lithium amide, sodium amide, lithium diisopropylamide, lithium dicyclohexylamide, lithium hexamethyldisilazide, sodium hexamethyldisilazide, potassium hexamethyldisilazide and the like), C₁₋₆ alkoxides of alkali metals or alkali earth metals (for example, sodium methoxide, sodium ethoxide, potassium tert-butoxide and the like) and other strong bases and the like;
2) Hydroxides of alkali earth metals or alkali earth metals (for example, sodium hydroxide, potassium hydroxide, lithium hydroxide, barium hydroxide and the like), carbonates of alkali metals or alkali earth metals (for example, sodium carbonate, potassium carbonate, cesium carbonate and the like), alkali metal hydrogencarbonates (for example, sodium hydrogencarbonate, potassium hydrogencarbonate and the like) and other inorganic bases and the like; and
3) Organic bases including tertiary amines such as triethylamine, diisopropylethylamine and N-methylmorpholine; strong basic amines such as DBU (1,8-diazabicyclo [5.4.0] undec-7-ene), DBN (1,5-diazabicyclo [4.3.0] non-5-ene) and the like; and basic heterocyclic compounds such as pyridine, dimethylaminopyridine, imidazole, 2,6-lutidine and the like.

Of these, a carbonate of an alkali metal or alkali earth metal, a hydroxide of an alkali metal or alkali earth metal, an alkali metal hydrogencarbonate, a hydride of an alkali metal or alkali earth metal, an amide of an alkali metal or alkali earth metal or a C₁₋₆ alkoxide of an alkali metal is preferred. A carbonate of an alkali metal or a hydroxide of an alkali metal or alkali earth metal is particularly desirable.

The base is used in the amount of normally 2 to 20 or preferably 2 to 8 mole equivalents per 1 mole of Compound (Ia-10).

This reaction can be made more efficient through the use of a catalyst. A phase transfer catalyst (e.g., tetrabutylammonium hydrogensulfate, tetrabutylammonium bromide or benzyl tributylammonium chloride, etc.) or the like can be used as the catalyst.

The catalyst is used in the amount of 0.01 to 2 or preferably 0.05 to 1 mole equivalents per 1 mole of Compound (Ia-10).

Compound (Ia-9) is used in the amount of normally 0.8 to 5 or preferably 1 to 3 mole equivalents per 1 mole of Compound (Ia-10).

The reaction temperature is normally -100 to 200°C or preferably 0 to 100°C. The reaction time is for example 0.1 to 100 hours.

Compound (Ia-3) can be manufactured by acid treating Compound (Ia-11). This reaction is performed using an acid in solvents that do not adversely affect the reaction.

Examples of solvents that do not adversely affect the reaction include alcohols, ethers, halogenated hydrocarbons, amide solvents (e.g., dimethylformamide, etc.), esters, water and the like. A mixture of 2 or more such solvents in suitable proportions can be used. Of these, an alcohol, ether or water is preferred.

Examples of acids include inorganic acids (e.g., hydrochloric acid, sulfuric acid, nitric acid and phosphoric acid, etc.), organic acids (e.g., acetic acid and methanesulfonic acid, etc.) and the like. Of these, hydrochloric acid, sulfuric acid or acetic acid is preferred.

The acid is used in the amount of normally 1 to 20 or preferably 1 to 10 mole equivalents per 1 mole of Compound (Ia-11).

The reaction temperature is normally -50 to 100°C or preferably 0 to 80°C. The reaction time is 0.2 to 24 hours for example or preferably about 1 to 20 hours.

### [Method G]

The Compound (Ib-2) used as a raw material compound in [Method B] above and the Compound (Ib-4) used as a raw material compound in [Method C] above can each be manufactured from Compound (Ib-6) according to the following schemes. 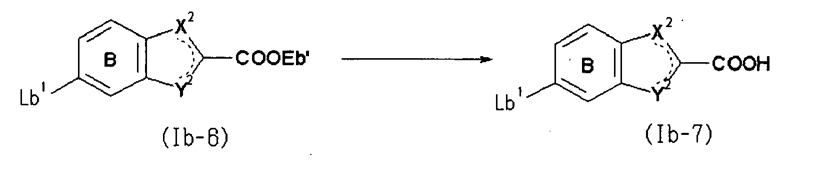 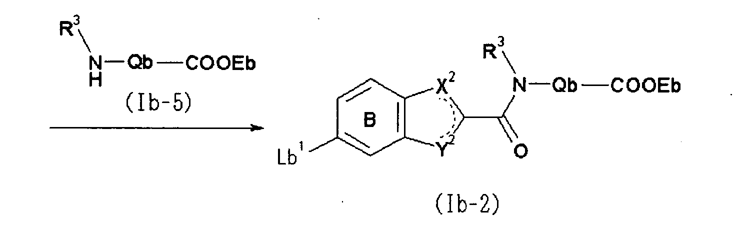 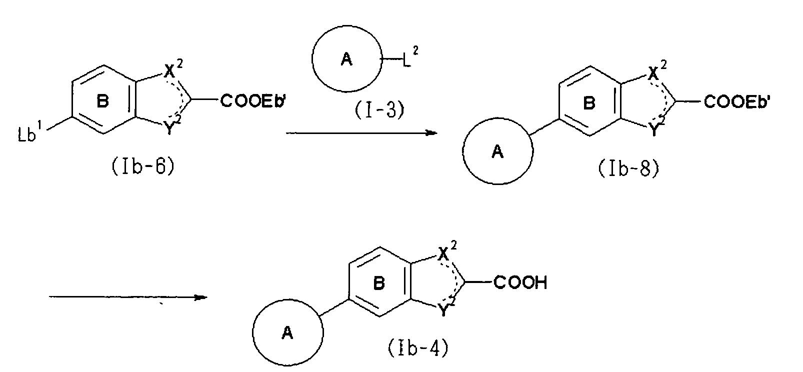 (wherein Eb' is an ester residue and the other symbols are as defined previously).

When Compound (Ib-6) is commercially available the commercial product can be used as is, or the compound can be manufactured according to known methods or their equivalents.

The ester residue represented by Eb' may be similar to the ester residue represented by Ea above.

The reaction that converts Compound (Ib-6) into Compound (Ib-7) and the reaction that converts Compound (Ib-8) into Compound (Ib-4) mean reactions that convert esters into carboxylic acids, and can be performed by methods such as those used in [Method A].

The reaction that converts Compound (Ib-7) into Compound (Ib-2) is an amide bond-forming reaction using a carboxylic acid and an amine compound (Ib-5), and can be performed by methods such as those used in [Method C].

The reaction that converts Compound (Ib-6) into Compound (Ib-8) is a coupling reaction using Compound (Ib-6) and Compound (I-3), and can be performed by methods such as those used in [Method B].

### [Method H]

In Compound (I'-2), the compounds represented by (I'-2a) and (I'-2b) can be manufactured by subjecting Compound (1-4) and Compound (1-5), respectively, to intramolecular cyclization reactions. 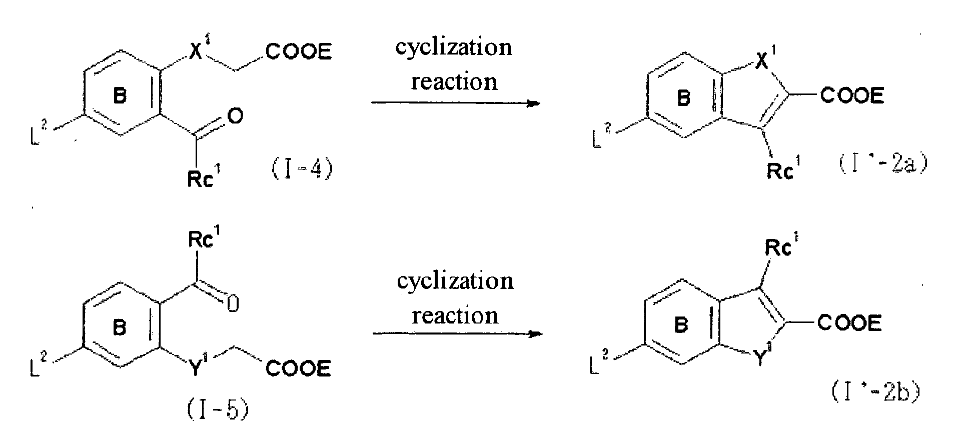 (wherein L² is a leaving group or a functional group capable of a cross-coupling reaction (for example, a trifluoromethanesulfonyloxy-, iodo-, bromo-, chloro- or the like),
Rc¹ is a hydrogen atom or hydrocarbon group,
X¹ and Y¹ are each optionally substituted spacers having 1 to 3 atoms in the main chain (for example, -O-, -S-, -SO-, -SO₂- -NRc²-, -OCH₂-, -SCH₂-, -NRc² CH₂-, -OCH₂CH₂-, - SCH₂CH₂-, -NRc²CH₂CH₂-, methylene, ethylene, trimethylene or the like, with Rc² being a hydrogen atom or substituent), and all other symbols are as defined previously.

Compound (I-4) and Compound (I-5) may be manufactured by known methods or their equivalents.

A halogen atom (e.g., chlorine, bromine or iodine) or trifluoromethylsulfonyloxy can be used as the leaving group represented by L .

The intramolecular cyclization reaction can be performed in the presence of a base if necessary in solvents that do not adversely affect the reaction.

Examples of solvents that do not adversely affect the reaction include hydrocarbons, alcohols, ethers, amides, sulfoxides, water and the like. A mixture of two or more such solvents in suitable proportions can also be used. Of these, an alcohol, ether, hydrocarbon or amide is preferred.

### Examples of bases for use in this reaction include

1) Hydrides of alkali metals or alkali earth metals (for example, lithium hydride, sodium hydride, potassium hydride, calcium hydride and the like), amides of alkali metals or alkali earth metals (for example, lithium amide, sodium amide, lithium diisopropylamide, lithium dicyclohexylamide, lithium hexamethyldisilazide, sodium hexamethyldisilazide, potassium hexamethyldisilazide and the like), C₁₋₆ alkoxides of alkali metals or alkali earth metals (for example, sodium methoxide, sodium ethoxide, potassium tert-butoxide and the like) and other strong bases for example;
2) Hydroxides of alkali earth metals or alkali earth metals (for example, sodium hydroxide, potassium hydroxide, lithium hydroxide, barium hydroxide and the like), carbonates of alkali metals or alkali earth metals (for example, sodium carbonate, potassium carbonate, cesium carbonate and the like), alkali metal hydrogencarbonates (for example, sodium hydrogencarbonate, potassium hydrogencarbonate and the like) and other inorganic bases for example; and
3) Organic bases including tertiary amines such as triethylamine, diisopropylethylamine and N-methylmorpholine; strong basic amines such as DBU (1,8-diazabicyclo [5.4.0] undec-7-ene), DBN (1,5-diazabicyclo [4.3.0] non-5-ene) and the like; and basic heterocyclic compounds such as pyridine, dimethylaminopyridine, imidazole, 2,6-lutidine and the like.

Of these, a C₁₋₆ alkoxide of an alkali metal, a hydride of an alkali earth metal or a strong basic amine or the like is preferred.

The base is used in the amount of normally 0.1 to 10 or preferably 0.1 to 5 mole equivalents per 1 mole of Compound (I-4) and Compound (I-5).

The reaction temperature is normally -100 to 200°C or preferably 0 to 150°C.

The reaction time is 0.1 to 100 hours, etc.

### [Method I]

In Compound (Ia-1), the compounds represented by (Ia-lb) and (Ia - 1c) can be manufactured by reacting Compound (Ia-2) with Compound (I-6) and Compound (I-7), respectively.

In Compound (Ib-1), the compounds represented by (Ib-1b) and (Ib-1c) can be manufactured by reacting Compound (Ib-2) with Compound (I-6) and Compound (1-7), respectively. 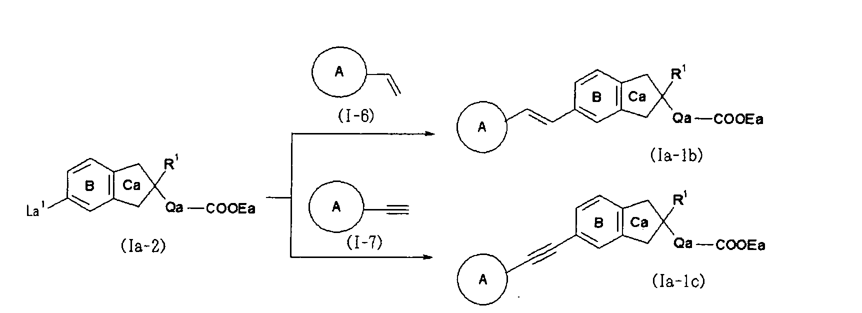 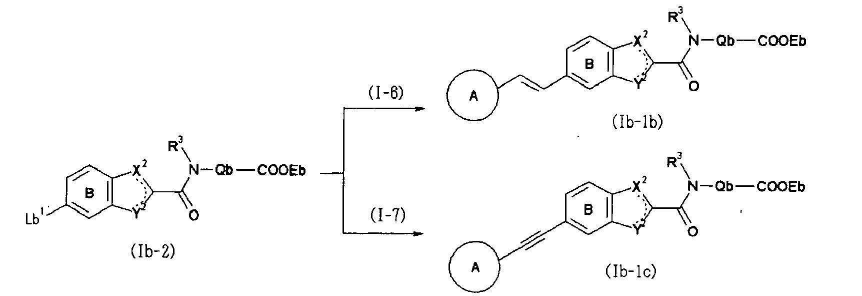 (wherein La and Lb¹ are each leaving groups or functional groups capable of a cross-coupling reaction with Compound (I-6) or (I-7), and all other symbols are as defined above).

When Compound (I-6) and Compound (I-7) are commercially available the commercial products can be used as is, or the compounds can be manufactured by known methods or their equivalents.

As explained above with respect to [Method B], the cross-coupling reaction may be a known coupling reaction such as a Suzuki reaction, Heck reaction, Stille reaction, Sonogashira reaction or the like.

Examples of functional groups capable of cross-coupling reactions that can be used for La¹ or Lb¹ include halogen atom (e.g., chlorine, bromine or iodine), trifluoromethylsulfonyloxy and the like. Bromine, iodine, trifluoromethylsulfonyloxy or the like is particularly desirable.

A halogen atom (e.g., chlorine, bromine or iodine), trifloromethylsulfonyloxy or the like can be used as the leaving group represented by La¹ or Lb¹.

This reaction can be performed as in [Method B].

The base is used in the amount of normally 0.1 to 10 mole equivalents or preferably 0.5 to 2 mole equivalents per 1 mole of Compound (Ia-2) or (Ib-2).

The metal catalyst is used in the amount of normally 0.01 to 1 mole equivalent or preferably 0.03 to 0.1 mole equivalents per 1 mole of Compound (Ia-2) and (Ib-2).

Compounds (1-6) and (1-7) are used in the amount of normally 0.5 to 10 mole equivalents or preferably 1 to 2 mole equivalents per 1 mole of Compound (Ia-2) or (Ib-2).

A microwave reactor can be used for this reaction. The reaction temperature in this case is normally 20 to 200°C or preferably 100 to 150°C, and the reaction time is normally 1 minute to 4 hours or preferably 1 minute to 30 minutes.

### [Method J]

In Compound (Ia-1), the compound represented as Compound (Ia-1d) can be for example manufactured by reacting Compound (Ia-2) and Compound (I-8).

In Compound (Ib-1), the Compound represented as Compound (Ib-1d) can be manufactured by reacting Compound (Ib-2) and Compound (I-8). 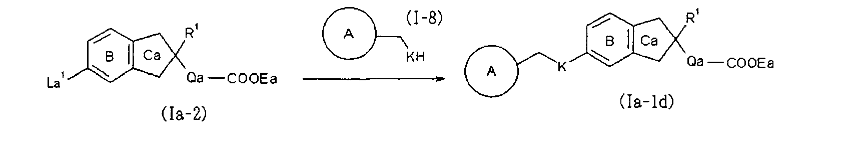 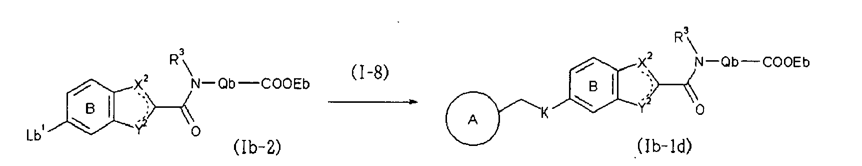 (wherein K is an oxygen atom or sulfur atom and the other symbols are as defined above).

When Compound (I-8) is commercially available the commercial product can be used as is, or the compound can be manufactured by known methods or their equivalents.

The reaction between Compound (Ia-2) or Compound (Ib-2) and Compound (I-8) can be performed using a base in solvents that do not adversely affect the reaction.

Examples of solvents that do not adversely affect the reaction include hydrocarbons, alcohols, ethers, halogenated hydrocarbons, nitriles, amides, ketones, sulfoxides, esters, water and the like. A mixture of two or more such solvents in suitable proportions can also be used. Of these, a nitrile, alcohol, ether, hydrocarbon or amide is preferred, and a nitrile, hydrocarbon or amide is especially preferred.

### Examples of bases for use in this reaction include:

1) Hydrides of alkali metals or alkali earth metals (for example, lithium hydride, sodium hydride, potassium hydride, calcium hydride and the like), amides of alkali metals or alkali earth metals (for example, lithium amide, sodium amide, lithium diisopropylamide, lithium dicyclohexylamide, lithium hexamethyldisilazide, sodium hexamethyldisilazide, potassium hexamethyldisilazide and the like), C₁₋₆ alkoxides of alkali metals or alkali earth metals (for example, sodium methoxide, sodium ethoxide, potassium tert-butoxide and the like) and other strong bases for example;
2) Hydroxides of alkali earth metals or alkali earth metals (for example, sodium hydroxide, potassium hydroxide, lithium hydroxide, barium hydroxide and the like), carbonates of alkali metals or alkali earth metals (for example, sodium carbonate, potassium carbonate, cesium carbonate and the like), alkali metal hydrogencarbonates (for example, sodium hydrogencarbonate, potassium hydrogencarbonate and the like) and other inorganic bases for example; and
3) Organic bases including tertiary amines such as triethylamine, diisopropylethylamine and N-methylmorpholine; strong basic amines such as DBU (1,8-diazabicyclo [5.4.0] undec-7-ene), DBN (1,5-diazabicyclo [4.3.0] non-5-ene) and the like; and basic heterocyclic compounds such as pyridine, dimethylaminopyridine, imidazole, 2,6-lutidine and the like.

Of these, a carbonate of an alkali metal or alkali earth metal, a hydroxide of an alkali metal or alkali earth metal, an alkali metal hydrogencarbonate, a hydride of an alkali metal or alkali earth metal, an amide or an alkali metal or alkali earth metal, or a C₁₋₆ alkoxide of an alkali metal is preferred, and a carbonate of an alkali metal or a hydroxide of an alkali metal or alkali earth metal is especially preferred.

The base is used in the amount of normally 2 to 20 mole equivalents or preferably 2 to 8 mole equivalents per 1 mole of Compound (Ia-2) or (Ib-2).

Compound (I-8) is used in the amount of normally 0.8 to 5 mole equivalents or preferably 1 to 3 mole equivalents per 1 mole of Compound (Ia-2) or (Ib-2).

The reaction temperature is normally -50 to 150°C or preferably 0 to 100°C.

The reaction time is for example 0.1 to 100 hours.

### [Method K]

The Compounds (Id-1) and (Ie-1) used as raw material compounds in [Method A] above can be manufactured for example by hydrolyzing Compounds (Id-2) and (Ie-2) and condensing the resulting compounds, respectively, with Compound (Id-4). 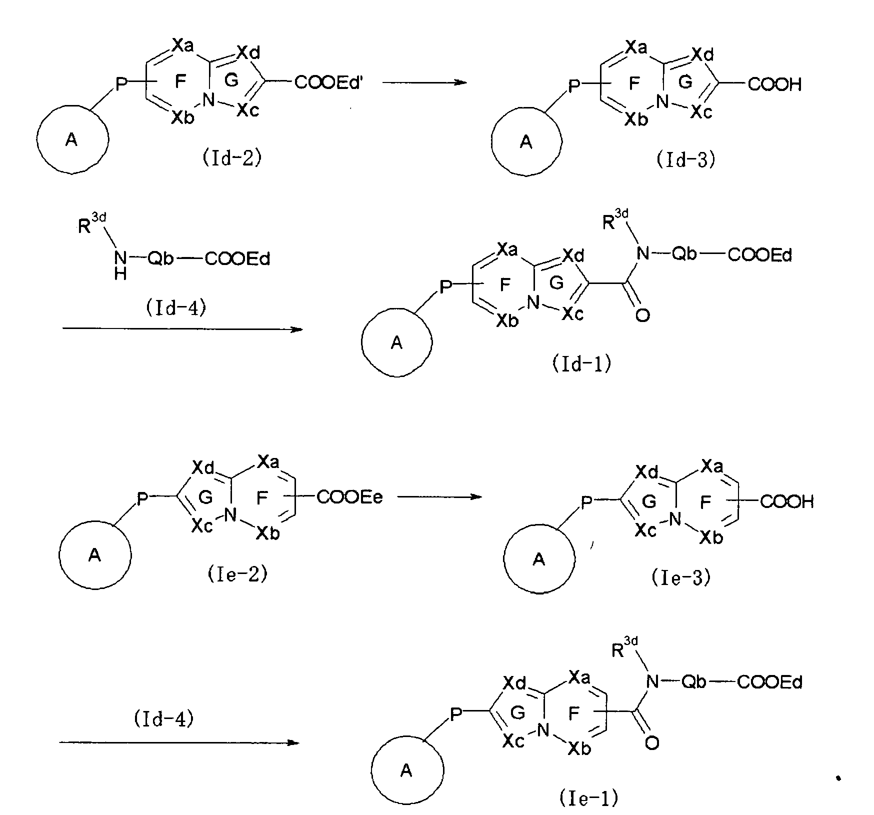 (wherein Ed' and Ee are ester residues and the other symbols are as defined previously).

When Compound (Id-4) is commercially available the commercial product can be used as is, or the compound can be manufactured by known methods or their equivalents.

The ester residue represented by Ed' or Ee may be similar to the ester residues given for Ea above.

The hydrolysis reaction is performed by methods similar to those given for [Method A] above.

The condensation reaction between Compound (Id-3) or Compound (Ie-3) and Compound (Id-4) is normally performed by methods similar to those given in [Method C] above using a condensing agent and a base.

### [Method L]

In Compound (Id-2), the compounds represented as Compound (Id-2a), Compound (Id-2b) and Compound (Id-2c) can be manufactured for example by reacting Compound (Id-5) with Compounds (I-3), (I-6) and (Ib-7), respectively. 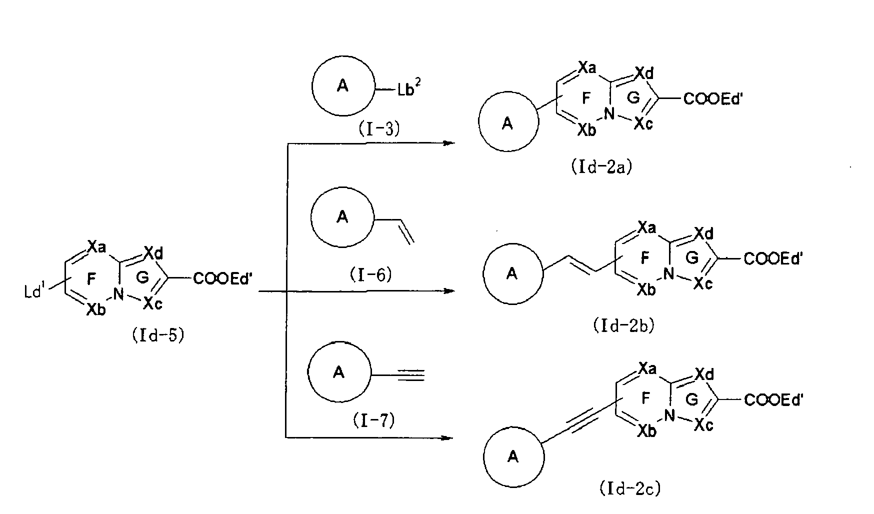 (wherein Ld¹ is a leaving group or a functional group capable of cross-coupling reactions with Compounds (I-3), (I-6) and (I-7) and Lb² is a functional group capable of a cross-coupling reaction with Compound (Id-5), or when ring A is a nitrogen-containing heterocyclic ring and Lb² is on a nitrogen atom Lb² is a hydrogen atom, and all other symbols are as defined previously].

This reaction is performed as in [Method B] and [Method I] above.

### [Method M]

In Compound (Id-2), the Compound represented as Compound (Id-2d) can be manufactured for example by reacting Compound (Id-5) with Compound (I-10). 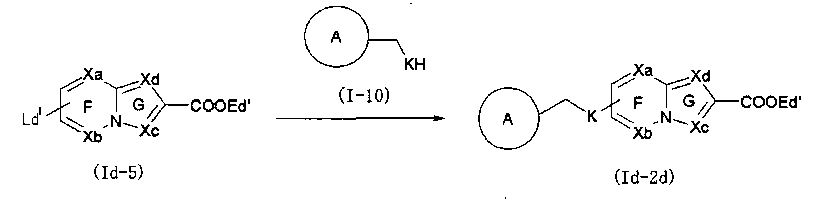 (wherein all symbols are as defined previously).

This reaction is performed as in [Method F] above.

### [Method N]

In Compound (Id-2), the Compound represented as Compound (Id-2f) can be manufactured for example by subjecting Compound (Id-2e) (Compound (Id-2d) in which K is a sulfur atom) to an oxidation reaction. 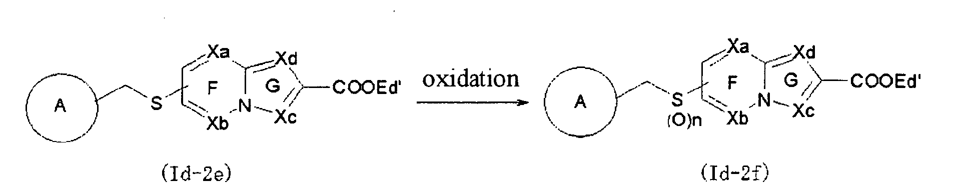 (wherein n is 1 or 2 and all other symbols are as defined previously).

This reaction is a sulfur atom oxidation reaction, and is normally performed using an oxidizing agent in solvents that do not adversely affect the reaction.

Examples of solvents that do not adversely affect the reaction include hydrocarbons, esters, ethers, nitriles, halogenated hydrocarbons, acetic acid, water and the like. A mixture of two or more such solvents in suitable proportions can also be used. Of these, a halogenated hydrocarbon or water is preferred.

An organic peracid (e.g., peracetic acid, m-chloroperbenzoic acid or the like), inorganic peroxide salt (e.g., sodium metaperiodate , potassium permanganate or the like), oxone, hydrogen peroxide solution or the like can be used for example as the oxidizing agent. Sodium metaperiodate is a gentle oxidizing agent which is used when manufacturing a sulfoxide.

The oxidizing agent is used in the amount of normally 0.5 to 5 mole equivalents or preferably 0.8 to 3 mole equivalents per 1 mole of Compound (Id-2e).

The reaction temperature is normally -50 to 100°C or preferably -20 to 80°C.

The reaction time is normally 0.5 to 48 hours or preferably 1 to 10 hours.

### [Method O]

In Compound (Id-2), the compound represented as Compound (Id-2g) can be manufactured for example by subjecting Compound (Id-2b) to a reduction reaction. 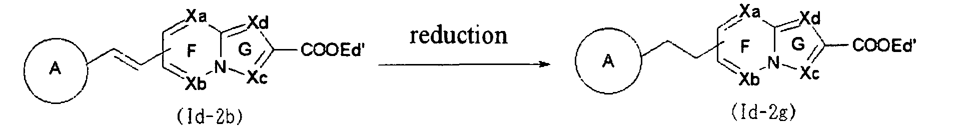 (wherein all symbols are as defined previously).

This reaction is a carbon-carbon double bond reduction reaction, and is normally performed using a reducing agent in the presence of a catalyst in solvents that do not adversely affect the reaction.

Examples of solvents that do not adversely affect the reaction include hydrocarbons, alcohols, ethers, esters, acetic acid, water and the like. A mixture of two or more such solvents in suitable proportions can also be used. Of these, an alcohol or ester or acetic acid is preferred.

The catalyst may be for example a palladium (palladium carbon, palladium black or the like), rhodium (rhodium carbon, rhodium aluminum or the like), ruthenium (ruthenium carbon or the like), platinum (platinum oxide or the like), or nickel (Raney nickel or the like) catalyst or the like.

The reducing agent is used in the amount of normally 0.01 to 1 mole equivalents or preferably 0.01 to 0.5 mole equivalents per 1 mole of Compound (Id-2b).

Hydrogen gas, hydrazine, 1,4-cyclohexadiene or the like can be used as the reducing agent.

The reducing agent is used in the amount of normally 0.5 to 10 mole equivalents or preferably 0.8 to 5 mole equivalents per 1 mole of Compound (Id-2b). When hydrogen gas is used as the reducing agent, the reaction is performed in a hydrogen atmosphere.

The reaction temperature is normally -20 to 100°C or preferably 0 to 80°C.

The reaction time is normally 0.5 to 48 hours or preferably 1 to 10 hours.

### [Method P]

In Compound (Ie-2), the compound represented as Compound (Ie-2a) can be manufactured for example by reacting Compound (Ie-4) and Compound (I-11). 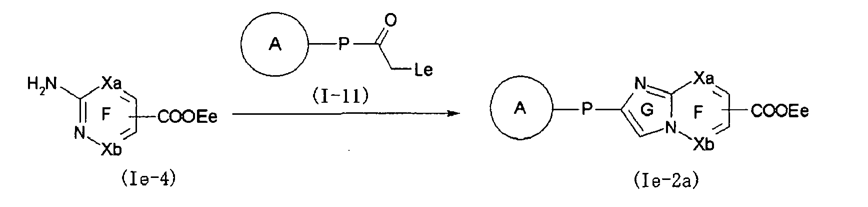 (wherein Le is a leaving group and the other symbols are as defined previously).

Compound (Ie-4) and Compound (I-11) can be manufactured by known methods or their equivalents.

A halogen atom (chlorine, bromine or iodine, etc.) or the like can be used as the leaving group represented by Le.

This reaction is a condensed imidazole forming reaction, and is normally performed in solvents that do not adversely affect the reaction.

Examples of solvents that do not adversely affect the reaction include hydrocarbons, alcohols, ethers, amides, halogenated hydrocarbons and the like. A mixture of two or more such solvents in suitable proportions can also be used. An alcohol is preferred.

Compound (I-11) is used in the amount of 0.5 to 3 mole equivalents or preferably 0.8 to 1.5 mole equivalents per 1 mole of Compound (Ie-4).

The reaction temperature is normally 50 to 200°C or preferably 60 to 100°C.

The reaction time is normally 0.5 to 48 hours or preferably 1 to 10 hours.

### [Method Q]

In Compound (Id-2), the compound represented as Compound (Id-2h) can be manufactured for example by reacting Compound (Id-6) and Compound (Id-7).

In Compound (Id-5), the compound represented as Compound (Id-5a) can be manufactured for example by reacting Compounds (Id-8) and (Id-7). 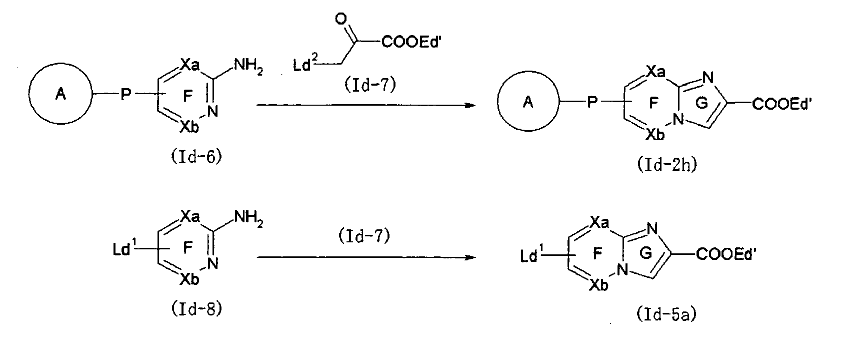 (wherein Ld² is a leaving group and the other symbols are as defined previously).

Compounds (Id-6) and (Id-7) and Compound (Id-8) can be manufactured by known methods or their equivalents.

A halogen atom (e.g., chlorine, bromine or iodine) or the like can be used as the leaving group represented by Ld².

This reaction is performed by methods similar to those of [Method P].

### [Method R]

Compound (Id-2i) can be manufactured for example by reacting Compound (Id-10) and Compound (Id-11). 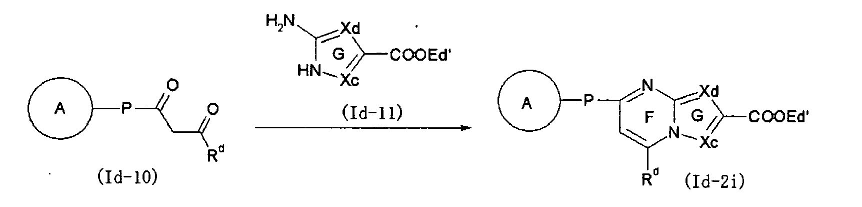 (whrein R^{d} is a hydrogen atom or hydrocarbon group and the other symbols are as defined previously).

Compound (Id-10) and Compound (Id-11) can be manufactured by known methods or their equivalents.

This reaction is a condensed pyrimidine forming reaction, and is normally performed in solvents that do not adversely affect the reaction.

Examples of solvents that do not adversely affect the reaction include hydrocarbons, alcohols, ethers and the like. A mixture of two or more such solvents in suitable proportions can also be used. An alcohol is preferred.

This reaction can be performed in the presence of a base if necessary. This base may be for example a carbonate of an alkali metal or alkali earth metal (e.g., sodium carbonate, potassium carbonate, cesium carbonate or the like), or an alkoxide of an alkali metal or alkali earth metal (e.g., sodium methoxide or sodium tert-butoxide, etc.) or the like.

The base is used in the amount of normally 0.1 to 10 mole equivalents or preferably 0.5 to 2 mole equivalents per 1 mole of Compound (Id-10).

Compound (Id-11) is used in the amount of normally 0.5 to 3 mole equivalents or preferably 0.8 to 1.5 mole equivalents per 1 mole of Compound (Id-10).

The reaction temperature is normally 50 to 200°C or preferably 60 to 100°C.

The reaction time is normally 0.5 to 48 hours or preferably 1 to 10 hours.

Of the Compounds (I), a compound in which W is a group biologically equivalent to a carboxyl can be manufactured by a known reaction method such as the methods described under [Method S], [Method V] and the like below.

### [Method S]

Of the Compounds (I), Compound (I-b) in which W is a 5-tetrazolylaminocarbonyl group can be manufactured for example by subjecting Compound (I-a) to an amidation reaction using 5-aminotetrazole and a condensing agent. 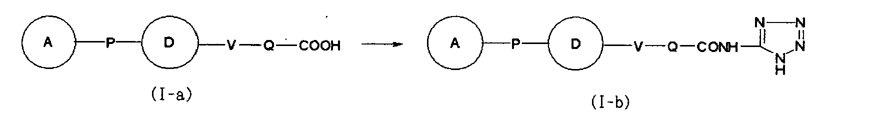 (wherein the symbols are as defined previously).

This reaction is normally performed using a condensing agent in solvents that do not adversely affect the reaction.

Examples of solvents that do not adversely affect the reaction include hydrocarbons, ethers and the like. A mixture of two or more such solvents in suitable proportions can also be used. Of these, an ether is preferred.

Carbonyldiimidazole or the like can be used as the condensing agent in this reaction.

The condensing agent is used in the amount of normally 1 to 3 mole equivalents or preferably 1 to 2 mole equivalents per 1 mole of Compound (I-a).

The reaction temperature is normally 50 to 200°C or preferably 60 to 100°C.

The reaction time is normally 0.5 to 48 hours or preferably 1 to 10 hours.

### [Method T]

Of the Compounds (I), Compound (I-c) in which W is a 5-tetrazolyl group can be manufactured for example by condensing Compound (I-a) with 3-aminopropionitrile to obtain amide Compound (I-12) (first step), then reacting this with trimethylsilyl azide under Mitsunobu reaction conditions to form a tetrazole ring (step 2), and finally removing the cyanoethyl group as a protecting group by alkali hydrolysis (step 3). 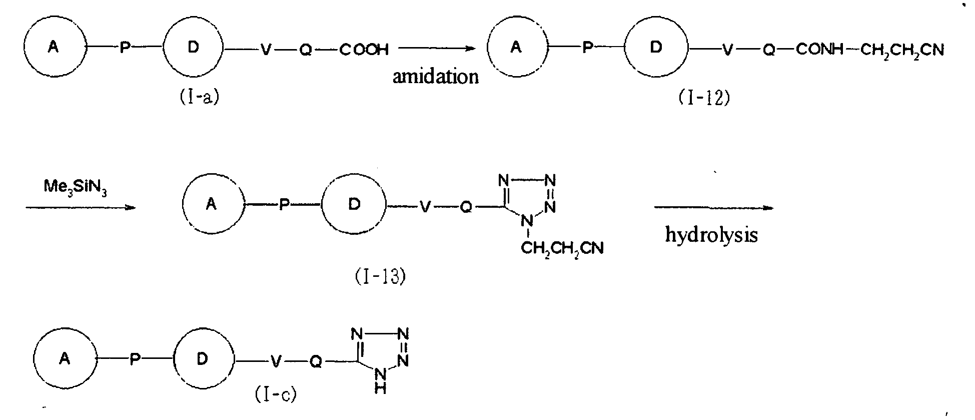 (wherein the symbols are as defined previously).

The amide reaction of the first step is normally performed by methods similar to those used in [Method C] using a condensing agent and a base.

The tetrazole ring forming reaction of the second step is normally performed using an azodicarboxylic acid derivative (e.g., diethyl azodicarboxylate) and a phosphine derivative (e.g., triphenylphosphine or tributylphosphine) in solvents that do not adversely affect the reaction.

Examples of solvents that do not adversely affect the reaction include hydrocarbons, ethers and the like. A mixture of two or more such solvents in suitable proportions can also be used. Of these, ethers are preferred.

The trimethylsilyl azide in this reaction is used in the amount of normally 1 to 3 mole equivalents or preferably 1 to 2 mole equivalents per 1 mole of Compound (I-12).

The azodicarboxylic acid derivative and phosphine derivative are both used in the amount of normally 1 to 5 mole equivalents or preferably 1.2 to 2 mole equivalents per 1 mole of Compound (I-12).

The reaction temperature is normally -40 to 100°C or preferably 0 to 60°C.

The reaction time is normally 0.5 to 48 hours or preferably 1 to 10 hours.

The alkali hydrolysis reaction of the third step is performed by methods similar to those of [Method A] above.

### [Method U]

Of the compounds (I), Compound (I-d) in which W is a 5-oxo-1,2,4-oxadiazole-5-yl group can be manufactured for example by first converting Compound (I-a) to an amide (step 1), then converting it to a nitrile by a dehydration reaction (step 2), then reacting it with a hydroxylamine to obtain an amidoxime (step 3), and finally cyclizing with a carbonylation reagent (step 4). 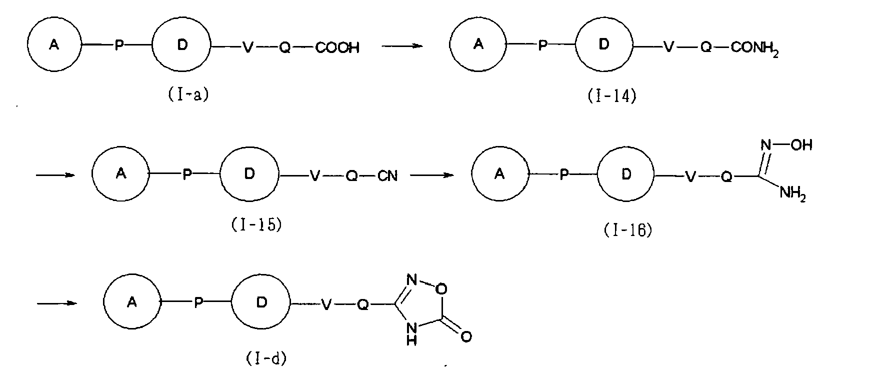 (wherein the symbols are as defined previously).

The reaction of step 1 in which a carboxylic acid is converted into an amide is normally performed by methods similar to those of [Method C] above using a condensing agent and a base.

The dehydration reaction of an amide to a nitrile in step 2 is normally performed in the presence of a base (pyridine, triethylamine or the like) using an acylating agent (benzenesulfonyl chloride, p-toluenesulfonyl chloride or the like) or halogenating agent (thionyl chloride, phosphoryl chloride or the like) in solvents that do not adversely affect the reaction.

Examples of solvents that do not adversely affect the reaction include hydrocarbons, ethers, halogenated hydrocarbons and the like. A mixture of two or more such solvents in suitable proportions can also be used. Of these, ethers are preferred The pyridine used as a base can also be used as a solvent.

The base is used in the amount of normally 1 to 100 mole equivalents or preferably 1 to 10 mole equivalents per 1 mole of Compound (I-14).

The acylating agent and halogenating agent are each used in the amount of normally 1 to 5 mole equivalents or preferably 1.2 to 2 mole equivalents per 1 mole of Compound (I-14).

The reaction temperature is normally -40 to 100°C or preferably 0 to 40°C.

The reaction time is normally 0.1 to 24 hours or preferably 0.3 to 3 hours.

The reaction of step 3 in which a hydroxylamine is added to a nitrile to obtain an amidoxime is normally performed in solvents that do not adversely affect the reaction. When a hydroxylamine hydrochloride is used, a base (for example, a tertiary amine such as pyridine or triethylamine or sodium hydroxide, potassium carbonate or the like) is used.

Examples of solvents that do not adversely affect the reaction include hydrocarbons, ethers, alcohols, water and the like. A mixture of two or more such solvents in suitable proportions can also be used. Of these, an ether or water is preferred.

The base is used in the amount of normally 1 to 2 mole equivalents or preferably 1 to 1.5 mole equivalents per 1 mole of hydroxylamine hydrochloride.

The hydroxylamine is used in the amount of normally 1 to 5 mole equivalents or preferably 1.2 to 2 mole equivalents per 1 mole of Compound (I-15).

The reaction temperature is normally -20 to 100°C or preferably 0 to 80°C. The reaction time is normally 0.1 to 48 hours or preferably 0.3 to 24 hours.

The reaction of step 4 in which the amidoxime is converted to 5-oxo-1,2,4-oxadiazole with a carbonylating agent is normally performed in the presence of a base in solvents that do not adversely affect the reaction.

Examples of solvents that do not adversely affect the reaction include hydrocarbons, halogenated hydrocarbons, ethers and the like. A mixture of two or more such solvents in suitable proportions can also be used. Of these, ethers are preferred.

A tertiary amine (e.g., pyridine, triethylamine or the like) for example can be used as the base.

The base is used in the amount of normally 2 to 10 mole equivalents or preferably 2 to 3 mole equivalents per 1 mole of Compound (I-16).

Carbonyldiimidazole, diethyl carbonate, triphosgene, 4-nitrophenyl chlorocarbonate or the like can be used as the carbonylating agent.

The carbonylating agent is used in the amount of normally 1 to 5 mole equivalents or preferably 1.2 to 2 mole equivalents per 1 mole of Compound (I-16).

The reaction temperature is normally -20 to 100°C or preferably 0 to 80°C.

The reaction time is normally 0.1 to 48 hours or preferably 0.3 to 24 hours.

### [Method V]

Of the Compounds (I), Compound (I-e) in which W is a sulfonylcarbamoyl group can be manufactured for example by subjecting Compound (Ia) and Compound (I-17) to a condensation reaction. 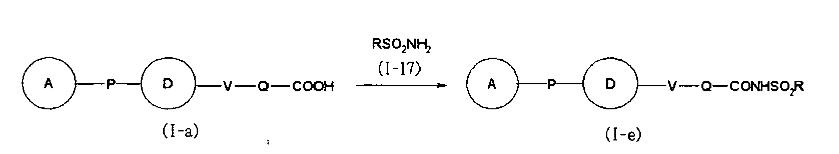 (wherein R is a hydrocarbon group or heterocyclic group and the other symbols are as defined previously).

When Compound (I-17) is commercially available the commercial product may be used as is, or the compound may be manufactured by known methods or their equivalents.

This reaction is normally performed using a condensing agent in solvents that do not adversely affect the reaction. A base may also be used if necessary.

Examples of solvents that do not adversely affect the reaction include hydrocarbons, ethers, amides and the like. A mixture of two or more such solvents in suitable proportions can also be used. Of these, ethers are preferred.

Carbonyldiimidazole or the like can be used as the condensing agent.

The condensing agent is used in the amount of normally 1 to 3 mole equivalents or preferably 1 to 2 mole equivalents per 1 mole of Compound (I-a).

A tertiary amine such as pyridine, triethylamine, 1,4-diazabicyclo[2,2,2]octane, 1,8-diazabicyclo[5.4.0]-7-undecene or the like is used as the base.

The base is used in the amount of 2 to 10 mole equivalents or preferably 2 to 3 mole equivalents per 1 mole of Compound (I-a).

Compound (I-17) is used in the amount of 0.5 to 3 mole equivalents or preferably 0.7 to 1.5 mole equivalents per 1 mole of Compound (I-a).

The reaction temperature is normally -20 to 100°C or preferably 0 to 50°C.

The reaction time is normally 0.5 to 48 hours or preferably 1 to 24 hours.

### [Method W]

The Compound (Ic-1) used as a raw material compound in [Method A] above can be manufactured for example by subjecting Compound (Ic-2) and Compound (Ic-3) to a condensation reaction. 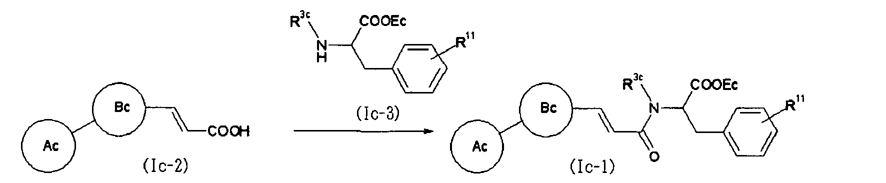 (wherein the symbols are as defined previously).

This reaction is normally performed by methods similar to those of [Method C] using a condensing agent and a base.

Compound (Ic-3) can be manufactured by known methods or their equivalents.

### [Method X]

The Compound (Ic-2) used as a raw material compound in [Method W] above can be manufactured for example by manufacturing Compound (Ic-5) from Compound (Ic-4) (first step) and then hydrolyzing the ester (second step). 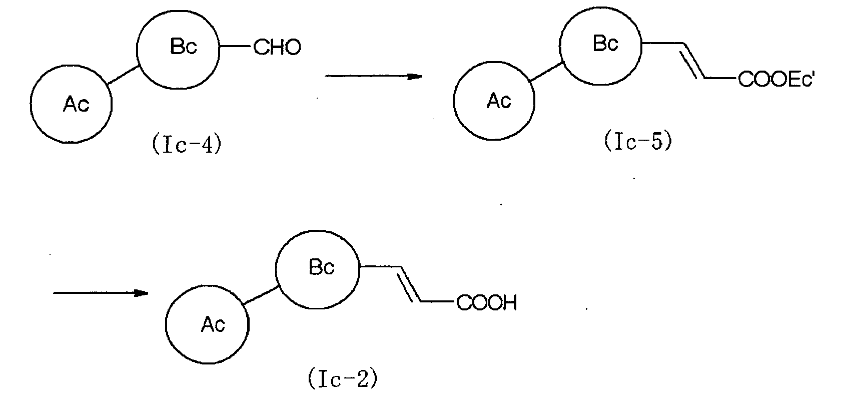 (wherein Ec' is an ester residue and the other symbols are as defined previously).

Compound (Ic-4) can be manufactured by known methods or their equivalents.

An ester residue such as those given for Ea above can be used as the ester residue represented by Ec'.

The first step is a reaction that converts an aldehyde into an acrylic acid ester, and a Wittig reaction, aldol condensation reaction or the like can be used for example.

When using a Wittig reaction, for example Compound (Ic-4) and a phosphonic acid diester (for example, dimethyl methoxycarbonylmethylphosphonate, diethyl ethoxycarbonylmethylphosphonate or the like) or phospholane (for example, (carbomethoxymethylene)triphenylphospholane or the like) are reacted in solvents that do not affect the reaction. When using a phosphonic acid diester, a base (e.g., sodium hydride, sodium amide, sodium methoxide, tert-butoxy potassium or the like) is used to generate anions in the reaction.

Examples of solvents that do not adversely affect the reaction include hydrocarbons, ethers, amides and the like. A mixture of two or more of these in suitable proportions can also be used. Of these, hydrocarbons or ethers are preferred.

The phosphonic acid diester or phospholane is used in the amount of normally 0.8 to 3 mole equivalents or preferably 1 to 1.5 mole equivalents per 1 mole of Compound (Ic-4).

The base is used in the amount of 1 to 3 mole equivalents or preferably 1 to 1.5 mole equivalents per 1 mole of Compound (Ic-4).

The reaction temperature when using a phosphonic acid diester is normally -20 to 50°C or preferably 0 to 40°C. When using a phospholane, it is normally 50 to 150°C or preferably 70 to 120°C.

The reaction time is normally 0.5 to 48 hours or preferably 1 to 24 hours.

When an aldol condensation reaction is used it can be performed by methods similar to those of [Method H] above.

The ester hydrolysis reaction of the second step can be performed by methods such as those of [Method A] above.

### [Method Y]

When substituents in Compounds (I), (I'), (Ia), (Ib), (Ic), (Id) and (Ie) include convertible functional groups (e.g., carboxyl, amino, hydroxyl, carbonyl, thiol, ester, sulfo, halogen atom and the like), these functional groups can be converted by various known methods or their equivalents to manufacture a variety of compounds.

For example, a carboxyl can be converted by a reaction such as esterification, reduction, amidation or conversion to an optionally protected amino group or the like.

An amino can be converted by a reaction such as amidation, sulfonylation, nitrosofication, alkylation, arylation, imidation or the like.

A hydroxyl can be converted by a reaction such as esterification, carbamoylation, sulfonylation, alkylation, arylation, oxidation, halogenation or the like.

A carbonyl can be converted by a reaction such as reduction, oxidation, imination (including oximation and hydrazonation), (thio)ketalation, alkylidenation, thiocarbonylation or the like.

A thiol can be converted by a reaction such as alkylation, oxidation or the like.

An ester can be converted by a reaction such as reduction, hydrolysis or the like.

A sulfo can be converted by a reaction such as sulfonamidation, reduction or the like.

A halogen atom can be converted by various nucleophilic substitutional reactions, coupling reactions and the like.

When a compound is obtained in free form by any of the aforementioned reactions of the present invention, it can be converted to a salt by ordinary methods, or if it is obtained as a salt, it can be converted to free form or to another salt by ordinary methods.

When a raw material compound in any of the various reactions used to manufacture Compounds (I), (I'), (Ia), (Ib), (Ic), (Id) and (Ie) above and the various reactions used to synthesize raw material compounds for these reactions has an amino, carboxyl or hydroxyl as a substituent, these groups may have introduced protective groups commonly used in peptide chemistry and the like, and after each reaction the protective groups may be removed if necessary to obtain the target compound.

The protective group of an amino may be a formyl; or a C₁₋₆ alkylcarbonyl (acetyl, ethylcarbonyl or the like), phenylcarbonyl, C₁₋₆ alkyl-oxycarbonyl (methoxycarbonyl, ethoxycarbonyl, tert-butoxycarbonyl (Boc) or the like), allyloxycarbonyl (Aloc), phenyloxycarbonyl, fluorenylmethyloxycarbonyl (Fmoc), C₇₋₁₀ aralkyl-carbonyl (benzylcarbonyl or the like), C₇₋₁₀ aralkyl-oxycarbonyl (benzyloxycarbonyl (Z) or the like), C₇₋₁₀ aralkyl (benzyl or the like), trityl, phthaloyl or N,N-dimethylaminomethylene or the like, any of which may have a substituent. A phenyl, halogen atom (for example, fluorine, chlorine, bromine or iodine, etc.), C₁₋₆ alkyl-carbonyl (methylcarbonyl, ethylcarbonyl or butylcarbonyl, etc.), nitro or the like may be used as a substituent in this case, and the number of substituents is 1 to about 3.

The protective group of a carboxyl may be a C₁₋₆ alkyl (methyl, ethyl, n-propyl, isopropyl, n-butyl or tert-butyl, etc.), allyl, benzyl, phenyl, trityl or trialkylilyl group or the like, any of which may have a substituent. A halogen atom (for example, fluorine, chlorine, bromine or iodine, etc.), formyl, C₁₋₆ alkyl-carbonyl (acetyl, ethylcarbonyl, butylcarbonyl or the like), nitro or the like may be used as a substituent in this case, and the number of substituents is 1 to about 3.

The protective group of a hydroxyl may be a C₁₋₆ alkyl (methyl, ethyl, n-propyl, isopropyl, n-butyl or tert-butyl, etc.), C₇₋₁₀ aralkyl (benzyl or the like), formyl, C₁₋₆ alkyl-carbonyl (acetyl, ethylcarbonyl or the like), benzoyl, C₇₋₁₀ aralkyl-carbonyl (benzylcarbonyl or the like), tetrahydropyranyl, furanyl or silyl group or the like, any of which may have a substituent. A halogen atom (for example, fluorine, chlorine, bromine or iodine, etc.), C₁₋₆ alkyl (methyl, ethyl, n-propyl or the like), phenyl, C₇₋₁₀ aralkyl (benzyl or the like), C₁₋₆ alkoxy (methoxy, ethoxy, n-propoxy or the like), nitro or the like may be used as a substituent in this case, and the number of substituents is 1 to about 4.

A known method or its equivalent can be used to remove a protective group, and for example a method of acid, base, reduction, ultraviolet, hydrazine, phenylhydrazine, sodium N-methyldithiocarbamate, tetrabutylammonium fluoride or palladium acetate treatment or the like can be used.

When Compound (I) is in the form of a configuration isomer, diastereomer, conformer and the like, these can be isolated as desired by the isolation and purification means described above.

When Compound (I) has stereoisomers, either a single isomer or a mixture of isomers is included in the present invention.

Compound (I) has the action of changing binding between GPR34 and a ligand preferably GPR34 antagonist activity, mast cell degranulation-inhibiting action, histamine release-inhibiting action, leukotriene production-inhibiting action, prostaglandin production-inhibiting action, IL-13 production-inhibiting action, tryptase secretion-inhibiting action, antigen-antibody reaction-inhibiting action and the like, and has low toxicity and few side-effects. Consequently, Compound (I) is useful as a safe medicament, such as for example a GPR34 receptor antagonist (including inverse agonist or partial agonist), mast cell degranulation ihibitor, histamine release ihibitor, eicosanoid production ihibitor, mast cell proliferation ihibitor, IL-13 production ihibitor, tryptase secretion ihibitor or antigen-antibody reaction ihibitor; or a preventive/therapeutic agent for immune disease [for example, inflammatory disease (e.g., pituitary abscess, thyroiditis, peritonitis, Crohn's disease, ulcerative colitis, erythema nodosum, chronic rheumatoid arthritis, systemic lupus erythematosus and the like), allergies (e.g., allergic conjunctivitis, allergic rhinitis, hay fever, metal allergies and the like), asthma, exudative otitis media, Ménière's disease, contact dermatitis, anaphylaxis, hives, myasthenia gravis, glomerulonephritis, Sjögren's syndrome, Basedow's disease, insulin resistant diabetes, atopic dermatitis, leukocyte abnormalities and the like], respiratory disease [for example, chronic obstructive pulmonary disease (e.g., chronic bronchitis or pulmonary edema), diffuse panbronchiolitis, cystic fibrosis, hypersensitity pneumonitis, idiopathic interstitial pneumonia, pulmonary fibrosis and the like], urinary tract disease (e.g., renal tubulointerstitial disease (fibrosis), interstitial cystitis, allergic cystitis and the like), cardiovascular disease (e.g., arteriosclerosis, acute coronary syndrome, atherosclerotic aortic aneurysm, cardiac anaphylaxis, heart failure, myocardial infarction, angina, arrhythmia, deep phlebothrombosis, post-PTCA restenosis and the like), ophthalmological disease (e.g., pterygium, vernal conjunctivitis, dry eye and the like), cancer (e.g., papillary thyroid carcinoma, non-small cell lung cancer, endometrial cancer, cervical cancer, stomach cancer, pancreatic cancer, lung cancer, kidney cancer, liver cancer, ovarian cancer, prostate cancer, bladder cancer, breast cancer, colon cancer, rectal cancer, Kaposi's sarcoma, mastocytoma and the like), digestive disease [including chronic liver disease, food allergies, allergic enteritis, milk protein-induced proctitis, digestive ulcers (e.g., stomach ulcer, duodenal ulcer, stomal ulcer, Zollinger-Ellison syndrome and the like), gastritis, reflux esophagitis, NUD (non-ulcer dyspepsia), gastric MALT lymphoma, ulcers caused by non-steroidal anti-inflammatory drugs, hyperacidity, ulcers and hyperacidity caused by post-surgical stress and the like], cerebral infarction, hyperlipidemia, acute renal failure, diabetes, obesity, edema, granuloma, atopic myelitis, neurofibroma, nasal mucosal hypersensitivity, Hodgkin's disease, endometrial hyperplasia, central nervous system disease [for example, neurodegenerative disease (e.g., Alzheimer's disease (familial Alzheimer's disease, early-onset Alzheimer's disease, sporadic Alzheimer's disease, etc.), Parkinson's disease, Down's syndrome, amyotrophic lateral sclerosis, prion disease (Creutzfeldt-Jakob disease), Huntington's chorea, diabetic neuropathy, multiple sclerosis and the like), psychological disease (e.g., schizophrenia, depression, bipolar disorder, anxiety disorder, attention deficit hyperactivity disorder, panic disorder and the like), and cerebrovascular disease (e.g., cerebral thrombosis, cerebral infarction, transient ischemic attack, etc.) and the like] and others.

When Compound (I) is used as these agents, it can be administered orally or parenterally by known methods, and normally it is mixed with pharmacologically acceptable carriers and administered orally as a solid preparation such as tablets, capsules, granules, powder or the like or parenterally as an intravenous, subcutaneous, intramuscular or other injection or suppository, sublingual tablet or the like. It can also be administered sublingually, subcutaneously, intramuscularly or the like as a sustained-release preparation such as sublingual tablets, microcapsules or the like.

The dosage of Compound (I) is not particularly limited and differs depending on the subject, administration route, symptoms and the like, but in the case of oral administration to an adult patient for treatment of allergies, a single dose is normally about 0.01 to 20 mg/kg body weight or preferably about 0.1 to 10 mg/kg body weight or more preferably about 0.1 to 2 mg/kg body weight, and these amounts are preferably administered about 1 to 3 times a day depending on symptoms.

The amount of Compound (I) contained in the aforementioned "agent (drug composition)" is about 0.01 to 100 wt% of the drug composition as a whole.

Various organic or inorganic carrier substances commonly used as pharmaceutical materials can be used as the aforementioned pharmacologically acceptable carriers, and are compounded as excipients, lubricants, binders and disintegrators in solid preparations and as solvents, solubilizers, suspending agents, isotonic agents, buffers, soothing agents and the like in liquid preparations. Preservatives, anti-oxidants, colorants, sweeteners and other pharmaceutical additives can also be used if necessary.

Desirable examples of the aforementioned excipients include lactose, sucrose, D-mannitol, starch, crystal cellulose, liquid anhydrous silicic acid and the like. Desirable examples of the aforementioned lubricants include magnesium stearate, calcium stearate, talc, colloidal silica and the like. Desirable examples of the aforementioned binders include crystal cellulose, sucrose, D-mannitol, dextrin, hydroxypropyl cellulose, hydroxypropyl methyl cellulose, polyvinyl pyrrolidine and the like. Desirable examples of the aforementioned disintegrators include starch, carboxymethyl cellulose, carboxymethyl cellulose calcium, croscarmellose sodium, carboxymethyl starch sodium and the like. Desirable examples of the aforementioned solvents include injectable water, alcohols, propylene glycol, macrogol, sesame seed oil, corn oil and the like. Desirable examples of the aforementioned solubilizers include polyethylene glycol, propylene glycol, D-mannitol, benzyl benzoate, ethanol, tris-aminomethane, cholesterol, triethanolamine, sodium carbonate, sodium citrate and the like. Desirable examples of the aforementioned suspending agents include stearyl triethanolamine, sodium lauryl sulfate, lauryl aminopriopionic acid, lecithin, benzalkonium chloride, benzethonium chloride, glycerin monostearate and other surfactants; and polyvinyl alcohol, polyvinyl pyrrolidine, carboxymethyl cellulose sodium, methyl cellulose, hydroxymethyl cellulose, hydroxyethyl cellulose, hydroxypropyl cellulose and other hydrophilic polymers and the like. Desirable examples of the aforementioned isotonic agents include sodium chloride, glycerin, D-mannitol and the like. Desirable examples of the aforementioned buffers include buffer solutions of phosphoric acid salts, acetic acid salts, carboxylic acid salts, citric acid salts and the like. Desirable examples of the aforementioned soothing agents include benzyl alcohol and the like. Desirable examples of the aforementioned preservatives include paraoxybenzoic acid esters, chlorobutanol, benzyl alcohol, phenethyl alcohol, dehydroacetic acid, sorbic acid and the like. Desirable examples of the aforementioned anti-oxidants include sulfites, ascorbic acid and the like.

Compound (I) can be made into an intravenous, subcutaneous or intramuscular injection by ordinary means after addition of suspending agents, solubilizers, stabilizers, isotonic agents, preservatives and the like. In this case it can also be made into a freeze-dried product by known methods if necessary.

When administered to humans for example, Compound (I) can safely be administered either orally or parenterally as a drug composition, either as is or after being mixed with suitable pharmacologically acceptable carriers, excipients and diluents.

Examples of the aforementioned drug composition include oral preparations (for example, powders, granules, capsules and tablets), injections, drops, external preparations (nasal preparations, transdermal preparations and the like), suppositories (for example rectal suppositories and vaginal suppositories) and the like.

These preparations can be manufactured by known methods commonly used in drug formulation.

Compound (I) can be made into an aqueous injection together with a dispersing agent (e.g., Tween 80 (Atlas Powder Co., U.S.), HCO60 (Nikko Chemicals), polyethylene glycol, carboxymethyl cellulose or sodium alginate, etc.), preservative (e.g., methyl paraben, propyl paraben or benzyl alcohol, etc.) and isotonic agent (e.g., sodium chloride, mannitol, sorbitol or glucose, etc.) and the like, or dissolved, suspended or emulsified in olive oil, sesame seed oil, cotton seed oil, corn oil or other vegetable oil or propylene glycol or the like to form an oil-based injection, and injected.

To obtain an orally administered preparation, Compound (I) can be pressure molded by known methods after addition of an excipient (e.g., lactose, sucrose or starch, etc.), disintegrator (e.g., starch or calcium carbonate, etc.), binder (e.g., starch, gum arabic, carboxymethyl cellulose, polyvinyl pyrrolidine or hydroxypropyl cellulose, etc.) or lubricant (e.g., talc, magnesium stearate or polyethylene glycol 6000, etc.) or the like, and can then be coated by known methods as necessary to mask the flavor or impart enteric or sustained-release properties to thereby obtain an orally administered preparation. Hydroxypropyl methyl cellulose, ethyl cellulose, hydroxymethyl cellulose, hydroxypropyl cellulose, polyoxyethylene glycol, Tween 80, Pluronic F68, cellulose acetate phthalate, hydroxypropyl methyl cellulose phthalate, hydroxymethyl cellulose acetate succinate, Eudragit (Rohm Co., Germany, methacrylic acid-acrylic acid copolymer) and dyes (e.g., iron oxide and titanium dioxide, etc.) and the like can be used as coating materials. In the case of an enteric preparation, an intermediate phase may be provided by known methods between the enteric phase and drug-containing phase in order to separate the two.

To obtain an external preparation, Compound (I) or a salt thereof can be made into a solid, semi-solid or liquid externally administered form by ordinary methods. For the solid form, Compound (I) or its salt can be used either as is or as a powder composition after addition and mixing of an excipient (e.g., glycol, mannitol, starch or crystal cellulose, etc.), viscosity improver (e.g., a natural gum, cellulose derivative or acrylic acid polymer, etc.) or the like. For the liquid form, it can be made into an oily or water-based suspension in roughly the same way as the injection described above. For the semi-solid form, it can be made into a water-based or oily gel or an ointment. In all these cases, a pH adjuster (e.g., carbonic acid, phosphoric acid, citric acid, hydrochloric acid or sodium hydroxide, etc.) or a preservative (e.g., a paraoxybenzoic acid ester, chlorobutanol or benzalkonium chloride, etc.) or the like can be added.

To obtain a suppository for example, Compound (I) can be made into an oily or water-based solid, semi-solid or liquid suppository by known methods. An oily base for use in the composition may consist for example of higher fatty acid glycerides (e.g., cacao butter or a Witepsol (Dynamite Nobel, Germany), etc.), medium fatty acids (e.g., a Miglyol (Dynamite Nobel, Germany), etc.) or a vegetable oil (e.g., sesame seed oil, soy bean oil or cottonseed oil, etc.) or the like. Examples of aqueous bases include polyethylene glycols and propylene glycol, while examples of aqueous gel bases include natural gums, cellulose derivatives, vinyl polymers, acrylic acid polymers and the like.

Examples of drugs that can be administered together with Compound (I) include the following, and either each drug may be administered orally or parenterally (as a nasal preparation, injection or suppository, etc.) or they may be compounded in a single preparation, and also the drugs can be formulated by mixing them with pharmacologically acceptable carriers, excipients, binders, diluents and the like and administered either separately or simultaneously. When the drugs are formulated separately, the separately formulated drugs can be mixed with a diluent or the like at the time of use and administered together, but individual preparations that have been separately formulated may also be administered to the same subject either simultaneously or with a time interval between administrations.

Examples of such "drugs" include alkylating agents (e.g., cyclophosphamide, busulfan and melphalan, etc.), antimetabolites (e.g., cytarabine, 6-mercaptopurine, methotrexate and 5-fluorouracil, etc.), anti-cancer antibiotics (e.g., daunorubicin, doxorubicin, pirarubicin, mitoxantrone, idarubicin, mitomycin and adriamycin, etc.), plant-derived anti-cancer agents (e.g., vincristine, vindesine, taxol and etoposide, etc.), enzyme drugs (e.g., L-asparaginase and the like), estrogens (e.g., estradiol, ethynyl estradiol, fosfestrol and chlorotrianisene, etc.), antiestrogens (e.g., epitiostanol, mepitiostane, tamoxifen and clomifene, etc.), luteal hormones (e.g., hydroxyprogesterone caproate, dydrogesterone, medroxyprogesterone, norethisterone and norethindrone, etc.), LHRH analogues (e.g., leuprorelin acetate and the like), cisplatin, carboplatin, transretinoic acid, interferon alpha, imatinib, anti-allergy agents [for example, mediator release inhibitors (e.g., sodium cromoglycate, tranilast, amlexanox, pemirolast potassium, tazanolast, ibudilast, repirinast, nedocromil sodium and cromoglycate lisetil hydrochloride, etc.), thromboxane A2 inhibitors (e.g., ozagrel hydrochloride, seratrodast, ramatroban and domitroban calcium, etc.), leukotriene receptor antagonists (e.g., pranlukast, zafirlukast and montelukast, etc.), Th2 cytokine inhibitors (e.g., suplatast tosilate, etc.), antihistamines (e.g., diphenhydramine, pyrilamine, chlorpheniramine, ketotifen fumarate, azelastine hydrochloride, oxatomide, mequitazine, terfenadine, emedastine fumarate, epinastine hydrochloride, ebastine, cetirizine and fexofenadine hydrochloride, etc.) and PAF antagonists (e.g., israpafant, etc.)] and the like, anti-inflammatories [including adrenocortical hormones (e.g., prednisolone, prednisone, dexamethasone, cortisone acetate, hydrocortisone and fludrocortisone acetate, etc.), non-steroidal anti-inflammatory drugs (e.g., aspirin, diflunisal, mefenamic acid, flufenamic acid, diclofenac sodium, alclofenac, fenbufen, amfenac sodium, indomethacin, sulindac, acemetacin, tolmethyl sodium, etodolac, ibuprofen, pranoprofen, naproxen, ketoprofen, tiaprofenic acid, sodium loxoprofen, oxaprozin, alminoprofen, zaltoprofen, flurbiprofen, phenylbutazone, oxyphenbutazone, piroxicam, tenoxicam, ampiroxicam, meloxicam, mepirizole, tiaramide, tinoridine and emorfazone, etc.), anti-inflammatory enzymes (e.g., serratiopeptidase, pronase, bromelain, trypsin and lysozyme hydrochloride, etc.) and the like], immunosuppressive drugs (e.g., cyclosporine, tacrolimus, cyclophosphamide, azathioprine, mizoribine and methotrexate, etc.), anti-arteriosclerotics [including statin hyperlipidemia treatment drugs (e.g., atorovastatin, pravastatin, simvastatin, paravastatin and pitavastatin, etc.), fibrate hyperlipidemia treatment drugs (e.g., clofibrate, bezafibrate, simfibrate and clinofibrate, etc.), ACAT inhibitors (e.g., CS-505, CS-747 and F-1394, etc.), ACE inhibitors (e.g., captopril, enalapril maleate, delapril hydrochloride, benazepril hydrochloride, cilazapril, satapril, analapril or lisinopril, etc.), angiotensin II receptor antagonists (e.g., losartan, candesartan, candesartan cilexetil, valsartan, telmesartan and olmesartan, etc.), Ca blockers (e.g., amlodipine, etc.), aldosterone antagonists (e.g., spironolactone, etc.), insulin sensitizers (e.g., pioglitazone, rosiglitazone and metformin, etc.), antithrombotic drugs (e.g., warfarin, heparin, low-molecular-weight heparin, argatroban, urokinase, ticlopidine, beraprost and dipyridamole, etc.) and nicotinic acid, etc.] and the like.

The sequence ID numbers given in the sequence tables of the Specifications of this +
- [SEQ ID NO:1]: Human GPR34 amino acid sequence
- [SEQ ID NO:2]: cDNA nucleotide sequence coding for human GPR34
- [SEQ ID NO:3]: Nucleotide sequence of Primer 1 used in PCR reaction in Test Example 3 below
- [SEQ ID NO:4]: Nucleotide sequence of Primer 2 used in PCR reaction in Test Example 3 below
- [SEQ ID NO:5]: Nucleotide sequence of primer used in PCR reaction in Test Example 1 below
- [SEQ ID NO:6]: Nucleotide sequence of primer used in PCR reaction in Test Example 1 below
- [SEQ ID NO:7]: Nucleotide sequence obtained in Test Example 1 below
- [SEQ ID NO:8]: Nucleotide sequence of primer used in PCR reaction in Test Example 1 below
- [SEQ ID NO:9]: Nucleotide sequence obtained in Test Example 1 below
- [SEQ ID NO:10]: Nucleotide sequence of primer used in PCR reaction in Test Example 1 below
- [SEQ ID NO:11]: Nucleotide sequence of primer used in PCR reaction in Test Example 1 below
- [SEQ ID NO:12]: Nucleotide sequence of probe used in PCR reaction in Test Example 1 below

The present invention is explained in more detail below using reference examples, examples, preparation examples and test examples, but the present invention is not limited thereby.

Compound purity was measured under the following HPLC conditions in the reference examples and examples.

| | | |
|---|---|---|
| Equipment: | Shimadzu LC-10Avp system | |
| Column: | CAPCEL PAK C18UG120 S-3, µm, 2.0 x 50 mm | |
| Solvent: | A solution: | Water containing 0.1% trifluoracetic acid |
| | B solution: | Acetonitrile containing 0.1% trifluoracetic acid |
| Gradient cycle: | 0.00 min. (A solution/B solution = 90/10), 4.00 min (A /B = 5/95), 5.50 min. (A /B = 5/95), 5.51 min. (A /B = 90/10), 8.00 min. (A /B = 90/10) | |
| Injection volume: | 2 µl, flow rate: 0.5 ml/min, detection method: UV 220 nm | |

Preparative HPLC purification was performed under the following conditions in the reference examples and examples.

| | | |
|---|---|---|
| Equipment: | Gilson, Inc. High Throughput Purification System | |
| Column: | YMC CombiPrep ODS-A, S-5 µm, 50 x 20 mm | |
| Solvent: | A solution: | Water containing 0.1% trifluoracetic acid |
| | B solution: | Acetonitrile containing 0.1% trifluoracetic acid |
| Gradient cycle: | 0.00 min. (A solution/B solution = 90/10), 1.00 min. (A/B = 90/10), 4.00 min. (A/B = 10/95), 8.50 min. (A/B = 10/95), 8.60 min. (A/B = 90/10), 8.70 min. (A/B = 90/10) | |
| Flow rate: | 20 ml/min, detection method: UV 220 nm | |

The mass spectrum (MS) was measured under the following conditions in the reference examples and examples.
Measurement equipment: Micromass Co. Platform II or Waters Co. ZMD
Ionization method: Atmospheric Pressure Chemical Ionization (APCI) or Electron Spray Ionization (ESI)

The HPLC-mass spectrum (LC-MS) was measured under the following conditions in the reference examples and examples.

| | | |
|---|---|---|
| Measurement Equipment: | | Waters ZMD HP1100 |
| Column: | CAPCELL PAK C18UG120, S-3 µm, 1.5 x 35 mm | |
| Solvent: | A solution: | Water containing 0.05% trifluoracetic acid |
| | B solution: | Acetonitrile containing 0.04% trifluoracetic acid |
| Gradient cycle: | 0.00 min. (A solution/B solution = 90/10), 2.00 min. (A/B = 5/95), 2.75 min. (A/B = 5/95), 2.76 min. (A/B = 90.10), 3.45 min. (A/B = 90/10) | |
| Injection volume: | 2 µl, flow rate: 0.5 ml/min, detection method: UV 220 nm | |
| Ionization method: | Electron Spray Ionization (ESI) | |

The ¹H-NMR spectrum was measured with a Varian Gemini 200 (200 MHz) and a Bruker Avance DPX-300 (300 MHz) using tetramethylsilane as the standard substance, and all δ values were given as ppm.

A Biotage Emrys Optimizer was used as the microwave synthesizer.

Unless otherwise specified, the numerical values given for mixed solvents represent the volume mixing ratios of each solvent. A % represents weight percent unless otherwise specified. Room temperature in the present specification signifies a temperature between about 10°C and about 35°C, but is not strictly limited to this range.

In addition, other symbols using in the document have the following meanings.
- s:: singlet
- d:: doublet
- t:: triplet
- q:: quartet
- m:: multiplet
- br:: broad
- J:: coupling constant
- Hz:: Hertz
- CDC1₃:: Chloroform-d
- DMSO-d₆:: Dimethylsulfoxide-d₆
- CD₃OD:: Methanol-d₄
- ¹H-NMR:: Proton nuclear magnetic resonance
- DMF:: N,N-dimethylformamide
- THF:: Tetrahydrofuran
- TFA:: Trifluoracetic acid
- WSCD:: Water-soluble carbodiimide (1-ethyl-3-(3-dimethylaminopropyl) carbodiimide
- HOBt:: 1-hydroxybenzotriazole
- Boc:: tert-butoxycarbonyl
- H-Gly-OH:: Glycine
- H-Ala-OH:: Alanine
- H-Val-OH:: Valine
- H-Leu-OH:: Leucine
- H-Ile-OH:: Isoleucine
- H-Ser-OH:: Serine
- H-Thr-OH:: Threonine
- H-Phe-OH:: Phenylalanine
- H-Tyr-OH:: Tyrosine
- H-Trp-OH:: Tryptophan
- H-Asp-OH:: Aspartic acid
- H-Asn-OH:: Asparagine
- H-Glu-OH:: Glutamic acid
- H-Gln-OH:: Glutamine
- H-Cys-OH:: Cysteine
- H-Met-OH:: Methionine
- H-Lys-OH:: Lysine
- H-Arg-OH:: Arginine
- H-Pro-OH:: Proline
- H-His-OH:: Histidine
- H-Asp(OMe)-OH:: Aspartic acid β-methyl ester
- H-Glu(OBzl)-OH:: Glutamic acid γ-benzyl ester

### Reference Example 1

### 1-(4-Bromo-2-hydroxyphenyl) ethanone

A dichloroethane solution (250 ml) of 1-bromo-3-methoxybenzene (30 g), acetyl chloride (15.5 g) and aluminum chloride (25.6 g) was refluxed for 4 hours. The reaction mixture was poured into ice water, and the organic layer was separated. The organic layer was washed with water, dried over magnesium sulfate and then concentrated. The residue was subjected to silica gel chromatography, and the title compound was obtained as a colorless oil (14.4 g, yield 42%) from the diethyl ether-hexane (1:10) eluate.
¹H-NMR (200MHz, CDCl₃) δ: 2.61 (3H, s), 7.04 (1H, dd, J=1.9, 8.5Hz), 7.18 (1H, d, J=1.8Hz), 7.58 (1H, d, J=8.4Hz)

### Reference Example 2

### Ethyl (2-acetyl-5-bromophenoxy)acetate

A mixture of 1-(4-bromo-2-hydroxyphenyl)ethanone (14.4 g), potassium carbonate (10.2 g), ethyl bromoacetate (8.2 ml) and N,N-dimethylformamide (100 ml) was stirred for 1 hour at room temperature. Water was added to the reaction mixture, which was then extracted with ethyl acetate. The extract was washed with water, dried over magnesium sulfate and concentrated. The resulting crude crystals were recrystallized from ethanol-water to obtain the title compound as colorless prisms (19.9 g, yield 98%).
Melting point: 103-105°C
¹H-NMR (200MHz, CDCl₃)δ: 1.32 (3H, t, J=7.1Hz), 2.70 (3H, s), 4.30 (2H, q, J=7.2Hz), 7.00 (1H, d, J=1.8Hz), 7.21 (1H, dd, J=1.6, 8.2Hz), 7.66 (1H, d, J=8.4Hz)

### Reference Example 3

### Ethyl 6-bromo-3-methyl-l-benzofuran-2-carboxylate

A toluene solution (200 ml) of (2-acetyl-5-bromophenoxy) ethyl acetate (18.8 g) and 1,8-diazabicyclo[5.4.0]-7-undecene (14.3 g) was refluxed for 2 hours. The reaction mixture was concentrated, diluted with water and extracted with ethyl acetate. The extract was washed with water, dried over magnesium sulfate and concentrated. The residue was subjected to silica gel chromatography, and the crude crystals obtained from the diethyl ether-hexane (1:10) eluate were recrystallized from ethanol-water to obtain the title compound as colorless needles (7.7 g, yield 43%).
Melting point: 55-56°C
¹H-NMR (200MHz, CDCl₃)δ: 1.44 (3H, t, J=7.2Hz), 2.57 (3H, s), 4.46 (2H, q, J=7.2Hz), 7.43 (1H, dd, J=1.6, 8.4Hz), 7.50 (1H, d, J=8.4Hz), 7.72 (1H, d, J=1.4Hz)

### Reference Example 4

### 1-(4-Bromo-2-hydroxyphenyl) butan-1-one

The title compound was obtained as in Reference Example 1 as a colorless oil (yield 47%).
Melting point: 31-32°C
¹H-NMR (200MHz, CDCl₃)δ: 1.02 (3H, t, J=7.2Hz), 1.68-1.87 (2H, m), 2.93(2H, t, J=7.2Hz), 7.03 (1H, dd, J=2.0, 8.6Hz), 7.18 (1H, d, J=2.0Hz), 7.61 (1H, d, J=8.6Hz), 12.5 (1H, s)

### Reference Example 5

### Ethyl (5-bromo-2-butyrylphenoxy)acetate

The title compound was obtained as in Reference Example 2 as colorless prisms (yield 88%).
Melting point: 91-92°C
¹H-NMR (200MHz, CDCl₃)δ: 0.96 (3H, t, J=7.4Hz), 1.32 (3H, t, J=7.2Hz), 1.64-1.80 (2H, m), 3.04 (2H, t, J=7.2Hz), 4.30 (2H, q, J=7.2Hz), 4.70 (2H, s), 6.97 (1H, d, J=1 .6Hz), 7.20 (1H, dd, J=1.6, 8.2Hz), 7.58 (1H, d, J=8.2Hz)

### Reference Example 6

### Ethyl 6-bromo-3-propyl-1-benzofuran-2-carboxylate

The title compound was obtained as in Reference Example 3 as a colorless oil (yield 58%).
¹H-NMR (200MHz, CDCl₃)δ: 0.98 (3H, t, J=7.3Hz), 1.44 (3H, t, J=7.2Hz), 1.63-1.81 (2H, m), 3.04 (2H, t, 7.6Hz), 4.45 (2H, q, J=7.2Hz), 7.42 (1H, dd, J=1.6, 8.4Hz), 7.52 (1H, d, J=7.8Hz), 7.72 (1H, d, J=1.8Hz)

### Reference Example 7

### Ethyl 5-bromo-1-benzothiophen-2-carboxylate

5-Bromo-2-fluorobenzaldehyde (25 g) and potassium carbonate (34 g) were suspended in N,N-dimethylformamide (125 ml), and ethyl thioglycolate (14.2 ml) was added dropwise. After being stirred for 30 minutes at room temperature, this was heated for 2 hours at 80°C. It was then poured into ice-cooled aqueous citric acid solution and extracted with ethyl acetate, the organic layer was washed with water and sodium chloride solution and dried over magnesium sulfate, and the solvent was evaporated. The precipitated crystals were filtered out and washed with hexane to obtain ethyl 5-bromo-1-benzothiophen-2-carboxylate (29.2 g) as colorless crystals.
¹H-NMR (300MHz, CDC1₃) 5 1.42 (3H, t, J = 7.2 Hz), 4.41 (2H, q, J = 7.2 Hz), 7.54 (1H, dd, J = 1.7, 8.7 Hz), 7.73 (1H, d, J = 8.7 Hz), 7.97 (1H, s), 8.01 (1H, d, J = 1.7 Hz)

### Reference Example 8

### Ethyl 2-amino-5-bromoindan-2-carboxylate

1) 4-Bromophthalic acid anhydride (5.0 g) was dissolved in tetrahydrofuran (15 ml), and a 1.9 M borane-methyl sulfide complex-tetrahydrofuran solution (35 ml) was added dropwise with ice cooling and refluxed for 8 hours. Methanol was added dropwise and refluxed for 2 hours, and the solvent was evaporated. Water was then added, and the mixture was extracted with ethyl acetate. The organic layer was washed with aqueous sodium hydrogencarbonate solution, water and sodium chloride solution and then dried over magnesium sulfate, and the solvent was evaporated. The residue was purified by silica gel column chromatography (elution solvent: ethyl acetate:hexane = 1:2) to obtain (4-bromo-1,2-phenylphenylene)dimethanol (3.2 g) as colorless crystals.
   ¹H-NMR (300MHz, CDCl₃) δ 2.65 (1H, br), 2.73 (1H, br), 4.70 (4H, s), 7.24 (1H, d, J = 8.1 Hz), 7.45 (1H, dd, J = 1.9, 8.1 Hz), 7.53 (1H, d, J = 1.9 Hz)
2) (4-Bromo-1,2-phenylphenylene)dimethanol (3.2 g) was suspended in dichloromethane (75 ml), and phosphorus tribromide (4.2 ml) was added dropwise with ice cooling. After 1 hour of agitation at room temperature, this was poured into ice water. The organic layer was washed with water and then dried over magnesium sulfate, and the solvent was evaporated. The residue was purified by silica gel column chromatography (elution solvent: ethyl acetate:hexane) to obtain 4-bromo-1,2-bis(bromomethyl) benzene (2.0 g) as colorless crystals.
   ¹H-NMR (300MHz, CDC1₃) δ 4.58 (2H, s), 4.59 (2H, s), 7.24 (1H, d, J = 8.1 Hz), 7.43 (1H, dd, J = 2.1, 8.1 Hz), 7.52 (1H, d, J = 2.1 Hz)
3) 4-Bromo-1,2-bis(bromomethyl) benzene (2 g) was dissolved in acetonitrile (70 ml), potassium carbonate (4.8 g) and tetrabutylammonium hydrogen sulfate (0.4 g) were added, ethyl isocyanoacetate (0.67 ml) was then added, and the mixture was heated for 21 hours at 80°C. This was filtered, and the solvent was evaporated from the filtrate. The residue was dissolved in diethyl ether, washed with water and sodium chloride solution, and dried over magnesium sulfate. The solvent was evaporated, and the residue was purified by silica gel column chromatography (elution solvent: ethyl acetate:hexane = 1:2) to obtain ethyl 5-bromo-2-isocyanoindan-2-carboxylate (0.87 g) as a pale yellow oil.
   ¹H-NMR (300MHz, CDCl₃) δ 1.36(3H, t, J= 7.1 Hz), 3.43(2H, dd, J= 10.0, 16.4 Hz), 3.61-3.72(2H, m), 4.33(2H, q, J= 7.1 Hz), 7.12(1H, d, J= 8.1 Hz), 7.37-7.40 (2H, m)
4) Ethyl 5-bromo-2-isocyanoindan-2-carboxylate (0.87 g) was dissolved in ethanol (15 ml), and hydrochloric acid (0.3 ml) was added and stirred overnight at room temperature. The solvent was evaporated, followed by extraction with water. The aqueous layer was washed with diethyl ether, an aqueous sodium hydrogencarbonate solution was added, and the mixture was extracted with ethyl acetate. The organic layer was washed with water and sodium chloride solution and dried over magnesium sulfate, and the solvent was evaporated to obtain ethyl 2-amino-5-bromoindan-2-carboxylate (0.75 g) as a pale yellow oil.
   ¹H-NMR (300MHz, CDCl₃) δ 1.29 (3H, t, J= 7.1 Hz), 2.84 (2H, dd, J= 10.7, 16.2 Hz), 3.45-3.57(2H, m), 4.23 (2H, q, J= 7.1 Hz), 7.08 (1H, d, J= 8.1 Hz), 7.31 (1H, d, J= 8.1 Hz), 7.35 (1H, s)

### Reference Example 9

### Ethyl 2-amino-5-(4-methylphenyl) indan-2-carboxylate

Ethyl 2-amino-5-bromoindan-2-carboxylate (1.0 g), 4-methylphenylboronic acid (0.72 g), tetrakis triphenylphosphine palladium (0.16 g, 4%), 2 M aqueous sodium carbonate solution (3.5 ml) and ethanol (3.5 ml) were added to 1,2-dimethoxyethane (10 ml). The mixture was heated for 4 minutes at 145°C using a microwave synthesizer in an argon atmosphere. This was then poured into water and extracted with ethyl acetate. The organic layer was washed with water and sodium chloride solution and dried over magnesium sulfate, and the solvent was evaporated. The residue was purified by silica gel column chromatography (elution solvent: ethyl acetate:hexane = 1:2) to obtain ethyl 2-amino-5-(4-methylphenyl) indan-2-carboxylate (0.62 g) as colorless crystals.
¹H-NMR (300MHz, CDCl₃) δ 1.30 (3H, t, J= 7.2 Hz), 2.39 (3H, s), 2.92 (2H, dd, J= 6.8, 16.0 Hz), 3.60 (2H, dd, J= 5.4, 16.0 Hz), 4.24 (2H, q, J= 7.2 Hz), 7.23 (2H, d, J= 8.0 Hz), 7.26-7.28 (1H, m), 7.39-7.42 (2H, m), 7.46 (2H, d, J= 8.0 Hz)

### Reference Example 10

### Ethyl 2-amino-5-(4-chlorophenyl) indan-2- carboxylate

Ethyl 2-amino-5-bromoindan-2-carboxylate (1.0 g), 4-chlorophenylboronic acid (0.66 g), tetrakis triphenylphosphine palladium (0.16 g), 2 M aqueous sodium carbonate solution (3.5 ml) and ethanol (3.5 ml) were added to 1,2-dimethoxyethane (10 ml). The mixture was heated for 4 minutes at 145°C using a microwave synthesizer in an argon atmosphere. It was then concentrated, water was added, and the mixture was extracted with ethyl acetate. The organic layer was washed with water and sodium chloride solution and dried over magnesium sulfate, and the solvent was evaporated. The residue was purified by silica gel column chromatography (elution solvent: ethyl acetate:hexane = 1:2) to obtain ethyl 2-amino-5-(4-chlorophenyl) indan-2-carboxylate
(0.62 g) as colorless crystals.
¹H-NMR (300MHz, CDCl₃) δ 1.31 (3H, t, J= 7.1 Hz), 2.92 (2H, dd, J= 6.2, 16.0 Hz), 3.60 (2H, dd, J= 4.8, 16.0 Hz), 4.24 (2H, q, J= 7.2 Hz), 7.28 (1H, d, J= 7.9 Hz), 7.34-7.41 (4H, m), 7.48 (2H, d, J=8.7Hz)

### Reference Example 11

### Ethyl 2-amino-5-(4-fluorophenyl) indan-2-carboxylate

Ethyl 2-amino-5-bromoindan-2-carboxylate (1.0 g), 4-fluorophenylboronic acid (0.59 g), tetrakis triphenylphosphine palladium (0.16 g), 2 M aqueous sodium carbonate solution (3.5 ml) and ethanol (3.5 ml) were added to 1,2-dimethoxyethane (10 ml). The mixture was heated for 4 minutes at 145°C using a microwave synthesizer in an argon atmosphere. It was then concentrated, water was added, and the mixture was extracted with ethyl acetate. The organic layer was washed with water and sodium chloride solution and dried over magnesium sulfate, and the solvent was evaporated. The residue was purified by silica gel column chromatography (elution solvent: ethyl acetate:hexane = 1:2) to obtain ethyl 2-amino-5-(4-fluorophenyl) indan-2-carboxylate (0.73 g) as a colorless oil.
¹H-NMR (300MHz, CDCl₃) δ 1.31 (3H, t, J= 7.1 Hz), 2.92 (2H, dd, J= 6.0, 16.1 Hz), 3.60 (2H, dd, J= 5.0, 16.0 Hz), 4.24 (2H, q, J= 7.1 Hz), 7.05-7.15 (2H, m), 7.27-7.31 (1H, m), 7.33-7.40 (2H, m), 7.46-7.55 (2H, m)

### Reference Example 12

### Ethyl 2-amino-5-(4-dimethylaminophenyl) indan-2- carboxylate

Ethyl 2-amino-5-bromoindan-2-carboxylate (1.0 g), 4-dimethylaminophenylboronic acid (0.7 g), tetrakis triphenylphosphine palladium (0.16 g), 2 M aqueous sodium carbonate solution (3.5 ml) and ethanol (3.5 ml) were added to 1,2-dimethoxyethane (10 ml). The mixture was heated for 4 minutes at 145°C using a microwave synthesizer in an argon atmosphere. It was then concentrated, water was added, and the mixture was extracted with ethyl acetate. The organic layer was washed with water and sodium chloride solution and dried over magnesium sulfate, and the solvent was evaporated. The residue was purified by silica gel column chromatography (elution solvent: ethyl acetate:hexane = 1:2) to obtain ethyl 2-amino-5-(4-dimethylaminophenyl) indan-2-carboxylate (0.45 g) as a pale brown oil.
¹H-NMR (300MHz, CDCl₃) δ 1.30 (3H, t, J= 7.2 Hz), 2.90 (2H, dd, J= 7.8, 16.1 Hz), 2.99 (6H, s), 3.60 (2H, dd, J= 7.0, 16.0 Hz), 4.24 (2H, q, J= 7.2 Hz), 6.79 (2H, d, J= 8.9 Hz), 7.24 (1H, d, J= 7.9 Hz), 7.36-7.40 (2H, m), 7.46 (2H, d, J= 8.9 Hz)

### Reference Example 13

### Ethyl 5-[3-(acetylamino)phenyl]-2-aminoindan-2-carboxylate

Ethyl 2-amino-5-bromoindan-2-carboxylate (1.0 g), 3-acetamidophenylboronic acid (0.76 g), tetrakis triphenylphosphine palladium (0.16 g), 2 M aqueous sodium carbonate solution (3.5 ml) and ethanol (3.5 ml) were added to 1,2-dimethoxyethane (10 ml). The mixture was heated for 4 minutes at 145°C using a microwave synthesizer in an argon atmosphere. It was then concentrated, water was added, and the mixture was extracted with ethyl acetate. The organic layer was washed with water and sodium chloride solution and dried over magnesium sulfate, and the solvent was evaporated. The residue was purified by silica gel column chromatography (elution solvent: ethyl acetate:hexane = 1:2) to obtain ethyl 5-[3-(acetylamino)phenyl]-2-aminoindan-2-carboxylate (0.56 g) as a colorless amorphous substance.
¹H-NMR (300MHz, CDCl₃) δ 1.31 (3H, t, J= 7.1 Hz), 2.20 (3H, s), 2.92 (2H, dd, J= 4.8, 16.1 Hz), 3.60 (2H, dd, J= 2.4, 16.0 Hz), 4.24 (2H, q, J= 7.1 Hz), 7.24-7.46 (7H, m), 7.70 (1H, s)

### Reference Example 14

### Ethyl 2-amino-5-[4-(trifluoromethoxy)-phenyl] indan-2-carboxylate

Ethyl 2-amino-5-bromoindan-2-carboxylate (1.0 g), 4-trifluoromethoxyphenylboric acid (0.7 g), tetrakis triphenylphosphine palladium (0.16 g), 2 M aqueous sodium carbonate solution (3.5 ml) and ethanol (3.5 ml) were added to 1,2-dimethoxyethane (10 ml). The mixture was heated for 4 minutes at 145°C using a microwave synthesizer in an argon atmosphere. It was then concentrated, water was added, and the mixture was extracted with ethyl acetate. The organic layer was washed with water and sodium chloride solution and dried over magnesium sulfate, and the solvent was evaporated. The residue was purified by silica gel column chromatography (elution solvent: ethyl acetate:hexane = 1:2) to obtain ethyl 2-amino-5-[4-(trifluoromethoxy)-phenyl] indan-2-carboxylate (0.83 g) as a colorless oil.
¹H-NMR (300MHz, CDCl₃) δ 1.31 (3H, t, J= 7.2 Hz), 2.93 (2H, dd, J= 6.0, 16.1 Hz), 3.61 (2H, dd, J= 4.4, 16.1 Hz), 4.25 (2H, q, J= 7.2 Hz), 7.21-7.33 (3H, m), 7.33-7.42 (2H, m), 7.55 (1H, d, J= 8.7 Hz)

### Reference Example 15

### Ethyl 2-amino-5-(3-chloro-4-fluorophenyl)-indan-2-carboxylate

Ethyl 2-amino-5-bromoindan-2-carboxylate (1.0 g), 3-chloro-4-fluorophenylboronic acid (0.74 g), tetrakis triphenylphosphine palladium (0.16 g), 2 M aqueous sodium carbonate solution (3.5 ml) and ethanol (3.5 ml) were added to 1,2-dimethoxyethane (10 ml). The mixture was heated for 4 minutes at 145°C using a microwave synthesizer in an argon atmosphere. It was then concentrated, water was added, and the mixture was extracted with ethyl acetate. The organic layer was washed with water and sodium chloride solution and dried over magnesium sulfate, and the solvent was evaporated. The residue was purified by silica gel column chromatography (elution solvent: ethyl acetate:hexane = 1:2) to obtain ethyl 2-amino-5-(3-chloro-4-fluorophenyl)-indan-2-carboxylate (0.87 g) as a colorless oil.
¹H-NMR (300MHz, CDC1₃) δ 1.31 (3H, t, J= 7.2 Hz), 2.92 (2H, dd, J= 5.1, 16.2 Hz), 3.60 (2H, dd, J= 3.8, 16.2 Hz), 4.25 (2H, q, J= 7.2 Hz), 7.12-7.23 (1H, m), 7.34-7.44 (4H, m), 7.57 (1H, dd, J= 2.3, 7.2 Hz)

### Reference Example 16

### Ethyl 2-amino-5-(3-thienyl) indan-2-carboxylate

Ethyl 2-amino-5-bromoindan-2-carboxylate (1.0 g), 3-thiopheneboronic acid (0.54 g), tetrakis triphenylphosphine palladium (0.16 g), 2 M aqueous sodium carbonate solution (3.5 ml) and ethanol (3.5 ml) were added to 1,2-dimethoxyethane (10 ml). The mixture was heated for 4 minutes at 145°C using a microwave synthesizer in an argon atmosphere. It was then concentrated, water was added, and the mixture was extracted with ethyl acetate. The organic layer was washed with water and sodium chloride solution and dried over magnesium sulfate, and the solvent was evaporated. The residue was purified by silica gel column chromatography (elution solvent: ethyl acetate:hexane = 1:2) to obtain ethyl 2-amino-5-(3-thienyl) indan-2-carboxylate (0.65 g) as a colorless oil.
¹H-NMR (300MHz, CDCl₃) δ 1.30 (3H, t, J= 7.1 Hz), 2.90 (2H, dd, J= 7.0, 16.0 Hz), 3.59 (2H, dd, J= 6.8, 16.0 Hz), 4.24 (2H, q, J= 7.1 Hz), 7.24 (1H, d, J= 9.4 Hz), 7.31-7.47 (5H, m)

The structural formulae for the compounds of Reference Example 3 and Reference Examples 6-16 are given in Table 1.

**(Table 1)**

| | | | |
|---|---|---|---|
| Reference Example 3 | 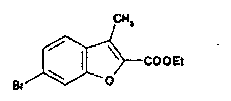 | Reference Example 11 | 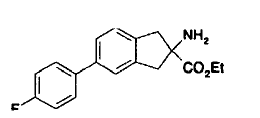 |
| Reference Example 6 | 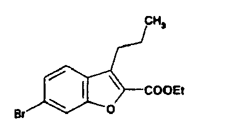 | Reference Example 12 | 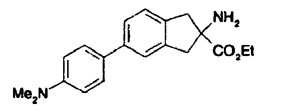 |
| Reference Example 7 | 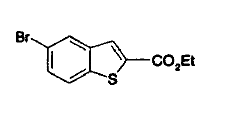 | Reference Example 13 | 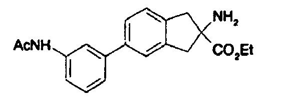 |
| Reference Example 8 | 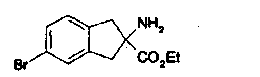 | Reference Example 14 | 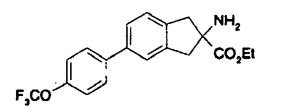 |
| Reference Example 9 | 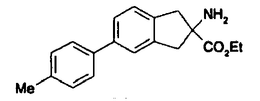 | Reference Example 15 | 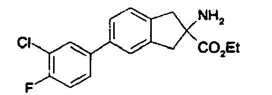 |
| Reference Example 10 | 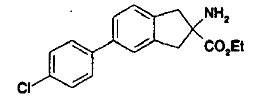 | Reference Example 16 | 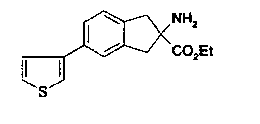 |

### Reference Example 17

### Ethyl 3 -amino-6-(4-chlorophenyl)- 1 -benzofuran-2-carboxylate

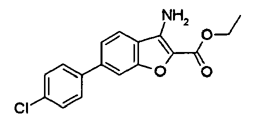

1) Sodium hydride (4.8 g) was added with ice cooling to a N,N-dimethylformamide solution (150 mL) of 4-bromo-2-fluorobenzonitrile (10.0 g) and ethyl glycolate (12.49 g), stirred for 30 minutes, and then stirred for 30 minutes at room temperature. Water was added to the reaction solution, which was then extracted with diethyl ether. The extract was washed successively with water and saturated sodium chloride solution, and then dried over anhydrous magnesium sulfate. The solvent was evaporated under reduced pressure, and the residue was purified by silica gel column chromatography and recrystallized from the ethyl acetate-hexane to obtain ethyl 3-amino-6-bromo-1-benzofuran-2-carboxylate (3.72 g) as pale yellow crystals.
   ¹H-NMR (300MHz, CDCl₃); δ 1.44 (3H, t, J=7.2Hz), 4.44 (2H, q, J=7.2Hz), 4.95 (2H, br s), 7.37 (1H, dd, J=1.5, 8.4Hz), 7.42 (1H, dd, J=0.6, 8.4Hz), 7.63 (1H, dd, J=0.6, 1.5Hz)
2) A mixture of the compound obtained in 1) above (2.84 g), 4-chlorophenylboronic acid (1.88 g), tetrakis(triphenylphosphine)palladium (0) (578 mg), 2 M aqueous sodium carbonate solution (10 mL) and 1,2-dimethoxyethane (50 mL) was stirred for 16 hours at 80°C. After the reaction water was added and the mixture was extracted with ethyl acetate. The extract was washed with saturated sodium chloride solution and dried over anhydrous magnesium sulfate. The solvent was evaporated under reduced pressure, and the precipitated crystals were filtered and washed with diethyl ether-hexane to obtain ethyl 3-amino-6-(4-chlorophenyl)-1-benzofuran-2-carboxylate (2.40 g) as pale yellow crystals.
   ¹H-NMR (300MHz, CDCl₃); δ 1.45 (3H, t, J=7.2Hz), 4.47 (2H, q, J=7.2Hz), 4.99 (2H, br s), 7.40-7.49 (3H, m), 7.50-7.65 (4H, m)

### Reference Example 18

### tert-Butyl 3-amino-5-(4-chlorophenyl)-1-benzofuran-2-carboxylate

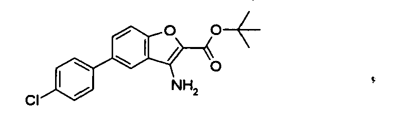

1) A mixture of 5-bromo-2-hydroxybenzonitrile (9.90 g), tert-butyl bromoacetate (11.70 g), potassium carbonate (8.29 g) and N,N-dimethylformamide (100 mL) was stirred for 16 hours at room temperature. Water was added to the reaction solution, which was then extracted with diethyl ether. The extract was washed with saturated sodium chloride solution, and then dried over anhydrous magnesium sulfate. The solvent was evaporated under reduced pressure, and the residue was purified by silica gel column chromatography to obtain tert-butyl (4-bromo-2-cyanophenoxy) acetate (16.31 g) as a colorless oil.
   ¹H-NMR (300MHz, CDCl₃); δ 1.47 (9H, s), 4.65 (2H, s), 6.72 (1H, d, J=9.0Hz), 7.60 (1H, dd, J=2.4, 9.0Hz), 7.69 (1H, d, J=2.4Hz)
2) Sodium hydride (2.51 g) was added with ice cooling to a N,N-dimethylformamide solution (100 mL) of the compound obtained in 1) above (16.31 g), and stirred for 1 hour at room temperature. Water was added to the reaction solution, which was then extracted with ethyl acetate. The extract was washed with saturated sodium chloride solution and dried over anhydrous magnesium sulfate. The solvent was evaporated under reduced pressure, and the residue was purified by silica gel column chromatography and recrystallized from ethyl acetate-hexane to obtain tert-butyl 3-amino-5-bromo-1-bonzofuran-2-carboxylate (6.14 g) as pale yellow crystals.
   ¹H-NMR (300MHz, CDCl₃), δ 1.64 (9H, s), 4.81 (2H, br s), 7.32 (1H, d, J=9.0Hz), 7.51 (1H, dd, J=1.8, 9.0Hz), 7.66 (1H, d, J=1.8Hz)
3) A mixture of the compound obtained in 2) above (3.12 g), 4-chlorophenylboronic acid (1.88 g), tetrakis(triphenylphosphine)palladium (0) (578 mg), 2 M aqueous sodium carbonate solution (10 mL) and 1,2-dimethoxyethane (60 mL) was stirred for 15 hours at 80°C. Water was added and the mixture was extracted with ethyl acetate. The extract was washed with saturated sodium chloride solution and dried over anhydrous magnesium sulfate. The solvent was evaporated under reduced pressure and the residue was purified by silica gel column chromatography (NH) and then recrystallized from ethyl acetate-hexane to obtain the title compound (1.80 g) as pale yellow crystals.
   ¹H-NMR (300MHz, CDCl₃); δ 1.66 (9H, s), 4.90 (2H, br s), 7.42 (2H, d, J=8.4Hz), 7.49 (1H, dd, J=0.6, 8.7Hz), 7.53 (2H, d, J=8.4Hz), 7.61 (1H, dd, J=1.8, 8.7Hz), 7.65 (1H, dd, J=0.6, 1.8Hz)

### Reference Example 19

### Methyl O-benzyltyrosinate hydrochloride

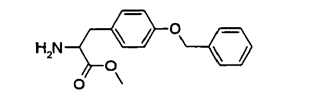

1) A mixture of tyrosine (DL form, 10.19 g), 10% hydrochloric acid-methanol solution (50 mL) and methanol (50 mL) was stirred for 24 hours at 60°C. The reaction solution was concentrated under reduced pressure. The residue was dissolved in methanol (50 mL), and triethylamine (15.6 mL) and tert-butyl dicarbonate (14.73 g) were added and stirred for 2.5 hours at room temperature. The reaction solution was concentrated under reduced pressure and water was added to the residue, which was then extracted with ethyl acetate. The extract was washed successively with 0.1 N hydrochloric acid and saturated sodium chloride solution, and dried over anhydrous magnesium sulfate. The residue was then recrystallized from ethyl acetate-hexane to obtain methyl N-(tert-butoxycarbonyl) tyrosinate (14.62 g) as colorless crystals.
   ¹H-NMR (300MHz, CDCl₃); δ 1.42 (9H, s), 2.90-3.10 (2H, m), 3.71 (3H, s), 4.25-5.30 (3H, m), 6.74 (2H, d, J=8.4Hz), 6.98 (2H, d, J=8.4Hz)
2) A mixture of the compound obtained in 1) above (5.91 g), benzyl bromide (2.85 mL), potassium carbonate (3.32 g) and N,N-dimethylformamide (50 mL) was stirred for 16 hours at room temperature. Water was added to the reaction solution, which was then extracted with diethyl ether. The extract was washed with saturated sodium chloride solution and then dried over anhydrous magnesium sulfate. The solvent was evaporated under reduced pressure, and the residue was purified by silica gel column chromatography to obtain methyl N-(tert-butoxycarbonyl)-O-benzyltyrosinate (7.28 g) as colorless crystals.
   ¹H-NMR (300MHz, CDCl₃); δ 1.42 (9H, s), 2.95-3.10 (2H, m), 3.71 (3H, s), 4.20-5.00 (2H, m), 5.04 (2H, s), 6.90 (2H, d, J=8.7Hz), 7.04 (2H, d, J=8.7Hz), 7.28-7.45 (5H, m)
3) A solution of the compound obtained in 2) above (7.28 g), 10% hydrochloric acid-methanol solution (50 mL) and methanol (80 mL) was stirred for 20 hours at room temperature, and then stirred for 5 hours at 60°C. The reaction solution was concentrated under reduced pressure, and the residue was recrystallized from methanol-diethyl ether to obtain the title compound (5.46) as colorless crystals.
   ¹H-NMR (300MHz, DMSO-d₆);δ 3.04 (1H, dd, J=7.2, 14.1Hz), 3.12 (1H, dd, J=6.0, 14.1Hz), 3.67 (3H, s), 4.20 (1H, dd, J=6.0, 7.2Hz), 5.09 (2H, s), 6.97 (2H, d, J=8.7Hz), 7.15 (2H, d, J=8.7Hz), 7.29-7.49 (5H, m), 8.60 (3H, br s)

### Reference Example 20

### Methyl O-(3-methylbenzyl)tyrosinate hydrochloride

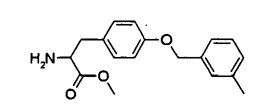

1) A mixture of methyl N-(tert-butoxycarbonyl) tyrosinate (1.00 g), 3-methylbenzyl bromide (0.74 g), potassium carbonate (0.55 g) and N,N-dimethylformamide (20 mL) was stirred for 16 hours at room temperature. Water was added to the reaction solution, which was then extracted with diethyl ether. The extract was washed with saturated sodium chloride solution, and then dried over anhydrous magnesium sulfate. The solvent was evaporated under reduced pressure, and the residue was purified by silica gel column chromatography to obtain methyl N-(tert-butoxycarbonyl)-O-(3-methylbenzyl) tyrosinate (1.00 g) as a colorless oil.
   ¹H-NMR (300MHz, CDCl₃); δ 1.42 (9H, s), 2.37 (3H, s), 2.95-3.10 (2H, m), 3.71 (3H, s), 4.30-5.00 (2H, m), 5.00 (2H, s), 6.90 (2H, d, J=8.7Hz), 7.04 (2H, d, J=8.7Hz), 7.12-7.30 (4H, m)
2) A solution of the compound obtained in 1) above (1.00 g), 10% hydrochloric acid-methanol solution (10 mL) and methanol (20 mL) was stirred for 1 hour at 60°C. The reaction solution was concentrated under reduced pressure, diethyl ether was added to the residue, and the precipitated crystals were filtered out to obtain the title compound (0.65 g) as colorless crystals. ¹H-NMR (300MHz, DMSO-d₆); δ 2.32 (3H, s), 3.06 (2H, m), 3.68 (3H, s), 4.23 (1H, t, J=6.6Hz), 5.04 (2H, s), 6.97 (2H, d, J=8.7Hz), 7.10-7.30 (6H, m), 8.48 (3H, br s)

### Reference Example 21

### Methyl O-(3-methoxybenzyl) tyrosinate hydrochloride

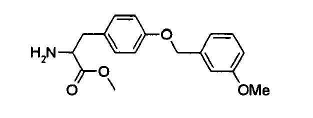

1) A mixture of methyl N-(tert-butoxycarbonyl) tyrosinate (1.00 g), 3-methoxybenzyl chloride (0.63 g), potassium carbonate (1.16 g) and N,N-dimethylformamide (20 mL) was stirred for 16 hours at room temperature. Water was added to the reaction solution, which was then extracted with diethyl ether. The extract was washed with saturated sodium chloride solution, and then dried over anhydrous magnesium sulfate. The solvent was evaporated under reduced pressure, and the residue was purified by silica gel column chromatography to obtain methyl N-(tert-butoxycarbonyl)-O-(3-methoxybenzyl) tyrosinate (0.79 g).
   ¹H-NMR (300MHz, CDCl₃); δ 1.42 (9H, m), 2.90-3.10 (2H, m), 3.71 (3H, s), 3.82 (3H, s), 4.25-5.00 (2H, m), 5.01 (2H, s), 6.83-6.93 (3H, m), 6.96-7.06 (4H, m), 7.29 (1H, t, J=7.8Hz)
2) A solution of the compound obtained in 1) above (0.79 g), 10% hydrochloric acid-methanol solution (10 mL) and methanol (20 mL) was stirred for 1 hour at 60°C. The reaction solution was concentrated under reduced pressure, diethyl ether was added to the residue, and the precipitated crystals were filtered out to obtain the title compound (0.61 g) as colorless crystals. ¹H-NMR (300MHz, DMSO-d₆); δ 3.05 (2H, m), 3.68 (3H, s), 3.76 (3H, s), 4.23 (1H, t, J=6.6Hz), 5.06 (2H, s), 6.88-7.00 (5H, m), 7.14 (2H, d, J=8.7Hz), 7.27-7.33 (1H, m), 8.46 (3H, br s)

### Example A1

### 5-(4-Chlorophenyl)-1-benzothiophene-2-carboxylic acid

1) Ethyl 5-bromo-1-benzothiophene-2-carboxylate (2.0 g), 4-chlorophenylboronic acid (1.2 g), 1 M potassium carbonate aqueous solution (15 mL) and ethanol (15 mL) were added to toluene (50 mL), and stirred for 30 minutes at room temperature in an argon atmosphere. Tetrakis triphenylphosphine palladium (0.24 g) was added and refluxed for 8 hours. The mixture was extracted with ethyl acetate, the organic layer was washed with water and sodium chloride solution and dried over magnesium sulfate, and the solvent was evaporated. The residue was purified by silica gel column chromatography (elution solvent: ethyl acetate), and the precipitated crystals were filtered out and washed with ethyl acetate-hexane to obtain ethyl 5-(4-chlorophenyl)-1-benzothiophene-2-carboxylate (1.9 g) as colorless crystals.
   ¹H-NMR (300MHz, CDCl₃) δ 1.43 (3H, t, J = 7.1 Hz), 4.43 (2H, q, J = 7.1 Hz), 7.52 (2H, d, J = 8.5 Hz), 7.57 (2H, d, J = 8.5 Hz), 7.65 (1H, dd, J = 1.8, 8.6 Hz), 7.93 (1H, d, J = 8.6 Hz), 8.02 (1H, d, J = 1.8 Hz), 8.10 (1H, s)
2) Ethyl 5-(4-chlorophenyl)-1-benzothiophene-2-carboxylate (1.9 g) and 1 N sodium hydroxide aqueous solution (20 mL) were refluxed for 3 hours in methanol (100 mL). 1 N hydrochloric acid (20 mL) was added, and the mixture was concentrated and then extracted with ethyl acetate. The organic layer was washed with water and sodium chloride solution and dried over magnesium sulfate, and the solvent was evaporated. The precipitated crystals were filtered out and washed with ethyl acetate-hexane to obtain 5-(4-chlorophenyl)-1-benzothiophene-2-carboxylic acid (1.6 g) as colorless crystals.
   ¹H-NMR (300MHz, DMSO-d₆) δ 7.56 (2H, d, J = 8.6 Hz), 7.78 (2H, d, J = 8.6 Hz), 7.82 (1H, dd, J = 2.0, 8.6 Hz), 8.14 (1H, d, J = 8.6 Hz), 8.15 (1H, s), 8.31 (1H, d, J = 2.0 Hz), 13.5 (1H, s)

### Example A2

### 5-[4-(Trifluoromethyl)phenyl]-1-benzothiophene-2-carboxylic acid

1) Ethyl 5-bromo-1-benzothiophene-2-carboxylate (2.0 g), 4-trifluoromethylphenylboronic acid (1.47 g), 1 M potassium carbonate aqueous solution (15 mL) and ethanol (15 mL) were added to toluene (50 mL), and stirred for 30 minutes at room temperature in an argon atmosphere. Tetrakis triphenylphosphine palladium (0.24 g) was added and refluxed for 8 hours. The mixture was extracted with ethyl acetate, the organic layer was washed with water and sodium chloride solution and dried over magnesium sulfate, and the solvent was evaporated. The precipitated crystals were filtered out and washed with ethyl acetate-hexane to obtain ethyl 5-[4-(trifluoromethyl)phenyl]-1-benzothiophene-2-carboxylate (2.2 g) as colorless crystals.
   ¹H-NMR (300MHz, CDCl₃) δ 1.44 (3H, t, J = 7.2 Hz), 4.43 (2H, q, J = 7.2 Hz), 7.70 (1H, dd, J = 1.7, 8.8 Hz), 7.74 (4H, s), 7.96 (1H, d, J = 8.5 Hz), 8.07 (1H, d, J = 1. 7 Hz), 8.12 (1H, s)
2) Ethyl 5-[4-(trifluoromethyl)phenyl]-1-benzothiophene-2-carboxylate (2.2 g) and 1 N aqueous sodium hydroxide solution (20 mL) were refluxed for 3 hours in methanol (100 mL). 1 N hydrochloric acid (20 mL) was added, and the mixture was concentrated and then extracted with ethyl acetate. The organic layer was washed with water and sodium chloride solution and dried over magnesium sulfate, and the solvent was evaporated. The precipitated crystals were filtered out and washed with ethyl acetate-hexane to obtain 5-[4-(trifluoromethyl)phenyl]-1-benzothiophene-2-carboxylic acid (2.0 g) as colorless crystals.
   ¹H-NMR (300MHz, CDCl₃) δ 7.77-7.78 (5H, m), 8.00 (1H, d, J = 8.5 Hz), 8.12 (1H, d, J = 1.7 Hz), 8.22 (1H, s)

### Example A3

### 5-(3-Thienyl)-1-benzothiophene-2-carboxylic acid

1) Ethyl 5-bromo-1-benzothiophene-2-carboxylate (2.0 g), 3-thiopheneboronic acid (0.99 g), 1 M potassium carbonate aqueous solution (15 mL) and ethanol (15 mL) were added to toluene (50 mL), and stirred for 30 minutes at room temperature in an argon atmosphere. Tetrakis triphenylphosphine palladium (0.24 g) was added and refluxed for 8 hours. The mixture was extracted with ethyl acetate, the organic layer was washed with water and sodium chloride solution and dried over magnesium sulfate, and the solvent was evaporated. The residue was purified by silica gel column chromatography (elution solvent: ethyl acetate/hexane) to obtain ethyl 5-(3-thienyl)-1-benzothiophene-2-carboxylate (1.9 g) as colorless crystals.
   ¹H-NMR (300MHz, CDCl₃) δ 1.43 (3H, t, J = 7.2 Hz), 4.42 (2H, q, J = 7.2 Hz), 7.39-7.49 (2H, m), 7.51-7.52 (1H, m) 7.71(1 H, dd, J = 1.5, 8.5Hz,) 7.88 (1H, d, J = 8.5 Hz), 8.07 (1H, d, J = 1.5 Hz) 8.08 (1H, s)
2) Ethyl 5-(3-thienyl)-1-benzothiophene-2-carboxylate (1.9 g) and 1 N sodium hydroxide aqueous solution (20 mL) were heated for 6 hours at 80°C in ethanol (150 mL). This was concentrated, 1 N hydrochloric acid (20 mL) was added, and the mixture was extracted with ethyl acetate. The organic layer was washed with water and sodium chloride solution and dried over magnesium sulfate, and the solvent was evaporated. The precipitated crystals were filtered out and washed with ethyl acetate-hexane to obtain 5-(3-thienyl)-1-benzothiophene-2-carboxylic acid (1.64 g) as colorless crystals.
   ¹H-NMR (300MHz, DMSO-d₆) δ 7.63 (1H, dd, J = 1.0, 5.0 Hz), 7.66-7.74 (1H m), 7.89 (1H, dd, J = 1.6, 8.6 Hz), 7.93-7.98 (1H, m), 8.08 (1 H, d, J = 8.6 Hz,) 8.10 (1H, s), 8.34 (1H, d, J = 1.6 Hz) 13.49 (1H, s)

### Example A4

### 5-(4-Methoxyphenyl)-1-benzothiophene-2-carboxylic acid

1) Ethyl 5-bromo-1-benzothiophene-2-carboxylate (2.0 g), 4-methoxyphenylboronic acid (1.2 g), 1 M potassium carbonate aqueous solution (15 mL) and ethanol (15 mL) were added to toluene (50 mL), and stirred for 30 minutes at room temperature in an argon atmosphere. Tetrakis triphenylphosphine palladium (0.24 g) was added and refluxed for 8 hours. The mixture was extracted with ethyl acetate, the organic layer was washed with water and sodium chloride solution and dried over magnesium sulfate, and the solvent was evaporated. The residue was purified by silica gel column chromatography (elution solvent: ethyl acetate/hexane) to obtain ethyl 5-(4-methoxyphenyl)-1-benzothiophene-2-carboxylate (1.89 g) as colorless crystals.
   ¹H-NMR (300MHz, CDCl₃) δ 1.43 (3H, t, J = 7.2 Hz), 3.87 (3H, s), 4.42 (2H, q, J = 7.2 Hz), 7.01 (2H, d, J = 8.7 Hz), 7. 58 (2H, d, J = 8.7 Hz), 7.66 (1H, dd, J = 1. 7, 8.5 Hz), 7.89 (1H, d, J = 8.5Hz), 8.01 (1H, d, J=1.7Hz), 8.09(1H,s)
2) Ethyl 5-(4-methoxyphenyl)-1-benzothiophene-2-carboxylate (1.89 g) and 1 N sodium hydroxide aqueous solution (20 mL) were heated for 6 hours at 80°C in ethanol (150 mL). This was concentrated, 1 N hydrochloric acid (20 mL) was added, and the mixture was extracted with ethyl acetate. The organic layer was washed with water and sodium chloride solution and dried over magnesium sulfate, and the solvent was evaporated. The precipitated crystals were filtered out and washed with ethyl acetate-hexane to obtain 5-(4-methoxyphenyl)-1-benzothiophene-2-carboxylic acid (1.63 g) as colorless crystals.
   ¹H-NMR (300MHz, DMSO-d₆) δ 3.82 (3H, s), 7.06 (2H, d, J = 8.9 Hz), 7.68 (2H, d, J = 8.9 Hz), 7.78 (1H, dd, J = 1.6, 8.6 Hz), 8.09 (1H, d, J = 8.6 Hz), 8.13 (1H, s), 8.23 (1H, d, J = 1.6 Hz), 13.50 (1H, s)

### Example A5

### 5-[3-(Acetylamino)phenyl]-1-benzothiophene-2-carboxylic acid

1) Ethyl 5-bromo-1-benzothiophene-2-carboxylate (2.0 g), 3-acetamidobenzene boronic acid (1.38 g), 1 M potassium carbonate aqueous solution (15 mL) and ethanol (15 mL) were added to toluene (50 mL), and stirred for 30 minutes at room temperature in an argon atmosphere. Tetrakis triphenylphosphine palladium (0.24 g) was added and refluxed for 8 hours. The mixture was extracted with ethyl acetate, the organic layer was washed with water and sodium chloride solution and dried over magnesium sulfate, and the solvent was evaporated. The residue was purified by silica gel column chromatography (elution solvent: ethyl acetate/hexane) to obtain ethyl 5-[3-(acetylamino)phenyl]-1-benzothiophene-2-carboxylate (1.91 g) as pale brown crystals. ¹H-NMR (300MHz, CDCl₃) δ 1.43 (3H, t, J = 7.1 Hz), 2.22 (3H, s), 4.43 (2H, q, J = 7.1 Hz), 7.34-7.54 (3H, m), 7.69 (1H, dd, J = 1.7, 8.5 Hz), 7.85 (1H, s), 7.91 (1H, d, J = 8.5 Hz), 8.05 (1H, d, J = 1.7Hz), 8.09(1H, s)
2) Ethyl 5-[3-(acetylamino)phenyl]-1-benzothiophene-2-carboxylate (1.91 g) and 1 N sodium hydroxide aqueous solution (15 mL) were heated for 1 hour at 80°C in ethanol (100 mL). This was concentrated, 1 N hydrochloric acid was added, and the mixture was extracted with ethyl acetate-tetrahydrofuran. The organic layer was washed with sodium chloride solution and dried over magnesium sulfate, and the solvent was evaporated. The precipitated crystals were filtered out and washed with ethyl acetate-hexane to obtain 5-[3-(acetylamino)phenyl]-1-benzothiophene-2-carboxylic acid (1.74 g) as pale brown crystals.
   ¹H-NMR (300MHz, DMSO-d₆) δ 2.08 (3H, s), 7.59 (1H, d, J = 6.6 Hz), 7.30-7.55 (2H, m), 7.75 (1H, d, J = 8.5 Hz), 7.97 (1H, s), 8.10-8.27 (3H, m), 10.07 (1H, s), 13.51 (1H, br)

### Example A6

### 5-(Pyridin-3-yl)-1-benzothiophene-2-carboxylic acid

1) Ethyl 5-bromo-1-benzothiophene-2-carboxylate (2.0 g), pyridine-3-boronic acid (0.95 g), 1 M potassium carbonate aqueous solution (15 mL) and ethanol (15 mL) were added to toluene (50 mL), and stirred for 30 minutes at room temperature in an argon atmosphere. Tetrakis(triphenylphosphine)palladium (0.24 g) was added and refluxed for 8 hours. The mixture was extracted with ethyl acetate, the organic layer was washed with water and sodium chloride solution and dried over magnesium sulfate, and the solvent was evaporated. The residue was purified by silica gel column chromatography (elution solvent: ethyl acetate/hexane) to obtain ethyl 5-(pyridin-3-yl)-1-benzothiophene-2-carboxylate (1.39 g) as pale yellow crystals. ¹H-NMR (300MHz, CDCl₃) δ 1.44 (3H, t, J = 7.2 Hz), 4.43 (2H, q, J = 7.2 Hz), 7.41 (1H, dd, J = 4.9, 7. 9 Hz), 7.68 (1H, dd, J = 1. 8, 8.6 Hz), 7.90-7.96 (1H, m), 7.97 (1H, d, J = 8.6 Hz), 8.06 (1H, d, J = 1.5 Hz), 8.12 (1H, s), 8.63 (1H, dd, J = 1.5, 4.8 Hz), 8.91 (1H, d, J = 1.8 Hz)
2) Ethyl 5-(pyridin-3-yl)-1-benzothiophene-2-carboxylate (1.39 g) and 1 N sodium hydroxide aqueous solution (15 mL) were heated for 1 hour at 80°C in ethanol (100 mL). This was concentrated, 1 N hydrochloric acid was added, and the precipitated sediment was filtered out and washed with water and ethyl acetate-hexane to obtain 5-(pyridin-3-yl)-1-benzothiophene-2-carboxylic acid (1.1 g) as light black crystals.
   ¹H-NMR (300MHz, DMSO-d₆) δ 7.53 (1H, dd, J = 4.7, 8.1 Hz), 7.88 (1H, dd, J = 1.5, 8.1 Hz), 8.07-8.28 (3R, m), 8.38 (1H, d, J = 1.6 Hz), 8.61 (1H, d, J = 4.7 Hz), 8.98 (1H, d, J = 2.5 Hz), 13.56 (1H, br)

### Example A7

### 5-[4-(Dimethylamino)phenyl]-1-benzothiophene-2-carboxylic acid

1) Ethyl 5-bromo-1-benzothiophene-2-carboxylate (2.0 g), 4-dimethylaminophenylboronic acid (1.27 g), 1 M potassium carbonate aqueous solution (15 mL) and ethanol (15 mL) were added to toluene (50 mL), and stirred for 30 minutes at room temperature in an argon atmosphere. Tetrakis(triphenylphosphine)palladium (0.24 g) was added and refluxed for 8 hours. The mixture was extracted with ethyl acetate, the organic layer was washed with water and sodium chloride solution and dried over magnesium sulfate, and the solvent was evaporated. The residue was purified by silica gel column chromatography (elution solvent: ethyl acetate/hexane) to obtain ethyl 5-[4-(dimethylamino)phenyl]-1-benzothiophene-2-carboxylate (1.36 g) as yellow crystals.
   ¹H-NMR (300MHz, CDCl₃) δ 1.43 (3H, t, J = 7.1 Hz), 3.01 (6H, s), 4.42 (2H, q, J = 7.1 Hz), 6.83 (2H, d, J = 8.9 Hz), 7.65 (2H, d, J = 8.9 Hz), 7.68 (1H, dd, J = 1.7, 8.5 Hz), 7.87 (1H, d, J = 8.5Hz), 8.00 (1H, d, J = 1.7 Hz), 8.08 (1H, s)
2) Ethyl 5-[4-(dimethylamino)phenyl]-1-benzothiophene-2-carboxylate (1.36 g) and 1 N sodium hydroxide aqueous solution (15 mL) were heated for 1 hour at 80°C in ethanol (150 mL). This was concentrated and extracted with ethyl acetate after addition of 1 N hydrochloric acid. The organic layer was washed with water and sodium chloride solution and dried over magnesium sulfate, and the solvent was evaporated to obtain 5-[4-(dimethylamino)phenyl]-1-benzothiophene-2-carboxylic acid (1.1 g) as yellow crystals.
   ¹H-NMR (300MHz, DMSO-d₆) δ 2.96 (6H, s), 6.83 (2H, d, J = 8.7 Hz), 7.59 (2H, d, J = 8.7 Hz), 7.75 (1H, d, J = 8.5 Hz), 8.03 (1H, d, J = 8.5 Hz), 8.11 (1H, s), 8.19 (1H, s), 13.42 (1H, br)

### Example A8

### 5-(4-Fluorophenyl)-1-benzothiophene-2-carboxylic acid

1) Ethyl 5-bromo-1-benzothiophene-2-carboxylate (2.0 g), 4-fluorophenyl boronic acid (1.08 g), 1 M potassium carbonate aqueous solution (15 mL) and ethanol (15 mL) were added to toluene (50 mL), and stirred for 30 minutes at room temperature in an argon atmosphere. Tetrakis(triphenylphosphine)palladium (0.24 g) was added and refluxed for 8 hours. The mixture was extracted with ethyl acetate, the organic layer was washed with water and sodium chloride solution and dried over magnesium sulfate, and the solvent was evaporated. The residue was purified by silica gel column chromatography (elution solvent: ethyl acetate/hexane) to obtain ethyl 5-(4-fluorophenyl)-1-benzothiophene-2-carboxylate (1.79 g) as colorless crystals. ¹H-NMR (300MHz, CDCl₃) δ 1.43 (3H, t, J = 7.2 Hz), 4.43 (2H, q, J = 7.2 Hz), 7.09-7.23 (2H, m), 7.53-7.71 (3H, m), 7.92 (1H, d, J = 8.5 Hz), 8.01 (1H, d, J = 1.5 Hz), 8.09 (1H, s)
2) Ethyl 5-(4-fluorophenyl)-1-benzothiophene-2-carboxylate (1.79 g) and 1 N sodium hydroxide aqueous solution (15 mL) were heated for 1 hour at 80°C in ethanol (100 mL). This was concentrated and extracted with ethyl acetate after addition of 1 N hydrochloric acid. The organic layer was washed with water and sodium chloride solution and dried over magnesium sulfate, and the solvent was evaporated to obtain 5-(4-fluorophenyl)-1-benzothiophene-2-carboxylic acid (1.43 g) as colorless crystals.
   ¹H-NMR (300MHz, DMSO-d₆) δ 7.29-7.36 (2H, m), 7.76-7.81 (3H, m), 8.11-8.15 (2H, m), 8.27 (1H, s), 13.50 (1H, br)

### Example A9

### 6-(4-Chlorophenyl)-3-methyl-1-benzofuran-2-carboxylic acid

1) A mixture of ethyl 6-bromo-3-methyl-1-benzofuran-2-carboxylate (2.3 g), (4-chlorophenyl) boronic acid (1.46 g), tetrakis(triphenylphosphine)palladium (1.08 g), 2 N sodium carbonate aqueous solution (20 mL) and 1,2-dimethoxyethane (30 mL) was refluxed for 3 hours in an argon atmosphere. The insoluble matter was filtered out, and water was added to the filtrate, which was then extracted with ethyl acetate. The extracted was washed with water, dried over magnesium sulfate and then concentrated. The residue was subjected to silica gel chromatography, and the raw crystals obtained from the ethyl acetate-hexane (1:5) eluate were recrystallized from ethyl acetate-hexane to obtain ethyl 6-(4-chlorophenyl)-3-methyl-1-benzofuran-2-carboxylate as colorless needles (1.73 g, yield 67%).
   ¹H-NMR (200MHz, CDCl₃)δ 1.46 (3H, t, J=7.2Hz), 2.62 (3H, s), 4.47 (2H, q, J=7.1Hz), 7.41-7.70 (7H, m)
2) A mixture of ethyl 6-(4-chlorophenyl)-3-methyl-1-benzofuran-2-carboxylate (1.70 g), lithium hydroxide monohydrate (0.45 g), tetrahydrofuran (30 mL) and water (5 mL) was stirred for 15 hours at 60°C. The reaction mixture was concentrated, diluted with water and adjusted to pH 3 with 1 N hydrochloric acid. The precipitated crystals were filtered out to obtain the title compound as colorless needles (1.35 g, yield 87%).
   ¹H-NMR (200MHz, DMSO-d₆)δ 2.56 (3H, s), 7.55 (2H, d, J=8.4Hz), 7.67 (1H, dd, J=1.6, 8.2Hz), 7.80 (2H, d, J=8.8Hz), 7.86 (1H, d, J=8.2Hz), 7.96 (1H, d, J=1.0Hz)

### Example A10

### 6-(4-Chlorophenyl)-3-propyl-1-benzofuran-2-carboxylic acid

1) Ethyl 6-(4-chlorphenyl)-3-propyl-1-benzofuran-2-carboxylate was obtained as colorless needles (yield 90%) from ethyl 6-bromo-3-propyl-1-benzofuran-2-carboxylate as in Example A9-1).
   ¹HNMR (200MHz, CDCl₃)δ 1.02 (3H, t, J=7.5Hz), 1.46 (3H, t, J=7.1Hz), 1.67-1.86 (2H, m), 3.09 (2H, t, J=7.5Hz), 4.47 (2H, q, J=7.1Hz), 7.41-7.59 (5H, m), 7.68-7.72 (2H, m)
2) The title compound was obtained as colorless needles (yield 85%) as in Example A9-2). ¹H-NMR (200MHz, DMSO-d₆)δ 0.94 (3H, t, J=7.4Hz), 1.61-1.79 (2H, m), 3.06 (2H, t, J=7.4Hz), 7.55 (2H, d, J=8.6Hz), 7.66 (1H, dd, J=1.5, 8.3Hz), 7.80 (2H, d, J=8.6Hz), 7.89 (1H, d, J=8.2Hz), 7.96 (1H,d, J=1.2Hz)

### Example A11

### 3-Methyl-6-{4-[(2-methyl-1H-benzimidazol-1-yl)methyl]phenyl }-1-benzofuran-2-carboxylic acid

1) A mixture of ethyl 6-bromo-3-methyl-1-benzofuran-2-carboxylate (6.36 g), 4-formylphenyl boronic acid (4.04 g), tetrakis(triphenylphosphine)palladium (0) (1.30 g), 2 M sodium carbonate aqueous solution (22.5 mL) and 1,2-dimethoxyethane (120 mL) was refluxed for 8 hours. The reaction solution was concentrated under reduced pressure, and extracted with ethyl acetate after addition of water. The extract was washed with saturated sodium chloride solution and dried over magnesium sulfate. The solvent was evaporated under reduced pressure, and the residue was purified by silica gel column chromatography to obtain ethyl 6-(4-formylphenyl)-3-methyl-1-benzofuran-2-carboxylate (6.36 g) as pale yellow crystals.
   ¹H-NMR (300MHz, CDCl₃) δ 1.46 (3H, t, J=7.1Hz), 2.63 (3H, s), 4.48 (2H, q, J=7.1Hz), 7.59 (1H, dd, J=1.5, 8.1Hz), 7.72 (1H, d, J=8.1Hz), 7.75-7.82 (3H, m), 7.99 (2H, d, J=8.1Hz), 10.08 (1H, s)
2) Sodium borohydride (937 mg) was added to a mixed tetrahydrofuran-methanol solution (50 mL-100 mL) of ethyl 6-(4-formylphenyl)-3-methyl-1-benzofuran-2-carboxylate (6.36 g), and stirred for 10 minutes at room temperature. The reaction soltuion was concentrated under reduced pressure, and extracted with ethyl acetate after addition of water. The extract was washed with saturated sodium chloride solution and dried over anhydrous magnesium sulfate. The solvent was evaporated under reduced pressure, and the residue was purified by silica gel column chromatography to obtain ethyl 6-[4-(hydroxymethyl)phenyl]-3-methyl-1-benzofuran-2-carboxylate (5.45 g) as colorless crystals.
   Melting point: 135-136°C (ethyl acetate-hexane)
   ¹H-NMR (300MHz, CDCl₃) δ 1.46 (3H, t, J=7.2Hz), 1.68 (1H, t, J=6.0Hz), 2.62 (3H, s), 4.47 (2H, q, J=7.2Hz), 4.77 (2H, d, J=6.0Hz), 7.48 (2H, d, J=8.1Hz), 7.56 (1H, dd, J=1.5, 8.1Hz), 7.64 (2H, d, J=8.1Hz), 7.68 (1H, d, J=8.1Hz), 7.74 (1H, d, J=1.5Hz)
3) Thionyl chloride (1.41 mL) and N,N-dimethylformamide (2 drops) were added to a dichloromethane solution (100 mL) of ethyl 6-[4-(hydroxymethyl)phenyl]-3-methyl-1-benzofuran-2-carboxylate (5.00 g), and stirred for 1 hour at room temperature. The reaction solution was concentrated under reduced pressure, and the residue was purified by silica gel column chromatography to obtain ethyl 6-[4-chloromethyl)phenyl]-3-methyl-l-benzofuran-2-carboxylate (5.90 g) as colorless crystals.
   Melting point: 99-100°C (ethyl acetate-hexane)
   ¹H-NMR (300MHz, CDCl₃) δ 1.46 (3H, t, J=7.2Hz), 2.62 (3H, s), 4.47 (2H, q, J=7.2Hz), 4.66 (2H, s), 7.50 (2H, d, J=8.1Hz), 7.55 (1H, dd, J=1.2, 8.1Hz), 7.63 (2H, d, J=8.1Hz), 7.69 (1H, d, J=8.1Hz), 7.74 (1H, d, J=1.2Hz)
4) Sodium hydride (462 mg) was added to a N,N-dimethylformamide solution (50 mL) of 2-methylbenzimidazole (1.49 g), and stirred for 15 minutes at room temperature. Ethyl 6-[4-chloromethyl)phenyl]-3- methyl-1-benzofuran-2-carboxylate (3.10 g) was then added to the reaction solution, and stirred for 3 hours at room temperature. Water was added to the reaction solution, which was then extracted with dichloromethane. The extract was washed with saturated sodium chloride solution and dried over anhydrous magnesium sulfate. The solvent was evaporated under reduced pressure, and the residue was purified by silica gel column chromatography to obtain ethyl 3-methyl-6-{4-[(2-methyl-1H-benzimidazol-1-yl)methyl]phenyl]-1-benzofuran-2-carboxylate (1.93 g) as pale yellow crystals.
   Melting point: 178-179°C (methanol)
   ¹H-NMR (300MHz, CDCl₃) δ 1.45 (3H, t, J=7.2Hz), 2.60 (3H, s), 2.62 (3H, s), 4.46 (2H, q, J=7.2Hz), 5.39 (2H, s), 7.15 (2H, d, J=8.4Hz), 7.18-7.28 (3H, m), 7.49 (1H, dd, J=1.5, 8.4Hz), 7.57 (2H, d, J=8.4Hz), 7.62-7.78 (3H, m)
5) 2 M Sodium hydroxide aqueous solution (2.5 mL) and water (25 mL) were added to a mixed tetrahydrofuran-ethanol solution (25 mL-25 mL) of ethyl 3-methyl-6-{4-[(2-methyl-1H-benzimidazol-1-yl)methyl]phenyl]-1-benzofuran-2-carboxylate (1.93 g), and stirred for 14 hours at 60°C. The reaction solution was neutralized with 1 N hydrochloric acid, and concentrated under reduced pressure. The precipitated solid was filtered out, washed successively with water, methanol and diethyl ether, and dried to obtain the title compound (1.75 g) as colorless crystals. Melting point: 295-297°C
   ¹H-NMR (300MHz, DMSO-d₆) δ 2.54 (3H, s), 2.56 (3H, s), 5.54 (2H, s), 7.13-7.20 (2H, m), 7.23 (2H, d, J=8.4Hz), 7.47-7.59 (2H, m), 7.63 (1H, dd, J=1.2, 8.4Hz), 7.73 (2H, d, J=8.4Hz), 7.83 (1H, d, J=8.4Hz), 7.90 (1H, d, J=1.2Hz), 13.44 (1H, br s)

The structural formulae for the compounds of Examples A1-A11 are shown in Table 2.

**(Table 2)**

| | | | |
|---|---|---|---|
| Example A1 | 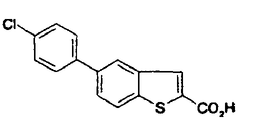 | Example A7 | 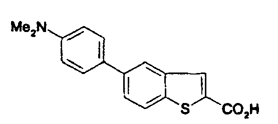 |
| Example A2 | 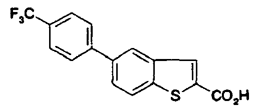 | Example A8 | 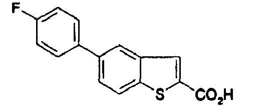 |
| Example A3 | 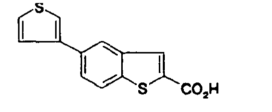 | Example A9 | 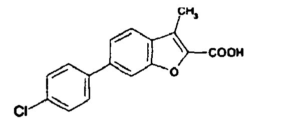 |
| Example A4 | 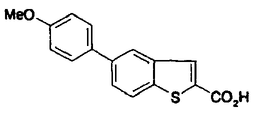 | Example A10 | 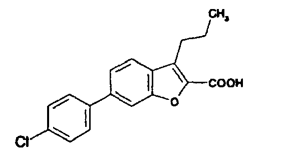 |
| Example A5 | 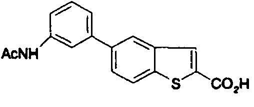 | Example A11 | 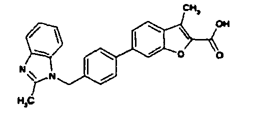 |
| Example A6 | 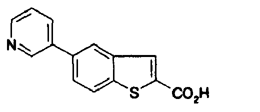 | | |

The Compounds of Examples A12-A22 were synthesized by the methods described in WO99/32468 (Table 3).
Example A12
   7-Phenyl-2-naphthoic acid
Example A13
   7-Phenyl-3,4-dihydro-2-naphthalenecarboxylic acid
Example A14
   6-(4-Methylphenyl)-2H-thiochromene-3-carboxylic acid
Example A15
   6-(4-Methylphenyl)-2H-chromene-3-carboxylic acid
Example A16
   2-Methyl-6-(4-methylphenyl)-3-quinolinecarboxylic acid
Example A17
   7-(4-Methylphenyl)-2,3-dihydro-1-benzothiepin-4-carboxylic acid
Example A18
   7-(4-Chlorophenyl)-2,3-dihydro-1-benzoxepin-4-carboxylic acid
Example A19
   1-Methyl-7-(4-methylphenyl)-2,3-dihydro-1H-1-benzazepin-4-carboxylic acid hydrochloride Example A20
   7-(4-Chlorophenyl)-2,3-dihydro-1-benzothiepin-4-carboxylic acid 1,1-dioxide
Example A21
   7-(4-Methylphenyl)-1-trifluoromethylsulfonyl-2,3-dihydro-1H-1-benzazepin-4-carboxylic acid Example A22
   2-(4-Methylphenyl)-6,7-dihydro-5H-benzo[7]annulene-8-carboxylic acid

**(Table 3)**

| | | |
|---|---|---|
| 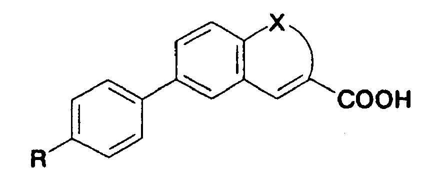 | | |

| Example | R | X |
|---|---|---|
| A12, | H | -CH=CH- |
| A13 | H | -CH₂CH₂- |
| A14 | Me | -S-CH₂- |
| A15 | Me | -O-CH₂- |
| A16 | Me | -N=C(Me)- |
| A17 | Me | -S-CH₂CH₂- |
| A18 | Cl | -O-CH₂CH₂- |
| A19 | Me | -N(Me)-CH₂CH₂- |
| A20 | Cl | -SO₂-CH₂CH₂- |
| A21 | Me | -N(SO₂CF₃)-CH₂CH₂- |
| A22 | Me | -CH₂CH₂CH₂- |

### Example A23

### 3-({[4-(Benzyloxy)phenyl]acetyl}amino)-6-(4-chlorophenyl)-1-benzofuran-2-carboxylic acid

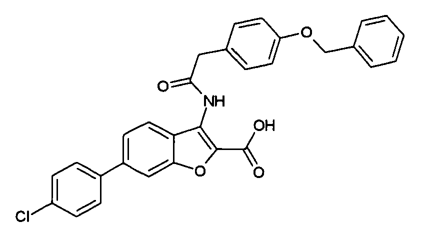

(1) 4-Benzyloxyphenylacetyl chloride (313 mg) was added at 70°C to a mixed toluene-tetrahydrofuran solution (3 mL-2 mL) of ethyl 3-amino-6-(4-chlorophenyl)-1-benzofuran-2-carboxylate (316 mg) and triethylamine (0.21 mL), and stirred at the same temperature. After 3.5 hours, triethylamine (0.21 mL) and 4-benzyloxyphenylacetyl chloride (313 mg) were added and stirred for a further 1 hour. This was cooled to room temperature, and water was added to the reaction solution, which was then extracted with ethyl acetate. The extract was washed successively with aqueous sodium hydroxide solution and saturated sodium chloride solution, and dried over anhydrous magnesium sulfate. The solvent was evaporated under reduced pressure, and the residue was purified by basic silica gel column chromatography and recrystallized from ethyl acetate to obtain ethyl 3-({[4-(benzyloxy)phenyl]acetyl}amino)-6-(4-chlorophenyl)-1-benzofuran-2-carboxylate (230 mg) as colorless crystals.
   ¹H-NMR (300MHz, CDCl₃); δ 1.40 (3H, t, J=7.2Hz), 3.80 (2H, s), 4.39 (2H, q, J=7.2Hz), 5.09 (2H, s), 7.03 (2H, d, J=8.4Hz), 7.28-7.50 (10H, m), 7.55 (2H, d, J=8.4Hz), 7.62 (1H, d, J=1.5Hz), 8.47 (1H, d, J=8.4Hz), 9.34 (1H, s)
(2) 2 N Sodium hydroxide solution (0.3 mL) and water (5 mL) were added to a tetrahydrofuran solution (7 mL) of the compound obtained in (1) (130 mg), and stirred for 1.5 hours at 70°C. The reaction solution was neutralized with 2 N hydrochloric acid, and then neutralized with diethyl ether. The extract was washed with saturated sodium chloride solution and then dried over anhydrous magnesium sulfate. The solvent was evaporated under reduced pressure, and the precipitated crystals were filtered out and washed with methanol to obtain the compound (79 mg) as colorless crystals.
   Melting point: 211-212°C
   ¹H-NMR (300MHz, DMSO-d₆); δ 3.76 (2H, s), 5.10 (2H, s), 7.00 (2H, d, J=8.7Hz), 7.30-7.49 (7H, m), 7.55 (2H, d, J=8.7Hz), 7.63 (1H, dd, J=1.5, 8.4Hz), 7.80 (2H, d, J=8.7Hz), 7.89 (1H, d, J=8.7Hz), 7.96 (1H, d, J=1.5Hz), 10.06 (1H, s), 13.56 (1H, br s)
   Elemental analysis: Calcd. for C₃₀H₂₂ClNO₅ 0.25 H₂O: C 69.77, H 4.39, N 2.71; Found: C 70.01, H 4.48, N 2.62

### Example A24

### 6-(4-Chlorophenyl)-3-[({4-[(4-fluorobenzyl)oxy]phenyl}acetyl)amino]-1-benzofuran-2-carboxylic acid

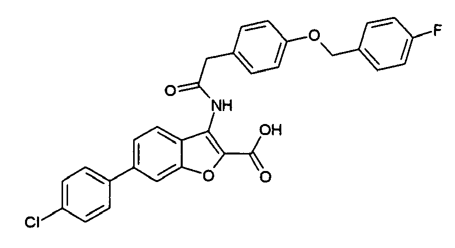

(1) A mixture of {4-[(4-fluorobenzyl)oxy]phenyl}acetic acid (781 mg), thionyl chloride (0.44 mL), N,N-dimethylformamide (2 drops) and tetrahydrofuran (15 mL) was stirred for 20 minutes at 60°C. The reaction solution was concentrated under reduced pressure to obtain [4-(4-fluorobenzyloxy)phenyl]acetyl chloride. Next, a toluene solution (1.5 mL) of the resulting [4-(4-fluorobenzyloxy)phenyl]acetyl chloride was added at 70°C to a mixed toluene-tetrahydrofuran solution (3 mL-2 mL) of ethyl 3-amino-6-(4-chlorophenyl)-1-benzofuran-2-carboxylate (316 mg) and triethylamine (0.62 mL), and stirred for 1 hour at the same temperature. This was cooled to room temperature, and water was added to the reaction solution, which was then extracted with ethyl acetate. The extract was washed successively with aqueous sodium hydroxide solution, hydrochloric acid and saturated sodium chloride solution, and dried over anhydrous magnesium sulfate. The solvent was evaporated under reduced pressure, and the residue was purified by basic silica gel column chromatography and recrystallized from ethyl acetate to obtain ethyl 6-(4-chlorophenyl)-3-[({4-[(-fluorobenzyl)oxy]phenyl}acetyl)amino]-1-benzofuran-2-carboxylate (152 mg) as colorless crystals.
   ¹H-NMR (300MHz, CDCl₃); δ 1.41 (3H, t, J=7.2Hz), 3.80 (2H, s), 4.40 (2H, q, J=7.2Hz), 5.04 (2H, s), 7.01 (2H, d, J=8.4Hz), 7.07 (2H, t, J=8.4Hz), 7.33 (2H, d, J=8.4Hz), 7.37-7.50 (5H, m), 7.55 (2H, d, J=8.4Hz), 7.63 (1H, d, J=1.2Hz), 8.48 (1H, d, J=8.4Hz), 9.36 (1H, s)
(2) The title compound (72 mg) was obtained as colorless crystals from the Compound (110 mg) obtained in (1) by operations similar to those of Example A23(2).
   Melting point: 205-206°C
   ¹H-NMR (300MHz, DMSO-d₆); δ 3.76 (2H, s), 5.08 (2H, s), 7.00 (2H, d, J=8.7Hz), 7.22 (2H, t, J=8.7Hz), 7.33 (2H, d, J=8.7Hz), 7.47-7.58 (4H, m), 7.63 (1H, dd, J=1.5, 8.7Hz), 7.80 (2H, d, J=8.7Hz), 7.89 (1H, d, J=8.7Hz), 7.96 (1H, d, J=1.5Hz), 10.07 (1H, s), 13.56 (1H, br s) Elemental analysis: Calcd. for C₃₀H₂₁ClFNO₅: C 67.99, H 3.99, N 2.64; Found: C 68.07, H 4.28, N 2.64

### Example A25

### 3-({3-[4-(Benzyloxy)phenyl]propanoyl}amino)-6-(4-chlorophenyl)-1-benzofuran-2-carboxylic acid

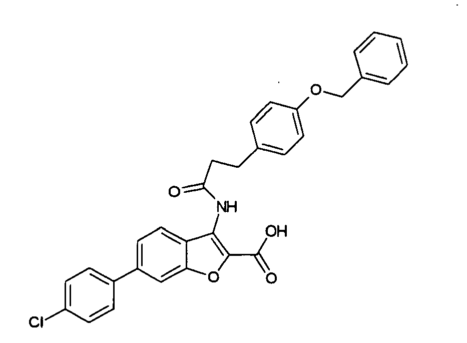

(1) A mixture of methyl 3-(4-hydroxyphenyl) propionate (1.57 g), benzyl bromide (1.24 mL), potassium carbonate (1.44 g) and N,N-dimethylformamide (30 mL) was stirred for 3.5 hours at room temperature. Water was added to the reaction solution, which was then extracted with diethyl ether. The extract was washed with saturated sodium chloride solution, and dried over anhydrous magnesium sulfate. The solvent was evaporated under reduced pressure, and the residue was recrystallized from hexane to obtain methyl 3-[4-(benzyloxy)phenyl] propionate (1.98 g) as colorless crystals.
   ¹H-NMR (300MHz, CDCl₃); δ 2.60 (2H, t, J=7.8Hz), 2.89 (2H, t, J=7.8Hz), 3.66 (3H, s), 5.04 (2H, s), 6.90 (2H, d, J=8.4Hz), 7.11 (2H, d, J=8.4Hz), 7.28-7.45 (5H, m)
(2) 2 N Sodium hydroxide aqueous solution (3.5 mL) and water (10 mL) were added to a tetrahydrofuran-methanol solution (20 mL-10 mL) of the compound obtained in (1) (1.60 g), and stirred for 1 hour at 60°C. The reaction solution was made acidic with 1 N hydrochloric acid, and then extracted with diethyl ether. The extract was washed with saturated sodium chloride solution, and dried over anhydrous magnesium sulfate. The solvent was evaporated under reduced pressure, and the residue was recrystallized from diethyl ether-hexane to obtain 3-[4-(benzyloxy)phenyl] propionic acid (1.27 g) as colorless crystals.
   ¹H-NMR (300MHz, CDCl₃); δ 2.65 (2H, t, J=7.8Hz), 2.91 (2H, t, J=7.8Hz), 5.04 (2H, s), 6.91 (2H, d, J=8.7Hz), 7.13 (2H, d, J=8.7Hz), 7.29-7.45 (5H., m)
(3) The compound obtained in (2) (769 mg), thionyl chloride (5 mL) and N,N-dimethylformamide (2 drops) were stirred for 1 hour at 70°C. The reaction solution was concentrated under reduced pressure to obtain 3-(4-benzyloxyphenyl) propionyl chloride. Next, a toluene-tetrahydrofuran solution (1 mL- mL) of the resulting 3-(4-benzyloxyphenyl) propionyl chloride was added at 70°C to a mixed toluene-tetrahydrofuran solution (2 mL-2 mL) of ethyl 3-amino-6-(4-chlorophenyl)-1-benzofuran-2-carboxylate (316 mg) and triethylamine (0.69 mL), and stirred for 30 minutes at the same temperature. This was cooled to room temperature, and water was added to the reaction solution, which was then extracted with ethyl acetate. The extract was washed successively with aqueous sodium hydroxide solution, hydrochloric acid and saturated sodium chloride solution, and dried over anhydrous magnesium sulfate. The solvent was evaporated under reduced pressure, and the residue was purified by basic silica gel column chromatography and recrystallized from ethyl acetate to obtain ethyl 3-({3-[4-(benzyloxy)phenyl]propanoyl}amino)-6-(4-chlorophenyl)-1-benzofuran-2-carboxylate (140 mg) as pale brown crystals.
   ¹H-NMR (300MHz, CDCl₃); δ 1.46 (3H, t, J=7.2Hz), 2.81 (2H, t, J=7.7Hz), 3.07 (2H, t, J=7.7Hz), 4.47 (2H, q, J=7.2Hz), 5.04 (2H, s), 6.92 (2H, d, J=8.4Hz), 7.19 (2H, d, J=8.4Hz), 7.30-7.52 (8H, m), 7.57 (2H, d, J=8.4Hz), 7.64 (1H, d, J=1.5Hz), 8.48 (1H, d, J=8.4Hz), 9.38 (1H, s)
(4) The title compound (70 mg) was obtained as colorless crystals from the compound (100 mg) obtained in (3) by operations similar to those of Example C123(2).
   Melting point: 209-210°C
   ¹H-NMR (300MHz, DMSO-d₆); δ 2.76 (2H, t, J=7.5Hz), 2.92 (2H, t, J=7.5Hz), 5.07 (2H, s), 6.95 (2H, d, J=8.4Hz), 7.22 (2H, d, J=8.4Hz), 7.27-7.46 (5H, m), 7.56 (2H, d, J=8.7Hz), 7.62 (1H, dd, J=1.2, 8.4Hz), 7.75-7.84 (3H, m), 7.96 (1H, d, J=1.2Hz), 9.94 (1H, s), 13.55 (1H, br s) Elemental analysis: Calcd. for C₃₁H₂₄ClNO₅·0.25 H₂O: C 70.19, H 4.66, N 2.64, Found: C 70.25, H4.66, N2.53

### Example A26

### 6-(4-Chlorophenyl)-1-benzothiophene-2-carboxylic acid

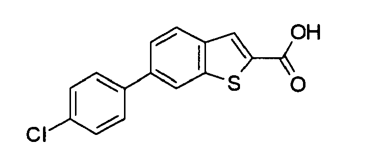

(1) A mixture of 4-bromo-2-fluorobenzaldehyde (10.15 g), methyl thioglycolate (5.30 mL), potassium carbonate (8.26 g) and N,N-dimethylformaldehyde (100 mL) was stirred for 30 minutes at room temperature and then stirred for 1 hour at 80°C. Water was added to the reaction solution, which was then extracted with diethyl ether. The extract was washed successively with aqueous sodium hydroxide solution and saturated sodium chloride solution, and dried over anhydrous magnesium sulfate. The solvent was evaporated under reduced pressure, and the residue was recrystallized from diethyl ether-hexane to obtain ethyl 6-(4-chlorophenyl)-1-benzothiophene-2-carboxylate (10.42 g) as colorless crystals.
   ¹H-NMR (300MHz, CDCl₃); δ 3.95 (3H, s), 7.52 (1H, dd, J=1.5, 8.4 Hz), 7.73 (1H, d, J=8.4Hz), 7.98-8.05 (2H, m)
(2) A mixture of the compound obtained in (1) (2.71 g), 4-chlorophenylboronic acid (1.88 g), tetrakis(triphenylphosphine)palladium (0) (578 mg), 2 M sodium carbonate aqueous solution (10 mL) and 1,2-dimethoxyethane (50 mL) was stirred for 16 hours at 80°C. Water was added to the reaction solution, which was then extracted with ethyl acetate. The extract was washed with saturated sodium chloride solution and dried over anhydrous magnesium sulfate. The solvent was evaporated under reduced pressure, and the residue was purified by basic silica gel column chromatography and recrystallized from ethyl acetate-hexane to obtain methyl 6-(4-chlorophenyl)-1-benzothiophene-2-carboxylate (2.39 g) as colorless crystals.
   ¹H-NMR (300MHz, CDCl₃); δ 3.96 (3H, s), 7.40-7.48 (2H, m), 7.55-7.63 (3H, m), 7.93 (1H, d, J=8.1Hz), 8.03 (1H, t, J=0.9Hz), 8.08 (1H, d, J=0.6Hz)
(3) 2 N Sodium hydroxide aqueous solution (3 mL) and water (50 mL) were added to a mixed tetrahydrofuran (50 mL)-ethanol (50 mL) solution of the compound obtained in (2) (1.80 g), and stirred for 2 hours at 60°C. The reaction solution was neutralized with 2 N hydrochloric acid, and the precipitated crystals were filtered out and washed successively with water, methanol and diethyl ether to obtain 6-(4-chlorophenyl)-1-benzothiophene-2-carboxylic acid (1.60 g) as colorless crystals.
   ¹H-NMR (300MHz, DMSO-d₆); δ 7.56 (2H, d, J=8.7Hz), 7.75-7.85 (3H, m), 8.08 (1H, d, J=8.4Hz), 8.13 (1H, s), 8.39 (1H, s), 13.52 (1H, br s)

### Example A27

### 5-(4-Isopropoxyphenyl)-1-benzothiophene-2-carboxylic acid

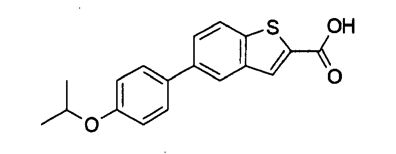

(1) A mixture of the ethyl 5-bromo-1-benzothiophene-2-carboxylate obtained in Reference Example 7 (1.00 g), 4-isopropoxyphenyl boronic acid (2.26 g), tetrakis(triphenylphosphine)palladium (0) (402 mg), 2 M sodium carbonate aqueous solution (3.5 mL) and 1,2-dimethoxyethane (16.5 mL) was exposed to microwaves for 10 minutes at 150°C. Water was added to the reaction solution, which was then extracted with ethyl acetate. The extract was washed with saturated sodium chloride solution, and dried over anhydrous magnesium sulfate. The solvent was evaporated under reduced pressure, and the residue was purified by basic silica gel column chromatography and recrystallized from ethyl acetate-hexane to obtain ethyl 5-(4-isopropoxyphenyl)-1-benzothiophene-2-carboxylate (0.76 g) as colorless crystals.
   ¹H-NMR (300MHz, CDCl₃); δ 1.38 (6H, d, J=6.0Hz), 1.43 (3H, t, J=7.2Hz), 4.42 (2H, q, J=7.2Hz), 4.61 (1H, m), 6.99 (2H, d, J=8.7Hz), 7.56 (2H, d, J=8.7Hz), 7.66 (1H, dd, J=1.5, 8.7Hz), 7.88 (1H, d, J=8.7Hz), 8.00 (1H, d, J=1.5Hz), 8.08 (1H, s)
(2) 1 N Sodium hydroxide aqueous solution (0.3 mL) and water (15 mL) were added to a tetrahydrofuran-ethanol mixed solution (15 mL-15 mL) of the compound (0.76 g) obtained in (1), and stirred for 1 hour at 60°C. The reaction solution was neutralized with 2 N hydrochloric acid and then extracted with ethyl acetate. The extract was washed with saturated sodium chloride solution and then dried over anhydrous magnesium sulfate. The solvent was evaporated under reduced pressure, and the precipitated crystals were filtered out and washed with ethyl acetate-hexane to obtain the title compound (0.54 g) as colorless crystals.
   ¹H-NMR (300MHz, DMSO-d₆); δ 1.30 (6H, d, J=6.0Hz), 4.68 (1H, m), 7.03 (2H, d, J=8.7Hz), 7.65 (2H, d, J=8.7Hz), 7.77 (1H, dd, J=1.5, 8.7Hz), 8.08 (1H, d, J=8.7Hz), 8.13 (1H, s), 8.23 (1H, d, J=1.5Hz), 13.48 (1H, br s)

### Example A28

### 5-(4-Propoxyphenyl)- l-benzothiophene-2-carboxylic acid

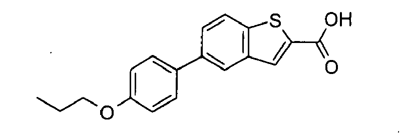

(1) A mixture of ethyl 5-bromo-1-benzothiophene-2-carboxylate (from Reference Example 7) (1.00 g), 4-methoxyphenyl boronic acid (2.21 g), tetrakis(triphenylphosphine)palladium (0) (300 mg), 2 M sodium carbonate aqueous solution (3.5 mL) and 1,2-dimethoxyethane (20 mL) was exposed to microwaves for 10 minutes at 150°C. Water was added to the reaction solution, which was then extracted with ethyl acetate. The extract was washed with saturated sodium chloride solution, and dried over anhydrous magnesium sulfate. The solvent was evaporated under reduced pressure, and the residue was purified by silica gel column chromatography and recrystallized from ethyl acetate-hexane to obtain ethyl 5-(4-propoxyphenyl)-1-benzothiophene-2-carboxylate (0.71 g) as colorless crystals.
   ¹H-NMR (300MHz, CDCl₃); δ 1.07 (3H, t, J=7.2Hz), 1.43 (3H, t, J=7.2Hz), 1.85 (2H, m), 3.98 (2H, t, J=6.6Hz), 4.42 (2H, q, J=7.2Hz), 7.00 (2H, d, J=8.7Hz), 7.56 (2H, d, J=8.7Hz), 7.66 (1H, dd, J=1.5, 8.7Hz), 7.89 (1H, d, J=8.7Hz), 8.00 (1H, d, J=1.5Hz), 8.08 (1H, s)
(2) 1 N Sodium hydroxide aqueous solution (3 mL) and water (15 mL) were added to a tetrahydrofuran-ethanol mixed solution (15 mL-15 mL) of the compound (0.71 g) obtained in (2 [sic]), and stirred for 1 hour at 60°C. The reaction solution was neutralized with 2 N hydrochloric acid and then extracted with ethyl acetate. The extract was washed with saturated sodium chloride solution and then dried over anhydrous magnesium sulfate. The solvent was evaporated under reduced pressure, and the precipitated crystals were filtered out and washed with ethyl acetate-hexane to obtain the title compound (0.54 g) as colorless crystals.
   ¹H-NMR (300MHz, DMSO-d₆); 5 1.00 (3H, t, J=7.2Hz), 1.76 (2H, m), 3.99 (2H, t, J=6.6Hz), 7.05 (2H, d, J=8.7Hz), 7.67 (2H, d, J=8.7Hz), 7.77 (1H, dd, J=1.5, 8.7Hz), 8.08 (1H, d, J=8.7Hz); 8.13 (1H, s), 8.23 (1H, d, J=1.5Hz), 13.49 (1H, br s)

### Example A29 .

### 5-(4-Methylphenyl)-1-benzothiophene-2-carboxylic acid

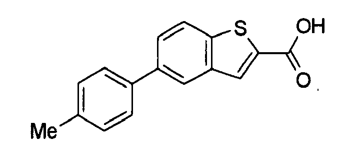

(1) A mixture of ethyl 5-bromo-1-benzothiophene-2-carboxylate (from Reference Example 7) (1.00 g), 4-methylphenyl boronic acid (1.67 g), tetrakis(triphenylphosphine)palladium (0) (300 mg), 2 M sodium carbonate aqueous solution (3.5 mL) and 1,2-dimethoxyethane (20 mL) was exposed to microwaves for 10 minutes at 150°C. Water was added to the reaction solution, which was then extracted with ethyl acetate. The extract was washed with saturated sodium chloride solution, and dried over anhydrous magnesium sulfate. The solvent was evaporated under reduced pressure, and the residue was purified by silica gel column chromatography and recrystallized from ethyl acetate-hexane to obtain ethyl 5-(4-methylphenyl)-1-benzothiophene-2-carboxylate (0.67 g) as colorless crystals.
   ¹H-NMR (300MHz, CDCl₃); δ 1.43 (3H, t, J=7.2Hz), 2.42 (3H, s), 4.42 (2H, q, J=7.2Hz), 7.28 (2H, d, J=8.1Hz), 7.54 (2H, d, J=8.1Hz), 7.69 (1H, dd, J=1.5, 8.4Hz), 7.90 (1H, d, J=8.4Hz), 8.04 (1H, d, J=1.5Hz), 8.09 (1H, s)
(2) 1 N Sodium hydroxide aqueous solution (3 mL) and water (15 mL) were added to a tetrahydrofuran-ethanol mixed solution (15 mL-15 mL) of the compound (0.67 g) obtained in (2 [sic]), and stirred for 1 hour at 60°C. The reaction solution was neutralized with 2 N hydrochloric acid and then extracted with ethyl acetate. The extract was washed with saturated sodium chloride solution and then dried over anhydrous magnesium sulfate. The solvent was evaporated under reduced pressure, and the precipitated crystals were filtered out and washed with ethyl acetate-hexane to obtain the title compound (0.50 g) as colorless crystals.
   ¹H-NMR (300MHz, DMSO-d₆); δ 2.36 (3H, s), 7.31 (2H, d, J=8.1Hz), 7.64 (2H, d, J=8.1Hz), 7.79 (1H, dd, J=1.5, 8.4Hz), 8.11 (1H, d, J=8.4Hz), 8.14 (1H, s), 8.26 (1H, d, J=1.5Hz), 13.49 (1H, br s)

### Example A30

### 5-(4-Chlorophenyl)-3-methyl-1-benzofuran-2-carboxylic acid

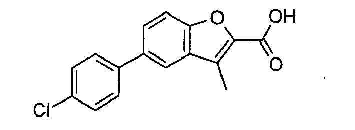

(1) A mixture of 5'-bromo-2'-hydroxyacetophenone (10.75 g), methyl bromoacetate (9.18 g), potassium carbonate (8.29 g) and N,N-dimethylformamide (100 mL) was stirred for 1.5 hours at room temperature. Water was added to the reaction solution, which was then extracted with diethyl ether. The extract was washed with saturated sodium chloride solution, and dried over anhydrous magnesium sulfate. The solvent was evaporated under reduced pressure, and the residue was recrystallized from diethyl ether-hexane to obtain methyl (2-acetyl-4-bromophenoxy)acetate (12.37 g) as colorless crystals.
   ¹H-NMR (300MHz, CDCl₃); δ 2.69 (3H, s), 3.82 (3H, s), 4.73 (2H, s), 6.72 (1H, d, J=8.7Hz), 7.53 (1H, dd, J=2.4, 8.7Hz), 7.87 (1H, d, J=2.4Hz)
(2) The compound (12.37 g) obtained in (1) and 1,8-diazabicyclo[5.4.0]undeca-7-ene (7.87 g) were refluxed for 2.5 hours in toluene (100 mL). The reaction solution was concentrated under reduced pressure, and water was added to the reaction solution, which was then extracted with ethyl acetate. The extract was washed successively with hydrochloric acid and saturated sodium chloride solution, and dried over anhydrous magnesium sulfate. The solvent was evaporated under reduced pressure, and the residue was purified by silica gel column chromatography to obtain methyl 5-bromo-3-methyl-1-benzofuran-2-carboxylate (6.32 g) as colorless crystals.
   ¹H-NMR (300MHz, CDC1₃); δ 2.56 (3H, s), 3.99 (3H, s), 7.42 (1H, d, J=9.OHz), 7.54 (1H, dd, J=1.8, 9.0Hz), 7.77 (1H, d, J=1.8Hz)
(3) A mixture of the compound (2.69 g) obtained in (2), 4-chlorophenylboronic acid (1.88 g), tetrakis(triphenylphosphine)palladium (0) (578 mg), 2 M sodium carbonate aqueous solution (10 mL) and 1,2-dimethoxyethane (50 mL) was stirred for 15 hours at 80°C. Water was added to the reaction solution, which was then extracted with ethyl acetate. The extract was washed with saturated sodium chloride solution, and dried over anhydrous magnesium sulfate. The solvent was evaporated under reduced pressure, and the residue was purified by basic silica gel column chromatography and recrystallized from ethyl acetate-hexane to obtain methyl 5-(4-chlorophenyl)-3-methyl-1-benzofuran-2-carboxylate (2.18 g) as colorless crystals.
   ¹H-NMR (300MHz, CDCl₃); δ 2.63 (3H, s), 4.00 (3H, s), 7.43 (2H, d, J=8.4Hz), 7.50-7.65 (4H, m), 7.75 (1H, dd, J=0.9, 1.8Hz)
(4) 2 N Sodium hydroxide aqueous solution (5 mL) and water (20 mL) were added to a tetrahydrofuran-methanol solution (30 mL-10 mL) of the compound obtained in (3) (1.85 g), and stirred for 2.5 hours at 60°C. The reaction solution was neutralized with 1 N hydrochloric acid, and then extracted with ethyl acetate. The extract was washed with saturated sodium chloride solution, and dried over anhydrous magnesium sulfate. The solvent was evaporated under reduced pressure, and the precipitated crystals were filtered out and washed with diethyl ether to obtain 5-(4-chlorophenyl)-3-methyl-1-benzofuran-2-carboxylic acid (1.46 g) as colorless crystals. ¹H-NMR (300MHz, DMSO-d₆); δ 2.59 (3H, s), 7.54 (2H, d, J=8.4Hz), 7.72 (1H, d, J=8.7Hz), 7.76-7.82 (3H, m), 8.07 (1H, d, J=1.5Hz), 13.51 (1H, br s)

### Example A31

### 6-(4-Chlorophenyl)-1-benzofuran-2-carboxylic acid

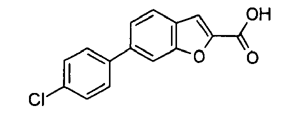

(1) Sodium hydride (5.64 g) was added with ice cooling to a N,N-dimethylformamide solution (150 mL) of 4-bromo-2-fluorobenzaldehyde (11.97 g) and ethyl glycolate (14.67 g), and stirred for 20 minutes. Water was added to the reaction solution, which was then extracted with diethyl ether. The extract was washed successively with water and saturated sodium chloride solution, dried over anhydrous magnesium sulfate, and passed through silica gel (NH). The solvent was evaporated under reduced pressure, and the residue was purified by silica gel column chromatography to obtain ethyl 6-bromo-1-benzofuran-2-carboxylate (3.71 g) as pale yellow crystals.
   ¹H-NMR (300MHz, CDCl₃); δ 1.43 (3H, t, J=7.2Hz), 4.45 (2H, q, J=7.2Hz), 7.44 (1H, dd, J=1.8, 8.4Hz), 7.49 (1H, s), 7.55 (1H, d, J=8.4Hz), 7.77 (1H, d, J=1.8Hz)
(2) A mixture of the compound (2.69 g) obtained in (1), 4-chlorophenylboronic acid (1.88 g), tetrakis(triphenylphosphine)palladium (0) (578 mg), 2 M sodium carbonate aqueous solution (10 mL) and 1,2-dimethoxyethane (50 mL) was stirred for 15 hours at 80°C. Water was added to the reaction solution, which was then extracted with ethyl acetate. The extract was washed with saturated sodium chloride solution and dried over anhydrous magnesium sulfate. The solvent was evaporated under reduced pressure, and the residue was purified by basic silica gel column chromatography and recrystallized from ethyl acetate-hexane to obtain ethyl 6-(4-chlorophenyl)-1-benzofuran-2-carboxylate (2.22 g) as pale yellow crystals.
   ¹H-NMR (300MHz, CDCl₃); δ 1.45 (3H, t, J=7.2Hz), 4.46 (2H, q, J=7.2Hz), 7.44 (2H, d, J=8.4Hz), 7.50-7.59 (4H, m), 7.71-7.77 (2H, m)
(3) 2 N Sodium hydroxide aqueous solution (5 mL) and water (15 mL) were added to a mixed tetrahydrofuran-ethanol (20 mL-10 mL) solution of the compound (1.90 g) obtained in (2), and stirred for 20 minutes at 60°C. The reaction solution was neutralized with 1 N hydrochloric acid, and extracted with ethyl acetate. The extract was washed with saturated sodium chloride solution, and dried over anhydrous magnesium sulfate. The solvent was evaporated under reduced pressure, and the residue was recrystallized from ethyl acetate-hexane to obtain 6-(4-chlorophenyl)-1-benzofuran-2-carboxylic acid (1.50) as colorless crystals.
   ¹H-NMR (300MHz, DMSO-d₆); δ 7.55 (2H, d, J=8.4Hz), 7.63-7.73 (2H, m), 7.81 (2H, d, J=8.4Hz), 7.87 (1H, d, J=8.4Hz), 8.03 (1H, s), 13.62 (1H, br s)

### Example A32

### 5-(4-Chlorophenyl)-1-benzofuran-2-carboxylic acid

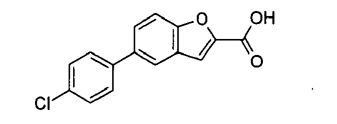

(1) A mixture of 5-bromosalicylaldehyde (10.05 g), ethyl bromoacetate (6.65 mL), potassium carbonate (8.29 g) and N,N-dimethylformamide (100 mL) was stirred for 3.5 hours at room temperature. Water was added to the reaction solution, which was then extracted with diethyl ether. The extract was washed with saturated sodium chloride solution, and dried over anhydrous magnesium sulfate. The solvent was evaporated under reduced pressure, and the residue was purified by silica gel column chromatography to obtain ethyl (4-bromo-2-formylphenoxy)acetate (14.36 g) as a colorless oil.
   ¹H-NMR (300MHz, CDCl₃); δ 1.30 (3H, t, J=7.2Hz), 4.28 (2H, q, J=7.2Hz), 4.75 (2H, s), 6.77 (1H, d, J=8.7Hz), 7.62 (1H, dd, J=2.7, 8.7Hz), 7.97 (1H, d, J=2.7Hz), 10.48 (1H, s)
(2) The compound obtained in (1) (14.36 g) and 1,8-diazobicyclo[5.4.0]undeca-7-ene (9.46 g) were refluxed for 2.5 hours in toluene. The reaction solution was concentrated under reduced pressure, and water was added to the residue, which was then extracted with ethyl acetate. The extract was washed successively with hydrochloric acid and saturated sodium chloride solution, and dried over anhydrous magnesium sulfate. The solvent was evaporated under reduced pressure, and the residue was purified by silica gel column chromatography to obtain ethyl 5-bromo-2-benzofuran-2-carboxylate (7.51 g) as colorless crystals.
   ¹H-NMR (300MHz, CDCl₃); δ 1.43 (3H, t, J=7.2Hz), 4.45 (2H, q, J=7.2Hz), 7.45 (1H, s), 7.47 (1H, d, J=8.7Hz), 7.54 (1H, dd, J=2.1, 8.7Hz), 7.82 (1H, d, J=2.1Hz)
(3) A mixture of the compound (7.51 g) obtained in (2), 4-chlorophenylboronic acid (5.24 g), tetrakis(triphenylphosphine)palladium (0) (1.62 g), 2 M sodium carbonate aqueous solution (28 mL) and 1,2-dimethoxyethane (150 mL) was stirred for 14 hours at 80°C. Water was added to the reaction solution, which was then extracted with ethyl acetate. The extract was washed with saturated sodium chloride solution, and dried over anhydrous magnesium sulfate. The solvent was evaporated under reduced pressure, and the residue was purified by basic silica gel column chromatography and recrystallized from ethyl acetate-hexane to obtain ethyl 5-(4-chlorophenyl)-1-benzofuran-2-carboxylate (4.40 g) as colorless crystals.
   ¹H-NMR (300MHz, CDCl₃); δ 1.45 (3H, t, J=7.2Hz), 4.47 (2H, q, J=7.2Hz), 7.43 (2H, d, J=8.7Hz), 7.53 (2H, d, J=8.7Hz), 7.56-7.68 (3H, m), 7.81-7.83 (1H, m)
(4) 1 N Sodium hydroxide aqueous solution (15 mL) and water (30 mL) were added at 60°C to a tetrahydrofuran-ethanol mixed solution (50 mL-25 mL) of the compound (4.00 g) obtained in (3), and stirred for 15 minutes at the same temperature. The reaction solution was neutralized with 1 N hydrochloric acid and then extracted with ethyl acetate. The extract was washed with saturated sodium chloride solution and then dried over anhydrous magnesium sulfate. The solvent was evaporated under reduced pressure, and the residue was recrystallized from ethyl acetate-hexane to obtain 5-(4-chlorophenyl)-1-benzofuran-2-carboxylic acid (3.18 g) as colorless crystals.
   ¹H-NMR (300MHz, DMSO-d₆); δ 7.54 (2H, d, J=8.4Hz), 7.69-7.83 (5H, m), 8.06 (1H, s), 13.65 (1H, br s)

### Example A33

### 6-(4-Chlorophenyl)imidazo[1,2-a]pyridine-2-carboxylic acid

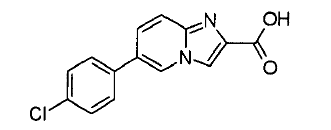

(1) A 1,2-dimethoxyethane solution (100 mL) of 2-amino-5-bromo-pyridine (5.19 g) and ethyl bromopyruvate (5.65 mL) was stirred for 2 hours at room temperature. The precipitated solid was filtered out, suspended in ethanol (180 mL) and refluxed for 2.5 hours. The reaction solution was concentrated under reduced pressure, and the residue was neutralized with saturated aqueous sodium hydrogencarbonate solution and extracted with chloroform. The extract was washed with saturated sodium chloride solution and then dried over anhydrous magnesium sulfate. The solvent was evaporated under reduced pressure, and the residue was recrystallized from ethyl acetate-hexane to obtain ethyl 6-bromoimidazo[1,2-a]pyridine-2-carboxylate (5.48 g) as colorless crystals.
   ¹H-NMR (300MHz, CDCl₃); δ 1.44 (3H, t, J=7.2Hz), 4.46 (2H, q, J=7.2Hz), 7.31 (1H, dd, J=1.8, 9.6Hz), 7.59 (1H, d, J=9.6Hz), 8.14 (1H, s), 8.29 (1H, d, J=1.8Hz)
(2) A mixture of the compound obtained in (1) (2.69 g), 4-chlorophenyl boronic acid (1.88 g), tetrakis(triphenylphosphine)palladium (0) (578 mg), 2 M sodium carbonate aqueous solution (10 mL) and 1,2-dimethoxyethane (60 mL) was stirred for 15 hours at 80°C. Water was added to the reaction solution, which was then extracted with ethyl acetate. The extract was washed with saturated sodium chloride solution and dried over anhydrous magnesium sulfate. The solvent was evaporated under reduced pressure, and the residue was purified by basic silica gel column chromatography and recrystallized from ethyl acetate-hexane to obtain ethyl 6-(4-chlorophenyl)imidazo[1,2-a]pyridine-2-carboxylate (1.75 g) as colorless crystals.
   ¹H-NMR (300MHz, CDCl₃); δ 1.46 (3H, t, J=7.2Hz), 4.48 (2H, q, J=7.2Hz), 7.45-7.55 (5H, m), 7.75 (1H, d, J=9.3Hz), 8.23 (1H, s), 8.27 (1H, dd, J=1.2, 1.8Hz)
(3) 1 N Sodium hydroxide aqueous solution (5 mL) and water (5 mL) were added at 60°C to a mixed tetrahydrofuran-ethanol solution (10 mL-10 mL) of the compound (1.0 g) obtained in (2), and stirred for 30 minutes at the same temperature. The reaction solution was neutralized with 1 N hydrochloric acid, and the precipitated crystals were filtered out and washed with diethyl ether to obtain the title compound (0.80 g) as colorless crystals.
   ¹H-NMR (300MHz, DMSO-d₆); δ 7.59 (2H, dt, J=2.4, 8.7Hz), 7.68-7.72 (2H, m), 7.75 (2H, dt, J=2.4, 8.7Hz), 8.47 (1H, s), 8.96 (1H, t, J=1.5Hz)

### Example A34

### 6-(4-Chlorophenyl)imidazo[1,2-b]pyridazine-2-carboxylic acid

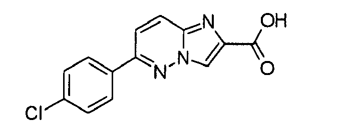

(1) A mixture of ethyl 6-chloroimidazo [1,2-b] pyridazine-2-carboxylate (2.26 g), 4-chlorophenyl boronic acid (1.88 g), tetrakis(triphenylphosphine)palladium (0) (578 mg), 2 M sodium carbonate aqueous solution (10 mL) and 1,2-dimethoxyethane (60 mL) was stirred for 18 hours at 80°C. Water was added to the reaction solution, which was then extracted with ethyl acetate. The extract was washed with saturated sodium chloride solution, and dried over anhydrous magnesium sulfate. The solvent was evaporated under reduced pressure, and the residue was purified by basic silica gel column chromatography and recrystallized from ethyl acetate-hexane to obtain ethyl 6-(4-chlorophenyl)imidazo[1,2-b]pyridazine-2-carboxylate (1.57 g) as colorless crystals.
   ¹H-NMR (300MHz, CDCl₃); δ 1.46 (3H, t, J=7.2Hz), 4.50 (2H, q, J=7.2Hz), 7.52 (2H, d, J=8.7Hz), 7.54 (1H, d, J=9.6Hz), 7.92 (2H, d, J=8.7Hz), 8.08 (1H, dd, J=0.6, 8.7Hz), 8.55 (1H, s)
(2) 1 N Sodium hydroxide aqueous solution (7 mL) and water (20 mL) were added at 60°C to a mixed tetrahydrofuran-ethanol solution (40 mL-20 mL) of the compound (1.37 g) obtained in (1), and stirred at the same temperature for 30 minutes. The reaction solution was neutralized with 1 N hydrochloric acid, and then concentrated under reduced pressure. The precipitated crystals were filtered out and washed successively with water, methanol and diethyl ether to obtain the title compound (1.09 g) as colorless crystals.
   ¹H-NMR (300MHz, DMSO-d₆); δ 7.66 (2H, d, J=8.7Hz), 7.94 (1H, d, J=9.6Hz), 8.12 (2H, d, J=8.7Hz), 8.30 (1H, d, J=9.6Hz), 8.81 (1H, s), 13 .00 (1H, brs)

### Example A3 5

### 6-(4-Chlorophenyl)imidazo[1,2-a]pyrimidine-2-carboxylic acid

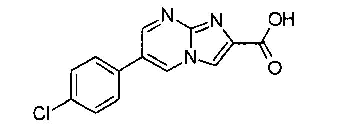

(1) A N,N-dimethylacetamide solution (150 mL) of 2-amino-5-bromopyrimidine (2.50 g) and ethyl bromopyruvate (3.61 mL) was stirred for 2 hours at room temperature and then stirred for 1 hour at 110°C. The reaction solution was neutralized with aqueous sodium hydroxide solution, and extracted with ethyl acetate. The extract was washed with saturated sodium chloride solution and then dried over anhydrous magnesium sulfate. The solvent was evaporated under reduced pressure, and the residue was purified by silica gel column chromatography to obtain 6-bromoimidazo[1,2-a]pyrimidine-2-carboxylic acid (0.36 g) as yellow crystals.
   ¹H-NMR (300MHz, CDCl₃); δ 1.44 (3H, t, J=7.2Hz), 4.45 (2H, q, J=7.2Hz), 8.43 (1H, s), 8.72 (1H, d, J=2.7Hz), 9.73 (1H, d, J=2.7Hz)
(2) A mixture of the compound (510 mg) obtained in (1), 4-chlorophenyl boronic acid (355 mg), tetrakis(triphenylphosphine)palladium (0) (110 mg), 2 M sodium carbonate aqueous solution (1.89 mL) and 1,2-dimethoxyethane (15 mL) was stirred for 18 hours at 80°C. Water was added to the reaction solution, which was then extracted with ethyl acetate. The extract was washed with saturated sodium chloride solution and dried over anhydrous magnesium sulfate. The solvent was evaporated under reduced pressure, and the residue was purified by basic silica gel column chromatography to obtain ethyl 6-(4-chlorophenyl)imidazo[1,2-a]pyrimidine-2-carboxylate (219 mg) as pale yellow crystals.
   ¹H-NMR (300MHz, CDCl₃); δ 1.45 (3H, t, J=7.2Hz), 4.45 (2H, q, J=7.2Hz), 7.52 (2H, d, J=8.7Hz), 7.58 (2H, d, J=8.7Hz), 8.49 (1H, s), 8.94 (1H, d, J=2.7Hz), 9.74 (1H, d, J=2.7Hz)
(3) 1 N Sodium hydroxide aqueous solution (1 mL) and water (5 mL) were added at 60°C to a mixed tetrahydrofuran-ethanol solution (7 mL-7 mL) of the compound (215 mg) obtained in (2), and stirred for 15 minutes at the same temperature. The reaction solution was neutralized with 1 N hydrochloric acid and extracted with ethyl acetate. The extract was washed with saturated sodium chloride solution, and dried over anhydrous magnesium sulfate. The solvent was evaporated under reduced pressure to obtain the title compound (170 mg) as pale yellow crystals.
   ¹H-NMR (300MHz, DMSO-d₆); δ 7.64 (2H, d, J=8.7Hz), 7.85 (2H, d, J=8.7Hz), 8.43 (1H, s), 9.12 (1H, d, J=2.7Hz), 9.68 (1H, d, J=2.7Hz), 13.50 (1H, brs)

### Example A36

### 7-(4-Chlorophenyl)imidazo[1,2-a]pyridine-2-carboxylic acid

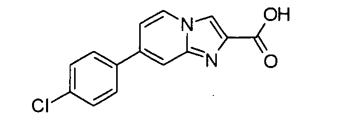

(1) A mixture of 2-chloro-4-iodopyridine (5.99 g), 4-chlorophenyl boronic acid (4.69 g), tetrakis(triphenylphosphine)palladium (0) (1.44 g), 2 M sodium carbonate aqueous solution (25 mL) and 1,2-dimethoxyethane (125 mL) was stirred for 18 hours at 80°C. Water was added to the reaction solution, which was then extracted with ethyl acetate. The extract was washed with saturated sodium chloride solution and dried over anhydrous magnesium sulfate. The solvent was evaporated under reduced pressure, and the residue was purified by basic silica gel column chromatography and recrystallized from ethyl acetate-hexane to obtain 2-chloro-4-(4-chlorophenyl) pyridine (2.07 g) as colorless crystals.
   ¹H-NMR (300MHz, CDCl₃); δ 7.40 (1H, dd, J=1.5, 5.4Hz), 7.48 (2H, d, J=8.7Hz), 7.51 (1H, d, J=1.5Hz), 7.55 (2H, d, J=8.7Hz), 8.44 (1H, d, J=5.4Hz)
(2) A mixture of the compound (1.12 g) obtained in (1), tris(dibenzylidenacetone) dipalladium (0) (46 mg), 2-(dicyclohexylphosphino)biphenyl (42 mg), lithium bis(trimethylsilyl)amido 1 M tetrahydrofuran solution (6 mL) and tetrahydrofuran (10 mL) was stirred for 16 hours at 65°C in an argon atmosphere. The reaction solution was cooled to room temperature, and a 1 M tetrahydrofuran solution (12 mL) of tetrabutyl ammonium fluoride was added and stirred for 5 hours at room temperature. Water was added to the reaction mixture, which was then made basic with aqueous sodium hydroxide solution and extracted with ethyl acetate. The extract was washed with saturated sodium chloride solution and dried over anhydrous magnesium sulfate. The solvent was evaporated under reduced pressure, and the residue was purified by basic silica gel column chromatography to obtain 2-amino-4-(4-chlorophenyl) pyridine (0.63 g) as pale yellow crystals.
   ¹H-NMR (300MHz, CDCl₃); δ 4.49 (2H, br s), 6.56 (1H, d, J=1.5Hz), 6.84 (1H, dd, J=1.5, 5.4Hz), 7.42 (2H, d, J=8.4Hz), 7.52 (2H, d, J=8.4Hz), 8.12 (1H, d, J=5.4Hz)
(3) A 1,2-dimethoxyethane solution (30 mL) of the compound (0.63 g) obtained in (2) and ethyl bromopyruvate (0. 58 mL) was stirred for 2 hours at room temperature, and the solvent was evaporated under reduced pressure. The precipitated solid was filtered out and washed with diethyl ether. The resulting solid was dissolved in ethanol (30 mL), and refluxed for 2.5 hours. The reaction solution was concentrated under reduced pressure, and the residue was made basic with saturated aqueous sodium hydrogencarbonate solution and extracted with ethyl acetate-tetrahydrofuran. The extract was washed with saturated sodium chloride solution and dried over anhydrous magnesium sulfate. The solvent was evaporated under reduced pressure, and the residue was purified by basic silica gel column chromatography and recrystallized from ethyl acetate-hexane to obtain ethyl 7-(4-chlorophenyl)imidazo[1,2-a]pyridine-2-carboxylate (493 mg) as colorless crystals.
   ¹H-NMR (300MHz, CDCl₃); δ 1.46 (3H, t, J=7.2Hz), 4.48 (2H, q, J=7.2Hz), 7.12 (1H, dd, J=1.8, 7.2Hz), 7.47 (2H, d, J=8.7Hz), 7.57 (2H, d, J=8.7Hz), 7.81-7.85 (1H, m), 8.17-8.22 (2H, m)
(4) 1 N Sodium hydroxide aqueous solution (2 mL) and water (10 mL) were added at 60°C to a mixed tetrahydrofuran-ethanol solution (10 mL-10 mL) of the compound (450 mg) obtained in (3), and stirred for 15 minutes at the same temperature. The reaction solution was neutralized with 1 N hydrochloric acid, and the precipitated crystals were filtered out and washed with diethyl ether to obtain the title compound (356 mg) as a colorless [substance].
   ¹H-NMR (300MHz, DMSO-d₆); δ 7.39 (1H, dd, J=1.5, 7.2Hz), 7.57 (2H, d, J=8.7Hz), 7.87 (2H, d, J=8.7Hz), 7.97 (1H, br s), 8.48 (1H, br s), 8.64 (1H, d, J=7.2Hz)

### Example A37

### 5-(4-Chlorphenyl)imidazo[1,2-a]pyridine-2-carboxylic acid

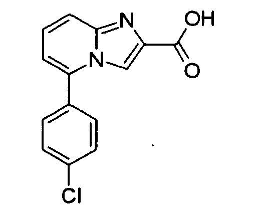

(1) An ethanol solution (300 mL) of 6-chloro-2-aminopyridine (14.88 g) and ethyl bromopyruvate (30 g) was refluxed overnight. The reaction solution was concentrated under reduced pressure, and extracted with water after addition of diethyl ether. The aqueous layer was made basic with aqueous sodium carbonate solution, and extracted with ethyl acetate-tetrahydrofuran. The extract was washed with saturated sodium chloride solution and dried over anhydrous magnesium sulfate. The solvent was evaporated under reduced pressure, and the residue was recrystallized from diisopropyl ether to obtain ethyl 5-chloroimidazo[1,2-a]pyridine-2-carboxylate (19.69 g) as colorless crystals.
   ¹H-NMR (CDCl₃, 400MHz); δ 1.45 (3H, t, J=7.2Hz), 4.48 (2H, q, J=7.2Hz), 6.98 (1H, dd, J=0.8, 7.2Hz), 7.26 (1H, dd, J=7.2, 9.2Hz), 7.67 (1H, dt, J=0.8, 9.2Hz), 8.38 (1H, d, J=0.8Hz)
(2) A mixture of the compound (1.28 g) obtained in (1), 4-chlorophenylboronic acid (1.78 g), tris(dibenzylidenacetone)dipalladium (0) chloroform adduct (0.34 g), 1,3-bis(2,6-diisopropylphenyl) imidazolium chloride (0.5 g), cesium carbonate (11.2 g) and dioxane (50 mL) was stirred for 24 hours at 80°C. The reaction solution was concentrated under reduced pressure, and ethyl acetate was added to the residue, which was then Celite filtered. Water was added to the filtrate, which was then extracted with ethyl acetate. The extract was washed with saturated sodium chloride solution and dried over anhydrous magnesium sulfate. The solvent was evaporated under reduced pressure, and the residue was purified by silica gel column chromatography and recrystallized from diisopropyl ether to obtain ethyl 5-(4-chlorophenyl)imidazo[1,2-a]pyridine-2-carboxylate (830 mg) as colorless crystals.
   ¹H-NMR (CDCl₃, 400MHz); δ 1.42 (3H, t, J=7.2Hz), 4.44 (2H, q, J=7.2Hz), 6.81 (1H, d, J=6.8Hz), 7.34 (1H, m), 7.56 (4H, m), 7.70 (1H, d, J=9.2Hz), 8.18 (1H, s)
(3) 1 N Sodium hydroxide aqueous solution (18 mL) was added to a tetrahydrofuran-ethanol solution (10 mL-10 mL) of the compound (2.7 g) obtained in (2), and stirred for 30 minutes at room temperature. The reaction solution was neutralized with 1 N hydrochloric acid, and extracted with chloroform after addition of citric acid solution. The extract was washed with saturated sodium chloride solution and dried over anhydrous magnesium sulfate. The solvent was evaporated under reduced pressure, and the residue was recrystallized from diethyl ether to obtain the title compound (1.58 g) as colorless crystals.
   ¹H-NMR (300MHz, DMSO-d₆); δ 7.04 (1H, d, J=6.9Hz), 7.48 (1H, dd, J=6.9, 9.0Hz), 7.60-7.75 (3H, m), 7.79 (2H, d, J=8.4Hz), 8.12 (1H, s)

### Example A38

### 7-(4-Chlorophenyl)pyrazolo[1,5-a]pyrimidine-2-carboxylic acid

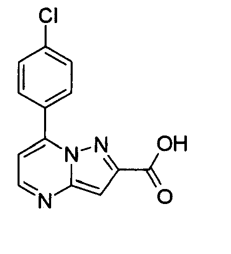

(1) A mixture of 5-nitro-3-pyrazole carboxylic acid (5.0 g), 10% hydrochloric acid-methanol solution (25 mL) and methanol (25 mL) was stirred for 16 hours at 65°C. The reaction solution was concentrated under reduced pressure to obtain methyl 5-nitro-3-pyrazole carboxylate (5.5 g) as pale yellow crystals.
   ¹H-NMR (300MHz, CDC1₃); δ 4.02 (3H, s), 7.42 (1H, s)
(2) 10% Palladium/carbon (500 mg) was added to a methanol solution (200 mL) of the compound (4.65 g) obtained in (1), and stirred for 2 hours in a hydrogen atmosphere. The catalyst was removed by filtration, and the filtrate was concentrated to obtain methyl 5-amino-3-pyrazole carboxylate (4.04 g) as light purple crystals.
   ¹H-NMR (300MHz, CDCl₃); δ 3.89 (3H, s), 4.50-6.50 (2H, br s), 6.05 (1H, s)
(3) A N,N-dimethylformamide dimethyl acetal solution (50 mL) of 4'-chloracetophenone (7.73 g) was refluxed for 16 hours. The reaction solution was concentrated, and diethyl ether-hexane was added to the residue. The precipitated crystals were filtered out and washed with hexane to obtain 1-(4-chlorophenyl)-3-(dimethylamino) propa-2-en-1-one (8.28 g) as orange crystals.
   ¹H-NMR (300MHz, CDCl₃); δ 2.94 (3H, s), 3.16 (3H, s), 5.66 (1H, d, J=12.3Hz), 7.38 (2H, d, J=8.7Hz), 7.81 (1H, d, J=12.3Hz), 7.84 (2H, d, J=8.7Hz)
(4) An acetic acid solution (150 mL) of the compound (4.04 g) obtained in (2) and the compound (6.00 g) obtained in (3) was stirred for 1 hour at 100°C. The reaction solution was concentrated, and the residue was extracted with ethyl acetate-tetrahydrofuran. The extract was washed successively with aqueous sodium hydroxide solution, hydrochloric acid and saturated sodium chloride solution, and dried over anhydrous magnesium sulfate. The solvent was evaporated under reduced pressure, and the residue was recrystallized from ethyl acetate to obtain methyl 7-(4-chlorophenyl)pyrazolo[1,5-a]pyrimidine-2-carboxylate (7.08 g) as pale yellow crystals.
   ¹H-NMR (300MHz, CDCl₃); δ 3.99 (3H, s), 7.03 (1H, d, J=4.5Hz), 7.34 (1H, s), 7.56 (2H, d, J=8.7Hz), 8.06 (2H, d, J=8.7Hz), 8.61 (1H, d, J=4.5Hz)
(5) 1 N Sodium hydroxide aqueous solution (5 mL) and water (10 mL) were added at 60°C to a mixed tetrahydrofuran-methanol solution (20 mL-10 mL) of the compound (0.86 g) obtained in (1), and stirred for 10 minutes at the same temperature. The reaction solution was neutralized with 1 N hydrochloric acid. The precipitated crystals were filtered out to obtain the title compound (0.70 g) as pale yellow crystals.
   ¹H-NMR (300MHz, DMSO-d₆); δ 7.24 (1H, s), 7.42 (1H, d, J=4.2Hz), 7.72 (2H, d, J=8.7Hz), 8.16 (2H, d, J=8.7Hz), 8.72 (1H, d, J=4.2Hz), 13.35 (1H, br s)

### Example A39

### 2-(4-Chlorophenyl)imidazo[1,2-a]pyridine-6-carboxylic acid

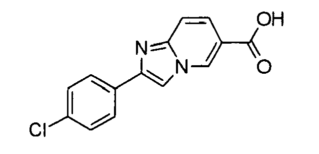

(1) A 1,2-dimethoxyethane solution (50 mL) of methyl 2-aminopyridine-5-carboxylate (1.52 g) and 4-chlorophenacyl bromide (2.33 g) was stirred for 40 hours at 80°C. The reaction solution was neutralized with saturated sodium hydrogencarbonate solution, and extracted with dichloromethane. The extract was washed with saturated sodium chloride solution, dried over anhydrous magnesium sulfate, and passed through silica gel. The solvent was evaporated under reduced pressure, and the residue was filtered and washed with ethyl acetate to obtain methyl 2-(4-chlorophenyl)imidazo[1,2-a]pyridine-6-carboxylate (1.98 g) as pale yellow crystals.
   ¹H-NMR (300MHz, CDCl₃); δ 3.97 (3H, s), 7.43 (2H, d, J=8.7Hz), 7.63 (1H, d, J=9.6Hz), 7.75 (1H, dd, J=1.8, 9.6Hz), 7.88-7.93 (3H, m), 8.90-8.92 (1H, m)
(2) 1 N Sodium hydroxide aqueous solution (5 mL) and water (20 mL) were added at 60°C to a mixed tetrahydrofuran-methanol solution (50 mL-25 mL) of the compound (1.00 g) obtained in (1), and stirred for 15 minutes at the same temperature. The reaction solution was neutralized with 1 N hydrochloric acid, and the precipitated crystals were filtered out and washed successively with water, methanol and diethyl ether to obtain the title compound (810 mg) as colorless crystals.
   ¹H-NMR (300MHz, DMSO-d₆); δ 7.53 (2H, d, J=8.4Hz), 7.60-7.75 (2H, m), 7.99 (2H, d, J=8.4Hz), 8.56 (1H, s), 9.23 (1H, s), 13.30 (1H, br s)

### Example A40

### 6-(4-Chlorophenyl)-5-methylimidazo[1,2-a]pyridine-2-carboxylic acid

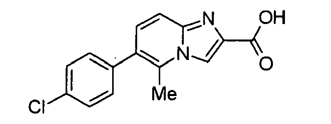

(1) A 1,2-dimethoxyethane solution (60 mL) of 6-amino-3-bromo-2-methylpyridine (2.50 g) and methyl bromopyruvate (2.70 g) was stirred for 2.5 hours at room temperature. The solvent was evaporated under reduced pressure, and the residue was filtered out, suspended in ethanol (60 mL) and refluxed for 2.5 hours. The reaction solution was concentrated under reduced pressure, and the residue was neutralized with saturated aqueous sodium hydrogencarbonate solution and extracted with ethyl acetate. The extract was washed with saturated sodium chloride solution and dried over anhydrous magnesium sulfate. The solvent was evaporated under reduced pressure, and the residue was recrystallized from ethyl acetate-hexane to obtain ethyl 6-bromo-5-methylimidazo[1,2-a]pyridine-2-carboxylate (2.28 g) as pale yellow crystals. ¹H-NMR (300MHz, CDCl₃); δ 1.45 (3H, t, J=7.2Hz), 2.77 (3H, s), 4.47 (2H, q, J=7.2Hz), 7.40 (1H, d, J=9.6Hz), 7.49 (1H, d, J=9.6Hz), 8.14 (1H, s)
(2) A mixture of the compound (2.00 g) obtained in (1), 4-chlorophenylboronic acid (1.32 g), tetrakis(triphenylphosphine)palladium (0) (408 mg), 2 M sodium carbonate aqueous solution (7.06 mL) and 1,2-dimethoxyethane (40 mL) was stirred for 15 hours at 80°C. Water was added to the reaction solution, which was then extracted with ethyl acetate. The extract was washed with saturated sodium chloride solution and dried over anhydrous magnesium sulfate. The solvent was evaporated under reduced pressure, and the residue was purified by basic silica gel column chromatography and recrystallized from ethyl acetate-hexane to obtain ethyl 6-(4-chlorophenyl)-5-methylimidazo[1,2-a] pyridine-2-carboxylate (0.42 g) as colorless crystals.
   ¹H-NMR (300MHz, CDCl₃); δ 1.47 (3H, t, J=7.2Hz), 2.56 (3H, s), 4.49 (2H, q, J=7.2Hz), 7.23 (1H, d, J=9.6Hz), 7.29 (2H, d, J=8.7Hz), 7.46 (2H, d, J=8.7Hz), 7.65 (1H, d, J=9.6Hz), 8.19 (1H, s)
(3) 1 N Sodium hydroxide aqueous solution (2 mL) and water (10 mL) were added at 60°C to a mixed tetrahydrofuran-ethanol solution (5 mL-2 mL) of the compound (370 mg) obtained in (2), and stirred for 20 minutes at the same temperature. The reaction solution was neutralized with 1 N hydrochloric acid, and the precipitated crystals were filtered out and washed successively with water, methanol and diethyl ether to obtain the title compound (304 mg) as colorless crystals.
   ¹H-NMR (300MHz, DMSO-d₆); δ 2.57 (3H, s), 7.33 (1H, d, J=9.3Hz), 7.47 (2H, d, J=8.4Hz), 7.57 (2H, d, J=8.4Hz), 7.61 (1H, d, J=9.3Hz), 8.45 (1H, s)

### Example A41

### 6-(4-Chlorophenyl)-8-methylimidazo[1,2-a]pyridine-2-carboxylic acid

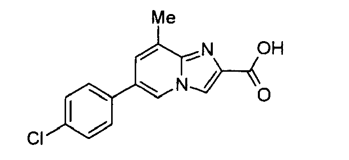

(1) A 1,2-dimethoxyethane solution (60 mL) of 2-amino-5-bromo-3-methylpyridine (2.50 g) and ethyl bromopyruvate (2.70 g) was stirred for 2.5 hours at room temperature. The solvent was evaporated under reduced pressure, and the residue was filtered out, suspended in ethanol (60 mL) and refluxed for 2 hours. The reaction solution was concentrated under reduced pressure, and the residue was neutralized with saturated aqueous sodium hydrogencarbonate solution and extracted with ethyl acetate-tetrahydrofuran. The extract was washed with saturated sodium chloride solution and dried over anhydrous magnesium sulfate. The solvent was evaporated under reduced pressure, and the residue was recrystallized from ethyl acetate-hexane to obtain ethyl 6-bromo-8-methylimidazo[1,2-a]pyridine-2-carboxylate (2.31 g) as pale yellow crystals.
   ¹H-NMR (300MHz, CDCl₃); δ 1.43 (3H, t, J=7.2Hz), 2.66 (3H, s), 4.46 (2H, q, J=7.2Hz), 7.12-7.14 (1H, m), 8.11 (1H, s), 8.15-8.17 (1H, m)
(2) A mixture of the compound (2.00 g) obtained in (1), 4-chlorophenylboronic acid (1.32 g), tetrakis(triphenylphosphine)palladium (0) (408 mg), 2 M sodium carbonate aqueous solution (7.06 mL) and 1,2-dimethoxyethane (40 mL) was stirred for 16 hours at 80°C. Water was added to the reaction solution, which was then extracted with ethyl acetate-tetrahydrofuran. The extract was washed with saturated sodium chloride solution and dried over anhydrous magnesium sulfate. The solvent was evaporated under reduced pressure, and the residue was purified by basic silica gel column chromatography and recrystallized from ethyl acetate-hexane to obtain ethyl 6-(4-chlorophenyl)-8-methylimidazo[1,2-a]pyridine-2-carboxylate (0.72 g) as colorless crystals.
   ¹H-NMR (300MHz, CDCl₃); δ 1.45 (3H, t, J=7.2Hz), 2.72 (3H, s), 4.48 (2H, q, J=7.2Hz), 7.25 (1H, s), 7.45 (2H, d, J=8.7Hz), 7.49 (2H, d, J=8.7Hz), 8.14 (1H, s), 8.21 (1H, s)
(3) 1 N Sodium hydroxide aqueous solution (3 mL) and water (15 mL) were added at 60°C to a mixed tetrahydrofuran-ethanol solution (15 mL-5 mL) of the compound (670 mg) obtained in (2), and stirred for 1 hour at the same temperature. The reaction solution was neutralized with 1 N hydrochloric acid, and the precipitated crystals were filtered out and washed successively with water, methanol and diethyl ether to obtain the title compound (550 mg) as colorless crystals.
   ¹H-NMR (300MHz, DMSO-d₆);δ 2.56 (3H, s), 7.53 (1H, s), 7.57 (2H, d, J=8.4Hz), 7.74 (2H, d, J=8.4Hz), 8.44 (1H, s), 8.80 (1H, s)

### Example A42

### 8-[(4-Chlorobenzyl)oxy]imidazo[1,2-a]pyridine-2-carboxylic acid

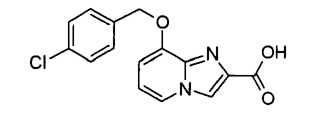

(1) Sodium hydride (660 mg) was added with ice cooling to a mixture of 2-amino-3-hydroxypyridine (1.65 g) and N,N-dimethylformamide (50 mL), and stirred for 20 minutes at room temperature. Next, 4-chlorobenzyl bromide (2.54 g) was added and stirred for 30 minutes at room temperature and then stirred for 30 minutes at 60°C. Water was added to the reaction solution, which was then extracted with diethyl ether. The extract was washed with saturated sodium chloride solution and dried over anhydrous magnesium sulfate. The solvent was evaporated under reduced pressure, and the residue was purified by silica gel column chromatography and recrystallized from ethyl acetate-hexane to obtain 2-amino-3-[(4-chlorobenzyl)oxy]pyridine (2.22 g) as pale yellow crystals.
   ¹H-NMR (300MHz, CDCl₃); δ 4.66 (2H, br s), 5.04 (2H, s), 6.59 (1H, dd, J=5.1, 7.8Hz), 6.93 (1H, dd, J=1.5, 7.8Hz), 7.32-7.41 (4H, m), 7.69 (1H, dd, J=1.5, 5.1Hz)
(2) A 1,2-dimethoxyethane solution (50 mL) of the compound obtained in (1) (2.18 g) and ethyl bromopyruvate (1.95 g) was stirred for 2.5 hours at room temperature. The solvent was evaporated under reduced pressure, and the residue was filtered out, suspended in ethanol (50 mL) and refluxed for 4 hours. The reaction solution was concentrated under reduced pressure, and the residue was neutralized with saturated aqueous sodium hydrogencarbonate solution and extracted with chloroform. The extract was washed with saturated sodium chloride solution and dried over anhydrous magnesium sulfate. The solvent was evaporated under reduced pressure, the residue was purified by silica gel column chromatography, and the resulting crystals were washed with ethyl acetate-hexane to obtain ethyl 8-[(4-chlorobenzyl)oxy]imidazo[1,2-a]pyridine-2-carboxylate (1.69 g) as colorless crystals.
   ¹H-NMR (300MHz, CDCl₃); δ 1.43 (3H, t, J=7.2Hz), 4.45 (2H, q, J=7.2Hz), 5.31 (2H, s), 6.46 (1H, d, J=7.8Hz), 6.70 (1H, dd, J=6.6, 7.8Hz), 7.34 (2H, d, J=8.7Hz), 7.43 (2H, d, J=8.7Hz), 7.76 (1H, d, J=6.6Hz), 8.17 (1H, s)
(3) 1 N Sodium hydroxide aqueous solution (5 mL) and water (30 mL) were added at 60°C to a mixed tetrahydrofuran-ethanol solution (20 mL-10 mL) of the compound (500 mg) obtained in (2), and stirred for 30 minutes at the same temperature. The reaction solution was neutralized with 1 N hydrochloric acid, and the precipitated crystals were filtered out and washed successively with water, methanol and diethyl ether to obtain the title compound (353 mg) as colorless crystals.
   ¹H-NMR (300MHz, DMSO-d₆); δ 5.60 (2H, s), 6.80 (1H, d, J=7.5Hz), 6.88 (1H, dd, J=6.6, 7.5Hz), 7.49 (2H, d, J=8.4Hz), 7.55 (2H, d, J=8.4Hz), 8.18 (1H, d, J=6.6Hz), 8.48 (1H, s)

### Example A43

### 6-(4-Chlorophenyl)-7-methylimidazo[1,2-a]pyridine-2-carboxylic acid

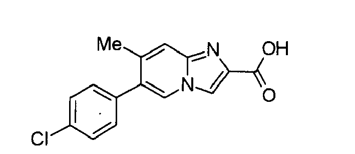

(1) A 1,2-dimethoxyethane solution (70 mL) of 2-amino-5-bromo-3-methylpyridine (4.00 g) and ethyl bromopyruvate (4.38 g) was stirred for 2.5 hours at room temperature. The solvent was evaporated under reduced pressure, and the residue was filtered out, suspended in ethanol (60 mL) and refluxed for 3 hours. The reaction solution was concentrated under reduced pressure, and the residue was neutralized with saturated aqueous sodium hydrogencarbonate solution and extracted with chloroform. The extract was washed with saturated sodium chloride solution, dried over anhydrous magnesium sulfate and passed through silica gel. The solvent was evaporated under reduced pressure, and the residue was recrystallized from ethyl acetate to obtain ethyl 6-bromo-7-methylimidazo[1,2-a]pyridine-2-carboxylate (3.57 g) as colorless crystals.
   ¹H-NMR (300MHz, CDCl₃); δ 1.44 (3H, t, J=7.2Hz), 2.46 (3H, s), 4.45 (2H, q, J=7.2Hz), 7.52 (1H, s), 8.08 (1H, s), 8.32 (1H, s)
(2) A mixture of the compound (1.5 g) obtained in (1), 4-chlorophenylboronic acid (1.25 g), palladium acetate (30 mg), 2-(dicyclohexylphosphino)biphenyl (94 mg), potassium fluoride (926 mg) and toluene (20 mL) was exposed to microwaves for 5 minutes at 160°C in two exposures. Water was added to the reaction solution, which was then extracted with ethyl acetate. The extract was washed with saturated sodium chloride solution and dried over anhydrous magnesium sulfate. The solvent was evaporated under reduced pressure, and the residue was purified by basic silica gel column chromatography and recrystallized from ethyl acetate-hexane to obtain ethyl 6-(4-chlorophenyl)-7-methylimidazo[1,2-a]pyridine-2-carboxylate (0.78 g) as colorless crystals.
   ¹H-NMR (300MHz, CDCl₃); δ 1.45 (3H, t, J=7.2Hz), 2.24 (3H, s), 4.46 (2H, q, J=7.2Hz), 7.27 (2H, d, J=8.4Hz), 7.44 (2H, d, J=8.4Hz), 7.52 (1H, s), 7.94 (1H, s), 8.12 (1H, s)
(3) 1 N Sodium hydroxide aqueous solution (5 mL) and water (20 mL) were added at 60°C to a mixed tetrahydrofuran-ethanol solution (10 mL-5 mL) of the compound (700 mg) obtained in (2), and stirred for 1 hour at the same temperature. The reaction solution was neutralized with 1 N hydrochloric acid, and the precipitated crystals were filtered out and washed successively with water, methanol and diethyl ether to obtain the title compound (550 mg) as colorless crystals.
   ¹H-NMR (300MHz, DMSO-d₆); δ 2.23 (3H, s), 7.48 (2H, d, J=8.7Hz), 7.53 (1H, s), 7.56 (2H, d, J=8.7Hz), 8.39 (1H, s), 8.46 (1H, s)

### Example A44

### 6-(4-Chlorophenyl)-5,7-dimethylimidazo[1,1-a]pyridine-2-carboxylic acid

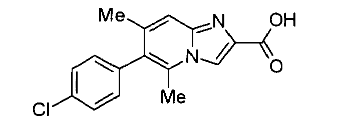

(1) A 1,2-dimethoxyethane solution (70 mL) of 2-amino-5-bromo-3-methylpyridine (4.00 g) and ethyl bromopyruvate (4.23 g) was stirred for 3 hours at room temperature. The solvent was evaporated under reduced pressure, and the residue was filtered out, suspended in ethanol (70 mL) and refluxed for 2 hours. The reaction solution was concentrated under reduced pressure, and the residue was neutralized with saturated aqueous sodium hydrogencarbonate solution and extracted with chloroform. The extract was washed with saturated sodium chloride solution, dried over anhydrous magnesium sulfate and passed through silica gel. The solvent was evaporated under reduced pressure, and the residue was recrystallized from ethyl acetate to obtain ethyl 6-bromo-5,7-dimethylimidazo[1,2-a]pyridine-2-carboxylate (2.81 g) as pale yellow crystals.
   ¹H-NMR (300MHz, CDCl₃); δ 1.45 (3H, t, J=7.2Hz), 2.49 (3H, s), 2.80 (3H, s), 4.47 (2H, q, J=7.2Hz), 7.46 (1H, s), 8.08 (1H, s)
(2) A mixture of the compound (1.8 g) obtained in (1), 4-chlorophenylboronic acid (1.42 g), palladium acetate (68 mg), 2-(dicyclohexylphosphino)biphenyl (184 mg), triethylamine (2.52 mL) and N,N-dimethylformamide (15 mL) was exposed to microwaves for 5 minutes at 180°C in two exposures. Water was added to the reaction solution, which was then extracted with diethyl ether. The extract was washed with saturated sodium chloride solution and dried over anhydrous magnesium sulfate. The solvent was evaporated under reduced pressure, and the residue was purified by basic silica gel column chromatography and recrystallized from ethyl acetate-hexane to obtain ethyl 6-(4-chlorophenyl)-5,7-dimethylimidazo[1,2-a]pyridine-2-carboxylate (0.64 g) as pale yellow crystals.
   ¹H-NMR (300MHz, CDCl₃); δ 1.46 (3H, t, J=7.2Hz), 2.07 (3H, d, J=0.6Hz), 2.34 (3H, s), 4.48 (2H, q, J=7.2Hz), 7.15 (2H, d, J=8.4Hz), 7.43-7.50 (3H, m), 8.08 (1H, d, J=0.6Hz)
(3) 1 N Sodium hydroxide aqueous solution (2 mL) and water (10 mL) were added at 60°C to a mixed tetrahydrofuran-ethanol solution (5 mL-5 mL) of the compound (600 mg) obtained in (2), and stirred for 1.5 hours at the same temperature. The reaction solution was neutralized with 1 N hydrochloric acid, and the precipitated crystals were filtered out and washed successively with water, methanol and diethyl ether to obtain the title compound (460 mg) as colorless crystals.
   ¹H-NMR (300MHz, DMSO-d₆); δ 2.03 (3H, s), 2.34 (3H, s), 7.33 (2H, d, J=8.4Hz), 7.47 (1H, s), 7.57 (2H, d, J=8.4Hz), 8.33 (1H, s)

### Example A45

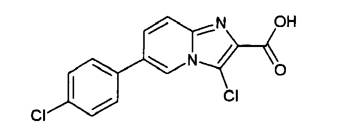

### 3-Chloro-6-(4-chlorophenyl)imidazo[1,2-a]pyridine-2-carboxylic acid

(1) A mixture of ethyl 6-(4-chlorophenyl)imidazo[1,2-a]pyridine-2-carboxylate (400 mg), N-chlorosuccinimide (190 mg), 2,2'-azobisisobutyronitrile (44 mg) and ethanol (8 mL) was exposed to microwaves for 5 minutes at 150°C. Water was added to the reaction solution, which was then extracted with ethyl acetate. The extract was washed with saturated sodium chloride solution and dried over anhydrous magnesium sulfate. The solvent was evaporated under reduced pressure and the residue was purified by silica gel column chromatography to obtain ethyl 3-chloro-6-(4-chlorophenyl)imidazo[1,2-a]pyridine-2-carboxylate (272 mg) as colorless crystals.
   ¹H-NMR (300MHz, CDCl₃); δ 1.48 (3H, t, J=7.2Hz), 4.51 (2H, q, J=7.2Hz), 7.25-7.57 (5H, m), 7.76 (1H, dd, J=0.9, 9.6Hz), 8.26 (1H, dd, J=0.9, 1.8Hz)
(2) 1 N Sodium hydroxide aqueous solution (1.5 mL) and water (10 mL) were added at 60°C to a mixed tetrahydrofuran-ethanol solution (5 mL-5 mL) of the compound (1.00 g) obtained in (1), and stirred for 1 hour at the same temperature. The reaction solution was neutralized with 1 N hydrochloric acid, and the precipitated crystals were filtered out and washed successively with water, methanol and diethyl ether to obtain the title compound (198 mg) as colorless crystals. ¹H-NMR (300MHz, DMSO-d₆);δ 7.59 (2H, d, J=8.7Hz), 7.78-7.83 (2H, m), 7.86 (2H, d, J=8.7Hz), 8.58-8.61 (1H, m), 13.22 (1H, br s)

### Example A46

### 3-Chloro-2-(4-chlorophenyl)imidazo[1,2-a]pyridine-6-carboxylic acid

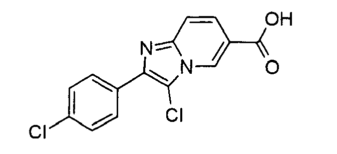

(1) A mixture of ethyl 2-(4-chlorophenyl)imidazo[1,2-a]pyridine-6-carboxylate (297 mg), N-chlorosuccinimide (152 mg) and methanol (8 mL) was exposed to microwaves for 5 minutes at 100°C. This was cooled to room temperature, and the precipitated crystals were filtered out to obtain methyl 3-chloro-2-(4-chlorophenyl)imidazo[1,2-a]pyridine-6-carboxylate (275 mg) as colorless crystals.
   ¹H-NMR (300MHz, CDCl₃); δ 4.00 (3H, s), 7.47 (2H, d, J=8.7Hz), 7.63 (1H, d, J=9.6Hz), 7.82 (1H, dd, J=1.5, 9.6Hz), 8.11 (2H, d, J=8.7Hz), 8.89 (1H, s)
(2) Lithium hydroxide monohydrate (100 mg) and water (10 mL) were added to a mixed tetrahydrofuran-ethanol solution (10 mL-10 mL) of the compound (275 mg) obtained in (1), and stirred for 30 minutes at 60°C. The reaction solution was neutralized with I N hydrochloric acid, and the precipitated crystals were filtered out and washed successively with water, methanol and diethyl ether to obtain the title compound (222 mg) as colorless crystals.
   ¹H-NMR (300MHz, DMSO-d₆); δ 7.61 (2H, d, J=8.7Hz), 7.74 (1H, dd, J=0.9, 9.3Hz), 7.79 (1H, dd, J=1.5, 9.3Hz), 8.14 (2H, d, J=8.7Hz), 8.80 (1H, dd, J=0.9, 1.5Hz), 13.58 (1H, br s)

### Example A47

### 2-(4-Chlorophenyl)-3-methylimidazo[1,2-a]pyridine-6-carboxylic acid

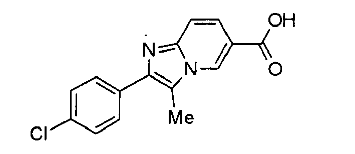

(1) A chloroform solution (75 mL) of 4'-chloropropiophenone (2.41 g) was added to an ethyl acetate suspension (150 mL) of copper bromide (9.48 g), and heated and stirred for 16 hours. The reaction solution was Celite filtered, and the filtrate was concentrated under reduced pressure. The residue was purified by silica gel column chromatography to obtain 2-bromo-1-(4-chlorophenyl)propan-1-one (1.52 g) as a pale yellow oil.
   ¹H-NMR (300MHz, CDCl₃); δ 1.90 (3H, d, J=6.6Hz), 5.22 (1H, q, J=6.6Hz), 7.47 (2H, d, J=8.7Hz), 7.97 (2H, d, J=8.7Hz)
(2) An ethanol solution (50 mL) of the compound (1.52 g) obtained in (1) and methyl 2-aminopyridine-5-carboxylate (0.93 g) was refluxed for 44 hours. The reaction solution was neutralized with saturated aqueous sodium hydrogencarbonate solution, and extracted with dichloromethane. The extract was washed with saturated sodium chloride solution and dried over anhydrous magnesium sulfate. The solvent was evaporated under reduced pressure, and the residue was purified by silica gel column chromatography and recrystallized from ethyl acetate-hexane to obtain methyl 2-(4-chlorophenyl)-3-methylimidazo[1,2-a]pyridine-6-carboxylate (0.68 g) as pale yellow crystals.
   ¹H-NMR (300MHz, CDCl₃); δ 2.70 (3H, s), 3.98 (3H, s), 7.46 (2H, d, J=8.4Hz), 7.62 (1H, d, J=9.6Hz), 7.72-7.78 (3H, m), 8.72 (1H, s)
(3) 1 N Sodium hydroxide aqueous solution (3 mL) and water (15 mL) were added at 60°C to a mixed tetrahydrofuran-methanol solution (20 mL-10 mL) of the compound (500 mg) obtained in (2), and stirred for 30 minutes at the same temperature. The reaction solution was neutralized with 1 N hydrochloric acid, and the precipitated crystals were filtered out and washed successively with water, methanol and diethyl ether to obtain the title compound (442 mg) as colorless crystals.
   ¹H-NMR (300MHz, DMSO-d₆); δ 2.71 (3H, s), 7.55 (2H, d, J=8.4Hz), 7.62 (1H, d, J=9.3Hz), 7.67 (1H, d, J=9.3Hz), 8.85 (1H, s), 13.32 (1H, br s)

### Example B1

### 5-(4-Chlorophenyl)-2-[(cyclopentylcarbonyl)amino]indan-2-carboxylic acid

0.5 ml of a dichloromethane and N,N-dimethylformamide (5:2) solution of cyclopentanecarboxylic acid (0.2 M) and 0.5 ml of a dichloromethane solution of 1-ethyl-3-(3-dimethylaminopropyl)carbodiimide hydrochloride (0.24 M) and 1-hydroxybenzotriazole (0.24 M) were added to 0.5 ml of a dichloromethane and N,N-dimethylformamide (5:2) solution of ethyl 2-amino-5-(4-chlorophenyl)indan-2-carboxylate (0.12 M), and shaken overnight at room temperature. Aqueous sodium hydrogencarbonate solution was added, and the mixture was extracted with dichloromethane. The organic layer was passed through a PTFE tube (polytetrafluoroethylene film processed tube) to obtain a solution containing the target compound, and the solvent was evaporated. Methanol (1 ml), THF (1 ml) and 1 N sodium hydroxide aqueous solution (0.5 ml) were added to the residue, and shaken overnight at room temperature. 1 N hydrochloric acid (0.5 ml) was added, and the solvent was evaporated. The residue was purified by preparative HPLC to obtain 5-(4-chlorophenyl)-2-[(cyclopentylcarbonyl)amino]indan-2-carboxylic acid (19.8 mg) (Table 4).

### Examples B2 through B116

The compounds of Examples B2 through B116 were obtained by reacting various carboxylic acids and amines by methods similar to those of Example B1.

The compounds of Examples B8 and B14, B22 and B28, B68 and B74, B82 and B88, B96 and B102 and B110 and B116, respectively, were each obtained by isolating the 2-mercaptobenzothiazole and 2-mercaptobenzothiazole-eliminated forms by preparative HPLC. The compounds of Examples B29 and B30, B32 and B33, B35 and B36, B37 and B38, B39 and B40, B41 and B42 and B44 and B45, respectively, were each obtained by isolating the dimethyl amino and monomethylamino forms by preparative HPLC. The compounds of Examples B12 and B13, B26 and B27, B46 and B47, B59 and B60, B72 and B73, B86 and B87, B100 and B101 and B114 and B115, respectively, were each obtained by isolating the carboxylic acid and ester forms by preparative HPLC. The compounds of Examples B29 through B47 were obtained as trifluoracetic acid salts (Table 4 through Table 9).
¹H-NMR data for typical compounds are given in Table 10.

**(Table 4)**

| | | | | |
|---|---|---|---|---|
| 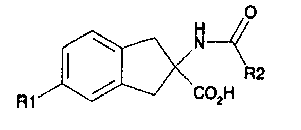 | | | | |

| Example | R1 | R2 | LC/MS | |
|---|---|---|---|---|
| | | | HPLC purity | m/e (M⁺+1) |
| B1 | 4-chlorophenyl | Cyclopentyl | 97% | 384 |
| B2 | 4-chlorophenyl | 2-pyrrolidon-5-yl | 98% | 399 |
| B3 | 4-chlorophenyl | 4-fluorobenzyl | 98% | 424 |
| B4 | 4-chlorophenyl | (2,5-dioxoimidazolidin-4-yl)methyl | 99% | 428 |
| B5 | 4-chlorophenyl | Benzyloxymethyl | 85% | 436 |
| B6 | 4-chlorophenyl | 2-(ethoxy)ethyl | 99% | 388 |
| B7 | 4-chlorophenyl | 2-(4-trifluorophenyl)ethyl | 100% | 488 |
| B8 | 4-chlorophenyl | 2-(1,3-benzothiazol-2-ylthio)ethyl | 99% | 509 |
| B9 | 4-chlorophenyl | 3-thienyl | 94% | 398 |
| B10 | 4-chlorophenyl | 4-methoxyphenyl | 98% | 422 |
| B11 | 4-chlorophenyl | 3-chlorophenyl | 99% | 426 |
| B12 | 4-chlorophenyl | Carboxymethyl | 96% | 374 |
| B13 | 4-chlorophenyl | Methoxycarbonylmethyl | 96% | 388 |
| B14 | 4-chlorophenyl | Vinyl | 96% | 342 |
| B15 | 4-fluorophenyl | Cyclopentyl | 100% | 368 |
| B16 | 4-fluorophenyl | 2-pyrrolidon-5-yl | 94% | 383 |
| B17 | 4-fluorophenyl | 4-fluorobenzyl | 99% | 408 |
| B18 | 4-fluorophenyl | (2,5-dioxoimidazolidin-4-yl)methyl | 93% | 412 |
| B19 | 4-fluorophenyl | Benzyloxymethyl | 98% | 420 |

**(Table 5)**

| | | | | |
|---|---|---|---|---|
| B20 | 4-fluorophenyl | 2-(ethoxy)ethyl | 99% | 372 |
| B21 | 4-fluorophenyl | 2-(4-trifluorophenyl)ethyl | 98% | 472 |
| B22 | 4-fluorophenyl | 2-(1,3-benzothiazol-2-ylthio)ethyl | 98% | 493 |
| B23 | 4-fluorophenyl | 3-thienyl | 98% | 382 |
| B24 | 4-fluorophenyl | 4-methoxyphenyl | 100% | 406. |
| B25 | 4-fluorophenyl | 3-chlorophenyl | 99% | 410 |
| B26 | 4-fluorophenyl | Carboxymethyl | 97% | 358 |
| B27 | 4-fluorophenyl | Methoxycarbonylmethyl | 96% | 372 |
| B28 | 4-fluorophenyl | Vinyl | 97% | 326 |
| B29 | 4-dimethylaminophenyl | Cyclopentyl | 82% | 393 |
| B30 | 4-methylaminophenyl | Cyclopentyl | 99% | 379 |
| B31 | 4-dimethylaminophenyl | 2-pyrrolidone-5-yl | 84% | 408 |
| B32 | 4-dimethylaminophenyl | 4-fluorobenzyl | 91% | 433 |
| B33 | 4-methylaminophenyl | 4-fluorobenzyl | 100% | 419 |
| B34 | 4-dimethylaminophenyl | (2,5-dioxoimidazolidin-4-yl)methyl | 88% | 437 |
| B35 | 4-dimethylaminophenyl | Benzyloxymethyl | 85% | 445 |
| B36 | 4-methylaminophenyl | Benzyloxymethyl | 92% | 431 |
| B37 | 4-dimethylaminophenyl | 2-(ethoxy)ethyl | 92% | 397 |
| B38 | 4-methylaminophenyl | 2-(ethoxy)ethyl | 92% | 383 |
| B39 | 4-dimethylaminophenyl | 2-(4-trifluorophenyl)ethyl | 87% | 497 |
| B40 | 4-methylaminophenyl | 2-(4-trifluorophenyl)ethyl | 95% | 483 |
| B41 | 4-dimethylaminophenyl | 3-thienyl | 95% | 407 |
| B42 | 4-methylaminophenyl | 3-thienyl | 100% | 393 |
| B43 | 4-dimethylaminophenyl | 4-methoxyphenyl | 87% | 431 |
| B44 | 4-dimethylaminophenyl | 3-chlorophenyl | 95% | 435 |
| B45 | 4-methylaminophenyl | 3-chlorophenyl | 100% | 421 |

**(Table 6)**

| | | | | |
|---|---|---|---|---|
| B46 | 4-dimethylaminophenyl | Carboxymethyl | 92% | 383 |
| B47 | 4-dimethylaminophenyl | Methoxycarbonylmethyl. | 85% | 397 |
| B48 | 3-acetoamidophenyl | Cyclopentyl | 91% | 407 |
| B49 | 3-acetoamidophenyl | 2-pyrrolidone-5-yl | 100% | 422 |
| B50 | 3-acetoamidophenyl | 4-fluorobenzyl | 99% | 447 |
| B51 | 3-acetoamidophenyl | (2,5-dioxoimidazolidin-4-yl)methyl | 95% | 451 |
| B52 | 3-acetoamidophenyl | Benzyloxymethyl | 100% | 459 |
| B53 | 3-acetoamidophenyl | 2-(ethoxy)ethyl | 96% | 411 |
| B54 | 3-acetoamidophenyl | 2-(4-trifluorophenyl)ethyl | 93% | 511 |
| B55 | 3-acetoamidophenyl | 2-(1,3-benzothiazol-2-ylthio)ethyl | 99% | 532 |
| B56 | 3-acetoamidophenyl | 3-thienyl | 93% | 421 |
| B57 | 3-acetoamidophenyl | 4-methoxyphenyl | 98% | 445 |
| B58 | 3-acetoamidophenyl | 3-chlorophenyl | 99% | 449 |
| B59 | 3-acetoamidophenyl | Carboxymethyl | 98% | 397 |
| B60 | 3-acetoamidophenyl | Methoxycarbonylmethyl | 95% | 411 |
| B61 | 4-trifluoromethoxyphenyl | Cyclopentyl | 98% | 434 |
| B62 | 4-trifluoromethoxyphenyl | 2-pyrrolidone-5-yl | 96% | 449 |
| B63 | 4-trifluoromethoxyphenyl | 4-fluorobenzyl | 95% | 474 |
| B64 | 4-trifluoromethoxyphenyl | (2,5-dioxoimidazolidin-4-yl)methyl | 86% | 478 |
| B65 | 4-trifluoromethoxyphenyl | Benzyloxymethyl | 96% | 486 |

**(Table 7)**

| | | | | |
|---|---|---|---|---|
| B66 | 4-trifluoromethoxyphenyl | 2-(ethoxy)ethyl | 100% | 438 |
| B67 | 4-trifluoromethoxyphenyl | 2-(4-trifluorophenyl)ethyl | 99% | 538 |
| B68 | 4-trifluoromethoxyphenyl | 2-(1,3-benzothiazol-2-ylthio)ethyl | 99% | 559 |
| B69 | 4-trifluoromethoxyphenyl | 3-thienyl | 99% | 448 |
| B70 | 4-trifluoromethoxyphenyl | 4-methoxyphenyl | 100% | 472 |
| B71 | 4-trifluoromethoxyphenyl | 3-chlorophenyl | 100% | 476 |
| B72 | 4-trifluoromethoxyphenyl | Carboxymethyl | 98% | 424 |
| B73 | 4-trifluoromethoxyphenyl | Methoxycarbonylmethyl | 90% | 438 |
| B74 | 4-trifluoromethoxyphenyl | Vinyl | 98% | 392 |
| B75 | 4-fluoro-3-chloromethoxyphenyl | Cyclopentyl | 99% | 402 |
| B76 | 4-fluoro-3-chloromethoxyphenyl | 2-pyrrolidone-5-yl | 96% | 417 |
| B77 | 4-fluoro-3-chloromethoxyphenyl | 4-fluorobenzyl | 97% | 442 |
| B78 | 4-fluoro-3-chloromethoxyphenyl | (2,5-dioxoimidazolidin-4-yl)methyl | 98% | 446 |
| B79 | 4-fluoro-3-chloromethoxyphenyl | Benzyloxymethyl | 99% | 454 |

**(Table 8)**

| | | | | |
|---|---|---|---|---|
| B80 | 4-fluoro-3-chloromethoxyphenyl | 2-(ethoxy)ethyl | 98% | 406 |
| B81 | 4-fluoro-3-chloromethoxyphenyl | 2-(4-trifluorophenyl)ethyl | 98% | 506 |
| B82 | 4-fluoro-3-chloromethoxyphenyl | 2-(1,3-benzothiazol-2-ylthio)ethyl | 100% | 527 |
| B83 | 4-fluoro-3-chloromethoxyphenyl | 3-thienyl | 88% | 416 |
| B84 | 4-fluoro-3-chloromethoxyphenyl | 4-methoxyphenyl | 100% | 440 |
| B85 | 4-fluoro-3-chloromethoxyphenyl | 3-chlorophenyl | 100% | 444 |
| B86 | 4-fluoro-3-chloromethoxyphenyl | Carboxymethyl | 98% | 392 |
| B87 | 4-fluoro-3-chloromethoxyphenyl | Methoxycarbonylmethyl | 91% | 406 |
| B88 | 4-fluoro-3-chloromethoxyphenyl | Vinyl | 99% | 360 |
| B89 | 4-methylphenyl | Cyclopentyl | 99% | 364 |
| B90 | 4-methylphenyl | 2-pyrrolidone-5-yl | 98% | 379 |
| B91 | 4-methylphenyl | 4-fluorobenzyl | 97% | 404 |
| B92 | 4-methylphenyl | (2,5-dioxoimidazolidin-4-yl)methyl | 80% | 408 |
| B93 | 4-methylphenyl | Benzyloxymethyl | 94% | 416 |
| B94 | 4-methylphenyl | 2-(ethoxy)ethyl | 100% | 368 |
| B95 | 4-methylphenyl | 2-(4-trifluorophenyl)ethyl | 99% | 468 |

**(Table 9)**

| | | | | |
|---|---|---|---|---|
| B96 | 4-methylphenyl | 2-(1,3-benzothiazol-2-ylthio)ethyl | 97% | 489 |
| B97 | 4-methylphenyl | 3-thienyl | 93% | 378 |
| B98 | 4-methylphenyl | 4-methoxyphenyl | 100% | 402 |
| B99 | 4-methylphenyl | 3-chlorophenyl | 99% | 406 |
| B100 | 4-methylphenyl | Carboxymethyl | 98% | 354 |
| B101 | 4-methylphenyl | Methoxycarbonylmethyl | 98% | 368 |
| B102 | 4-methylphenyl | Vinyl | 97% | 322 |
| B103 | 3-thienyl | Cyclopentyl | 100% | 356 |
| B104 | 3-thienyl | 2-pyrrolidone-5-yl | 89% | 371 |
| B105 | 3-thienyl | 4-fluorobenzyl | 98% | 396 |
| B106 | 3-thienyl | (2,5-dioxoimidazolidin-4-yl)methyl | 82% | 400 |
| B107 | 3-thienyl | Benzyloxymethyl | 100% | 408 |
| B108 | 3-thienyl | 2-(ethoxy)ethyl | 99% | 360 |
| B109 | 3-thienyl | 2-(4-trifluorophenyl)ethyl | 99% | 460 |
| B110 | 3-thienyl | 2-(1,3-benzothiazol-2-ylthio)ethyl | 92% | 481 |
| B111 | 3-thienyl | 3-thienyl | 99% | 370 |
| B112 | 3-thienyl | 4-methoxyphenyl | 100% | 394 |
| B113 | 3-thienyl | 3-chlorophenyl | 98% | 398 |
| B114 | 3-thienyl | Carboxymethyl | 87% | 346 |
| B115 | 3-thienyl | Methoxycarbonylmethyl | 98% | 360 |
| B116 | 3-thienyl | Vinyl | 96% | 314 |

**(Table 10)**

| Compound | ¹H NMR (300 MHz, CDCl₃+CD₃OD; δ ppm |
|---|---|
| Example B5 | 3.33-3.42 (2H, m), 3.74 (2H, dd, J=6.6, 17.0 Hz), 3.96 (2H, s), 4.53 (2H, s), 7.22-7.33 (6H, m), 7.37-7.43 (4H, m), 7.50 (2H, d, J=7.7 Hz) |
| Example B21 | 2.49 (2H, t, J=7.6 Hz), 2.98 (2H, t, J=7.6 Hz), 3.26 (2H, dd, J=8.1, 16.6 Hz), 3.68 (2H, dd, J=7.1, 16.7 Hz), 7.04-7.15 (3H, m), 7.21-7.31 (3H, m), 7.34-7.39 (2H, m), 7.45-7.54 (4H, m) |
| Example B38 | 1.11 (3H, t, J=7.0 Hz), 2.44 (2H, t, J=5.9 Hz), 2.97 (3H, s), 3.30 (2H, dd, J=4.8, 16.7 Hz), 3.39-3.47 (2H, m), 3.63 (2H, t, J=5.8 Hz), 3.71 (2H, dd, J=6.5, 16.7 Hz), 7.11 (2H, d, J=7.5 Hz), 7.23-7.28 91H, m), 7.34-7.40 (2H, m), 7.53 (2H, d, J=8.7 Hz) |
| Example B61 | 1.47-1.89 (8H, m), 2.49-2.61 (1H, m), 3.32 (2H, dd, J=5.6, 17.0 Hz), 3.71 (2H, dd, J=7.4, 16.7 Hz), 6.84 (1H, s), 7.23-7.30 (3H, m), 7.35-7.41 (2H, m), 7.57 (2H, dd, J=1.5, 8.5 Hz) |
| Example B84 | 3.51 (2H, dd, J=5.7, 16.8 Hz), 3.80 (2H, dd, J=7.3, 17.0 Hz), 3.85 (3H, s), 6.92 (2H, d, J=8.9 Hz), 7.20 (1H, t, J=8.8 Hz), 7.28-7.46 (4H, m), 7.60 (1H, dd, J=2.3, 7.0 Hz), 7.76 (2H, d, J=8.9 Hz) |
| Example B102 | 2.39 (3H, s), 3.33-3.40 (2H, m), 3.74 (2H, dd, J=6.9, 16.5 Hz), 5.65 (1H, dd, J=1.4, 10.1 Hz), 6.14 (1H, dd, J=10.2, 17.0 Hz), 6.29 (1H, d, J=17.0 Hz), 7.21-7.28 (3H, m), 7.38-7.49 (4H, m) |
| Example B115 | 3.27-3.36 (4H, m), 3.64-3.78 (5H, m), 7.23 (1H, d, J=8.1 Hz), 7.34-7.47 (4H, m), 8.05 (1H, s) |

### Example B117

### 2-{[(1,3-Benzothiazol-2-ylthio)acetyl]amino}-5-(4-chlorophenyl)indan-2-carboxylic acid

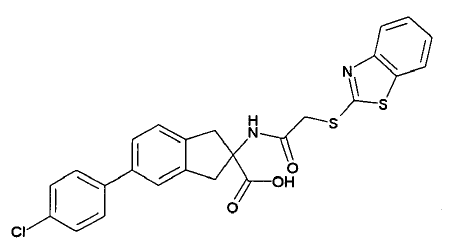

1) WSCD hydrochloride (115 mg, 0.60 mmol), HOBt (81 mg, 0.60 mmol) and triethylamine (168 µL, 1.2 mmol) were added with ice cooling to a DMF (1 mL)-dichloromethane (9 mL) solution of (1,3-benzothiazol-2-ylthio)acetic acid (114 mg, 0.50 mmol), and stirred for 30 minutes at room temperature. Ethyl 2-amino-5-(4-chlorophenyl)indan-2-carboxylate hydrochloride (105 mg, 0.30 mmol) was added and stirred overnight at room temperature. Water was added to the reaction mixture, which was then extracted twice with ethyl acetate. The organic layers were combined, washed with water and saturated sodium chloride solution, and dried over magnesium sulfate. The drying agent was filtered out, and the filtrate was concentrated under reduced pressure. The residue was purified by silica gel chromatography (eluate: ethyl acetate/hexane = 1/4 to 1/1) to obtain ethyl 2-{[(1,3-benzothiazol-2-ylthio)acetyl]amino}-5-(4-chlorophenyl)indan-2-carboxylate (90.3 mg, 58%) as a colorless oil.
   ¹H-NMR (300MHz, CDCl₃) δ: 8.49 (1H, s), 7.75-7.66 (2H, m), 7.44-7.41 (5H, m), 7.33-7.30 (1H, m), 7.23-7.22 (2H, d), 7.14-7.12 (1H, d), 4.27-4.19 (2H, q), 3.92 (2H, s), 3.69-3.60 (2H, m), 3.29-3.23 (2H, d), 1.30-1.24 (3H, t)
   LC-MS 523 [M+H]⁺
2) A 1N-NaOH aqueous solution (344 µL) was added with ice cooling to a methanol (1 mL)-THF (1 mL) solution of ethyl 2-{[(1,3-benzothiazol-2-ylthio)acetyl]amino}-5-(4-chlorophenyl) indan-2-carboxylate (90.3 mg, 0.172 mmol), and stirred overnight at room temperature. 1 N-hydrochloric acid (400 µL) was added, and the mixture was extracted twice with ethyl acetate. The organic layers were combined, washed with saturated sodium chloride solution, dried over magnesium sulfate, and concentrated under reduced pressure. The residue was recrystallized from a mixed ethyl acetate-hexane solvent to obtain the title compound (40.2 mg, 47%) as white crystals.
   ¹H-NMR (300MHz, DMSO-d₆) δ: 12.58 (1H, br s), 8.97 (1H, s), 7.97-7.95 (1H, d), 7.72-7.64 (3H, m), 7.51-7.29 (7H, m), 4.13 (2H, s), 3.58-3.49 (2H, m), 3.31-3.19 (2H, m,)
   LC-MS 495 [M+H]⁺
   Melting point: 205-207°C

### Example B118

### 2-{[3-(tert-Butoxycarbonylamino)propionyl]amino}-5-(4-chlorophenyl) indan-2-carboxylic acid

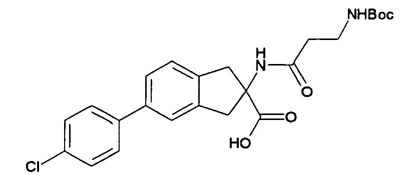

1) Ethyl 2-{[3-(tert-butoxycarbonylamino)propionyl]amino}-5-( 4-chlorophenyl)indan-2-carboxylate (146 mg, 100%) was obtained as a colorless solid by methods similar to those of Example B117-1) from Boc-β-Ala-OH (135 mg, 0.51 mmol) and ethyl 2-amino-5-(4-chlorophenyl)indan-2-carboxylate (105 mg, 0.30 mmol).
   ¹H-NMR (300MHz, DMSO-d₆) δ: 8.48 (1H, s), 7.67-7.63 (2H, m), 7.50-7.44 (4H, m), 7.30-7.28 (1H, d), 6.69-6.65 (1H, t), 4.27-4.20 (2H, m), 3.56-3.47 (2H, m), 3.25-3.16 (2H, m), 3.12-3.05 (2H,q), 2.24-2.19 (2H, t), 1.34 (9H, s), 1.23-l.18 (3H, m)
2) The title compound (93 mg, 67%) was obtained as white crystals by methods similar to those of Example B117-2) from ethyl 2-{[3-(tert-butoxycarbonylamino)propionyl]amino}-5-(4-chlorophenyl)indan-2-carboxylate (147 mg, 0.30 mmol).
   ¹H-NMR (300MHz, DMSO-d₆) δ: 12.42 (1H, s), 8.48 (1H, s), 7.67-7.63 (2H, m), 7.50-7.44 (4H, m), 7.30-7.28 (1H, d), 6.69-6.65 (1H, t), 3.56-3.47 (2H, m), 3.25-3.16 (2H, m), 3.12-3.05 (2H, q), 2.24-2.19 (2H, t), 1.34 (9H, s)
   LC-MS 481 [M+H]⁺
   Melting point: 212-214°C

### Example B119

### 2-{[N-(tert-Butoxycarbonyl)-D-tryptophyl]amino}-5-(4-chlorophenyl)indan-2-carboxylic acid

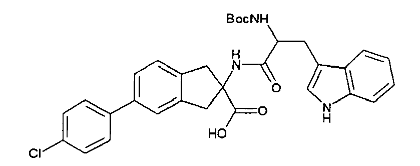

1) Ethyl 2-{[N-(tert-butoxycarbonyl)-D-tryptophyl]amino}-5-(4-chlorophenyl)indan-2-carboxylate (173 mg, 96%) was obtained as a colorless solid by methods similar to those of Example B117-1) from Boc-D-Trp-OH (155 mg, 0.51 mmol) and ethyl 2-amino-5-(4-chlorophenyl)indan-2-carboxylate (105 mg, 0.30 mmol).
   ¹H-NMR (300MHz, DMSO-d₆) δ: 10.78 (1H, s), 8.55 (1H, s), 7.68-7.45 (6H, m),7.32-7.29 (2H, d), 7.06-6.93 (3H, m), 6.63-6.60 (1H, d), 6.18-6.16 (1H, d), 4.27-4.20 (3H, m), 3.64-3.19 (4H, m), 3.06-2.82 (2H, m), 1.17 (9H, s)
2) The title compound (115 mg, 70%) was obtained as white crystals by methods similar to those of Example B117-2) from ethyl 2-{[N-(tert-butoxycarbonyl)-D-tryptophyl]amino}-5-(4-chlorophenyl)indan-2-carboxylate (170 mg, 0.28 mmol).
   ¹H-NMR (300MHz, DMSO-d₆) δ: 12.60 (1H, br s), 10.78 (1H, s), 8.55 (1H, s), 7.68-7.45 (6H, m),7.32-7.29 (2H, d), 7.06-6.93 (3H, m), 6.63-6.60 (1H, d), 6.18-6.16 (1H, d), 4.21 (1H, m), 3.64-3.19 (4H, m), 3.06-2.82 (2H ,m), 1.17 (9H, s)
   LC-MS 596 [M+H]⁺
   Melting point: 158°C

### Example B120

### 2-{[N-(tert-Butoxycarbonyl)phenylalanyl]amino}-5-(4-chlorophenyl)indan-2-carboxylic acid

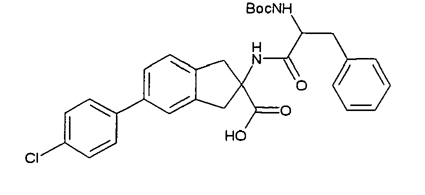

1) Ethyl 2-{[N-(tert-butoxycarbonyl)phenylalanyl]amino }-5-(4-chlorophenyl)indan-2-carboxylate (154 mg, 91%) was obtained as a colorless solid by methods similar to those of Example B117-1) from Boc-Phe-OH (135 mg, 0.51 mmol) and ethyl 2-amino-5-(4-chlorophenyl)indan-2-carboxylate (105 mg, 0.30 mmol).
   ¹H-NMR (300MHz, CDCl₃) δ: 7.68-7.25 (12H, m), 6.35 (1H, s), 5.37 (1H, br s), 5.16 (1H, br s), 4.27-4.20 (2H, m), 3.71-3.49 (2H, m), 3.38-3.26 (3H, m), 2.40-2.36 (2H, t), 1.42 (9H, s), 1.23-1.18 (3H, m)
2) The title compound (90 mg, 74%) was obtained as white crystals by methods similar to those of Example B117-2) from ethyl 2-{[N-(tert-butoxycarbonyl)phenylalanyl]amino}-5-(4-chlorophenyl)indan-2-carboxylate (128 mg, 0.23 mmol). ¹H-NMR (300MHz, DMSO-d₆) δ: 12.50 (1H, s), 8.53 (1H, s), 7.68-7.65 (2H, d), 7.51-7.47 (4H, m), 7.40-7.22 (6H, m), 6.96-6.94 (1H, d), 4.15 (1H, m), 3.58-3.14 (4H, m), 2.91-2.71 (2H, m), 1.25 (9H, s)
   LC-MS 557 [M+H]⁺
   Melting point: 140-142°C

### Example B121

### 5-(4-Chlorophenyl)-2-[(3-phenylalanyl)amino]indan-2-carboxylic acid hydrochloride

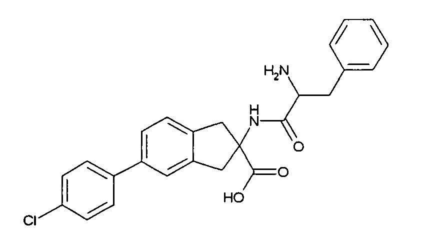

4 N HCl ethyl acetate solution (1mL) was added to ethyl acetate (1 mL) from 2-{[N-(tert-butoxycarbonyl)phenylalanyl]amino}-5-(4-chlorophenyl)indan-2-carboxylic acid (50 mg, 0.094 mmol), and stirred overnight at room temperature. The reaction mixture was concentrated under reduced pressure, and the residue was purified by preparative HPLC to obtain the title compound (12.4 mg, 30%) as white crystals.
¹H-NMR (300MHz, DMSO-d₆) δ: 12.50 (1H, s), 8.21 (1H, s), 7.69-7.66 (2H, d), 7.53-7.44 (4H, m), 7.30-7.18 (6H, m), 3.74 (3H, br s), 3.56-3.43 (3H,m), 3.32-3.21 (2H, m), 3.00-2.93 (4H, m), 2.70-2.66 (1H, m)
LC-MS 435 [M+H]⁺
Melting point: decomposes at 288-290°C

### Example B122

### 5-(4-Chlorophenyl)-2-(D-tryptophylamino)indan-2-carboxylic acid hydrochloride

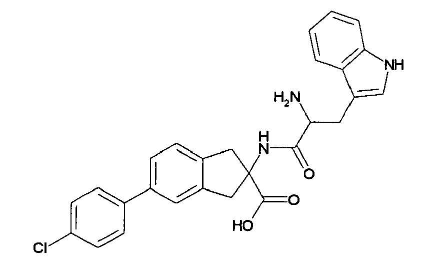

The title compound (23 mg, 48%) was obtained as white crystals by methods similar to those of Example B121 from 2-{[N-(tert-butoxycarbonyl)-D-tryptophyl]amino}-5-(4-chlorophenyl)indan-2-carboxylic acid (60 mg, 0.10 mmol).
¹H-NMR (300MHz, DMSO-d₆) δ: 11.0 (1H, s), 9.07 (1H, s), 8.14 (1H, s), 7.69-7.64 (3H, m), 7.52-7.47 (4H, m), 7.36-7.33 (2H, m), 7.20 (1H, d), 7.19-6. 97 (2H, m), 3.92-3.91 (1H, m), 3.64-3.07 (6H, m)
LC-MS 474 [M+H]⁺
Melting point: 168-170°C

### Example B123

### 5-(4-Chlorophenyl)-2-[(morpholine-4-ylacetyl)amino]indan-2-carboxylic acid hydrochloride

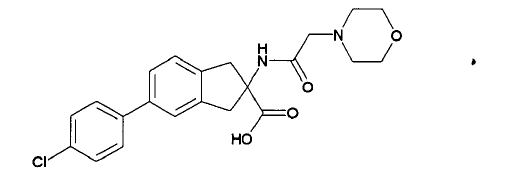

1) Bromoacetyl chloride (177 µL, 2.13 mmol) ethyl was added with ice cooling to a N,N-dimethylacetamide (2 mL) solution of 2-amino-5-(4-chlorophenyl)indan-2-carboxylate (500 mg, 1.42 mmol) and triethylamine (300 J.1L, 2.13 mmol), and stirred for 1 hour at room temperature. Ice water was added, and the mixture was extracted twice with ethyl acetate. The organic layers were combined, washed with 10% sodium bicarbonate solution and saturated sodium chloride solution, and dried by addition of magnesium sulfate. The drying agent was filtered out, and the filtrate was condensed under reduced pressure. The residue was purified by silica gel chromatography (eluate: ethyl acetate/hexane = 1/10 to 1/1) to obtain ethyl 2[(bromoacetyl)amino]-5-(4-chlorophenyl)indan-2-carboxylate (558 mg, 90%) as a colorless oil. ¹H-NMR (300MHz, CDCl₃) δ: 7.51-7.47 (2H, m), 7.41-7.37 (4H, m), 7.28-7.26 (1H, m), 7.08 (1H, s), 4.28-4.21 (2H, m), 4.01 (2H, s), 3.75-3.67 (2H, m), 3.42-3.33 (2H, m), 1.28-1.24 (3H, t) LC-MS 437 [M+H]⁺
2) A dichloromethane solution of ethyl 2[(bromoacetyl)amino]-5-(4-chlorophenyl)indan-2-carboxylate (218 mg, 0.5 mmol) and morpholine (300 µL) was stirred overnight at room temperature. The reaction mixture was poured into dichloromethane-water, and the organic layer was isolated. The organic layer was washed with sodium bicarbonate solution and saturated sodium chloride solution, and dried by addition of magnesium sulfate. The drying agent was filtered out, and the filtrate was concentrated under reduced pressure. The residue was purified by basic silica gel chromatography (eluate: ethyl acetate/hexane = 1/4 to 1/1) to obtain ethyl 5-(4-chlorophenyl)-2-[(morpholine-4-ylacetyl)amino]indan-2-carboxylate (210 mg, 95%) as a colorless oil.
   ¹H-NMR (300MHz, CDCl₃) δ : 7.57 (1H, s), 7.50-7.46 (2H, d), 7.41-7.38 (3H, m), 7.30-7.26 (2H, m), 4.27-4.13 (2H, q), 3.74-3.60 (6H, m), 3.38-3.31 (2H, m), 2.96 (2H, s), 2.50-2.47 (4H, t), 1.28-1.24 (3H, t) LC-MS 443 [M+H]⁺
3) 1 N-NaOH aqueous solution (474 µL) was added with ice cooling to a methanol (1 mL)-THF (1 mL) solution of ethyl 5-(4-chlorophenyl)-2-[(morpholine-4-ylacetyl)amino]indan-2-carboxylate (105 mg, 0.24 mmol), and stirred overnight at room temperature. The reaction solution was concentrated under reduced pressure, and 1 N-hydrochloric acid (900 µL) was added. The precipitated white solid was filtered out and washed with water to obtain the title compound (35.2 mg, 36%).
   ¹H-NMR (300MHz, CDCl₃ δ: 12.80 (1H, br s), 10.80 (1H, s), 9.56 (1H, s), 7.67-7.64 (2H, d), 7.51-7.46 (4H, m), 7.33-7.30 (1H, d), 3.95-3.85 (8H, m), 3.62-3.54 (2H, m), 3.54-3.29 (4H, m)
   LC-MS 415 [M+H]⁺
   Melting point: decomposes at 253-255°C

### Example B124

### 5-(4-Chlorophenyl)-2-{[(4-phenylpiperazine-1-yl)acetyl]amino}indan-2-carboxylic acid

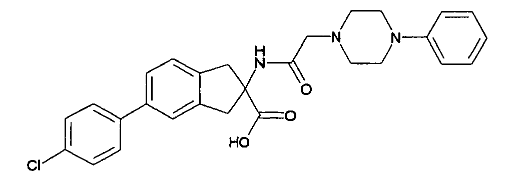

1) 4-Phenylpiperazine (110 µL, 0.72 mmol) and potassium carbonate (166 mg, 1.2 mmol) were added to a THF solution of ethyl 2-[(bromoacetyl)amino]-5-(4-chlorophenyl)indan-2-carboxylate (139 mg, 0.32 mmol), and stirred overnight at 40°C. Water was added, the reaction was completed, and the mixture was extracted twice with ethyl acetate. The organic layers were combined, washed with sodium bicarbonate solution and saturated sodium chloride solution, and dried by addition of magnesium sulfate. The drying agent was filtered out, and the filtrate was condensed under reduced pressure. The residue was purified by silica gel chromatography (eluate: ethyl acetate/hexane = 1/4 to 1/0) to obtain ethyl 5-(4-chlorophenyl)-2-{[(4-phenylpiperazine-1-yl)acetyl]amino}indan-2-carboxylate (115 mg, 74%) as a colorless oil.
   ¹H-NMR (300MHz, CDCl₃) δ: 7.64 (1H, s), 7.49-7.47 (2H, d), 7.40-7.37 (4H, m), 7.29-7.21 (3H, m), 6.89-6.83 (3H, m), 4.28-4.22 (2H, q), 3.74-3.66 (2H, m), 3.37-3.31 (2H, d), 3.09-3.02 (6H, m), 2.65-2.62 (4H, m), 1.30-1.25 (3H, t) LC-MS 518 [M+H]⁺
2) The title compound (27.7 mg, 26%) was obtained as white crystals by methods similar to those of Example B117-2) from ethyl 5-(4-chlorophenyl)-2-{[(4-phenylpiperazine-1-yl)acetyl]amino}indan-2-carboxylate (115 mg, 0.22 mmol).
   ¹H-NMR (300MHz, DMSO-d₆) δ: 12.40 (1H, s), 8.22 (1H, s), 7.68-7.65 (2H, d), 7.51-7.46 (4H, m), 7.32-7.29 (1H, d), 7.21-7.16 (2H, t), 6.90-6.87 (2H, d), 6.78-6.74 (1H, t), 3.58-3.49 (2H, m), 3.32-3.27 (2H, m), 3.09 (4H, s), 2.99 (2H, s), 2.57 (4H, s)
   LC-MS 490 [M+H]⁺
   Melting point: 247°C

### Example B125

### 2-(3-Aminopropionylamino)-5-(4-chlorophenyl)indan-2-carboxylic acid hydrochloride

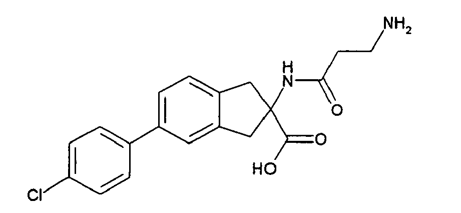

4 N HCl ethyl acetate solution (1 mL) was added to an ethyl acetate (1 mL) solution of 2-{[3-(tert-butoxycarbonylamino)propionyl]amino}-5-(4-chlorophenyl)indan-2-carboxylic acid (45 mg, 0.098 mmol), and stirred overnight at room temperature. The precipitated white solid was filtered out to obtain the title compound (28 mg, 72%).
¹H-NMR (300MHz, DMSO-d₆) δ: 12.60 (1H, br s), 8.77 (1H, s), 7.97 (3H, s), 7.67-7.64 (2H, d),7.50-7.45 (4H, m), 7.31-7.29 (1H, d), 3.66-3.50 (2H, m), 3.35-3.22 (4H, m), 2.96-2.92 (2H, t)
LC-MS: 359 [M+H]⁺
Melting point: Decomposes at 240-243°C

### Example B126

### 2-({[2-(Benzyloxy)phenyl]acetyl}amino)-5-(4-chlorophenyl)indan-2-carboxylic acid

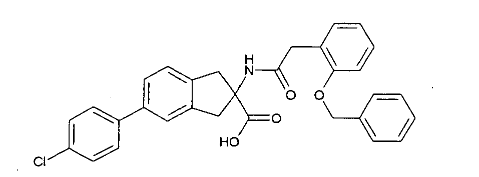

1) Ethyl 2-({[2-(benzyloxy)phenyl]acetyl}amino)-5-(4-chlorophenyl)indan-2-carboxylate (164 mg, 100%) was obtained as a colorless oil by methods similar to those of Example B117-1) from 2-(benzyloxy)phenylacetic acid (123 mg, 0.51 mmol) and ethyl 2-amino-5-(4-chlorophenyl) indan-2-carboxylate (105 mg, 0.30 mmol).
   ¹H-NMR (300MHz, CDCl₃) δ: 7.49-7.12 (14H, m), 7.00-6.48 (2H, m), 6.48 (1H, s), 5.01 (2H, s), 4.18-4.10 (2H, m), 3.59-3.47 (4H, m), 3.08-2.98 (2H, m), 1.11-1.06 (3H, t)
   LC-MS 540 [M+H]⁺
2) The title compound (87 mg, 70%) was obtained as white crystals by methods similar to those of Example B117-2) from ethyl 2-({[2-(benzyloxy)phenyl]acetyl}amino)-5-(4-chlorophenyl)indan-2-carboxylate (135 mg, 0.25 mmol).
   ¹H-NMR (300MHz, DMSO-d₆) δ: 12.40 (1H, s), 8.60 (1H, s), 7.66-7.63 (2H, d), 7.51-7.40 (5H, m), 7.32-7.14 (6H, m), 6.99-6.96 (1H, d), 6.88-6.85 (2H, d), 5.06 (2H, s), 3.62-3.41(4H, m), 3.26-3.18 (2H, m)
   LC-MS: 512 [M+H]⁺
   Melting point: 245°C
   Elemental analysis: Calcd. for C₃₁H₂₆NO₄Cl•0.5 H₂O: C 71.46, H 5.22, N 2.69; Found: C 71.72, H 5.25, N 2.47

### Example B127

### 5-(4-Chlorophenyl)-2-{[(4-chlorophenyl)acetyl]amino}indan-2-carboxylic acid

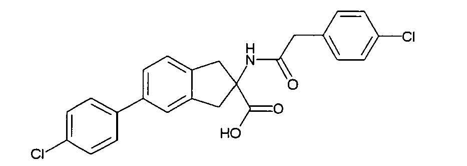

1) Ethyl 5-(4-chlorophenyl)-2-{[(4-chlorophenyl)acetyl]amino}indan-2-carboxylate (142 mg, 100%) was obtained as a colorless oil by methods similar to those of Example B117-1) from 4-chlorophenylacetic acid (85 mg, 0.51 mmol) and ethyl 2-amino-5-(4-chlorophenyl) indan-2-carboxylate (105 mg, 0.30 mmol).
   ¹H-NMR (300MHz, CDCl₃) δ: 7.51-7.49 (2H, d), 7.36-7.33 (2H, d), 7.23-7.18 (3H, m), 7.03-6.97 (4H, m), 5.99 (1H, s), 4.23-4.16 (2H, q), 3.70-3.61 (2H, m), 3.49 (2H, s), 3.28-3.20 (2H, m), 1.26-1.18 (3H, t)
   LC-MS: 468 [M+H]⁺
2) The title compound (82 mg, 85%) was obtained as white crystals by methods similar to those of Example B117-2) from ethyl 5-(4-chlorophenyl)-2-{ [(4-chlorophenyl)acetyl]amino}indan-2-carboxylate (102 mg, 0.22 mmol).
   ¹H-NMR (300MHz, DMSO-d₆) δ: 12.40 (1H, s), 8.72 (1H, s), 7.67-7.65 (2H, d), 7.51-7.45 (4H, m), 7.34-7.23 (5H, m), 3.57-3.49 (2H, m), 3.42 (2H, s), 3.25 (2H, m)
   LC-MS: 440 [M+H]⁺
   Melting point: 251°C

### Example B128

### 2-[({4-[bis(4-Fluorophenyl)methyl]piperazine-1-yl}acetyl)amino]-5-(4-chlorophenyl)indan-2-carboxylic acid

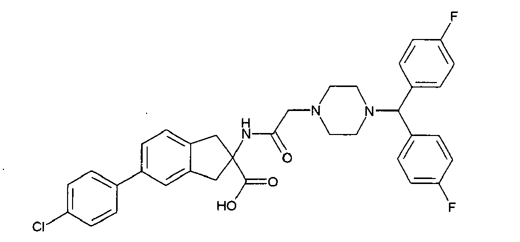

1) Ethyl 2-[({4-[bis(4-fluorophenyl)methyl]piperazine-1-yl}acetyl)amino]-5-(4-chlorophenyl)indan-2-carboxylate (283 mg, 100%) was obtained as a colorless oil by methods similar to those of Example B124-1) from ethyl 2-[(bromoacetyl)amino]-5-(4-chlorophenyl)indan-2-carboxylate (175 mg, 0.40 mmol) and 4-[bis(4-fluorophenyl)methyl]piperazine (232 mg, 0.80 mmol).
   ¹H-NMR (300MHz, DMSO-d₆) δ: 8.25 (1H, s), 7.63-7.60 (2H, m), 7.49-7.46 (3H, m), 7.43-7.39 (5H, m), 7.31-7.28 (1H, d), 7.13-7.07 (4H, t), 4.40 (1H, s), 4.31-4.02 (2H, m), 3.56-3.47 (2H, m), 3.28-3.20 (2H, m), 2.90 (2H, s), 2.44 (4H, br s), 2.23 (4H, br s), 1.13-1.11 (3H, t)
   LC-MS 644 [M+H]⁺
2) The title compound (219 mg, 95%) was obtained as white crystals by methods similar to those of Example B117-2) from ethyl 2-[({4-[bis(4-fluorophenyl)methyl]piperazine-1-yl} acetyl)amino]-5-(4-chlorophenyl)indan-2-carboxylate.
   ¹H-NMR (300MHz, DMSO-d₆) δ: 12.70 (1H, br s), 10.16 (1H, br s), 9.30 (1H, s), 7.66-7.64 (2H, d), 7.52-7.46 (8H, m), 7.33-7.30 (1H, d), 7.19-7.13 (4H, m), 4.56(1H, s), 3.92 (2H, s), 3.62-3.22 (7H, m), 2.77 (2H, br s), 2.42-2.27 (2H, m)
   LC-MS: 616 [M+H]⁺
   Melting point: 184°C

### Example B129

### 5-(4-Chlorophenyl)-2-({[6-(4-chlorophenyl)-3-methyl-1-benzofuran-2-yl]carbonyl}amino)indan-2-carboxylic acid

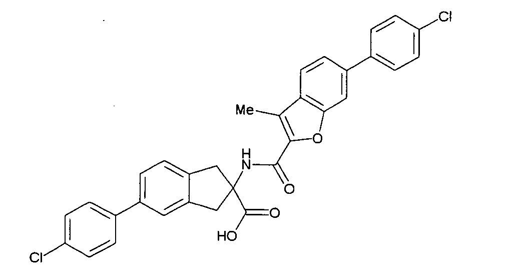

1) Ethyl 5-(4-chlorophenyl)-2-({[6-(4-chlorophenyl)-3-methyl-1-benzofuran-2-yl]carbonyl}amino)indan-2-carboxylate (174 mg, 100%) was obtained as a white solid by methods similar to those of Example B117-1) from 6-(4-chlorophenyl)-3-methyl-1-benzofuran-2-carboxylic acid (146 mg, 0.51 mmol) and ethyl 2-amino-5-(4-chlorophenyl)indan-2-carboxylate (105 mg, 0.30 mmol).
   ¹H-NMR (300MHz, DMSO-d₆) δ: 9.14(1H, s), 7.84-7.74 (4H, m), 7.69-7.64 (3H, m), 7.55-7.48 (6H, m), 7.35-7.33 (1H, d), 4.18-4.11 (2H, q), 3.68-3.51 (4H, m), 2.54 (3H, s), 1.18-1.14 (3H, t)
   LC-MS 584 [M+H]⁺
2) The title compound (93 mg, 72%) was obtained as white crystals by methods similar to those of Example B117-2) from ethyl 5-(4-chlorophenyl)-2-({[6-(4-chlorophenyl)-3-methyl-1-benzofuran-2-yl]carbonyl}amino)indan-2-carboxylate (136 mg, 0.23 mmol)
   ¹H-NMR (300MHz, DMSO-d₆) δ: 12.64 (1H, s), 8.97 (1H, s), 7.84-7.81 (2H, d), 7.77-7.74 (2H, d), 7.69-7.64 (3H, m), 7.55-7.49 (6H, m), 7.35-7.32 (1H, d), 3.68-3.50 (4H, m), 2.55 (3H, s)
   LC-MS 556 [M+H]⁺
   Melting point: 257°C
   Elemental analysis: Calcd. for C₃₂H₂₃NO₄Cl₂: C 69.07, H 4.17, N 2.52; Found: C 68.93, H 4.34, N 2.29

### Example B130

### 5-(4-Chlorophenyl)-2-{[(4-fluorobenzyl)sulfonyl]amino}indan-2-carboxylic acid

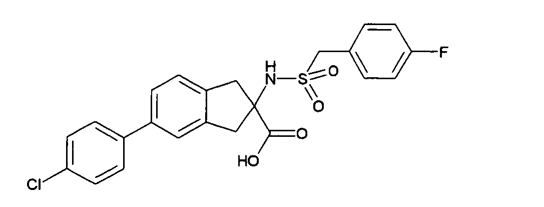

1) Triethylamine (0.3 mL) and (4-fluorophenyl)methanesulfonyl chloride (101 mg, 0.48 mmol) were added to a THF (5 mL)-dichloromethane (5 mL) solution of ethyl 2-amino-5-(4-chlorophenyl) indan-2-carboxylate hydrochloride (105 mg, 0.30 mmol), and stirred overnight at 40°C. 10% sodium bicarbonate solution was added to the reaction mixture, which was then extracted twice with ethyl acetate. The organic layers were combined, washed with 10% sodium bicarbonate solution and saturated sodium chloride solution, and dried by addition of magnesium sulfate. The drying agent was filtered out, and the mixture was concentrated under reduced pressure. The residue was purified by silica gel chromatography (elution solvent: ethyl acetate/hexane = 1/10-1/1) to obtain ethyl 5-(4-chlorophenyl)-2-{[(4-fluorobenzyl)sulfonyl]amino}indan-2-carboxylate (74 mg, 50%) as a colorless oil.
   ¹H-NMR (300MHz, DMSO-d₆) δ: 7.82 (1H, s), 7.69-7.66 (2H, d), 7.55-7.49 (4H, m), 7.34-7.32 (2H, d), 7.14-7.08 (4H, m), 4,27 (2H, s), 4.19-4.13 (2H, q), 3.54-3.44 (2H, t), 3.36-3.26 (2H, m), 1.23-1.18 (3H, t)
   LC-MS 510 [M+H]⁺
2) 1 N NaOH aqueous solution (300 µL) was added with ice cooling to a methanol (1 mL)-THF (1 mL) solution of ethyl 5-(4-chlorophenyl)-2-{[(4-fluorobenzyl)sulfonyl]amino}indan-2-carboxylate (74 mg, 0.15 mmol), and stirred overnight at room temperature. 1 N-hydrochloric acid (400 µL) was added, and the mixture was extracted twice with ethyl acetate. The organic layers were combined, washed with saturated sodium chloride solution and dried by addition of magnesium sulfate, and concentrated under reduced pressure. The residue was recrystallized from a mixed ethyl acetate-hexane solvent to obtain the title compound (50 mg, 72%) as white crystals.
   ¹H-NMR (300MHz, DMSO-d₆) δ: 12.9 (1H, s), 7.69-7.66 (3H, m), 7.54-7.48 (4H, m), 7.34-7.31 (1H, d), 7.15-7.02 (4H, m), 4.28 (2H, s), 3.54-3.45 (2H, m), 3.35-3.25 (2H, m)
   LC-MS 482 [M+H]⁺
   Melting point: 210-213°C
   Elemental analysis: Calcd. for C₂₃H₁₉NO₄SFCl**·**0.1 H₂O: C 59.59, H 4.22, N 3.02; Found: C 59.50, H 4.30, N 2.68

### Example B131

### 2-{[(4-Fluorophenyl)acetyl]amino}-5-(4-methoxyphenyl)indan-2-carboxylic acid

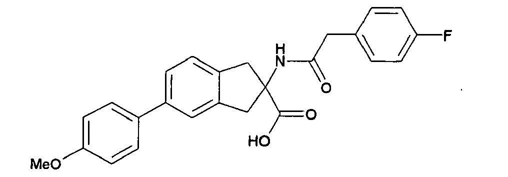

1) Ethyl 5-bromo-2-{[(4-fluorophenyl)acetyl]amino}indan-2-carboxylate (3.49 g, 83%) was obtained as a yellow solid by methods similar to those of Example B117-1) from 4-fluorophenylacetic acid (2.6 g, 17 mmol) and ethyl 2-amino-5-bromoindan-2-carboxylate (2.8 g, 10 mmol).
   ¹H-NMR (300MHz, CDCl₃) δ: 7.31-7.26 (2H, d), 7.21-7.18 (2H, m), 7.04-6.97 (3H, m), 5.99 (1H, s), 4.21-4.14 (2H, q), 3.61-3.48 (4H, m), 3.26-3.15 (2H, t), 1.21-1.17 (3H, t)
   LC-MS 421 [M+H]⁺
2) Ethyl 5-bromo-2-{[(4-fluorophenyl)acetyl]amino}indan-2-carboxylate (420 mg, 1.0 mmol), 4-methoxyphenyl boronic acid (182 mg, 1.2 mmol), 2 M potassium carbonate aqueous solution (1.0 mL) and ethanol (1.5 mL) were added to deaerated dimethoxyethane (7 mL), and stirred for 30 minutes at room temperature in an argon atmosphere, and tetrakis(triphenylphosphine)palladium (52 mg, 3%) was added and refluxed overnight. The reaction mixture was filtered, and the filtrate was diluted with ethyl acetate, washed with 10% sodium bicarbonate solution and saturated sodium chloride solution, and dried over magnesium sulfate. The drying agent was filtered out, and the solvent was evaporated. The residue was purified by silica gel chromatography (elution solvent: ethyl acetate/hexane = 1/10-1/1) to obtain ethyl 2-{[(4-fluorophenyl) acetyl]amino}-5-(4-methoxyphenyl)indan-2-carboxylate (269 mg, 64%) as a colorless oil.
   ¹H-NMR (300MHz, CDCl₃) δ: 7.51-7.49 (2H, d), 7.36-7.33 (2H, d), 7.23-7.18 (3H, m), 7.03-6.97 (4H, m), 5.99 (1H, s), 4.23-4.16 (2H, q), 3.85 (3H, s), 3.70-3.61 (2H, m), 3.49 (2H, s), 3.28-3.20 (2H, m), 1.26-1.18 (3H, t)
   LC-MS 448 [M+H]⁺
3) The title compound (195 mg, 80%) was obtained as white crystals by methods similar to those of Example B117-2) from ethyl 2-{ [(4-fluorophenyl) acetyl]amino}-5-(4-methoxyphenyl)indan-2-carboxylate (242 mg, 0.58 mmol).
   ¹H-NMR (300MHz, DMSO-d₆) δ: 12.39 (1H, s), 8.70 (1H, s), 7.58-7.55 (2H, d), 7.44-7.39 (2H, m), 7.27-7.22 (3H, m), 7.12-7.06 (2H, t), 7.02-6.99 (2H ,d), 3.78 (3H, s), 3.56-3.48 (2H, m), 3.41 (2H, s), 3.24-3.15 (2H, m)
   LC-MS 420 [M+H]⁺
   Melting point: 227°C

### Example B132

### 5-(4-Chlorophenyl)-2-{[3-(4-fluorophenyl)propanoyl]amino}indan-2-carboxylic acid

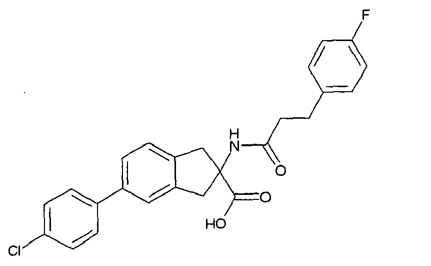

1) Ethyl 5-(4-chlorophenyl)-2-{ [3-(4-fluorophenyl)propanoyl]amino}indan-2-carboxylate (140 mg, 100%) was obtained as a colorless oil by methods similar to those of Example B117-1) from 3-(4-fluorophenyl)propionic acid (86 mg, 0.51 mmol) and ethyl 2-amino-5-(4-chlorophenyl)indan-2-carboxylate hydrochloride (105 mg, 0.30 mmol).
   ¹H-NMR (300MHz, CDC1₃) δ: 7.51-7.46 (2H, d), 7.41-7.35 (4H, m), 7.25-7.22 (1H, d), 7.19-7.09 (2H, m), 6.99-6,87 (2H, m), 5.93 (1H, s), 4.26-4.19 (2H, q), 3.69-3.61 (2H, m), 3.26-3.19 (2H, m), 2.96-2.91 (2H, t), 2.47-2.42 (2H, t), 1.27-1.22 (3H, t)
   LC-MS 466 [M+H]⁺
2) The title compound (97 mg, 69%) was obtained as white crystals by methods similar to those of Example B117-2) from ethyl 5-(4-chlorophenyl)-2-{[3-(4-fluorophenyl)propanoyl]amino}indan-2-carboxylate (150 mg, 0.32 mmol).
   ¹H-NMR (300MHz, DMSO-d₆) δ: 12.41 (1H, s), 8.42 (1H, s), 7.67-7.64 (2H, d, J=8.7Hz), 7.51-7.44 (4H, m), 7.30-7.27 (1H, d), 7.22-7.18 (2H, m), 7.06-7.00 (2H, m), 3.55-3.47 (2H, m), 3.23-3.11 (2H, m), 2.80-2.75 (2H, t, J=7.5Hz), 2.37-2.32 (2H, t, J=7.7Hz)
   LC-MS 438 [M+H]⁺
   Melting point: 220°C

### Example B133

### 5-(4-Chlorophenyl)-2-[(4-fluorobenzoyl)amino]indan-2-carboxylic acid

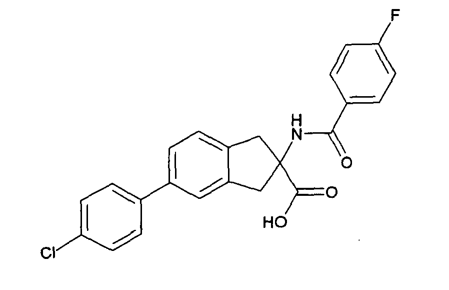

1) Ethyl 5-(4-chlorophenyl)-2-[(4-fluorobenzoyl)amino]indan-2-carboxylate (116 mg, 100%) was obtained as a colorless oil by methods similar to those of Example B117-1) from 4-fluorobenzoic acid (71 mg, 0.51 mmol) and ethyl 2-amino-5-(4-chlorophenyl)indan-2-carboxylate hydrochloride (95 mg, 0.27 mmol).
   ¹H-NMR (300MHz, CDCl₃) δ: 7.80-7.74 (2H, m), 7.50-7.48 (2H, d), 7.40-7.38 (4H, m), 7.30-7.27 (1H, m), 7.11-7.05 (2H, t), 6.70 (1H, s), 4.30-4.23 (2H, q), 3.81-3.73 (2H, m), 3.50-3.43 (2H, m), 1.28-1.23 (3H, t)
   LC-MS 438 [M+H]⁺
2) The title compound (81 mg, 71%) was obtained as white crystals by methods similar to those of Example B117-2) from ethyl 5-(4-chlorophenyl)-2-[(4-fluorobenzoyl)amino]indan-2-carboxylate (115 mg, 0.26 mmol).
   ¹H-NMR (300MHz, DMSO-d₆) δ: 12.48 (1H, s), 8.90 (1H, s), 7.95-7.91 (2H, m), 7.77-7.65 (2H, d, J=8.7Hz), 7.53-7.45 (4H, m), 7.33-7.29 (3H, m), 3.68-3.59 (2H, m), 3.48-3.39 (2H, m)
   LC-MS 410 [M+H]⁺
   Melting point: 251°C

### Example B134

### 5-(4-Chlorophenyl)-2-[({4-[(4-fluorobenzyl)oxy]phenyl}acetyl)amino]indan-2-carboxylic acid

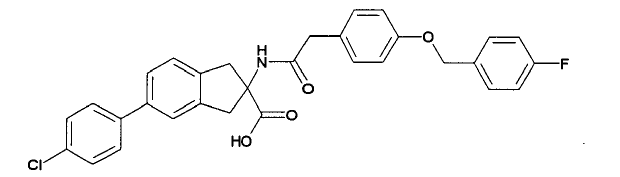

1) WSCD hydrochloride (384 mg, 2.0 mmol), HOBt (270 mg, 2.0 mmol) and triethylamine (600 µL, 4.28 mmol) were added with ice cooling to a DMF (2 mL)-dichloromethane (20 mL) solution of 4-(4-fluorobenzyloxy)phenylacetic acid (442 mg, 1.70 mmol), and stirred for 30 minutes at room temperature. Ethyl 2-amino-5-(4-chlorophenyl)indan-2-carboxylate hydrochloride (353 mg, 1.0 mmol) was added and stirred overnight at room temperature. Water was added to the reaction mixture, which was then extracted twice with ethyl acetate. The organic layers were combined, washed with water and saturated sodium chloride solution, and dried over magnesium sulfate. The drying agent was filtered out, and the filtrate was concentrated under reduced pressure. The residue was purified by silica gel chromatography (elution solvent: ethyl acetate/hexane = 1/4-1/1) to obtain ethyl 5-(4-chlorophenyl)-2-[({4-[(4-fluorobenzyl)oxy]phenyl}acetyl)amino]indan-2-carboxylate (550 mg, 100%) as a white solid. ¹H-NMR (300MHz, CDCl₃) δ: 7.49-7.46 (2H, d), 7.40-7.34 (5H, m), 7.36-7.21 (1H, m), 7.17-7.14 (2H, d), 7.10-7.04 (3H, m), 6.92-6.89 (2H, d), 5.99 (1H, s), 4.99 (2H, s), 4.23-4.16 (2H, q), 3.69-3.61 (2H, m), 3.48 (2H, s), 3.29-3.21 (2H, m), 1.23-1.19 (3H, t)
   LC-MS 558 [M+H]⁺
2) 1 N-NaOH aqueous solution (2.0 mL) was added with ice cooling to a methanol (5 mL)-THF (5 mL) solution of ethyl 5-(4-chlorophenyl)-2-[({4-[(4-fluorobenzyl)oxy]phenyl}acetyl)amino]indan-2-carboxylate (567 mg, 1.0 mmol), and stirred overnight at room temperature. 1 N-hydrochloric acid (2.2 mL) was added, and the mixture was extracted twice with ethyl acetate. The organic layers were combined, washed with saturated sodium chloride solution and dried by addition of magnesium sulfate, and concentrated under reduced pressure. The residue was recrystallized from a mixed ethyl acetate-hexane solvent to obtain the title compound (444 mg, 81%) as white crystals.
   ¹H-NMR (300MHz, DMSO-d₆) δ: 12.40 (1H, s), 8.65 (1H, s), 7.68-7.64 (2H, d), 7.51-7.45 (6H, m), 7.31-7.28 (1H, d, J=7.8Hz), 7.23-7.17 (2H, m), 7.14-7.11 (2H, d), 6.91-6.88 (2H, d, J=8.4Hz), 5.04 (2H, s), 3.58-3.49 (2H, m), 3.34 (2H, s), 3.25-3.16 (2H, m)
   LC-MS 530 [M+H]⁺
   Melting point: 208-209°C
   Elemental analysis: Calcd. for C₃₁H₂₅NO₄ClF: C 70.25, H 4.75, N 2.64; Found: C 70.22, H 4.70, N 2.49

### Example B135

### 5-(4-Chlorophenyl)-2-({[4-(methylsulfonyl)phenyl]acetyl}amino)indan-2-carboxylic acid

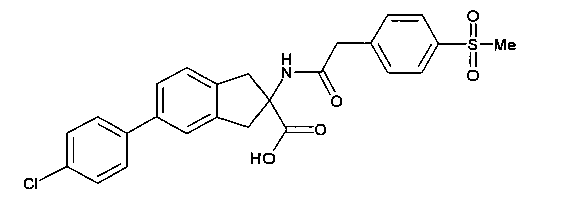

1) Ethyl 5-(4-chlorophenyl)-2-({[4-(methylsulfonyl)phenyl]acetyl}amino)indan-2-carboxylate (155 mg, 100%) was obtained as a colorless oil by methods similar to those of Example B117-1) from 4-methylsulfonylphenylacetic acid (109 mg, 0.51 mmol) and ethyl 2-amino-5-(4-chlorophenyl)indan-2-carboxylate hydrochloride (105 mg, 0.30 mmol).
   ¹H-NMR (300MHz, CDCl₃) δ: 7.91-7.88 (2H, d, J=8.1Hz), 7.50-7.47 (4H, d, J=6.9Hz), 7.41-7.38 (4H, d), 7.26-7.24 (1H, m), 6.14 (1H, s), 4.25-4.18 (2H, q), 3.70-3.62 (4H, m), 3.34-3.27 (2H, m), 3.03 (3H, s), 1.23-1.19 (3H, t)
   LC-MS 512 [M+H]⁺
2) The title compound (100 mg, 73%) was obtained as white crystals by methods similar to those of Example B117-2) from ethyl 5-(4-chlorophenyl)-2-({[4-(methylsulfonyl)phenyl]acetyl}amino)indan-2-carboxylate (139 mg, 0.27 mmol).
   ¹H-NMR (300MHz, DMSO-d₆) δ: 12.45 (1H, s), 8.83 (1H, s), 7.85-7.82 (2H, d), 7.68-7.65 (2H, d), 7.52-7.46 (6H, m), 7.33-7.30 (1H, d), 3.59-3.51 (4H, m), 3.32-3.19 (5H, m)
   LC-MS 484 [M+H]⁺
   Melting point: 216°C
   Elemental analysis: Calcd. for C₂₅H₂₂NO₅SCl•0.2 H₂O: C 61.58, H 4.63, N 2.87; Found: C 61.62, H 4.49, N 2.72

### Example B136

### 5-(4-Chlorophenyl)-2-{[(2-chlorophenyl)acetyl]amino}indan-2-carboxylic acid

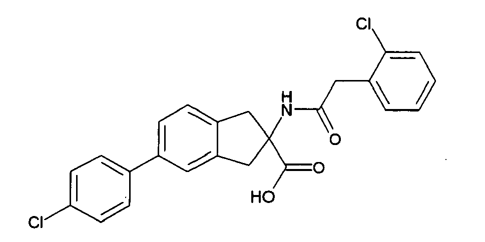

1) Ethyl 5-(4-chlorophenyl)-2-{[(2-chlorophenyl)acetyl]amino}indan-2 carboxylate (137 mg, 98%) was obtained as a colorless oil by methods similar to those of Example B117-1) from 2-chlorophenylacetic acid (85 mg, 0.51 mmol) and ethyl 2-amino-5-(4-chlorophenyl)indan-2-carboxylate hydrochloride (105 mg, 0.30 mmol).
   ¹H-NMR (300MHz, CDCl₃) δ: 7.49-7.47 (2H, d), 7.40-7.34 (6H, m), 7.26-7.19 (3H, m), 6.17 (1H, s), 4.21-4.13 (2H, q), 3.79-3.61 (4H, m), 3.33-3.26 (2H, m), 1.20-1.15 (3H, t)
   LC-MS 468 [M+H]⁺
2) The title compound (98 mg, 84%) was obtained as white crystals by methods similar to those of Example B117-2) from ethyl 5-(4-chlorophenyl)-2-{[(2-chlorophenyl)acetyl]amino}indan-2 carboxylate (123 mg, 0.26 mmol).
   ¹H-NMR (300MHz, DMSO-d₆) δ: 12.45 (1H, s), 8.83 (1H, s), 7.68-7.65 (2H, d), 7.52-7.46 (4H, m), 7.41-7.23 (5H, m), 3.57-3.51 (4H, m), 3.29-3.21 (2H, m)
   LC-MS 440 [M+H]⁺
   Melting point: 222°C
   Elemental analysis: Calcd. for C₂₄H₁₉NO₃Cl₂●0.2 H₂O: C 64.93, H 4.40, N 3.15; Found: C 65.06, H4.30,N3.06

### Example B137

### 2-[(1,3-Benzothiazol-6-ylcarbonyl)amino]-5-(4-chlorophenyl)indan-2-carboxylic acid

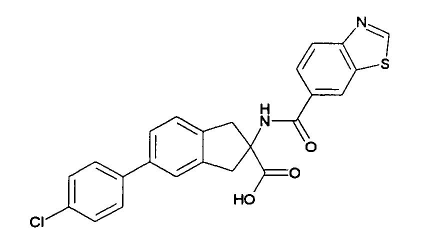

1) Ethyl 2- [(1,3 -benzothiazol-6-ylcarbonyl)amino] - 5 -(4-chlorophenyl)indan-2-carboxyl ate (108 mg, 46%) was obtained as a colorless oil by methods similar to those of Example B117-1) from 1,3-benzothiazol-6-carboxylic acid (158 mg, 0.85 mmol) and ethyl 2-amino-5-(4-chlorophenyl)indan-2-carboxylate hydrochloride (176 mg, 0.50 mmol).
   ¹H-NMR (300MHz, CDCl₃) δ: 9.11 (1H, s), 8.44 (1H, s), 8.16-8.13 (1H, d), 7.88-7.85 (1H, d), 7.51-7.48 (2H, d), 7.42-7.40 (4H,m), 7.32-7.29 (1H, m), 6.85 (1H, s), 4.15-4.08 (2H, q), 3.85-3.76 (2H, m), 3.55-3.44 (2H, m), 1.26-1.23 (3H, t)
   LC-MS 477 [M+H]⁺
2) The title compound (76 mg, 75%) was obtained as white crystals by methods similar to those of Example B117-2) from ethyl 2-[(1,3-benzothiazol-6-ylcarbonyl)amino]-5-(4-chlorophenyl)indan-2-carboxylate (108 mg, 0.23 mmol).
   ¹H-NMR (300MHz, DMSO-d₆) δ: 12.52 (1H, s), 9.53 (1H, s), 8.66 (1H, s), 8.16-8.13 (1H, d), 8.03-8.00 (1H, d), 7.69-7.66 (2H, d), 7.55-7.47 (4H, m), 7.35-7.32 (2H, d), 3.71-3.62 (2H, m), 3.52-3.43 (2H, m)
   LC-MS 449 [M+H]⁺
   Melting point: 210°C

### Example B138

### 5-(4-Chlorophenyl)-2-([(3-chlorophenyl)acetyl]amino)indan-2-carboxylic acid

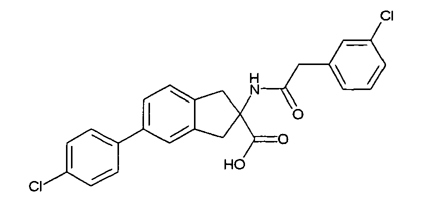

1) Ethyl 5-(4-chlorophenyl)-2-{[(3-chlorophenyl)acetyl]amino}indan-2-carboxylate (140 mg, 100%) was obtained as a colorless oil by methods similar to those of Example B117-1) from 3-chlorophenylacetic acid (85 mg, 0.51 mmol) and ethyl 2-amino-5-(4-chlorophenyl)indan-2-carboxylate hydrochloride (105 mg, 0.30 mmol).
   ¹H-NMR (300MHz, CDCl₃) δ: 7.49-7.46 (2H, d), 7.40-7.36 (4H, m), 7.26-7.15 (4H, m), 7.14-7.12 (1H, m), 6.05 (1H, s), 4.24-4.17 (2H, q), 3.70-3.62 (2H, m), 3.51-3.49 (2H, s), 3.34-3.31 (2H, m), 1.23-1.18 (3H, t)
   LC-MS 468 [M+H]⁺
2) The title compound (89 mg, 65%) was obtained as white crystals by methods similar to those of Example B117-2) from ethyl 5-(4-chlorophenyl)-2-{[(3-chlorophenyl)acetyl]amino}indan-2-carboxylate (146 mg, 0.31 mmol).
   ¹H-NMR (300MHz, DMSO-d₆) δ: 12.46 (1H, s), 8.76 (1H, s), 7.68-7.65 (2H, d), 7.52-7.45 (4H, m), 7.32-7.28 (3H, m), 7.18-7.16 (2H, d), 3.59-3.50 (2H, m), 3.45 (2H, s), 3.26-3.17 (2H, m)
   LC-MS 440 [M+H]⁺
   Melting point: 234°C

### Example B139

### 5-(4-Chlorophenyl)-2-[(2-phenylpropanoyl)amino]indan-2-carboxylic acid

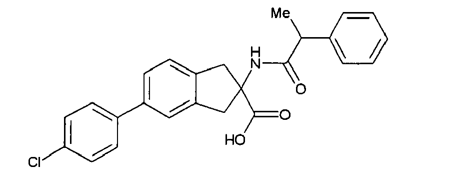

1) Ethyl 5-(4-chlorophenyl)-2-[(2-phenylpropanoyl)amino]indan-2-carboxylate (114 mg, 85%) was obtained as a colorless oil by methods similar to those of Example B117-1) from 2-phenylpropionic acid (77 mg, 0.51 mmol) and ethyl 2-amino-5-(4-chlorophenyl)indan-2-carboxylate hydrochloride (105 mg, 0.30 mmol).
   ¹H-NMR (300MHz, CDCl₃) δ: 7.48-7.46 (2H, d), 7.40-7.18 (10H, m), 5.96 (1H, s), 4.16-4.11 (2H, q), 3.72-3.49 (3H, m), 3.31-3.11 (2H, m), 1.48-1.43 (3H,m), 1.26-1.23 (3H, t)
   LC-MS 448 [M+H]⁺
2) The title compound (72 mg, 68%) was obtained as white crystals by methods similar to those of Example B117-2) from ethyl 5-(4-chlorophenyl)-2-[(2-phenylpropanoyl)amino]indan-2-carboxylate (114 mg, 0.25 mmol).
   ¹H-NMR (300MHz, DMSO-d₆) δ: 12.34 (1H, s), 8.60 (1H, s), 7.65-7.63 (2H, d), 7.50-7.43 (4H, m), 7.31-7.19 (6H, m), 3.62-3.49 (3H, m), 3.27-3.15 (2H, m), 1.30-1.28 (3H, d)
   LC-MS 420 [M+H]⁺
   Melting point: 200-201 °C

### Example B140

### 5-(4-Chlorophenyl)-2-[({4-[(4-fluorobenzoyl)amino]phenyl}acetyl)amino]indan-2-carboxylic acid

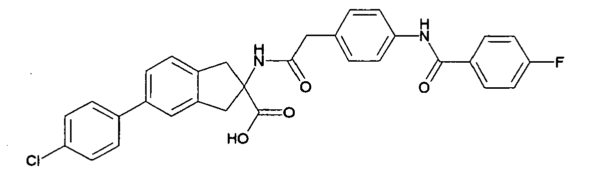

1) Ethyl 5-(4-chlorophenyl)-2-[({4-[(4-fluorobenzoyl)amino]phenyl}acetyl)amino]indan-2-carboxylate (136 mg, 80%) was obtained as a white solid by methods similar to those of Example B117-1) from 4-(4-fluorobenzoylamino)phenylacetic acid (40 mg, 0.51 mmol) and ethyl 2-amino-5-(4-chlorophenyl)indan-2-carboxylate hydrochloride (105 mg, 0.30 mmol).
   ¹H-NMR (300MHz, DMSO-d₆) δ: 10.21(1H, s), 8.85 (1H, s), 8.05-8.01 (2H, m), 7.67-7.65 (4H, d), 7.53-7.47 (4H, m), 7.39-7.31 (3H, m), 7.21-7.19 (2H, d), 4.07-3.99 (2H, q), 3.57-3.51 (2H, m), 3.39 (2H, s), 3.27-3.18 (2H, m), 1.08-1.06 (3H, t)
   LC-MS 571 [M+H]⁺
2) The title compound (50 mg, 43%) was obtained as white crystals by methods similar to those of Example B117-2) from ethyl 5-(4-chlorophenyl)-2-[({4-[(4-fluorobenzoyl)amino]phenyl}acetyl)amino]indan-2-carboxylate (124 mg, 0.22 mmol).
   ¹H-NMR (300MHz, DMSO-d₆) δ: 12.40 (1H, br s), 10.21 (1H, s), 8.70 (1H, s), 8.05-8.00 (2H, m), 7.68-7.63 (4H, m), 7.51-7.45 (4H, m), 7.39-7.33 (3H, m), 7.21-7.18 (2H, d), 3.59-3.50 (2H, m), 3.40 (2H, s), 3.27-3.18 (2H, m)
   LC-MS 543 [M+H]⁺
   Melting point: 252-254°C

### Example B141

### 5-(4-Chlorophenyl)-2-[({4-[(2-fluorobenzyl)oxy]phenyl}acetyl)amino]indan-2-carboxylic acid

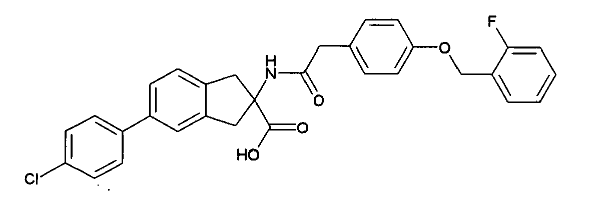

1) WSCD hydrochloride (115 mg, 0.60 mmol), HOBt (81 mg, 0.60 mmol) and triethylamine (168 µL, 1.2 mmol) were added with ice cooling to a DMF (1 mL)-dichloromethane (9 mL) solution of4-(2-fluorobenzyloxy)phenylacetic acid (124 mg, 0.51 mmol), and stirred for 30 minutes at room temperature. Ethyl 2-amino-5-(4-chlorophenyl)indan-2-carboxylate hydrochloride (105 mg, 0.30 mmol) was added and stirred overnight at room temperature. Water was added to the reaction mixture, which was then extracted twice with ethyl acetate. The organic layers were combined, washed with water and saturated sodium chloride solution, and dried over magnesium sulfate. The drying agent was filtered out, and the filtrate was concentrated under reduced pressure. The residue was purified by silica gel chromatography (elution solvent: ethyl acetate/hexane = 1/4-1/1) to obtain ethyl 5-(4-chlorophenyl)-2-[({4-[(2-fluorobenzyl)oxy]phenyl}acetyl)amino]indan-2-carboxylate (170 mg, 100%) as a colorless oil. ¹H-NMR (300MHz, CDCl₃) δ: 7.54-7.42 (2H, m), 7.40-7.05 (11H, m), 6.94-6.91 (2H, d), 5.99 (1H, s), 5.10 (2H, s), 4.13-4.08 (2H, q), 3.69-3.61 (2H, m), 3.49 (2H, s), 3.37-3.21 (2H, m), 1.28-1.23 (3H, t) LC-MS 558 [M+H]⁺
2) 1 N-NaOH aqueous solution (640 µL) was added with ice cooling to a methanol (1 mL)-THF (1 mL) solution of ethyl 5-(4-chlorophenyl)-2-[({4-[(2-fluorobenzyl)oxy]phenyl}acetyl)amino]indan-2-carboxylate (178 mg, 0.32 mmol), and stirred overnight at room temperature. 1 N-hydrochloric acid (700 µL) was added, and the mixture was extracted twice with ethyl acetate. The organic layers were combined, washed with saturated sodium chloride solution and dried by addition of magnesium sulfate, and concentrated under reduced pressure. The residue was recrystallized from a mixed ethyl acetate-hexane solvent to obtain the title compound (108 mg, 64%) as white crystals.
   ¹H-NMR (300MHz, DMSO-d₆) δ: 12.40 (1H, br s), 8.64 (1H, s), 7.67-7.65 (2H, d), 7.56-7.38 (6H, m), 7.31-7.20 (3H, m), 7.15-7.12 (2H, d), 6.93-6.90 (2H, d), 5.09 (2H, s), 3.57-3.49 (2H, m), 3.38 (2H, s), 2.51-2.49 (2H, m)
   LC-MS 530 [M+H]⁺
   Melting point: 232-233°C
   Elemental analysis: Calcd. for C₃₁H₂₅NO₄ClF: C 70.25, H 4.75, N 2.64; Found: C 70.04, H 4.70, N 2.39

### Example B142

### 5-(4-Chlorophenyl)-2-[({4-[(3-fluorobenzyl)oxy]phenyl}acetyl)amino]indan-2-carboxylic acid

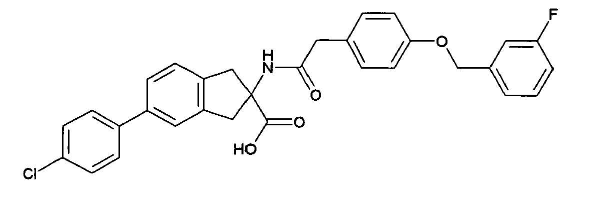

1) Ethyl 5-(4-chlorophenyl)-2-[({4-[(3-fluorobenzyl)oxy]phenyl}acetyl)amino]indan-2-carboxylate (160 mg, 96%) was obtained as a white solid by methods similar to those of Example B117-1) from 4-(3-fluorobenzyloxy)phenylacetic acid (124 mg, 0.51 mmol) and ethyl 2-amino-5-(4-chlorophenyl)indan-2-carboxylate hydrochloride (105 mg, 0.30 mmol).
   ¹H-NMR (300MHz, CDCl₃) δ: 7.50-7.45 (2H, d), 7.40-7.32 (5H, d), 7.26-7.12 (5H, s), 7.04-6.98 (1H, m), 6.96-6.89 (2H, d), 5.99 (1H, s), 5.03 (2H, s), 4.23-4.16 (2H, q), 3.69-3.61 (2H, m), 3.48 (2H, s), 3.28-3.21 (2H, m), 1.26-1.18 (3H, t)
   LC-MS 558 [M+H]⁺
2) The title compound (114 mg, 75%) was obtained as white crystals by methods similar to those of Example B117-2) from ethyl 5-(4-chlorophenyl)-2-[({4-[(3-fluorobenzyl)oxy]phenyl}acetyl)amino]indan-2-carboxylate (160 mg, 0.28 mmol).
   ¹H-NMR (300MHz, DMSO-d₆) δ: 12.40 (1H, br s), 8.66 (1H, s), 7.67-7.65 (2H, d), 7.51-7.39 (6H, m), 7.31-7.28 (3H, m), 7.15-7.12 (2H, d), 6.92-6.89 (2H, d), 5.08 (2H, s), 3.58-3.49 (2H, m), 3.34 (2H, s), 3.25-3.17 (2H, m)
   LC-MS 530 [M+H]⁺
   Melting point: 209°C
   Elemental analysis: Calcd. for C₃₁H₂₅NO₄ClF: C 70.25, H 4.75, N 2.64; Found: C 70.04, H 4.70, N 2.39

### Example B143

### 2-[({4-[(4-Chlorobenzyl)oxy]phenyl}acetyl)amino]-5-(4-chlorophenyl)indan-2-carboxylic acid

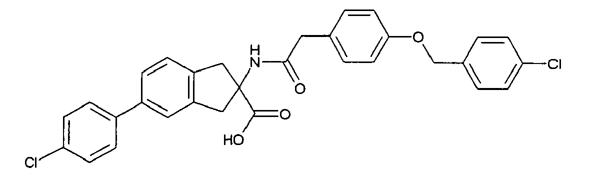

1) Ethyl 2-[({4-[(4-chlorobenzyl)oxy]phenyl}acetyl)amino]-5-(4-chlorophenyl)indan-2-carboxylate (172 mg, 100%) was obtained as a white solid by methods similar to those of Example B117-1) from 4-(4-chlorobenzyloxy)phenylacetic acid (141 mg, 0.51 mmol) and ethyl 2-amino-5-(4-chlorophenyl)indan-2-carboxylate hydrochloride (105 mg, 0.30 mmol).
   ¹H-NMR (300MHz, CDCl₃) δ: 7.49-7.45 (2H, d), 7.40-7.35 (8H, m), 7.26-7.21 (1H, t), 7.16-7.13 (2H, d), 6.91-6.90 (2H, d), 6.00 (1H, s), 5.00 (2H, s), 4.23-4.09 (2H, q), 3.69-3.60 (2H, m), 3.48 (2H, s), 3.28-3.21 (2H, m), 1.28-1.18 (3H, t)
   LC-MS 574 [M+H]⁺
2) The title compound (105 mg, 58%) was obtained as white crystals by methods similar to those of Example B117-2) from ethyl 2-[((4-[(4-chlorobenzyl)oxy]phenyl)acetyl)amino]-5-(4-chlorophenyl)indan-2-carboxylate (190 mg, 0.33 mmol).
   ¹H-NMR (300MHz, DMSO-d₆) δ: 12.41 (1H, s), 8.66 (1H ,s), 7.67-7.65 (2H, d), 7.51-7.45 (8H, m), 7.31-7.29 (1H, d), 7.14-7.12 (2H, d), 6.91-6.88 (2H, d), 5.06 (2H, s), 3.58-3.50 (2H, m), 3.34 (2H, s), 3.25-3.16 (2H, m)
   LC-MS 546 [M+H]⁺
   Melting point: 230-232°C
   Elemental analysis: Calcd. for C₃₁H₂₅NO₄Cl₂•0.2 H₂O: C 67.69, H 4.65, N 2.54, Found: C 67.47, H4.57,N2.47

### Example B144

### 2-[({4-[(2-Chloro-4-fluorobenzyl)oxy]phenyl}acetyl)amino]-5-(4-chlorophenyl)indan-2-carboxylic acid

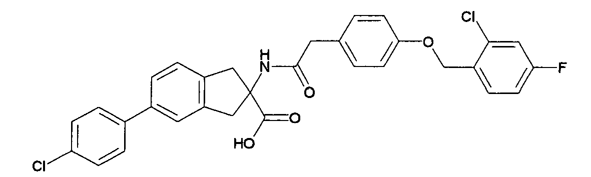

1) Ethyl 2-[({4-[(2-chloro-4-fluorobenzyl)oxy]phenyl}acetyl)amino]-5-(4-chlorophenyl)indan-2-carboxylate (177 mg, 100%) was obtained as a white solid by methods similar to those of Example B117-1) from 4-[(2-chloro-4-fluorobenzyloxy)phenylacetic acid (150 mg, 0.51 mmol) and ethyl 2-amino-5-(4-chlorophenyl)indan-2-carboxylate hydrochloride (105 mg, 0.30 mmol).
   ¹H-NMR (300MHz, CDCl₃) δ: 7.53-7.43 (3H, m), 7.40-7.34 (4H, m), 7.26-7.15 (4H, m), 7.04-6.97 (1H, m), 6.91-6.89 (2H, d), 6.03 (1H, s), 5.08 (2H, s), 4.23-4.09 (2H, m), 3.69-3.59 (2H, m), 3.48 (2H, s), 3.29-3.21 (2H, m), 1.28-1.12 (3H, t) LC-MS 592 [M+H]⁺
2) The title compound (116 mg, 58%) was obtained as white crystals by methods similar to those of Example B117-2) from ethyl 2-[({4-[(2-chloro-4-fluorobenzyl)oxy]phenyl}acetyl)amino]-5-(4-chlorophenyl)indan-2-carboxylate (211 mg, 0.35 mmol).
   ¹H-NMR (300MHz, DMSO-d₆) δ: 12.40 (1H, s), 8.65 (1H, s), 7.66-7.61 (3H, m), 7.53-7.45 (5H, m), 7.31-7.23 (2H, m), 7.16-7.13 (2H, d), 6.93-6.90 (2H, d), 5.08 (2H, s), 3.57-3.49 (2H, m), 3.35 (2H, s), 3.25-3.16 (2H, m)
   LC-MS 564 [M+H]⁺
   Melting point: 200-201°C
   Elemental analysis: Calcd. for C₃₁H₂₄NO₄Cl₂F: C 65.97, H 4.29, N 2.48; Found: C 65.78, H 4.28, N 2.26

### Example B145

### 5-(4-Chlorophenyl)-2-{[(4-{[4-(trifluoromethyl)benzyl]oxy}phenyl)acetyl]amino}indan-2-carboxylic acid

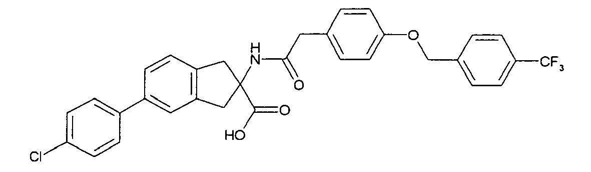

1) Ethyl 5-(4-chlorophenyl)-2-{[(4-{[4-(trifluoromethyl)benzyl]oxy}phenyl)acetyl]amino}indan-2-carboxylate (182 mg, 100%) was obtained as a white solid by methods similar to those of Example B117-1) from 4-[4-(trifluoromethyl)benzyloxy]phenylacetic acid (158 mg, 0.51 mmol) and ethyl 2-amino-5-(4-chlorophenyl)indan-2-carboxylate hydrochloride (105 mg, 0.30 mmol).
   ¹H-NMR (300MHz, CDCl₃) δ: 7.65-7.63 (2H, d), 7.54-7.40 (4H, m), 7.40-7.33 (4H, m), 7.26-7.21 (3H, m), 6.94-6.90 (2H, d), 6.00 (1H, s), 5.09 (2H, s), 4.23-4.15 (2H, q), 3.69 (2H, m), 3.48 (2H, s), 3.29-3.21 (2H, m), 1.26-1.20 (3H, t)
   LC-MS 608 [M+H]⁺
2) The title compound (154 mg, 80%) was obtained as white crystals by methods similar to those of Example B117-2) from ethyl 5-(4-chlorophenyl)-2-{ [(4-{ [4-(trifluoromethyl)benzyl]oxy}phenyl)acetyl]amino}indan-2-carboxylate (198 mg, 0.33 mmol). ¹H-NMR (300MHz, DMSO-d₆) δ: 12,40 (1H, s), 8.64 (1H, s), 7.77-7.73 (2H, d), 7.67-7.64 (4H, m), 7.51-7.45 (4H, m), 7.31-7.28 (1H, d), 7.15-7.12 (2H, d), 6,93-6.89 (2H, d), 5.19 (2H, s), 3.57-3.48 (2H, m), 3.34 (2H, s), 3.24-3.15 (2H, m)
   LC-MS 580 [M+H]⁺
   Melting point: 237-238°C

### Example B146

### 5-(4-Chlorophenyl)-2-({[4-(3-furylmethoxy)phenyl]acetyl}amino)indan-2-carboxylic acid

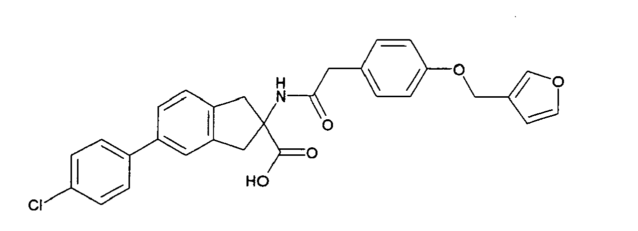

1) WSCD hydrochloride (115 mg, 0.60 mmol), HOBt (81 mg, 0.60 mmol) and triethylamine (168 µL, 1.2 mmol) were added with ice cooling to a DMF (1 mL)-dichloromethane (9 mL) solution of4-(3-furylmethoxy)phenylacetic acid (118 mg, 0.51 mmol), and stirred for 30 minutes at room temperature. Ethyl 2-amino-5-(4-chlorophenyl)indan-2-carboxylate hydrochloride (105 mg, 0.30 mmol) was added and stirred overnight at room temperature. Water was added to the reaction mixture, which was then extracted twice with ethyl acetate. The organic layers were combined, washed with water and saturated sodium chloride solution, and dried by addition of magnesium sulfate. The drying agent was filtered out, and the filtrate was concentrated under reduced pressure. The residue was purified by silica gel chromatography (elution solvent: ethyl acetate/hexane = 1/4-1/1) to obtain ethyl 5-(4-chlorophenyl)-2-({[4-(3-furylmethoxy)phenyl]acetyl}amino)indan-2-carboxylate (147 mg, 92%) as a colorless oil.
   ¹H-NMR (300MHz, CDCl₃) δ: 7.49-7.33 (8H, m), 7.26-7.14 (3H, m), 6.91-6.89 (2H, d), 6.47 (1H, s), 5.99 (1H, s), 4.90 (2H, s), 4.23-4.16 (2H, q), 3.69-3.61 (2H, m), 3.48 (2H, s), 3.28-3.21 (2H, m), 1.24-1.19 (3H, t)
   LC-MS 530 [M+H]⁺
2) The title compound (116 mg, 81%) was obtained as white crystals by methods similar to those of Example B117-2) from ethyl 5-(4-chlorophenyl)-2-({ [4-(3-furylmethoxy)phenyl]acetyl}amino)indan-2-carboxylate (147 mg, 0.28 mmol).
   ¹H-NMR (300MHz, DMSO-d₆) δ: 12.40 (1H, s), 8.64 (1H, s), 7.76 (1H, s), 7.67-7.65 (3H, m), 7.51-7.45 (3H, m), 7.31-7.28 (2H, d), 7.13-7.10 (2H, d), 6.89-6.87 (2H, d), 6.55 (1H, s), 4.90 (2H, s), 3.57-3.48 (2H, m), 3.34 (2H, s), 3.25-3.16 (2H, m)
   LC-MS 502 [M+H]⁺
   Melting point: 200-202°C
   Elemental analysis: Calcd. for C₂₉H₂₄NOₛCl: C 69.39, H 4.82, N 2.79; Found: C 69.17, H 4.55, N 2.49

### Example B147

### 5-(4-Chlorophenyl)-2-({[4-(3-thienylmethoxy)phenyl]acetyl}amino)indan-2-carboxylic acid

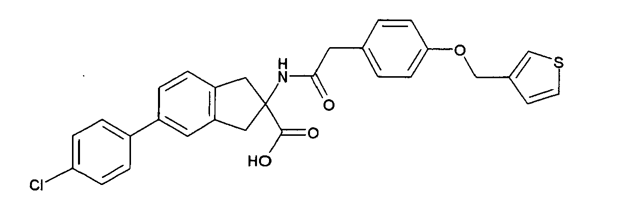

1) WSCD hydrochloride (115 mg, 0.60 mmol), HOBt (81 mg, 0.60 mmol) and triethylamine (168 µL, 1.2 mmol) were added with ice cooling to a DMF (1 mL)-dichloromethane (9 mL) solution of 4-(3-thienylmethoxy)phenylacetic acid (126 mg, 0.51 mmol), and stirred for 30 minutes at room temperature. Ethyl 2-amino-5-(4-chlorophenyl)indan-2-carboxylate hydrochloride (105 mg, 0.30 mmol) was added and stirred overnight at room temperature. Water was added to the reaction mixture, which was then extracted twice with ethyl acetate. The organic layers were combined, washed with water and saturated sodium chloride solution, and dried over magnesium sulfate. The drying agent was filtered out, and the filtrate was concentrated under reduced pressure. The residue was purified by silica gel chromatography (elution solvent: ethyl acetate/hexane = 1/4-1/1) to obtain ethyl 5-(4-chlorophenyl)-2-({[4-(3-thienylmethoxy)phenyl]acetyl}amino)indan-2-carboxylate (155 mg, 95%) as a colorless oil.
   ¹H-NMR (300MHz, CDCl₃) δ: 7.49-7.48 (2H, d), 7.46-7.30 (6H, m), 7.26-7.21 (1H, m), 7.16-7.12 (3H, m), 6.92-6.89 (2H, d), 5.99 (1H, s), 5.04 (2H, s), 4.23-4.16 (2H, q), 3.69-3.61 (2H, m), 3.48 (2H, s), 3.28-3.21 (2H, m), 1.26-1.19 (3H, t)
   LC-MS 546 [M+H] ⁺
2) 1 N-NaOH aqueous solution (568 µL) was added with ice cooling to a methanol (1 mL)-THF (1 mL) solution of ethyl 5-(4-chlorophenyl)-2-({[4-(3-thienylmethoxy)phenyl]acetyl}amino)indan-2-carboxylate (155 mg, 0.28 mmol), and stirred overnight at room temperature. 1 N-hydrochloric acid (600 µL) was added, and the mixture was extracted twice with ethyl acetate. The organic layers were combined, washed with saturated sodium chloride solution and dried by addition of magnesium sulfate, and concentrated under reduced pressure. The residue was recrystallized from a mixed ethyl acetate-hexane solvent to obtain the title compound (116 mg, 79%) as white crystals.
   ¹H-NMR (300MHz, DMSO-d₆) δ: 12.40 (1H, s), 8.65 (1H, s), 7.67-7.64 (2H, d), 7.54-7.44 (6H, m), 7.32-7.28 (1H, d), 7.17-7.10 (3H, m), 6.91-6.88 (2H, d), 5.04 (2H, s), 3.57-3.49 (2H, m), 3.34 (2H, s), 3.25-3.16 (2H, m)
   LC-MS 518 [M+H]⁺
   Melting point: 224-226°C
   Elemental analysis: Calcd. for C₂₉H₂₄NO₄SCl: C 67.24, H 4.67, N 2.70; Found: C 67.15, H 4.55, N 2.48

### Example B148

### N-{[2-({[4-(Benzyloxy)phenyl]acetyl} amino)-5-(4-chlorophenyl)-2,3 -dihydro-1H-inden-2-yl]carbonyl}glycine

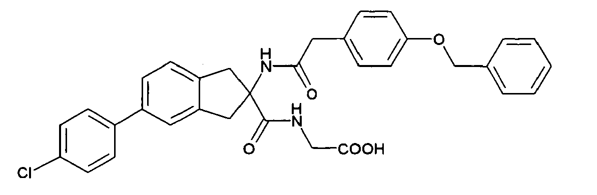

1) Diethyl cyanophosphate (23 µL, 0.015) mmol and triethylamine (56 µL, 0.04 mmol) were added with ice cooling to a DMF (1 mL) solution of 2-({[4-(benzyloxy)phenyl]acetyl}amino)-5-(4-chlorophenyl)indan-2-carboxylic acid (51 mg, 0.010 mmol), and stirred for 30 minutes at room temperature. Glycine methyl ester hydrochloride (25 mg, 0.02 mmol) was added and stirred overnight at room temperature. Water was added to the reaction mixture, which was then extracted twice with ethyl acetate. The organic layers were combined, washed with water and saturated sodium chloride solution, and dried over magnesium sulfate. The drying agent was filtered out, and the filtrate was concentrated under reduced pressure. The residue was purified by silica gel chromatography (elution solvent: ethyl acetate/hexane =1/2-2/1) to obtain methyl N-{[2-({[4-(benzyloxy)phenyl]acetyl}amino)-5-(4-chlorophenyl)-2,3-dihydro-1H-inden-2-yl]carbonyl}glycinate (52 mg, 90%) as a colorless oil. ¹H-NMR (300MHz, CDCl₃) δ: 7.48-7.46 (2H, d), 7.40-7.36 (9H, m), 7.26-7.23 (1H, m), 7.10-7.03 (3H, m), 6.91-6.89 (2H, d), 6.12 (1H, s), 5.01 (2H, s), 4.00-3.99 (2H, d), 3.72 (3H, s), 3.63-3.55 (2H, m), 3.51-3.44 (4H, m) LC-MS 583 [M+H]⁺
2) The title compound (36 mg, 71%) was obtained as white crystals by methods similar to those of Example B117-2) from methyl N-{[2-({[4-(benzyloxy)phenyl]acetyl}amino)-5-(4-chlorophenyl)-2,3-dihydro-1H-inden-2-yl]carbonyl}glycinate (52 mg, 0.089 mmol).
   ¹H-NMR (300MHz, DMSO-d₆) δ: 12.50 (1H, s), 8.52 (1H, s), 8.01-7.98 (1H, t, J=5.7Hz), 7.68-7.65 (2H, d), 7.50-7.27 (10H, m), 7.09-7.06 (2H, d), 6.87-6.84 (2H, d), 5.03 (2H, s), 3.72-3.69 (2H, d, J=5.7Hz), 3.60-3.52 (2H, m), 3.37 (2H, s), 3.25-3.16 (2H, m)
   LC-MS 569 [M+H]⁺
   Melting point: 217-218°C

### Example B149

### N-({5-(4-Chlorophenyl)-2-[({4-[(4-fluorobenzyl)oxy]phenyl}acetyl)amino]-2,3-dihydro-1H-inden-2-yl}carbonyl)glycine

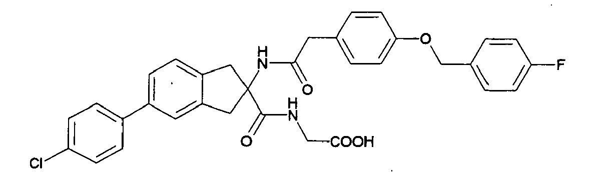

1) Methyl N-({5-(4-chlorophenyl)-2-[({4-[(4-fluorobenzyl)oxy]phenyl}acetyl)amino]-2,3-dihydro-1H-inden-2-yl}carbonyl)glycinate (117 mg, 98%) was obtained as a white solid by methods similar to those of Example B148-1) from 5-(4-chlorophenyl)-2-[({4-[(4-fluorobenzyl)oxy]phenyl]acetyl)amino]indan-2-carboxylic acid (106 mg, 0.20 mmol) and glycine methyl ester hydrochloride (38 mg, 0.30 mmol).
   ¹H-NMR (300MHz, CDCl₃) δ: 7.49-7.46 (2H, d), 7.40-7.35 (6H, m), 7.26-7.23 (1H, m), 7.11-6.98 (5H, m), 6.88-6.86 (2H, d), 6.21 (1H, s), 4.96 (2H, s), 4.00-3.99 (2H, d), 3.72 (3H, s), 3.62-3.45 (6H, m)
   LC-MS 601 [M+H]⁺
2) The title compound (54 mg, 47%) was obtained as white crystals by methods similar to those of Example B117-2) from methyl N-({5-(4-chlorophenyl)-2-[({4-[(4-fluorobenzyl)oxy]phenyl}acetyl)amino]-2,3-dihydro-1H-inden-2-yl}carbonyl)glycinate (117 mg, 0.20 mmol).
   ¹H-NMR (300MHz, DMSO-d₆) δ: 12.50 (1H, br s), 8.53 (1H, s), 8.03-7.99 (1H, t, J=5.7Hz), 7.68-7.65 (2H, d, J=8.4Hz), 7.50-7.44 (6H, m), 7.30-7.28 (1H, d), 7.23-7.17 (2H, m), 7.10-7.07 (2H, d, J=8.4Hz), 6.87-6.84 (2H, d), 5.01 (2H, s), 3.72-3.70 (2H, d, J=8.4Hz), 3.60-3.52 (2H, m), 3.38 (2H, s), 3.25-3.16 (2H, m)
   LC-MS 587 [M+H]⁺
   Melting point: 209-210°C

### Example B150

### N-({5-(4-Chlorophenyl)-2-[({4-[(4-fluorobenzyl)oxy]phenyl}acetyl)amino]-2,3-dihydro-1H-inden-2-yl}carbonyl)-β-alanine

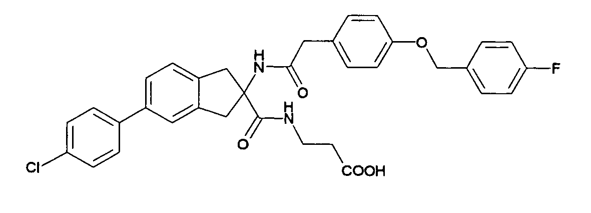

1) Methyl N-({5-(4-chlorophenyl)-2-[({4-[(4-fluorobenzyl)oxy]phenyl}acetyl)amino]-2,3-dihydro-1H-inden-2-yl}carbonyl)-β-alaninate (96 mg, 78%) was obtained as a white solid by methods similar to those of Example B148-1) from 5-(4-chlorophenyl)-2-[({4-[(4-fluorobenzyl)oxy]phenyl}acetyl)amino]indan-2-carboxylic acid (106 mg, 0.20 mmol) and β-alanine methyl ester hydrochloride (46 mg, 0.30 mmol).
   ¹H-NMR (300MHz, CDCl₃) δ: 7.49-7.46 (2H, d), 7.40-7.33 (6H, m), 7.26-7.21 (2H, m), 7.11-6.98 (4H, m), 6.89-6.86 (3H, m), 6.20 (1H, s), 5.00 (2H, s), 3.62 (3H, s), 3.54-3.39 (8H, m), 2.51-2.48 (2H, t) LC-MS 615 [M+H]⁺
2) The title compound (63 mg, 67%) was obtained as white crystals by methods similar to those of Example B117-2) from methyl N-({5-(4-chlorophenyl)-2-[({4-[(4-fluorobenzyl)oxy]phenyl}acetyl)amino]-2,3-dihydro-1H-inden-2-yl}carbonyl)-β-alaninate (96 mg, 0.16 mmol).
   ¹H-NMR (300MHz, DMSO-d₆) δ: 12.40 (1H, br s), 8.48 (1H, s), 7.68-7.64 (3H, m), 7.50-7.44 (6H, m), 7.28-7.16 (3H, m), 7.10-7.07 (2H, d, J=8.4Hz), 6.88-6.85 (2H, d, J=8.4Hz), 5.01 (2H, s), 3.54-3.46 (2H, m), 3.35 (2H, s), 3.27-3.11 (4H, m), 2.34-2.78 (2H, t)
   LC-MS 601 [M+H]⁺
   Melting point: 214°C

### Example B151

### 5-(4-Chlorophenyl)-2-({[4-(2-thienylmethoxy)phenyl]acetyl}amino)indan-2-carboxylic acid

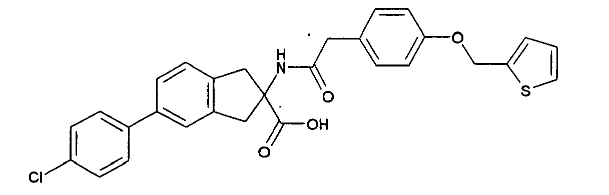

1) Ethyl 5-4-chlorophenyl)-2-({[4-(2-thienylmethoxy)phenyl]acetyl}amino)indan-2-carboxylate (156 mg, 95%) was obtained as a colorless oil by methods similar to those of Example B117-1) from 4-(2-thienylmethoxy)phenylacetic acid (126 mg, 0.51 mmol) and ethyl 2-amino-5-(4-chlorophenyl)indan-2-carboxylate hydrochloride (105 mg, 0.30 mmol).
   ¹H-NMR (300MHz, CDCl₃) δ: 7.50-7.47 (2H, d), 7.40-7.31 (5H, m), 7.26-7.21 (1H, m), 7.17-7.14 (2H, d), 7.10-7.09 (1H, d), 7.00-6.99 (1H, m), 6. 94-6.91 (2H, d), 5.98 (1H, s), 5.19 (2H, s), 4.23-4.13 (2H, q), 3.69-3.61 (2H, m), 3.48 (2H, s), 3.28-3.21 (2H, m), 1.23-1.18 (3H, t)
   LC-MS 546 [M+H]⁺
2) IN-NaOH aqueous solution (576 µL) was added with ice cooling to a methanol (1 mL)-THF (1 mL) solution of ethyl 5-(4-chlorophenyl)-2-({[4-(2-thienylmethoxy)phenyl]acetyl}amino)indan-2-carboxylate (156 mg, 0.29 mmol), and stirred overnight at room temperature. 1 N-hydrochloric acid (600 µL) was added, and the mixture was extracted twice with ethyl acetate. The organic layers were combined, washed with saturated sodium chloride solution, dried over magnesium sulfate, and concentrated under reduced pressure. The residue was recrystallized from a mixed ethyl acetate-hexane solvent to obtain the title compound (82 mg, 55%) as white crystals.
   ¹H-NMR (300MHz, DMSO-d₆) δ: 12.40 (1H, br s), 8.64 (1H, s), 7.67-7.65 (2H, d), 7.54-7.44 (5H, m), 7.31-7.28 (1H, d), 7.19-7.18 (1H, d), 7.14-7.11 (2H, d), 7.04-7.01 (1H, m), 6.92-6.89 (2H, d), 5.24 (2H, s), 3.56-3.48 (2H, m), 3.33 (2H, s), 3.25-3.16 (2H, m)
   LC-MS 518 [M+H]⁺
   Melting point: 201°C
   Elemental analysis: Calcd. for C₂₉H₂₄NO₄SCl: C 67.24, H 4.67, N 2.70; Found: C 67.11, H 4.54, N 2.54

### Example B152

### 5-(4-Chlorophenyl)-2-[({4-[(2-methyl-1,3-thiazol-4-yl)methoxy]phenyl)acetyl)amino]indan-2-carboxylic acid

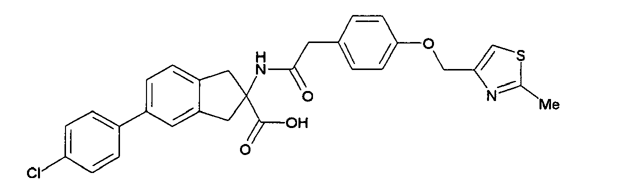

1) Ethyl 5-(4-chlorophenyl)-2-[({4-[(2-methyl-1,3-thiazol-4-yl)methoxy]phenyl}acetyl)amino]indan-2-carboxylate (197 mg, 56%) was obtained as a colorless oil by methods similar to those of Example B117-1) from 4-[(2-methyl-1,3-thiazol-4-yl)methoxy]phenylacetic acid (250 mg, 0.95 mmol) and ethyl 2-amino-5-(4-chlorophenyl)indan-2-carboxylate hydrochloride (223 mg, 0.63 mmol).
   ¹H-NMR (300MHz, DMSO-d₆) δ: 8.80 (1H, s), 7.67-7.65 (2H, d), 7.52-7.46 (5H, m), 7.33-7.30 (1H, d), 7.15-7.13 (2H, s), 6.94-6.92 (2H, d), 5.06 (2H, s), 4.07-3.97 (2H, q), 3.59-3.50 (2H, m), 3,32 (2H, s), 3.25-3.16 (2H, m), 2.65 (3H, s), 1.19-1.11 (3H, t)
   LC-MS 561 [M+H]⁺
2) The title compound (118 mg, 63%) was obtained as white crystals by methods similar to those of Example B117-2) from ethyl 5-(4-chlorophenyl)-2-[({4-[(2-methyl-1,3-thiazol-4-yl)methoxy]phenyl}acetyl)amino]indan-2-carboxylate (198 mg, 0.35 mmol).
   ¹H-NMR (300MHz, DMSO-d₆) δ: 12.40 (1H, s), 8.66 (1H, s), 7.68-7.64 (2H, d, J=8.4Hz), 7.51-7.45 (5H, m), 7.32-7.29 (1H, d, J=7.8Hz), 7.14-7.11 (2H, d, J=9.9Hz), 6.93-6.90 (2H, d, J=8.7Hz), 5.05 (2H, s), 3.58-3.49 (2H, m), 3.35 (2H, s), 3.25-3.16 (2H, m), 2.65 (3H, s)
   LC-MS 533 [M+H]⁺
   Melting point: 209-210°C
   Elemental analysis: Calcd. for C₂₉H₂₅N₂O₄SCl: C 65.34, H 4.73, N 5.26; Found: C 65.35, H 4.66, N 5.23

### Example B153

### 5-(4-Chlorophenyl)-2-[({4-[(2,6-dichlorobenzyl)oxy]phenyl}acetyl)amino]indan-2-carboxylic acid

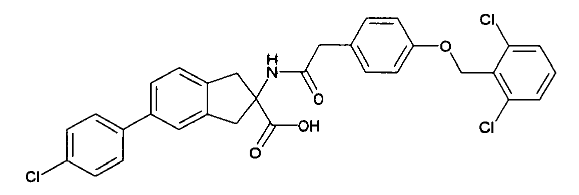

1) Ethyl 5-(4-chlorophenyl)-2-[({4-[(2,6-dichlorobenzyl)oxy]phenyl}acetyl)amino]indan-2-carboxylate (186 mg, 100%) was obtained as a colorless oil by methods similar to those of Example B117-1) from 4-(2,6-dichlorobenzyloxy)phenylacetic acid (159 mg, 0.51 mmol) and ethyl 2-amino-5-(4-chlorophenyl)indan-2-carboxylate hydrochloride (105 mg, 0.30 mmol).
   ¹H-NMR (300MHz, CDCl₃) δ: 7.49-7.45 (2H, d), 7.40-7.33 (6H, m), 7.28-7.17 (4H, m), 7.00-6.95 (2H, d), 6.01 (1H, s), 5.24 (2H, s), 4.23-4.16 (2H, q), 3.70-3.62 (2H, m), 3.50 (2H, s), 3.30-3.22 (2H, m), 1.26-1.23 (3H, t)
   LC-MS 608 [M+H]⁺
2) The title compound (132 mg, 74%) was obtained as white crystals by methods similar to those of Example B117-2) from ethyl 5-(4-chlorophenyl)-2-[({4-[(2,6-dichlorobenzyl)oxy]phenyl}acetyl)amino]indan-2-carboxylate (186 mg, 0.30 mmol)
   ¹H-NMR (300MHz, DMSO-d₆) δ: 12.40 (1H, s), 8.68 (1H, s), 7.76-7.65 (2H, d), 7.58-7.44 (7H, m), 7.32-7.30 (1H, d, J=7.8Hz), 7.18-7.15 (2H, d, J=8.7Hz), 6.97-6.94 (2H, d, J=8.7Hz), 5.18 (2H, s), 3.58-3.50 (2H, m), 3.37 (2H, s), 3.26-3.17 (2H, m)
   LC-MS 580 [M+H]⁺
   Melting point: 204-205°C
   Elemental analysis: Calcd. for C₃₁H₂₄NO₄Cl₃: C 64.10, H 4.16, N 2.41; Found: C 64.06, H 4.14, N 2.31

### Example B154

### 5-(4-Chlorophenyl)-2-({[4-(2,3-dihydro-1H-inden-1-yloxy)phenyl]acetyl}amino)indan-2-carboxylic acid

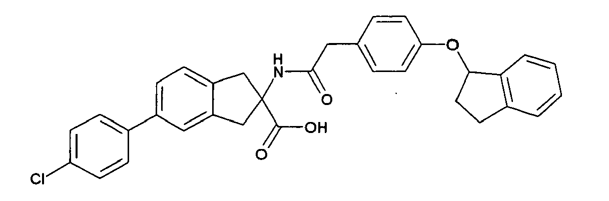

1) Ethyl 5-(4-chlorophenyl)-2-({[4-(2,3-dihydro-1H-inden-1-yloxy)phenyl]acetyl}amino)indan-2-carboxylate (170 mg, 100%) was obtained as a colorless oil by methods similar to those of Example B117-1) from 4-(2,3-dihydro-1H-inden-1-yloxy)phenylacetic acid (134 mg, 0.51 mmol) and ethyl 2-amino-5-(4-chlorophenyl)indan-2-carboxylate hydrochloride (105 mg, 0.30 mmol).
   ¹H-NMR (300MHz, CDCl₃) δ: 7.45-7.22 (11H, m), 7.18-7.15 (2H, d), 6.96-6.93 (2H, d), 6.05 (1H, s), 5.74-5.71 (1H, t), 4.34-4.17 (2H, q), 3.70-3.62 (2H, m), 3.50 (2H, s), 3.30-3,23 (2H, m), 3.18-3.08 (1H, m), 2.96-2.86 (1H, m), 2.59-2.48 (1H, m), 2.26-2.13 (1H, m), 1.28-1.23 (3H, t)
   LC-MS 566 [M+H]⁺
2) 1 N-NaOH aqueous solution (624 µL) was added with ice cooling to a methanol (1 mL)-THF (1 mL) solution of ethyl 5-(4-chlorophenyl)-2-({[4-(2,3-dihydro-1H-inden-1-yloxy)phenyl]acetyl}amino)indan-2-carboxylate (177 mg, 0.31 mmol), and stirred overnight at room temperature. 1 N-hydrochloric acid (700 µL) was added, and the mixture was extracted twice with ethyl acetate. The organic layers were combined, washed with saturated sodium chloride solution, dried by addition of magnesium sulfate, and concentrated under reduced pressure. The residue was recrystallized from a mixed ethyl acetate-hexane solvent to obtain the title compound (124 mg, 74%) as white crystals.
   ¹H-NMR (300MHz, DMSO-d₆) δ: 12.41 (1H, s), 8.66 (1H, s), 7.67-7.65 (2H, d), 7.50-7.45 (4H, m), 7.35-7.27 (4H, m), 7.24-7.19 (1H, m), 7.16-7.13 (2H, d, J=8.4Hz), 6.94-6.91 (2H, d, J=8.4Hz), 5.81-5.78 (1H, m), 3.58-3.49 (2H, m), 3.32 (2H, s), 3.26-3.18 (2H, m), 3.06-3.00 (1H, m), 2.91-2.81 (1H, m), 2.58-2.44 (1H, m), 2.05-2.00 (1H, m)
   LC-MS 538 [M+H]⁺
   Melting point: 194-195°C
   Elemental analysis: Calcd. for C₃₃H₂₈NO₄Cl: C 73.67, H 5.25, N 5.21; Found: C 73.42, H 5.21, N 2.53

### Example B155

### 5-(4-Chlorophenyl)-2-({[4-(2,3-dihydro-1H-inden-2-yloxy)phenyl]acetytl}amino)indan-2-carboxylic acid

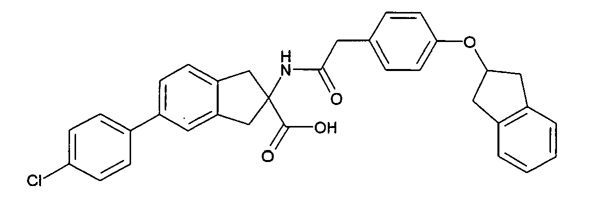

1) Ethyl 5-(4-chlorophenyl)-2-({[4-(2,3-dihydro-1H-inden-2-yloxy)phenyl]acetyl}amino)indan-2-carboxylate (163 mg, 96%) was obtained as a colorless oil by methods similar to those of Example B117-1) from 4-(2,3-dihydro-1H-inden-2-yloxy)phenylacetic acid (134 mg, 0.51 mmol) and ethyl 2-amino-5-(4-chlorophenyl)indan-2-carboxylate hydrochloride (105 mg, 0.30 mmol).
   ¹H-NMR (300MHz, CDCl₃) δ: 7.48-7.46 (2H, d), 7.45-7.34 (4H, m), 7.26-7.12 (7H, m), 6.85-6.82 (2H, d), 6.00 (1H, s), 5.16-5.09 (1H, m), 4.23-4.16 (2H, q), 3.70-3.61 (2H, m), 3.48 (2H, s), 3.39-3.12 (6H, m), 1.28-1,24 (3H, t)
   LC-MS 566 [M+H]⁺
2) 1 N-NaOH aqueous solution (576 µL) was added with ice cooling to a methanol (1 mL)-THF (1 mL) solution of ethyl 5-(4-chlorophenyl)-2-({[4-(2,3-dihydro-1H-inden-2-yloxy)phenyl]acetyl}amino)indan-2-carboxylate (163 mg, 0.28 mmol), and stirred overnight at room temperature. 1 N-hydrochloric acid (600 µL) was added, and the mixture was extracted twice with ethyl acetate. The organic layers were combined, washed with saturated sodium chloride solution, dried by addition of magnesium sulfate, and concentrated under reduced pressure. The residue was recrystallized from a mixed ethyl acetate-hexane solvent to obtain the title compound (118 mg, 76%) as white crystals.
   ¹H-NMR (300MHz, DMSO-d₆) δ: 12.41 (1H, s), 8.66 (1H, s), 7.67-7.65 (2H, d), 7.51-7.45 (4H, m), 7.32-7.11 (7H, m), 6.84-6.81 (2H, d, J=8.7 Hz), 5.20-5.16 (1H, m), 3.58-3.50 (2H, m), 3.38-3.26 (4H, m), 3.22-3.17 (2H, m), 3.02-2.96 (2H, dd)
   LC-MS 538 [M+H]⁺
   Melting point: 233-234°C
   Elemental analysis: Calcd. for C₃₃H₂₈NO₄Cl: C 73.67, H 5.25, N 2.60; Found: C 73.47, H 5.11, N 2.38

### Example B156

### 5-(4-Chlorophenyl)-2-[({4-[(2-methyl-1,3-oxazol-4-yl)methoxy]phenyl}acetyl)amino]indan-2-carboxylic acid

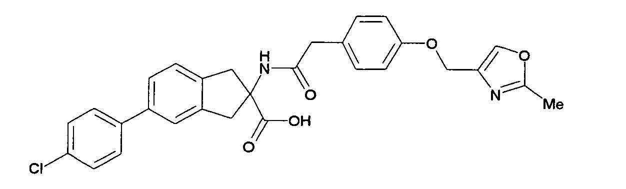

1) Ethyl 5-(4-chlorophenyl)-2-[({4-[(2-methyl-1,3-oxazol-4-yl)methoxy]phenyl}acetyl)amino]indan-2-carboxylate (164 mg, 100%) was obtained as a colorless oil by methods similar to those of Example B117-1) from 4-[(2-methyl-1,3-oxazol-4-yl)methoxy]phenylacetic acid (99 mg, 0.40 mmol) and ethyl 2-amino-5-(4-chlorophenyl)indan-2-carboxylate hydrochloride (105 mg, 0.30 mmol).
   ¹H-NMR (300MHz, CDCl₃) δ: 7.56 (1H, s), 7.47-7.45 (2H, d), 7.40-7.34 (4H, m), 7.26-7.21 (1H, t), 7.14-7.12 (2H, d), 6.95-6.90 (2H, d), 5.98 (1H, s), 4.92 (2H, s), 4.23-4.11 (2H, q), 3.69-3.61 (2H, m), 3.48 (2H, s), 3.28-3.20 (2H, m), 2.47 (3H, s), 1.28-1.19 (3H, t)
   LC-MS 545 [M+H]⁺
2) The title compound (113 mg, 83%) was obtained as white crystals by methods similar to those of Example B117-2) from ethyl 5-(4-chlorophenyl)-2-[({4-[(2-methyl-1,3-oxazol-4-yl)methoxy]phenyl}acetyl)amino]indan-2-carboxylate (145 mg, 0.26 mmol).
   ¹H-NMR (300MHz, DMSO-d₆) δ: 12.40 (1H, s), 8.65 (1H, s), 8.01 (1H, s), 7.68-7.65 (2H, d), 7.51-7.45 (4H, m), 7.32-7.29 (1H, d), 7.14-7.11 (2H, d), 6.91-6.88 (2H, d), 4.87 (2H, s), 3.58-3.49 (2H, m), 3.34 (2H, s), 3.25-3.16 (2H, m), 2.40 (3H, s)
   LC-MS 517 [M+H]⁺
   Melting point: 187-189°C
   Elemental analysis: Calcd. for C₂₉H₂₅N₂O₅Cl: C 67.38, H 4.87, N 5.42; Found: C 67.36, H 4.86, N 5.27

### Example B157

### 5-(4-Chlorophenyl)-2-({4-[(2-methyl-1H-benzimidazol-1-yl)methyl]benzoyl}amino)indan-2-carboxylic acid

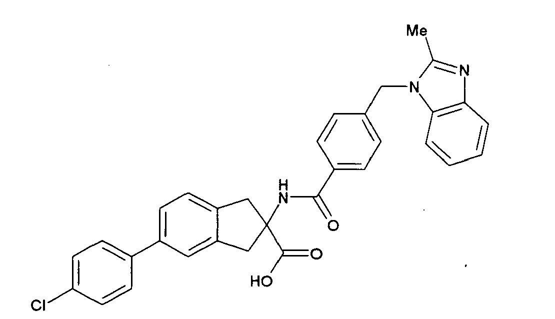

1) Ethyl 5-(4-chlorophenyl)-2-({4-[(2-methyl-1H-benzimidazol-l-yl)methyl]benzoyl}amino)indan-2-carboxylate (111 mg, 66%) was obtained as a colorless oil by methods similar to those of Example B117-1) from 4-[(2-methyl-1H-benzimidazol-1-yl)methyl]benzoic acid hydrochloride (121 mg, 0.40 mmol) and ethyl 2-amino-5-(4-chlorophenyl)indan-2-carboxylate hydrochloride (105 mg, 0.30 mmol).
   ¹H-NMR (300MHz, CDCl₃) δ: 7.73-7.71 (3H, m), 7.49-7.45 (2H, d), 7.40-7.37 (4H, m), 7.28-7.12 (4H, m), 7.08-7.05 (2H, d), 6.77 (1H, s), 5.34 (2H, s), 4.29-4.21 (2H, q), 3.79-3.71 (2H, m), 3.51-3.43 (2H, m), 2.53 (3H, s), 1.28-1.19 (3H, t)
   LC-MS 564 [M+H]⁺
2) The title compound (64 mg, 60%) was obtained as white crystals by methods similar to those of Example B117-2) from ethyl 5-(4-chlorophenyl)-2-({4-[(2-methyl-1H-benzimidazol-1-yl)methyl]benzoyl}amino)indan-2-carboxylate (111 mg, 0.20 mmol).
   ¹H-NMR (300MHz, DMSO-d₆) δ: 12.46 (1H, s), 8.83 (1H, s), 7.77-7.75 (2H, d, J=7.5Hz), 7.67-7.64 (2H, d, J=8.7Hz), 7.56-7.39 (6H, m), 7.31-7.29 (1H, d, J=8.1Hz), 7.19-7.16 (4H, m), 5.52 (2H, s), 3.65-3.56 (2H, m), 3.44-3.36 (2H, m), 2.51(3H, s)
   LC-MS 536 [M+H]⁺
   Melting point: 258-259°C
   Elemental analysis: Calcd. for C₃₂H₂₆N₃O₃Cl•0.4 H₂O: C 70.75, H 4.97, N 7.75; Found: C 70.86, H 5.04, N 7.51

### Example B158

### 2-({[4-(1,3-Benzothiazol-2-yloxy)phenyl]acetyl}amino)-5-(4-chlorophenyl)indan-2-carboxylic acid

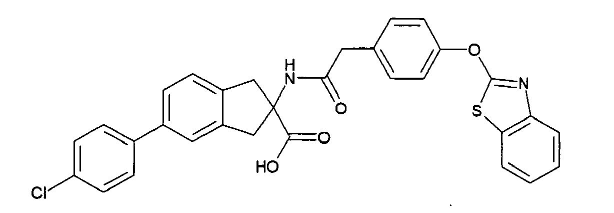

1) Ethyl 2-({[4-(1,3-benzothiazol-2-yloxy)phenyl]acetyl}amino)-5-(4-chlorophenyl)indan-2-carboxylate (163 mg, 93%) was obtained as a colorless oil by methods similar to those of Example B117-1) from 4-(1,3-benzothiazol-2-yloxy)phenylacetic acid (114 mg, 0.40 mmol) and ethyl 2-amino-5-(4-chlorophenyl)indan-2-carboxylate hydrochloride (105 mg, 0.30 mmol).
   ¹H-NMR (300MHz, CDCl₃) δ: 7.73-7.61 (2H, m), 7.48-7.45 (2H, d), 7.42-7.24 (11H, m), 6.08 (1H, s), 4.25-4.18 (2H, q), 3.72-3.64 (2H, m), 3.57 (2H, s), 3.33-3.26 (2H, m), 1.26-1.23 (3H, t)
   LC-MS 583 [M+H]⁺
2) 1 N-NaOH aqueous solution (500 µL) was added with ice cooling to a methanol (1 mL)-THF (1 mL) solution of ethyl 2-({[4-(1,3-benzothiazol-2-yloxy)phenyl]acetyl}amino)-5-(4-chlorophenyl)indan-2-carboxylate (146 mg, 0.25 mmol), and stirred overnight at room temperature. 1 N-hydrochloric acid (600 µL) was added, and the mixture was extracted twice with ethyl acetate. The organic layers were combined, washed with saturated sodium chloride solution, dried by addition of magnesium sulfate, and concentrated under reduced pressure. The residue was recrystallized from a mixed ethyl acetate-hexane solvent to obtain the title compound (35.9 mg, 25%) as white crystals.
   ¹H-NMR (300MHz, DMSO-d₆) δ: 12.40 (1H, s), 8.76 (1H, s), 7.94-7.91 (1H, d), 7.71-7.65 (3H, m), 7.52-7.40 (5H, m), 7.35-7.30 (6H, m), 3.60-3.50 (4H, m), 3.32-3.26 (2H, m)
   LC-MS 555 [M+H]⁺
   Melting point: 241-243°C
   Elemental analysis: Calcd. for C₃₁H₂₃N₂O₄SCl•0.5 H₂O: C 65.59, H 4.33, N 4.93; Found: C 65.51, H4.24, N4.84

### Example B159

### 5-(4-Chlorophenyl)-2-{[(2-methyl-1H-benzimidazol-1-yl)acetyl]amino}indan-2-carboxylic acid hydrochloride

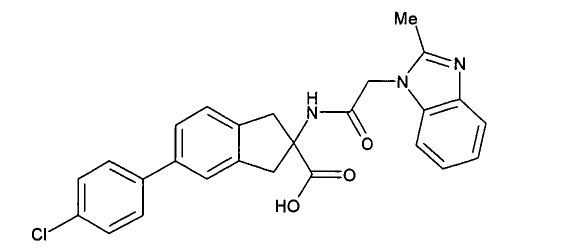

1) 2-Methyl-1H-benzimidazole (82 mg, 0.62 mmol) and potassium carbonate (107 mg, 0.78 mmol) were added to a DMF solution of ethyl 2-[(bromoacetyl)amino]-5-(4-chlorophenyl)indan-2-carboxylate (226 mg, 0.52 mmol), and stirred overnight at 80°C. Water was added, the reaction was completed, and the mixture was extracted twice with ethyl acetate. The organic layers were combined, washed with sodium bicarbonate solution and saturated sodium chloride solution, and dried by addition of magnesium sulfate. The drying agent was filtered out, and the filtrate was concentrated under reduced pressure. The residue was purified by silica gel chromatography (eluate: ethyl acetate/hexane = 1/1-1/10) to obtain ethyl 5-(4-chlorophenyl)-2-{[(2-methyl-1H-benzoimidazol-1-yl)acetyl]amino}indan-2-carboxylate (173 mg, 69%) as a colorless oil.
   ¹H-NMR (300MHz, CDCl₃) δ: 7.47-7.17 (11H, m), 6.08 (1H, s), 4.73 (2H, s), 4.25-4.18 (2H, q), 3.67-3.60 (2H, m), 3.24-3.17 (2H, m), 2.63 (3H, s), 1.26-1.23 (3H, t)
2) The title compound (46 mg, 54%) was obtained as white crystals by methods similar to those of Example B123-3) from ethyl 5-(4-chlorophenyl)-2-{[(2-methyl-1H-benzimidazol-1-yl)acetyl]amino}indan-2-carboxylate (87 mg, 0.18 mmol).
   ¹H-NMR (300MHz, DMSO-d₆) δ: 12.70 (1H, s), 9.39 (1H, s), 7.82-7.78 (2H, m), 7.68-7.66 (2H, d), 7.55-7.49 (6H, m), 7.36-7.33 (1H, d, J=7.8Hz), 5.22 (2H, s), 3.59-3.53 (2H, m), 3.32-3.27 (2H, m), 2.76 (3H, s)
   Melting point: 284-285°C
   LC-MS 460 [M+H]⁺
   Elemental analysis: Calcd. for C₂₆H₂₂N₃O₃Cl•HCl-0.1**·**H₂O: C 60.07, H 4.96, N 8.08; Found: C 59.73, H 4.56, N 7.84

### Example B160

### 5-(2,6-Dimethylphenyl)-2-[({4-[(4-fluorobenzyl)oxy]phenyl}acetyl)amino]indan-2-carboxylic acid

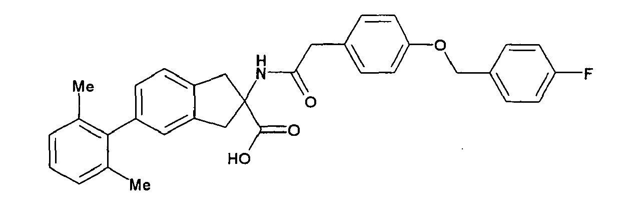

1) Ethyl 2-[(tert-butoxycarboxyl)amino]-1-(2,6-dimethylphenyl)indan-2-carboxylate (660 mg, 62%) was obtained as a colorless oil by methods similar to those of Example B131-2) from ethyl 5-bromo-2-[(tert-butoxycarbonyl)amino]indan-2-carboxylate (1.0 g, 2.6 mmol) and 2,6-dimethylphenylboronic acid (520 mg, 3.1 mmol).
   ¹H-NMR (300MHz, CDCl₃) δ: 7,24-7.07 (4H, m), 6.96-6.94 (2H, d), 5.13 (1H, s), 4.27-4.20 (2H, q), 3.71-3.63 (2H, m), 3.29-3.20(2H, m), 2.04 (3H, s), 2.02 (3H, s), 1.42 (9H, s), 1.29-1.24 (3H, t)
2) An ethyl acetate solution (4 mL) of 4 N-HCl was added to an ethyl acetate solution of ethyl 2-[(tert-butoxycarboxyl)amino]-1-(2,6-dimethylphenyl)indan-2-carboxylate (660 mg, 1.61 mmol), and stirred overnight at 40°C. The precipitated solids were filtered out to obtain ethyl 2-amino-5-(2,6-dimethylphenyl)indan-2-carboxylate hydrochloride (447 mg, 80%) as a white solid. ¹H-NMR (300MHz, CDCl₃) δ: 8.92 (3H, s,), 7.38-7.35 (2H, d, J=7.5Hz), 7.20-6.98 (5H, m), 4.28-4.21 (2H, q), 3.65-3.58 (2H, m), 3.42-3.33 (2H, m), 2.00 (3H, s), 1.96 (3H, s), 1.30-1.15 (3H, t)
   LC-MS 309 [M+H]⁺
3) Ethyl 5-(2,6-dimethylphenyl)-2-[({4-[(4-fluorobenzyl)oxy]phenyl}acetyl)amino]indan-2-carboxylate (70 mg, 43%) was obtained as a white solid by methods similar to those of Example B117-1) from ethyl 2-amino-5-(2,6-dimethylphenyl)indan-2-carboxylate hydrochloride (104 mg, 0.30 mmol) and 4-(4-fluorobenzyloxy)phenylacetic acid (125 mg, 0.50 mmol).
   ¹H-NMR (300MHz, CDCl₃) δ: 7.42-7.37 (2H, m), 7.21-7.04 (8H, m), 6.95-6.88 (4H, m), 5.99 (1H, s), 5.00 (2H, s), 4.23-4.11 (2H, q), 3.67-3.61 (2H, d), 3.51(2H, s), 3.27-3.21 (2H, m), 2.04-2.01 (6H, s), 1.28-1.18 (3H, t) LC-MS 552 [M+H]⁺
4) The title compound (48 mg, 71%) was obtained as white crystals by methods similar to those of Example B117-2) from ethyl 5-(2,6-dimethylphenyl)-2-[({4-[(4-fluorobenzyl)oxy]phenyl}acetyl)amino]indan-2-carboxylate (70 mg, 0.13 mmol).
   ¹H-NMR (300MHz, DMSO-d₆) δ: 12.39 (1H, s), 8.66 (1H, s), 7.50-7.45 (2H, m), 7.29-7.08 (8H, m), 6.96 (1H, s), 6.91-6.88 (3H, m), 5.04 (2H, s), 3.57-3.49 (2H, m), 3.35 (2H, s), 3.24-3.17 (2H, m), 1.97 (3H, s), 1.96 (3H, s)
   LC-MS 524 [M+H]⁺
   Melting point: 210-212°C
   Elemental analysis: Calcd. for C₃₃H₃₀NO₄F•0.2 H₂O: C 75.18, H 5.78, N 2.68; Found: C 75.26, H5.80,N2.54

### Example B161

### 5-(4-Chlorophenyl)-2-({[4-(4-fluorophenoxy)phenyl]acetyl}amino)indan-2-carboxylic acid

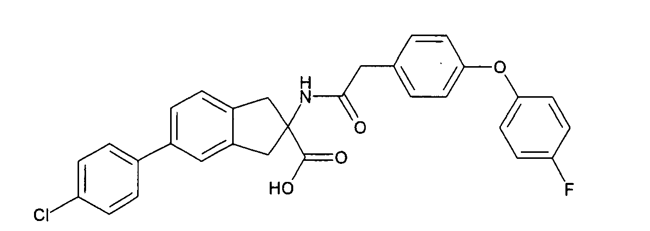

1) Ethyl 5-(4-chlorophenyl)-2-(({[4-(4-ftuorophenoxy)phenyl] acety!} amino)indan-2-carboxylate (155 mg, 100%) was obtained as a colorless oil by methods similar to those of Example B117-1) from 4-(4-fluorophenoxy)phenylacetic acid (96 mg, 0.40 mmol) and ethyl 2-amino-5-(4-chlorophenyl)indan-2-carboxylate hydrochloride (105 mg, 0.30 mmol).
   ¹H-NMR (300MHz, DMSO-d₆) δ: 8.85 (1H, s), 7.67-7.65 (2H, d), 7.53-7.47 (4H, m), 7.33-7.31 (1H, d), 7.25-7.18 (4H, m), 7.04-6.99 (2H, m), 6.94-6.90 (2H, d), 4.05-3.98 (2H, q), 3.59-3.51 (2H, m), 3.39 (2H, s), 3.26-3.17 (2H, m), 1.06-1.01 (3H, t)
   LC-MS 544 [M+H]⁺
2) 1 N-NaOH aqueous solution (600 µL) was added with ice cooling to a methanol (1 mL)-THF (1 mL) solution of ethyl 5-(4-chlorophenyl)-2-({[4-(4-fluorophenoxy)phenyl]acetyl}amino)indan-2-carboxylate (163 mg, 0.30 mmol), and stirred overnight at room temperature. 1 N-hydrochloric acid (650 µL) was added, and the mixture was extracted twice with ethyl acetate. The organic layers were combined, washed with saturated sodium chloride solution, dried by addition of magnesium sulfate, and concentrated under reduced pressure. The residue was recrystallized from a mixed ethyl acetate-hexane solvent to obtain the title compound (119 mg, 77%) as white crystals.
   ¹H-NMR (300MHz, DMSO-d₆) δ: 12.43 (1H, s), 8.70 (1H, s), 7.67-7.64 (2H, d, J=8.4Hz), 7.51-7.45 (4H, m), 7.32-7.29 (1H, d, J=7.8Hz), 7.23-7.17 (4H, m), 7.05-7.01 (2H, m), 6.90-6.88 (2H, d, J=8.7Hz), 3.58-3.49 (2H, m), 3.40 (2H, s), 3.26-3.17 (2H, m)
   LC-MS 516 [M+H]⁺
   Melting point: 232-234°C
   Elemental analysis: Calcd. for C₃₀H₂₃NO₄ClF: C 69.84, H 4.49, N 2.71; Found: C 69.60, H 4.51, N 2.57

### Example B162

### 5-(4-Chlorophenyl)-2-[({4-[(4-fluorobenzyl)amino]phenyl}acetyl)amino]indan-2-carboxylic acid

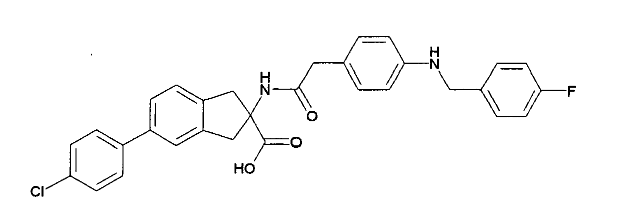

1) Ethyl 5-(4-chlorophenyl)-2-[({4-[(4-fluorobenzyl)amino]phenyl}acetyl)amino]indan-2-carboxylate (70 mg, 42%) was obtained as a colorless oil by methods similar to those of Example B117-1) from 4-(4-fluorobenzylamino)phenylacetic acid (96 mg, 0.33 mmol) and ethyl 2-amino-5-(4-chlorophenyl)indan-2-carboxylate hydrochloride (105 mg, 0.30 mmol).
   ¹H-NMR (300MHz, DMSO-d₆) δ: 8.69 (1H, s), 7.67-7.64 (2H, d), 7.51-7.45 (4H, m), 7.38-7.28 (3H, m), 7.14-7.09 (2H, t), 6.90-6.87 (2H, d), 6.47-6.44 (2H, d), 6.13-6.09 (1H, t), 4.22-4.20 (2H, d), 4.02-3.95 (2H, q), 3.57-3.48 (2H, m), 3.25-3.14 (4H, m), 1.05-1.00 (3H, t)
   LC-MS 557 [M+H]⁺
2) 1 N-NaOH aqueous solution (250 µL) was added with ice cooling to a methanol (1 mL)-THF (1 mL) solution of ethyl 5-(4-chlorophenyl)-2-[({4-[(4-fluorobenzyl)amino]phenyl}acetyl)amino]indan-2-carboxylate (70 mg, 0.13 mmol), and stirred overnight at room temperature. 1 N-hydrochloric acid (300 µL) was added, and the mixture was extracted twice with ethyl acetate. The organic layers were combined, washed with saturated sodium chloride solution, dried by addition of magnesium sulfate, and concentrated under reduced pressure. The residue was recrystallized from a mixed ethyl acetate-hexane solvent to obtain the title compound (42 mg, 64%) as white crystals.
   ¹H-NMR (300MHz, DMSO-d₆) δ: 12.36 (1H, s), 8.56 (1H, s,), 7.67-7.64 (2H, d), 7.51-7.44 (4H, m), 7.3 8-7.27 (3H, m), 7.15-7.09 (2H, m), 6.89-6.86 (2H, d), 6.47-6.44 (2H, d), 6.10 (1H, m), 4.21-4.19 (2H, d), 3.56-3.47 (2H, m), 3.23-3.14 (4H, m)
   LC-MS 529 [M+H]⁺
   Melting point: 188-190°C

### Example B163

### 5-(4-Chlorophenyl)-2-({[4-(pyridin-4-ylmethoxy)phenyl]acetyl}amino)indan-2-carboxylic acid

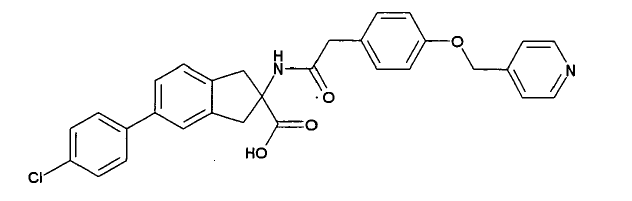

1) Ethyl 5-(4-chlorophenyl)-2-({[4-(pyridin-4-ylmethoxy)phenyl]acetyl}amino)indan-2-carboxylate (151 mg, 93%) was obtained as a colorless oil by methods similar to those of Example B117-1) from 4-(pyridin-4-ylmethoxy)phenylacetic acid (106 mg, 0.40 mmol) and ethyl 2-amino-5-(4-chlorophenyl)indan-2-carboxylate hydrochloride (105 mg, 0.30 mmol).
   ¹H-NMR (300MHz, CDCl₃) δ: 8.62-8.61 (2H, d), 7.49-7.47 (2H, d), 7.45-7.32 (6H, m), 7.24-7.16 (3H, m), 6.91-6.89 (2H, d), 6.00 (1H, s), 5.06 (2H, s), 4.23-4.16 (2H, q), 3.69-3.61 (2H, m), 3.49 (2H, s), 3.29-3.22 (2H, m), 1.23-1.18 (3H, t)
   LC-MS 541 [M+H]⁺
2) The title compound (90 mg, 63%) was obtained as yellow crystals by methods similar to those of Example B117-2) from ethyl 5-(4-chlorophenyl)-2-({[4-(pyridin-4-ylmethoxy)phenyl]acetyl}amino)indan-2-carboxylate (151 mg, 0.28 mmol).
   ¹H-NMR (300MHz, DMSO-d₆) δ: 12.40 (1H, s), 8.64 (1H, s), 8.57-8.55 (2H, d), 7.67-7.65 (2H, d), 7.50-7.40 (6H, m), 7.31-7.29 (1H, d), 7.15-7.12 (2H, d), 6.92-6.89 (2H, d), 5.14 (2H, s), 3.58-3.49 (2H, m), 3.34 (2H, s), 3.25-3.21 (2H, m)
   Melting point: 128°C
   Elemental analysis: Calcd. for C₃₀H₂₅N₂O₄Cl•0.5 H₂O: C 69.03, H 5.02, N 5.37; Found: C 68.89, H 5.07, N 5.10

### Example B164

### 5-(4-Chlorophenyl)-2-({[4-(3-thienyloxy)phenyl]acetyl}amino)indan-2-carboxylic acid

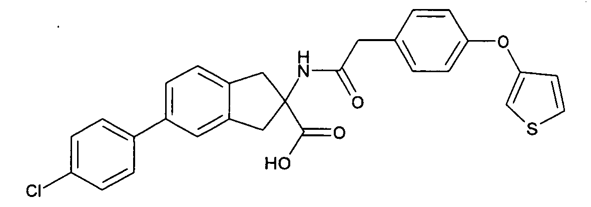

1) Ethyl 5-(4-chlorophenyl)-2-({[4-(3-thienyloxy)phenyl]acetyl}amino)indan-2-carboxylate (159 mg, 100%) was obtained as a colorless oil by methods similar to those of Example B117-1) from 4-(3-thienyloxy)phenylacetic acid (94 mg, 0.40 mmol) and ethyl 2-amino-5-(4-chlorophenyl)indan-2-carboxylate hydrochloride (105 mg, 0.30 mmol).
   ¹H-NMR (300MHz, CDCl₃) δ: 7.49-7.35 (6H, m), 7.26-7.18 (4H, m), 7.01-6.99 (2H, d), 6.84-6.82 (1H, d), 6.59-6.58 (1H, m), 6.02 (1H, s), 4.16-4.09 (2H, m), 3.69-3.62 (2H, m), 3.51 (2H, s), 3.31-3.23 (2H, m), 1.24-1.19 (3H, t)
   LC-MS 532 [M+H]⁺
2) The title compound (93 mg, 62%) was obtained as white crystals by methods similar to those of Example B117-2) from ethyl 5-(4-chlorophenyl)-2-({[4-(3-thienyloxy)phenyl]acetyl}amino)indan-2-carboxylate (174 mg, 0.30 mmol).
   ¹H-NMR (300MHz, DMSO-d₆) δ: 12.40 (1H, s), 8.68 (1H, s), 7.67-7.65 (2H, d), 7.56-7.45 (5H, m), 7.31-7.29 (1H, d), 7.22-7.20 (2H, d), 6.96-6.85 (4H, m), 3.58-3.49 (2H, m,), 3.39 (2H, s), 3.26-3.17 (2H, m)
   Melting point: 226-228°C
   Elemental analysis: Calcd. for C₂₈H₂₂NO₄SCI: C 66.73, H 4.40, N 2.78; Found: C 66.66, H 4.36, N 2.56

### Example B165

### 5-(4-Chlorophenyl)-2-[({4-[(3,5-dimethylisoxazol-4-yl)methoxy]phenyl}acetyl)amino]indan-2-carboxylic acid

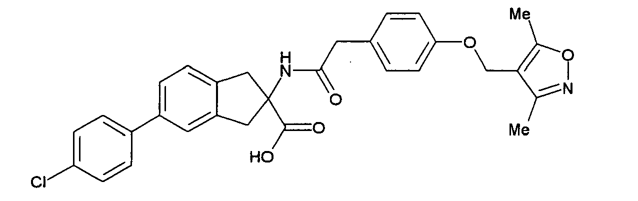

1) Ethyl 5-(4-chlorophenyl)-2-[({4-[(3,5-dimethytisoxazol-4-yl)methoxy]phenyl}acetyl)amino]indan-2-carboxylate (104 mg, 62%) was obtained as a colorless oil by methods similar to those of Example B117-1) from 4-[(3,5-dimethylisoxazol-4-yl)methoxy]phenylacetic acid (102 mg, 0.40 mmol) and ethyl 2-amino-5-(4-chlorophenyl)indan-2-carboxylate hydrochloride (105 mg, 0.30 mmol).
   ¹H-NMR (300MHz, CDCl₃) δ: 7.49-7.45 (2H, d), 7.40-7.35 (4H, m), 7.25-7.17 (3H, m), 6.91-6.90 (2H, d), 6.02 (1H, s), 4.76 (2H, s), 4.24-4.16 (2H, m), 3.70-3.62 (2H, m), 3.49 (2H, s), 3.31-3.23 (2H, m), 2.39 (3H, s), 2.28 (3H, s), 1.28-1.19 (3H, m)
   LC-MS 559 [M+H]⁺
2) The title compound (74 mg, 75%) was obtained as white crystals by methods similar to those of Example B117-2) from ethyl 5-(4-chlorophenyl)-2-[({4-[(3,5-dimethylisoxazol-4-yl)methoxy]phenyl}acetyl)amino]indan-2-carboxylate (104 mg, 0.18 mmol).
   ¹H-NMR (300MHz, DMSO-d₆) δ: 12.41 (1H, s), 8.66 (1H, s), 7.68-7.65 (2H, d), 7.51-7.45 (4H, m), 7.31-7.29 (1H, d, J=7.5Hz), 7.15-7.13 (2H, d, J=8.7Hz), 6.91-6.88 (2H, d, J=8.4Hz), 4.86 (2H, s), 3.57-3.49 (2H, m), 3.35 (2H, s), 3.25-3.16 (2H, m), 2.49 (3H, s), 2.38 (3H, s)
   LC-MS 531 [M+H]⁺
   Melting point: 200-201°C
   Elemental analysis: Calcd. for C₃₀H₂₇N₂O₅Cl•0.1 H₂O: C 67.62, H 5.14, N 5.26; Found: C 67.52, H 5.07, N 5.14

### Example B166

### 5-(4-Chlorophenyl)-2-({[1-(4-fluorobenzoyl)piperidin-4-yl]acetyl}amino)indan-2-carboxylic acid

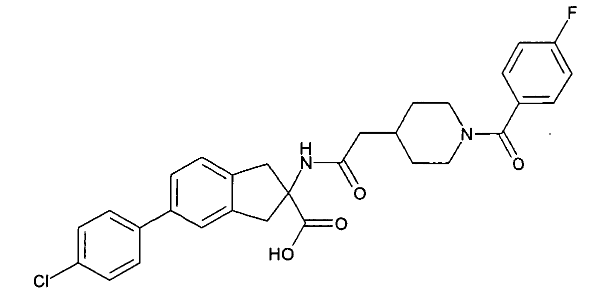

1) Ethyl 5-(4-chlorophenyl)-2-({[1-(4-fluorobenzoyl)piperidin-4-yl]acetyl}amino)indan-2-carboxylate (165 mg, 100%) was obtained as a colorless oil by methods similar to those of Example B117-1) from 1-(4-fluorobenzoyl)piperidin-4-ylacetic acid (101 mg, 0.40 mmol) and ethyl 2-amino-5-(4-chlorophenyl)indan-2-carboxylate hydrochloride (105 mg, 0.30 mmol).
   ¹H-NMR (300MHz, CDCl₃) δ: 7.63-7.61 (2H, d), 7.40-7.35 (6H, m), 7.28-7.25 (1H, m), 7.09-7.04 (2H, m), 6.01 (1H, s), 4.67 (1H, br s), 4.24-4.16 (2H, q), 3.73-3.65 (3H, m), 3.32-3.24 (2H, m), 3.03-2.81 (2H, br s), 2.11 (3H, br s), 1.77 (2H, br s), 1.28-1.16 (5H, m)
   LC-MS 563 [M+H]⁺
2) The title compound (127 mg, 79%) was obtained as white crystals by methods similar to those of Example B117-2) from ethyl 5-(4-chlorophenyl)-2-({[1-(4-fluorobenzoyl)piperidin-4-yl]acetyl}amino)indan-2-carboxylate (165 mg, 0.18 mmol).
   ¹H-NMR (300MHz, DMSO-d₆) δ: 12.41 (1H, s), 8.43 (1H, s), 7.67-7.64 (2H, d, J=8.7Hz), 7.50-7.39 (6H, m), 7.30-7.22 (3H, m), 4.37 (1H, br s), 3.56-3.48 (3H, m), 3.24-3.15 (2H, m), 3.03-2.73 (2H, br s), 2.04-1.93 (3H, m), 1.67-1.62 (2H, br s), 1.19-1.11 (2H, br s)
   LC-MS 535 [M+H]⁺
   Melting point: 242°C
   Elemental analysis: Calcd. for C₃₀H₂₈N₂O₄ClF•0.4 H₂O: C 66.45, H 5.35, N 5.17; Found: C 66.55, H 5.27, N5.10

### Example B167

### 5-(4-Chlorophenyl)-2-[({1-[(4-fluorophenyl)acetyl]piperidin-4-yl}acetyl)amino]indan-2-carboxylic acid

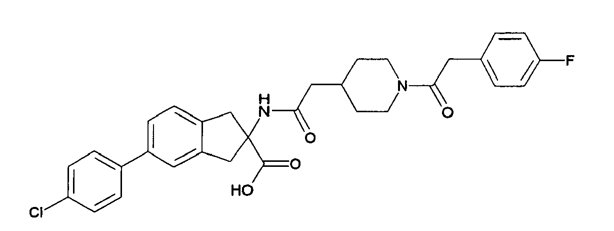

1) Ethyl 5-(4-chlorophenyl)-2-[({1-[(4-fluorophenyl)acetyl]piperidin-4-yl}acetyl)amino]indan-2-carboxylate (154 mg, 89%) was obtained as a colorless oil by methods similar to those of Example B117-1) from 1-[(4-fluorophenyl)acetyl]piperidin-4-ylacetic acid (112 mg, 0.40 mmol) and ethyl 2-amino-5-(4-chlorophenyl)indan-2-carboxylate hydrochloride (105 mg, 0.30 mmol).
   ¹H-NMR (300MHz, CDCl₃) δ: 7.45-7.44 (2H, d), 7.41-7.37 (3H, m), 7.33-7.16 (3H, m), 7.00-6.95 (2H, m), 6.07 (1H, s), 4.63-4.58 (1H, d), 4.26-4.19(2H, q), 3.84-3.80 (2H, d), 3.72-3.64 (4H, m), 3.31-3.23 (2H, m), 3.02-2.93 (1H, m), 2.60-2.51 (1H, m), 2.06-1.98 (3H, m), 1.75-1.62 (2H, m), 1.24-1.22 (3H, m), 1.09-1.01 (1H, m), 0.90-0.85 (1H, m)
   LC-MS 577 [M+H]⁺
2) The title compound (108 mg, 74%) was obtained as white crystals by methods similar to those of Example B117-2) from ethyl 5-(4-chlorophenyl)-2-[({1-[(4-fluorophenyl)acetyl]piperidin-4-yl}acetyl)amino]indan-2-carboxylate (154 mg, 0.27 mmol).
   ¹H-NMR (300MHz, DMSO-d₆) δ: 12.41 (1H, s), 8.43 (1H, s), 7.67-7.64 (2H, d), 7.51-7.44 (4H, m), 7.31-7.26 (3H, m), 7.13-7.07 (2H, t), 4.32-4.28 (1H, d, J=12.6Hz,), 3.91-3.87 (1H, d, J=12.9Hz), 3.67 (2H, t), 3.57-3.48 (2H, m), 3.23-3.14 (2H, m), 2.99-2.91 (1H, t), 1.99-1.87 (2H, m), 1.63-1.59 (2H, d), 1.25 (1H, br s), 0.96-0.88 (3H, m)
   LC-MS 549 [M+H]⁺
   Melting point: 196°C
   Elemental analysis: Calcd. for C₃₁H₃₀N₂O₄ClF•0.2 H₂O: C 67.37, H 5.54, N 5.07; Found: C 67.21, H 5.77, N 5.35

### Example B168

### 2-{[4-(1,3-Benzothiazol-2-ylthio)-butanoyl]amino}-5-(4-chlorophenyl)indan-2-carboxylic acid

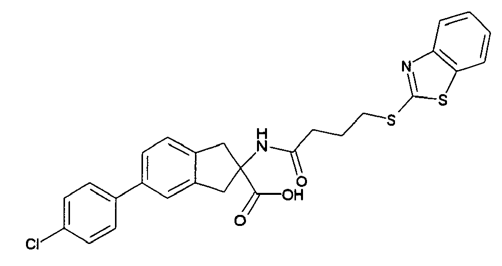

1) Ethyl 2- {[4-(1,3-benzothiazol-2-ylthio)-butanoyl]amino}-5-(4-chlorophenyl)indan-2-carboxylate (140 mg, 85%) was obtained as a colorless oil by methods similar to those of Example B117-1) from 4-(1,3-benzothiazol-2-ylthio)butanic acid (129 mg, 0.50 mmol) and ethyl 2-amino-5-(4-chlorophenyl)indan-2-carboxylate hydrochloride (105 mg, 0.30 mmol).
   ¹H-NMR (300MHz, CDCl₃) δ: 7.78-7.77 (2H, m), 7.49-7.46 (2H, d), 7.39-7.36 (5H, m), 7.27-7.24 (2H, m), 6.34 (1H, s), 4.27-4.20 (2H, q), 3.73-3.66 (2H, m), 3.44-3.39 (2H, t), 3.34-3.27 (2H, m), 2.41-2.36 (2H, t, J=6.0Hz), 2.21-2.16 (2H, t), 1.28-1.22 (3H, t)
   LC-MS 551 [M+H]⁺
2) The title compound (84 mg, 75%) was obtained as white crystals by methods similar to those of Example B117-2) from ethyl 2-{[4-(1,3-benzothiazol-2-ylthio)butanoyl]amino}-5-(4-chlorophenyl)indan-2-carboxylate (120 mg, 0.22 mmol).
   ¹H-NMR (300MHz, DMSO-d₆) δ: 12.42 (1H, br s), 8.51 (1H, s), 8.00-7.97 (1H, d), 7.84-7.81 (1H, d), 7.65-7.62 (2H, m), 7.49-7.27 (7H, s), 3.57-3.47 (2H, m), 3.37-3.17 (4H, m), 2.29-2.24 (2H, t),2.07-1.95 (2H, m)
   LC-MS 523 [M+H]⁺
   Melting point: 210-212°C

### Example 169

### 2-{[(1,3-Benzoxazol-2-ylthio)acetyl]amino}-5-(4-chlorophenyl)indan-2-carboxylic acid

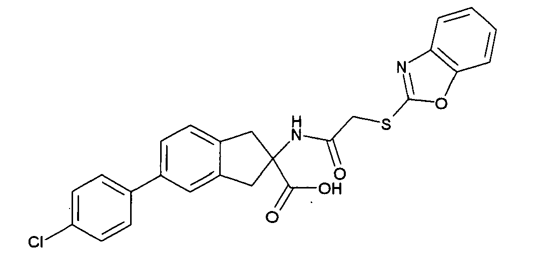

1) Ethyl 2-{[(1,3-benzoxazol-2-ylthio)acetyl]amino}-5-(4-chlorophenyl)indan-2-carboxylate (118 mg, 78%) was obtained as a colorless oil by methods similar to those of Example B117-1) from (1,3-benzoxazol-2-ylthio)acetic acid (107 mg, 0.50 mmol) and ethyl 2-amino-5-(4-chlorophenyl)indan-2-carboxylate hydrochloride (105 mg, 0.30 mmol).
   ¹H-NMR (300MHz, CDCl₃) δ: 8.26 (1H, s), 7.47-7.44 (2H, d), 7.38-7.35 (5H, m), 7.26-7.14 (4H, m), 4.21-4.11 (2H, q), 3.81 (2H, s), 3.68-3.58 (2H, m), 3.29-3.21 (2H, m), 1.23-1.17 (3H, t)
   LC-MS 507 [M+H]⁺
2) The title compound (29 mg, 32%) was obtained as white crystals by methods similar to those of Example B117-2) from ethyl 2-{[(1,3-benzoxazol-2-ylthio)acetyl]amino}-5-(4-chlorophenyl)indan-2-carboxylate (98 mg, 0.19 mmol).
   ¹H-NMR (300MHz, DMSO-d₆) δ:12.42 (1H, s), 8.57 (1H, s), 7.67-7.64 (3H, m), 7.52-7.45 (6H, m), 7.32-7.29 (2H, d), 3.58-3.50 (2H, m), 3.22-3.17 (2H, m), 3.04 (2H, s)
   LC-MS 479 [M+H]⁺
   Melting point: 227-230°C

### Example B170

### 5-(4-Chlorophenyl)-2-{[4-(2-naphthylthio)butanoyl]amino}indan-2-carboxylic acid

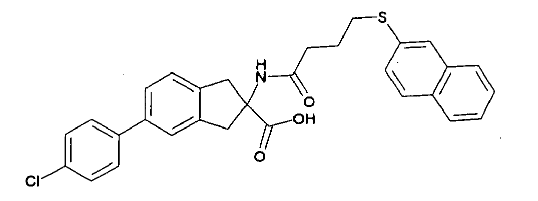

1) Ethyl 5-(4-chlorophenyl)-2-{[4-(2-naphthylthio)butanoyl]amino}indan-2-carboxylate (261 mg, 96%) was obtained as a colorless oil by methods similar to those of Example B117-1) from 4-(2-naphthylthio)butanic acid (297 mg, 0.85 mmol) and ethyl 2-amino-5-(4-chlorophenyl)indan-2-carboxylate (158 mg, 0.50 mmol).
   ¹H-NMR (300MHz, CDCl₃) δ : 7.75-7.70 (4H, m), 7.48-7.34 (9H, m), 7.25-7.23 (1H, d), 6.04 (1H, s), 4.25-4.17 (2H, q), 3.70-3.62 (2H, m), 3.27-3.20 (2H, m), 3.12-3.06 (2H, t), 2.36-2.32 (2H, t), 2.06-1.97 (2H, m), 1.28-1.21 (3H, t)
   LC-MS 544 [M+H]⁺
2) The title compound (150 mg, 69%) was obtained as white crystals by methods similar to those of Example B117-2) from ethyl 5-(4-chlorophenyl)-2-{[4-(2-naphthylthio)butanoyl]amino}indan-2-carboxylate (229 mg, 0.42 mmol).
   ¹H-NMR (300MHz, DMSO-d₆) δ: 12.40 (1H, s), 8.50 (1H, s), 7.86-7.81 (4H, m), 7.65-7.63 (2H, d, J=8.4 Hz), 7.51-7.38 (6H, m), 7.30-7.27 (2H, d, J=7.8Hz), 3.57-3.49 (2H, m), 3.26-3.17 (2H, m), 3.08-3.03 (2H, t, J=7.1Hz), 2.27-2.22 (2H, t, J=7.1Hz), 1.86-1.81 (2H, m)
   LC-MS 516 [M+H]⁺
   Melting point: 210-212°C

### Example B171

### 5-(4-Chlorophenyl)-2-{[(2-naphthylthio)acetyl]amino}indan-2-carboxylic acid

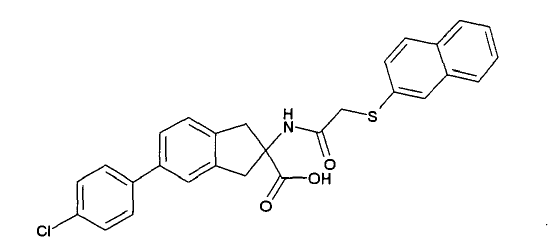

1) Ethyl 5-(4-chlorophenyl)-2-{[(2-naphthylthio)acetyl]amino}indan-2-carboxylate (183 mg, 89%) was obtained as a colorless oil by methods similar to those of Example B117-1) from (2-naphthylthio)acetic acid (148 mg, 0.68 mmol) and ethyl 2-amino-5-(4-chlorophenyl)indan-2-carboxylate (126 mg, 0.40 mmol).
   ¹H-NMR (300MHz, CDCl₃) δ: 7.67-7.61 (4H, m), 7.43-7.38 (4H, m), 7.32-7.24 (5H, m), 7.16 (1H, s), 7.09-7.07 (2H, d), 4.20-4.09 (2H, m), 3.68-3.60 (3H, m), 3.22-3.17 (2H, d), 1.18-1.13 (3H, t)
   LC-MS 516 [M+H]⁺
2) The title compound (123 mg, 81%) was obtained as white crystals by methods similar to those of Example B117-2) from ethyl 5-(4-chlorophenyl)-2-{[(2-naphthylthio)acetyl]amino}indan-2-carboxylate (160 mg, 0.31 mmol).
   ¹H-NMR (300MHz, DMSO-d₆) δ: 12.40 (1H, s), 8.83 (1H, s), 7.81-7.75 (3H, m), 7.67-7.64 (3H, m), 7.51-7.39 (7H, m), 7.29-7.27 (1H, d), 3.74 (2H, s), 3.58-3.49 (2H, m), 3.25-3.16 (2H, m)
   LC-MS 488 [M+H]⁺
   Melting point: 223-225°C
   Elemental analysis: Calcd. for C₂₈H₂₂NO₃SCl•0.1 H₂O: C 68.66, H 4.56, N 2.86; Found: C 68.52, H4.54,N2.82

### Example B172

### 5-(4-Chlorophenyl)-2-{[3-(2-naphthylthio)propanoyl]amino}indan-2-carboxylic acid

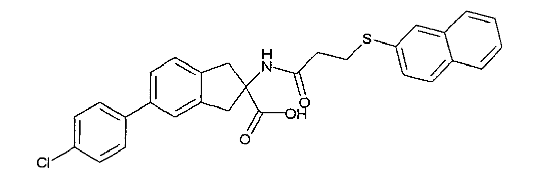

1) Ethyl 5-(4-chlorophenyl)-2-{[3-(2-naphthylthio)propanoyl]amino}indan-2-carboxylate (60 mg, 57%) was obtained as a colorless solid by methods similar to those of Example B117-1) from 3-(2-naphthylthio)propanic acid (80 mg, 0.34 mmol) and ethyl 2-amino-5-(4-chlorophenyl)indan-2-carboxylate (45 mg, 0.20 mmol).
   ¹H-NMR (300MHz, CDCl₃) δ: 7.72-7.67 (4H, m), 7.49-7.26 (10H, m), 6.11 (1H, s), 4.28-4.20 (2H, q), 3.71-3.63 (2H, m), 3.31-3.25 (4H, m), 2.49-2.44 (2H, t), 1.28-1.24 (3H, t)
   LC-MS 530 [M+H]⁺
2) The title compound (27 mg, 45%) was obtained as white crystals by methods similar to those of Example B117-2) from ethyl 5-(4-chlorophenyl)-2-{[3-(2-naphthylthio)propanoyl]amino}indan-2-carboxylate (60 mg, 0.11 mmol).
   ¹H-NMR (300MHz, DMSO-d₆) δ: 12.40 (1H, s), 8.51 (1H, s), 7.86-7.76 (4H, m), 7.66-7.63 (2H, d), 7.50-7.38 (7H, m), 7.30-7.27 (1H, d), 3.56-3.48 (2H, m), 3.24-3.15 (4H, m), 2.47-2.43 (2H, m)
   LC-MS 502 [M+H]⁺
   Melting point: 215°C
   Elemental analysis: Calcd. for C₂₉H₂₄NO₃SCl•0.5 H₂O: C 68.15, H 4.93, N 2.74; Found: C 68.38, H 4.80, N 2.64

### Example B173

### 5-(4-Chlorophenyl)-2-{[(4-methoxyphenyl)acetyl]amino}indan-2-carboxylic acid

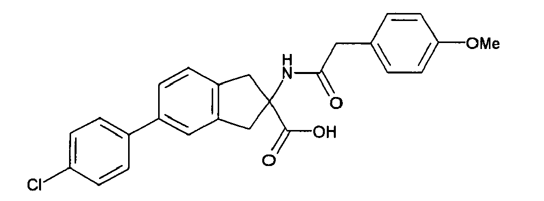

1) Ethyl 5-(4-chlorophenyl)-2-{[(4-methoxyphenyl)acetyl]amino}indan-2-carboxylate (119 mg, 85%) was obtained as a colorless solid by methods similar to those of Example B117-1) from 4-methoxyphenylacetic acid (85 mg, 0.51 mmol) and ethyl 2-amino-5-(4-chlorophenyl)indan-2-carboxylate (105 mg, 0.30 mmol).
   ¹H-NMR (300MHz, CDC1₃) δ: 7.49-7.46 (2H, d), 7.39-7.33 (4H, m), 7.26-7.13 (3H, m), 6.86-6.83 (2H, d), 5.99 (1H, s), 4.23-4.16 (2H, q), 3.78 (3H, s), 3.69-3.61 (2H, m), 3.48 (2H, s), 3.28-3.21 (2H, m), 1.24-1.29 (3H, t)
   LC-MS 464 [M+H]⁺
2) The title compound (26 mg, 30%) was obtained as white crystals by methods similar to those of Example B117-2) from ethyl 5-(4-chlorophenyl)-2-{ [(4-methoxyphenyl)acetyl]amino}indan-2-carboxylate (94 mg, 0.20 mmol).
   ¹H-NMR (300MHz, DMSO-d₆) δ: 12.40 (1H, s), 8.64 (1H, s), 7.67-7.64 (2H, d), 7.51-7.44 (4H, m), 7.31-7.28 (1H, d), 7.13-7.11 (2H, d), 6.83-6.80 (2H, d), 3.70 (3H, s), 3.57-3.49 (2H, m), 3.32 (2H, s), 3.21-3.16 (2H, m)
   LC-MS 436 [M+H]⁺
   Melting point: 231-233°C
   Elemental analysis: Calcd. for C₂₅H₂₂NO₄Cl•0.5 H₂O: C 67.49, H 5.21, N 3.15; Found: C 67.52, H 4.92, N 3.13

### Examples B174

### 2-({[4-(Benzyloxy)phenyl]acetyl}amino)-5-(4-chlorophenyl)indan-2-carboxylic acid

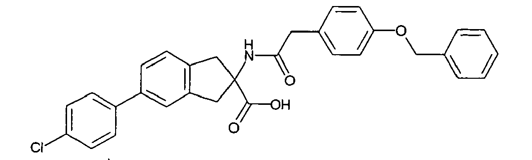

1) WSCD hydrochloride (115 mg, 0.60 mmol), HOBt (81 mg, 0.60 mmol) and triethylamine (168 µL, 1.2 mmol) were added with ice cooling to a DMF (1 mL)-dichloromethane (9 mL) solution of 4-benzyloxyphenylacetic acid (124 mg, 0.51 mmol), and stirred for 30 minutes at room temperature. Ethyl 2-amino-5-(4-chlorophenyl)indan-2-carboxylate hydrochloride (105 mg, 0.30 mmol) was added and stirred overnight at room temperature. Water was added to the reaction mixture, which was then extracted twice with ethyl acetate. The organic layers were combined, washed with water and saturated sodium chloride solution, and dried over magnesium sulfate. The drying agent was filtered out, and the filtrate was concentrated under reduced pressure. The residue was purified by silica gel chromatography (eluate: ethyl acetate/ hexane = 1/4-1/1) to obtain ethyl 2-({[4-(benzyloxy)phenyl]acetyl}amino)-5-(4-chlorophenyl)indan-2-carboxylate (142 mg, 88%) as a colorless oil.
   ¹H-NMR (300MHz, CDCl₃) δ: 7.49-7.34 (10H, m), 7.26-7.21 (1H, t), 7.16-7.13 (2H, d), 6.93-6.90 (2H, d), 5.99 (1H, s), 5.05 (2H, s), 4.23-4.15 (2H, q), 3.68-3.61 (2H, m), 3.48 (2H, s), 3.28-3.21 (2H, m), 1.23-1.19 (3H, t)
   LC-MS 540 [M+H]⁺
2) 1 N-NaOH aqueous solution (1.0 mL) was added with ice cooling to a methanol (2 mL)-THF (2 mL) solution of ethyl 2-({[4-(benzyloxy)phenyl]acetyl}amino)-5-(4-chlorophenyl)indan-2-carboxylate (143 mg, 0.27 mmol), and stirred overnight at room temperature. 1 N-hydrochloric acid (1.1 mL) was added, and the mixture was extracted twice with ethyl acetate. The organic layers were combined, washed with saturated sodium chloride solution, dried by addition of magnesium sulfate, and concentrated under reduced pressure. The residue was recrystallized from a mixed ethyl acetate-hexane solvent to obtain the title compound (62 mg, 45%) as white crystals.
   ¹H-NMR (300MHz, DMSO-d₆) δ: 12.40 (1H, s), 8.65 (1H, s), 7.67-7.64 (2H, d), 7.50-7.29 (10H, m), 7.14-7.10 (2H, d), 6.91-6.88 (2H, d), 5.05 (2H, s), 3.57-3.48 (2H, m), 3.33 (2H, s), 3.24-3.15 (2H,m)
   LC-MS 512 [M+H]⁺
   Melting point: 234°C
   Elemental analysis: Calcd. for C₃₁H₂₆NO₄Cl: C 72.36, H 4.86, N 2.59; Found: C 72.49, H 5.02, N 2.59

### Example B175

### 5-(4-Chlorophenyl)-2-[(pyridin-3-ylacetyl)amino]indan-2-carboxylic acid hydrochloride

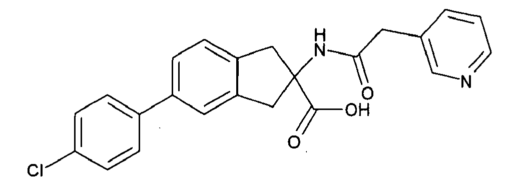

1) Ethyl 5-(4-chlorophenyl)-2-[(pyridin-3-ylacetyl)amino]indan-2-carboxylate (110 mg, 85%) was obtained as a colorless solid by methods similar to those of Example B117-1) from 3-pyridylacetic acid (88 mg, 0.51 mmol) and ethyl 2-amino-5-(4-chlorophenyl)indan-2-carboxylate (105 mg, 0.30 mmol).
   ¹H-NMR (300MHz, CDCl₃) δ: 8.50-8.48 (1H, m), 8.44 -8.43 (1H, d), 7.66-7.64 (1H, d), 7.48-7.45 (2H, d), 7.40-7.35 (4H, m), 7.27-7.22 (2H, m), 6.32 (1H, s), 4.22-4.15 (2H, q), 3.70-3.63 (2H, m), 3.49 (2H, s), 3.32-3.25 (2H, m), 1.21-1.16 (3H, t)
   LC-MS 435 [M+H]⁺
2) The title compound (96 mg, 100%) was obtained as white crystals by methods similar to those of Example B123-3) from ethyl 5-(4-chlorophenyl)-2-[(pyridin-3-ylacetyl)amino]indan-2-carboxylate (92 mg, 0.21 mmol).
   ¹H-NMR (300MHz, DMSO-d₆) δ: 12.5 (1H, br s), 8.80 (1H, s), 8.43 (2H, s), 7.67-7.64 (3H, m), 7.52-7.47 (4H, m), 7.32-7.30 (2H, m), 3.53 (4H, m), 3.32-3.24 (2H, m)
   LC-MS 407 [M+H]⁺
   Melting point: 288°C

### Example B176

### 5-(4-Chlorophenyl)-2-[(1H-indol-3-ylacetyl)amino]indan-2-carboxylic acid

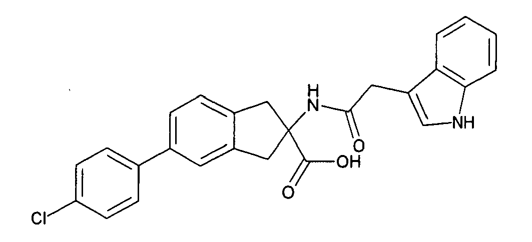

1) Ethyl 5 -(4-chlorophenyl)-2- [(1H-indol-3-ylacetyl) amino] indan-2-carboxylate (122 mg, 86%) was obtained as a colorless solid by methods similar to those of Example B117-1) from 1H-indole-3-acetic acid (89 mg, 0.51 mmol) and ethyl 2-amino-5-(4-chlorophenyl)indan-2-carboxylate (105 mg, 0.30 mmol).
   ¹H-NMR (300MHz, CDCl₃) δ: 8.20 (1H, s), 7.50-7.05 (11H, m), 6.26 (1H, s), 4.23-4.16 (2H, q), 3.70-3.58 (4H, m), 3.20-3.15 (2H, d), 1.24-1.18 (3H, t)
   LC-MS 473 [M+H]⁺
2) The title compound (76 mg, 78%) was obtained as white crystals by methods similar to those of Example B117-2) from ethyl 5-(4-chlorophenyl)-2-[(1H-indol-3-ylacetyl)amino]indan-2-carboxylate (104 mg, 0.22 mmol).
   ¹H-NMR (300MHz, DMSO-d₆) δ: 12.42 (1H, s), 10.81 (1H, s), 8.65 (1H, s), 7.67-7.65 (2H, d), 7.51-7.42 (5H, m), 7.31-7.29 (2H, d), 7.14-7.13 (1H, d), 7.05-7.00 (1H, t), 6.91-6.86 (1H, t), 3.58-3.49 (4H, m), 3.31-3.19 (2H, m)
   LC-MS 445 [M+H]⁺
   Melting point: 209-211°C
   Elemental analysis: Calcd. for C₂₆H₂₁N₂O₃Cl•0.2 H₂O: C 69.63, H 4.81, N 6.24; Found: C 69.80, H 4.82, N 6.24

### Example B177

### 2-[(Biphenyl-4-ylacetyl)amino]-5-(4-chlorophenyl)indan-2-carboxylic acid

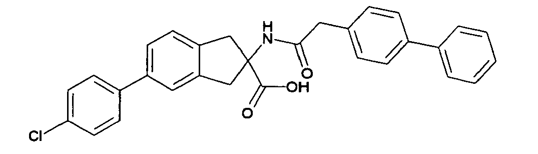

1) Ethyl 2-[(biphenyl-4-ylacetyl)amino]-5-(4-chlorophenyl)indan-2-carboxylate (99 mg, 65%) was obtained as a colorless solid by methods similar to those of Example B117-1) from 4-biphenylacetic acid (108 mg, 0.51 mmol) and ethyl 2-amino-5-(4-chlorophenyl)indan-2-carboxylate (105 mg, 0.30 mmol).
   ¹H-NMR (300MHz, CDCl₃) δ: 7.57-7.26 (16H, m), 6.09 (1H, s), 4.24-4.16 (2H, q), 3.73-3.57 (4H, m), 3.31-3.24 (2H, m), 1.23-1.19 (3H, t)
   LC-MS 510 [M+H]⁺
2) The title compound (58 mg, 67%) was obtained as white crystals by methods similar to those of Example B117-2) from ethyl 2-[(biphenyl-4-ylacetyl)amino]-5-(4-chlorophenyl)indan-2-carboxylate (87 mg, 0.18 mmol).
   ¹H-NMR (300MHz, DMSO-d₆) δ: 12.40 (1H, s), 8.74 (1H, s), 7.67-7.29 (16H, m), 3.59-3.47 (4H, m), 3.31-3.18 (2H, m)
   LC-MS 482 [M+H]⁺
   Melting point: 240-243°C
   Elemental analysis: Calcd. for C₃₀H₂₄NO₃Cl•0.3 H₂O: C 73.93, H 5.09, N 2.87; Found: C 74.00, H 4.94, N2.83

### Example B178

### 5-(4-Chlorophenyl)-2-[(pyridin-2-ylacetyl)amino]indan-2-carboxylic acid

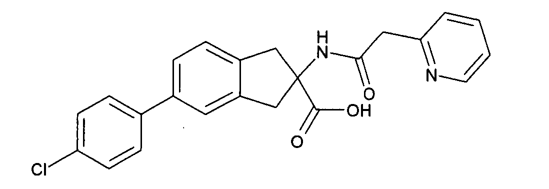

1) Ethyl 5-(4-chlorophenyl)-2-[(pyridin-2-ylacetyl)amino]indan-2-carboxylate (106 mg, 82%) was obtained as a colorless solid by methods similar to those of Example B117-1) from 2-pyridylacetic acid hydrochloride (89 mg, 0.51 mmol) and ethyl 2-amino-5-(4-chlorophenyl)indan-2-carboxylate (105 mg, 0.30 mmol).
   ¹H-NMR (300MHz, CDCl₃) δ: 8.48-8.45 (1H, d), 8.04 (1H, s), 7.65-7.62 (1H, t), 7.49-7.46 (2H, m), 7.41-7.35 (4H, m), 7.27-7.23 (2H, m), 7.17 (1H, m), 4.18-4.11 (2H, q), 3.72-3.64 (4H, m), 3.34-3.27 (2H, m), 1.15-1.11 (3H, t)
   LC-MS 435 [M+H]⁺
2) The title compound (12 mg, 13%) was obtained as white crystals by methods similar to those of Example B123-3) from ethyl 5-(4-chlorophenyl)-2-[(pyridin-2-ylacetyl)amino]indan-2-carboxylate (92 mg, 0.21 mmol).
   ¹H-NMR (300MHz, DMSO-d₆) δ: 12.50 (1H, s), 8.79 (1H, s), 8.44-8.42 (1H, d), 7.70-7.64 (3H, m), 7.51-7.45 (4H, m), 7.33-7.29 (2H, m), 7.24-7.23 (1H, m), 3.62-3.49 (4H, m), 3.31-3.19 (2H, m)
   LC-MS 407 [M+H]⁺
   Melting point: 189°C

### Example B179

### 5-(4-Chlorophenyl)-2-{[(2S)-2-hydroxy-2-phenylacetyl]amino}indan-2-carboxylic acid

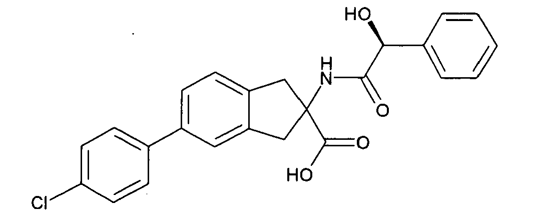

1) Ethyl 5-(4-chlorophenyl)-2-{[(2S)-2-hydroxy-2-phenylacetyl]amino}indan-2-carboxylate (76 mg, 57%) was obtained as a colorless solid by methods similar to those of Example B117-1) from (S)-2-hydroxy-2-phenylacetic acid (78 mg, 0.51 mmol) and ethyl 2-amino-5-(4-chlorophenyl)indan-2-carboxylate (105 mg, 0.30 mmol).
   ¹H-NMR (300MHz, CDCl₃) δ: 7.49-7.21 (12H, m), 6.85 (1H, s), 5.03 (1H, s), 4.20-4.08 (2H, m), 3.72-3.63 (2H, m), 3.35-3.26 (3H, m), 1,23-1,18 (3H, t)
   LC-MS 450 [M+H]⁺
2) The title compound (31 mg, 51%) was obtained as white crystals by methods similar to those of Example B117-2) from ethyl 5-(4-chlorophenyl)-2-{[(2S)-2-hydroxy-2-phenylacetyl]amino}indan-2-carboxylate (66 mg, 0.21 mmol).
   ¹H-NMR (300MHz, DMSO-d₆) δ: 12.60 (1H, s), 8.42 (1H, s), 7.67-7.64 (2H, d), 7.51-7.24 (10H, m), 6.16-6.14 (1H, d), 4.92-4.90 (1H, d), 3.52-3.47 (2H, m), 3.36-3.27 (2H, m)
   LC-MS 422 [M+H]⁺
   Melting point: 156°C

### Example B180

### 5-(4-Chlorophenyl)-2-({[(4-fluorophenyl)amino]carbonyl}amino)indan-2-carboxylic acid

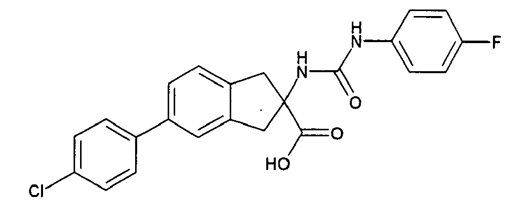

1) 4-Fluorophenyl isocyanate (41 µL, 0.35 mmol) was added to a THF (3 mL)-dichloromethane (3 mL) solution of ethyl 2-amino-5-(4-chlorophenyl)indan-2-carboxylate (105 mg, 0.30 mmol) and triethylamine (42 µL, 0.30 mmol), and stirred overnight. The reaction mixture was concentrated under reduced pressure, and the residue was purified by silica gel chromatography (eluate: ethyl acetate/hexane = 1/4-1/1) to obtain ethyl 5-(4-chlorophenyl)-2-({[(4-fluorophenyl)amino]carbonyl}amino)indan-2-carboxylate (118 mg, 87%) as a white solid. ¹H-NMR (300MHz, CDCl₃) δ: 7.49-7.45 (2H, m), 7.41-7.37 (4H, m), 7.28-7.19 (3H, m), 6.97-6.91 (2H, m), 6.41 (1H, s), 5.30 (1H, s), 4.29-4.22 (2H, q), 3.74-3.66 (2H, m), 3.38-3.30 (2H, m), 1.28-1.18 (3H, t)
   LC-MS 453 [M+H]⁺
2) The title compound (74 mg, 73%) was obtained as white crystals by methods similar to those of Example B117-2) from ethyl 5-(4-chlorophenyl)-2-({[(4-fluorophenyl)amino]carbonyl}amino)indan-2-carboxylate (108 mg, 0.24 mmol).
   ¹H-NMR (300MHz, DMSO-d₆) δ: 12.55 (1H, s), 8.47 (1H, s), 7.67-7.65 (2H, d), 7.53-7.30 (7H, m), 7.07-7.01 (2H, m), 6.85 (1H, s), 3.58-3.49 (2H, m), 3.29-3.19 (2H, m)
   LC-MS 425 [M+H]⁺
   Melting point: 177°C

### Example B181

### 2-({[3-(Benzyloxy)phenyl]acetyl}amino)-5-(4-chlorophenyl)indan-2-carboxylic acid

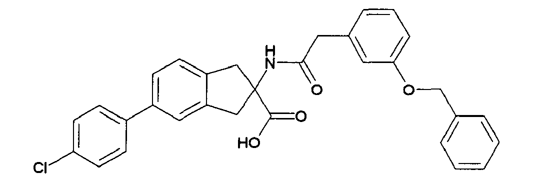

1) Ethyl 2-({[3-(benzyloxy)phenyl]acetyl}amino)-5-(4-chlorophenyl)indan-2-carboxylate (164 mg, 100%) was obtained as a colorless oil by methods similar to those of Example B117-1) from 3-(benzyloxy)phenylacetic acid (123 mg, 0.51 mmol) and ethyl 2-amino-5-(4-chlorophenyl)indan-2-carboxylate (105 mg, 0.30 mmol).
   ¹H-NMR (300MHz, CDCl₃) δ: 7.61-7.33 (11H, m), 7.26-7.22 (2H, d), 6.88-6.82 (3H, m), 6.06 (1H, s), 5.01 (2H, s), 4.22-4.11 (2H, m), 3.68 (2H, s), 3.63-3.55 (2H, m), 3.28-3.20 (2H, m), 1.23-1.18 (3H, t)
   LC-MS 540 [M+H]⁺
2) The title compound (75 mg, 75%) was obtained as white crystals by methods similar to those of Example B117-2) from ethyl 2-({[3-(benzyloxy)phenyl]acetyl}amino)-5-(4-chlorophenyl)indan-2-carboxylate (143 mg, 0.26 mmol).
   ¹H-NMR (300MHz, DMSO-d₆) δ: 12.44 (1H, s), 8.71 (1H, s), 7.66-7.63 (2H, d), 7.51-7.29 (10H, m), 7.19-7.17 (1H, t), 6.90-6.78 (3H, m), 5.02 (2H, s), 3.58-3.49 (2H, m), 3.40 (2H, s), 3.26-3.17 (2H, m)
   LC-MS 512 [M+H]⁺
   Melting point: 207°C
   Elemental analysis: Calcd. for C₃₁H₂₆NO₄Cl•0.2 H₂: C 71.21, H 5.16, N 2.71; Found: C 71.19, H 5.11, N 2.75

### Example B182

### 2-({[4-(Benzyloxy)phenyl]acetyl}amino)-5-(4-chlorophenyl)-N-1H-tetrazol-5-ylindan-2-carboxamide

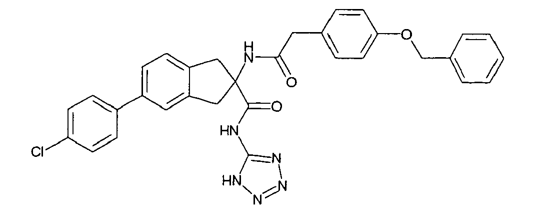

(1) Carbodiimidazole (35.7 mg, 2.2 mmol) was added to a tetrahydrofuran solution (4 mL) of 2-({[4-(benzyloxy)phenyl]acetyl}amino)-5-(4-chlorophenyl)indan-2-carboxylic acid (Example B174) (102 mg, 0.2 mmol) and 5-aminotetrazole (20 mg, 0.22 mmol), and stirred for 1 day at 70-80°C. The precipitated white solid was filtered out, washed with 1 N hydrochloric acid and water, and recrystallized from a mixed tetrahydrofuran-hexane solvent to obtain the title compound (31.8 mg, 27%) as colorless crystals.
   LC-MS 579 [M+H]⁺
   ¹H-NMR (300MHz, DMSO-d₆); δ3.19-3.28 (m, 2H), 3.43 (s, 2H), 3.64-3.71 (m, 2H), 5.04 (s, 2H), 6.86-6.89 (d, J=8.7Hz, 2H), 7.09-7.12 (d, J=8.7Hz, 2H), 7.31-7.41 (m, 6H), 7.43-7.53 (m, 4H), 7.65-7.68 (d, J=8.7Hz, 2H), 8.78 (s, 1H), 11.8 (br s, 1H)

### Example B183

### 5-(4-Chlorophenyl)-2-{[(4-phenoxyphenyl)acetyl]amino}indan-2-carboxylic acid

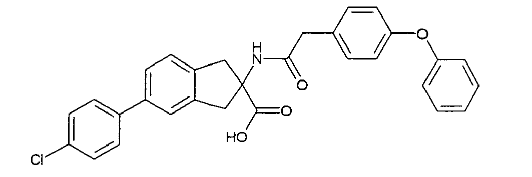

(1) A pyridine-xylene mixed solution (25 mL-25 mL) of methyl 4-hydroxyphenylacetate (1.67 g, 10 mmol), bromobenzene (1.88 g, 12 mmol) and potassium carbonate (2.76 g, 20 mmol) was stirred for 2 hours at 110°C in an argon gas atmosphere. Copper monoxide (1.85 g, 25 mmol) was added and stirred overnight at 140°C. The reaction solution was filtered, the filtrate was concentrated under reduced pressure, and the residue was purified by silica gel column chromatography (eluate: ethyl acetate/hexane = 1/10-1/4) to obtain methyl 4-phenoxyphenylacetate (948 mg, 39%) as a colorless oil.
   LC-MS 243 [M+H]⁺
   ¹H-NMR (300MHz, CDCl₃); δ3.61 (s, 2H), 3.71 (s, 3H), 6.94-7.02 (m, 4H), 7.07-7.12 (m, 1H), 7.22-7.26 (m, 2H), 7.30-7.33 (m, 2H)
(2) 1 N-NaOH aqueous solution (7.8 mL) was added to an ethanol (20 mL) solution of methyl 4-phenoxyphenylacetate (948 mg, 3.9 mmol), and stirred for 5 hours at room temperature. The reaction solution was concentrated under reduced pressure, and 1 N hydrochloric acid aqueous solution (10 mL) was added. The precipitated white solid was filtered out and washed successively with water and ether to obtain 4-phenoxyphenylacetic acid (813 mg, 91%) as a white solid.
   LC-MS 229 [M+H]⁺
   ¹H-NMR (300MHz, CDCl₃); δ 6.92-7.01 (m, 4H), 7.10-7.14 (t, J=6.6Hz, 1H), 7.25-7.28 (d, J=8.4Hz, 2H), 7.35-7.41 (m, 2H)
(3) (3-Dimethylaminopropyl) ethylcarbodiimide hydrochloride (115 mg, 0.60 mmol), 1-hydroxybenzotriazole (81 mg, 0.60 mmol) and triethylamine (168 µL, 1.2 mmol) were added with ice cooling to a DMF (1 mL)-CH₂Cl₂ (9 mL) solution of 4-phenoxyphenylacetic acid (116 mg, 0.51 mmol), and stirred for 30 minutes at room temperature. Ethyl 2-amino-5-(4-chlorophenyl)indan-2-carboxylate (105 mg, 0.30 mmol) was added and stirred overnight at room temperature. Water was added to the reaction mixture, which was then extracted twice with ethyl acetate. The organic layers were combined, washed with water and saturated sodium chloride solution, and dried over magnesium sulfate. The drying agent was filtered out, and the filtrate was concentrated under reduced pressure. The residue was purified by silica gel column chromatography (eluate: ethyl acetate/hexane = 1/4-1/1) to obtain ethyl 5-(4-chlorophenyl)-2-{[(4-phenoxyphenyl)acetyl]amino}indan-2-carboxylate (151 mg, 96%) as a colorless oil.
   LC-MS 526 [M+H]⁺
   ¹H-NMR (300MHz, CDCl₃); δ1.20-1.26 (m, 3H), 3.24-3.32 (m, 2H), 3.54 (s, 2H), 3.62-3.70 (m, 2H), 4.17-4.24 (m, 2H), 6.04-(s, 1H), 6.91-7.01 (m, 4H), 7.08-7.13 (t, J=7.2Hz, 1H), 7.19-7.33 (m, 9H), 7.35-7.38 (d, J=8.4Hz, 2H)
(4) 1 N-NaOH aqueous solution (574 µL) was added with ice cooling to a MeOH (2 mL)-THF (2 mL) solution of ethyl 5-(4-chlorophenyl)-2-{[(4-phenoxyphenyl)acetyl]amino}indan-2-carboxylate (151 mg, 0.287 mmol), and stirred overnight at room temperature. 1 N hydrochloric acid aqueous solution (600 µL) was added, and the mixture was extracted twice with ethyl acetate. The organic layers were combined, washed with saturated sodium chloride solution, dried by addition of magnesium sulfate, and concentrated under reduced pressure. The residue was recrystallized from a mixed ethyl acetate-hexane solvent to obtain the title compound (99 mg, 70%) as white crystals.
   LC-MS 498 [M+H]⁺
   ¹H-NMR (300MHz, DMSO-d₆); 83.18-3,27 (m, 2H), 3.42 (s, 2H), 3.51-3.59 (m, 2H), 6.90-6.99 (m, 4H), 7.10-7.15 (t, J=7.5Hz, 1H), 7.22-7.25 (d, J=8.7Hz, 2H), 7.30-7.40 (m, 3H), 7.46-7.52 (m, 4H), 7.65-7.67 (d, J=8.4Hz, 2H), 8.72 (s, 1H), 12.50 (br s, 1H)
   Melting point: 227-228°C
   Elemental analysis: Calcd. for C₃₀H₂₄NO₄Cl: C 72.36, H 4.86, N 2.81; Found: C 72.32, H 4.90, N 2.63

### Example B184

### 5-(2-Chlorophenyl)-2-[({4-[(4-fluorobenzyl)oxy]phenyl}acetyl}amino}indan-2-carboxylic acid

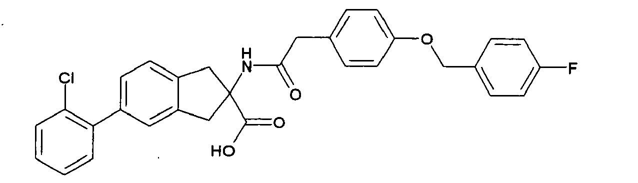

(1) (3-Dimethylaminopropyl)ethylcarbodiimide hydrochloride (1.35 g, 7.04 mmol), 1-hydroxybenzotriazole (950 mg, 7.04 mmol) and triethylamine (3.52 mL, 25 mmol) were added with ice cooling to a DMF (2 mL)-CH₂Cl₂ (18 mL) solution of {4-[(4-fluorobenzyl)oxy]phenyl}acetic acid (1.19 g, 4.58 mmol), and stirred for 30 minutes at room temperature. Ethyl 2-amino-5-bromoindan-2-carboxylate (1.00 g, 3.52 mmol) was added and stirred overnight at room temperature. Water was added to the reaction mixture, which was then extracted twice with ethyl acetate. The organic layers were combined, washed with water and saturated sodium chloride solution, and dried over magnesium sulfate. The drying agent was filtered out, and the filtrate was concentrated under reduced pressure. The residue was purified by silica gel column chromatography (eluate: ethyl acetate/hexane = 1/4-1/1) to obtain ethyl 5-bromo-2-[({4-[(4-fluorobenzyl)oxy]phenyl}acetyl)amino]indan-2-carboxylate (1.50 g, 82%) as a colorless oil.
LC-MS 526 [M+H]⁺
(2) Ethyl 5-bromo-2-[({4-[(4-fluorobenzyl)oxy]phenyl}acetyl)amino]indan-2-carboxylate (316 mg, 0.60 mmol), 2-chlorophenylboronic acid (113 mg, 0.72 mmol), tetrakis triphenylphosphine palladium (27 mg), 2 M sodium carbonate aqueous solution (600 µL), dimethoxyethane (2 mL) and ethanol (300 µL) were stirred overnight at 80°C in an argon gas atmosphere. The reaction solution was filtered, and the filtrate was diluted with ethyl acetate and washed twice with saturated sodium bicarbonate solution and once with saturated sodium chloride solution. The organic layer was dried by addition of anhydrous magnesium sulfate The drying agent was filtered out, and the filtrate was concentrated under reduced pressure. The residue was purified by silica gel column chromatography (eluate: ethyl acetate/hexane = 2/98-1/1) and recrystallized from a mixed ethyl acetate-hexane solvent to obtain ethyl 5-(2-chlorophenyl)-2-[({4-[4-fluorobenzyl)oxy]phenyl}acetyl)amino]indan-2-carboxylate (219 mg, 65%) as a white solid.
LC-MS 558 [M+H]⁺
¹H-NMR (300MHz, CDCl₃); δ1.19-1.24 (t, J=7.2Hz, 3H), 3.21-3.27 (d, J=16.8Hz, 2H), 3.49 (s, 2H), 3.64-3.70 (d, J=16.8Hz, 2H), 4.16-4.23 (q, J=7.2Hz, 3H), 5.00 (s, 2H), 5.97 (s, 1H), 6.89-6.92 (d, J=8.7Hz, 2H), 6.89-6.93 (m, 2H), 7.15-7.17 (d, J=8.7Hz, 2H), 7.21-7.33 (m, 6H), 7.37-7.47 (m, 3H)
(3) 1 N-NaOH aqueous solution (600 µL) was added with ice cooling to a MeOH (2 mL)-THF (2 mL) solution of ethyl 5-(2-chlorophenyl)-2-[({4-[4-fluorobenzyl)oxy]phenyl}acetyl)amino]indan-2-carboxylate (167 mg, 0.30 mmol), and stirred overnight at room temperature. 1 N HCl aqueous solution (650 µL) was added, and the mixture was extracted twice with ethyl acetate. The organic layers were combined, washed with saturated sodium chloride solution, dried by addition of magnesium sulfate, and concentrated under reduced pressure The residue was recrystallized from a mixed ethyl acetate-hexane solvent to obtain the title compound (122 mg, 77%) as white crystals.
LC-MS 530 [M+H]⁺
¹H-NMR (300MHz, DMSO-d₆); δ3.18-3.24 (d, J=16.2Hz, 2H), 3.35 (s, 2H), 3.51-3.57 (d, J=16.8Hz, 2H), 5.04 (s, 2H), 6.88-6.91 (d, J=8.7Hz, 2H), 7.12-7.31 (m, 7H), 7.35-7.40 (m, 3H), 7.41-7.51 (m, 3H), 8.68 (s, 1H), 12.50 (br s, 1H)
Melting point: 213-215°C

| | | | | |
|---|---|---|---|---|
| Elemental analysis: | Calcd. for C₃₁H₂₅NO₄ClF: | C 70.25, | H 4.75, | N 2.64 |
| | Found: | C 70.06, | H 4.77, | N 2.51 |

### Example B185

### 5-(3-Chlorophenyl)-2-[({4-[(4-fluorobenzyl)oxy]phenyl}acetyl)amino]indan-2-carboxylic acid

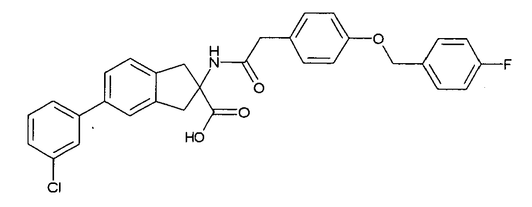

(1) Ethyl 5-(3-chlorophenyl)-2-[({4-[(4-fluorobenzyl)oxy]phenyl}acetyl)amino]indan-2-carboxylate (206 mg, 86%) was obtained as a white solid as in Example B 184(2) from ethyl 5-bromo-2-[({4-[(4-fluorobenzyl)oxy]phenyl}acetyl)amino]indan-2-carboxylate (230 mg, 0.43 mmol) and 3-chlorophenylboronic acid (82 mg, 0.52 mmol).
   LC-MS 558 [M+H]⁺
   ¹H-NMR (300MHz, DMSO-d₆); õ1.01-1.54 (t, J=7.2Hz, 3H), 3.16-3.25 (m, 2H), 3.33 (s, 2H), 3.50-3.59 (m, 2H), 3.97-4.04 (q, J=7.2Hz, 2H), 5.05 (s, 2H), 6.90-6.93 (d, J=8.7Hz, 2H), 7.15-7.24 (m, 4H), 7.31-7.33 (d, J=7.8Hz, 1H), 7.39-7.68 (m, 8H), 8.80 (s, 1H)
(2) The title compound (117 mg, 73%) was obtained as a white solid as in Example B184(3) from ethyl 5-(3-chlorophenyl)-2-[({4-[(4-fluorobenzyl)oxy]phenyl}acetyl)amino]indan-2-carboxylate (167 mg, 0.3 mmol).
   LC-MS 530 [M+H]⁺
   ¹H-NMR (300MHz, DMSO-d₆); δ3.16-3.27 (m, 2H), 3.34 (s, 2H), 3.50-3.58 (m, 2H), 5.04 (s, 2H), 6.88-6.91 (d, J=8.7Hz, 2H), 7.11-7.24 (m, 4H), 7.30-7.32 (d, J=8.1Hz, 1H), 7.39-7.69 (m, 8H), 8.67 (s, 1H), 12.40 (br s, 1H)
   Melting point: 173-174°C
   Elemental analysis: Calcd. for C₃₁H₂₅NO₄ClF: C 70.25, H 4.75, N 2.64; Found: C 70.41, H 4.74, N 2.46

### Example B186

### 5-(5-Chloro-2-thienyl)-2-[({4-[(4-fluorobenzyl)oxy]phenyl}acetyl)amino]indan-2-carboxylic acid

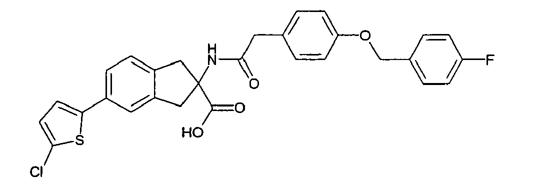

(1) Ethyl 5-(5-chloro-2-thienyl)-2-[({4-[(4-fluorobenzyl)oxy]phenyl}acetyl)amino]indan-2-carboxylate (199 mg, 59%) was obtained as a white solid as in Example B184(2) from ethyl 5-bromo-2-[({4-[(4-fluorobenzyl)oxy]phenyl}acetyl)amino]indan-2-carboxylate (316 mg, 0.60 mmol) and 3-chlorophenylboronic acid (117 mg, 0.72 mmol).
   LC-MS 564 [M+H]⁺
   ¹H-NMR (300MHz, CDCl₃); δ1.21-1.30 (m, 3H), 3.15-3.28 (m, 2H), 3.49 (s, 2H), 3.56-3.66 (m, 2H), 4.13-4.24 (m, 2H), 5.02 (s, 2H), 6.00 (s, 1H), 6.88-6.94 (m, 3H), 7.03-7.18 (m, 6H), 7.28-7.44 (m, 4H)
(2) The title compound (9.3 mg, 5.8%) was obtained as a white solid as in Example B184(3) from ethyl 5-(5-chloro-2-thienyl)-2-[({4-[(4-fluorobenzyl)oxy]phenyl}acetyl)amino]indan-2-carboxylate (169 mg, 0.3 mmol).
   LC-MS 536 [M+H]⁺
   ¹H-NMR (300MHz, DMSO-d₆); δ3.16-3.21 (m, 2H), 3.46-3.54 (m, 4H), 5.04 (s, 2H), 6.88-6.91 (d, J=8.1Hz, 2H), 7.11-7.47 (m, 1H), 8.66 (s, 1H), 12.50 (br s, 1H)
   Melting point: 214-216°C

### Example B187

### 2-{ [(4-{[3-(tert-Butoxycarbonyl)benzyl]oxy}phenyl)acetyl]amino}-5-(4-chlorophenyl)indan-2-carboxylic acid

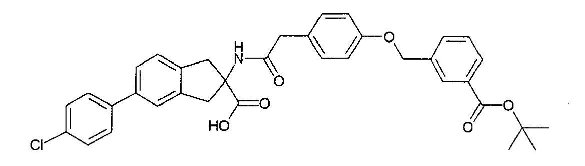

(1) Sodium tetrahydroboride (746 mg, 17.7 mmol) was added with ice cooling to a THF (10 mL)-MeOH (40 mL) solution of tert-butyl 3-formylbenzoate (1.83 g, 8.87 mmol), and stirred for 1 hour with ice cooling. Saturated aqueous ammonium chloride solution was added to complete the reaction. The reaction solution was concentrated under reduced pressure, and extracted twice with ethyl acetate. The organic layer was washed successively with water and saturated sodium chloride solution, and dried over magnesium sulfate. The drying agent was filtered out, and the filtrate was concentrated under reduced pressure to obtain tert-butyl 3-(hydroxymethyl)benzoate (1.83 g, 99%) as a colorless oil.
   ¹H-NMR (300MHz, CDCl₃); δ1.62 (s, 9H), 4.75 (s, 2H), 7.39-7.44 (t, J=7.8Hz, 1H), 7.53-7.56 (d, J=7.5Hz, 1H), 7.91-7.93 (d, J=7.8Hz, 1H), 7.97 (s, 1H)
(2) A toluene solution (2.51 mL, 5.76 mmol) of 40% diethyl azodicarboxylate was added with ice cooling to a THF solution (20 mL) of methyl 4-hydroxyphenylacetate (797 mg, 4.80 mmol), tert-butyl 3-(hydroxymethyl)benzoate (1.0 g, 4.80 mmol) and triphenylphosphine (1.51 g, 5.76 mmol), and stirred overnight at room temperature. The reaction solution was concentrated under reduced pressure, and the residue was purified by silica gel column chromatography (eluate: ethyl acetate/hexane = 2/98-1/2) to obtain tert-butyl 3-{[(2-methoxy-2-oxoethyl)phenoxy]methyl}benzoate (532 mg, 31%) as a colorless oil.
   ¹H-NMR (300MHz, CDCl₃); δ1.60 (s, 9H), 3.57 (s, 2H), 3.69 (s, 3H), 5.08 (s, 2H), 6.93-6.96 (m, 2H), 7.18-7.22 (d, J=8.7Hz, 2H), 7.41-7.46 (t, J=7.8Hz, 1H), 7.59-7.61 (d, J=7.8Hz, 1H), 7.94-7.96 (d, J=7.8Hz, 1H), 8.04 (s, 1H)
(3) 1 N-NaOH aqueous solution (3 mL) was added with ice cooling to a MeOH (6 mL)-THF (5 mL) solution oftert-butyl 3-{[4-(2-methoxy-2-oxoethyl)phenoxy]methyl)benzoate (532 mg, 1.49 mmol), and stirred for 1 hour at room temperature. The reaction solution was concentrated under reduced pressure, 1 N hydrochloric acid aqueous solution (4 mL) was added, and the mixture was extracted twice with ethyl acetate. The organic layers were combined, washed with saturated sodium chloride solution, dried by addition of magnesium sulfate, and concentrated under reduced pressure to obtain (4-{[3-(tert-butoxycarbonyl)benzyl]oxy}phenyl)acetic acid as a colorless oil (502 mg, 98%).
   ¹H-NMR (300MHz, CDCl₃); δ1.60 (s, 9H), 3.59 (s, 2H), 5.08 (s, 2H), 6.92-6.97 (m, 2H), 7.18-7.23 (m, 2H), 7.41-7.46 (t, J=7.5Hz, 1H), 7.59-7.61 (d, J=7.8Hz, 1H), 7.94-7.96 (d, J=7.8Hz, 1H), 8.04 (s, 1H)
(4) Ethyl 2-{[(4-{[3-(tert-butoxycarbonyl)benzyl]oxy}phenyl)acetyl]amino}-5-(4-chlorophenyl)indan-2-carboxylate (298 mg, 82%) was obtained as a colorless oil as in Example B183(3) from (4-{[3-(tert-butoxycarbonyl)benzyl]oxy}phenyl)acetic acid (330 mg, 0.96 mmol) and ethyl 2-amino-5-(4-chlorophenyl)indan-2-carboxylate hydrochloride (200 mg, 0.57 mmol).
   LC-MS 662 [M+H]⁺
   ¹H-NMR (300MHz, CDCl₃); δ1.23-1.28 (m, 3H), 1.60 (s, 9H), 3.21-3.29 (m, 2H), 3.48 (s, 2H), 3.61-3.69 (m, 2H), 4.16-4.23 (q, J=7.2Hz, 2H), 5.06 (s, 2H), 5.99 (s, 1H), 6.91-6.93 (d, J=8.4Hz, 2H), 7.14-7.17 (d, J=8.7Hz, 2H), 7.21-7.26 (m, 1H), 7.34-7.49 (m, 7H), 7.57-7.60 (d, J=7.8Hz, 1H), 7.94-7.96 (d, J=7.8Hz, 1H), 8.04 (s, 1H)
(5) The title compound (191 mg, 100%) was obtained as a white solid as in Example B183(4) from ethyl 2-{[(4-{[3-(tert-butoxycarbonyl)benzyl]oxy}phenyl)acetyl]amino}-5-(4-chlorophenyl)indan-2-carboxylate (200 mg, 0.31 mmol).
   ¹H-NMR (300MHz, DMSO-d₆); δ1.55 (s, 9H), 3.16-3.25 (m, 2H), 3.34 (s, 2H), 3.49-3.57 (m, 2H), 5.13 (s, 2H), 6.90-6.93 (d, J=8.7Hz, 2H), 7.12-7.15 (d, J=8.7Hz, 2H), 7.29-7.31 (d, J=8.7Hz, 1H), 7.45-7.54 (m, 5H), 7.65-7.69 (m, 3H), 7.84-7.87 (d, J=7.8Hz, 1H), 7.96 (s, 1H), 8.66 (s, 1H), 12.40 (br s, 1H)
   Melting point: 177-179°C
   Elemental analysis: Calcd. for C₃₆H₃₄N0₆Cl: C 70.64, H 5.60, N 2.29; Found: C 70.63, H 5.76, N 2.161

### Example B188

### 2-[({4-[(3-Carboxybenzyl)oxy]phenyl}acetyl)amino]-5-(4-chlorophenyl)indan-2-carboxylic acid

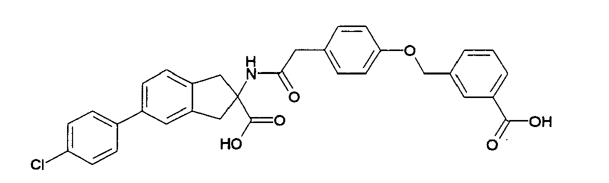

(1) Trifluoracetic acid (2 mL) was added with ice cooling to a dichloromethane (2 mL) solution of 2- {[(4-{[3-(tert-butoxycarbonyl)benzyl]oxy} phenyl)acetyl] amino} -5-(4-chlorophenyl)indan-2-carboxylic acid (Example B187) (100 mg, 0.16 mmol), and stirred for 1 hour at room temperature. The reaction solution was concentrated under reduced pressure, and the residue was recrystallized from ethyl acetate-hexane to obtain the title compound (74 mg, 81%) as white crystals.
   LC-MS 556 [M+H]⁺
   ¹H-NMR (300MHz, DMSO-d₆); δ3.16-3.27 (m, 2H), 3.34 (s, 2H), 3.49-3.57 (m, 2H), 5.14 (s, 2H), 6.90-6.93 (d, J=8.7Hz, 2H), 7.12-7.15 (d, J=8.7Hz, 2H), 7.29-7.31 (d, J=7.8Hz, 1H), 7.45-7.54 (m, 5H), 7.65-7.68 (m, 3H), 7.88-7.90 (d, J=7.5Hz, 1H), 8.01 (s, 1H), 8.66 (s, 1H), 12.40 (br s, 1H), 13.00 (br s, 1H)
   Melting point: 220-222°C
   Elemental analysis: Calcd. for C₃₂H₂₆NO₆Cl: C 68.68, H 4.75, N 2.50; Found: C 68.64, H 4.72, N 2.29

### Example B189

### 2-{[(4-{[2-(tert-Butoxycarbonyl)benzyl]oxy} phenyl)acetyl]amino}-5-(4-chlorophenyl)indan-2-carboxylic acid

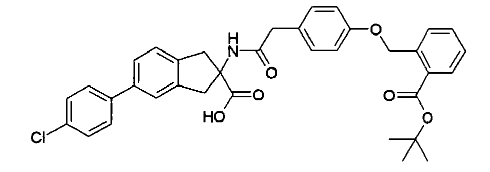

(1) Sodium tetrahydroborate (387 mg, 9.21 mmol) was added with ice cooling to a THF (10 mL)-MeOH (40 mL) solution of tert-butyl 2-formylbenzoate (950 mg, 4.61 mmol), and stirred for 1 hour with ice cooling. Saturated aqueous ammonium chloride solution was added, and stirred for 30 minutes. The reaction solution was concentrated under reduced pressure, and extracted twice with ethyl acetate. The organic layer was washed successively with water and saturated sodium chloride solution, and dried over magnesium sulfate. The drying agent was filtered out, and the filtrate was concentrated under reduced pressure. The residue was purified by silica gel column chromatography (eluate: ethyl acetate/hexane = 2/98-1/2) to obtain tert-butyl 2-(hydroxymethyl)benzoate (730 mg, 76%) as a colorless oil.
   ¹H-NMR (300MHz, CDCl₃); δ 1.62 (s, 9H), 3.97-4.02 (t, J=7.5Hz, 1H), 4.73-4.75 (d, J=7.2Hz, 2H), 7.33-7.52 (m, 3H), 7.91-7.94 (d, J=6.6Hz, 1H)
(2) A toluene solution (3.6 mL, 4.20 mmol) of 40% diethyl azodicarboxylate was added with ice cooling to a THF solution (20 mL) of methyl 4-hydroxyphenylacetate (730 mg, 3.50 mmol), tert-butyl 2-(hydroxymethyl)benzoate (582 mg, 3.50 mmol) and triphenylphosphine (1.10 g, 4.20 mmol), and stirred overnight at room temperature. The reaction solution was concentrated under reduced pressure, and the residue was purified by silica gel column chromatography (eluate: ethyl acetate/hexane = 0/100-1/2) to obtain tert-butyl 2-{[4-(2-methoxy-2-oxoethyl)phenoxy]methyl}benzoate (713 mg, 57%) as a colorless oil.
   ¹H-NMR (300MHz, CDCl₃); δ1.56 (s, 9H), 3.57 (s, 2H), 3.69 (s, 3H), 5.44 (s, 2H), 6.93-6.96 (d, J=9.0Hz, 2H), 7.18-7.21 (d, J=8.7Hz, 2H), 7.33-7.38 (t, J=7.2Hz, 1H), 7.48-7.53 (t, J=7.5Hz, 1H), 7.68-7.71 (d, J=7.5Hz, 1H), 7.93-7.96 (d, J=7.8Hz, 1H)
(3) 1 N-NaOH aqueous solution (4 mL) was added to an EtOH (10 mL)-THF (5 mL) solution of tert-butyl 2-{[4-(2-methoxy-2-oxoethyl)phenoxy]methyl}benzoate (713 mg, 20 mmol), and stirred for 3 hours at room temperature. The reaction solution was concentrated under reduced pressure, and washed with diethyl ether. 1 N HCl aqueous solution (5 mL) was added, and the mixture was extracted twice with ethyl acetate. The organic layers were combined, washed with saturated sodium chloride solution, dried by addition of magnesium sulfate, and concentrated under reduced pressure to obtain (4-{[2-(tert-butoxycarbonyl)benzyl]oxy}phenyl)acetic acid as a colorless oil (560 mg, 82%).
   LC-MS 365 [M+H]⁺
   ¹H-NMR (300MHz, CDC1₃); 61.59 (s, 9H), 3.59 (s, 2H), 5.44 (s, 2H), 6.92-6.96 (d, J=8.7Hz, 2H), 7.17-7.21 (d, J=8.7Hz, 2H), 7.33-7.37 (m, 1H), 7.48-7.53 (m, 1H), 7.68-7.70 (d, J=7.5Hz, 1H), 7.9.3-7.96 (dd, J=1.2, 6.6Hz, 1H)
(4) Ethyl 2-{[(4-{[2-(tert-butoxycarbonyl)benzyl]oxy}phenyl)acetyl]amino}-5-(4-chlorophenyl)indan-2-carboxylate (281 mg, 77%) was obtained as in Example B183(3) as a colorless oil from (4-{[2-(tert-butoxycarbonyl)benzyl]oxy}phenyl)acetic acid (330 mg, 0.96 mmol) and ethyl 2-amino-5-(4-chlorophenyl)indan-2-carboxylate hydrochloride (200 mg, 0.57 mmol).
   LC-MS 662 [M+H]⁺
   ¹H-NMR (300MHz, CDCl₃); δ 1.54 (s, 9H), 3.20-3.28 (m, 2H), 3.49 (s, 2H), 3.61-3.69 (m, 2H), 4.16-4.22 (m, 2H), 5.43 (s, 2H), 5.97 (s, 1H), 6.92-6.95 (d, J=8.7Hz, 2H), 7.13-7.21 (m, 3H), 7.32-7.40 (m, 6H), 7.46-7.51 (m, 3H), 7.67-7.69 (d, J=6.9Hz, 1H), 7.93-7.96 (d, J=9.0Hz, 1H)
(5) The title compound (157 mg, 83%) was obtained as a white solid as in Example B183(4) from ethyl 2-{[(4-{[2-(tert-butoxycarbonyl)benzyl]oxy}phenyl)acetyl]amino}-5-(4-chlorophenyl)indan-2-carboxylate (198 mg, 0.31 mmol).
   ¹H-NMR (300MHz, DMSO-d₆); δ 1.48 (s, 9H), 3.16-3.25 (m, 2H), 3.35 (s, 2H), 3.49-3.58 (m, 2H), 5.31 (s, 2H), 6.85-6.88 (d, J=8.4Hz, 2H), 7.12-7.15 (d, J=8.7Hz, 2H), 7.29-7.31 (d, J=7.8Hz, 1H), 7.42-7.51 (m, 5H), 7.54-7.70 (m, 4H), 7.80-7.83 (d, J=7.2Hz, 1H), 8.67 (s, 1H), 12.40 (br s, 1H)
   Melting point: 170-171°C
   Elemental analysis: Calcd. for C₃₆H₃₄NO₆Cl: C 70.64, H 5.60, N 2.29; Found: C 70.72, H 5.90, N 2.03

### Example B190

### 2-[({4-[(2-Carboxybenzyl)oxy]phenyl}acetyl)amino]-5-(4-chlorophenyl)indan-2-carboxylic acid

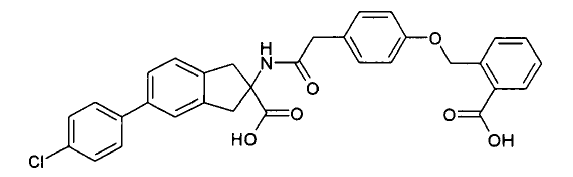

(1) Trifluoracetic acid (1 mL) was added with ice cooling to a dichloromethane solution (1 mL) of 2-{[(4-{[2-(tert-butoxycarbonyl)benzyl]oxy}phenyl)acetyl]amino}-5-(4-chlorophenyl)indan-2-carboxylic acid (Example B189) (100 mg, 0.16 mmol), and stirred overnight at room temperature. The reaction solution was concentrated under reduced pressure, and the residue was recrystallized from a mixed ethyl acetate-hexane solvent to obtain the title compound (75.5 mg, 83%) as white crystals.
   LC-MS 556 [M+H]⁺
   ¹H-NMR (300MHz, DMSO-d₆); δ 3.16-3.25 (m, 2H), 3.34 (s, 2H), 3.34-3.57 (m, 2H), 5.41 (s, 2H), 6.85-6.88 (d, J=8.7Hz, 2H), 7.12-7.15 (d, J=8.7Hz, 2H), 7.29-7.31 (d, J=8.1Hz, 1H), 7.41-7.68 (m, 9H), 7.90-7.93 (d, 1H), 8.67 (s, 1H), 12.50 (br s, 1H), 13.10 (br s, 1H)
   Melting point: 209-211°C
   Elemental analysis: Calcd. for C₃₂H₂₆NO₆Cl: C 68.08, H 4.75, N 2.50; Found: C 68.65, H 4.75, N 2.30

### Example B191

### N-[5-(4-Chlorophenyl)-2-(2H-tetrazol-5-yl)-2,3-dihydro-1H-inden-2-yl]-2-{4-[(4-fluorobenzyl)oxy]phenyl}acetamide

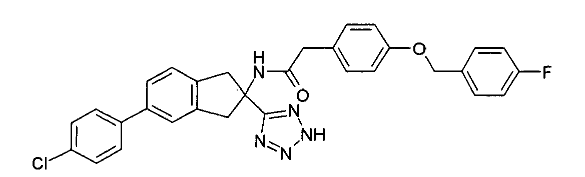

(1) (3-Dimethylaminopropyl)ethylcarbodiimide hydrochloride (192 mg, 1.00 mmol), 1-hydroxybenzotriazole (135 mg, 1.00 mmol) and triethylamine (300 µL, 2.1 mmol) were added with ice cooling to a DMF (2 mL)-dichloromethane (9 mL) solution of 5-(4-chlorophenyl)-2-[({4-[(4-fluorobenzyl)oxy]phenyl}acetyl)amino]indan-2-carboxylic acid (Example B134) (250 mg, 0.47 mmol), and stirred for 30 minutes at room temperature. 3-aminopropane nitrile ethyl (40 mg, 0.56 mmol) was added and stirred overnight at room temperature. Water was added to the reaction mixture, which was then extracted twice with ethyl acetate. The organic layers were combined, washed with water and saturated sodium chloride solution, and dried over magnesium sulfate. The drying agent was filtered out, and the filtrate was concentrated under reduced pressure. The residue was recrystallized form a mixed chloroform-hexane solvent to obtain 5-(4-chlorophenyl)-N-(2-cyanoethyl)-2-[({4-[(-fluorobenzyl)oxy]phenyl}acetyl)amino]indan-2-carboxamide as a white solid (229 mg, 84%)
   LC-MS 582 [M+H]⁺
   ¹H-NMR (300MHz, DMSO-d₆); δ 2.58-2.62 (t, J=6.3Hz, 2H), 3.12-3.21 (m, 2H), 3.26-3.28 (m, 4H), 3.50-3.57 (m, 2H), 5.02 (s, 2H), 6.85-6.88 (d, J=8.4Hz, 2H), 7.07-7.10 (d, J=8.4Hz, 2H), 7.17-7.23 (t, J=8.7Hz, 2H), 7.28-7.30 (d, J=7.8Hz, 1H), 7.44-7.50 (m, 6H), 7.65-7.68 (d, J=8.7Hz, 2H), 8.11-8.14 (t, J=5.7Hz, 1H), 8.55 (s, 1H)
(2) Diethyl azodicarboxylate (205 µL, 1.30 mmol) was added dropwise with ice cooling into a THF (10 mL) solution of 5-(4-chlorophenyl)-N-(2-cyanoethyl)-2-[({4-[(-fluorobenzyl)oxy]phenyl}acetyl)amino]indan-2-carboxamide (229 mg, 0.39 mmol), trimethylsilyl azide (179 µL, 1.30 mmol) and triphenylphosphine (341 mg, 1.30 mmol), and stirred for 1 day at room temperature. Trimethylsilyl azide (179 µL, 1.30 mmol), triphenylphosphine (341 mg, 1.30 mmol) and diethyl azodicarboxylate (205 µL, 1.30 mmol) were added and stirred for 1 day. A 5% aqueous diammonium cerium (IV) nitrate solution (40 mL) was added with ice cooling to the reaction solution, which was then stirred for 30 minutes and extracted twice with ethyl acetate. The organic layer was washed successively with water and saturated sodium chloride solution, and dried over magnesium sulfate. The drying agent was filtered out, and the filtrate was concentrated under reduced pressure. The residue was purified by silica gel column chromatography (eluate: ethyl acetate/hexane = 20/80-100/0) and then by preparative HPLC to obtain a white solid. This was dissolved in a mixed MeOH (1 mL)-THF (1 mL) solvent, 1 N NaOH aqueous solution (1 mL) was added, and the mixture was stirred for 2 days at room temperature and then refluxed for 1 day. 1 N hydrochloric acid aqueous solution (1.5 mL) was added, and the mixture was extracted twice with ethyl acetate. The organic layer was washed with saturated sodium chloride solution, and dried by addition of magnesium sulfate. The drying agent was filtered out, and the filtrate was concentrated under reduced pressure. The residue was purified by preparative HPLC and recrystallized from a mixed ethyl acetate-hexane solvent to obtain the title compound as a white solid (9.3 mg, 14%).
   LC-MS 554 [M+H]⁺
   ¹H-NMR (300MHz, DMSO-d₆); δ 3.51 (s, 2H), 3.54-3.70 (m, 4H), 5.01 (s, 2H), 6.84-6.87 (d, J=8.4Hz, 2H), 7.04-7.07 (d, J=8.7Hz, 2H), 7.17-7.23 (m, 3H), 7.33-7.36 (d, J=8.1Hz, 1H), 7.44-7.55 (m, 7H), 7.66-7.69 (d, J=8.7Hz, 2H)
   Melting point: 108-110°C

### Example B192

### 2-[({4-[(3-Aminobenzyl)oxy]phenyl}acetyl)amino]-5-(4-chlorophenyl)indan-2-carboxylic acid

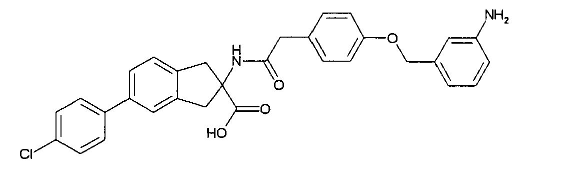

(1) Phosphorus tribromide (2.13 mL, 22.5 mmol) was added dropwise to a dichloromethane (40 mL) solution of 3-nitrobenzyl alcohol (2.3 g, 15 mmol), and stirred for 2 hours at room temperature. The reaction solution was poured into ice water, and stirred for 30 minutes. The organic layer was isolated, washed successively with water and saturated sodium chloride solution, and dried by addition of magnesium sulfate. The drying agent was filtered out, and the filtrate was concentrated under reduced pressure and diluted with dimethylformamide (20 mL). Methyl 4-hydroxyphenylacetate (2.49 g, 15 mmol), sodium iodide (100 mg) and potassium acetate (4.15 g, 30 mmol) were added and stirred for 2 hours at room temperature. Water was added to the reaction solution, which was then extracted twice with ethyl acetate. The organic layer washed twice with water and once with saturated sodium chloride solution, and dried by addition of magnesium sulfate. The drying agent was filtered out, and the filtrate was concentrated under reduced pressure. The residue was purified by silica gel column chromatography (eluate: ethyl acetate/hexane = 10/90-1/2) and then recrystallized from ethanol to obtain methyl {4-[(3-nitrobenzyl)oxy]phenyl}acetate (1.87 g, 41%) as a white solid.
   ¹H-NMR (300MHz, CDCl₃), δ 3.60 (s, 2H), 3.69 (s, 3H), 5.15 (s, 2H), 6.92-6.95 (d, J=8.7Hz, 2H), 7.21-7.24 (d, J=8.7Hz, 2H), 7.54-7.59 (t, J=8.1Hz, 1H), 7.76-7.78 (d, J=7.5Hz, 1H), 8.18-8.20 (d, J=7.8Hz, 1H)
(2) 1 N-NaOH aqueous solution (8.2 mL) was added to an ethanol (20 mL)-tetrahydrofuran (10 mL) solution of methyl {4-[(3-nitrobenzyl)oxy]phenyl}acetate (1.89 g, 6.27 mmol), and stirred for 30 minutes at 60°C. The reaction solution was concentrated under reduced pressure, and washed with diethyl ether. 1 N HCl aqueous solution (9 mL) was added, and the precipitated white solid was filtered out and washed with water and diethyl ether to obtain {4-[(3-nitrobenzyl)oxy]phenyl}acetic acid (1.66 g, 88%) as a white solid
   ¹H-NMR (300MHz, DMSO-d₆); δ 3.47 (s, 2H),5.25 (s, 2H), 6.96-6.99 (d, J=8.7Hz, 2H), 7.17-7.20 (d, J=8.7Hz, 2H), 7.67-7.73 (t, J=7.8Hz, 1H), 7.90-7.92 (d, J=7.8Hz, 1H), 8.18-8.21 (d, J=7.8Hz, 1H), 8.31 (s, 1H)
(3) Ethyl 5-(4-chlorophenyl)-2-[({4-[(3-nitrobenzyl)oxy]phenyl}acetyl)amino]indan-2-carboxylate was obtained as a colorless oil (291 mg, 99%) as in Example B183(3) from {4-[(3-nitrobenzyl)oxy]phenyl}acetic acid (187 mg, 0.65 mmol) and ethyl 2-amino-5-(4-chlorophenyl)indan-2-carboxylate (0.50 mmol).
   ¹H-NMR (300MHz, CDCl₃); δ 1.19-1.26 (m, 3H), 3.22-3.29 (m, 2H), 3.49 (s, 2H), 3.61-3.69 (m, 2H), 4.16-4.23 (m, 2H), 5.12 (s, 2H), 6.00 (s, 1H), 6.91-6.93 (d, J=8.4Hz, 2H), 7.17-7.26 (m, 4H), 7.35-7.40 (m, 4H), 7.46-7.49 (m, 2H), 7.54-7.59 (t, J=7.8Hz, 1H), 7.73-7.76 (d, J=8.1Hz, 1H), 8.18-8.21 (d, J=8.1 Hz, 1H), 8.31 (s, 1H)
(4) A mixture of ethyl 5-(4-chlorophenyl)-2-[({4-[(3-nitrobenzyl)oxy]phenyl}acetyl)amino]indan-2-carboxylate (291 mg, 0.50 mmol), iron powder (139 mg, 2.5 mmol), calcium chloride (28 mg, 0.25 mmol), ethanol (24 mL) and water (6 mL) was refluxed for 2 hours. The reaction solution was diluted with tetrahydrofuran, and Celite filtered. The filtrate was concentrated under reduced pressure, dissolved in ethyl acetate, washed with water and saturated sodium chloride solution, and dried by addition of magnesium sulfate. The drying agent was filtered out, and the filtrate was concentrated under reduced pressure. The residue was recrystallized from an ethyl acetate-hexane mixed solvent to obtain ethyl 2-[({4-[(3-aminobenzyl)oxy]phenyl}acetyl)amino]-5-(4-chlorophenyl)indan-2-carboxylate (235 mg, 85%) as a white solid.
   LC-MS 555 [M+H]⁺
   ¹H-NMR (300MHz, DMSO-d₆); δ 1.01-1.06 (t, J=7.2Hz, 3H), 3.16-3.24 (m, 2H), 3.32 (s, 2H), 3.50-3.58 (m, 2H), 3.97-4.04 (q, J=7.2Hz, 2H), 4.90 (s, 2H), 5.09 (s, 2H), 6.47-6.54 (m, 2H), 6.61 (s, 1H), 6.86-6.89 (d, J=8.4Hz, 2H), 6.97-7.02 (t, J=7.8Hz, 1H), 7.11-7.14 (d, J=8.7Hz, 2H), 7.30-7.33 (d, J=8.1Hz, 1H), 7.46-7.52 (m, 4H), 7.65-7.68 (d, J=8.4Hz, 2H), 8.79 (s, 1H)
(5) The title compound (91 mg, 71%) was obtained as a white solid as in Example B183(4) from ethyl 2-[({4-[(3-aminobenzyl)oxy]phenyl}acetyl)amino]-5-(4-chlorophenyl)indan-2-carboxylate (135 mg, 0.25 mmol).
   LC-MS 527 [M+H]⁺
   ¹H-NMR (300MHz, DMSO-d₆); δ 3.16-3.25 (m, 2H), 3.38 (s, 2H), 3.49-3.58 (m, 2H), 4.89 (s, 2H), 6.48-6.54 (m, 2H), 6.61 (s, 1H), 6.85-6.88 (d, J=8.4Hz, 2H), 6.97-7.02 (t, J=7.8Hz, 1H),7.10-7.13 (d, J=8.4Hz, 2H), 7.29-7.31 (d, J=8.1Hz, 1H), 7.45-7.51 (m, 4H), 7.65-7.68 (d, J=8.7Hz, 2H), 8.65 (s, 1H)
   Melting point: 191-192°C
   Elemental analysis: C₃₁H₂₇N₂O₄Cl (containing 0.2 mol H₂O) C 70.17, H 5.20, N 5.28; Found: C 70.16, H 5. 11, N 5.24

### Example B193

### 5-(4-Chlorophenyl)-2-({[4-({3-[(methylsulfonyl)amino]benzyl}oxy)phenyl]acetyl}amino)indan-2-carboxylic acid

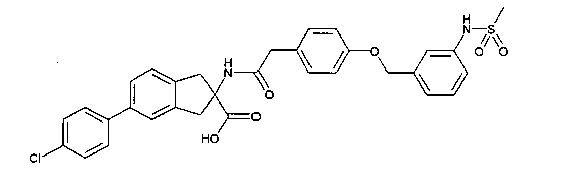

(1) Triethylamine (30 µL, 0.22 mmol) and methanesulfonyl chloride (17 µL, 0.22 mmol) were added to a THF (2 mL) solution of ethyl 2-[({4-[(3-aminobenzyl)oxy]phenyl)acetyl)amino]-5-(4-chlorophenyl)indan-2-carboxylate (Example B192(4)) (100 mg, 0.18 mmol), and stirred for 2 hours at room temperature. Triethylamine (30 µL, 0.22 mmol) and methanesulfonyl chloride (17 µL, 0.22 mmol) were further added and stirred for 2 hours. Water was added to the reaction solution, and stirred for 15 minutes. This was extracted twice with ethyl acetate, washed with water and saturated sodium chloride solution, and dried by addition of magnesium sulfate. The drying agent was filtered out, and the filtrate was concentrated under reduced pressure. The residue was purified by silica gel column chromatography (eluate: ethyl acetate/hexane = 1/2-10/3) to obtain ethyl 5-(4-chlorophenyl)-2-({[4-({3-[(methylsulfonyl)amino]benzyl}oxy)phenyl]acetyl}amino)indan-2-carboxylate (98 mg, 86%) as a colorless oil.
   LC-MS 633 [M+H]⁺
   ¹H-NMR (300MHz, DMSO-d₆); δ 1.15-1.20 (t, J=7.2Hz, 3H), 2.97 (s, 3H), 3.16-3.25 (m, 2H), 3.33 (s, 2H), 3.50-3.58 (m, 2H), 3.99-4.07 (q, J=7.2Hz, 2H), 5.05 (s, 2H), 6.89-6.92 (d, J=8.7Hz, 2H), 7.12-7.17 (m, 4H), 7.27-7.36 (m, 3H), 7.46-7.52 (m, 4H), 7.65-7.68 (d, J=8.7Hz, 2H), 8.79 (s, 1H), 9.77 (s, 1H)
(2) The title compound (63 mg, 67%) was obtained as a white solid as in Example B183(4) from ethyl 5-(4-chlorophenyl)-2-({[4-({3-[(methylsulfonyl)amino]benzyl}oxy)phenyl]acetyl}amino)indan-2-carboxylate (98 mg, 0.15 mmol).
   LC-MS 605 [M+H]⁺
   ¹H-NMR (300MHz, DMSO-d₆); 2.97 (s, 3H), 3.16-3.25 (m, 2H), 3.41 (s, 2H), 3.49-3.57 (m, 2H), 5.04 (s, 2H), 6.88-6.91 (d, J=8.7Hz, 2H), 7.11-7.17 (m, 4H), 7.27-7.36 (m, 3H), 7.45-7.51 (m, 4H), 7.65-7.68 (d, J=8.4Hz, 2H), 8.65 (s, 1H), 9.78 (s, 1H), 12.40 (br s, 1H)
   Melting point: 169-170°C
   Elemental analysis: Calcd. for C₃₂H₂₉N₂O₆SCl: C 63.52, H 4.83, N 4.63; Found: C 63.40, H 5.00, N 4.76

### Example B194

### 2-[({4-[(2-Aminobenzyl)oxy]phenyl}acetyl)amino]-5-(4-chlorophenyl)indan-2-carboxylic acid

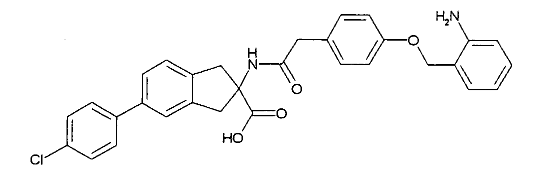

(1) Methyl 4-hydroxyphenylacetate (1.66 g, 10 mmol), sodium iodide (20 mg) and potassium carbonate (2.76 g, 20 mmol) were added to a DMF solution (10 mL) of 2-nitrobenzyl bromide (2.6 g, 12 mmol), and stirred overnight at room temperature. Water was added to the reaction solution, which was then extracted twice with ethyl acetate. The organic layer washed twice with water and once with saturated sodium chloride solution, and dried by addition of magnesium sulfate. The drying agent was filtered out, and the filtrate was concentrated under reduced pressure. The residue was purified by silica gel column chromatography (eluate: ethyl acetate/hexane = 10/90-1/1) to obtain methyl {4-[(2-nitrobenzyl)oxy]phenyl}acetate (2.97 g, 98%) as a colorless oil.
   ¹H-NMR (300MHz, CDCl₃); δ 3.58 (s, 2H), 3.69 (s, 3H), 5.48 (s, 2H), 6.93-6.96 (d, J=8.4Hz, 2H), 7.20-7.26 (d, J=8.1Hz, 2H), 7.46-7.51 (t, J=7.8Hz, 1H), 7.65-7.70 (t, J=7.8Hz, 1H), 7.87-7.90 (d, J=7.8Hz, 1H), 8.15-8.18 (d, J=8.1Hz, 1H)
(2) 4-[(2-Nitrobenzyl)oxy]phenylacetic acid (576 mg, 20%) was obtained as a brown solid as in Example B192(2) from methyl {4-[(2-nitrobenzyl)oxy]phenyl}acetate (2.97 g, 9.8 mmol). ¹H-NMR (300MHz, DMSO-d₆); 3.49 (s, 2H), 5.44 (s, 2H), 6.93-6.97 (m, 2H), 7.17-7.19 (d, J=8.7Hz, 2H), 7.59-7.66 (m, 1H), 7.75-7.78 (d, J=8.7Hz, 2H), 8.11-8.14 (d, J=8.1Hz, 1H), 12.40 (br s, 1H)
(3) Ethyl 5-(4-chlorophenyl)-2-[({4-[(2-nitrobenzyl)oxy]phenyl}acetyl)amino]indan-2-carboxylate (189 mg, 65%) was obtained as a white solid as in Example B183(3) from 4-[(2-nitrobenzyl)oxy]phenylacetic acid (187 mg, 0.65 mmol) and ethyl 2-amino-5-(4-chlorophenyl)indan-2-carboxylate (176 mg, 0.5 mmol).
   LC-MS 585 [M+H]⁺
   ¹H-NMR (300MHz, DMSO-d₆); δ 0.96-1.05(m, 3H), 3.16-3.25 (m, 2H), 3.34 (s, 2H), 3.50-3.58 (m, 2H), 5.43 (s, 2H), 6.90-6.93 (d, J=8.7Hz, 2H), 7.14-7.17 (d, J=8.7Hz, 2H), 7.30-7.33 (d, J=7.8Hz, 1H), 7.43-7.52 (m, 4H), 7.58-7.65 (m, 3H), 7.71-7.78 (m, 2H), 8.10-8.13 (d, J=8.1Hz, 1H), 8.81 (s, 1H)
(4) Ethyl 2-[({4-[(2-aminobenzyl)oxy]phenyl}acetyl)amino]-5-(4-chlorophenyl)indan-2-carboxylate (181 mg, 100%) was obtained as a white solid as in Example B192(4) from ethyl 5-(4-chlorophenyl)-2-[({4-[(2-nitrobenzyl)oxy]phenyl}acetyl)amino]indan-2-carboxylate (189 mg, 0.32 mmol).
   LC-MS 555 [M+H]⁺
   ¹H-NMR (300MHz, DMSO-d₆); δ 1.02-1.07 (t, J=7.2Hz, 3H), 3.16-3.25 (m, 2H), 3.32 (s, 2H), 3.50-3.58 (m, 2H), 3.97-4.04 (q, J=7.2Hz, 2H), 4.93 (s, 2H), 5.01 (s, 2H), 6.51-6.56 (t, J=7.2Hz, 1H), 6.66-6.69 (d, J=7.8Hz, 1H), 6.91-6.94 (d, J=8.4Hz, 2H), 6.99-7.04 (t, J=7.8Hz, 1H), 7.12-7.16 (m, 3H), 7.30-7.33 (d, J=7.8Hz, 1H), 7.46-7.52 (m, 4H), 7.65-7.68 (d, J=7.8Hz, 2H), 8.78 (s, 1H)
(5) The title compound (59 mg, 69%) was obtained as a white solid as in Example B183(4) from ethyl 2-[({4-[(2-aminobenzyl)oxy]phenyl}acetyl)amino]-5-(4-chlorophenyl)indan-2-carboxylate (90 mg, 0.16 mmol).
   LC-MS 527 [M+H]⁺
   ¹H-NMR (300MHz, DMSO-d₆); δ 3.16-3.25 (m, 2H), 3.38 (s, 2H), 3.49-3.57 (m, 2H), 4.92 (s, 2H), 5.01 (br s, 1H), 6.52-6.56 (t, J=7.2Hz, 1H), 6.66-6.68 (d, J=7.2Hz, 1H), 6.90-6.93 (d, J=8.7Hz, 2H), 6.99-7.04 (t, J=7.8Hz, 1H), 7.11-7.16 (m, 3H), 7.29-7.31 (d, J=7.8Hz,1H), 7.45-7.51 (m, 4H), 7.65-7.68 (d, J=8.7Hz, 2H), 8.65 (s, 1H), 12.40 (br s, 1H)
   Melting point: 149-150°C
   Elemental analysis: Calcd. for C₃₁H₂₇N₂O₄Cl (containing 0.2 mol H₂O): C 70.17, H 5.20, N 5.28; Found: C 70.24, H 5.34, N 5.38

### Example B195

### 5-(4-Chlorophenyl)-2-({[4-({2-[(methylsulfonyl)amino]benzyl} oxy)phenyl]acetyl}amino)indan-2-carboxylic acid

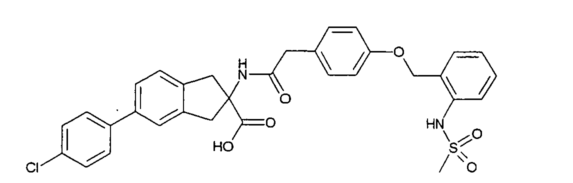

(1) Ethyl 5-(4-chlorophenyl)-2-({[4-({2-[(methylsulfonyl)amino]benzyl}oxy)phenyl]acetyl}amino)indan-carboxylate (35 mg, 34%) was obtained as a colorless oil as in Example B193(1) from ethyl 2-[({4-[(2-aminobenzyl)oxy]phenyl}acetyl)amino]-5-(4-chlorophenyl)indan-2-carboxylate (Example B194(4)) (91.2 mg, 0.16 mmol).
   LC-MS 633 [M+H]⁺
   ¹H-NMR (300MHz, CDCl₃); δ 1.18-1.23 (t, J=7.2Hz, 3H), 3.02 (s, 3H), 3.22-3.30 (m, 2H), 3.44-3.51 (m, 4H), 3.61-3.69 (m, 2H), 4.15-4.23 (q, J=7.2Hz, 2H), 5.11 (s, 2H), 6.01 (s, 1H), 6.91-6.94 (d, J=8.7Hz, 2H), 7.16-7.26 (m, 4H), 7.34-7.42 (m, 6H), 7.46-7.49 (d, J=8.7Hz, 2H), 7.60-7.61 (d, J=3.0Hz, 1H)
(2) The title compound (14 mg, 42%) was obtained as a white solid as in Example B183(4) from ethyl 5-(4-chlorophenyl)-2-({[4-({2-[(methyl sulfonyl)amino]benzyl}oxy)phenyl]acetyl}amino)indan-carboxylate (35 mg, 0.055 mmol).
   LC-MS 605 [M+H]⁺
   ¹H-NMR (300MHz, DMSO-d₆); δ 3.00 (s, 3H), 3.16-3.25 (m, 2H), 3.35 (s, 2H), 3.49-3.57 (m, 2H), 5.18 (s, 2H), 6.87-6.90 (d, J=8.7Hz, 2H), 7.12-7.15 (d, J=8.7Hz, 2H), 7.25-7.51 (m, 9H), 7.65-7.67 (d, J=8.7Hz, 2H), 8.65 (s, 1H), 9.24 (s, 1H), 12.40 (br s, 1H)

### Example 196

### 5-(4-Chlorophenyl)-2-({[6-(4-chlorophenyl)imidazo[1,2-a]pyridine-2-yl]carbonyl}amino)indan-2-carboxylic acid

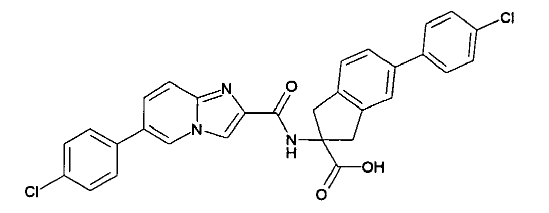

(1) Ethyl 5-(4-chlorophenyl)-2-({[6-(4-chlorophenyl)imidazo[1,2-a]pyridine-2-yl)carbonyl}amino)indan-2-carboxylate (186 mg, 65%) was obtained as a white solid as in Example B183(3) from 6-(4-chlorophenyl)imidazo[1,2-a]pyridine-2-carboxylic acid (164 mg, 0.6 mmol) and ethyl 2-amino-5-(4-chlorophenyl)indan-2-carboxylate (158 mg, 0.5 mmol).
   LC-MS 570 [M+H]⁺
   ¹H-NMR (300MHz, DMSO-d₆); δ 1.12-1.16 (t, J=7.2Hz, 3H), 3.46-3.68 (m, 4H), 4.08-4.15 (q, J=7.2Hz, 2H), 7.32-7.35 (d, J=7.8Hz, 1H), 7.47-7.76 (m, 12H), 8.38 (s, 1H), 8.83 (s, 1H), 8.98 (s, 1H)
(2) The title compound (137 mg, 78%) was obtained as a white solid as in Example B183(4) from ethyl 5-(4-chlorophenyl)-2-({[6-(4-chlorophenyl)imidazo[1,2-a]pyridine-2-yl]carbonyl}amino)indan-2-carboxylate (186 mg, 0.33 mmol).
   LC-MS 542 [M+H]⁺
   ¹H-NMR (300MHz, DMSO-d₆); δ 3.51-3.66 (m, 4H), 7.31-7.34 (d, J=8.1Hz, 1H), 7.46-7.76 (m, 12H), 8.37 (s, 1H), 8.67 (s, 1H), 8.99 (s, 1H)
   Melting point: Decomposes at 298°C
   Elemental analysis: Calcd. for C₃₀H₂₁N₃O₃Cl₂: C 66.43, H 3.90, N 7.75; Found: C 66.21, H 3.94, N 7.66

### Example C1

### N-[5-(4-Methoxyphenyl)-1-benzothiophene-2-ylcarbonyl] glycine

5-(4-Methoxyphenyl)-1-benzothiophene-2-carboxylic acid (1.3 mmol), 1-hydroxybenzotriazole (1.9 mmol) and 1-ethyl-3-(3-dimethylaminopropyl)carbodiimide hydrochloride (1.9 mmol) were dissolved in N,N-dimethylformamide (6.5 mL), and shaken for 2 hours at room temperature. An N,N-dimethylformamide solution (0.55 mL) of 0.24 M glycine t-butyl ester hydrochloride and 0.48 M triethylamine was added to the reaction solution (0.5 mL), which was then shaken overnight at room temperature. This was extracted with dichloromethane after addition of water. The organic layer was passed through a PTFE tube (polytetrafluoroethylene film processed tube) to obtain a solution containing the target compound. The solvent was evaporated, and the residue was purified by preparative HPLC to obtain a t-butyl ester. Trifluoracetic acid (0.5 mL) was added, and left standing overnight. The solvent was evaporated, acetonitrile (0.5 mL) was added, and the solvent was evaporated. The residue was purified by preparative HPLC to obtain N-[5-(4-methoxyphenyl)-1-benzothiophene-2-ylcarbonyl]glycine (27.6 mg) (Table 11).
NMR Data (Table 15)

### Examples C2-C94

The compounds of Example C2 through Example C94 were obtained by methods similar to those of Example C1 by reacting various carboxylic acids and amines. Examples C11 and C9, C23 and C21, C35 and C33, C47 and C45, C59 and C57 and C93 and C91 were obtained by isolating the benzyl and de-benzylized forms by preparative HPLC. The compounds of Examples C49-C60 and C72-C82 were obtained as trifluoracetic acid salts (Table 11 through Table 14). ¹H-NMR data for typical compounds are given in Table 15.

**(Table 11)**

| | | | | |
|---|---|---|---|---|
| 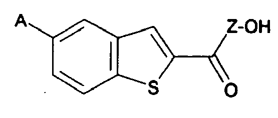 | | | | |

| Example | A | Z-OH | LC/MS | |
|---|---|---|---|---|
| | | | HPLC purity | m/e (M⁺+ 1) |
| C1 1 | 4-methoxyphenyl | Gly-OH | 92% | 342 |
| C2 | 4-methoxyphenyl | L-Ala-OH | 91% | 356 |
| C3 | 4-methoxyphenyl | L-Leu-OH | 91% | 398 |
| C4 | 4-methoxyphenyl | L-Ile-OH | 92% | 398 |
| C5 | 4-methoxyphenyl | L-Val-OH | 93% | 384 |
| C6 | 4-methoxyphenyl | L-Phe-OH | 91% | 432 |
| C7 | 4-methoxyphenyl | L-Ser-OH | 88% | 372 |
| C8 | 4-methoxyphenyl | L-Met-OH | 99% | 416 |
| C9 | 4-methoxyphenyl | L-Glu-OH | 99% | 414 |
| C10 | 4-methoxyphenyl | L-Asp(OMe)-OH | 81% | 414 |
| C11 | 4-methoxyphenyl | L-Glu(OBzl)-OH | 96% | 504 |
| C12 | 4-methoxyphenyl | L-Asp-OH | 93% | 400 |
| C13 | 4-trifluoromethyl phenyl | Gly-OH | 96% | 380 |
| C14 | 4-trifluoromethyl phenyl | L-Ala-OH | 97% | 394 |
| C15 | 4-trifluoromethyl phenyl | L-Leu-OH | 97% | 436 |
| C16 | 4-trifluoromethyl phenyl | L-Ile-OH | 94% | 436 |
| C17 | 4-trifluoromethyl phenyl | L-Val-OH | 99% | 422 |

**(Table 12)**

| | | | | |
|---|---|---|---|---|
| C18 | 4-trifluoromethyl phenyl | L-Phe-OH | 99% | 470 |
| C19 | 4-trifluoromethyl phenyl | L-Ser-OH | 88% | 410 |
| C20 | 4-trifluoromethyl phenyl | L-Met-OH | 97% | 454 |
| C21 | 4-trifluoromethyl phenyl | L-Glu-OH | 98% | 452 |
| C22 | 4-trifluoromethyl phenyl | L-Asp(OMe)-OH | 94% | 452 |
| C23 | 4-trifluoromethyl phenyl | L-Glu(OBzl)-OH | 95% | 542 |
| C24 | 4-trifluoromethyl phenyl | L-Asp-OH | 99% | 438 |
| C25 | 4-fluorophenyl | Gly-OH | 99% | 330 |
| C26 | 4-fluorophenyl | L-Ala-OH | 98% | 344 |
| C27 | 4-fluorophenyl | L-Leu-OH | 97% | 386 |
| C28 | 4-fluorophenyl | L-Ile-OH | 98% | 386 |
| C29 | 4-fluorophenyl | L-Val-OH | 97% | 372 |
| C30 | 4-fluorophenyl | L-Phe-OH | 98% | 420 |
| C31 | 4-fluorophenyl | L-Ser-OH | 95% | 360 |
| C32 | 4-fluorophenyl | L-Met-OH | 98% | 404 |
| C33 | 4-fluorophenyl | L-Glu-OH | 99% | 402 |
| C34 | 4-fluorophenyl | L-Asp(OMe)-OH | 96% | 402 |
| C35 | 4-fluorophenyl | L-Glu(OBzl)-OH | 94% | 492 |
| C36 | 4-fluorophenyl | L-Asp-OH | 99% | 388 |
| C37 | 4-chlorophenyl | Gly-OH | 99% | 346 |
| C38 | 4-chlorophenyl | L-Ala-OH | 98% | 360 |
| C29 | 4-chlorophenyl | L-Leu-OH | 97% | 402 |

**(Table 13)**

| | | | | |
|---|---|---|---|---|
| C40 | 4-chlorophenyl | L-Ile-OH | 97% | 402 |
| C41 | 4-chlorophenyl | L-Val-OH | 98% | 388 |
| C42 | 4-chlorophenyl | L-Phe-OH | 96% | 436 |
| C43 | 4-chlorophenyl | L-Ser-OH | 87% | 376 |
| C44 | 4-chlorophenyl | L-Met-OH | 97% | 420 |
| C45 | 4-chlorophenyl | L-Glu-OH | 97% | 418 |
| C46 | 4-chlorophenyl | L-Asp(OMe)-OH | 97% | 418 |
| C47 | 4-chlorophenyl | L-Glu(OBzl)-OH | 97% | 508 |
| C48 | 4-chlorophenyl | L-Asp-OH | 99% | 404 |
| C49 | 4-dimethylaminophenyl | Gly-OH | 87% | 355 |
| C50 | 4-dimethylaminophenyl | L-Ala-OH | 98% | 369 |
| C51 | 4-dimethylaminophenyl | L-Leu-OH | 96% | 411 |
| C52 | 4-dimethylaminophenyl | L-Ile-OH | 97% | 411 |
| C53 | 4-dimethylaminophenyl | L-Val-OH | 94% | 397 |
| C54 | 4-dimethylaminophenyl | L-Phe-OH | 81% | 445 |
| C55 | 4-dimethylaminophenyl | L-Ser-OH | 84% | 385 |
| C56 | 4-dimethylaminophenyl | L-Met-OH | 90% | 429 |
| C57 | 4-dimethylaminophenyl | L-Glu-OH | 94% | 427 |
| C58 | 4-dimethylaminophenyl | L-Asp(OMe)-OH | 94% | 427 |
| C59 | 4-dimethylaminophenyl | L-Glu(OBzl)-OH | 95% | 517 |
| C60 | 4-dimethylaminophenyl | L-Asp-OH | 86% | 413 |
| C61 | 3-acetamidophenyl | Gly-OH | 88% | 369 |
| C62 | 3-acetamidophenyl | L-Ala-OH | 97% | 383 |
| C63 | 3-acetamidophenyl | L-Leu-OH | 97% | 425 |
| C64 | 3-acetamidophenyl | L-Ile-OH | 97% | 425 |
| C65 | 3-acetamidophenyl | L-Val-OH | 98% | 411 |
| C66 | 3-acetamidophenyl | L-Phe-OH | 96% | 459 |
| C67 | 3-acetamidophenyl | L-Ser-OH | 95% | 399 |

**(Table 14)**

| | | | | |
|---|---|---|---|---|
| C68 | 3-acetamidophenyl | L-Met-OH | 97% | 443 |
| C69 | 3-acetamidophenyl | L-Asp(OMe)-OH | 100% | 441 |
| C70 | 3-acetamidophenyl | L-Glu(OBzl)-OH | 83% | 531 |
| C71 | 3-acetamidophenyl | L-Asp-OH | 99% | 427 |
| C72 | 3-pyridyl | Gly-OH | 96% | 313 |
| C73 | 3-pyridyl | L-Ala-OH | 92% | 327 |
| C74 | 3-pyridyl | L-Leu-OH | 94% | 369 |
| C75 | 3-pyridyl | L-Ile-OH | 99% | 369 |
| C76 | 3-pyridyl | L-Val-OH | 99% | 355 |
| C77 | 3-pyridyl | L-Phe-OH | 98% | 403 |
| C78 | 3-pyridyl | L-Ser-OH | 98% | 343 |
| C79 | 3-pyridyl | L-Met-OH | 99% | 387 |
| C80 | 3-pyridyl | L-Asp(OMe)-OH | 89% | 385 |
| C81 | 3-pyridyl | L-Glu(OBzl)-OH | 90% | 475 |
| C82 | 3-pyridyl | L-Asp-OH | 98% | 371 |
| C83 | 3-thienyl | Gly-OH | 99% | 318 |
| C84 | 3-thienyl | L-Ala-OH | 100% | 332 |
| C85 | 3-thienyl | L-Leu-OH | 97% | 374 |
| C86 | 3-thienyl | L-Ile-OH | 98% | 374 |
| C87 | 3-thienyl | L-Val-OH | 98% | 360 |
| C88 | 3-thienyl | L-Phe-OH | 99% | 408 |
| C89 | 3-thienyl | L-Ser-OH | 99% | 348 |
| C90 | 3-thienyl | L-Met-OH | 96% | 392 |
| C91 | 3-thienyl | L-Glu-OH | 99% | 390 |
| C92 | 3-thienyl | L-Asp(OMe)-OH | 96% | 390 |
| C93 | 3-thienyl | L-Glu(OBzl)-OH | 94% | 480 |
| C94 | 3-thienyl | L-Asp-OH | 98% | 376 |

**(Table 15)**

| Compound | ¹H NMR (300 MHz, CD₃OD); δ ppm |
|---|---|
| Example C1 | 3.85 (3H, s), 4.12 (2H, s), 7.03 (2H, d, J=8.9 Hz), 7.63 (2H, d, J=8.9 Hz), 7.69 (1H, dd, J=1.7, 8.5 Hz), 7.95 (1H, d, J=8.5 Hz), 8.01 (1H, s), 8.07 (1H, d, J=1.7 Hz) |
| Example C15 | 0.99-1.03 (6H, m), 1.75-1.89 (3H, m), 4.66-4.70 (1H, m), 7.77-7.79 (3H, m), 7.90 (2H, d, J=8.3 Hz), 8.04 (1H, d, J=8.5 Hz), 8.13 (1H, s), 8.21 (1H, d, J=1.5 Hz) |
| Example C29 | 1.08 (6H, dd, J=2.6, 6.8 Hz), 2.24-2.38 (1H, m), 4.51 (1H, d, J=6.4 Hz), 7.16-7.26 (2H, m), 7.66-7.76 (3H, m), 7.98 (1H, d, J=8.5 Hz), 8.11 (1H, d, J=1.5 Hz), 8.15 (1H, s) |
| Example C43 | 3.97-4.07 (2H, m), 4.73 (1H, t, J=4.6 Hz), 7.47 (2H, d, J=8.5 Hz), 7.65-7.77 (3H, m), 8.00 (1H, d, J=8.7 Hz), 8.12 (1H, s), 8.14 (1H, d, J=1.5 Hz). |
| Example C68 | 2.12 (3H, s), 2.16 (3H, s), 2.15-2.36 (2H, m), 2.55-2.76 (2H, m), 4.79 (1H, dd, J=4.7, 9.4 Hz), 7.38-7.47 (2H, m), 7.48-7.56 (1H, m), 7.72 (1H, dd, J=1.7, 8.5 Hz), 7.93-7.96 (1H, m), 7.99 (1H, d, J=8.5 Hz), 8.10 (1H, s), 8.12 (1H, d, J=1.7 Hz) |

### Example C107

### N-{[6-(4-Chlorophenyl)-3 -methyl-1-benzofuran-2-yl] carbonyl glycine

1) A N,N-dimethylformamide solution (1.5 ml) of 6-(4-chlorophenyl)-3-methyl-1-benzofuran-2-carboxylic acid (30 mg), glycine ethyl ester hydrochloride (29 mg), N,N-diisopropyl ethylamine (27 mg) and benzotriazol-1-yloxytris(dimethylamino) phosphonium hexafluorophosphate (70 mg) was stirred for 18 hours at room temperature. Water was added to the reaction mixture, and the precipitated crystals were filtered out and washed with diethyl ether to obtain ethyl N-{[6-(4-chlorophenyl)-3-methyl-1-benzofuran-2-yl]carbonyl}glycinate (Example C107A) as colorless crystals (35.6 mg, yield 91%). Purity 98%. M+H: 372.
2) A mixture of ethyl N-{[6-(4-chlorophenyl)-3-methyl-1-benzofuran-2-yl]carbonyl}glycinate (29 mg), lithium hydroxide monohydrate (7 mg), tetrahydrofuran (1 ml) and water (0.5 ml) was stirred for 17 hours at room temperature. The reaction mixture was made acidic with 1 N hydrochloric acid. The precipitated crystals were filtered out to obtain the title compound as colorless crystals (22 mg, yield 82%). Purity 97%. M+H: 344.

### Examples C95-C106 and C108-C111

The ester compounds of Example C95A through Example C106 A and Example C108A through Example C111 A were obtained by methods similar to those of Example C107-1) (Table 16 through Table 20).

These ester compounds were hydrolyzed by methods similar to those of Example C107-2) to obtain the compounds of Example C95 through Example C106 and Example C108 through Example C 111 (Table 21 through Table 25).

**(Table 16)**

| Example | Structural Formula | Compound | Yield | HPLC Purity | m/e (M⁺+ 1) |
|---|---|---|---|---|---|
| C95A | 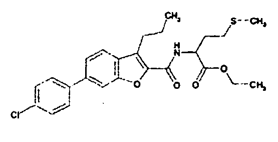 | Ethyl N-{[6-(4-chlorophenyl)-3-propyl-1-benzofuran-2-yl]carbonyl)methioninate | 77% | 96% | 474 |
| C96A | 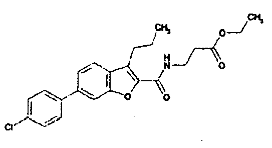 | Ethyl N-{[6-(4-chlorophenyl)-3-propyl-1-benzofuran-2-yl]carbonyl)-β-alaninate | 86% | 99% | 414 |
| C97A | 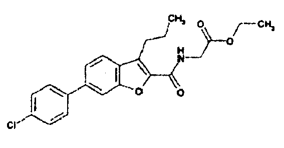 | Ethyl N-{[6-(4-chlorophenyl)-3-propyl-1-benzofuran-2-yl]carbonyl)glycinate | 86% | 97% | 400 |

**(Table 17)**

| | | | | | |
|---|---|---|---|---|---|
| C98A | 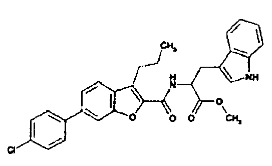 | Methyl N-{[6-(4-chlorophenyl)-3-propyl-1-benzofuran-2-yl]carbonyl)tryptophanate | 81% | 95% | 515 |
| C99A | 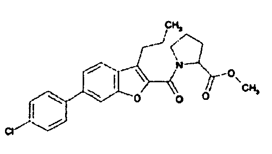 | Methyl 1-([6-(4-chlorophenyl)-3-propyl-1-benzofuran-2-yl]carbonyl)prolinate | 84% | 84% | 426 |
| C100A | 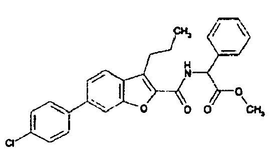 | Methyl ({[6-(4-chlorophenyl)-3-propyl-1-benzofuran-2-yl]carbonyl} amino)(phenyl)acetate | 98% | 96% | 462 |
| C101A | 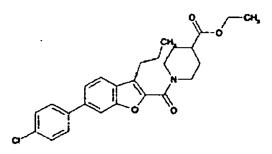 | Ethyl 1-{[6-(4-chlorophenyl)-3-propyl-1-benzofuran-2-yl]carbonyl) piperidine-4-carboxylate | 98% | 83% | 454 |

**(Table 18)**

| | | | | | |
|---|---|---|---|---|---|
| C102A | 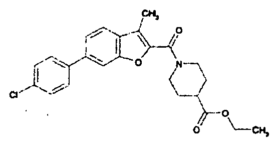 | Ethyl 1-{[6-(4-chlorophenyl)-3-methyl-1-benzofuran-2-yl]carbonyl) piperidine-4-carboxylate | 98% | 80% | 426 |
| C103A | 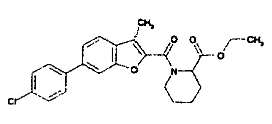 | Ethyl 1-{[6-(4-chlorophenyl)-3-methyl-1-benzofuran-2-yl]carbonyl) piperidine-2-carboxylate | 98% | 86% | 426 |
| C 104A | 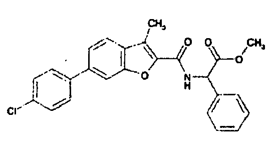 | Methyl ({[6-(4-chlorophenyl)-3-methyl-1-benzofuran-2-yl]carbonyl } amino)(phenyl)acetate | 88% | 98% | 434 |
| C105A | 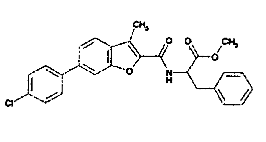 | Methyl N-{[6-(4-chlorophenyl)-3-methyl-1-benzofuran-2-yl]carbonyl) phenylalaninate | 98% | 98% | 448 |

**(Table 19)**

| | | | | | |
|---|---|---|---|---|---|
| C106A | 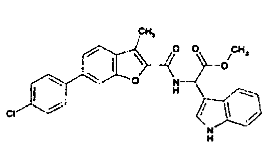 | Methyl ({[6-(4-chlorophenyl)-3-methyl-1-benzofuran-2-yl]carbonyl } amino)(1H-indole-3-yl)acetate | 98% | 97% | 473 |
| C107A | 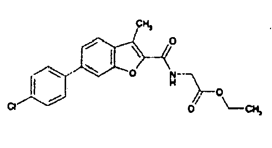 | Ethyl N-{[6-(4-chlorophenyl)-3-methyl-1-benzofuran-2-yl]carbonyl)glycinate | 91% | 98% | 372 |
| C108A | 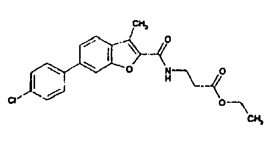 | Ethyl N-{[6-(4-chlorophenyl)-3-methyl-1-benzofuran-2-yl]carbonyl)-β-alaninate | 84% | 99% | 386 |
| C109A | 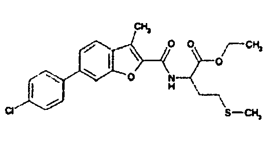 | Ethyl N-{[6-(4-chlorophenyl)-3-methyl-1-benzofuran-2-yl]carbonyl)methioninate | 89% | 97% | 446 |

**(Table 20)**

| | | | | | |
|---|---|---|---|---|---|
| C110A | 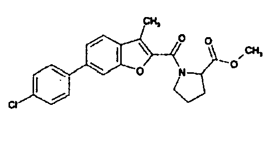 | Methyl 1-{[6-(4-chlorophenyl)-3-methyl-1-benzofuran-2-yl]carbonyl) prolinate | 80% | 92% | 398 |
| C111A | 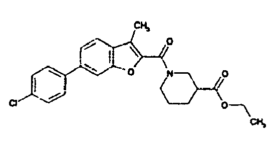 | Ethyl 1-{[6-(4-chlorophenyl)-3-methyl-1-benzofuran-2-yl]carbonyl) piperidine-3-carboxylate | 98% | 81% | 426 |

**(Table 21)**

| Example | Structural Formula | Compound | Yield | HPLC Purity | m/e (M⁺+1) |
|---|---|---|---|---|---|
| C95 | 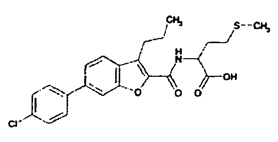 | N-{[6-(4-chlorophenyl)-3-propyl-1-benzofuran-2-yl]carbonyl)methionine | 46% | 95% | 446 |
| C96 | 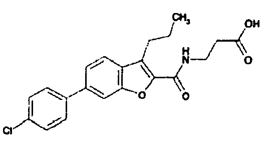 | N-{[6-(4-chlorophenyl)-3- propyl-1-benzofuran-2-yl]carbonyl)-β-alanine | 37% | 100% | 386 |
| C97 | 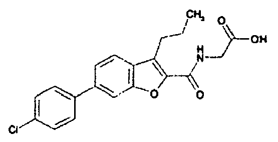 | N-{[6-(4-chlorophenyl)-3- propyl-1-benzofuran-2-yl]carbonyl)glycine | 57% | 98% | 372 |
| C98 | 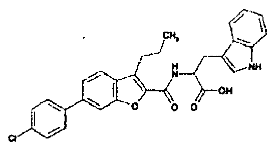 | N-{[6-(4-chlorophenyl)-3-propyl-1-benzofuran-2-yl]carbonyl)tryptophan | 64% | 98% | 501 |

**(Table 22)**

| | | | | | |
|---|---|---|---|---|---|
| C99 | 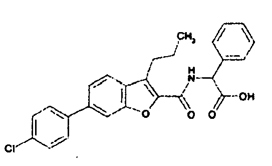 | ({[6-(4-chlorophenyl)-3-propyl-1-benzofuran-2-yl]carbonyl}amino) (phenyl)acetic acid | 98% | 98% | 448 |
| C100 | 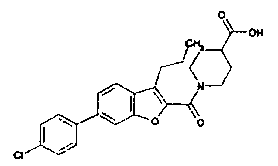 | 1-{[6-(4-chlorophenyl)-3-propyl-1-benzofuran-2-yl]carbonyl) piperidine-4-carboxylic acid | 76% | 94% | 426 |
| C101 | 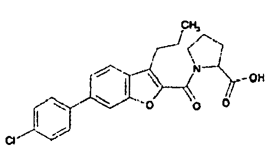 | 1-{[6-(4-chlorophenyl)-3-propyl-1-benzofuran-2-yl]carbonyl)proline | 51% | 85% | 412 |
| C102 | 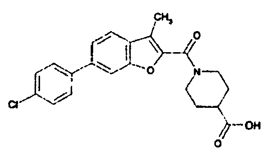 | 1-{[6-(4-chlorophenyl)-3-methyl-1-benzofuran-2-yl]carbonyl) piperidine-4-carboxylic acid | 80% | 86% | 398 |

**(Table 23)**

| | | | | | |
|---|---|---|---|---|---|
| C103 | 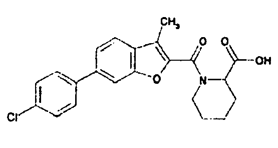 | 1-{[6-(4-chlorophenyl)-3-methyl-1-benzofuran-2-yl]carbonyl) piperidine-2-carboxylic acid | 65% | 94% | 398 |
| C104 | 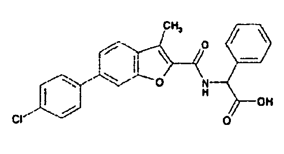 | ({[6-(4-chlorophenyl)-3-methyl-1-benzofuran-2-yl]carbonyl} amino) (phenyl)acetic acid | 68% | 98% | 420 |
| C105 | 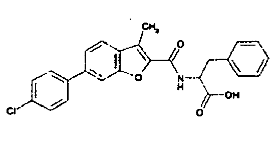 | N-{[6-(4-chlorophenyl)-3-methyl-1-benzofuran-2-yl]carbonyl)phenylalanine | 99% | 97% | 434 |
| C106 | 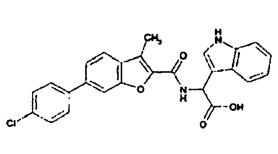 | ({[6-(4-chlorophenyl)-3-methyl-1-benzofuran-2-yl]carbonyl} amino)(1H-indole-3-yl)acetic acid | 99% | 98% | 459 |

**(Table 24)**

| | | | | | |
|---|---|---|---|---|---|
| C107 | 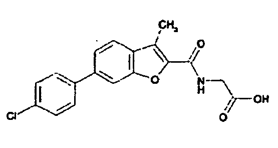 | N-{[6-(4-chlorophenyl)-3-methyl-1-benzofuran-2-yl]carbonyl)glycine | 82% | 97% | 344 |
| C108 | 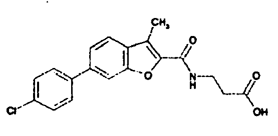 | N-{[6-(4-chlorophenyl)-3-methyl-1-benzofuran-2-yl]carbonyl)-β-alanine | 77% | 98% | 358 |
| C109 | 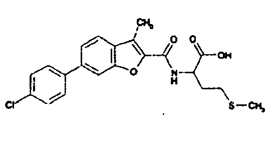 | N-{[6-(4-chlorophenyl)-3-methyl-1-benzofuran-2-yl]carbonyl)methionine | 99% | 89% | 418 |

**(Table 25)**

| | | | | | |
|---|---|---|---|---|---|
| C110 | 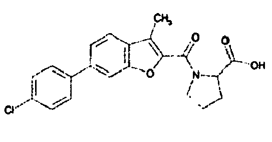 | 1-{[6-(4-chlorophenyl)-3-methyl-1-benzofuran-2-yl]carbonyl)proline | 99% | 96% | 384 |
| C111 | 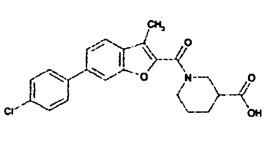 | 1-{[6-(4-chlorophenyl)-3-methyl-1-benzofuran-2-yl]carbonyl) piperidine-3-carboxylic acid | 64% | 80% | 398 |

### Example C112

### N-{[6-(4-Chlorophenyl)-3-methyl-1-benzofuran-2-yl]carbonyl}-L-glutamic acid γ-benzyl ester

1) WSCD hydrochloride (300 mg, 1.5 mmol), HOBt (81 mg, 0.60 mmol) and triethylamine (168 µL, 1.2 mmol) were added with ice cooling to a DMF (1 mL)-dichloromethane (9 mL) solution of 6-(4-chlorophenyl)-3-methyl-1-benzofuran-2-carboxylic acid (286 mg, 1 mmol), and stirred for 30 minutes at room temperature. L-glutamic acid γ-benzyl ester α-tert-butyl ester hydrochloride (329 mg, 1 mmol) was added and stirred overnight at room temperature. Water was added to the reaction mixture, which was then extracted twice with ethyl acetate. The organic layers were combined, washed with water and saturated sodium chloride solution, and dried over magnesium sulfate. The drying agent was filtered out, and the filtrate was concentrated under reduced pressure. The residue was purified by silica gel chromatography (eluate: ethyl acetate/hexane = 1/10-1/3) to obtain N-{[6-(4-chlorophenyl)-3-methyl-1-benzofuran-2-yl]carbonyl}-L-glutamic acid γ-benzyl ester α-tert-butyl ester (476 mg, 85%) as a white powder.
   ¹H-NMR (300MHz, CDC1₃) δ: 1.51 (9H, s), 2.05-2.20 (1H, m), 2.30-2.60 (3H, m), 2.62 (3H, s), 4.76 (1H, m), 5.09 (2H, s), 7.21 (1H, d, J=7.4Hz), 7.25-7.33 (5H, m), 7.40-7.70 (7H, m)
2) Trifluoracetic acid (5 ml) was added with ice cooling to a methanol (5 ml) solution of N-{[6-(4-chlorophenyl)-3-methyl-1-benzofuran-2-yl]carbonyl}-L-glutamic acid γ-benzyl ester α-tert-butyl ester (476 mg, 0.85 mmol), and stirred for 2 hours at room temperature. The reaction solution was concentrated, dissolved in ethyl acetate, washed with water and saturated sodium chloride solution, dried over magnesium sulfate and concentrated under reduced pressure. This was pulverized from hexane to obtain the title compound (186 mg, 43%) as a white powder. ¹H-NMR (300MHz, CDCl₃) δ: 2.15-2.80 (4H, m), 2.63 (3H, s), 4.75-4.90 (1H, m), 5.13 (2H, s), 7.26-7.70 (13H, m)
   LC-MS 506 [M+H]⁺

### Example C113

### 4-Bromo-2-({[6-(4-chlorophenyl)-3-methyl-1-benzofuran-2-yl]carbonyl}amino)indan-2-carboxylic acid

1) WSCD hydrochloride (115 mg, 0.60 mmol), HOBt (81 mg, 0.60 mmol) and triethylamine (168 µL, 1.2 mmol) were added with ice cooling to a DMF (1mL)-dichloromethane (9 mL) solution of 6-(4-chlorophenyl)-3-methyl-1-benzofuran-2-carboxylic acid (146 mg, 0.51 mmol), and stirred for 30 minutes at room temperature. Ethyl 2-amino-4-bromoindan-2-carboxylate (85 mg, 0.3 mmol) was added and stirred overnight at room temperature. Water was added to the reaction mixture, which was then extracted twice with ethyl acetate. The organic layers were combined, washed with water and saturated sodium chloride solution, and dried over magnesium sulfate. The drying agent was filtered out, and the filtrate was concentrated under reduced pressure. The residue was purified by silica gel chromatography (eluate: ethyl acetate/hexane = 1/10-1/1) to obtain ethyl 4-bromo-2-({[6-(4-chlorophenyl)-3-methyl-1-benzofuran-2-yl]carbonyl}amino)indan-2-carboxylate (158 mg, 96%) as a white solid.
   ¹H-NMR (300MHz, DMSO-d₆) δ: 9.27 (1H, s), 7.84-7.82 (2H, d), 7.77-7.75 (2H, d), 7.68-7.65 (1H, dd), 7.56-7.53 (2H, d), 7.43-7.40 (1H, d), 7.28-7.26 (1H, d), 7.18-7.13 (1H, t), 4.17-4.10 (2H, q), 3.67-3.65 (2H, d), 3.59-3.45 (2H, t), 2.54 (3H, s), 1.20-1.13 (3H, t)
   LC-MS 553 [M+H]⁺
2) 1N-NaOH aqueous solution (484 µL) was added with ice cooling to a methanol (1 mL)-THF (1 mL) solution of ethyl 4-bromo-2-({[6-(4-chlorophenyl)-3-methyl-1-benzofuran-2-yl]carbonyl}amino)indan-2-carboxylate (133 mg, 0.24 mmol), and stirred overnight at room temperature. 1N-hydrochloric acid (450 µL) was added, and the mixture was extracted twice with ethyl acetate. The organic layers were combined, washed with saturated sodium chloride solution, dried by addition of magnesium sulfate, and concentrated under reduced pressure. The residue was recrystallized from a mixed ethyl acetate-hexane solvent to obtain the title compound (95 mg, 75%) as white crystals.
   ¹H-NMR (300MHz, DMSO-d₆) δ: 12.7 (1H, s), 9.09 (1H, s), 7.85-7.75 (4H, m), 7.68-7.65 (1H, d), 7.56-7.53 (2H, d), 7.41-7.39 (1H, d), 7.27-7.25 (1H, d), 7.17-7.12 (1H, t), 3.63-3.44 (4H, m), 2.55 (3H, s)
   LC-MS 525 [M+H]⁺
   Melting point: 230°C

### Example C114

### 5-Bromo-2-({[6-(4-chlorophenyl)-3-methyl-1-benzofuran-2-yl] carbonyl } amino)indan-2-carboxylic acid

1) Ethyl 5-bromo-2-({[6-(4-chlorophenyl)-3-methyl-1-benzofuran-2-yl]carbonyl}amino)indan-2-carboxylate (112 mg, 68%) was obtained as a white solid by methods similar to those of Example C113-1) from 6-(4-chlorophenyl)-3-methyl-1-benzofuran-2-carboxylic acid (146 mg, 0.51 mmol) and ethyl 2-amino-5-bromoindan-2-carboxylate (85 mg, 0.3 mmol).
   ¹H-NMR (300MHz, DMSO-d₆) δ: 9.27 (1H, s), 7.84-7.82 (2H, d), 7.77-7.75 (2H, d), 7.68-7.65 (1H, dd), 7.56-7.53 (2H, d), 7.43-7.40 (1H, d), 7.28-7.26 (1H, d), 7.18-7.13 (1H , t), 4.17-4.10 (2H, q), 3.67-3.65 (2H, d), 3.59-3.45 (2H, t), 2.54 (3H, s), 1.20-1.13 (3H, t)
   LC-MS 553 [M+H]⁺
2) The title compound (84 mg, 85%) was obtained as white crystals by methods similar to those of Example C113-2) from ethyl 5-bromo-2-({[6-(4-chlorophenyl)-3-methyl-1-benzofuran-2-yl]carbonyl}amino)indan-2-carboxylate (105 mg, 0.19 mmol).
   ¹H-NMR (300MHz, DMSO-d₆) δ : 12.66 (1H, s), 7.84-7.75 (4H, m), 7.67-7.64 (2H, d), 7.55-7.53 (2H, d), 7.44 (1H, s), 7.37-7.34 (1H, d), 7.22-7.19 (1H, d), 3.62-3.41 (4H, m), 2.55 (3H,s)
   LC-MS 525 [M+H]⁺
   Melting point: 227°C

### Example C115

### O-Benzyl-N-{[6-(4-chlorophenyl)-3-methyl-1-benzofuran-2-yl]carbonyl}-L-tyrosine

1) WSCD hydrochloride (115 mg, 0.60 mmol), HOBt (81 mg, 0.60 mmol) and triethylamine (168 µL, 1.2 mmol) were added with ice cooling to a DMF (2 mL)-dichloromethane (2 mL) solution of 6-(4-chlorophenyl)-3-methyl-1-benzofuran-2-carboxylic acid (143 mg, 0.50 mmol), and stirred for 30 minutes at room temperature. Methyl O-benzyl-L-tyrosinate hydrochloride (129 mg, 0.40 mmol) was added and stirred overnight at room temperature. Water was added to the reaction mixture, which was then extracted twice with ethyl acetate. The organic layers were combined, washed with water and saturated sodium chloride solution, and dried over magnesium sulfate. The drying agent was filtered out, and the filtrate was concentrated under reduced pressure. The residue was purified by silica gel chromatography (eluate: ethyl acetate/hexane = 1/10-1/1) to obtain methyl O-benzyl-N-{[6-(4-chlorophenyl)-3-methyl-1-benzofuran-2-yl]carbonyl}-L-tyrosinate (150 mg, 93%) as a colorless oil.
   ¹H-NMR (300MHz, CDCl₃) δ: 7.67-7.30 (12H, m), 7.11-7.04 (3H, m), 6.94-6.90 (2H, d), 5.08-5.03 (3H, m), 3.76 (3H, s), 3.22-3.20 (2H, d), 2.64 (3H, s)
   LC-MS 554 [M+H]⁺
2) 1N-NaOH aqueous solution (979 µL) was added with ice cooling to a methanol (1 mL)-THF (2 mL) solution of O-benzyl-N-{[6-(4-chlorophenyl)-3-methyl-1-benzofuran-2-yl]carbonyl}-L-tyrosinate (271 mg, 0.49 mmol), and stirred overnight at room temperature. 1N-hydrochloric acid (1 mL) was added, and the mixture was extracted twice with ethyl acetate. The organic layers were combined, washed with saturated sodium chloride solution, dried by addition of magnesium sulfate, and concentrated under reduced pressure. The residue was recrystallized from a mixed ethyl acetate-hexane solvent to obtain the title compound (75 mg, 28%) as white crystals.
   ¹H-NMR (300MHz, DMSO-d₆) δ : 12.40 (1H, br s), 7.93 (1H, s), 7.83-7.76 (4H, m), 7.66-7.63 (1H, d), 7.53-7.50 (2H, d), 7.41-7.28 (5H, m), 7.09-7.06 (2H, d), 6.82-6.79 (2H, d), 4.98 (2H, s), 4.23-4.21 (1H, d), 3.17-3.14 (2H, t), 2.55 (3H, s)
   LC-MS 540 [M+H]⁺
   Melting point: 143-144°C

### Example C116

### O-Benzyl-N- {[6-(4-chlorophenyl)-3-methyl-1-benzofuran-2-yl]carbonyl }-D-tyrosine

1) Methyl O-benzyl-N-{[6-(4-chlorophenyl)-3-methyl-1-benzofuran-2-yl]carbonyl}-D-tyrosinate (150 mg, 93%) was obtained as a colorless oil by methods similar to those of Example 115-1) from 6-(4-chlorophenyl)-3-methyl-1-benzofuran-2-carboxylic acid (143 mg, 0.50 mmol) and methyl O-benzyl-D-tyrosinate hydrochloride (129 mg, 0.40 mmol).
   ¹H-NMR (300MHz, CDCl₃) δ : 7.67-7.30 (12H, m), 7.11-7.04 (3H, m), 6.94-6.90 (2H, d), 5.08-5.03 (3H, m), 3.76 (3H, s), 3.22-3.20 (2H, d), 2.64 (3H, s)
   LC-MS 554 [M+H]⁺
2) The title compound (85 mg, 58%) was obtained as white crystals by methods similar to those of Example 115-2) from methyl O-benzyl-N-{[6-(4-chlorophenyl)-3-methyl-1-benzofuran-2-yl]carbonyl}-D-tyrosinate (150 mg, 0.27 mmol).
   ¹H-NMR (300MHz, DMSO-d₆) δ : 12.90 (1H, s), 8.51-8.49 (1H, d), 7.83-7.78 (4H, m), 7.68-7.65 (1H, d), 7.56-7.54 (2H, d), 7.43-7.27 (5H, m), 7.22-7.19 (2H, d), 6.93-6.90 (2H, d), 5.03 (2H, s), 4.67-4.60 (1H, m), 3.14-3.11 (2H, m), 2.49 (3H, s)
   LC-MS 540 [M+H]⁺
   Melting point: 113-114°C
   Elemental analysis: Calcd. for C₃₂H₂₆NO₅Cl: C 71.17, H 4.85, N 2.59; Found: C 70.85, H 4.80, N 2.40

### Example C117

### N-{[6-(4-Chlorophenyl)-3-methyl-1-benzofuran-2-yl]carbonyl}-O-(4-fluorobenzyl)-DL-tyrosine

1) Methyl N-{[6-(4-chlorophenyl)-3-methyl-1-benzofuran-2-yl]carbonyl}-O-(4-fluorobenzyl)-DL-tyrosinate (208 mg, 91%) was obtained as a colorless oil by methods similar to those of Example 115-1) from 6-(4-chlorophenyl)-3-methyl-1-benzofuran-2-carboxylic acid (143 mg, 0.50 mmol) and methyl O-(4-fluorobenzyl)-DL-tyrosinate hydrochloride (136 mg, 0.40 mmol).
   ¹H-NMR (300MHz, CDCl₃) δ : 7.67-7.35 (9H, m), 7.12-7.02 (5H, m), 6.90-6.88 (2H, d), 5.09-5.03 (1H, m), 4.99 (2H, s), 3.77 (3H, s), 3.21-3.19 (2H, m), 2.64 (3H, s)
   LC-MS 572 [M+H]⁺
2) The title compound (97 mg, 57%) was obtained as white crystals by methods similar to those of Example 115-2) from methyl N-{[6-(4-chlorophenyl)-3-methyl-1-benzofuran-2-yl] carbonyl}-O-(4-fluorobenzyl)-DL-tyrosinate (174 mg, 0.30 mmol).
   ¹H-NMR (300MHz, DMSO-d₆) δ : 12.91 (1H, br s), 8.50-8.48 (1H, d, J=7.8 Hz), 7.88 (1H, s), 7.83-7.78 (3H, m), 7.68-7.65 (1H, d), 7.56-7.53 (2H, d), 7.48-7.44 (2H, m), 7.22-7.18 (4H, m), 6.92-6.90 (2H, d), 5.01 (2H, s), 4.67-4.59 (1H, m), 3.18-3.07 (2H, m), 2.51 (3H, s)
   LC-MS 558 [M+H]⁺
   Melting point: 147-149°C

### Example C118

### N-{[6-(4-Chlorophenyl)-3-methyl-1-benzofuran-2-yl]carbonyl}-O-(3-thienylmethyl)-L-tyrosine

1) Methyl O-(3-thienylmethyl)-N-{[6-(4-chlorophenyl)-3-methyl-1-benzofuran-2-yl]carbonyl}-L-tyrosinate (127 mg, 76%) was obtained as a colorless oil by methods similar to those of Example 115-1) from 6-(4-chlorophenyl)-3-methyl-1-benzofuran-2-carboxylic acid (115 mg, 0.40 mmol) and methyl O-(3-thienylmethyl)-L-tyrosinate hydrochloride (98 mg, 0.30 mmol). ¹H-NMR (300MHz, CDCl₃) δ : 7.67-7.43 (7H, m), 7.34-7.31 (2H, m), 7.14-7.04 (4H, m), 6.91-6,90 (2H, d), 5.04 (2H, s), 4.16-4.08 (1H, m), 3.77 (3H, s), 3.22-3.20 (2H, m), 2.64 (3H, s)
   LC-MS 560 [M+H]⁺
2) 1N-NaOH aqueous solution (456 µL) was added with ice cooling to a methanol (1 mL)-THF (1 mL) solution of methyl N-{[6-(4-chlorophenyl)-3-methyl-1-benzofuran-2-yl]carbonyl}-0-(3-thienylmethyl)-L-tyrosinate (128 mg, 0.23 mmol), and stirred overnight at room temperature. 1N-hydrochloric acid (1 mL) was added, and the mixture was extracted twice with ethyl acetate. The organic layers were combined, washed with sodium chloride solution, dried over magnesium sulfate and then concentrated under reduced pressure. The residue was recrystallized from an ethyl acetate-hexane mixed solvent to obtain the title compound (79 mg, 64%) as white crystals.
   ¹H-NMR (300MHz, DMSO-d₆) δ : 12.90 (1H, s), 8.49-8.46 (1H, d, J=8.1 Hz), 7.88 (1H, s), 7.83-7.79 (3H, m), 7.68-7.65 (1H, dd), 7.56-7.50 (4H, m), 7.21-7.19 (2H, d, J=8.7Hz), 7.15-7.14 (1H, d), 6.92-6.89 (2H, d), 5.01 (2H, s), 4.66-4.59 (1H, m), 3.18-3.12 (2H, m), 2.51 (3H, s)
   LC-MS 546 [M+H]⁺
   Melting point: 111-113°C
   Elemental analysis: Calcd. for C₃₀H₂₄NO₅SCl•0.2 H₂O: C 65.56, H 4.47, N 2.54; Found: C 65.47, H 4.44, N 2.39

### Example C119

### N- { [6-(4-Chlorophenyl)-3-methyl-1-benzofuran-2-yl] carbonyl} -O-(3-furylmethyl)-L-tyrosine

1) Methyl N-{[6-(4-chlorophenyl)-3-methyl-1-benzofuran-2-yl]carbonyl}-O-(3-furylmethyl)-L-tyrosinate (151 mg, 93%) was obtained as a colorless oil by methods similar to those of Example 115-1) from 6-(4-chlorophenyl)-3-methyl-1-benzofuran-2-carboxylic acid (115 mg, 0.40 mmol) and methyl O-(3-furylmethyl)-L-tyrosinate hydrochloride (98 mg, 0.30 mmol). ¹H-NMR (300MHz, CDCl₃) δ : 7.67-7.41 (9H, m), 7.09-7.04 (3H, m), 6.91-6.88 (2H, d), 6.47 (1H, s), 5.09-5.03 (1H, m), 4.91 (2H, s), 3.77 (3H, s), 3.27-3.20 (2H, m), 2.63 (3H, s)
   LC-MS 544 [M+H]⁺
2) 1N-NaOH aqueous solution (522 µL) was added with ice cooling to a methanol (1 mL)-THF (1mL) of methyl O-(3-furylmethyl)-N-{[6-(4-chlorophenyl)-3-methyl-1-benzofuran-2-yl]carbonyl}-L-tyrosinate (142 mg, 0.26 mmol), and stirred overnight at room temperature. 1 N-hydrochloric acid (1 mL) was added, and the mixture was extracted twice with ethyl acetate. The organic layers were combined, washed with sodium chloride solution, dried over magnesium sulfate and then concentrated under reduced pressure. The residue was recrystallized from an ethyl acetate-hexane mixed solvent to obtain the title compound (50.7 mg, 37%) as white crystals.
   ¹H-NMR (300MHz, DMSO-d₆) δ : 12.90 (1H, s), 8.50-8.47 (1H, d), 7.88 (1H, d), 7.83-7.79 (3H, m), 7.74 (1H, s), 7.68-7.62 (2H, m), 7.56-7.54 (2H, d), 7.22-7.19 (2H, d), 6.91-6.88 (2H, d), 6.53 (1H, d), 4.88 (2H, s), 4.67-4.60 (1H, m), 3.28-3.12 (2H, m), 2.51 (3H, s)
   LC-MS 530 [M+H]⁺
   Melting point: 108-110°C
   Elemental analysis: Calcd. for C₃₀H₂₄NO₆Cl•0.2 H₂O: C 67.53, H 4.61, N 2.62; Found: C 67.56, H 4.66, N 2.42

### Example C120

### N-[(3-Methyl-6-{4-[(2-methyl-1 H-benzimidazol-1-yl)methyl]phenyl}-1-benzofuran-2-yl)carbonyl]glycine

1) A mixture of 3-methyl-6-{4-[(2-methyl-1H-benzimidazol-1-yl)methyl]phenyl}-1-benzofuran-2-carboxylic acid (200 mg), glycine ethyl ester hydrochloride (84 mg), (3-dimethylaminopropyl)ethyl carbodiimide hydrochloride (116 mg), 1-hydroxybenzotriazole (82 mg), triethylamine (0.17 mL) and N,N-dimethylformamide (5 mL) was stirred for 20 hours at room temperature. Water was added to the reaction mixture, which was then extracted with ethyl acetate. The extract was washed with saturated sodium chloride solution, and dried over anhydrous magnesium sulfate. The solvent was evaporated under reduced pressure, and the residue was purified by silica gel column chromatography to obtain ethyl N-[(3-methyl-6-{4-[(2-methyl-1H-benzimidazol-1-yl)methyl]phenyl}-1-benzofuran-2-yl)carbonyl]glycinate (223 mg) as colorless crystals.
   Melting point: 184-186°C (ethyl acetate-hexane)
   ¹H-NMR (300MHz, CDCl₃) δ 1.33 (3H, t, J=7.2Hz), 2.62 (3H, s), 2.64 (3H, s), 4.25 (2H, d, J=5.4Hz), 4.28 (2H, q, J=7.2Hz), 5.40 (2H, s), 7.08-7.30 (6H, m), 7.49 (1H, dd, J=1.5, 8.1Hz), 7.55-7.68 (4H, m), 7.73-7.78 (1H, m)
2) 2 M Sodium hydroxide aqueous solution (0.5 mL) and water (1 mL) were added to a tetrahydrofuran-ethanol mixed solution (3 mL-1.5 mL) of ethyl N-[(3-methyl-6-{4-[(2-methyl-1H-benzimidazol-1-yl)methyl]phenyl}-1-benzofuran-2-yl)carbonyl]glycinate (133 mg), and stirred for 1 hour at 60°C. The reaction solution was neutralized with 1 N hydrochloric acid, and the precipitated solid was filtered out, washed successively with water, methanol and diethyl ether and dried to obtain the title compound (96 mg) as colorless crystals.
   Melting point: 248-250°C
   ¹H-NMR (300MHz, DMSO-d₆)δ 2.55 (3H, s), 2.67 (3H, s), 3.93 (2H, d, J=5.7Hz), 5.62 (2H, s), 7.20-7.40 (4H, m), 7.55-7.90 (7H, m), 8.71 (1H, m), 12.62 (1H, br s)

### Example C121

### N-[(3-Methyl-6-{ 4-[(2-methyl-1H-benzimidazol-1-yl)methyl]phenyl}-1-benzofuran-2-yl)carbonyl]-(3-alanine

1) A mixture of 3-methyl-6-{4-[(2-methyl-1H-benzimidazol-1-yl)methyl]phenyl}-1-benzofuran-2-carboxylic acid (200 mg), β-alanine ethyl ester hydrochloride (93 mg), (3-dimethylaminopropyl)ethyl carbodiimide hydrochloride (116 mg), 1-hydroxybenzotriazole (82 mg), triethylamine (0.17 mL) and N,N-dimethylformamide (5 mL) was stirred for 20 hours at room temperature. Water was added to the reaction mixture, which was then extracted with ethyl acetate. The extract was washed with saturated sodium chloride solution, and dried over anhydrous magnesium sulfate. The solvent was evaporated under reduced pressure, and the residue was purified by silica gel column chromatography to obtain ethyl N-[(3-methyl-6-{4-[(2-methyl-1H-benzimidazol-1-yl)methyl]phenyl}-1-benzofuran-2-yl)carbonyl]-(3-alaninate (262 mg) as colorless crystals.
   Melting point: 196-197°C (ethyl acetate-hexane)
   ¹H-NMR (300MHz, CDCl₃)δ 1.28 (3H, t, J=7.2Hz), 2.62 (3H, s), 2.63 (3H, s), 2.67 (2H, t, J=6.0Hz), 3.75 (2H, q, J=6.0Hz), 4.19 (2H, q, J=7.2Hz), 5.40 (2H, s), 7.13-7.32 (6H, m), 7.48 (1H, dd, J=1.5, 8.1Hz), 7.55-7.68 (4H, m), 7.74-7.79 (1H, m)
2) 2 M Sodium hydroxide aqueous solution (0.5 mL) and water (1 mL) were added to a tetrahydrofuran-ethanol mixed solution (3 mL-1.5 mL) of ethyl N-[(3-methyl-6-{4-[(2-methyl-1H-benzimidazol-1-yl)methyl]phenyl}-1-benzofuran-2-yl)carbonyl]-β-alaninate (145 mg), and stirred for 1 hour at 60°C. The reaction solution was neutralized with 1 N hydrochloric acid, and extracted with tetrahydrofuran-ethyl acetate. The extract was washed with saturated sodium chloride solution and dried over anhydrous magnesium sulfate. The solvent was evaporated under reduced pressure, and the residue was washed with diethyl ether and dried to obtain the title compound (65 mg) as colorless crystals.
   Melting point: 260-263°C
   ¹H-NMR (300MHz, DMSO-d₆)δ 2.45-2.55 (2H, m), 2.54 (3H, s), 2.61 (3H, s), 3.42-3.52 (2H, m), 5.57 (2H, s), 7.15-7.30 (4H, m), 7.50-7.80 (7H, m), 8.47 (1H, t, J=5.7Hz), 12.20 (1H, br s)

The structural formulae for the Compounds of Examples C112 through C121 are shown in Table 26.

**(Table 26)**

| | | | |
|---|---|---|---|
| Example C112 | 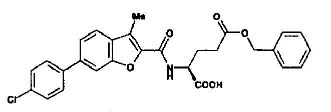 | Example C117 | 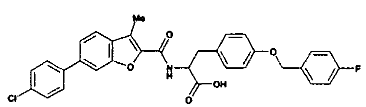 |
| Example C113 | 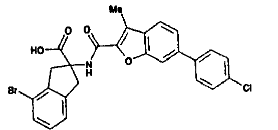 | Example C118 | 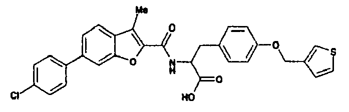 |
| Example C114 | 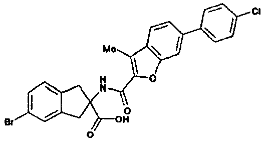 | Example C119 | 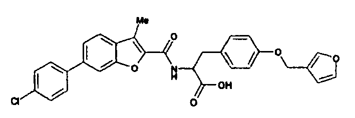 |
| Example C115 | 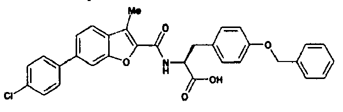 | Example C120 | 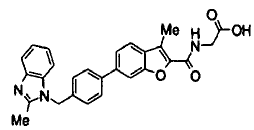 |
| Example C116 | 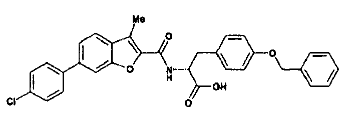 | Example C121 | 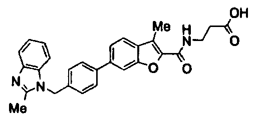 |

### Example C122

### O-Benzyl-N-{[6-(4-chlorophenyl)-1-benzothiophene-2-yl]carbonyl}tyrosine

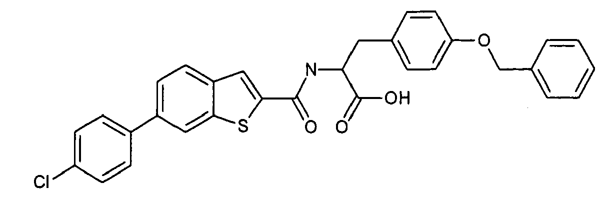

(1) A mixture of 6-(4-chlorophenyl)-1-benzothiophene-2-carboxylic acid (289 mg), methyl O-benzyl tyrosinate hydrochloride (340 mg), (3-dimethylaminopropyl)ethyl carbodiimide hydrochloride (230 mg), 1-hydroxybenzotriazole (162 mg), triethylamine (0.28 mL) and N,N-dimethylformamide (15 mL) was stirred overnight at room temperature. Water was added to the reaction mixture, which was then extracted with ethyl acetate. The extract was washed successively with hydrochloric acid, sodium hydroxide aqueous solution and saturated sodium chloride solution, and dried over anhydrous magnesium sulfate. The solvent was evaporated under reduced pressure, and the residue was purified by silica gel column chromatography to obtain methyl O-benzyl-N-{[6-(4-chlorophenyl)-1-benzothiophene-2-yl]carbonyl}tyrosinate (512 mg) as colorless crystals.
   Melting point: 200-201°C (ethyl acetate-hexane)
   ¹H-NMR (300MHz, CDCl₃); δ 3.19 (1H, dd, J=5.1, 14.1Hz), 3.26 (1H, dd, J=5.7, 14.1Hz), 3.79 (3H, s), 5.04 (2H, s), 5.07 (1H, dd, J=5.1, 5.7Hz), 6.56 (1H, d, J=7.8Hz), 6.92 (2H, d, J=8.7Hz), 7.07 (2H, d, J=8.7Hz), 7.28-7.47 (7H, m), 7.55-7.62 (3H, m), 7.73 (1H, s), 7.89 (1H, d, J=8.4Hz), 8.02 (1H, s)
   Elemental analysis: Calcd. for C₃₂H₂₆ClNO₄S: C 69.12, H 4.71, N 2.52; Found: C 69.04, H 4.58, N 2.30
(2) 2 N Sodium hydroxide aqueous solution (0.5 mL) and water (2.5 mL) were added to a tetrahydrofuran-methanol mixed solution (4 mL-2 mL) of the compound obtained in (1) (255 mg), and stirred for 1 hour at 60°C. The reaction solution was neutralized with 2 N hydrochloric acid, and extracted with ethyl acetate-tetrahydrofuran. The extract was washed with saturated sodium chloride solution and dried over anhydrous magnesium sulfate. The solvent was evaporated under reduced pressure, and the residue was recrystallized from tetrahydrofuran-diethyl ether to obtain the title compound (187 mg) as colorless crystals.
   Melting point: 237-239°
   ¹H-NMR (300MHz, DMSO-d₆); δ 3.01 (1H, dd, J=10.5, 13.8Hz), 3.15 (1H, dd, J=4.5, 13.8Hz), 4.50-4.61 (1H, m), 5.03 (2H, s), 6. 91 (2H, d, J=8.7Hz), 7. 21-7. 43 (7H, m), 7. 5 (2H, d, J=8.7Hz), 7. 75 (1H, dd, J=1.5, 8.4Hz), 7.82 (2H, d, J=8.7Hz), 8.05 (1H, d, J=8.4Hz), 8.18 (1H, s), 8.34-8.38 (1 K m), 9.02 (1 K d, J=8.4Hz), 12.58 (1H, br s)
   Elemental analysis: Calcd. for C₃₁H₂₄ClNO₄S: C 68.69, H 4.46, N 2.58; Found: C 68.61, H 4.35, N 2.41

### Example C123

### O-Benzyl-N-{[5-(4-chlorophenyl)-1-benzothiophene-2-yl]carbonyl}tyrosine

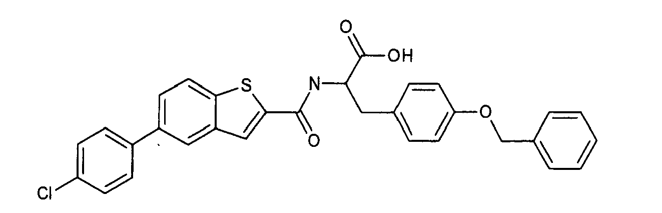

(1) Methyl O-benzyl-N-{[5-(4-chlorophenyl)-1-benzothiophene-2-yl]carbonyl}tyrosinate (477 mg) was obtained as colorless crystals by operations similar to those of Example C122(1) from 5-(4-chlorophenyl)-1-benzothiophene-2-carboxylic acid (289 mg) and methyl O-benzyl tyrosinate hydrochloride (340 mg).
   ¹H-NMR (300MHz, CDCl₃); δ 3.15-3.30 (2H, m), 3.79 (3H, s), 5.04 (2H, s), 5.04-5.10 (1H, m), 6.57 (1H, d, J=7.5Hz), 6.92 (2H, d, J=8.7Hz), 7.06 (2H, d, J=8.7Hz), 7.31-7.47 (7H, m), 7.57 (2H, d, J=8.7Hz), 7.63 (1H, dd, J=1.8, 8.7Hz), 7.76 (1H, s), 7.92 (1H, d, J=8.7Hz), 7.98 (1H, d, J=1.8Hz)
(2) The title compound (223 mg) was obtained as colorless crystals by operations similar to those of Example C122(2) from the compound (300 mg) obtained in (1).
   Melting point: 206-207°C (ethyl acetate-hexane)
   ¹H-NMR (300MHz, DMSO-d₆); 6 3.02 (1H, dd, J=10.5, 13.8Hz), 3.15 (1H, dd, J=1.2, 13.8Hz), 4.50-4.60 (1H, m), 5.02 (2H, s), 6.91 (2H, d, J=8.7Hz), 7.25 (2H, d, J=8.7Hz), 7.28-7.43 (5H, m), 7.56 (2H, d, J=8.4Hz), 7.77 (1H, dd, J=1.8, 8.7Hz), 7.80 (2H, d, J=8.4Hz), 8.10 (1H, d, J=8.7Hz), 8.20 (1H, s), 8.26 (1H, d, J=1.8Hz), 9.06 (1H, d, J=8.4Hz), 12.90 (1H, br s)
   Elemental analysis: Calcd. for C₃₁H₂₄ClNO₄S: C 68.69, H 4.46, N 2.58; Found: C 68.40, H 4.33, N 2.53

### Example C124

### N-{[6-(4-Chlorophenyl)-1-benzothiophene-2-yl]carbonyl}-O-(3-furylmethyl) tyrosine

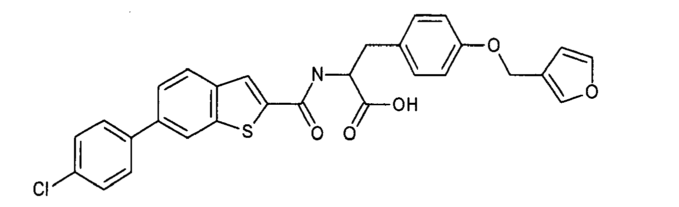

(1) Methyl N-{[6-(4-chlorophenyl)-1-benzothiophene-2-yl]carbonyl}-O-(3-furylmethyl) tyrosinate (153 mg) was obtained as colorless crystals by operations similar to those of Example C122 (1) from 6-(4-chlorophenyl)-1-benzothiophene-2-carboxylic acid (92 mg) and methyl O-(3-furylmethyl) tyrosinate hydrochloride (100 mg).
   ¹H-NMR (300 MHz, CDCl₃); δ 3.15-3.30 (2H, m), 3.79 (3H, s), 4.91 (2H, s), 5.03-5.10 (1H, m), 6.47 (1H, d, J=0.9 Hz), 6.56 (1H, d, J=7.5 Hz), 6.90 (2H, d, J=8.7 Hz), 7.07 (2H, d, J=8.7 Hz), 7.40-7.62 (7H, m), 7.74 (1H, s), 7.89 (1H, d, J=8.4 Hz), 8.02 (1H, s).
(2) The title compound (110 mg) was obtained as colorless crystals by operations similar to those of Example C122(2) from the compound (143 mg) obtained in (1).
   Melting point: 200-201°C (ethyl acetate-hexane)
   ¹H-NMR (300 MHz- DMSO-d₆): δ 3.01 (1H, dd, J=10.5, 13.8 Hz), 3.15 (1H, dd, J=4.5, 13.8 Hz), 4.50-4.60 (1H, m), 4.88 (2H, s), 6.52 (1H, d, J=1.5 Hz), 6.90 (2H, d, J=8.7 Hz), 7.24 (2H, d, J=8.7 Hz), 7.55 (2H, d, J=8.7 Hz), 7.62 (1H, t, J=1.5 Hz), 7.73-7.83 (4H, m), 8.05 (1H, d, J=8.4 Hz), 8.18 (1H, s), 8.36 (1H, br s), 9.02 (1H, d, 8.4 Hz), 12.85 (1H, br s)
   Elemental analysis: Calcd. for C₂₉H₂₂ClNO₅S: C 65.47, H 4.17, N 2.63; Found: C 65.42, H 4.11, N 2.67

### Example C125

### N-{[5-(4-Chlorophenyl)-1-benzothiophene-2-yl]carbonyl}-O-(3-furylmethyl) tyrosine

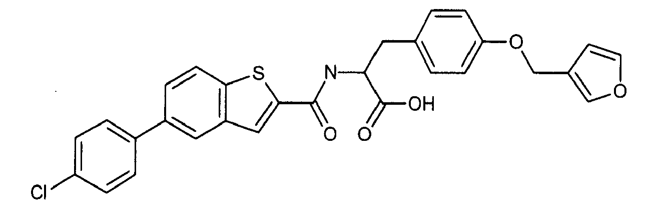

(1) Methyl N-{[5-(4-chlorophenyl)-1-benzothiophene-2-yl]carbonyl}-O-(3-furylmethyl) tyrosinate (153 mg) was obtained as colorless crystals by operations similar to those of Example C122 (1) from 5-(4-chlorophenyl)-1-benzothiophene-2-carboxylic acid (92 mg) and methyl O-(3-furylmethyl)tyrosinate hydrochloride (100 mg).
   ¹H-NMR (300MHz, CDCl₃); δ 3.15-3.30 (2H, m), 3.79 (3H, s), 4.91 (2H, s), 5.03-5.10 (1H, m), 6.47 (1H, d, J=0.9Hz), 6.56 (1H, d, J=7.5Hz), 6.90 (2H, d, J=8.7Hz), 7.07 (2H, d, J=8.7Hz), 7.40-7.51 (4H, m), 7.57 (2H, d, J=8.7Hz), 7.63 (1H, dd, J=1.8, 8.4Hz), 7.76 (1H, s), 7.92 (1H, d, J=8.4Hz), 7.98 (1H, d, J=1.5Hz)
(2) The title compound (87 mg) was obtained as colorless crystals by operations similar to those of Example C122(2) from the compound (143 mg) obtained in (1).
   Melting point: 172-173°C (ethyl acetate-hexane)
   ¹H-NMR (300MHz, DMSO-d₆); δ 3.01 (1H, dd, J=10.8, 13.8Hz), 3.15 (1H, dd, J=4.5, 13.8Hz), 4.50-4.60 (1H, m), 4.88 (2H, s), 6.52 (1H, d, J=1.5Hz), 6.89 (2H, d, J=8.4Hz), 7.24 (2H, d, J=8.4Hz), 7.56 (2H, d, J=8.4Hz), 7.62 (1H, t, J=1.5Hz), 7.72-7.82 (4H, m), 8.10 (1H, d, J=8.4Hz), 8.20 (1H, s), 8.26 (1H, d, J=1.5Hz), 9.06 (1H, d, J=8.1Hz), 12.84 (1H, br s)
   Elemental analysis: Calcd. for C₂₉H₂₂ClNO₅S•0.25 H₂O; C 64.92, H 4.23, N 2.61; Found: C 64.92, H 4.27, N 2.63

### Example C126

### N-{[5-(4-Chlorophenyl)-1-benzothiophene-2-yl]carbonyl}-O-(3-fluorobenzyl) tyrosine

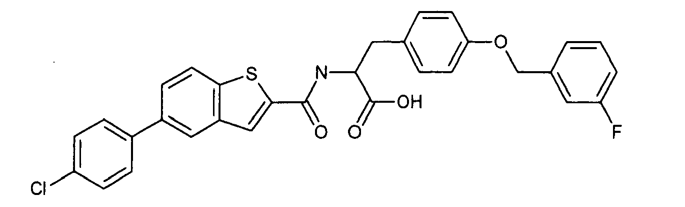

(1) Methyl N-{[5-(4-chlorophenyl)-1-benzothiophene-2-yl]carbonyl}-O-(3-furylbenzyl)tyrosinate (139 mg) was obtained as colorless crystals by operations similar to those of Example C122(1) from 5-(4-chlorophenyl)-1-benzothiophene-2-carboxylic acid (100 mg) and methyl O-(3-fluorobenzyl)tyrosinate hydrochloride (119 mg).
   ¹H-NMR (300MHz, CDCl₃); δ 3.15-3.30 (2H, m), 3.79 (3H, s), 5.04 (2H, s), 5.04-5.10 (1H, m), 6.57 (1H, d, J=7.5Hz), 6.90 (2H, d, J=8.7Hz), 6.95-7.20 (5H, m), 7.29-7.38 (1H, m), 7.45 (2H, d, J=8.7Hz), 7. 5 7 (2H, d, J=8 . 7Hz), 7.63 (1H, dd, J=1.8, 8.4Hz), 7.76 (1H, s), 7.92 (1H, d, J=8.4Hz), 7.98 (1H, d, J=1.8Hz)
(2) The title compound (108 mg) was obtained as colorless crystals by operations similar to those of Example C122(2) from the compound (134 mg) obtained in (1).
   Melting point: 184-185°C (ethyl acetate-hexane)
   ¹H-NMR (300MHz, DMSO-d₆); δ 3.02 (1H, dd, J=10.5, 13.8Hz), 3.16 (1H, dd, J=4.5, 13.8Hz), 4.50-4.62 (1H, m), 5.06 (2H, s), 6.92 (2H, d, J=8.7Hz), 7.07-7.29 (5H, m), 7.35-7.44 (1H, m), 7.56 (2H, d, J=8.7Hz), 7.75-7.83 (3H, m), 8.10 (1H, d, J=8.4Hz), 8.20 (1H, s), 8.26 (1H, d, J=1.5Hz), 9.07 (1H, d, J=8.1Hz), 12.84 (1H, br s)
   Elemental analysis: Calcd. for C₃₁H₂₃ClFNO₄S•0.25 H₂O: C 65.95, H 4.20, N 2.48; Found: C 65.93, H 4.19, N 2.46

### Example C127

### N-{[5-(4-Chlorophenyl)-1-benzothiophene-2-yl]carbonyl}-O-(3-methylbenzyl)tyrosine

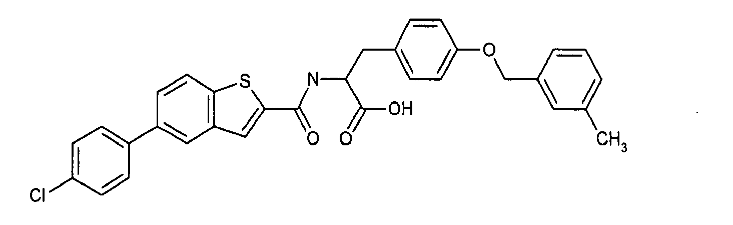

(1) Methyl N-{[5-(4-chlorophenyl)-1-benzothiophene-2-yl]carbonyl}-O-(3-methylbenzyl)tyrosinate (147 mg) was obtained as colorless crystals by operations similar to those of Example C122 (I) from 5-(4-chlorophenyl)-1-benzothiophene-2-carboxylic acid (100 mg) and methyl O-(3-methylbenzyl)tyrosinate hydrochloride (118 mg).
   ¹H-NMR (300MHz, CDCl₃); δ 2.36 (3H, s), 3.15-3.30 (2H, m), 3.79 (3H, s), 5.00 (2H, s), 5.00-5.10 (1H, m), 6.5 (1H, d, J=7.5Hz), 6.92 (2H, d, J=8.7Hz), 7.07 (2H, d, J=8.7Hz), 7.10-7.30 (4H, m), 7.45 (2H, d, J=8.7Hz), 7.57 (2H, d, J=8.7Hz), 7.63 (1H, dd, J=1.8, 8.4Hz), 7.76 (1H, s), 7.91 (1H, d, J=8.4Hz), 7.98 (1H, d, J=1.8Hz)
(2) The title compound (116 mg) was obtained as colorless crystals by operations similar to those of Example C122(2) from the compound (142 mg) obtained in (1).
   Melting point: 192-193°C (ethyl acetate-hexane)
   ¹H-NMR (300MHz, DMSO-d₆); δ 2.28 (3H, s), 3.01 (1H, dd, J=10.5, 13.8Hz), 3.15 (1H, dd, J=4.2, 13.8Hz), 4.50-4.62 (1H, m), 4.98 (2H, s), 6.90 (2H, d, J=8.7Hz), 7.07-7.27 (6H, m); 7.56 (2H, d, J=8.7Hz), 7.77 (1H, dd, J=1.8, 8.7Hz), 7.80 (2H, d, J=8.7Hz), 8.10 (1H, d, J=8.7Hz), 8.20 (1H, s), 8.26 (1H, d, J=1.8Hz), 9.05 (1H, d, J=8.7Hz), 12.85 (1H, br s)
   Elemental analysis: Calcd. for C₃₂H₂₆CINO₄S: C 69.12, H 4.71, N 2.52; Found: C 68.88, H 4.74, N 2.49

### Example C128

### N-{[5-(4-Chlorophenyl)-1-benzothiophene-2-yl]carbonyl}-O-(3-methoxybenzyl)tyrosine

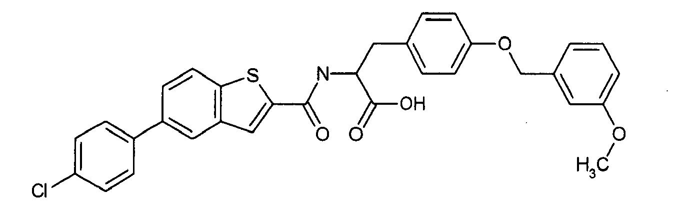

(1) Methyl N-{[5-(4-chlorophenyl)-1-benzothiophene-2-yl]carbonyl}-O-(3-methoxybenzyl)tyrosinate (172 mg) was obtained as colorless crystals by operations similar to those of Example C122(1) from 5-(4-chlorophenyl)-1-benzothiophene-2-carboxylic acid (100 mg) and methyl O-(3-methoxybenzyl)tyrosinate hydrochloride (123 mg).
   ¹H-NMR (300MHz, CDCl₃); δ 3.15-3.30 (2H, m), 3.79 (3H, s), 3.81 (3H, s), 5.02 (2H, s), 5.02-5.10 (1H, m), 6.58 (1H, d, J=7.5Hz), 6.83-7.09 (7H, m), 7.26-7.32 (1H, m), 7.45 (2H, d, J=8.7Hz), 7.57 (2H, d, J=8.7Hz), 7.63 (1H, dd, J=1.8, 8.4Hz), 7.76 (1H, s), 7.91 (1H, d, J=8.4Hz), 7.98 (1H, d, J=1.8Hz)
(2) The title compound (112 mg) was obtained as colorless crystals by operations similar to those of Example C122(2) from the compound (167 mg) obtained in (1).
   Melting point: 175-177°C (ethyl acetate-hexane)
   ¹H-NMR (300MHz, DMSO-d₆); δ 3.02 (1H, dd, J=10.5, 13.8Hz), 3.15 (1H, dd, J=4.2, 13.8Hz), 3.72 (3H, s), 4.50-4.60 (1H, m), 5.00 (2H, s), 6.82-6.99 (5H, m), 7.22-7.29 (3H, m), 7.56 (2H, d, J=8.7Hz), 7.74-7.84 (3H, m), 8.10 (1H, d, J=8.4Hz), 8.20 (1H, s), 8.26 (1H, d, J=1.2Hz), 9.05 (1H, d, J=8.4Hz), 12.84 (1H, br s)
   Elemental analysis: Calcd. for C₃₂H₂₆ClNO₅S•0.25 H₂O: C 66.66, H 4.63, N 2.43; Found: C 66.71, H 4.60, N 2.45

### Example C129

### O-(4-Fluorobenzyl)-N-{[5-(4-methoxyphenyl)-1-benzothiophene-2-yl]carbonyl}tyrosine

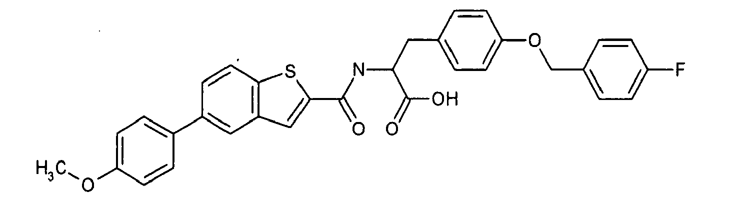

(1) Methyl O-(4-fluorobenzyl)-N-{[5-(4-methoxyphenyl)-1-benzothiophene-2-yl]carbonyl}tyrosinate (169 mg) was obtained as colorless crystals by operations similar to those of Example C122(1) from 5-(4-methoxyphenyl)-1-benzothiophene-2-carboxylic acid (100 mg) and methyl O-(4-fluorobenzyl)tyrosinate hydrochloride (119 mg).
   ¹H-NMR (300MHz, CDCl₃); δ 3.15-3.30 (2H, m), 3.79 (3H, s), 3.87 (3H, s), 4.99 (2H, s), 5.03-5.10 (1H, m), 6.56 (1H, d, J=7.5Hz), 6.90 (2H, d, J=8. 7Hz), 6.99-7.10 (6H, m), 7.35-7.42 (2H, m), 7.57 (2H, d, J=8.7Hz), 7.65 (1H, dd, J=1.5, 8.4Hz), 7.76 (1H, s), 7.89 (1H, d, J=8.4Hz), 7.97 (1H, d, J=1.5Hz)
(2) The title compound (133 mg) was obtained as colorless crystals by operations similar to those of Example C122(2) from the compound (169 mg) obtained in (1).
   Melting point: 192-193°C (ethyl acetate-hexane)
   ¹H-NMR (300MHz, DMSO-d₆); δ 3.01 (1H, dd, J=10.5, 13.8Hz), 3.15 (1H, dd, J=4.2, 13.8Hz), 3.82 (3H, s), 4.52-4.60 (1H, m), 5.01 (2H, s), 6.91 (2H, d, J=8.7Hz), 7.07 (2H, d, J=8.7Hz), 7.13-7.22 (2H, m), 7.25 (2H, d, J=8.7Hz), 7.42-7.49 (2H, m), 7.68-7.75 (3H, m), 8.05 (1H, d, J=8.7Hz), 8.17-8.20 (2H, m), 9.03 (1H, d, J=8.1Hz), 12.84 (1H, br s)
   Elemental analysis: Calcd. for C₃₂H₂₆FNO₅: C 69.17, H 4.72, N 2.52; Found (%): C 69.12, H 4.71, N 2.57

### Example C130

### O-(4-Fluorobenzyl)-N-{[5-(4-isopropoxyphenyl)-1-benzothiophene-2-yl]carbonyl}tyrosine

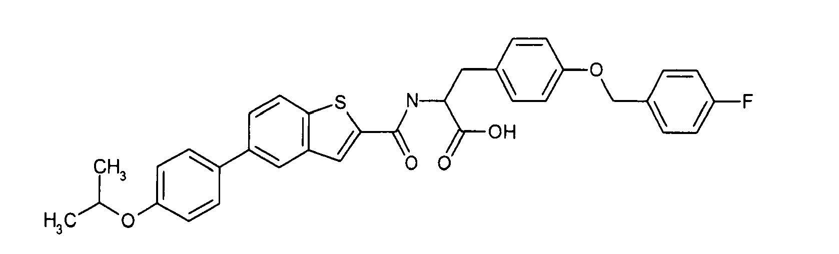

(1) Methyl O-(4-fluorobenzyl)-N-{[5-(4-isopropoxyphenyl)-1-benzothiophene-2-yl]carbonyl}tyrosinate (174 mg) was obtained as colorless crystals by operations similar to those of Example C122(1) from 5-(4-isopropoxyphenyl)-1-benzothiophene-2-carboxylic acid (109 mg) and methyl O-(4-fluorobenzyl)tyrosinate hydrochloride (119 mg).
   ¹H-NMR (300MHz, CDCl₃);δ 1.38 (6H, d, J=6.0Hz), 3.15-3.30 (2H, m), 3.79 (3H, s), 4.61 (1H, m), 4.99 (2H, s), 5.03-5.09 (1H, m), 6.56 (1H, d, J=7.5Hz), 6.90 (2H, d, J=8.7Hz), 6.99 (2H, d, J=8.7Hz), 7.03-7.11 (4H, m), 7.35-7.43 (2H, m), 7.55 (2H, d, J=8.7Hz), 7.65 (1H, dd, J=1.5, 8.4Hz), 7.75 (1H, s), 7.88 (1H, d, J=8.4Hz), 7.96 (1H, d, J=1.5Hz)
(2) The title compound (128 mg) was obtained as colorless crystals by operations similar to those of Example C122(2) from the compound (174 mg) obtained in (1).
   Melting point: 204-205°C (ethyl acetate-hexane)
   ¹H-NMR (300MHz, DMSO-d₆);δ 1.30 (6H, d, J=6.0Hz), 3.01 (1H, dd, J=10.8, 13.8Hz), 3.15 (1H, dd, J=4. 5, 13.8Hz), 4.52-4.60 (1H, m), 4.68 (1H, m), 5.00 (2H, s), 6.91 (2H, d, J=8.7Hz), 7.04 (2H, d, J=8.7Hz), 7.13-7.22 (2H, m), 7.26 (2H, d, J=8.7Hz), 7.42-7.48 (2H, m), 7.68 (2H, d, J=8.7Hz), 7.72 (1H, dd, J=1.5, 8.4Hz), 8.04 (1H, d, J=8.4Hz), 8.17 (1H, d, J=1.5Hz), 8.18 (1H, s), 9.03 (1H, d, J=8.1Hz), 12.84 (1H, br s)
   Elemental analysis: Calcd. for C₃₄H₃₀FNO₅S : C 69.96, H 5.18, N 2.40; Found: C 70.04, H 5.24, N 2.46

### Example C131

### O-(4-Fluorobenzyl)-N-([5-(4-propoxyphenyl)-1-benzothiophene-2-yl]carbonyl)tyrosine

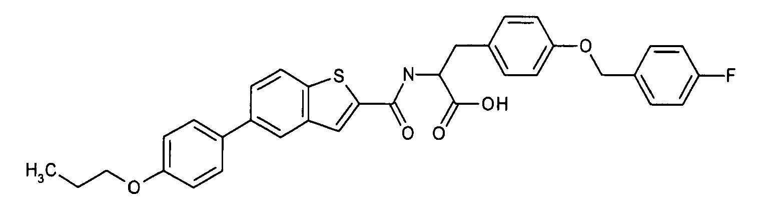

(1) Methyl O-(4-fluorobenzyl)-N-{[5-(4-propoxyphenyl)-1-benzothiophene-2-yl]carbonyl}tyrosinate (192 mg) was obtained as colorless crystals by operations similar to those of Example C122(1) from 5-(4-propoxyphenyl)-1-benzothiophene-2-carboxylic acid (109 mg) and methyl O-(4-fluorobenzyl) tyrosinate hydrochloride (119 mg).
   ¹H-NMR (300MHz, CDCl₃); δ 1.07 (3H, t, J=7.2Hz), 1.85 (2H, m), 3.15-3.30 (2H, m), 3.79 (3H, s), 3.98 (2H, t, J=6.6Hz), 4.99 (2H, s), 5.03-5.10 (1H, m), 6.55 (1H, d, J=7.5Hz), 6.90 (2H, d, J=8.7Hz), 7.01 (2H, d, J=8.7Hz), 7.01-7.10 (4H, m), 7.35-7.43 (2H, m), 7.56 (2H, d, J=8.7Hz), 7.65 (1H, dd, J=1.5, 8.4Hz), 7.75 (1H, s), 7.88 (1H, d, J=8.4Hz), 7.97 (1H, d, J=1.5Hz)
(2) The title compound (99 mg) was obtained as colorless crystals by operations similar to those of Example C122(2) from the compound (192 mg) obtained in (1).
   Melting point: 211-212°C (ethyl acetate-hexane)
   ¹H-NMR (300MHz, DMSO-d₆); δ 1.00 (3H, t, J=7.5Hz), 1.76 (2H, m), 3.01 (1H, dd, J=10.5, 13.8Hz), 3.15 (1H, dd, J=4.5, 13.8Hz), 3.99 (2H, t, J=6.6Hz), 4.56 (1H, m), 5.00 (2H, s), 6.91 (2H, d, J=8.7Hz), 7.05 (2H, d, J=8.7Hz), 7.13-7.22 (2H, m), 7.26 (2H, d, J=8.7Hz), 7.42-7.49 (2H, m), 7.66-7.75 (3H, m), 8.04 (1H, d, J=8.4Hz), 8.16-8.20 (2H, m), 9.04 (1H, d, J=8.1Hz), 12.84 (1H, br s)
   Elemental analysis: Calcd. for C₃₄H₃₀FNO₅S: C 69.96, H 5.18, N 2.40; Found: C 69.94, H 5.14, N 2.37

### Example C132

### O-(4-Fluorobenzyl)-N-{[5-(4-methylphenyl)-1-benzothiophene-2-yl]carbonyl}tyrosine

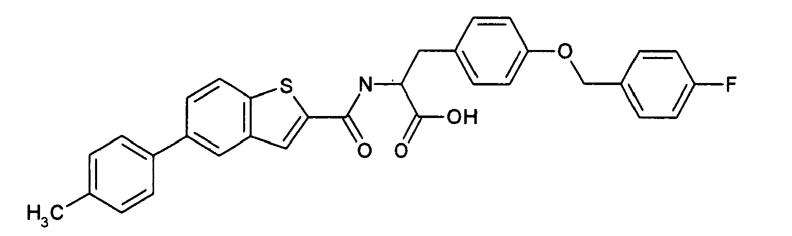

(1) Methyl O-(4-fluorobenzyl)-N-{[5-(4-methylphenyl)-1-benzothiophene-2-yl]carbonyl}tyrosinate (173 mg) was obtained as colorless crystals by operations similar to those of Example C122(1) from 5-(4-methylphenyl)-1-benzothiophene-2-carboxylic acid (94 mg) and methyl O-(4-fluorobenzyl)tyrosinate hydrochloride (119 mg).
   ¹H-NMR (300MHz, CDCl₃); δ 2.42 (3H, s), 3.15-3.30 (2H, m), 3.79 (3H, s), 4.99 (2H, s), 5.03-5.10 (1H, m), 6.55 (1H, d, J=7.8Hz), 6.90 (2H, d, J=8.4Hz), 7.02-7.10 (4H, m), 7.25-7.30 (2H, m), 7.35-7.43 (2H, m), 7.54 (2H, d, J=8.4Hz), 7.67 (1H, dd, J=1.5, 8.4Hz), 7.76 (1H, s), 7.90 (1H, d, J=8.4Hz), 8.00 (1H, d, J=1.5Hz)
(2) The title compound (120 mg) was obtained as colorless crystals by operations similar to those of Example C122(2) from the compound (173 mg) obtained in (1).
   ¹H-NMR (300MHz, DMSO-d₆); δ 2.37 (3H, s), 3.02 (1H, dd, J=10.5, 13.8Hz), 3.15 (1H, d, J=4.5, 13.8Hz), 4.57 (1H, m), 5.01 (2H, s), 6.91 (2H, d, J=8.4Hz), 7.13-7.34 (6H, m), 7.42-7.48 (2H, m), 7.65 (2H, d, J=8.4Hz), 7.74 (1H, dd, J=1.8, 8.4Hz), 8.06 (1H, d, J=8.4Hz), 8.19 (1H, s), 8.20 (1H, d, J=1. 8Hz), 9.04 (1H, d, J=8.1Hz), 12.84 (1H, br s)
   Elemental analysis: Calcd. for C₃₂H₂₆FNO₄S: C 71.23, H 4.86, N 2.60; Found: C 71.24, H 4.80, N 2.57

### Example C133

### N-{[5-(4-Chlorophenyl)-1-benzothiophene-2-yl]carbonyl}-O-(4-fluorobenzyl)tyrosine

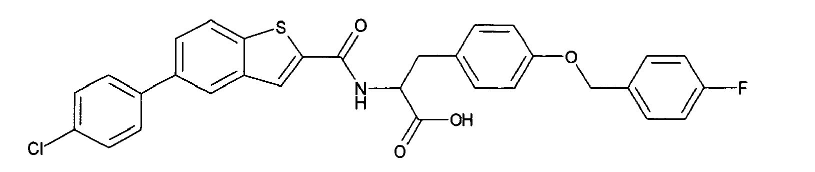

(1) A mixture of methyl N-(tert-butoxycarbonyl)tyrosinate (0.59 g, 2 mmol), 4-fluorobenzyl bromide (450 mg, 2.4 mmol), potassium carbonate (415 mg) and dimethylformamide (3 mL) was stirred overnight at room temperature. Ethyl acetate and water were added to separate the solution, and the organic layer was washed twice with water and once with saturated sodium chloride solution and dried over magnesium sulfate. After removal of the drying agent by filtration, this was concentrated under reduced pressure. The residue was purified by silica gel column chromatography (eluate: ethyl acetate/hexane = 1/10-1/1) to obtain methyl N-tert-butoxycarbonyl)-O-(4-fluorobenzyl)tyrosinate (720 mg, 93%) as a white solid.
   ¹H-NMR (300MHz, CDCl₃); δ1.42 (s, 9H), 2.96-3.09 (m, 2H), 3.71 (s, 3H), 4.53-4.55 (m, 1H), 4.94-4.99 (m, 3H), 6.87-6.90 (d, J=8.7Hz, 2H), 7.03-7.11 (m, 4H), 7.37-7.41 (m, 3H)
(2) 4 N Hydrogen chloride ethyl acetate solution (1 mL) was added to an ethyl acetate (3 mL) solution of methyl N-(tert-butoxycarbonyl)-O-(4-fluorobenzyl)tyrosinate (720 mg, 1.87 mmol), and stirred overnight at room temperature. The precipitated white solid was filtered out and washed successively with ethyl acetate and diethyl ether to obtain methyl O-(4-fluorobenzyl)tyrosinate hydrochloride (568 mg, 89%) as a white solid.
   LC-MS 304 [M+H]⁺
   ¹H-NMR (300MHz, DMSO-d₆); δ2.99-3.12 (m, 2H), 3.68 (s, 3H), 4.21-4.26 (m, 1H), 5.00 (s, 2H), 6.96-6.99 (d, J=8.4Hz, 2H), 7.13-7.25 (m, 4H), 7.47-7.52 (m, 2H), 8.45 (s, 3H)
(3) A mixture of 5-(4-chlorophenyl)-1-benzothiophene-2-carboxylic acid (144 mg, 0.50 mmol), methyl O-(4-fluorobenzyl)tyrosinate hydrochloride (153 mg, 0.45 mmol), (3-dimethylaminopropyl)ethyl carbodiimide hydrochloride (192 mg, 1.00 mmol), 1-hydroxybenzotriazole (135 mg, 1.00 mmol), triethylamine (300 µL, 2.15 mmol), N,N-dimethylformamide (2 mL) and dichloromethane (4 mL) was stirred overnight at room temperature. Water was added to the reaction solution, which was then extracted with ethyl acetate. The extract was washed with water and saturated sodium chloride solution and then dried over anhydrous magnesium sulfate. The drying agent was removed by filtration, the solvent was evaporated under reduced pressure, and the residue was purified by silica gel column chromatography (eluate: ethyl acetate/hexane = 1/10-1/1) and recrystallized from a mixed ethyl acetate-hexane solvent to obtain methyl N-{[5-(4-chlorophenyl)-1-benzothiophene-2-yl]carbonyl}-O-(4-fluorobenzyl)tyrosinate (227 mg, 88%) as a white solid.
   LC-MS 574 [M+H]⁺
   ¹H-NMR (300MHz, CDCl₃); δ 3.16-3.30 (m, 2H), 3.79 (s, 3H), 4.99 (s, 2H), 5.03-5.09 (m, 1H), 6.58-6.60 (d, J=7.5Hz, 1H), 6.88-6.91 (d, J=8.7Hz, 2H), 7.03-7.09 (m, 4H), 7.35-7.46 (m, 4H), 7.54-7.57 (m, 2H), 7.61-7.64 (m, 1H), 7.76 (s, 1H), 7.90-7.93 (d, J=8.4Hz, 1H), 7.98 (s, 1H)
(4) 1 M Sodium hydroxide aqueous solution (795 µL) was added to a tetrahydrofuran-methanol mixed solution (2 mL-2 mL) of methyl N-{[5-(4-chlorophenyl)-1-benzothiophene-2-yl]carbonyl}-O-(4-fluorobenzyl)tyrosinate (227 mg, 0.39 mmol), and stirred for 2 hours at room temperature. The reaction solution was neutralized with 1 N hydrochloric acid (850 µL) and extracted twice with ethyl acetate, and the organic layer was washed with saturated sodium chloride solution and dried over magnesium sulfate. After removal of the drying agent by filtration, this was concentrated under reduced pressure. The residue was recrystallized from ethyl acetate-hexane to obtain the title compound (179 mg, 81%) as a white solid.
   LC-MS 560 [M+H]⁺
   ¹H-NMR (300MHz, DMSO-d₆); δ 2.97-3.14 (m, 2H), 4.53-4.61 (m, 1H), 5.00 (s, 2H), 6.89-6.92 (d, J=8.4Hz, 2H), 7.14-7.27 (m, 4H), 7.43-7.48 (m, 2H), 7.54-7.57 (d, J=8.4Hz, 2H), 7.75-7.82 (m, 3H), 8.09-8.12 (d, J=8.7Hz, 1H), 8.20 (s, 1H), 8.26(d, J=1.8Hz, 1H), 9.06-9.09 (d, J=8.1Hz, 1H), 12.80 (br s, 1H)
   Melting point: 189-190°C
   Elemental analysis: Calcd. for C₃₁H₂₃NO₄SClF: C 66.48, H 4.14, N 2.50; Found: C 66.39, H 4.16, N 2.46

### Example C134

### N-{ [5-(4-Chlorophenyl)-1-benzothiophene-2-yl]carbonyl }-O-(4-methylbenzyl)tyrosine

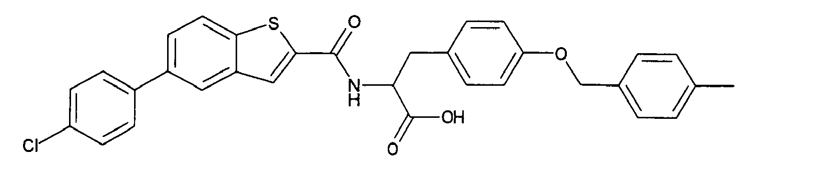

(1) A mixture of methyl N-(tert-butoxycarbonyl)tyrosinate (2.95 g, 10 mmol), 4-methylbenzyl bromide (2.22 g, 12 mmol), potassium carbonate (2.76 g) and dimethyl formamide (20 mL) was stirred overnight at room temperature. Ethyl acetate and water were added to separate the solution, and the organic layer was washed twice with water and once with saturated sodium chloride solution and dried over magnesium sulfate. After removal of the drying agent by filtration, this was concentrated under reduced pressure. The residue was purified by silica gel column chromatography (eluate: ethyl acetate/hexane = 1/10-1/1) to obtain methyl N-(tert-butoxycarbonyl)-O-(4-methylbenzyl)tyrosinate (3.10 g, 77%) as a white solid.
   ¹H-NMR (300MHz, CDCl₃); δ1.42(s, 9H), 2.36 (s, 3H), 2.95-3.08 (m, 2H), 3.70 (s, 3H), 4.53-4.55 (d, J=6.9Hz, 1H), 4.94-4.99 (m, 3H), 6.87-6.91 (d, J=8.7Hz, 2H), 7.01-7.04 (d, J=8.4Hz, 2H), 7.17-7.20 (d, J=7.8Hz, 2H), 7.29-7.32 (d, J=8.1Hz, 2H)
(2) 4 N Hydrogen chloride ethyl acetate solution (4 mL) was added to an ethyl acetate (10 mL) solution of methyl N-(tert-butoxycarbonyl)-O-(4-methylbenzyl)tyrosinate (3.10 g, 7.76 mmol), and stirred overnight at room temperature. The precipitated white solid was filtered out and washed successively with ethyl acetate and diethyl ether to obtain methyl O-(4-methylbenzyl)tyrosinate hydrochloride (2.52 g, 90%) as a white solid.
   ¹H-NMR (300MHz, DMSO-d₆); δ2.30 (s, 3H), 3.00-3.05 (m, 2H), 3.68 (s, 3H), 4.21-4.26 (t, J=6.6Hz, 1H), 5.03 (s, 2H), 6.94-6.97 (d, J=8.7Hz, 2H), 7.12-7.21 (m, 4H), 7.31-7.33 (d, J=7.8Hz, 2H), 8.40 (s, 3H)
(3) A mixture of 5-(4-chlorophenyl)-1-benzothiophene-2-carboxylic acid (144 mg, 0.50 mmol), methyl O-(4-methylbenzyl)tyrosinate hydrochloride (151 mg, 0.45 mmol), (3-dimethylaminopropyl)ethyl carbodiimide hydrochloride (192 mg, 1.00 mmol), 1-hydroxybenzotriazole (135 mg, 1.00 mmol), triethylamine (300 µL, 2.1 mmol), N,N-dimethylformamide (2 mL) and dichloromethane (4 mL) was stirred overnight at room temperature. Water was added to the reaction solution, which was then extracted with ethyl acetate. The extract was washed with water and saturated sodium chloride solution and then dried over anhydrous magnesium sulfate. The drying agent was removed by filtration, the solvent was evaporated under reduced pressure, and the residue was purified by silica gel column chromatography (eluate: ethyl acetate/hexane = 1/10-1/1) and recrystallized from a mixed ethyl acetate-hexane solvent to obtain methyl N-{[5-(4-chlorophenyl)-1-benzothiophene-2-yl]carbonyl}-O-(4-methylbenzyl)tyrosinate (191 mg, 75%) as a white solid.
   LC-MS 570 [M+H]⁺
   ¹H-NMR (300MHz, CDCl₃); δ 2.35 (s, 3H), 3.18-3.27 (m, 2H), 3.79 (s, 3H), 4.99 (s, 2H), 5.03-5.09 (m, 1H), 6.56-6.59 (d, 7.5Hz, 1H), 6.90-6.92 (d, J=8.7Hz, 2H), 7.04-7.07 (d, 8.7Hz, 2H), 7.17-7.19 (d, J=7.8Hz, 2H), 7.29-7.32 (d, J=7.8Hz, 2H), 7.43-7.46 (d, J=8.7Hz, 2H), 7.55-7.58 (d, J=8.4Hz, 2H), 7.61-7.65 (dd, J=1.5, 6.9Hz, 1H), 7.76 (s, 1H), 7.90-7.93 (d, J=8.7Hz, 1H), 7.98 (s, 1H)
(4) 1 M Sodium hydroxide aqueous solution (670 µL) was added to a tetrahydrofuran-methanol mixed solution (2 mL-2 mL) of methyl N-{[5-(4-chlorophenyl)-1-benzothiophene-2-yl]carbonyl}-O-(4-methylbenzyl)tyrosinate (191 mg, 0.34 mmol), and stirred for 2 hours at room temperature. The reaction solution was neutralized with 1 N hydrochloric acid (700 µL), and the precipitated white solid was filtered out and washed successively with water and diethyl ether to obtain the title compound (164 mg, 88%) as a white solid.
   LC-MS 556 [M+H]⁺
   ¹H-NMR (300MHz, DMSO-d₆); δ 2.27 (s, 3H), 2.97-3.18 (m, 2H), 4.52-4.59 (m, 1H), 4.97 (s, 2H), 6.88-6.91 (d, J=8.7Hz, 2H), 7.13-7.16 (d, J=8.1Hz, 2H), 7.22-7.29 (m, 4H), 7.54-7.57 (d, J=8.7Hz, 2H), 7.75-7.82 (m, 3H), 8.09-8.12 (d, J=8.4Hz, 1H), 8.20 (s, 1H), 8.26 (J=1.2Hz, 1H), 9.05-9.07 (d, 8.1Hz, 1H), 12.80 (br s, 1H)
   Melting point: 208°C
   Elemental analysis: Calcd. for C₃₂H₂₆NO₄SCl: C 69.12, H 4.71, N 2.52; Found: C 69.09, H 4.70, N 2.57

### Example C135

### O-(4-Chlorobenzyl)-N-([5-(4-chlorophenyl)-1-benzothiophene-2-yl]carbonyl)tyrosine

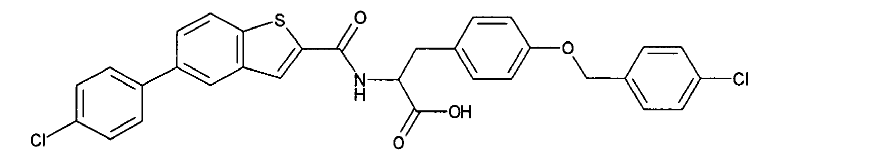

(1) A mixture of methyl N-(tert-butoxycarbonyl)tyrosinate (2.95 g, 10 mmol), 4-chlorobenzyl chloride (1.93 g, 12 mmol), potassium carbonate (2.76 g) and dimethylformamide (20 mL) was stirred overnight at room temperature. Ethyl acetate and water were added to separate the solution, and the organic layer was washed twice with water and once with saturated sodium chloride solution and dried over magnesium sulfate. After removal of the drying agent by filtration, this was concentrated under reduced pressure. The residue was recrystallized from an ethyl acetate-hexane mixed solvent to obtain methyl N-(tert-butoxycarbonyl)-O-(4-chlorobenzyl)tyrosinate (2.83 g, 67%) as a white solid
   ¹H-NMR (300MHz, CDCl₃); δ1.42 (s, 9H), 2.95-3.09 (m, 2H), 3.71 (s, 3H), 4.53-4.55 (m, 1H), 4.91-5.00 (m, 3H), 6.86-6.89 (d, J=8.4Hz, 2H), 7.02-7.05 (d, J=8.4Hz, 2H), 7.35 (s, 4H)
(2) 4 N Hydrogen chloride ethyl acetate solution (3.36 mL) was added to an ethyl acetate (5 mL) solution of methyl N-(tert-butoxycarbonyl)-O-(4-chlorobenzyl)tyrosinate (2.83 g, 6.74 mmol), and stirred overnight at room temperature. The precipitated white solid was filtered out and washed successively with ethyl acetate and diethyl ether to obtain methyl O-(4-chlorobenzyl)tyrosinate hydrochloride (0.78 g, 92%) as a white solid.
   ¹H-NMR (300MHz, DMSO-d₆); δ2.99-3.14 (m, 2H), 3.67 (s, 3H), 4.19-4.24 (t, J=6.6Hz, 1H), 5.09 (s, 2H), 6.95-6.98 (d, J=8.4Hz, 2H), 7.14-7.17 (d, J=8.4Hz, 2H), 7.43-7.49 (m, 4H), 8.54 (s, 3H)
(3) A mixture of 5-(4-chlorophenyl)-1-benzothiophene-2-carboxylic acid (144 mg, 0.50 mmol), methyl O-(4-chlorobenzyl)tyrosinate hydrochloride (160 mg, 0.45 mmol), (3-dimethylaminopropyl)ethyl carbodiimide hydrochloride (192 mg, 1.00 mmol), 1-hydroxybenzotriazole (135 mg, 1.00 mmol), triethylamine (300 µL, 2.1 mmol), N,N-dimethylformamide (2 mL) and dichloromethane (4 mL) was stirred overnight at room temperature. Water was added to the reaction solution, which was then extracted with ethyl acetate. The extract was washed with water and saturated sodium chloride solution and then dried over anhydrous magnesium sulfate. The drying agent was removed by filtration, the solvent was evaporated under reduced pressure and the residue was purified by silica gel column chromatography (eluate: ethyl acetate/hexane = 1/10-1/1) and recrystallized from a mixed ethyl acetate-hexane solvent to obtain methyl N-{[5-(4-chlorophenyl)-1-benzothiophene-2-yl]carbonyl}-O-(4-chlorobenzyl)tyrosinate (218 mg, 82%) as a white solid
   LC-MS 590 [M+H]⁺
   ¹H-NMR (300MHz, DMSO-d₆); δ 3.00-3.17 (m, 2H), 3.66 (s, 3H), 4.59-4.67 (m, 1H), 5.04 (s, 2H), 6.90-6.93 (d, J=8.7Hz, 2H), 7.23-7.25 (d, J=8.4Hz, 2H), 7.33-7.45 (m, 4H), 7.54-7.58 (d, J=8.4Hz, 2H), 7.76-7.81 (m, 3H), 8.09-8.12 (d, J=8.7Hz, 1H), 8.21 (s, 1H), 8.26-8.27 (d, J=1.5Hz, 1H), 9.21-9.23 (d, J=7.8Hz, 1H)
(4) 1 M Sodium hydroxide aqueous solution (738 µL) was added to a tetrahydrofuran-methanol mixed solution (2 mL-2 mL) of methyl N-{[6-(4-chlorophenyl)-benzothiophene-2-yl]carbonyl}-O-(4-chlorobenzyl)tyrosinate (218 mg, 0.37 mmol), and stirred for 1.5 hours at room temperature. The reaction solution was neutralized with 1 N hydrochloric acid (750 µL), extracted twice with ethyl acetate, washed with saturated sodium chloride solution and dried over magnesium sulfate. After removal of the drying agent by filtration, this was concentrated under reduced pressure. The residue was recrystallized from a mixed ethyl acetate-hexane solvent to obtain the title compound (178 mg, 84%) as a white solid.
   LC-MS 576 [M+H]⁺
   ¹H-NMR (300MHz, DMSO-d₆); δ 2.98-3.19 (m, 2H), 4.53-4.61 (m, 1H), 5.03 (s, 2H), 6.89-6.92 (d, J=8.4Hz, 2H), 7.24-7.27 (d, J=8.7Hz, 2H), 7.38-7.45 (m, 4H), 7.54-7.58 (m, 2H), 7.75-7.82 (m, 3H), 8.09-8.12 (d, J=8.7Hz, 1H), 8.20 (s, 1H), 8.26 (d, J=1.2Hz, 1H), 9.06-9.09 (d, J=8.1Hz, 1H), 12.80 (br s, 1H)
   Melting point: 196-197°C
   Elemental analysis: Calcd. for C₃₁H₂₃NO₄SCl₂: C 64.59, H 4.02, N 2.43; Found: C 64.46, H 3.99, N 2.31

### Example C136

### N-{[5-(4-Chlorophenyl)-1-benzothiophene-2-yl]carbonyl}-O-(4-cyanobenzyl)tyrosine

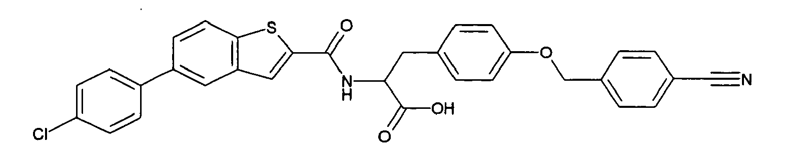

(1) A mixture of methyl N-(tert-butoxycarbonyl)tyrosinate (2.95 g, 10 mmol), 4-cyanobenzyl bromide (2.35 g, 12 mmol), potassium carbonate (2.76 g) and dimethylformamide (20 mL) was stirred for 2 hours at room temperature and 2 hours at 50°C. Ethyl acetate and water were added to separate the solution, and the organic layer was washed twice with water and once with saturated sodium chloride solution and dried over magnesium sulfate. After removal of the drying agent by filtration, this was concentrated under reduced pressure. The residue was recrystallized from an ethyl acetate-hexane mixed solvent to obtain methyl N-(tert-butoxycarbonyl)-O-(4-cyanobenzyl)tyrosinate (3.72 g, 91%) as a white solid.
   ¹H-NMR (300MHz, CDCl₃); δ 1.32 (s, 9H), 2.89-3.10 (m, 2H), 3.71 (s, 3H), 4.53-4.56 (d, J=7.2Hz, 1H), 4.95-4.97 (d, J=7.8Hz, 1H), 5.17 (s, 2H), 6.86-6.89 (d, J=8.7Hz, 2H), 7.04-7.07 (d, J=8.7Hz, 2H), 7.52-7.55 (d, J=8.4Hz, 2H), 7.67-7.69 (d, J=8.4Hz, 2H)
(2) 4 N Hydrogen chloride ethyl acetate solution (1.2 mL) was added to an ethyl acetate (5 mL)-tetrahydrofuran (2 mL) solution of methyl N-(tert-butoxycarbonyl)-O-(4-cyanobenzyl)tyrosinate (1.00 g, 2.43 mmol), and stirred overnight at room temperature. The precipitated white solid was filtered out and washed successively with ethyl acetate and diethyl ether to obtain methyl O-(4-cyanobenzyl)tyrosinate hydrochloride (0.78 g, 92%) as a white solid.
   LC-MS 311 [M+H]⁺
   ¹H-NMR (300MHz, DMSO-d₆); δ 2.99-3.13 (m, 2H), 3.67 (s, 3H), 4.20-4.25 (t, J=6.6Hz, 1H), 5.21 (s, 2H), 6.97-7.00 (d, J=8.7Hz, 2H), 7.15-7.17 (d, J=8.7Hz, 2H), 7.62-7.65 (d, J=8.4Hz, 2H), 7.86-7.89 (d, J=8.1Hz, 2H), 8.52 (s, 3H)
(3) A mixture of 5-(4-chlorophenyl)-1-benzothiophene-2-carboxylic acid (144 mg, 0.50 mmol), methyl O-(4-cyanobenzyl)tyrosinate hydrochloride (156 mg, 0.45 mmol), (3-dimethylaminopropyl)ethyl carbodiimide hydrochloride (192 mg, 1.00 mmol), 1-hydroxybenzotriazole (135 mg, 1.00 mmol), triethylamine (300 µL, 2.1 mmol), N,N-dimethylformamide (2 mL) and dichloromethane (4 mL) was stirred overnight at room temperature. Water was added to the reaction solution, which was then extracted with ethyl acetate. The extract was washed with water and saturated sodium chloride solution and then dried over anhydrous magnesium sulfate. The drying agent was removed by filtration, the solvent was evaporated under reduced pressure and the residue was purified by silica gel column chromatography (eluate: ethyl acetate/hexane = 1/10-1/1) and recrystallized from a mixed ethyl acetate-hexane solvent to obtain methyl N-{[5-(4-chlorophenyl)-1-benzothiophene-2-yl]carbonyl}-O-(4-cyanobenzyl)tyrosinate (200 mg, 77%) as a white solid.
   LC-MS 581 1 [M+H] ⁺
   ¹H-NMR (300MHz, DMSO-d₆); δ3.01-3.17 (m, 2H), 3.66 (s, 3H), 4.60-4,67 (m, 1H), 5.16 (s, 2H), 6.91-6. 94 (d, J=8.7Hz, 2H), 7.24-7.26 (d, J=8. 7Hz, 2H), 7.55-7.61 (m, 4H), 7.76-7.84 (m, 5H), 8.09-8.12 (d, J=8.4Hz, 1H), 8.21 (s, 1H), 8.26-8.27 (d, J=1.2Hz, 1H), 9.21-9.23 (d, J=7.8Hz,1H)
(4) 1 M Sodium hydroxide aqueous solution (690 µL) was added to a tetrahydrofuran-methanol mixed solution (2 mL-2 mL) of methyl N-{[5-(4-chlorophenyl)-1-benzothiophene-2-yl)carbonyl}-O-(4-cyanobenzyl)tyrosinate (200 mg, 0.35 mmol), and stirred for 2 hours at room temperature. The reaction solution was neutralized with 1 N hydrochloric acid (750 µL), extracted twice with ethyl acetate, washed with saturated sodium chloride solution and dried over magnesium sulfate. After removal of the drying agent by filtration, this was concentrated under reduced pressure. The residue was recrystallized from a mixed ethyl acetate-hexane solvent and then recrystallized again from a mixed ethanol-isopropyl ethyl ether solvent to obtain the title compound (129 mg, 66%) as white crystals.
   LC-MS 567 [M+H]⁺
   ¹H-NMR (300MHz, DMSO-d₆); δ 2.97-3.18 (m, 2H), 4.52-4.60 (m, 1H), 5.15 (s, 2H), 6.90-6.93 (d, J=8.7Hz, 2H), 7.24-7.27 (d, J=8.7Hz, 2H), 7.54-7.61 (m, 4H), 7.75-7.84 (m, 5H), 8.09-8.11 (d, J=8.4Hz, 1H), 8.20 (s, 1H), 8.26 (d, J=1.5Hz, 1H), 9.05-9.07 (d, J=7.8Hz, 1H), 12.80 (br s, 1H)
   Melting point: 164°C
   Elemental analysis: Calcd. for C₃₂H₂₃N₂O₄SCl: C 67.78, H 4.09, N 4.94; Found: C 67.55, H 4.10, N 4.81

### Example C137

### N-{[5-(4-Chlorophenyl)-1-benzothiophene-2-yl]carbonyl}-O-(4-ethylbenzyl)tyrosine

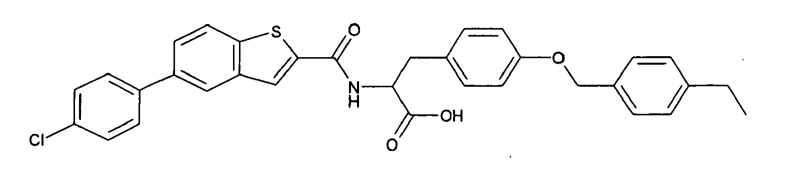

(1) A mixture of methyl N-(tert-butoxycarbonyl)tyrosinate (2.95 g, 10 mmol), 4-ethylbenzyl chloride (1.50 g, 12 mmol), potassium carbonate (2.76 g) and dimethylformamide (15 mL) was stirred for 3 hours at room temperature. Ethyl acetate and water were added to separate the solution, and the organic layer was washed twice with water and once with saturated sodium chloride solution and dried over magnesium sulfate. After removal of the drying agent by filtration, this was concentrated under reduced pressure. The residue was purified by silica gel chromatography (eluate: ethyl acetate/hexane = 1/10-1/1) to obtain a white solid (3.13 g). 4 N hydrogen chloride ethyl acetate solution (3.87 mL) was added to an ethyl acetate (5 mL)-tetrahydrofuran (2 mL) solution of this solid, and stirred overnight at room temperature. The precipitated white solid was filtered out and washed successively with ethyl acetate and diethyl ether to obtain methyl O-(4-ethylbenzyl)tyrosinate hydrochloride (1.61 g, 47%) as a white solid. ¹H-NMR (300MHz, DMSO-d₆); δ 1.15-1.21 (m, 3H), 2.57-2.71 (m, 2H), 2.99-3.13 (m, 2H), 3.67 (s, 3H), 4.19-4.25 (m, 1H), 5.04 (s, 2H), 6.94-6.97 (d, J=8.7Hz, 2H), 7.13-7.16 (d, J=8.7Hz, 2H), 7.21-7.23 (d, J=7.8Hz, 2H), 7.33-7.36 (d, J=8.1Hz, 2H), 8.53 (s, 3H)
(2) A mixture of 5-(4-chlorophenyl)-1-benzothiophene-2-carboxylic acid (144 mg, 0.50 mmol), methyl O-(4-ethylbenzyl)tyrosinate hydrochloride (153 mg, 0.45 mmol), (3-dimethylaminopropyl)ethyl carbodiimide hydrochloride (192 mg, 1.00 mmol), 1-hydroxybenzotriazole (135 mg, 1.00 mmol), triethylamine (300 µL, 2.1 mmol), N,N-dimethylformamide (2 mL) and dichloromethane (4 mL) was stirred overnight at room temperature. Water was added to the reaction solution, which was then extracted with ethyl acetate. The extract was washed with water and saturated sodium chloride solution and then dried over anhydrous magnesium sulfate. The drying agent was removed by filtration, the solvent was evaporated under reduced pressure, and the residue was purified by silica gel column chromatography (eluate: ethyl acetate/hexane = 1/10-1/1) and recrystallized from a mixed ethyl acetate-hexane solvent to obtain methyl N-{[5-(4-chlorophenyl)-1-benzothiophene-2-yl]carbonyl}-O-(4-ethylbenzyl)tyrosinate (112 mg, 43%) as a white solid.
   LC-MS 584 [M+H]⁺
   ¹H-NMR (300MHz, DMSO-d₆); δ 1.12-1.17 (t, J=7.5Hz, 3H), 2.51-2.65 (m, 2H), 3.00-3.16 (m, 2H), 3.66 (s, 3H), 4.59-4.63 (m, 1H), 4.99 (s, 2H), 6.89-6.92 (d, J=8.4Hz, 2H), 7.16-7.32 (m, 6H), 7.55-7.57 (d, J=8.7Hz, 2H), 7.76-7.82 (m, 3H), 8.09-8.12 (d, J=8.4Hz, 1H), 8.21 (s, 1H), 8.27 (s, 1H), 9.20-9.23 (s, J=7.8Hz, 1H)
(3) 1 M Sodium hydroxide aqueous solution (380 µL) was added to a tetrahydrofuran-methanol mixed solution (2 mL-2 mL) of methyl N-{[5-(4-chlorophenyl)-1-benzothiophene-2-yl]carbonyl}-O-(4-ethylbenzyl)tyrosinate (112 mg, 0.19 mmol), and stirred for 1 hour at 60°C. The reaction solution was neutralized with 1 N hydrochloric acid (400 µL), extracted twice with ethyl acetate, washed with saturated sodium chloride solution and dried over magnesium sulfate. After removal of the drying agent by filtration, this was recrystallized from ethyl acetate-hexane to obtain the title compound (96.9 mg, 88%) as colorless crystals.
   LC-MS 570 [M+H]⁺
   ¹H-NMR (300MHz, DMSO-d₆); δ 1.12-1.17 (t, J=7.8Hz, 3H), 2.55-2.73 (m, 2H), 2.97-3.18 (m, 2H), 4.52-4.59 (m, 1H), 4.98 (s, 2H), 6.88-6.91 (d, J=8.4Hz, 2H), 7.16-7.32 (m, 6H), 7.54-7.57 (d, J=8.7Hz, 2H), 7.75-7.82 (m, 3H), 8.09-8.12 (d, J=8.4Hz, 1H), 8,20 (s, 1H), 8.26 (s, 1H), 9.04-9.07 (d, J=8.1 Hz, 1H), 12.80 (br s, 1H)
   Melting point: 205°C
   Elemental analysis: Calcd. for C₃₃H₂₈NO₄SCl (containing 1 mol H₂O): C 67.39, H 5.14, N 2.38; Found: C 67.20, H 4.86, N 2.34

### Example C138

### N-{[5-(4-Chlorophenyl)-1-benzothiophene-2-yl]carbonyl }-O-[4-(trifluoromethyl)benzyl]tyrosine

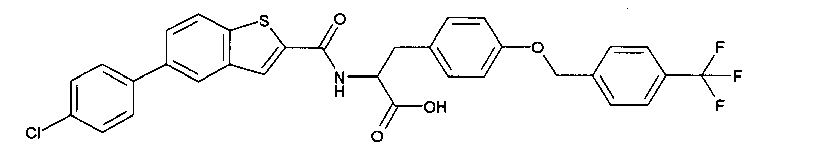

(1) A mixture of methyl N-(tert-butoxycarbonyl)tyrosinate (2.95 g, 10 mmol), 4-(trifluoromethyl) benzyl bromide (2.87 g, 12 mmol), potassium carbonate (2.76 g) and dimethylformamide (15 mL) was stirred for 4 hours at room temperature. Ethyl acetate and water were added to separate the solution, and the organic layer was washed twice with water and once with saturated sodium chloride solution and dried over magnesium sulfate. After removal of the drying agent by filtration, this was concentrated under reduced pressure. The residue was purified by silica gel chromatography (eluate: ethyl acetate/hexane = 1/10-1/1) to obtain methyl N-(tert-butoxycarbonyl)-O-[4-(trifluoromethyl)benzyl]tyrosinate (4.04 g, 100%) as a white solid.
   ¹H-NMR (300MHz, CDCl₃); δ 1.414 (s, 9H), 2.95-3.10 (m, 2H), 3.71 (s, 3H), 4.53-4.56 (d, J=7.2Hz, 1H), 4.95-4.97 (d, J=7.5 Hz, 1H), 5.10 (s, 2H), 6.86-6.91 (d, J=8.7Hz, 2H), 7.03-7.06 (d, J=8.7Hz, 2H), 7.53-7.55 (d, J=8.1 Hz, 2H), 7.63-7.66 (d, J=8.4Hz, 2H)
(2) 4 N Hydrogen chloride ethyl acetate solution (5 mL) was added to an ethyl acetate (5 mL)-tetrahydrofuran (4 mL) solution of methyl N-(tert-butoxycarbonyl)-O-[4-(trifluoromethyl)benzyl]tyrosinate (4.04 g, 10 mmol), and stirred overnight at room temperature. The precipitated white solid was filtered out and washed successively with ethyl acetate and diethyl ether to obtain methyl O-[4-(trifluoromethyl)benzyl]tyrosinate hydrochloride (3.15 g, 81 %) as a white solid.
   LC-MS 354 [M+H]⁺
   ¹H-NMR (300MHz, DMSO-d₆); δ 3.00-3.14 (m, 2H), 3.67 (s, 3H), 4.19-4.24 (t, J=6.3Hz, 2H), 5.22 (s, 2H), 6.97-7.00 (d, J=8.7Hz, 2H), 7.15-7.18 (d, J=8.7Hz, 2H), 7.65-7.68 (d, J=8.1Hz, 2H), 7.75-7.78 (d, J=8.4Hz, 2H), 8.56 (s, 3H)
(3) A mixture of 5-(4-chlorophenyl)-1-benzothiophene-2-carboxylic acid (144 mg, 0.50 mmol), methyl O-[4-trifluoromethyl)benzyl]tyrosinate hydrochloride (175 mg, 0.45 mmol), (3-dimethylaminopropyl)ethyl carbodiimide hydrochloride (192 mg, 1.00 mmol), 1-hydroxybenzotriazole (135 mg, 1.00 mmol), triethylamine (300 µL, 2.1 mmol), N,N-dimethylformamide (2 mL) and dichloromethane (4 mL) was stirred overnight at room temperature. Water was added to the reaction solution, which was then extracted with ethyl acetate. The extract was washed with water and saturated sodium chloride solution and then dried over anhydrous magnesium sulfate. The drying agent was removed by filtration, the solvent was evaporated under reduced pressure, and the residue was purified by silica gel column chromatography (eluate: ethyl acetate/hexane = 1/10-1/1) and recrystallized from a mixed ethyl acetate-hexane solvent to obtain methyl N-{[5-(4-chlorophenyl)-1-benzothiophene-2-yl]carbonyl}-O-[4-(trifluoromethyl)benzyl]tyrosinate (252 mg, 90%) as a white solid.
   LC-MS 624 [M+H]⁺
   ¹H-NMR (300MHz, DMSO-d₆); δ 3.01-3.17 (m, 2H), 3.66 (s, 3H), 4.60-4.68 (m, 1H), 5.17 (s, 2H), 6.92-6.95 (d, J=8.4Hz, 2H), 7.24-7.27 (d, J=8.7Hz, 2H), 7.52-7.64 (m, 4H), 7.71-7.82 (m, 5H), 8.07-8.13 (m, 2H), 8.21 (s, 1H), 8.26 (s, 1H), 9.21-9.24 (d, J=8.1 Hz, 1H)
(4) 1 M Sodium hydroxide aqueous solution (810 µL) was added to a tetrahydrofuran-methanol mixed solution (1 mL-1 mL) of methyl N-{[5-(4-chlorophenyl)-1-benzothiophene-2-yl]carbonyl }-O-[4-(trifluoromethyl)benzyl]tyrosinate (252 mg, 0.41 mmol), and stirred for 1 hour at 60°C. The reaction solution was neutralized with 1 N hydrochloric acid (850 µL), extracted twice with ethyl acetate, washed with saturated sodium chloride solution and dried over magnesium sulfate. After removal of the drying agent by filtration, this was recrystallized from an ethyl acetate-hexane mixed solution to obtain the title compound (195 mg, 79%) as colorless crystals.
   LC-MS 610 [M+H]⁺
   ¹H-NMR (300MHz, DMSO-d₆); δ 2.98-3.19 (m, 2H), 4.53-4.61 (m, 1H), 5.15 (s, 2H), 6.91-6.94(d, J=8.7Hz, 2H), 7.25-7.28 (d, J=8.7Hz, 2H), 7.54-7.64 (m, 4H), 7.71-7.82 (m, 5H), 8.09-8.11 (d, J=8.4Hz, 1H), 8.20-8.26 (m, 2H), 9.06-9.09 (d, J=8.1Hz, 1H), 12.80(br s, 1H)
   Melting point: 198°C
   Elemental analysis: Calcd. for C₃₂H₂₃NO₄SClF₃: C 63.00, H 3.80, N 2.30; Found: C 62.98, H 3.78, N 2.32

### Example C139

### O-Benzyl-N-{[5-(phenylethynyl)-1-benzothiophene-2-yl]carbonyl}tyrosine

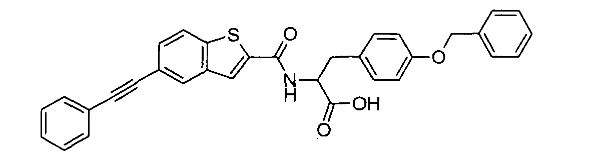

1) A mixture of ethyl 5-bromo-2-benzothiophene-2-carboxylate (1.0 g, 3.51 mmol), N,N-dimethylformamide (10 ml), CuI (67 mg, 0.35 mmol), tetrakis(triphenylphosphine)palladium (0) (0.24 g, 0.21 mmol), ethynyl benzene (0.89 ml, 8.10 mmol) and diethylamine (4.88 g, 66.7 mmol) was heated for 6 minutes at 120°C by a microwave reactor. The reaction solution was diluted with ethyl acetate, washed with water, and dried over anhydrous magnesium sulfate. The solvent was evaporated under reduced pressure, and the residue was purified by silica gel column chromatography (eluate: 10-50% ethyl acetate hexane) to obtain ethyl 5-(phenylethynyl)-1-benzothiophene-2-carboxylate (1.01 g, 94%) as a brown powder.
   ¹H-NMR (300MHz, CDCl₃) δ: 1.43 (3H, t, J=7.2Hz), 4.42 (2H, q, J=7.2Hz), 7.35-7.39 (3H, m), 7.54-7.61 (3H, m), 7.83 (1H, d, J=8.7Hz), 8.04 (2H, d, J=6.6Hz)
   LC-MS 307 [M+H]⁺
2) 5-(Phenylethynyl)-1-benzothiophene-2-carboxylic acid (0.25 g, 93%) was obtained as a pale brown powder by methods similar to those of Reference Example 17(3) from ethyl 5-(phenylethynyl)-1-benzothiophene-2-carboxylate (0.30 g, 0.98 mmol).
   ¹H-NMR (300MHz, DMSO-d₆)δ: 7.44-7.46 (3H, m), 7.57-7.67 (3H, m), 8.10-8.14 (2H, m), 8.24 (1H, s), 13.61 (1H, br s)
   LC-MS 279 [M+H]⁺
3) A mixture of 5-(phenylethynyl)-1-benzothiophene-2-carboxylic acid (0.18 g, 0.65 mmol), N,N-dimethylformamide (5 ml), methyl O-benzyltyrosinate hydrochloride (0.24 g, 0.72 mmol), triethylamine (0.18 ml, 1.30 mmol), (3-dimethylaminopropyl)ethyl carbodiimide hydrochloride (0.14 g, 0.72 mmol) and 1-hydroxybenzotriazole (46 mg, 0.33 mmol) was stirred for 16 hours at room temperature. The reaction solution was diluted with ethyl acetate, washed with water, and dried over anhydrous magnesium sulfate. The solvent was evaporated under reduced pressure, and the residue was purified by silica gel column chromatography (eluate: 10-30% ethyl acetate hexane) to obtain methyl O-benzyl-N-{[5-(phenylethynyl)-1-benzothiophene-2-yl]carbonyl}tyrosinate (0.29 g, 83%) as an oil.
   ¹H-NMR (300MHz, CDCl₃)δ: 3.16-3.29 (2H, m), 3.79 (3H, s), 5.02-5.08 (3H, m), 6.55 (1H, d, J=7.5Hz), 6.92 (2H, d, J=8.7Hz), 7.06 (2H, d, J=8.7Hz), 7.30-7.44 (9H, m), 7.54-7.61 (3H, m), 7.68 (1H, s), 7.83 (1H, d, J=8.7Hz), 8.01 (1H, s)
   LC-MS 546 [M+H]⁺
4) The title compound (0.18 g, 75%) was obtained as colorless crystals by methods similar to those of Example C 122(2) from methyl O-benzyl-N-{[5-(phenylethynyl)-1-benzothiophene-2-yl]carbonyl}tyrosinate (0.25 g, 0.46 mmol).
   ¹H-NMR (300MHz, DMSO-d₆)δ: 2.97-3.18 (2H, m); 4.53-4.61 (1H, m), 5.03 (2H, s), 6.91 (2H, d, J=8.7Hz), 7.24 (2H, d, J=8.7Hz), 7.27-7.49 (8H, m), 7.57-7.62 (3H, m), 8.08 (1H, d, J=8.4Hz), 12.85 (1H, br s)
   LC-MS 532 [M+H]⁺
   Melting point: 198-199°C
   Elemental analysis: Calcd. for C₃₃H₂₅NO₄S: C 74.56, H 4.26, N 2.63; Found: C 74.48, H 4.82, N 2.64

### Example C140

### O-(4-Methylbenzyl)-N-{ [5-(phenylethynyl)-1-benzothiophene-2-yl]carbonyl }tyrosine

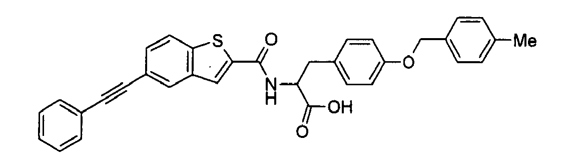

1) Methyl O-(4-methylbenzyl)-N-{[5-(phenylethynyl)-1-benzothiophene-2-yl]carbonyl}tyrosinate (0.28 g, 82%) was obtained as a white powder by methods similar to those of Example 139(3) from 5-(phenylethynyl)-1-benzothiophene-2-carboxylic acid (0.17 g, 0.61 mmol) and methyl O-(4-methylbenzyl)tyrosinate hydrochloride (0.23 g, 0.67 mmol).
   ¹H-NMR (300MHz, CDCl₃)δ: 2.36 (3H, s), 3.15-3.28 (2H, m), 3.79 (3H, s), 5.00-5.08 (3H, m), 6.55 (1H, d, J=7.5Hz), 6.91 (2H, d, J=8.7Hz), 7.05 (2H, d, J=8.7Hz), 7.18 (2H, d, J=8.7Hz), 7.30 (2H, d, J=7.8Hz), 7.34-7.41 (3H, m), 7.54-7.61 (3H, m), 7.68 (1H, s), 7.83 (1H, d, H=8.4Hz), 8.01 (1H, s)
   LC-MS 560 [M+H]⁺
2) The title compound (0.15 g, 71%) was obtained as colorless crystals by methods similar to those of Example C122(2) from methyl O-(4-methylbenzyl)-N-{[5-(phenylethynyl)-1-benzothiophene-2-yl]carbonyl}tyrosinate (0.20 g, 0.38 mmol).
   ¹H-NMR (300MHz, DMSO-d₆)δ: 2.96-3.17 (2H, m), 3.31 (3H, s), 4.52-4.60 (1H, m), 4.97 (2H, s), 6.89 (2H, d, J=8.4Hz), 7.15 (2H, d, J=7.8Hz), 7.23 (2H, d, J=8.4Hz), 7.28 (2H, d, J=8.1Hz), 7.44-7.46 (3H, m), 7.58-7.61 (3H, m), 8.08 (1H, d, J=8.4Hz), 8.18 (2H, d, J=11.4Hz), 9.09 (1H, d,J=8.1Hz), 12.85 (1H, br s)
   LC-MS 546 [M+H]⁺
   Melting point: 204-207°C
   Elemental analysis: Calcd. for C₃₄H₂₇NO₄S: C 74.84, H 4.89, N 2.57; Found: C 74.70, H 5.00, N 2.43

### Example C141

### O-(4-Chlorolbenzyl)-N-{[5-(phenylethynyl)-1-benzothiophene-2-yl]carbonyl}tyrosine

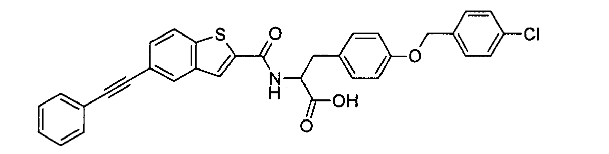

(1) Methyl O-(4-chlorobenzyl)-N-{ [5-(phenylethynyl)-1-benzothiophene-2-yl]carbonyl}tyrosinate (0.18 g, 58%) was obtained as a white powder by methods similar to those of Example 139(3) from 5-(phenylethynyl)-1-benzothiophene-2-carboxylic acid (0.15 g, 0.54 mmol) and methyl O-(4-chlorobenzyl)tyrosinate hydrochloride (0.23 g, 0.65 mmol).
   ¹H-NMR (300MHz, CDCl₃)δ: 3.15-3.29 (2H, m), 3.79 (3H, s), 5.00-5.08 (3H, m), 6.55 (1H, d, J=7.5Hz), 6.89 (2H, d, J=8.7Hz), 7.06 (2H, d, J=8.7Hz), 7.36 (7H, m), 7.53-7.60 (3H, m), 7.69 (1H, s), 7.83 (1H, d, J=8.4Hz), 8.01 (1H, s)
   LC-MS 580 [M]⁺
(2) The title compound (0.12 g, 80%) was obtained as colorless crystals by methods similar to those of Example C122(2) from methyl O-(4-chlorobenzyl)-N-{[5-(phenylethynyl)-1-benzothiophene-2-yl]carbonyl}tyrosinate (0.15 g, 0.26 mmol).
   ¹H-NMR (300MHz, DMSO-d₆)δ: 2.97-3.18 (2H, m), 4.52-4.60 (1H, m), 5.03 (2H, s), 6.90 (2H, d, J=8.4Hz), 7.24 (2H, d, J=8.4Hz), 7.39-7.49 (7H, m), 7.57-7.61 (3H, m), 8.08 (1H, d, J=8.7Hz), 8.17 (1H, s), 8.21 (1H, s), 9.08 (1H, d, J=8.4Hz), 12.9 (1H, br s)
   LC-MS 567 [M+H]⁺
   Melting point: 216-221°C
   Elemental analysis: Calcd. for C₃₃H₂₄NO₄SCl: C 70.02, H 4.27, N 2.47; Found: C 70.03, H 4.33, N 2.39

### Example C142

### O-Benzyl-N-{[5-(4-chlorophenyl)-3-methyl1-benzofuran-2-yl]carbonyl}tyrosine

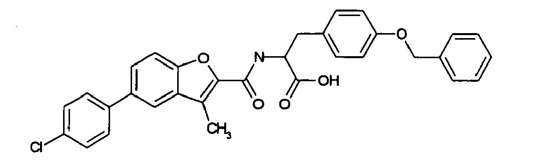

(1) Methyl O-benzyl-N-{[5-(4-chlorophenyl)-3-methyl 1-benzofuran-2-yl]carbonyl}tyrosinate (463 mg) was obtained as colorless crystals by operations similar to those of Example C122(1) from 5-(4-chlorophenyl)-3-methyl-1-benzofuran-2-carboxylic acid (287 mg) and methyl O-benzyltyrosinate hydrochloride (340 mg).
   ¹H-NMR (300MHz, CDCl₃); δ 2.65 (3H, s), 3.21 (2H, d, J=6.3Hz), 3.77 (3H, s), 5.02-5.10 (1H, m), 5.04 (2H, s), 6.92 (2H, d, J=8.7Hz), 7.03-7.13 (3H, m), 7.28-7.62 (11H, m), 7.73 (1H, d, J=1. 2Hz)
(2) The title compound (172 mg) was obtained as colorless crystals by methods similar to those of Example C122(2) from the compound (252 mg) obtained in (1).
   Melting point: 209-210°C (tetrahydrofuran-diethyl ether)
   ¹H-NMR (300MHz, DMSO-d₆); δ 2.53 (3H, s), 3.05-3.20 (2H, m), 4.55-4.67 (1H, m), 5.03 (2H, s), 6.91 (2H, d, J=8.7Hz), 7.21 (2H, d, J=8.7Hz), 7.25-7.44 (5H, m), 7.54 (2H, d, J=8.7Hz), 7.70 (1H, d, J=8.7Hz), 7.78 (1H, d, J=8.7Hz), 7.79 (2H, d, J=8.7Hz), 8.02 (1H, d, J=1.8Hz), 8.56 (1H, d, J=8.1Hz), 12.58 (1H,br s)
   Elemental analysis: Calcd. for C₃₂H₂₆ClNO₅: C 71.17, H 4.85, N 2.59; Found: C 71.09, H 4.71, N 2.41

### Example C143

### O-Benzyl-N-{[6-(4-chlorophenyl)-1-benzofuran-2-yl]carbonyl}tyrosine

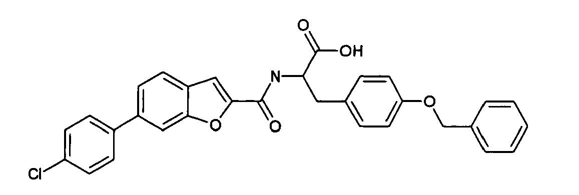

(1) Methyl O-benzyl-N-{[6-(4-chlorophenyl)-1-benzofuran-2-yl]carbonyl}tyrosinate (434 mg) was obtained as colorless crystals by operations similar to those of Example C122(1) from 6-(4-chlorophenyl)-1-benzofuran-2-carboxylic acid (273 mg) and methyl O-benzyltyrosinate hydrochloride (340 mg).
   ¹H-NMR (300MHz, CDCl₃); δ 3.15-3.29 (2H, m), 3.78 (3H, s), 5.03 (2H, s), 5.03-5.12 (1H, m), 6.91 (2H, d, J=8.7Hz), 7.03-7.11 (3H, m), 7.30-7.60 (11H, m), 7.67 (1H, s), 7.72 (1H, d, J=8.4Hz)
(2) The title compound (227 mg) was obtained as colorless crystals by methods similar to those of Example C122(2) from the compound (284 mg) obtained in (1).
   Melting point: 170-171°C (ethyl acetate-hexane)
   ¹H-NMR (300MHz, DMSO-d₆); δ 3.00-3.20 (2H, m), 4.55-4.65 (1H, m), 5.02 (2H, s), 6.91 (2H, d, J=8.7Hz), 7.22 (2H, d, J=8.7Hz), 7.25-7.44 (5H, m), 7.55 (2H, d, J=8.4Hz), 7.61 (1H, d, J=0.6Hz), 7.66 (1H, dd, J=1.5, 8.4Hz), 7.80 (2H, d, J=8.4Hz), 7.86 (1H, d, J=8.4Hz), 7.96 (1H, s), 8.84 (1H, d, J=8.4Hz), 12.90 (1H, br s)
   Elemental analysis: Calcd. for C₃₁H₂₄ClNO₅: c 69.09, H 4.76, N 2.60; Found: C 69.09, H 4.65, N 2.60

### Example C144

### O-Benzyl-N-({6-[(E)-2-phenylethenyl]-1H-benzimidazol-2-yl}carbonyl)tyrosine

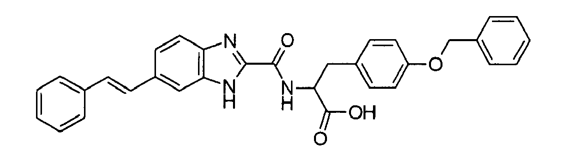

(1) A mixture of 5-bromo-2-fluoronitrobenzene (2.46 g, 11.2 mmol), styrene (1.92 ml, 16.7 mmol), acetonitrile (11 ml), palladium acetate (24 mg, 0.11 mmol), tris(2-methylphenyl)phosphine (0.13 g, 0.43 mmol) and triethylamine (1.56 ml, 11.2 mmol) was heated for 5 minutes at 150°C by a microwave reactor. The reaction solution was diluted with ethyl acetate, washed with water, and dried over anhydrous magnesium sulfate. The solvent was evaporated under reduced pressure to obtain 1-fluoro-2-nitro-4-[(E)-2-phenylethenyl]benzene (0.68 g, 25%) as a pale brown powder.
   ¹H-NMR (300MHz, CDCl₃)δ: 7.04(1H, d, J=16Hz), 7.15 (1H, d, J=16Hz), 7.25-7.42 (4H, m), 7.50-7.54 (2H, m), 7.71-7.76 (1H, m), 8.18 (1H, dd, J=2.1, 6.9Hz)
(2) A mixture of 1-fluoro-2-nitro-4-[(E)-2-phenylethenyl]benzene (1.53 g, 6.30 mmol), N,N-dimethylformaldehyde (10 ml), ammonium chloride (1.01 g, 18.9 mmol) and triethylamine (3.51 ml, 25.2 mmol) was heated for 20 minutes at 150°C by a microwave synthesizer. The reaction solution was diluted with ethyl acetate, washed with water, and dried over anhydrous magnesium sulfate. The solvent was evaporated under reduced pressure to obtain 1-amino-2-nitro-4-[(E)-2-phenylethenyl]benzene (1.13 g, 75%) as a yellow powder.
   ¹H-NMR (300MHz, CDCl₃)δ: 6.14 (2H, br s), 6.83 (1H, d, J=8.7Hz), 7.00(2H, s), 7.24-7.29 (1H, m), 7.34-7.39 (2H, m), 7.47-7.50 (2H, m), 7.60 (1H, dd, J=2.1, 8.7Hz), 8.22 (1H, d, J=2.1Hz)
   LC-MS 241 [M+H]⁺
(3) 4-[(E)-2-Phenylethenyl]benzene-1,2-diamine (0.57 g, 66%) was obtained as a reddish-brown powder by methods similar to those of Example C167(3) from 1-amino-2-nitro-4-[(E)-2-phenylethenyl]benzene (1.00 g, 4.16 mmol).
   ¹H-NMR (300MHz, CDCl₃)δ: 4.49 (2H, s), 4.70 (2H, s), 6.49 (1H, d, J=8.1Hz), 6.64 (1H, dd, J=1.8, 7.8Hz), 6.75-6.81 (2H, m), 6.97 (1H, d, J=16Hz), 7.14-7.19 (1H, m), 7.31 (2H, t, J=7.8Hz), 7.48 (2H, d, J=7.5Hz)
   LC-MS 211 [M+H]⁺
(4) Methyl O-benzyl-N-{[6-(2-phenylethenyl)-1H-benzimidazol-2-yl]carbonyl}tyrosinate (0.19 g, 48%) was obtained as a white powder by methods similar to those of Example C167(4) and (5) from 4-[(E)-2-phenylethenyl]benzene-1,2-diamine (0.19 g, 1.10 mmol).
   ¹H-NMR (300MHz, CDCl₃)δ: 3.14-3.29 (2H, m), 3.75 (3H, s), 5.00-5.09 (3H, m), 6.90 (2H, d, J=8.1Hz), 7.10-7.43 (9H, m), 7.49-7.63 (6H, m), 7.78 (1H, d, J=8.4Hz), 7.90-7.96 (2H, m), 10.64 (1H, s)
   LC-MS 532 [M+H]⁺
(5) The title compound (0.13 g, 81%) was obtained as pale brown crystals by methods similar to those of Example C167(6) from methyl O-benzyl-N-{[6-[(E)-2-phenylethenyl]-1H-benzimidazol-2-yl]carbonyl}tyrosinate (0.17 g, 0.32 mmol).
   ¹H-NMR (300MHz, DMSO-d₆)δ: 3.17 (2H, d, J=6.3Hz), 4.65-4.73 (1H, m), 5.02 (2H, s), 6.91 (2H, d, J=8.4Hz), 7.19-7. 53 (12.5H, m), 7.61-7. 74 (4H, m), 7.91 (0.5H, s), 8.79-8.84 (1H, m), 12.97 (1R, br s), 13.30 (1H, br s)
   LC-MS 518 [M+H]⁺
   Melting point: 247°C (decomposed)
   Elemental analysis: Calcd. for C₃₂H₂₇N₃O₄: C 74.26, H 5.26, N 8.12; Found: C 74.15, H 5.22, N 8.27

### Example C145

### O-Benzyl-N-{[7-(4-chlorophenyl)-2H-thiochromene-3-yl]carbonyl}tyrosine

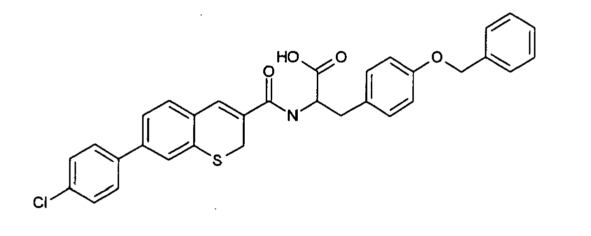

(1) A mixture of 4-bromo-2-fluorobenzaldehyde (10.19 g), methyl 3-mercaptopropionate (6.65 mL), potassium carbonate (8.29 g) and N,N-dimethylformamide (100 mL) was stirred for 16 hours at room temperature. Water was added to the reaction solution, which was then extracted with diethyl ether. The extract was washed with saturated sodium chloride solution and then dried over anhydrous magnesium sulfate. The solvent was evaporated under reduced pressure, and the residue was purified by silica gel column chromatography to obtain methyl 3-[(5-bromo-2-formylphenyl)thio]propionate (11.2 g) as pale yellow crystals.
   ¹H-NMR (300MHz, CDCl₃); δ 2.72 (2H, t, J=7.5Hz), 3.24 (2H, t, J=7.5Hz), 3.72 (3H, s), 7.47 (1H, dd, J=1.8, 8.1Hz), 7.56 (1H, d, J=1.8Hz), 7.69 (1H, d, J=8.1Hz), 10.27 (1H, s)
(2) tert-Butoxy potassium (1.55 g) was added to a tert-butanol solution (200 mL) of the compound (13.98 g) obtained in (1), and refluxed for 30 minutes. The reaction solution was concentrated under reduced pressure, and water was added to the residue, which was then extracted with ethyl acetate. The extract was washed with saturated sodium chloride solution and then dried over anhydrous magnesium sulfate. The solvent was evaporated under reduced pressure, and the residue was purified by silica gel column chromatography to obtain methyl 7-bromo-2H-thiochromene-3-carboxylate (3.25 g) as pale yellow crystals.
   ¹H-NMR (300MHz, CDCl₃); δ 3.72 (2H, d, J=1.2Hz), 3.84 (3H, s), 7.07 (1H, d, J=8.1Hz), 7.25 (1H, dd, J=1.8, 8.1Hz), 7.42 (1H, d, J=1.8Hz), 7.49 (1H, s)
(3) A mixture of the compound obtained in (2) (2.37 g), 4-chlorophenyl boronic acid (1.56 g), tetrakis(triphenylphosphine)palladium (0) (481 mg), 2 M sodium carbonate aqueous solution (8.3 mL) and 1,2-dimethoxyethane (50 mL) was stirred for 14 hours at 80°C. Water was added to the reaction solution, which was then extracted with ethyl acetate. The extract was washed with saturated sodium chloride solution and then dried over anhydrous magnesium sulfate. The solvent was evaporated under reduced pressure, and the residue was purified by basic silica gel column chromatography to obtain methyl 7-(4-chlorophenyl)-2H-thiochromene-3-carboxylate (2.39 g) as pale yellow crystals.
   ¹H-NMR (300MHz, CDCl₃); δ 3.78 (2H, d, J=1.2Hz), 3.86 (3H, s), 7.28-7.33 (2H, m), 7.41 (2H, d, J=8.7Hz), 7.45-7.48 (1H, m), 7.51 (2H, d, J=8.7Hz), 7.59 (1H, s)
(4) 1 N Sodium hydroxide aqueous solution (5 mL) and water (15 mL) were added at 60°C to a tetrahydrofuran-methanol mixed solution (20 mL-10 mL) of the compound (2.39 g) obtained in (3), and stirred for 30 minutes at the same temperature. The reaction solution was neutralized with 2 N hydrochloric acid, and extracted with ethyl acetate-tetrahydrofuran. The extract was washed with saturated sodium chloride solution and then dried over anhydrous magnesium sulfate. The solvent was evaporated under reduced pressure, and the precipitated crystals were filtered out and washed with diethyl ether-hexane to obtain 7-(4-chlorophenyl)-2H-thiochromene-3-carboxylic acid (2.04 g) as yellow crystals.
   ¹H-NMR (300MHz, DMSO-d₆); δ 3.73 (2H, d, J=0.6Hz), 7.46-7.63 (6H, m), 7.75 (2H, d, J=8.7Hz), 12.90 (1H, br s)
(5) Methyl O-benzyl-N-{[7-(4-chlorophenyl)-2H-thiochromene-3-yl]carbonyl}tyrosinate (863 mg) was obtained as pale yellow crystals by operations similar to those of Example C122(1) from the compound (606 mg) obtained in (4) and methyl O-benzyl tyrosinate hydrochloride (680 mg).
   ¹H-NMR (300MHz, CDCl₃); δ 3.10-3.25 (2H, m), 3.67 (1H, dd, J=0.6, 15.3Hz), 3.76 (1H, dd, J=0.6, 15.3Hz), 3.78 (3H, s), 4.92-5.00 (1H, m), 5.05 (2H, s), 6.34 (1H, d, J=7.5Hz), 6.92 (2H, d, J=8.7Hz), 7.01 (1H, s), 7.05 (2H, d, J=8.7Hz), 7.21 (1H, d, J=7.8Hz), 7.28-7.47 (9H, m), 7.51 (2H, d, J=8.7 Hz)
(6) The title compound (204 mg) was obtained as pale yellow crystals by operations similar to those of Example C 122(2) from the compound (285 mg) obtained in (5).
   Melting point: 225-227°C (tetrahydrofuran-diethyl ether)
   ¹H-NMR (300MHz, DMSO-d₆); δ 2.95 (1H, dd, J=10.2, 13.8Hz), 3.09 (1H, dd, J=1.5, 13.8Hz), 3.62 (1H, d, J=15.6Hz), 3.70 (1H, d, J=15.6Hz), 4.40-4.50 (1H, m), 5.05 (2H, s), 6.93 (2H, d, J=8.7Hz), 7.22 (2H, d, J=8.7Hz), 7.27-7.55 (10H, m), 7.61 (1H, d, J=1.5Hz), 7.74 (2H, d, J=8.7Hz), 8.46 (1H, d, J=8.1Hz), 12.80 (1H, br s)
   Elemental analysis: Calcd. for C₃₂H₂₆ClNO₄S•0.25 H₂O C 68.56, H 4.76, N 2.50; Found: C 68.59, H 4.71, N 2.37

### Example C146

### O-Benzyl-N-{[6-(4-chlorophenyl)-2-oxo-1,2-dihydroquinolin-3-yl]carbonyl}tyrosine

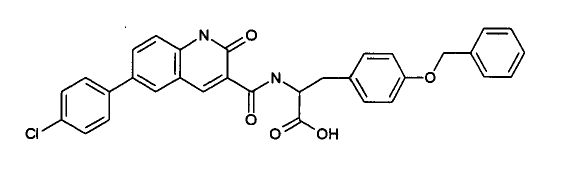

(1) Trifluoromethanesulfonic anhydride (7.67 mL) was added with ice cooling to a pyridine-dichloromethane solution (10 mL-50 mL) of 5-hydroxy-2-nitrobenzaldehyde (5.0 g), and stirred for 30 minutes at room temperature. Water was added to the reaction solution, which was then extracted with ethyl acetate. The extract was washed successively with 1 N hydrochloric acid and saturated sodium chloride solution, dried over anhydrous magnesium sulfate, and passed through silica gel. The solvent was evaporated under reduced pressure to obtain 3-formyl-4-nitrophenyl methanesulfonate (7.27 g) as a brown oil. A mixture of the resulting 3-formyl-4-nitrophenyl methanesulfonate (7.27 g), 4-chlorophenylboronic acid (4.56 g), tetrakis(triphenylphosphine)palladium (0) (1.40 g), 2 M sodium carbonate aqueous solution (25 mL) and 1,2-dimethoxyethane (100 mL) was stirred for 1 hour at 80°C. Water was added to the reaction solution, which was then extracted with ethyl acetate. The extract was washed with saturated sodium chloride solution and then dried over anhydrous magnesium sulfate. The solvent was evaporated under reduced pressure, and the residue was purified by silica gel column chromatography and recrystallized from ethyl acetate-hexane to obtain 4'-chloro-4-nitrobiphenyl-3-carbaldehyde (3.98 g) as pale yellow crystals.
   ¹H-NMR (300MHz, CDCl₃); δ 7.50 (2H, d, J=8.7Hz), 7.60 (2H, d, J=8.7Hz), 7.90 (1H, dd, J=2.4, 8.4Hz), 8.11 (1H, d, J=2.4Hz), 8.23 (1H, d, J=8.4Hz), 10.52 (1H, s)
(2) A carbon tetrachloride solution (7 mL) of titanium tetrachloride (3.35 mL) was added with ice cooling to tetrahydrofuran (120 mL). Next, dimethyl maronate (2.21 g) and a tetrahydrofuran solution (8 mL) of the compound (3.98 g) obtained in (1) were added with ice cooling. A tetrahydrofuran solution (15 mL) of pyridine (4.81 g) was then added gradually with ice cooling, and the solution was warmed gradually to room temperature and stirred for 15 hours. Water was added to the reaction solution, which was then extracted with diethyl ether. The extract was washed successively with water, saturated sodium chloride solution, saturated sodium hydrogencarbonate aqueous solution and saturated sodium chloride solution, and dried over anhydrous magnesium sulfate. The solvent was evaporated under reduced pressure and the residue was recrystallized from ethyl acetate-hexane to obtain dimethyl [(4'-chloro-4-nitrobiphenyl-3-yl)methylene]malonate (4.92 g) as yellow crystals.
   ¹H-NMR (300MHz, CDCl₃); δ 3.61 (3H, s), 3.90 (3H, s), 7.48 (2H, d, J=8.7Hz), 7.53 (2H, d, J=8.7Hz), 7.61 (1H, d, J=1.8Hz), 7.73 (1H, dd, J=1.8, 8.7Hz), 8.28 (1H, s), 8.30 (1H, s)
(3) Iron (7.31 g) was added at 80°C to an acetic acid solution (100 mL) of the compound (4.92 g) obtained in (2), and stirred for 15 hours at the same temperature. The reaction solution was cooled to room temperature and Celite filtered, and the filtrate was concentrated under reduced pressure. The residue was filtered and washed with ethyl acetate-hexane to obtain methyl 6-(4-chlorophenyl)-2-oxo-1,2-dihydroquinoline-3-carboxylate (660 mg) as pale yellow crystals.
   ¹H-NMR (300MHz, DMSO-d₆); δ 3.82 (3H, s), 7.40 (1H, d, J=8.7Hz), 7.55 (2H, d, J=8.7Hz), 7.74 (2H, d, J=8.7Hz), 7.96 (1H, dd, J=1.8, 8.7Hz), 8.18 (1H, d, J=1.8Hz), 8.58 (1H, s), 12.17 (1H, br s)
(4) A mixture of the compound (215 mg) obtained in (3), lithium hydroxide monohydrate (200 mg), tetrahydrofuran (10 mL), methanol (10 mL) and water (10 mL) was stirred for 48 hours at 70°C. The reaction solution was neutralized with 1 N hydrochloric acid, and the precipitated crystals were filtered out and washed successively with water, methanol and diethyl ether to obtain 6-(4-chlorophenyl)-2-oxo-1,2-dihydroquinoline-3-carboxylic acid (202 mg) as yellow crystals.
   ¹H-NMR (300MHz, DMSO-d₆); δ 7.57 (2H, d, J=8.7Hz), 7.60 (1H, d, J=8.7Hz), 7.78 (2H, d, J=8.7Hz), 8.09 (1H, d, J=2.1, 8.7Hz), 8.38 (1H, d, J=2.1Hz), 8.97 (1H, s)
(5) A mixture of the compound obtained in (4) (100 mg), methyl O-benzyltyrosinate hydrochloride (107 mg), (3-dimethylaminopropyl)ethyl carbodiimide hydrochloride (77 mg), 1-hydroxybenzotriazole (54 mg), triethylamine (0.14 mL) and N,N-dimethylformamide (5 mL) was stirred for 20 hours at room temperature and then for 5 hours at 60°C. Water was added to the reaction solution, which was then extracted with ethyl acetate-tetrahydrofuran. The extract was washed with saturated sodium chloride solution and then dried over anhydrous magnesium sulfate. The solvent was evaporated under reduced pressure, and the residue was purified by silica gel column chromatography to obtain methyl O-benzyl-N-{[6-(4-chlorophenyl)-2-oxo-1,2-dihydroquinoline-3-yl]carbonyl}tyrosinate (96 mg) as pale yellow crystals.
   ¹H-NMR (300MHz, CDCl₃); δ 3.18-3.30 (2H, m), 3.77 (3H, s), 4.78 (1H, d, J=11.7Hz), 4.87 (1H, d, J=11.7Hz), 5.10-5.17 (1H, m), 6.89 (2H, d, J=8.7Hz), 7.18 (2H, d, J=8.7Hz), 7.20-7.35 (6H, m), 7.41 (2H, d, J=8.7Hz), 7.50 (2H, d, J=8.7Hz), 7.76 (1H, dd, J=1.8, 8.7Hz), 7.87 (1H, d, J=1.8Hz), 9.02 (1H, s), 10.35 (1H, d, J=7.5Hz), 12.09 (1R, br s)
(6) 1 N Sodium hydroxide aqueous solution (1 mL) and water (10 mL) were added at 60°C to a tetrahydrofuran-methanol mixed solution (5 mL-5 mL) of the compound (90 mg) obtained in (5), and stirred for 1 hour at the same temperature. The reaction solution was neutralized with 1 N hydrochloric acid, and extracted with ethyl acetate-tetrahydrofuran. The extracted was washed with saturated sodium chloride solution, and dried over anhydrous magnesium sulfate. The solvent was evaporated under reduced pressure, and the precipitated crystals were filtered out and washed with methanol-diethyl ether to obtain the title compound (50 mg) as yellow crystals.
   Melting point: 298-299°C
   ¹H-NMR (300MHz, DMSO-d₆); δ 3.02 (1H, dd, J=7.2, 13.8Hz), 3.14 (1H, dd, J=5.4, 13.8Hz), 4.68-4.78 (1H, m), 5.04 (2H, s), 6.92 (2H, d, J=8.7Hz), 7.15 (2H, d, J=8.7Hz), 7.27-7.46 (5H, m), 7.51 (1H, d, J=8.7Hz), 7.55 (2H, d, J=8.7Hz), 7.77 (2H, d, J=8.7Hz), 8.01 (1H, dd, J=2.1, 8.7Hz), 8.31 (1H, d, J=2.1Hz), 8.90 (1H, s), 10.14 (1H, d, J=7.5Hz), 12.54 (1H, s), 12.96 (1H, br s) Elemental analysis: Calcd. for C₃₂H₂₅ClN₂O₅: C 69.50, H 4.56, N 5.07; Found: C 69.23, H 4.63, N 5.16

### Example C147

### N-{[3-Amino-6-(4-chlorophenyl)-1-benzofuran-2-yl]carbonyl}-O-benzyltyrosine

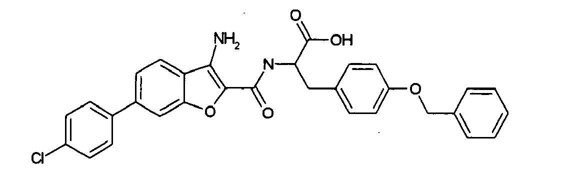

(1) A tetrahydrofuran (10 mL) solution of ethyl 3-amino-6-(4-chlorophenyl)-1-benzofuran-2-carboxylate (316 mg), di-tert-butyl bicarbonate (546 mg) and 4-dimethylaminopyridine (10 mg) was refluxed for 2 hours. Ethanol (10 mL), 1 N sodium hydroxide aqueous solution (5 mL) and water (5 mL) were added to the reaction solution, which was then stirred for 1 hour at 60°C. Water was added to the reaction solution, and the aqueous layer was washed with diethyl ether, made acidic with 1 N hydrochloric acid and then extracted with diethyl ether. The extract was washed with saturated sodium chloride solution and then dried over anhydrous magnesium sulfate, and the solvent was evaporated under reduced pressure to obtain 3-[bis(tert-butoxycarbonyl)amino]-6-(4-chlorophenyl)-1-benzofuran-2-carboxylic acid (380 mg) as pale brown crystals.
   ¹H-NMR (300MHz, CDCl₃); δ 1.44 (18H, s), 7.46 (2H, d, J=8.7Hz), 7.55-7.62 (4H, m), 7.75 (1H, s)
(2) A mixture of the compound (490 mg) obtained in (1), methyl O-benzyltyrosinate hydrochloride (340 mg), (3-dimethylaminopropyl)ethyl carbodiimide hydrochloride (230 mg), 1-hydroxybenzotriazole (162 mg), triethylamine (0.5 mL) and N,N-dimethylformamide (15 mL) was stirred overnight at room temperature. Water was added to the reaction solution, which was then extracted with diethyl ether. The extract was washed successively with sodium hydroxide aqueous solution and saturated sodium chloride solution and then dried over anhydrous magnesium sulfate. The solvent was evaporated under reduced pressure, the residue was dissolved in methanol-ethyl acetate (5 mL-5 mL), and 4 N hydrogen chloride-ethyl acetate solution (10 mL) was added and stirred for 1.5 5 hours at room temperature and 1 hour at 60°C. The reaction solution was concentrated, made basic with saturated sodium hydrogencarbonate aqueous solution, and extracted with ethyl acetate. The extract was washed with saturated sodium chloride solution and then dried over anhydrous magnesium sulfate. The solvent was evaporated under reduced pressure, and the residue was purified by silica gel column chromatography to obtain a mixture of O-benzyl-N-{[3-[bis(tert-butoxycarbonyl)amino]-6-(4-chlorophenyl)-1-benzofuran-2-yl]carbonyl}tyrosine methyl ester and ethyl ester The resulting mixture was dissolved in tetrahydrofuran-methanol (5 mL-5 mL), and 1 N sodium hydroxide aqueous solution (1 mL) and water (5 mL) were added and stirred for 1 hour at room temperature and then 30 minutes at 60°C. The reaction solution was made acidic with 1 N hydrochloric acid, and extracted with diethyl ether. The extract was washed with saturated sodium chloride solution and then dried over anhydrous magnesium sulfate. The solvent was evaporated under reduced pressure, and the residue was recrystallized from methanol and then recrystallized from ethyl acetate-hexane to obtain the title compound (78 mg) as pale brown crystals.
   Melting point: 250°C (decomposed, ethyl acetae-hexane)
   ¹H-NMR (300MHz, DMSO-d₆); δ 3.05-3.15 (2H, m), 4.55-4.65 (1H, m), 5.03 (2H, s), 6.09 (2H, br s), 6.91 (2H, d, J=8.7Hz), 7.19 (2H, d, J=8.7Hz), 7.26-7.45 (5H, m), 7.54 (2H, d, J=8.7Hz), 7.58 (1H, dd, J=1.5, 8.4Hz), 7.73 (1H, d, J=1.5Hz), 7.78 (2H, d, J=8.7Hz), 7.84 (1H, d, J=7.8Hz), 7.93 (1H, d, J=8.4Hz), 12. 70 (1H, br s)
   Elemental analysis: Calcd. for C₃₁H₂₅ClN₂O₅•0.25 H₂O: C 68.26, H 4.71, N 5.14; Found: C 68.45, H 4.62, N 5.07

### Example C148

### O-Benzyl-N-{[5-(4-chlorophenyl)-1-benzofuran-2-yl]carbonyl }tyrosine

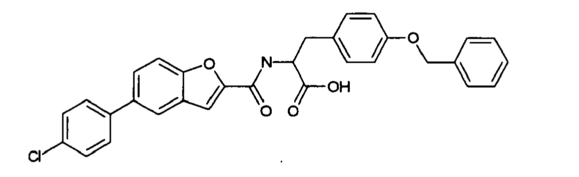

(1) Methyl O-benzyl-N-{[5-(4-chlorophenyl)-1-benzofuran-2-yl]carbonyl}tyrosinate (480 mg) was obtained as colorless crystals by operations similar to those of Example C122(1) from 5-(4-chlorophenyl)-1-benzofuran-2-carboxylic acid (273 mg) and methyl O-benzyl tyrosinate hydrochloride (340 mg).
   ¹H-NMR (300MHz, CDCl₃); δ 3.15-3.30 (2H, m), 3.78 (3H, s), 5.03 (2H, s), 5.03-5.12 (1H, m), 6.91 (2H, d, J=8.7Hz), 7.05-7.12 (3H, m), 7.30-7.46 (7H, m), 7.50-7.63 (5H, m), 7.80-7.83 (1H, m)
(2) The title compound (260 mg) was obtained as colorless crystals by operations similar to those of Example C122(2) from the compound (378 mg) obtained in (1).
   Melting point: 250-251°C (methanol-diethyl ether)
   ¹H-NMR (300MHz, DMSO-d₆); δ 3.05 (1H, dd, J=9.9, 14.1Hz), 3.16 (1H, dd, J=4.5, 14.1Hz), 4.57-4.67 (1H, m), 5.02 (2H, s), 6.91 (2H, d, J=8.4Hz), 7.22 (2H, d, J=8.4Hz), 7.26-7.44 (5H, m), 7.54 (2H, d, J=8.4Hz), 7.63 (1H, s), 7.71-7.78 (4H, m), 8.06 (1H, s), 8.91 (1H, d, J=8.1Hz), 12.58 (1H, s)
   Elemental analysis: Calcd. for C₃₁H₂₄ClNO₅: C 70.79, H 4.60, N 2.66; Found: C 70.62, H 4.49, N 2.60

### Example C149

### N-{[3-Amino-5-(4-chlorophenyl)-1-benzofuran-2-yl]carbonyl}-O-benzyltyrosine

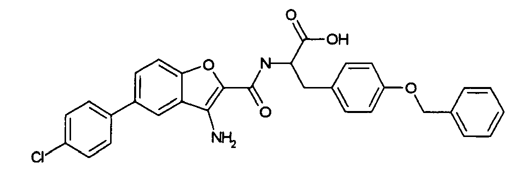

(1) Trifluoracetic acid anhydride (0.27 mL) was added to a dichloromethane solution (10 mL) of tert-butyl 3-amino-5-(4-chlorophenyl)-1-benzofuran-2-carboxylate (344 mg), and stirred for 20 hours at room temperature. The reaction solution was concentrated and extracted with diethyl ether after addition of water. The extract was washed with saturated sodium chloride solution and then dried over anhydrous magnesium sulfate. The solvent was evaporated under reduced pressure, and the residue was washed with hexane to obtain tert-butyl 5-(4-chlorophenyl)-3-[(trifluoracetyl)amino]-1-benzofuran-2-carboxylate (364 mg) as colorless crystals.
   ¹H-NMR (300MHz, CDCl₃); δ 1.69 (9H, s), 7.42 (2H, d, J=8.4Hz), 7.55-7.61 (3H, m), 7.70 (1H, dd, J=1.8, 8.7Hz), 8.58 (1H, d, J=1.8Hz), 10.45 (1H, br s)
(2) Trifluoracetic acid (5 mL) was added to a dichloromethane solution (5 mL) of the compound (364 mg) obtained in (1), and stirred for 20 hours at room temperature. The reaction solution was concentrated under reduced pressure, and the residue was washed with diethyl ether-hexane to obtain 5-(4-chlorophenyl)-3-[(trifluoracetyl)amino]-1-benzofuran-2-carboxylic acid (239 mg) as pale brown crystals.
   ¹H-NMR (300MHz, DMSO-d₆); δ 7.56 (2H, d, J=8.7Hz), 7.75 (2H, d, J=8.7Hz), 7.84 (1H, d, J=8.7Hz), 7.88 (1R, dd, J=1.8, 8.7Hz), 7.99 (1H, d, J=1.8Hz), 11.52 (1H, br s)
(3) A mixture of the compound (239 mg) obtained in (2), methyl O-benzyltyrosinate hydrochloride (212 mg), (3-dimethylaminopropyl)ethyl carbodiimide hydrochloride (144 mg), 1-hydroxybenzotriazole (102 mg), triethylamine (0.4 mL) and N,N-dimethylformamide (7 mL) was stirred overnight at room temperature. Water was added to the reaction solution, which was then extracted with ethyl acetate. The extract was washed with saturated sodium chloride solution and then dried over anhydrous magnesium sulfate. The solvent was evaporated under reduced pressure, and the residue was purified by silica gel column chromatography to obtain methyl O-benzyl-N-({5-(4-chlorophenyl)-3-[(trifluoracetyl)amino]-1-benzofuran-2-yl}carbonyl) tyrosinate (198 mg) as pale yellow crystals.
   ¹H-NMR (300MHz, CDCl₃); δ 3.15-3.30 (2H, m), 3.80 (3H, s), 5.00-5.10 (1H, m), 5.04 (2H, s), 6.92 (2H, d, J=8.7Hz), 6.99 (1H, d, J=8.1 Hz), 7.08 (2H, d, J=8.7Hz), 7.28-7.62 (10H, m), 7.69 (1H, dd, J=1.8, 8.7Hz), 8.66 (1H, d, J=1.8Hz), 11.12 (1H, s)
(4) 1 N Sodium hydroxide aqueous solution (2 mL) and water (7 mL) were added at 60°C to a tetrahydrofuran-methanol solution (10 mL-5 mL) of the compound (198 mg) obtained in (3), and stirred at that temperature for 16 hours. Water was added to the reaction solution, which was then extracted with diethyl ether. The extract was washed with saturated sodium chloride solution and then dried over anhydrous magnesium sulfate. The solvent was evaporated under reduced pressure, and the residue was recrystallized from ethyl acetate-hexane to obtain the title compound (77 mg) as pale yellow crystals.
   Melting point: 250°C (disintegrated, ethyl acetate-hexane)
   ¹H-NMR (300MHz, DMSO-d₆); δ 3.05-3.15 (2H, m), 4.55-4.65 (1H, m), 5.04 (2H, s), 6.08 (2H, br s), 6.92 (2H, d, J=8.7Hz), 7.21 (2H, d, J=8.7Hz), 7.27-7.45 (7H, m), 7.56 (2H, d, J=8.4Hz), 7.69-7.78 (3H, m), 7.94 (1H, d, J=8.1Hz), 8.22 (1H, d, J=1.8Hz), 12.58 (1H, br s)
   Elemental analysis: Calcd. for C₃₁H₂₅ClN₂O₅•1 H₂O: C 66.61, H 4.87, N 5.01; Found: C 66.87, H 4.65, N 4.93

### Example C150

### N-{[5-(4-Chlorophenyl)-3-methyl-1-benzofuran-2-yl]carbonyl}-O-(3-furylmethyl)tyrosine

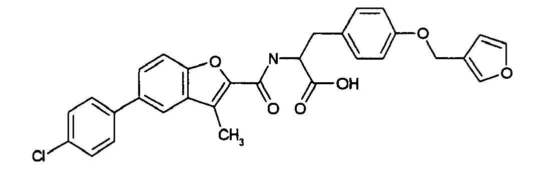

(1) Methyl N-{[5-(4-chlorophenyl)-3-methyl-1-benzofuran-2-yl]carbonyl}-O-(3-furylmethyl)tyrosinate (160 mg) was obtained as colorless crystals by operations similar to those of Example C122(1) from 5-(4-chlorophenyl)-3-methyl-1-benzofuran-2-carboxylic acid (92 mg) and methyl O-(3-furylmethyl)tyrosinate hydrochloride (100 mg).
   ¹H-NMR (300MHz, CDCl₃); δ 2.65 (3H, s), 3.15-3.30 (2H, m), 3.77 (3H, s), 4.91 (2H, s), 5.00-5.10 (1H, m), 6.47 (1H, d, J=0.9Hz), 6.90 (2H, d, J=8.7Hz), 7.07 (1H, d, J=8.1Hz), 7.10 (2H, d, J=8.7Hz), 7.40-7.63 (8H, m), 7.73 (1H, d, J=1.5Hz)
(2) The title compound (85 mg) was obtained as colorless crystals by operations similar to those of Example C122(2) from the compound (150 mg) obtained in (1).
   Melting point: 196-197°C (ethyl acetate-hexane)
   ¹H-NMR (300MHz, DMSO-d₆); δ 2.53 (3H, s), 3.10-3.20 (2H, m), 4.57-4.67 (1H, m), 4.88 (2H, s), 6.53 (1H, d, J=I.SHz), 6.90 (2H, d, J=8.7Hz), 7.20 (2H, d, J=8.7Hz), 7.54 (2H, d, J=8.7Hz), 7.63 (1H, t, J=I.SHz), 7.68-7.81 (5H, m), 8.02 (1H, d, J=1.5Hz), 8.57 (1H, d, J=7.8Hz), 12.85 (1H, br s)
   Elemental analysis: Calcd. for C₃₀H₂₄ClNO₆: C 67.99, H 4.56, N 2.64; Found: C 67.82, H 4.47, N 2.60

### Example C151

### N-{[6-(4-Chlorophenyl)-1 -benzofuran-2-yl]carbonyl}-O-(3-furylmethyl)tyrosine

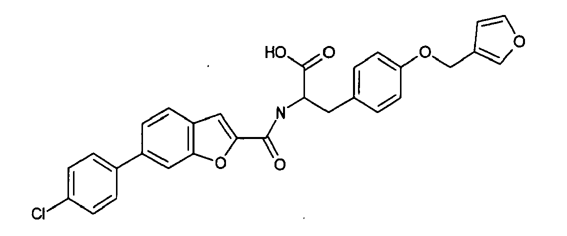

(1) Methyl N-{[6-(4-chlorophenyl)-1-benzofuran-2-yl]carbonyl}-O-(3-furylmethyl)tyrosinate (158 mg) was obtained as colorless crystals by methods similar to those of Example C122(1) from 6-(4-chlorophenyl)-1-benzofuran-2-carboxylic acid (87 mg) and methyl O-(3-furylmethyl)tyrosinate hydrochloride (100 mg).
   ¹H-NMR (300MHz, CDCl₃); δ 3.15-3.30 (2H, m), 3.78 (3H, s), 4.90 (2H, s), 5.05-5.10 (1H, m), 6.47 (1H, s), 6.89 (2H, d, J=8.7Hz), 7.40-7.11 (3H, m), 7.40-7. 59 (8H, m), 7.67 (1H, s), 7.72 (1H, d, J=8.1 Hz)
(2) The title compound (52 mg) was obtained as colorless crystals by methods similar to those of Example C122(2) from the compound (138 mg) obtained in (1).
   Melting point: 159-160°C (ethyl acetate-hexane)
   ¹H-NMR (300MHz, DMSO-d₆); δ 3.05 (1H, dd, J=10.2, 13.8Hz), 3.16 (1H, dd, J=4.2, 13.8Hz), 4.55-4.65 (1H, m), 4.88 (2H, s), 6.51-6.54 (1H, m), 6.89 (2H, d, J=8.7Hz), 7.21 (2H, d,J=8.7Hz), 7.55 (2H, d, J=8.7Hz), 7.60-7.69 (3H, m), 7.73 (1H, s), 7.80 (2H, d, J=8.7Hz), 7.86 (1H, d, J=8.4Hz), 7.96 (1H, s), 8.83 (1H, d, J=8.1Hz), 12.89 (1H, br s)
   Elemental analysis: Calcd. for C₂₉H₂₂ClNO₆•0.25 H₂O: C 66.93, H 4.36, N 2.69; Found: C 67.06, H4.23,N2.64

### Example C152

### N-{[5-(4-Chlorophenyl)-1-benzofuran-2-yl]carbonyl}-O-(3-furylmethyl)tyrosine

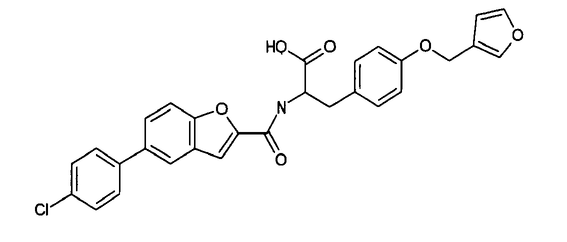

(1) Methyl N-{[5-(4-chlorophenyl)-1-benzofuran-2-yl]carbonyl}-O-(3-furylmethyl)tyrosinate (150 mg) was obtained as colorless crystals by methods similar to those of Example C122(1) from 5-(4-chlorophenyl)-1-benzofuran-2-carboxylic acid (87 mg) and methyl O-(3-furylmethyl)tyrosinate hydrochloride (100 mg).
   ¹H-NMR (300MHz, CDCl₃); δ 3.15-3.30 (2H, m), 3.78 (3H, s), 4.90 (2H, m), 5.04-5.11 (1H, m), 6.47 (1H, d, J=0.9Hz), 6.89 (2H, d, J=8.7Hz), 7.05-7.12 (3H, m), 7.40-7.64 (9H, m), 7.81 (1H, d, J=0.9Hz)
(2) The title compound (90 mg) was obtained as colorless crystals by methods similar to those of Example C 122(2) from the compound (130 mg) obtained in (1).
   Melting point: 211-212°C (ethyl acetate-hexane)
   ¹H-NMR (300MHz, DMSO-d₆); δ 3.05 (1H, dd, J=9.9, 13.8Hz), 3.16 (1H, dd, J=4.5, 13.8Hz), 4.55-4.65 (1H, m), 4.87 (2H, s), 6.50-6.53 (1H, m), 6.89 (2H, d, J=8.7Hz), 7.22 (2H, d, J=8.7Hz), 7.53 (2H, d, J=8.7Hz), 7.60-7.63 (2H, m), 7.70-7.78 (5H, m), 8.06 (1H, s), 8.89 (1H, d, J=7.8Hz), 12.88(1H, br s)
   Elemental analysis: Calcd. for C₂₉H₂₂ClNO₆: C 67.51, H 4.30, N 2.71; Found: C 67.27, H 4.29, N 2.71

### Example C153

### N-{[6-(4-Chlorophenyl)-1-benzofuran-2-yl]carbonyl}-O-(3-fluorobenzyl)tyrosine

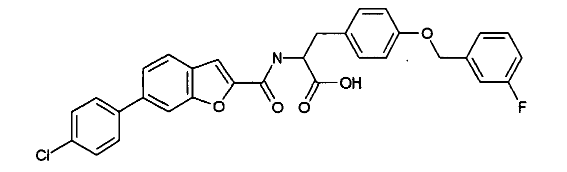

(1) Methyl N-{[6-(4-chlorophenyl)-1-benzofuran-2-yl]carbonyl}-O-(3-fluorobenzyl)tyrosinate (162 mg) was obtained as colorless crystals by methods similar to those of Example C122(1) from 6-(4-chlorophenyl)-1-benzofuran-2-carboxylic acid (95 mg) and methyl O-(3-fluorobenzyl)tyrosinate hydrochloride (119 mg).
   ¹H-NMR (300MHz, CDCl₃); δ 3.15-3.30 (2H, m), 3.78 (3H, s), 5.03 (2H, s), 5.03-5.11 (1H, m), 6.89 (2H, d, J=8.7Hz), 6.95-7.19 (6H, m), 7.28-7.38 (1H, m), 7.45 (2H, d, J=8.7Hz), 7.48-7.54 (2H, m), 7.57 (2H, d, J=8.7Hz), 7.67 (1H, s), 7.72 (1H, d, J=8.1Hz)
(2) The title compound (116 mg) was obtained as colorless crystals by operations similar to those of Example C122(2) from the compound (157 mg) obtained in (1).
   Melting point: 162-163°C (ethyl acetate-hexane)
   ¹H-NMR (300MHz, DMSO-d₆); δ 3.06 (1H, dd, J=10.2, 13.8Hz), 3.16 (1H, dd, J=4.5, 13.8Hz), 4.55-4.65 (1H, m), 5.05 (2H, s), 6.91 (2H, d, J=8.7Hz), 7.08-7.27 (5H, m), 7.35-7.44 (1H, m), 7.55 (2H, d, J=8.7Hz), 7.61 (1H, s), 7.66 (1H, dd, J=1.2, 8.4Hz), 7.79 (2H, d, J=8.7Hz), 7.86 (1H, d, J=8.4Hz), 7.95 (1H, s), 8.83 (1H, d, J=8.4Hz), 12.90 (1H, br s)
   Elemental analysis: Calcd. for C₃₁H₂₃ClFNO₅: C 68.45, H 4.26, N 2.57; Found: C 68.21, H 4.32, N 2.45

### Example C154

### N-{[6-(4-Chlorophenyl)-1-benzofuran-2-yl]carbonyl }-O-(3-methylbenzyl)tyrosine

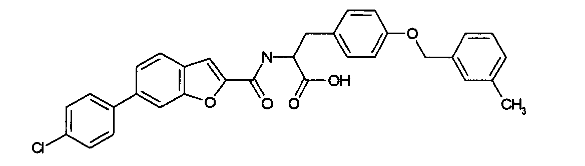

(1) Methyl N-{[6-(4-chlorophenyl)-1-benzofuran-2-yl]carbonyl}-O-(3-methylbenzyl)tyrosinate (161 mg) was obtained as colorless crystals by operations similar to those of Example C122(1) from 6-(4-chlorophenyl)-1-benzofuran-2-carboxylic acid (95 mg) and methyl O-(3-methylbenzyl)tyrosinate hydrochloride (118 mg).
   ¹H-NMR (300MHz, CDCl₃); δ 2.35 (3H, s), 3.15-3.28 (2H, m), 3.78 (3H, s), 4.98 (2H, s), 5.02-5.10 (1H, m), 6.91 (2H, d, J=8.7Hz), 7.05-7.29 (7H, m), 7.44 (2H, d, J=8.7Hz), 7.47-7.53 (2H, m), 7.56 (2H, d, J=8.7Hz), 7.66 (1H, s), 7.71 (1H, d, J=8.1 Hz)
(2) The title compound (110 mg) was obtained as colorless crystals by operations similar to those of Example C122(2) from the compound (156 mg) obtained in (1).
   Melting point: 157-158°C (ethyl acetate-hexane)
   ¹H-NMR (300MHz, DMSO-d₆); δ 2.28 (3H, s), 3.06 (1H, dd, J=10.2, 13.8Hz), 3.16 (1H, dd, J=4.5, 13.8Hz), 4.55-4.65 (1H, m), 4.98 (2H, s), 6.90 (2H, d, J=8.7Hz), 7.07-7.27 (6H, m), 7.55 (2H, d, J=8.7Hz), 7.61 (1H, s), 7.66 (1H, dd, J=1.5, 8.4Hz), 7.79 (2H, d, J=8.7Hz), 7.86 (1H, d, J=8.4Hz), 7.95 (1H, s), 8.83 (1H, d, J=8.4Hz), 12.89 (1H, br s)
   Elemental analysis: Calcd. for C₃₂H₂₆ClNO₅•0.25 H₂O: C 70.59, H 4.91, N 2.57; Found: C 70.60, H 4.84, N 2.44

### Example C155

### N-{[6-(4-Chlorophenyl)-1-benzofuran-2-yl]carbonyl }-O-(3-methoxybenzyl)tyrosine

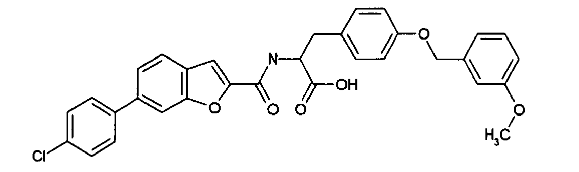

(1) Methyl N-{[6-(4-chlorophenyl)-1-benzofuran-2-yl]carbonyl}-O-(3-methoxybenzyl)tyrosinate (187 mg) was obtained as a colorless oil by operations similar to those of Example C122(1) from 6-(4-chlorophenyl)-1-benzofuran-2-carboxylic acid (95 mg) and methyl O-(3-methoxybenzyl)tyrosinate hydrochloride (123 mg).
   ¹H-NMR (300MHz, CDCl₃); δ 3.15-3.27 (2H, m), 3.78 (3H, s), 3.80 (3H, s), 5.00 (2H, s), 5.02-5.11 (1H, m), 6.82-7.01 (5H, m), 7.04-7.11 (3H, m), 7.24-7.31 (1H, m), 7.44 (2H, d, J=8.7Hz), 7.47-7.53 (2H, m), 7.56 (2H, d, J=8.7Hz), 7.67 (1H, s), 7.71 (1H, d, J=8.4Hz)
(2) The title compound (118 mg) was obtained as colorless crystals by operations similar to those of Example C122(2) from the compound (182 mg) obtained in (1).
   Melting point: 150-151°C (ethyl acetate-hexane)
   ¹H-NMR (300MHz, DMSO-d₆); δ 3.05 (1H, dd, J=9.9, 13.8Hz), 3.16 (1H, dd, J=4.8, 13.8Hz), 3.72 (3H, s), 4.55-4.65 (1H, m), 5.00 (2H, s), 6.82-7.00 (5H, m), 7.18-7.30 (3H, m), 7.55 (2H, d, J=8.7Hz), 7.61 (1H, d, J=0.6Hz), 7.66 (1H, dd, J=1.5, 8.4Hz), 7.80 (2H, d, J=8.7Hz), 7.86 (1H, d, J=8.4Hz), 7.95 (1H, s), 8.83 (1H, d, J=8.4Hz), 12.89 (1H, br s)
   Elemental analysis: Calcd. for C₃₂H₂₆ClNO₆, : C 69.13, H 4.71, N 2.52; Found: C 68.96, H 4.66, N 2.44

### Example C156

### (2S)-[4-(Benzyloxy)phenyl]({[6-(4-chlorophenyl)-3-methyl-1-benzofuran-2-yl]carbonyl}amino)acetic acid

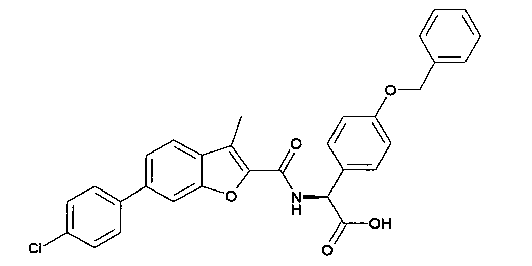

(1) Thionyl chloride (4.2 mL) was added dropwise with ice cooling into methanol (60 mL), and stirred for 15 minutes. (2S)-amino(4-hydroxyphenyl)acetic acid (5.0 g, 29.9 mmol) was added and stirred for 3 days at room temperature and then for 3 hours at 70°C. The reaction mixture was concentrated under reduced pressure, and the residue was recrystallized from a methanol-diethyl ether mixed solvent to obtain methyl (2S)-amino(4-hydroxyphenyl)acetate hydrochloride (6.27 g, 96%) as a white solid.
   ¹H-NMR (300MHz, DMSO-d₆); δ3.71 (s, 3H), 5.13 (s, 1H), 6.82-6.84 (d, J=8.4Hz, 2H), 7.27-7.29 (d, J=8.4Hz, 2H), 8.87 (s, 3H), 9.90 (s, 1H)
(2) A mixture of methyl (2S)-amino(4-hydroxyphenyl)acetate hydrochloride (6.27 g, 28.8 mmol), Boc₂O (7.55 g, 34.6 mmol), triethylamine (4.8 mL, 34.6 mmol) and tetrahydrofuran (100 mL) was stirred overnight at 60°C. The reaction mixture was concentrated under reduced pressure, diluted with ethyl acetate, and washed with water (twice) and saturated sodium chloride solution. The organic layer was dried by addition of anhydrous magnesium sulfate. The drying agent was removed by filtration, and the filtrate was concentrated under reduced pressure. The residue was recrystallized from an ethyl acetate-hexane mixed solution to obtain methyl (2S)-[(tert-butoxycarbonyl)amino](4-hydroxyphenyl)acetate (6.96 g, 86%) as a white solid.
   ¹H-NMR (300MHz, CDCl₃); δ1.26 (s, 9H), 3.71 (s, 3H), 5.22-5.29 (m, 2H), 5.51 (s, 1H), 6.75-6.79 (d, J=8.7Hz, 2H), 7.19-7.22(d, J=8.7Hz, 2H)
(3) A mixture of methyl (2S)-[(tert-butoxycarbonyl)amino](4-hydroxyphenyl)acetate (1.18 g, 4.20 mmol), benzyl bromide (0.861 g, 5.03 mmol), potassium carbonate (1.16 g) and dimethylformamide (20 mL) was stirred for 3 hours at room temperature. Ethyl acetate and water were added to separate the solution, and the organic layer was washed twice with water and once with saturated sodium chloride solution and dried over magnesium sulfate. After removal of the drying agent by filtration , this was concentrated under reduced pressure. The residue was recrystallized from an ethyl acetate-hexane mixed solvent to obtain methyl (2S)-[4-(benzyloxy)phenyl][tert-butoxycarbonyl)amino]acetate (0.92 g, 59%) as a white solid.
   ¹H-NMR (300MHz, CDCl₃); δ1.43 (s, 9H), 3.72 (s, 3H), 5.05 (s, 2H), 5.24-5.27 (d, J=7.5Hz, 1H), 5.46 (br s, 1H), 6.92-6.97 (m, 2H), 7.30-7.44 (m, 7H)
(4) 4 N Hydrogen chloride-ethyl acetate solution (5 mL) was added to an ethyl acetate (10 mL) solution of methyl (2S)-[4-(benzyloxy)phenyl][tert-butoxycarbonyl)amino]acetate (0.92 g, 2.48 mmol), and stirred overnight at room temperature. The precipitated white solid was filtered out and washed successively with ethyl acetate and diethyl ether to obtain methyl (2S)-amino[4-(benzyloxy)phenyl]acetate hydrochloride (0.72 g, 93%) as a white solid.
   ¹H-NMR (300MHz, DMSO-d₆); δ3.71 (s, 3H), 5.04 (s, 2H), 5.23 (s, 1H), 7.08-7.11(d, J=9.0Hz, 2H), 7.33-7.46 (m, 7H), 8.75 (s, 3H)
(5) A mixture of 6-(4-chlorophenyl)-1-benzofuran-2-carboxylic acid (146 mg, 0.51 mmol), methyl (2S)-amino[4-(benzyloxy)phenyl]acetate hydrochloride (92 mg, 0.30 mmol), (3-dimethylaminopropyl)ethyl carbodiimide hydrochloride (115 mg, 0.60 mmol), 1-hydroxybenzotriazole (81 mg, 0.60 mmol), triethylamine (300 µL, 1.2 mmol), N,N-dimethylformamide (2 mL) and dichloromethane (4 mL) was stirred overnight at room temperature. Water was added to the reaction solution, which was then extracted with ethyl acetate. The extract was washed with water and saturated sodium chloride solution and then dried over anhydrous magnesium sulfate. The drying agent was removed by filtration, the solvent was evaporated under reduced pressure and the residue was purified by silica gel column chromatography (eluate: ethyl acetate/hexane = 1/10-1/1) and then further purified by preparative HPLC to obtain methyl (2S)-[4-(benzyloxy)phenyl]({[6-(4-chlorophenyl)-3-methyl-1-benzofuran-2-yl]carbonyl}amino)acetate (68 mg, 42%) as a colorless oil.
   LC-MS 540 [M+H]⁺
   ¹H-NMR (300MHz, CDCl₃); δ2.62 (s, 3H), 3.79 (s, 3H), 5.07 (s, 2H), 5.70-5.72 (d, J=7.2Hz, 1H), 6.98-7.02 (m, 2H), 7.30-7.66 (m, 15H)
(6) 1 M Sodium hydroxide aqueous solution (252 µL) was added to a tetrahydrofuran-methanol mixed solution (1 mL-1 mL) of methyl (2S)-[4-(benzyloxy)phenyl]({[6-(4-chlorophenyl)-3-methyl-1-benzofuran-2-yl]carbonyl}amino)acetate (68 mg, 0.13 mmol), and stirred for 2 hours at room temperature. The reaction solution was neutralized with 1 N hydrochloric acid (300 µL), and then extracted twice with ethyl acetate, washed with saturated sodium chloride solution, and dried over magnesium sulfate. After removal of the drying agent by filtration, this was concentrated under reduced pressure. The residue was recrystallized from an ethyl acetate-hexane mixed solvent to obtain the title compound (44 mg, 66%) as a white solid.
   LC-MS 526 [M+H]⁺
   ¹H-NMR (300MHz, DMSO-d₆); δ2.57 (s, 3H), 5.11 (s, 2H), 5.47-5.50 (s, J=7.2Hz, 1H), 7.00-7.03 (d, J=8.7Hz, 2H), 7.30-7.46 (m,7H), 7.54-7.56 (d, J=8.4Hz, 2H), 7.65-7.69 (dd, J=1.5, 6.9Hz, 1H), 7.78-7.84 (m, 3H), 7.93 (d, J=0.9Hz, 1H), 8.61-8.64 (d, J=7.2Hz, 1H), 13.05 (br s, 1H)
   Melting point: 178°C
   Elemental analysis: Calcd. for C₃₁H₂₄NO₅Cl (containing 0.5 mol H₂O), : C 69.71, H 4.47, N 2.30; Found: C 69.60, H 4.71, N 2.62

### Example C157

### N-{[6-(4-Chlorophenyl)-1-benzofuran-2-yl]carbonyl}-O-(4-fluorobenzyl) tyrosine

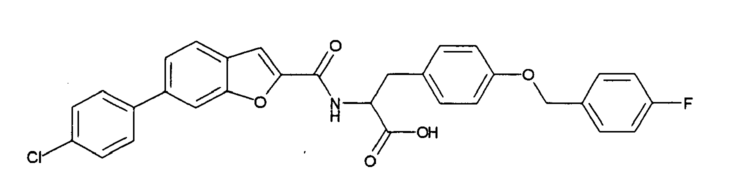

(1) A mixture of 6-(4-chlorophenyl)-1-benzofuran-2-carboxylic acid (136 mg, 0.50 mmol), methyl O-(4-fluorobenzyl)tyrosinate hydrochloride (153 mg, 0.45 mmol), (3-dimethylaminopropyl)ethyl carbodiimide hydrochloride (192 mg, 1.00 mmol), 1-hydroxybenzotriazole (135 mg, 1.00 mmol), triethylamine (300 µL, 2.1 mmol), N,N-dimethylformamide (2 mL) and dichloromethane (4 mL) was stirred overnight at room temperature. Water was added to the reaction solution, which was then extracted with ethyl acetate. The extract was washed with water and saturated sodium chloride solution and then dried over anhydrous magnesium sulfate. The drying agent was removed by filtration, the solvent was evaporated under reduced pressure, and the residue was purified by silica gel column chromatography (eluate: ethyl acetate/hexane = 1/10-1/1) to obtain methyl N-{[6-(4-chlorophenyl)-1-benzofuran-2-yl]carbonyl}-O-(4-fluorobenzyl)tyrosinate (234 mg, 93%) as a colorless oil.
   LC-MS 558 [M+H]⁺
   ¹H-NMR (300MHz, CDCl₃); δ 3.15-3.29 (m, 2H), 3.78 (s, 3H), 4.98 (s, 2H), 5.05-5.11 (m, 1H), 6.88-6.91 (d, J=8.7Hz, 2H), 7.03-7.10 (m, 5H), 7.36-7.47 (m, 8H), 7.49-7.52 (m, 2H)
(2) 1 M Sodium hydroxide aqueous solution (840 µL) was added to a tetrahydrofuran-methanol mixed solution (2 mL-2 mL) of methyl N-{[6-(4-chlorphenyl)-1-benzofuran-2-yl]carbonyl}-O-(4-fluorobenzyl)tyrosinate (234 mg, 0.42 mmol), and stirred for 2 hours at room temperature. The reaction solution was neutralized with 1 N hydrochloric acid (1000 µL), extracted twice with ethyl acetate, washed with saturated sodium chloride solution and then dried over anhydrous magnesium sulfate. After removal of the drying agent by filtration, this was concentrated under reduced pressure. The residue was recrystallized from an ethyl acetate-hexane mixed solvent to obtain the title compound (196 mg, 86%) as a white solid.
   LC-MS 544 [M+H]⁺
   ¹H-NMR (300MHz, DMSO-d₆); δ 3.02-3.20 (m, 2H), 4.58-4.66 (m, 1H), 5.00 (s, 2H), 6.89-6.92 (d, J=8.7Hz, 2H), 7.15-7.24 (d, J=8.7Hz, 2H), 7.43-7.48 (m, 2H), 7.53-7.56 (d, J=8.7Hz, 2H), 7.61-7.68 (m, 2H), 7.78-7.87 (m, 3H), 7.95 (s, 1H), 8.82-8.85 (d, J=8.1Hz, 1H), 12.80 (br s, 1H)
   Melting point: 166-168°C
   Elemental analysis: Calcd. for C₃₁H₂₃NO₅ClF, : C 68.45, H 4.26, N 2.57; Found: C 68.29, H 4.31, N 2.50

### Example C158

### N-{[6-(4-Chlorophenyl)-1-benzofuran-2-yl]carbonyl}-O-(4-methylbenzyl)tyrosine

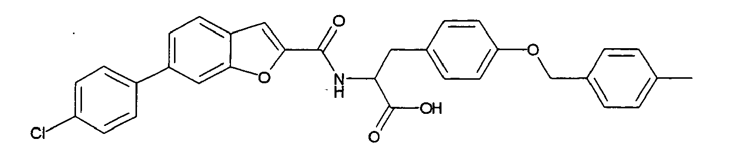

(1) A mixture of 6-(4-chlorophenyl)-1-benzofuran-2-carboxylic acid (136 mg, 0.50 mmol), methyl O-(4-methylbenzyl)tyrosinate hydrochloride (151 mg, 0.45 mmol), (3-dimethylaminopropyl)ethyl carbodiimide hydrochloride (192 mg, 1.00 mmol), 1-hydroxybenzotriazole (135 mg, 1.00 mmol), triethylamine (300 µL, 2.1 mmol), N,N-dimethylformamide (2 mL) and dichloromethane (4 mL) was stirred overnight at room temperature. Water was added to the reaction solution, which was then extracted with ethyl acetate. The extract was washed with water and saturated sodium chloride solution and then dried over anhydrous magnesium sulfate. The drying agent was removed by filtration, the solvent was evaporated under reduced pressure, and the residue was purified by silica gel column chromatography (eluate: ethyl acetate/hexane = 1/10-1/1) to obtain methyl N-{[6-(4-chlorophenyl)-1-benzofuran-2-yl]carbonyl}-O-(4-methylbenzyl)tyrosinate (216 mg, 87%) as a colorless oil.
   LC-MS 554 [M+H]⁺
   ¹H-NMR (300MHz, CDCl₃); δ 2.34 (s, 3H), 3.20-3.28 (m, 2H), 3.78 (s, 3H), 4.98 (s, 2H), 5.04-5.11 (m, 1H), 6.89-6.92 (d, J=8.4Hz, 2H), 7.06-7.09 (d, J=8.7Hz, 3H), 7.16-7.19 (d, J=8.1Hz, 2H), 7.29-7.33 (m, 2H), 7.43-7.58 (m, 6H); 7.67-7.73 (m, 2H)
(2) 1 M Sodium hydroxide aqueous solution (780 µL) was added to a tetrahydrofuran-methanol mixed solution (2 mL-2 mL) of methyl N-{[6-(4-chlorphenyl)-1-benzofuran-2-yl]carbonyl}-O-(4-methylbenzyl)tyrosinate (216 mg, 0.39 mmol), and stirred for 2 hours at room temperature. The reaction solution was neutralized with 1 N hydrochloric acid (800 µL), extracted twice with ethyl acetate, washed with saturated sodium chloride solution and then dried over magnesium sulfate. After removal of the drying agent by filtration, this was concentrated under reduced pressure. The residue was recrystallized from an ethyl acetate-hexane mixed solvent to obtain the title compound (116 mg, 55%) as a white solid.
   LC-MS 540 [M+H]⁺
   ¹H-NMR (300MHz, DMSO-d₆); δ 2.27(s, 3H), 3.02-3.19 (m, 2H), 4.58-4.66 (m, 1H), 4.97 (s, 2H), 6.87-6.90 (d, J=8.7Hz, 2H), 7.14-7.29 (m, 6H), 7.53-7.56 (d, J=8.4Hz, 2H), 7.61 (s, 1H), 7.65-7+.68 (dd, J=1.5, 6.6Hz, 1H), 7.78-7.81 (m, 3H), 7.95 (s, 1H), 8.82-8.85 (d, J=8.1Hz, 1H), 12.80 (br s, 1H)
   Melting point: 204°C
   Elemental analysis: Calcd. for C₃₂H₂₆NO₅Cl, : C 71.17, H 4.85, N 2.59; Found: C 70.98, H 4.83, N 2.58

### Example C159

### O-(4-Chlorobenzyl)-N-{[6-(4-chlorophenyl)-1-benzofuran-2-yl] carbonyl }tyrosine

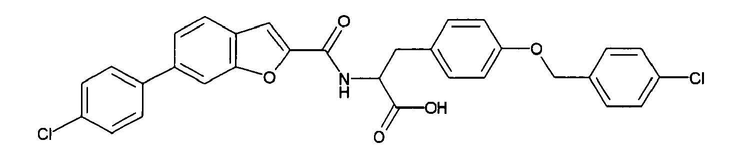

(1) A mixture of 6-(4-chlorophenyl)-1-benzofuran-2-carboxylic acid (136 mg, 0.50 mmol), methyl O-(4-chlorobenzyl)tyrosinate hydrochloride (160 mg, 0.45 mmol), (3-dimethylaminopropyl)ethyl carbodiimide hydrochloride (192 mg, 1.00 mmol), 1-hydroxybenzotriazole (135 mg, 1.00 mmol), triethylamine (300 µL, 2.1 mmol), N,N-dimethylformamide (2 mL) and dichloromethane (4 mL) was stirred overnight at room temperature. Water was added to the reaction solution, which was then extracted with ethyl acetate. The extract was washed with water and saturated sodium chloride solution and then dried over anhydrous magnesium sulfate. The drying agent was removed by filtration, the solvent was evaporated under reduced pressure and the residue was purified by silica gel column chromatography (eluate: ethyl acetate/hexane = 1/10-1/1) to obtain methyl N-{[6-(4-chlorophenyl)-1-benzofuran-2-yl]carbonyl}-O-(4-chlorobenzyl)tyrosinate (258 mg, 100%) as a colorless oil.
   LC-MS 574 [M+H]⁺
   ¹H-NMR (300MHz, DMSO-d₆); δ 3.03-3.18 (m, 2H), 3.66 (s, 3H), 4.64-4.72 (m, 1H), 5.03 (s, 2H), 6.89-6.92 (d, J=8.7Hz, 2H), 7.20-7.23 (d, J=8.4Hz, 2H), 7.39-7.45 (m, 4H), 7.54-7.57 (d, J=6.9Hz, 2H), 7.62 (s, 1H), 7.65-7.68 (dd, J=1.2, 6.9Hz, 1H), 7.78-7.88 (m, 3H), 7.95 (s, 1H), 9.05-9.07 (d, J=8.1Hz, 1H)
(2) 1 M Sodium hydroxide aqueous solution (900 µL) was added to a tetrahydrofuran-methanol mixed solution (2 mL-2 mL) of methyl N-{[6-(4-chlorophenyl)-1-benzofuran-2-yl]carbonyl}-O-(4-chlorobenzyl)tyrosinate (258 mg, 0.45 mmol), and stirred for 2 hours at room temperature. The reaction solution was neutralized with 1 N hydrochloric acid (1000 µL), extracted twice with ethyl acetate, washed with saturated sodium chloride solution and dried over magnesium sulfate. After removal of the drying agent by filtration, this was concentrated under reduced pressure. The residue was recrystallized from an ethyl acetate-hexane mixed solvent to obtain the title compound (212 mg, 83%) as a white solid.
   LC-MS 560 [M+H]⁺
   ¹H-NMR (300MHz, DMSO-d₆); δ 3.02-3.20 (m, 2H), 4.59-4.66 (m, 1H), 5.03 (s, 2H), 6.89-6.92 (d, J=8.7Hz, 2H), 7.21-7.24 (d, J=8.7Hz, 2H), 7.39-7.45 (m, 4H), 7.53-7.56 (d, J=8.7Hz, 2H), 7.61 (s, 1H), 7.65-7.68 (dd, J=1.5, 6.9Hz, 1H), 7.78-7.88 (m, 3H), 7.95 (s, 1H), 8.83-8.86 (d, J=8.4Hz, 1H), 12.80 (br s, 1H)
   Melting point: 195°C
   Elemental analysis: Calcd. for C₃₁H₂₃NO₅Cl₂, :C 66.44, H 4.14, N 2.50; Found: C 66.24, H 4.09, N 2.45

### Example C160

### N- {[6-(4-Chlorophenyl)-1-benzofuran-2-yl]carbonyl }-O-(4-cyanobenzyl)tyrosine

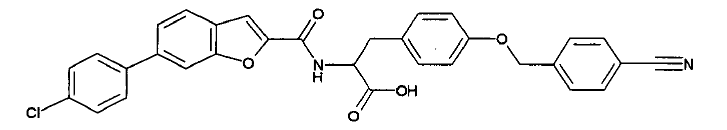

(1) A mixture of 6-(4-chlorophenyl)-1-benzofuran-2-carboxylic acid (136 mg, 0.50 mmol), methyl O-(4-cyanobenzyl)tyrosinate hydrochloride (156 mg, 0.45 mmol), (3-dimethylaminopropyl)ethyl carbodiimide hydrochloride (192 mg, 1.00 mmol), 1-hydroxybenzotriazole (135 mg, 1.00 mmol), triethylamine (300 µL, 2.1 mmol), N,N-dimethylformamide (2 mL) and dichloromethane (4 mL) was stirred overnight at room temperature. Water was added to the reaction solution, which was then extracted with ethyl acetate. The extract was washed with water and saturated sodium chloride solution and then dried over anhydrous magnesium sulfate. The drying agent was removed by filtration, the solvent was evaporated under reduced pressure, and the residue was purified by silica gel column chromatography (eluate: ethyl acetate/hexane = 1/10-1/1) to obtain methyl N-{[6-(4-chlorophenyl)-1-benzofuran-2-yl]carbonyl}-O-(4-cyanobenzyl)tyrosinate (254 mg, 100%) as a colorless oil.
   LC-MS 565 [M+H]⁺
   ¹H-NMR (300MHz, DMSO-d₆); δ 3.04-3.18 (m, 2H), 3.66 (s, 3H), 4.65-4.72 (m, 1H), 5.16 (s, 2H), 6.91-6.93 (d, J=8.7Hz, 2H), 7.21-7.24 (d, J=8.4Hz, 2H), 7.54-7.68 (m, 6H), 7.78-7.88 (m, 5H), 7.95 (s, 1H), 9.05-9.08 (d, J-8.1Hz, 1H)
(2) 1 M Sodium hydroxide aqueous solution (900 µL) was added to a tetrahydrofuran-methanol mixed solution (2 mL-2 mL) of methyl N-{[6-(4-chlorophenyl)-1-benzofuran-2-yl]carbonyl}-O-(4-cyanobenzyl)tyrosinate (254 mg, 0.45 mmol), and stirred for 2 hours at room temperature. The reaction solution was neutralized with 1 N hydrochloric acid (1000 µL), extracted twice with ethyl acetate, washed with saturated sodium chloride solution and dried over magnesium sulfate. After removal of the drying agent by filtration, this was concentrated under reduced pressure. The residue was purified by preparative HPLC to obtain the title compound (81.4 mg, 33%) as white crystals.
   ¹H-NMR (300MHz, DMSO-d₆); δ3.02-3.20 (m, 2H), 4.58-4.65 (m, 1H), 5.15 (s, 2H), 6.90-6.93 (d, J=8.7Hz, 2H), 7.22-7.24 (d, J=8.4Hz, 2H), 7.53-7.68 (m, 6H), 7.78-7.87 (m, 5H), 7.95 (s, 1H), 8.83-8.85 (d, J=8.4Hz, 1H), 12.80 (br s, 1H)
   Melting point: 204°C
   Elemental analysis: Calcd. for C₃₂H₂₃N₂O₅Cl (containing 0.8 mol H₂O), : C 67.98, H 4.39, N 4.95; Found: C 67.94, H 4.38, N 4.87

### Example C161

### N-{[6-(4-Chlorophenyl)-1-benzofuran-2-yl]carbonyl}-O-(4-ethylbenzyl)tyrosine

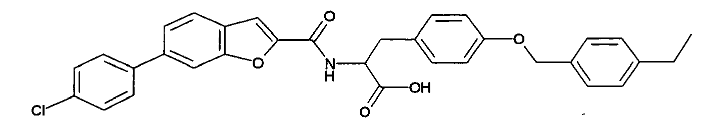

(1) A mixture of 6-(4-chlorophenyl)-1-benzofuran-2-carboxylic acid (136 mg, 0.50 mmol), methyl O-(4-ethylbenzyl)tyrosinate hydrochloride (153 mg, 0.45 mmol), (3-dimethylaminopropyl)ethyl carbodiimide hydrochloride (192 mg, 1.00 mmol), 1-hydroxybenzotriazole (135 mg, 1.00 mmol), triethylamine (300 µL, 2.1 mmol), N,N-dimethylformamide (2 mL) and dichloromethane (4 mL) was stirred overnight at room temperature. Water was added to the reaction solution, which was then extracted with ethyl acetate. The extract was washed with water and saturated sodium chloride solution and then dried over anhydrous magnesium sulfate. The drying agent was removed by filtration, the solvent was evaporated under reduced pressure, and the residue was purified by silica gel column chromatography (eluate: ethyl acetate/hexane = 1/10-1/1) to obtain methyl N-{[6-(4-chlorophenyl)-1-benzofuran-2-yl]carbonyl}-O-(4-ethylbenzyl)tyrosinate (247 mg, 97%) as a colorless oil.
   LC-MS 568 [M+H]⁺
   ¹H-NMR (300MHz, DMSO-d₆); δ1.06-1.20 (m, 3H), 2.53-2.65 (m, 3H), 3.03-3.18 (m, 2H), 3.66 (s, 3H), 4.64-4.72 (m, 1H), 4.98 (s, 2H), 6.88-6.91 (d, J=8.7Hz, 2H), 7.17-7.40 (m, 6H), 7.54-7.56 (d, J=8.7 Hz, 2H), 7.17-7.40 (m, 6H), 7.54-7.56 (d, J=8.4Hz, 2H), 7.62-7.68 (m, 2H), 7.78-7.88 (m, 3H), 7.95 (s, 1H), 9.04-9.06 (d, J=7.8Hz, 1H)
(2) 1 M Sodium hydroxide aqueous solution (870 µL) was added to a tetrahydrofuran-methanol mixed solution (2 mL-2 mL) of methyl N-{[6-(4-chlorophenyl)-1-benzofuran-2-yl]carbonyl}-O-(4-ethylbenzyl) tyrosinate (247 mg, 0.43 mmol), and stirred for 1 hour at 60°C. The reaction solution was neutralized with 1 N hydrochloric acid (900 µL), extracted twice with ethyl acetate, washed with saturated sodium chloride solution and dried over magnesium sulfate. After removal of the drying agent by filtration, this was recrystallized from ethyl acetate-hexane to obtain the title compound (148.5 mg, 62%) as colorless crystals.
   LC-MS 554 [M+H]⁺
   ¹H-NMR (300MHz, DMSO-d₆); δ 1.12-1.17 (t, J=7.5 Hz, 3H), 2.51-2.65 (m, 2H), 3.02-3.19 (m, 1H), 4.97 (s, 2H), 6.88-6.91 (d, J=8.7Hz, 2H), 7.17-7.23 (m, 4H), 7.36-7.38 (d, J=7.5Hz, 2H), 7.53-7.56 (d, J=8.4Hz, 2H), 7.62-7.68 (m, 2H), 7.78-7.87 (m, 3H), 7.95 (s, 1H), 8.82-8.85 (d, J=8.1Hz, 1H), 12.80 (br s, 1H)
   Melting point: 194°C
   Elemental analysis: Calcd. for C₃₃H₂₈NO₅Cl, : C 71.54, H 5.09, N 2.53; Found: C 71.37, H 5.05, N 2.41

### Example C162

### N-{[6-(4-Chlorophenyl)-1-benzofuran-2-yl]carbonyl}-O-[4-trifluoromethyl)benzyl]tyrosine

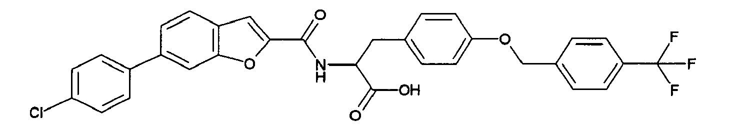

(1) A mixture of 6-(4-chlorophenyl)-1-benzofuran-2-carboxylic acid (65.8 mg, 0.241 mmol), methyl O-[4-(trifluoromethyl)benzyl]tyrosinate hydrochloride (113 mg, 0.290 mmol), (3-dimethylaminopropyl)ethyl carbodiimide hydrochloride (115 mg, 0.60 mmol), 1-hydroxybenzotriazole (81 mg, 0.60 mmol), triethylamine (168 µL, 1.2 mmol), N,N-dimethylformamide (2 mL) and dichloromethane (4 mL) was stirred overnight at room temperature. Water was added to the reaction solution, which was then extracted with ethyl acetate. The extract was washed with water and saturated sodium chloride solution and then dried over anhydrous magnesium sulfate. The drying agent was removed by filtration, the solvent was evaporated under reduced pressure, and the residue was purified by silica gel column chromatography (eluate: ethyl acetate/hexane = 1/10-1/1) to obtain methyl N-{[5-(4-chlorophenyl)-1-benzofuran-2-yl]carbonyl}-O-[4-(trifluoromethyl)benzyl]tyrosinate (143 mg, 100%) as a colorless oil.
   LC-MS 608 [M+H]⁺
   ¹H-NMR (300MHz, DMSO-d₆); δ 3.12-3.23 (m, 2H), 3.66 (s, 3H), 4.65-4.72 (m, 1H), 5.16 (s, 2H), 6.92-6.94 (d, J=8.4Hz, 2H), 7.21-7.24 (d, J=8.7Hz, 2H), 6.92-6.94 (d, J=8.4Hz, 2H), 7.21-7.24 (d, J=8.7Hz, 2H), 7.56-7.95 (m, 12H), 9.05-9.08 (d, J=8.1Hz, 1H)
(2) 1 N Sodium hydroxide aqueous solution (472 µL) was added to a tetrahydrofuran-methanol mixed solution (1 mL-1 mL) of methyl N-{[6-(4-chlorophenyl)-1-benzofuran-2-yl]carbonyl}-O-[4-(trifluoromethyl)benzyl]tyrosinate (143 mg, 0.23 mmol), and stirred for 1 hour at 60°C. The reaction solution was neutralized with 1 N hydrochloric acid (500 µL), extracted twice with ethyl acetate, washed with saturated sodium chloride solution and dried over magnesium sulfate. After removal of the drying agent by filtration, this was recrystallized from an ethyl acetate-hexane mixed solution to obtain the title compound (113 mg, 81%) as colorless crystals.
   LC-MS 594 [M+H]⁺
   ¹H-NMR (300MHz, DMSO-d₆); δ 3.07-3.15 (m, 2H), 4.53-4.65 (m, 1H), 5.15 (s, 2H), 6.91-6.94 (d, 8.7Hz, 2H), 7.22-7.25 (d, J=8.7Hz, 2H), 7.53-7.87 (m, 11H), 7.95 8(s, 1H), 8.83-8.86 (d, 1H, J=8.1Hz), 12.80 (br s, 1H)
   Melting point: 203°C
   Elemental analysis: Calcd. for C₃₂H₂₃NO₅ClF₃, : C 64.71, H 3.90, N 2.36; Found: C 64.61, H 3.92, N 2.26

### Example C163

### O-Benzyl-N-{[6-(4-chlorophenyl)-1H-indol-2-yl]carbonyl}tyrosine

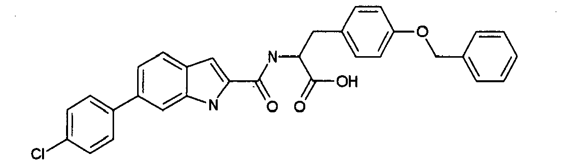

(1) A 20% ethanol solution (22.1 mL) of sodium ethoxide was added slowly with ice cooling to a toluene solution (100 mL) of4-bromobenzaldehyde (10.0 g) and ethyl azidoacetate (7.0 g), and stirred for 3 hours at room temperature. Water was added to the reaction solution, which was then extracted with ethyl acetate. The extract was washed with saturated sodium chloride solution and then dried over anhydrous magnesium sulfate. The solvent was evaporated under reduced pressure, and the residue was purified by silica gel column chromatography to obtain ethyl (2Z)-2-azido-3-(4-bromophenyl)acrylate (6.35 g) as a yellow oil.
   ¹H-NMR (300MHz, CDCl₃); δ 1.40 (3H, t, J=7.2Hz), 4.37 (2H, q, J=7.2Hz), 6.82 (1H, s), 7.50 (2H, dt, J=1.4, 8.4Hz), 7.49 (2H, dt, J=1.4, 8.4Hz)
(2) A xylene solution (200 mL) of the compound (6.35 g) obtained in (1) was heated and refluxed for 40 minutes. The reaction solution was concentrated under reduced pressure, and the precipitated crystals were filtered out and washed with hexane to obtain 6-bromo-1H-indole-2-carboxylic acid (2.98 g) as colorless crystals.
   ¹H-NMR (300MHz, CDCl₃); δ 1.42 (3H, t, J=7.2Hz), 4.42 (2H, q, J=7.2Hz), 7.17-7.21 (1H, m), 7.26 (1H, dd, J=1.8, 8.7Hz), 7.55 (1H, d, J=8.7Hz), 7.58-7.61 (1H, m), 8.88 (1H, br s)
(3) A mixture of the compound (2.68 g) obtained in (2), 4-chlorophenylboronic acid (1.88 g), tetrakis(triphenylphosphine)palladium (0) (578 mg), 2 M sodium carbonate aqueous solution (10 mL) and 1,2-dimethoxyethane (70 mL) was stirred for 14 hours at 80°C. Water was added to the reaction solution, which was then extracted with ethyl acetate. The extract was washed with saturated sodium chloride solution and then dried over anhydrous magnesium sulfate. The solvent was evaporated under reduced pressure, and the residue was purified by basic silica gel column chromatography and recrystallized from ethyl acetate-hexane to obtain ethyl 6-(4-chlorophenyl)-1H-indole-2-carboxylate (1.96 g) as colorless crystals.
   ¹H-NMR (300MHz, CDCl₃); δ 1.43 (3H, t, J=7.2Hz), 4.42 (2H, q, 7.2Hz), 7.23-7.26 (1H, m), 7.37 (1H, dd, J=1.5, 8.4Hz), 7.43 (2H, d, J=8.4Hz), 7. 5 6-7. 59 (1H, m), 7.57 (2H, d, J=8.4Hz), 7.75 (1H, d, J=8.4Hz), 8.89 (1H, br s)
(4) 2 N Sodium hydroxide aqueous solution (5 mL) and water (5 mL) were added to a tetrahydrofuran-ethanol mixed solution (10 mL-5 mL) of the compound (1.0 g) obtained in (3), and stirred for 20 minutes at 60°C. The reaction solution was diluted with water, and the aqueous layer was washed with diethyl ether. The resulting aqueous layer was neutralized with 2 N hydrochloric acid and extracted with diethyl ether. The extract was washed with saturated sodium chloride solution and then dried over anhydrous magnesium sulfate. The solvent was evaporated under reduced pressure to obtain 6-(4-chlorophenyl)-1H-indole-2-carboxylic acid (0.64 g) as a pale brown powder.
   ¹H-NMR (300MHz, DMSO-d₆); δ 7.11 (1H, d, J=1.5Hz), 7.37 (1H, dd, J=1.5, 8.7Hz), 7.52 (2H, d, J=8.4Hz), 7.64 (1H, s), 7.69 (2H, d, J=8.4Hz), 7.73 (1H, d, J=8.7Hz), 11.88 (1H, s), 13.02 (1H, br s)
(5) A mixture of the compound (271 mg) obtained in (4), methyl O-benzyltyrosinate hydrochloride (340 mg), (3-dimethylaminopropyl) ethyl carbodiimide hydrochloride (230 mg), 1-hydroxybenzotriazole (162 mg), triethylamine (0.28 mL) and N,N-dimethylformamide (15 mL) was stirred for 19 hours at room temperature. Water was added to the reaction solution, which was then extracted with ethyl acetate-tetrahydrofuran. The extract was washed successively with hydrochloric acid, sodium hydroxide aqueous solution and saturated sodium chloride solution, and then dried over anhydrous magnesium sulfate. The solvent was evaporated under reduced pressure and the residue was recrystallized from ethyl acetate-hexane to obtain methyl O-benzyl-N-{[6-(4-chlorophenyl)-1H-indole-2-yl]carbonyl}tyrosinate (350 mg) as colorless crystals.
   Melting point: 196-198°C
   ¹H-NMR (300MHz, CDCl₃); δ 3.13-3.29 (2H, m), 3.78 (3H, s), 5.04 (2H, s), 5.05-5.10 (1H, m), 6.59 (1H, d, J=7.8Hz), 6.84-6.86 (1H, m), 6.91 (2H, d, J=8.7Hz), 7.06 (2H, d, J=8.7Hz), 7.30-7.45 (8H, m), 7.55-7.61 (3H, m), 7.70 (1H, d, J=8.4Hz), 9.17 (1H, s)
   Elemental analysis: Calcd. for C₃₂H₂₇ClN₂O₄, : C 71.30, H 5.05, N 5.20; Found: C 71.25, H 5.05, N 5.04
(6) 2 N Sodium hydroxide aqueous solution (1 mL) and water (1 mL) were added to a tetrahydrofuran-methanol mixed solution (6 mL-3 mL) of the compound (250 mg) obtained in (5), and stirred for 5 minutes at 70°C. The reaction solution was neutralized with 2 N hydrochloric acid and extracted with diethyl ether. The extract was washed with saturated sodium chloride solution and then dried over anhydrous magnesium sulfate. The solvent was evaporated under reduced pressure, and the residue was recrystallized from ethyl acetate-hexane to obtain the title compound (211 mg) as colorless crystals.
   Melting point: 202-204°C
   ¹H-NMR (300MHz, DMSO-d₆); δ 3.01 (1H, dd, J=10.5, 13.8Hz), 3.15 (1H, dd, J=3.9, 13.8Hz), 4.55-4.65 (1H, m), 5.01 (2H, s), 6.90 (2H, d, J=8.7Hz), 7.21-7.43 (9H, m), 7.51 (2H, d, J=8.7Hz), 7.62 (1H, s), 7.68 (2H, d, J=8.7Hz), 7.73 (1H, d, J=8.4Hz), 8.72 (1H, d, J=8.4Hz), 11.67 (1H, s), 12.85(1H,brs)
   Elemental analysis: Calcd. for C₃₁H₂₅ClN₂O₄, : C 70.92, H 4.80, N 5.34; Found: C 70.73, H 4.83, N 5.21

### Example C164

### O-Benzyl-N-{[6-(4-chlorophenyl)-1-methyl-1H-indole-2-yl]carbonyl}tyrosine

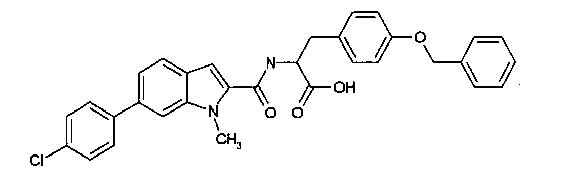

(1) Sodium hydride (120 mg) was added to a N,N-dimethylformaldehyde solution (10 mL) of the compound (749 mg) obtained in (3) of Example C163, and stirred for 10 minutes at room temperature, and a N,N-dimethylformaldehyde solution (1 mL) of methyl iodide (426 mg) was then added with ice cooling and stirred for 20 minutes at room temperature. Water was added to the reaction solution, which was then extracted with ethyl acetate. The extract was washed with saturated sodium chloride solution and then dried over anhydrous magnesium sulfate. The solvent was evaporated under reduced pressure, and the residue was purified by silica gel column chromatography to obtain ethyl 6-(4-chlorophenyl)-1-methyl-1H-indole-2-carboxylate (720 mg) as colorless crystals.
   ¹H-NMR (300MHz, CDCl₃); δ 1.42 (3H, t, J=7.2Hz), 4.13 (3H, s), 4.39 (2H, q, J=7.2Hz), 7.32 (1H, d, J=0.6Hz), 7.36 (1H, dd, J=1.5, 8.4Hz), 7.43 (2H, dt, J=2.4, 8.7Hz), 7.52 (1H, br s), 7.60 (2H, dt, J=2.4, 8.7Hz), 7.72 (1H, dd, J=0.6, 8.4Hz)
(2) 6-(4-Chlorophenyl)-1-methyl-1H-indole-2-carboxylic acid (430 mg) was obtained as a white powder by operations similar to those of Example C163(4) from the compound (600 mg) obtained in (1).
   ¹H-NMR (300MHz, DMSO-d₆); δ 4.10 (3H, s), 7.24 (1H, s), 7.44 (1H, dd, J=1.5, 8.4Hz), 7.53 (2H, d, J=8.7Hz), 7.75 (1H, d, J=8.4Hz), 7.82 (2H, d, J=8.7Hz), 7.87 (1H, s), 12.98 (1H, br s)
(3) Methyl O-benzyl-N-{[6-(4-chlorophenyl)-1-methyl-1H-indole-2-yl]carbonyl}tyrosinate (426 mg) was obtained as colorless crystals by operations similar to those of Example C163(5) from the compound (286 mg) obtained in (2).
   Melting point: 155-157°C (ethyl acetate-hexane)
   ¹H-NMR (300MHz, CDCl₃); δ 3.11-3.30 (2H, m), 3.79 (3H, s), 4.07 (3H, s), 5.00-5.09 (1H, m), 5.05 (2H, s), 6.58 (1H, d, J=7.5Hz), 6.82 (1H, s), 6.93 (2H, d, J=8.4Hz), 7.08 (2H, d, J=8.4Hz), 7.30-7.46 (8H, m), 7.51 (1H, s), 7.60 (2H, d, J=8.4Hz), 7.68 (1H, d, J=8.1Hz)
   Elemental analysis: Calcd. for C₃₃H₂₉ClN₂O₄, : C 71.67, H 5.29, N 5.07; Found: C 71.70, H 5.32, N4.97
(4) The title compound (138 mg) was obtained as colorless crystals by operations similar to those of Example C163(6) from the compound (300 mg) obtained in (3).
   Melting point: 205-206°C (ethyl acetate-hexane)
   ¹H-NMR (300MHz, DMSO-d₆); δ 3.00 (1H, dd, J=10.5, 13.8Hz), 3.14 (1H, dd, J=4.5, 13.8Hz), 3.95 (3H, s), 4.51-4.61 (1H, m), 5.03 (2H, s), 6.92 (2H, d, J=8.7Hz), 7.13 (1H, s), 7.25 (2H, d, J=8.7Hz), 7.28-7.45 (6H, m), 7.52 (2H, d, J=8.7Hz), 7.74 (1H, d, J=8.1Hz), 7.77-7.83 (3H, m), 8.71 (1H, d, J=8.4Hz), 12.80 (1H, br s)
   Elemental analysis: Calcd. for C₃₂H₂₇CIN₂O₄•0.25 H₂O, : C 70.71, H 5.10, N 5.15; Found: C 70.45,H5.11, N 5.05

### Example C165

### O-Benzyl-N-{ [5-(4-chlorophenyl)-1H-indol-2-yl] carbonyl } tyrosine

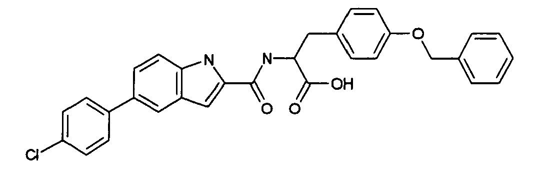

(1) Trifluoromethanesulfonic anhydride (2.59 mL) was added with ice cooling to a dichloromethane-pyridine mixed solution (50 mL-10 mL) of ethyl 5-hydroxyindole-2-carboxylate (2.26 g), and stirred for 30 minutes at room temperature. Water was added to the reaction solution, which was then extracted with diethyl ether. The extract was washed with saturated sodium chloride solution and dried over anhydrous magnesium sulfate, and the solvent was evaporated under reduced pressure to obtain ethyl 5-{[(tfifluoromethyl)sulfonyl]oxy}-1H-indole-2-carboxylate (3.67 g) as pale yellow crystals. A mixture of the resulting ethyl 5-{[(trifluoromethyl)sulfonyl]oxy}-1H-indole-2-carboxylate (3.67 g), 4-chlorophenylboronic acid (2.04 g), tetrakis(triphenylphosphine)palladium (0) (629 mg), 2 M sodium carbonate aqueous solution (11 mL) and 1,2-dimethoxyethane (70 mL) was stirred for 1 hour at 80°C. Water was added to the residue, which was then extracted with ethyl acetate. The extract was washed with saturated sodium chloride solution and then dried over anhydrous magnesium sulfate. The solvent was evaporated under reduced pressure, and the residue was purified by silica gel column chromatography to obtain ethyl 5-(4-chlorophenyl)-1H-indole-2-carboxylate (2.24 g) as colorless crystals.
   ¹H-NMR (300MHz, CDCl₃); δ 1.43 (3H, t, J=7.2Hz), 4.43 (2H, q, J=7.2Hz), 7.25-7.27 (1H, m), 7.41 (2H, d, J=8.7Hz), 7.45-7.65 (4H, m), 7.85 (1H, s), 8.97 (1H, br s)
(2) 5-(4-Chlorophenyl)-1H-indole-2-carboxylic acid (483 mg) was obtained by operations similar to those of Example C163(4) from the compound (700 mg) obtained in (1). This compound was used in the following reaction without purification.
(3) Methyl O-benzyl-N-{[5-(4-chlorophenyl)-1H-indol-2-yl]carbonyl}tyrosinate (500 mg) was obtained as colorless crystals by operations similar to those of Example C163(5) from the compound (483 mg) obtained in (2).
   ¹H-NMR (300MHz, CDCl₃); δ 3.15-3.30 (2H, m), 3.79 (3H, s), 5.04 (2H, s), 5.05-5.13 (1H, m), 6.62 (1H, d, J=7.8Hz), 6.87 (1H, d, J=1.8Hz), 6.91 (2H, d, J=8.7Hz), 7.06 (2H, d, J=8.7Hz), 7.30-7.60 (11H, m), 7.80 (1H, s), 9.22 (1H, br s)
(4) The title compound (45 mg) was obtained as colorless crystals by operations similar to those of Example C163(6) from the compound (450 mg) obtained in (3).
   Melting point: 239-240°C (ethyl acetate)
   ¹H-NMR (300MHz, DMSO-d₆); δ 3.01 (1H, dd, J=10.5, 13.8Hz), 3.15 (1H, dd, J=4.5, 13.8Hz), 4.55-4.65 (1H, m), 5.01 (2H, s), 6.90 (2H, d, J=8.7Hz), 7.20-7.55 (12H, m), 7.71 (2H, d, J=8.7Hz), 7.93 (1H, s), 8.75 (1H, d, J=8.1Hz), 11.65 (1H, s), 12.80 (1H, br s)
   Elemental analysis: Calcd. for C₃₁H₂₅ClN₂O₄**•**0.5 H₂O, : C 69.72, H 4.91, N 5.25; Found: C 69.88, H 4.77, N 5.20

### Example C166

### O-Benzyl-N-{[5-(4-chlorophenyl)-1-methyl-1H-indol-2-yl]carbonyl }tyrosine

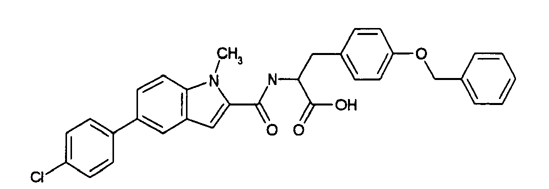

(1) Ethyl 5-(4-chlorophenyl)-1-methyl-1H-indole-2-carboxylate (493 mg) was obtained as colorless crystals by operations similar to those of Example C164(1) from the compound (505 mg) obtained in Example C165(1).
   ¹H-NMR (300MHz, CDCl₃); δ 1.43 (3H, t, J=7.2Hz), 4.12 (3H, s), 4.39 (2H, q, J=7.2Hz), 7.34 (1H, s), 7.41 (2H, d, J=8.4Hz), 7.45 (1H, d, J=9.0Hz), 7.54-7.59 (3H, m), 7.83 (1H, d, J=1.5Hz)
(2) 5-(4-Chlorophenyl)-1-methyl-1H-indole-2-carboxylic acid (282 mg) was obtained by operations similar to those of Example C163(4) from the compound (443 mg) obtained in (1). This compound was used in the following reaction without purification.
(3) Methyl O-benzyl-N-{[5-(4-chlorophenyl)-1-methyl-1H-indol-2-yl]carbonyl}tyrosinate (350 mg) was obtained as colorless crystals by operations similar to those of Example C163(5) from the compound (282 mg) obtained in (2).
   ¹H-NMR (300MHz, CDCl₃); δ 3.13-3.30 (2H, m), 3.79 (3H, s), 4.05 (3H, s), 5.00-5.10 (1H, m), 5.05 (2H, s), 6.59 (1H, d, J=7.8Hz), 6.84 (1H, s), 6.93 (2H, d, J=8.7Hz), 7.08 (2H, d, J=8.7Hz), 7.30-7.60 (11H, m), 7.79 (1H, s)
(4) The title compound (25 mg) was obtained as colorless crystals by operations similar to those of Example C 163(6) from the compound (300 mg) obtained in (3).
   Melting point: 238-239°C (ethyl acetate)
   ¹H-NMR (300MHz, DMSO-d₆); δ 3.01 (1H, dd, J=10.5, 13.5Hz), 3.14 (1H, dd, J=4.5, 13.5Hz), 3.91 (3H, s), 4.52-4.62 (1H, m), 5.03 (2H, s), 6.92 (2H, d, J=8.4Hz), 7.15-7.65 (12H, m), 7.74 (2H, d, J=8.4Hz), 7.96 (1H, s), 8.74 (1H, d, J=8.4Hz), 12.80 (1H, br s)
   Elemental analysis: Calcd. for C₃₂H₂₇ClN₂O₄•0.5 H₂O, : C 70.13, H 5.15, N 5.11; Found: C 70.32, H 4.89, N 5.14

### Example C167

### O-Benzyl-N-{[6-(4-chlorophenyl)-1H-benzimidazol-2-yl]carbonyl}tyrosine

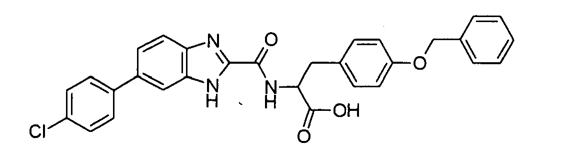

(1) A mixture of 5-bromo-2-fluoronitrobenzene (10.0 g, 45.5 mmol), 1,2-dimethoxyethane (80 ml), 4-chlorophenylboronic acid (9.26 g, 59.2 mmol), tetrakis(triphenylphosphine)palladium (0) (1.58 g, 1.37 mmol) and 2 M sodium carbonate aqueous solution (45.5 ml) was stirred for 16 hours at 80°C. Water was added to the reaction solution, which was then extracted with ethyl acetate. The extract was washed with saturated sodium chloride solution and dried over anhydrous magnesium sulfate. The solvent was evaporated under reduced pressure, and the residue was purified by silica gel column chromatography and recrystallized from ethyl acetate-hexane to obtain 4'-chloro-4-fluoro-3-nitrobiphenyl (8.13 g, 71%) as a colorless solid.
   ¹H-NMR (300MHz, CDCl₃)δ: 7.31-7.56 (5H, m), 7.78-7.83 (1H, m), 8.23 (1H, dd, J=2.4, 6.9Hz)
(2) A mixture of 4'-chloro-4-fluoro-3-nitrobiphenyl (2.00 g, 7.96 mmol), N,N-dimethylformaldehyde (30 ml), triethylamine (5.55 ml, 39.8 mmol) and ammonium chloride (2.13 g, 39.8 mmol) was stirred for 16 hours at 50°C in a stainless-steel pressure-resistant tube. The reaction solution was diluted with ethyl acetate, washed with water, and dried over anhydrous magnesium sulfate. The solvent was evaporated under reduced pressure, and the residue was recrystallized from ethyl acetate-hexane to obtain 4'-chloro-4-amino-3-nitriobiphenyl (1.24 g, 63%) as yellow crystals.
   ¹H-NMR (300MHz, CDCl₃)δ: 6.13 (2H, br s), 6.90 (1H, d, J=8.7Hz), 7.40 (2H, d, J=8.7Hz), 7.48 (2H, d, J=8.7Hz), 7.60 (1H, dd, J=2.4, 8.7Hz), 8.35 (1H, s)
(3) A mixture of 4'-chloro-4-amino-3-nitriobiphenyl (1.20 g), tetrahydrofuran (10 ml), methanol (10 ml), water (5 ml), concentrated aqueous ammonia (0.5 ml) and sodium dithionite (4.22 g) was stirred for 1 hour at 60°C. The reaction solution was filtered, the filtrate was concentrated under reduced pressure, and the residue was diluted with ethyl acetate and washed with water. This was dried over anhydrous magnesium sulfate, the solvent was evaporated under reduced pressure, and the residue was recrystallized from ethyl acetate-hexane to obtain 4'-chlorobiphenyl-3,4-diamine (0.75 g, 71%) as a pale brown powder.
   ¹H-NMR (300MHz, CDCl₃)δ: 3.46 (4H, br s), 6.76 (1H, d, J=8.7Hz), 6.92-6.94 (2H, m), 7.34 (2H, d, J=8.4Hz), 7.44 (2H, d, J=8.4Hz)
   LC-MS 219 [M+H]⁺
(4) 4'-Chlorobiphenyl-3,4-diamine (0.70 g, 3.20 mmol) was dissolved in acetic acid (10 ml), and methyl 2,2,2-trichloroacetimidate (0.62 g, 3.50 mmol) was added dropwise with water cooling. This was stirred for 2 hours at room temperature and suction filtered to obtain 6-(4-chlorophenyl)-2-(trichloromethyl)-1H-benzimidazole (0.94 g, 85%) as a pale brown powder.
   ¹H-NMR (300MHz, DMSO-d₆)δ: 7.03 (1H, s), 7:20 (1H, s), 7.37 (1H, s), 7.54 (2H, d, J=8.4Hz), 7.74 (2H, d, J=8.8Hz), 13.7 (1H, br s)
   LC-MS 347 [M+H]⁺
(5) 2 N Sodium hydroxide aqueous solution (40 ml) was added with ice cooling to a tetrahydrofuran solution (10 ml) of 6-(4-chlorophenyl)-2-(trichloromethyl)-1H-benzimidazole (6.90 g, 2.60 mmol). After 5 minutes, the reaction solution was made acidic with 2 N hydrochloric acid, and the brown sediment was filtered out and washed with water to obtain 6-(4-chlorophenyl)-1H-benzimidazole-2-carboxylic acid (0.81 g, 93%) as a crude product. This crude product was dried under reduced pressure and then dissolved in N,N-dimethylformaldehyde (5 ml), and methyl O-benzyltyrosinate hydrochloride (0.29 g, 0.92 mmol), (3-dimethylaminopropyl)ethyl carbodiimide hydrochloride (0.26 g, 1,38 mmol), 1-hydroxybenzotriazole (50 mg, 0.37 mmol) and N,N-diisopropyl ethylamine (0.94 ml, 5.52 mmol) were added and stirred for 2 hours at room temperature. The reaction solution was diluted with ethyl acetate, washed with water, dried over magnesium sulfate and concentrated under reduced pressure. The residue was purified by silica gel column chromatography (eluate: 10-50% ethyl acetate-hexane) to obtain methyl O-benzyl-N-{[6-(4-chlorophenyl)-1H-benzimidazol-2-yl]carbonyl}tyrosinate (0.64 g, 46%) as a pale brown powder.
   ¹H-NMR (300MHz, CDCl₃)δ: 3.15-3.30 (2H, m), 3.74 (3H, s), 5.00-5.09 (3H, m), 6.88 (2H, d, J=8.4Hz), 7.68 (0.5H, s), 7.85 (0.5H, d, J=8.4Hz), 7.97-8.03 (1.5H, m), 10.9 (1H, br s)
   LC-MS 540 [M]⁺
(6) A mixture of methyl O-benzyl-N-{[6-(4-chlorophenyl)-1H-benzimidazol-2-yl]carbonyl}tyrosinate (0.21 g, 0.39 mmol), tetrahydrofuran (4 ml), methanol (0.5 ml), water (3 ml) and lithium hydroxide monohydrate (33 mg, 0.78 mmol) was stirred for 40 minutes at room temperature. The reaction solution was neutralized with 1 N hydrochloric acid, and the crystals were filtered out and recrystallized from ethyl acetate-hexane to obtain the title compound (0.16 g, 76%) as pale brown crystals.
   ¹H-NMR (300MHz, DMSO-d₆)δ: 3.09-3.23 (2H, m), 3.33 (1H, br s), 4.31-4.33 (1H, m), 4.99 (2H, s), 6.82 (2H, d, J=8.7Hz), 7.10 (2H, d, J=8.7Hz), 7.27-7.42 (5H, m), 7.51-7.60 (3.5H, m), 7.71-7.80 (3H, m), 7.97 (0.5H, s), 8.59 (1H, d, J=6.9Hz), 13.4 (1H, br s)
   LC-MS 526 [M+H]⁺
   Melting point: 260-261°C
   Elemental analysis: Calcd. for C₃₀H₂₄N₃O₄Cl•H₂O, : C 66.24, H 4.82, N 7.72; Found: C 65.99, H 4.95, N 7.68

### Example C168

### N-{ [6-(4-Chlorophenyl)-1 H-benzimidazol-2-yl]carbonyl }-O-(4-fluorobenzyl)tyrosine

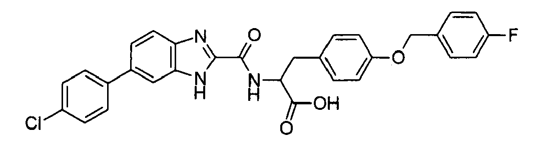

(1) Methyl N-{[6-(4-chlorophenyl)-1H-benzimidazol-2-yl]carbonyl}-O-(4-fluorobenzyl)tyrosinate (0.19 g, 37%) was obtained as a pale brown powder by methods similar to those of Example C167-5) from 6-(4-chlorophenyl)-1H-benzimidazole-2-carboxylic acid (0.25 g, 0.92 mmol) and methyl O-(4-fluorobenzyl)tyrosinate hydrochloride (0.31 g, 0.92 mmol). ¹H-NMR (300MHz, DMSO-d₆)δ: 3.18 (2H, d, J=7.2Hz), 3.68 (3H, s), 4.75-4.79 (1H, m), 5.00 (2H, s), 6.90 (2H, d, J=8.4Hz), 7.15-7.22 (4H, m), 7.43-7.63 (5.5H, m), 7.71-7.75 (2.5H, m), 7.83 (0.5H, d, J=8.7Hz), 7.98 (0.5H, s), 9.11-9.16 (1H, m), 13.35 (0.5H, s), 13.37 (0.5H, s)
   LC-MS 558 [M]⁺
(2) The title compound (0.12 g, 75%) was obtained as pale brown crystals by methods similar to those of Example 167(6) from methyl N-{[6-(4-chlorophenyl)-1H-benzimidazole-2-yl]carbonyl}-O-(4-fluorobenzyl)tyrosinate (0.17 g, 0.30 mmol).
   ¹H-NMR (300MHz, DMSO-d₆)δ: 3.16-3.19 (2H, m), 4.67-4.71 (1H, m), 5.00 (2H, s), 6.90 (2H, d, J=8.4Hz), 7.15-7.22 (4H, m), 7.43-7.62 (5.5H, m), 7.71-7.75 (2.5H, m), 7.82 (0.5H, d, J=8.4Hz), 7.98 (0.5H, s), 8.86 (1H, t, J=7.2Hz), 13.0 (1H, br s), 13.3 (0.5H, s), 13 .39 (0.5H, s)
   LC-MS 544 [M+H]⁺
   Melting point: 258-259°C
   Elemental analysis: Calcd. for C₃₀H₂₃N₃O₄ClF, : C 66.24, H 4.26, N 7.72; Found: C 65.26, H 4.30, N 7.59

### Example D1

### O-Benzyl-N-[(2E)-3-(4'-chlorobiphenyl-3-yl)propa-2-enoyl]tyrosine

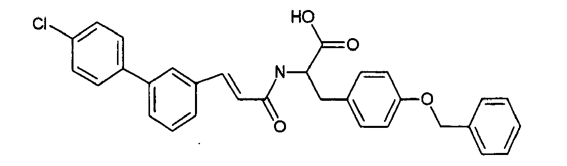

(1) A mixture of 3-bromocinnamic acid (454 mg), methyl O-benzyltyrosinate hydrochloride (680 mg), (3-dimethylaminopropyl)ethyl carbodiimide hydrochloride (460 mg), 1-hydroxybenzotriazole (324 mg), triethylamine (0.7 mL) and N,N-dimethylformamide (10 mL) was stirred overnight at room temperature. Water was added to the reaction solution, which was then extracted with diethyl ether. The extract was washed with saturated sodium chloride solution and then dried over anhydrous magnesium sulfate. The solvent was evaporated under reduced pressure, and the residue was purified by silica gel column chromatography to obtain methyl O-benzyl-N-[(2E)-3-(3-bromophenyl)propa-2-enoyl]tyrosinate (969 mg) as a colorless oil.
   ¹H-NMR (300MHz, CDCl₃); δ 3.08-3.22 (2H, m), 3.76 (3H, s), 4.92-5.02 (1H, m), 5.04 (2H, s), 6.06 (1H, d, J=7.8Hz), 6.3 (1H, d, J=15.6Hz), 6.90 (2H, d, J=8.7Hz), 7.02 (2H, d, J=8.7Hz), 7.21-7.51 (8H, m), 7.56 (1H, d, J=15.6Hz), 7.65 (1H, s)
(2) A mixture of the compound (969 mg) obtained in (1), 4-chlorophenylboronic acid (367 mg), tetrakis(triphenylphosphine)palladium (0) (113 mg), 2 M sodium carbonate aqueous solution (1.96 mL) and 1,2-dimethoxyethane (15 mL) was stirred for 14 hours at 80°C. Water was added to the reaction solution, which was then extracted with ethyl acetate. The extract was washed with saturated sodium chloride solution and then dried over anhydrous magnesium sulfate. The solvent was evaporated under reduced pressure, and the residue was purified by basic silica gel column chromatography and recrystallized from ethyl acetate-hexane to obtain methyl O-benzyl-N-[(2E)-3-(4'-chlorobiphenyl-3-yl)propa-2-enoyl]tyrosinate (552 mg) as colorless crystals.
   ¹H-NMR (300MHz, CDCl₃); δ 3.10-3.23 (2H, m), 3.76 (3H, s), 4.96-5.05 (1H, m), 5.03 (2H, s), 6.07 (1H, d, J=7.5Hz), 6.46 (1H, d, J=15.6Hz), 6.90 (2H, d, J=8.7Hz), 7.03 (2H, d, J=8.7Hz), 7.30-7.57 (12H, m), 7.67 (1H, s), 7.70 (1H, d, J=15.6Hz)
(3) 1 N Sodium hydroxide aqueous solution (1 mL) and water (7 mL) were added at 60°C to a tetrahydrofuran-methanol mixed solution (10 mL-5 mL) of the compound (368 mg) obtained in (2), and stirred for 15 minutes at that temperature. The reaction solution was neutralized with 1 N hydrochloric acid, and extracted with diethyl ether. The extract was washed with saturated sodium chloride solution and then dried over anhydrous magnesium sulfate. The solvent was evaporated under reduced pressure, and the residue was recrystallized from methanol-diethyl ether to obtain the title compound (183 mg) as colorless crystals.
   Melting point: 194-195°C
   ¹H-NMR (300MHz, DMSO-d₆); δ 2.87 (1H, dd, J=9.6, 14.1Hz), 3.06 (1H, dd, J=4.8, 14.1Hz), 4.48-4.58 (1H, m), 5.05 (2H, s), 6.83 (1H, d, J=15.9Hz), 6.92 (2H, d, J=8.7Hz), 7.17 (2H, d, J=8.7Hz), 7.27-7.61 (10H, m), 7.68 (1H, d, J=7.5Hz), 7.75 (2H, d, J=8.7Hz), 7.85 (1H, s), 8.34 (1H, d, J=8.1Hz), 12.58 (1H, br s)
   Elemental analysis: Calcd. for C₃₁H₂₆ClNO₄, C 72.72, H 5.12, N 2.74; Found: C 72.57, H 5.08, N 2.70

### Example D2

### O-Benzyl-N-[(2E)-3-(4'-chlorobiphenyl-4-yl) propa-2-enoyl]tyrosine

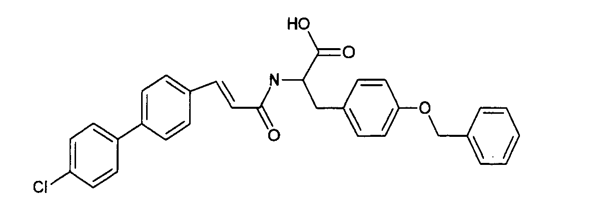

(1) A mixture of 4-bromocinnamic acid (454 mg), methyl O-benzyltyrosinate hydrochloride (680 mg), (3-dimethylaminopropyl)ethyl carbodiimide hydrochloride (460 mg), 1-hydroxybenzotriazole (324 mg), triethylamine (0.7 mL) and N,N-dimethylformamide (10 mL) was stirred overnight at room temperature. Water was added to the reaction solution, which was then extracted with diethyl ether-tetrahydrofuran. The extract was washed with saturated sodium chloride solution and then dried over anhydrous magnesium sulfate. The solvent was evaporated under reduced pressure, and the residue was purified by silica gel column chromatography to obtain methyl O-benzyl-N-[(2E)-3-(4-bromophenyl)propa-2-enoyl]tyrosinate (840 mg) as colorless crystals.
   ¹H-NMR (300MHz, CDCl₃); δ 3.08-3.23 (2H, m), 3.76 (3H, s), 4.95-5.05 (1H, m), 5.03 (2H, s), 6.05 (1R, d, J=7.5Hz), 6.38 (1H, d, J=15.6Hz), 6.90 (2H, d, J=8.7Hz), 7.02 (2H, d, J=8.7Hz), 7.30-7.45 (7H, m), 7.50 (2H, d, J=8.7Hz), 7.57 (1H, d, J=15.6Hz)
(2) A mixture of the compound (780 mg) obtained in (1), 4-chlorophenylboronic acid (297 mg), tetrakis(triphenylphosphine)palladium (0) (91 mg), 2 M sodium carbonate aqueous solution (1.58 mL) and 1,2-dimethoxyethane (10 mL) was stirred for 15 hours at 80°C. Water was added to the reaction solution, which was then extracted with ethyl acetate. The extract was washed with saturated sodium chloride solution and then dried over anhydrous magnesium sulfate. The solvent was evaporated under reduced pressure, and the residue was purified by basic silica gel column chromatography and recrystallized from ethyl acetate-hexane to obtain methyl O-benzyl-N-[(2E)-3-(4'-chlorobiphenyl-4-yl)propa-2-enoyl]tyrosinate (398 mg) as colorless crystals.
   ¹H-NMR (300MHz, CDCl₃);δ 3.09-3.23(2H, m), 3.77 (3H, s), 4.98-5.04 (1H, m), 5.04 (2H, s), 6.07 (1H, d, J=7.8Hz), 6.43 (1H, d, J=15.6Hz), 6.91 (2H, d, J=8.7Hz), 7.04 (2H, d, J=8.7Hz), 7.30-7.45 (7H, m), 7.50-7.59 (6H, m), 7.67 (1H, d, J=15.6Hz)
(3) 1 N Sodium hydroxide aqueous solution (1 mL) and water (7 mL) were added at 60°C to a tetrahydrofuran-methanol mixed solution (10 mL-5 mL) of the compound (348 mg) obtained in (2), and stirred for 15 minutes at that temperature. The reaction solution was neutralized with 1 N hydrochloric acid, and extracted with diethyl ether. The extract was washed with saturated sodium chloride solution and then dried over anhydrous magnesium sulfate. The solvent was evaporated under reduced pressure, and the precipitated crystals were filtered out and washed with diethyl ether to obtain the title compound (158 mg) as colorless crystals.
   Melting point: 253-254°C
   ¹H-NMR (300MHz, DMSO-d₆); δ 2.87 (1H, dd, J=9.3, 13.8Hz), 3.06 (1H, dd, J=4.5, 13.8Hz), 4.48-4.58 (1H, m), 5.05 (2H, s), 6.77 (1H, d, J=15 . 9Hz), 6.92 (2H, d, J=8.4Hz), 7.17 (2H, d, J=8.4Hz), 7.27-7.47 (6H, m), 7.53 (2H, d, J=8.7Hz), 7.62-7.78 (6H, m), 8.38 (1H, d, J=8.1Hz), 12.56 (1H, br s)
   Elemental analysis: Calcd. for C₃₁H₂₆ClNO₄·0.25 H₂O, : C 72.09, H 5.17, N 2.71; Found: C 72.26; H 4.93, N 2.64

### Example D3

### O-Benzyl-N-{3-[6-(4-chlorophenyl)pyridin-2-yl]acryloyl}tyrosine

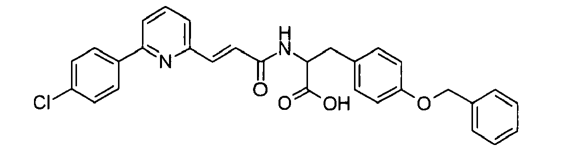

(1) 6-(4-Chlorophenyl)-2-formylpyridine (0.74 g, 63%) was obtained as a white powder by methods similar to those of Example D1(2) from 6-bromo-2-formylpyridine (1.0 g, 5.38 mmol). ¹H-NMR (300MHz, CDCl₃)δ: 7.49 (2H, d, J=8.7Hz), 7.36 (7H, m), 7.53-7.60 (3H, m), 7.69 (1H, s), 7.83 (1H, d, J=8.4Hz), 8.01 (1H, s)
   LC-MS 218 [M+H]⁺
(2) Sodium hydride (0.16 g, 3.86 mmol) was added to a tetrahydrofuran solution (10 ml) of ethyl diethylphosphonoacetate (1.03 g, 3.86 mmol), and stirred for 15 minutes. A dichloromethane solution (5 ml) of 6-(4-chlorophenyl)-2-formylpyridine (0.70 g, 3.22 mmol) was added to this and stirred for 16 hours at room temperature. The solvent was evaporated under reduced pressure, and the residue was separated with dichloromethane and sodium chloride solution. The organic layer was dried over anhydrous sodium sulfate, the solvent was evaporated under reduced pressure, and the residue was purified by silica gel column chromatography (eluate: 10-50% ethyl acetate hexane) to obtain ethyl 3-[6-(4-chlorophenyl)pyridin-2-yl]acrylate (0.54 g, 58%) as a white powder.
   ¹H-NMR (300MHz, CDCl₃)δ: 1.36 (3H, t, J=7.2Hz), 4.30 (2H, q, J=7.2Hz), 7.09 (1H, d, J=15.6Hz), 7.35 (1H, d, J=7.2Hz), 7.45 (2H, d, J=8.7Hz), 7.68-7.81 (3H, m), 8.03 (2H, d, J=8.4Hz)
   LC-MS 288 [M+H]⁺
(3) 3-[6-(4-Chlorophenyl)pyridine-2-yl]acrylic acid (0.15 g, 79%) was obtained as a white powder by methods similar to those of Example D1(3) from ethyl 3-[6-(4-chlorophenyl)pyridine-2-yl]acrylate (0.21 g, 0.73 mmol).
   ¹H-NMR (300MHz, DMSO-d₆)δ: 6.97 (1H, d, J=15.6Hz), 7.58 (2H, d, J=8.4Hz), 7.64-7.71 (2H, m), 7.94-8.03 (2H, m), 8.20 (2H, d, J=8.7Hz), 12.61 (1G, br s)
   LC-MS 260 [M+H]⁺
(4) The title compound (0.07 g, 60%) was obtained as colorless crystals from 3-[6-(4-chlorophenyl)pyridin-2-yl]acrylic acid (0.12 g, 0.46 mmol) and methyl O-benzyltyrosinate hydrochloride (0.15 g, 0.50 mmol) by methods similar to those of Example D1(1) and D1(2) performed in sequence.
   ¹H-NMR (300MHz, DMSO-d₆)δ: 2.84-2.91 (1H, m), 3.04-3.11 (1H, m), 4.51-4.58 (1H, m), 5.04 (2H, s), 6.92 (2H, d, J=8.7Hz), 7.18 (2H, d, 8.7Hz), 7.27-7.60 (10H, m), 7.91-7.99 (2H, m), 8.21 (2H, d, J=8.7Hz), 8.65 (1H, d, J=8.1 Hz), 12.72 (1H, br s)
   LC-MS 513 [M+H]⁺
   Melting point: 226-228°C
   Elemental analysis: Calcd. for C₃₀H₂₅O₄Cl•0.1 H₂O, : C 70.00, H 4.93, N 5.44; Found: C 69.76,H4.89,N5.38

### Example D4

### O-Benzyl-N- {(2E)-3-[5-(4-chlorophenyl)-2-furyl]propa-2-enoyl}tyrosine

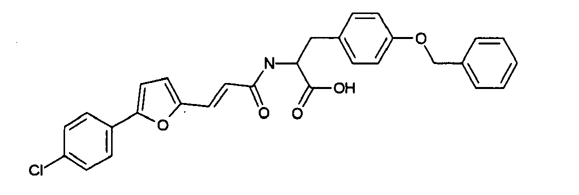

(1) A mixture of (2E)-3-[5-(4-chlorophenyl)-2-furyl]acrylic acid (249 mg), methyl O-benzyltyrosinate hydrochloride (322 mg), (3-dimethylaminopropyl)ethyl carbodiimide hydrochloride (230 mg), 1-hydroxybenzotriazole (162 mg), triethylamine (0.42 mL) and N,N-dimethylformamide (8 mL) was stirred overnight at room temperature. Water was added to the reaction solution, which was then extracted with diethyl ether. The extract was washed with saturated sodium chloride solution and then dried over anhydrous magnesium sulfate. The solvent was evaporated under reduced pressure, and the residue was purified by silica gel column chromatography to obtain methyl O-benzyl-N-{(2E)-3-[5-(4-chlorophenyl)-2-furyl]propa-2-enoyl}tyrosinate (430 mg) as colorless crystals.
   ¹H-NMR (300MHz, CDCl₃); δ 3.09-3.21 (2H, m), 3.76 (3H, s), 4.95-5.05 (1H, m), 5.04 (2H, s), 6.04 (1H, d, J=7.8Hz), 6.38 (1H, d, J=15.3Hz), 6.64 (1H, d, J=3.6Hz), 6.71 (1H, d, J=3.6Hz), 6.91 (2H, d, J=8.7Hz), 7.03 (2H, d, J=8.7Hz), 7.30-7.45 (8H, m), 7.64 (2H, d, J=8.7Hz)
(2) 1 N Sodium hydroxide aqueous solution (1 mL) and water (5 mL) were added at 60°C to a tetrahydrofuran-methanol mixed solution (3 mL-3 mL) of the compound (300 mg) obtained in (1), and stirred for 20 minutes at that temperature. The reaction solution was neutralized with 1 N hydrochloric acid, and the precipitated crystals were filtered out and washed successively with water, methanol and diethyl ether to obtain the title compound (246 mg) as colorless crystals. Melting point: 224-225°C
   ¹H-NMR (300MHz, DMSO-d₆); δ 2.84 (1H , dd, J=9.6, 13.8Hz), 3.05 (1H, dd, J=4.5, 13.8Hz), 4.45-4.55 (1R, m), 5.05 (2H, s), 6.65 (1H, d, J=15.6Hz), 6.90 (1R, d, J=3.6Hz), 6.92 (2H, d, J=8.7Hz), 7.14 (1H, d, J=3.6Hz), 7.17 (2H, d, J=8.7Hz), 7.21 (1H, d, J=15.6Hz), 7.27-7.45 (5H, m), 7.55 (2H, d, J=8.7Hz), 7.79 (2H, d, J=8.7Hz), 8.42 (1H, d, J=8.1Hz), 12.74 (1H, s) Elemental analysis: Calcd. for C₂₉H₂₄ClNO₅, : C 69.39, H 4.82, N 2.79; Found: C 69.09, H 4.72, N 2.76

### Example E1

### O-Benzyl-N-{[6-(4-chlorophenyl)imidazo[1,2-a]pyridin-2-yl]carbonyl}tyrosine

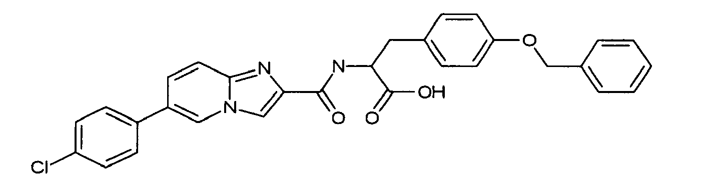

(1) A mixture of 6-(4-chlorophenyl)imidazo[1,2-a]pyridine-2-carboxylic acid (273 mg), methyl O-benzyltyrosinate hydrochloride (340 mg), (3-dimethylaminpropyl)ethyl carbodiimide hydrochloride (230 mg), 1-hydroxybenzotriazole (162 mg), triethylamine (0.5 mL) and N,N-dimethylformamide (15 mL) was stirred overnight at room temperature. Water was added to the reaction solution, which was then extracted with ethyl acetate-tetrahydrofuran. The extract was washed successively with hydrochloric acid, sodium hydroxide aqueous solution and saturated sodium chloride solution, and then dried over anhydrous magnesium sulfate. The solvent was evaporated under reduced pressure, and the residue was purified by silica gel column chromatography and recrystallized from ethyl acetate-hexane to obtain methyl O-benzyl-N-{[6-(4-chlorophenyl)imidazo[1,2-a]pyridin-2-yl]carbonyl}tyrosinate (353 mg) as colorless crystals. ¹H-NMR (300MHz, CDCl₃); δ 3.12-3.27 (2H, m), 3.73 (3H, s), 5.03 (2H, s), 5.03-5.13 (1H, m), 6.90 (2H, d, J=8.7Hz), 7.13 (2H, d, J=8.7Hz), 7.31-7.53 (10H, m), 7.64 (1H, d, J=9.6Hz), 7.79 (1H, d, J=8.4Hz), 8.16 (1H, s), 8.26-8.29 (1H, m)
   Elemental analysis: Calcd. for C₃₁H₂₆ClN₃O₄, : C 68.95, H 4.85, N 7.78; Found: C 68.94, H 4.90, N 7.72
(2) 1 N Sodium hydroxide aqueous solution (1 mL) and water (5 mL) were added at 60°C to a tetrahydrofuran-methanol mixed solution (10 mL-5 mL) of the compound (270 mg) obtained in (1), and stirred for 15 minutes at that temperature. The reaction solution was neutralized with 1 N hydrochloric acid. The precipitated crystals were filtered out and washed successively with water, methanol and diethyl ether to obtain the title compound (200 mg) as colorless crystals. Melting point: 270-271°C
   ¹H-NMR (3.00MHz, DMSO-d₆); δ 3.10-3.20 (2H, m), 4.60-4.70 (1H, m), 5.02 (2H, s), 6.90 (2H, d, J=8.7Hz), 7.15 (2H, d, J=8.7Hz), 7.27-7.45 (5H, m), 7.58 (2H, d, J=8.7Hz), 7.69-7.73 (2H, m), 7.76 (2H, d, J=8.7Hz), 8.23 (1H, d, J=7.8Hz), 8.35 (1H, s), 8.96-9.00 (1H, m), 13.00 (1H, br s) Elemental analysis: Calcd. for C₃₀H₂₄ClN₃O₄, : C 68.50, H 4.60, N 7.99; measured & values: C 68.39, H 4.56, N7.89

### Example E2

### N-{[6-(4-Chlorophenyl)imidazo[1,2-a]pyridin-2-yl]carbonyl}-O-(3-thienylmethyI)-L-tyrosine

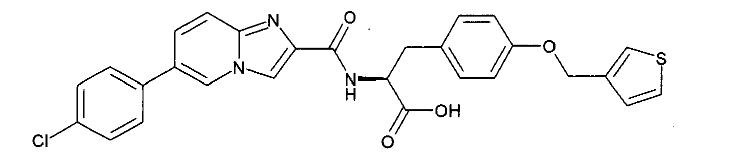

(1) Phosphorus tribromide (1.42 mL, 15 mmol) was added dropwise with ice cooling into a dichloromethane solution of 3-thienylmethanol (1.14 g, 10 mmol), and stirred for 2 hours at room temperature. The reaction solution was poured into ice water, and the organic layer was separated, washed with water, saturated sodium hydrogencarbonate aqueous solution and saturated sodium chloride solution and then dried by addition of magnesium sulfate. The drying agent was removed by filtration, and the filtrate was concentrated under reduced pressure. The residue was dissolved in dimethyl formamide (5 mL). Methyl N-(tert-butoxycarbonyl)-L-tyrosinate (2.06 g, 7 mmol) and potassium carbonate (2.07 g, 15 mmol) were added to this solution and stirred for 4 hours at room temperature. Water was stirred to the reaction solution, which was then stirred for 10 minutes and extracted twice with ethyl acetate. The organic layer was washed with water and saturated sodium chloride solution, and dried by addition of magnesium sulfate. The drying agent was removed by filtration, and the filtrate was concentrated under reduced pressure. The residue was purified by silica gel column chromatography (eluate: ethyl acetate/hexane = 1/10-1/1) to obtain methyl N-(tert-butoxycarbonyl)-O-(3-thienylmethyl)-L-tyrosinate (2.70 g, 98%) as a white solid.
   ¹H-NMR (300MHz, CDCl₃); δ 1.42 (s, 9H), 2.96-3.09 (m, 2H), 3.71 (s, 3H), 4.53-4.55 (m, 1H), 4.94-4.97 (br s, 1H), 5.05 (s, 2H), 6.88-6.92 (d, J=8.7Hz, 2H), 7.02-7.05 (d, J=8.7Hz, 2H), 7.13-7.15 (m, 1H), 7.31-7.36 (m, 2H)
(2) 4 N Hydrogen chloride-ethyl acetate solution (5 mL) was added to an ethyl acetate (15 mL) solution of methyl N-(tert-butoxycarbonyl)-O-(3-thienylmethyl)-L-tyrosinate (2.7 g, 6.90 mmol), and stirred overnight at room temperature. The precipitated white solid was filtered out and washed successively with ethyl acetate and diethyl ether to obtain methyl O-(3-thienylmethyl)tyrosinate hydrochloride (1.83 g, 81%) as a white solid.
   ¹H-NMR (300MHz, DMSO-d₆); δ 2.99-3.33 (m, 2H), 3.68 (s, 3H), 4.19-4.26 (m, 1H), 5.07 (s, 2H), 6.96-6.98 (d, J=8.4Hz, 2H), 7.13-7.18 (m, 3H), 7.54-7.57 (m, 2H), 8.52 (s, 3H)
(3) A mixture of 6-(4-chlorophenyl)imidazo[1,2-a]pyridine-2-carboxylic acid (109 mg, 0.40 mmol), methyl O-(3-thienylmethyl)-L-tyrosinate hydrochloride (98 mg, 0.30 mmol), (3-dimethylaminpropyl)ethyl carbodiimide hydrochloride (115 mg, 0.60 mmol), 1-hydroxybenzotriazole (81 mg, 0.60 mmol), triethylamine (300 µL, 2.1 mmol), N,N-dimethylformamide (2 mL) and dichloromethane (6 mL) was stirred overnight at room temperature. Water was added to the reaction solution, which was then extracted with ethyl acetate. The extract was washed with water and saturated sodium chloride solution, and dried over anhydrous magnesium sulfate. The drying agent was removed by filtration, the solvent was evaporated under reduced pressure, and the residue was purified by silica gel column chromatography (eluate: ethyl acetate/hexane = 1/4-2/1) to obtain methyl N-{[6-(4-chlorophenyl)imidazo[1,2-a]pyridin-2-yl]carbonyl}-O-(3-thienylmethyl)-L-tyrosinate (143 mg, 87%) as a white solid.
   LC-MS 546 [M+H]⁺
   ¹H-NMR (300MHz, DMSO-d₆); δ 3.13-3.15 (d, J=6.9Hz, 2H), 3.66 (s, 3H), 4.71-4.78 (m, 1H), 5.02 (s, 2H), 6.88-6.91 (d, J=8.7Hz, 2H), 7.13-7.18 (m, 3H), 7.51-7.60 (m, 4H), 7.71-7.77 (m, 4H), 8.34 (s, 1H), 8.45-8.48 (d, J=7.8Hz, 1H), 8.98 (s, 1H)
(4) 1 M Sodium hydroxide aqueous solution (530 µL) was added to a tetrahydrofuran-methanol mixed solution (4 mL-2 mL) of methyl N-{[6-(4-chlorophenyl)imidazo[1,2-a]pyridin-2-yl]carbonyl}-O-(3-thienylmethyl)-L-tyrosinate (143 mg, 0.26 mmol), and stirred for 2 hours at 60°C. The reaction solution was neutralized with 1 N hydrochloric acid (550 µL), and the precipitated white solid was filtered out and washed successively with water and diethyl ether. The residue was recrystallized from a tetrahydrofuran-diisopropyl ether mixed solvent to obtain the title compound (100 mg, 72%) as a white solid.
   LC-MS 532 [M+H]⁺
   ¹H-NMR (300MHz, DMSO-d₆); δ 3.05-3.19 (m, 2H), 4.24 (m, 1H), 4.98 (s, 2H), 6.78-6.81 (d, J=8.4Hz, 2H), 7.04-7.06 (d, J=8.4Hz, 2H), 7.13-7.15 (m, 1H), 7.50-7.52 (m, 2H), 7.56-7.59 (d, J=8.7Hz, 2H), 7.68 (s, 2H), 7.74-7.77 (d, J=8.4Hz, 2H), 8.20-8.22 (d, J=6.6Hz, 1H), 8.30 (s, 1H), 8.96 (s, 1H)
   Melting point: 203°C

### Example E3

### O-Benzyl-N-{[6-(4-chlorophenyl)imidazo[1,2-b]pyridazin-2-yl]carbonyl}tyrosine

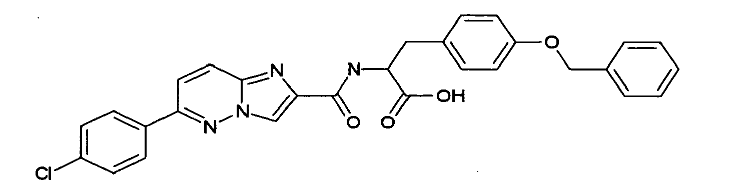

(1) A mixture of 6-(4-chlorophenyl)imidazo[1,2-b]pyridazine-2-carboxylic acid (274 mg), methyl O-benzyltyrosinate hydrochloride (340 mg), (3-dimethylaminpropyl)ethyl carbodiimide hydrochloride (230 mg), 1-hydroxybenzotriazole (162 mg), triethylamine (0.35 mL) and N,N-dimethylformamide (10 mL) was stirred overnight at room temperature. Water was added to the reaction solution, which was then extracted with ethyl acetate. The extract was washed successively with hydrochloric acid and saturated sodium chloride solution, and then dried over anhydrous magnesium sulfate. The solvent was evaporated under reduced pressure, and the residue was purified by silica gel column chromatography and recrystallized from ethyl acetate-hexane to obtain methyl O-benzyl-N-{[6-(4-chlorophenyl)imidazo[1,2-b]pyridazin-2-yl]carbonyl}tyrosinate (335 mg) as colorless crystals.
   ¹H-NMR (300MHz, CDCl₃); δ 3.12-3.28 (2H, m), 3.74 (3H, s), 5.03 (2H, s), 5.02-5.12 (1H, m), 6.90 (2H, d, J=8.7Hz), 7.12 (2H, d, J=8.7Hz), 7.28-7.45 (5H, m), 7.49-7.55 (3H, m), 7.80 (1H, d, J=8.4Hz), 7.92 (2H, d, J=8.7Hz), 7.98 (1H, d, J=9.3Hz), 8.50 (1H, s)
(2) 1 N Sodium hydroxide aqueous solution (1 mL) and water (5 mL) were added at 60°C to a tetrahydrofuran-methanol mixed solution (10 mL-10 mL) of the compound (250 mg) obtained in (1), and stirred for 15 minutes at that temperature. The reaction solution was neutralized with 1 N hydrochloric acid and extracted with ethyl acetate-tetrahydrofuran. The extract was washed with saturated sodium chloride solution, and dried over anhydrous magnesium sulfate. The solvent was evaporated under reduced pressure, and the precipitated crystals were filtered out and washed with methanol-diethyl ether to obtain the title compound (184 mg) as colorless crystals.
   Melting point: 242-243°C ¹H-NMR (300MHz, DMSO-d₆); δ 3.10-3.22 (2H, m), 4.65-4.75 (1H, m), 5.03 (2H, s), 6.90 (2H, d, J=8.4Hz), 7.17 (2H, d, J=8.4Hz), 7.27-7.44 (5H, m), 7.65 (2H, d, J=8.4Hz), 7.95 (1H, d, J=9.6Hz), 8.12 (2H, d, J=8.4Hz), 8.26-8.34 (2H, m), 8.69 (1H, s), 13.00 (1H, br s)
   Elemental analysis: Calcd. for C₂₉H₂₃ClN₄O₄, : C 66.10, H 4.40, N 10.63; Found: C 66.00, H 4.37, N 10.74

### Example E4

### O-Benzyl-N-{[6-(4-chlorophenyl)imidazo[1,2-a]pyrimidin-2-yl]carbonyl }tyrosine

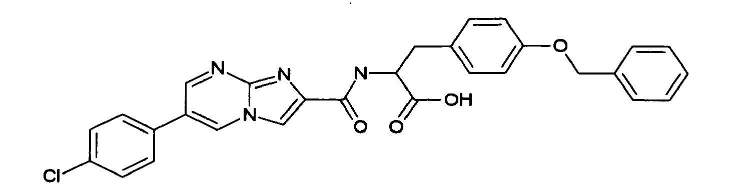

(1) A mixture of 6-(4-chlorophenyl)imidazo[1,2-a]pyrimidine-2-carboxylic acid (170 mg), methyl O-benzyltyrosinate hydrochloride (200 mg),), (3-dimethylaminpropyl)ethyl carbodiimide hydrochloride (143 mg), 1-hydroxybenzotriazole (101 mg), triethylamine (0.26 mL) and N,N-dimethylformamide (10 mL) was stirred overnight at room temperature. Water was added to the reaction solution, which was then extracted with ethyl acetate. The extract was washed with hydrochloric acid and saturated sodium chloride solution, and dried over anhydrous magnesium sulfate. The solvent was evaporated under reduced pressure, and the residue was purified by silica gel column chromatography and recrystallized from ethyl acetate-hexane to obtain methyl O-benzyl-N-{[6-(4-chlorophenyl)imidazo[1,2-a]pyrimidin-2-yl]carbonyl}tyrosinate (158 mg) as colorless crystals.
   ¹H-NMR (300MHz, CDCl₃); δ 3.15-3.27 (2H, m), 3.80 (3H, s), 5.00-5.06 (1H, m), 5.04 (2H, s), 6.42 (1H, d, J=7.8Hz), 6.92 (2H, d, J=8.7Hz), 7.07 (2H, d, J=8.7Hz), 7.30-7.45 (5H, m), 7.50 (2H, d, J=8.7Hz), 7.57 (2H, d, J=8.7Hz), 8.16 (1H, s), 8.92 (1H, d, J=2.7Hz), 9.89 (1H, d, J=2.7Hz)
(2) 1 N Sodium hydroxide aqueous solution (1 mL) and water (10 mL) were added at 60°C to a tetrahydrofuran-methanol mixed solution (10 mL-10 mL) of the compound (130 mg) obtained in (1), and stirred for 15 minutes at that temperature. The reaction solution was neutralized with 1 N hydrochloric acid, and extracted with ethyl acetate-tetrahydrofuran. The extract was washed with saturated sodium chloride solution, and then dried over anhydrous magnesium sulfate. The solvent was evaporated under reduced pressure, and the precipitated crystals were filtered out and washed with methanol-diethyl ether to obtain the title compound (45 mg) as colorless crystals.
   Melting point: 252-253°C
   ¹H-NMR (300MHz, DMSO-d₆); δ 3.10-3.20 (2H, m), 4.55-4.67 (1H, m), 5.02 (2H, s), 6.89 (2H, d, J=8.4Hz), 7.16 (2H, d, J=8.4Hz), 7.26-7.45 (5H, m), 7.62 (2H, d, J=8.4Hz), 7.82 (2H, d, J=8.4Hz), 8.26 (1H, s), 8.35-8.41 (1H, m), 9.03 (1H, d, J=2.4Hz), 9.35 (1H, d, J=2.4Hz), 12.90 (1H, br s)
   Elemental analysis: Calcd. for C₂₉H₂₃ClN₄O₄•0.75 H₂O, : C 64.40, H 4.57, N 10.37; Found: C 64.57, H 4.41, N 10.41

### Example E5

### 4-(Benzylamino)-N-{[6-(4-chlorophenyl)imidazo[1,2-a]pyridin-2-yl]carbonyl}phenylalanine

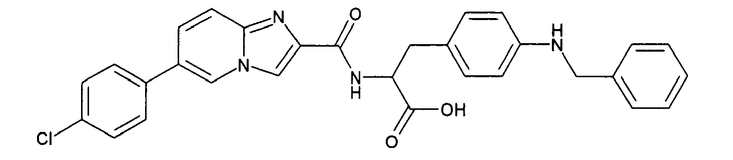

(1) Thionyl chloride (7.25 mL) was added dropwise with ice cooling into methanol (100 mL), and stirred for 30 minutes. 4-nitrophenylalanine (10.5 g, 50 mmol) was added, stirred overnight at room temperature, and then stirred for 3 hours at 40°C. The reaction solution was concentrated under reduced pressure to about 1/3, and diethyl ether was added. The precipitated white solid was filtered out and washed with diethyl ether to obtain methyl 4-nitrophenylalaninate hydrochloride (11.57 g, 89%) as a white solid.
   ¹H-NMR (300MHz, DMSO-d₆); δ 3.22-3.33 (m, 2H), 3.71 (s, 3H), 4.38-4.43 (t, J=6.9Hz, 1H), 7.55-7.58 (d, J=8.7Hz, 2H), 8.20-8.23 (m, 2H), 8.60 (s, 3H)
(2) Triethylamine (3.7 mL, 26.8 mmol) was added dropwise with ice cooling into a mixture of methyl 4-nitrophenylalaninate hydrochloride (5.00 g, 22.3 mmol), di-tert-butyl dicarbonate (5.84 g, 26.8 mmol) and tetrahydrofuran (100 mL), and stirred for 2 hours at room temperature. The reaction solution was concentrated under reduced pressure, diluted with ethyl acetate, and washed with water and saturated sodium chloride solution. The organic layer was dried by addition of magnesium sulfate. The drying agent was removed by filtration, and the filtrate was concentrated under reduced pressure. The residue was purified by silica gel column chromatography (eluate: ethyl acetate/hexane = 1/10-1/1) to obtain methyl N-(tert-butoxycarbonyl)-4-nitrophenylalaninate (5.68 g, 79%) as a white solid.
   ¹H-NMR (300MHz, CDCl₃); δ 1.41 (s, 9H), 3.09-3,15 (m, 1H), 3.24-3.31 (m, 1H), 3.74 (s, 3H), 4.63-4.65 (d, J=7.2Hz, 1H), 5.03-5.05 (d, J=6.0Hz, 1H), 7.30-7.33 (d, J=8.7Hz, 2H), 8.15-8.17 (d, J=7.2Hz, 2H)
(3) A mixture of methyl N-(tert-butoxycarbonyl)-4-nitrophenylalaninate (5.68 g, 17.5 mmol), iron powder (4.89 g, 87.5 mmol), calcium chloride (971 mg, 8.75 mmol), ethanol (250 mL) and water (62 mL) was heated and refluxed for 1.5 hours. The reaction solution was diluted with tetrahydrofuran, and Celite filtered. The filtrate was concentrated under reduced pressure, and the precipitated white solid was filtered out and washed with water to obtain methyl 4-amino-N-(tert-butoxycarbonyl)phenylalaninate (4.72 g, 92%) as a white solid.
   LC-MS 317 [M+Na]⁺
   ¹H-NMR (300MHz, DMSO-d₆); δ 1.34 (s, 9H), 2.62-2.81 (m, 2H), 3.58 (s, 3H), 3.98-4.06 (m, 1H), 4.89 (s, 2H), 6.44-6.47 (d, J=8.4Hz, 2H), 6.84-6.86 (d, J=8.1Hz, 2H), 7.14-7.17 (d, J=7.8Hz, 1H)
(4) A tetrahydrofuran (100 mL) solution of methyl 4-amino-N-(tert-butoxycarbonyl)phenylalaninate (4.72 g, 16.1 mmol), benzaldehyde (1.79 mL, 17.7 mmol) and acetic acid (100 µL) was heated and refluxed for 3 hours. Sodium triacetoxyboride (7.18 g, 32.2 mmol) was added with ice cooling, and stirred overnight at room temperature. Water was added to the reaction solution, which was then stirred for 30 minutes. This was extracted twice with ethyl acetate, and the organic layer was washed successively with saturated sodium bicarbonate solution and saturated sodium chloride solution and dried by addition of magnesium sulfate. The drying agent was removed by filtration, and the filtrate was concentrated under reduced pressure. The residue was recrystallized from an ethyl acetate-hexane mixed solvent to obtain methyl 4-(benzylamino)-N-(tert-butoxycarbonyl)phenylalaninate (5.17 g, 84%) as a white solid.
   ¹H-NMR (300MHz, CDC1₃); δ 1.42 (s, 9H), 2.96-2.98 (d, J=5.1Hz, 2H), 3.71 (s, 3H), 3.99 (m, 1H), 4.31 (s, 2H), 4.49-4.52 (d, J=7.8Hz, 1H), 4.93-4.95 (d, J=7.5Hz, 1H), 6.55-6.58 (d, J=8.4Hz, 2H), 6.90-6.93 (d, J=8.7Hz, 2H), 7.30-7.36 (m, 5H)
(5) 4 N Hydrogen chloride-ethyl acetate solution (8 mL) was added to an ethyl acetate (50 mL) solution of 4-(benzylamino)-N-(tert-butoxycarbonyl)phenylalanine (5.17 g, 1.34 mmol), and stirred for 3 hours at room temperature and overnight at 60°C. The precipitated yellow solid was filtered out and washed with ethyl acetate to obtain methyl 4-(benzylamino)phenylalaninate hydrochloride (4.80 g, 100%) as a yellow solid.
   ¹H-NMR (300MHz, DMSO-d₆); δ 2.92-3.07 (m, 2H), 3.66 (s, 3H), 4.32 (s, 2H), 6.80 (br s, 2H), 7.00-7.03 (d, J=8.4Hz, 2H), 7.23-7.41 (m, 5H), 8.52 (s, 3H)
(6) A mixture of 6-(4-chlorophenyl)imidazo[1,2-a]pyridine-2-carboxylic acid (164 mg, 0.60 mmol), methyl 4-(benzylamino)phenylalaninate hydrochloride (143 mg, 0.50 mmol), (3-dimethylaminopropyl)ethyl carbodiimide hydrochloride (192 mg, 1.00 mmol), 1-hydroxybenzotriazole (135 mg, 1.00 mmol), triethylamine (300 µL, 2.1 mmol), N,N-dimethylformamide (2 mL) and dichloromethane (6 mL) was stirred overnight at room temperature. Water was added to the reaction solution, which was then extracted with chloroform. The extract was washed with water and saturated sodium chloride solution and then dried over anhydrous magnesium sulfate. The drying agent was removed by filtration, the solvent was evaporated under reduced pressure, and the residue was purified by silica gel column chromatography (eluate: ethyl acetate/hexane = 1/4-1/1) to obtain methyl 4-(benzylamino)-N-{[6-(4-chlorophenyl)imidazo[1,2-a]pyridi-2-yl]carbonyl}phenylalaninate (60 mg, 22%) as a white solid.
   LC-MS 539 [M+H]⁺
   ¹H-NMR (300MHz, DMSO-d₆); δ 3.00-3.02 (d, J=5.7Hz, 2H), 3.64 (s, 3H), 4.19-4.21 (d, J=6.0Hz, 2H), 4.63-4.70 (m, 1H), 6.08-6.12 (t, J=6.OHz, 1H), 6.46-6.49 (d, J=8.4Hz, 2H), 6.89-6.92 (d, J=8.4Hz, 2H), 7.17-7.22 (m, 1H), 7.57-7.60 (d, J=8.7Hz, 2H), 7.71-7.77 (m, 4H), 8.29-8.34 (m, 2H), 8.98 (s, 1H)
(7) 1 M Sodium hydroxide aqueous solution (222 µL) was added to a tetrahydrofuran-methanol mixed solution (6 mL-2 mL) of methyl 4-(benzylamino)-N-{[6-(4-chlorophenyl)imidazo[1,2-a]pyridin-2-yl]carbonyl}phenylalaninate (60 mg, 0.11 mmol), and stirred for 30 minutes at 60°C. The reaction solution was neutralized with 1 N hydrochloric acid (250 µL), and the precipitated white solid was filtered out and washed successively with water and diethyl ether to obtain the title compound (45 mg, 78%) as a white solid.
   LC-MS 525 [M+H]⁺
   ¹H-NMR (300MHz, DMSO-d₆); δ 2.95-3.02 (m, 2H), 4.20 (s, 2H), 4.58-4.64 (m, 1H), 6.07 (br s, 1H), 6.45-6.48 (d, J=8.4Hz, 2H), 6.89-6.91 (d, J=8.4Hz, 2H), 7.17-7.34 (m, 5H), 7.57-7.60 (d, J=8.7Hz, 2H), 7.64-7.77 (m, 4H), 8.07-8.10 (d, J=8.1Hz, 1H), 8.34 (s, 1H), 8.98 (s, 1H), 12.90 (br s, 1 H)
   Melting point: 260°C
   Elemental analysis: Calcd. for C₃₀H₂₅N₄O₃Cl, : C 68.63, H 4.80, N 10.67; Found: C 68.35, H 4.75, N 10.70

### Example E6

### N-{[6-(4-Chlorophenyl)imidazo[1,2-a]pyridin-2-yl]carbonyl}-O-(2-fluorobenzyl)tyrosine

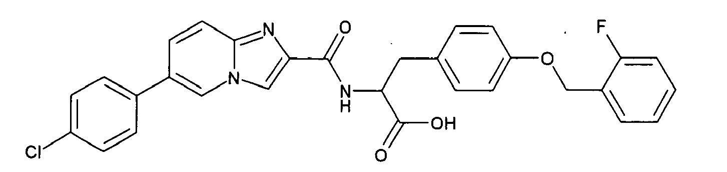

(1) A mixture of methyl N-(tert-butoxycarbonyl)tyrosinate (0.59 g, 2 mmol), 2-fluorobenzyl bromide (454 mg, 2.4 mmol), potassium carbonate (415 mg) and dimethyl formamide (3 mL) was stirred overnight at room temperature. Ethyl acetate and water were added to separate the solution, and the organic layer was washed twice with water and once with saturated sodium chloride solution, and dried over magnesium sulfate. This was concentrated under reduced pressure after removal of the drying agent by filtration. The residue was purified by silica gel column chromatography (eluate: ethyl acetate/hexane = 1/10-1/1) to obtain methyl N-(tert-butoxycarbonyl)-O-(2-fluorobenzyl)tyrosinate (694 mg, 90%) as a white solid.
   ¹H-NMR (300MHz, CDCl₃); δ 1.42 (s, 9H), 2.96-3.09 (m, 2H), 3.71 (s, 3H), 4.51-4.58 (m, 1H), 4.95-4.97 (d, J=7.8Hz, 1H), 5.11 (s, 2H), 6.90-6.93 (d, J=8.7Hz, 2H), 7.03-7.12 (m, 4H), 7.27-7.35 (m, 1H), 7.47-7.53 (m, 1H)
(2) 4 N Hydrogen chloride-ethyl acetate solution (1 mL) was added to an ethyl acetate (3 mL) solution of methyl N-(tert-butoxycarbonyl)-O-(2-fluorobenzyl) tyrosinate (694 mg, 1.80 mmol), and stirred overnight at room temperature. The precipitated white solid was filtered out and washed successively with ethyl acetate and diethyl ether to obtain methyl O-(2-fluorobenzyl)tyrosinate hydrochloride (497 mg, 81%) as a white solid.
   LC-MS 304 [M+H]⁺
   ¹H-NMR (300MHz, DMSO-d₆); δ 3.00-3.13 (m, 2H), 3.68 (s, 3H), 4.22-4.26 (t, J=6.3Hz, 1H), 5.12 (s, 2H), 6.98-7.01 (d, J=8.7Hz, 2H), 7.15-7.18 (d, J=8.4Hz, 2H), 7.22-7.29 (m, 2H), 7.39-7.47 (m, 1H), 7.53-7.58 (m, 1H), 8.49 (s, 3H)
(3) A mixture of 6-(4-chlorophenyl)imidazo[1,2-a]pyridine-2-carboxylic acid (164 mg, 0.60 mmol), methyl O-(2-fluorobenzyl)tyrosinate hydrochloride (170 mg, 0.50 mmol), (3-dimethylaminopropyl)ethyl carbodiimide hydrochloride (192 mg, 1.00 mmol), 1-hydroxybenzotriazole (135 mg, 1.00 mmol), triethylamine (300 µL, 2.1 mmol), N,N-diemthylformamide (2 mL) and dichloromethane (6 mL) was stirred overnight at room temperature. Water was added to the reaction solution, which was then extracted with ethyl acetate. The extract was washed with water and saturated sodium chloride solution and then dried over anhydrous magnesium sulfate. The drying agent was removed by filtration, the solvent was evaporated under reduced pressure, and the residue was recrystallized from a chloroform-hexane mixed solvent to obtain methyl N-{[6-(4-chlorophenyl)imidazo[1,2-a]pyridin-2-yl]carbonyl]-O-(2-fluorobenzyl)tyrosinate (229 mg, 82%) as a white solid.
   LC-MS 558 [M+H]⁺
   ¹H-NMR (300MHz, DMSO-d₆); δ 3.14-3.16 (d, J=7.2Hz, 2H), 3.66 (s, 3H), 4.71-4.78 (m, 1H), 5.07 (s, 2H), 6.91-6.94 (d, J=8.7Hz, 2H), 7.17-7.26 (m, 4H), 7.36-7.44 (m, 1H), 7.50-7.60 (m, 3H), 7. 71-7.74 (m, 4H), 8.34 (s, 1H), 8.46-8.49 (d, J=8.1 Hz, 1H), 8.98 (s, 1H)
(4) 1 M Sodium hydroxide aqueous solution (538 µL) was added to a tetrahydrofuran-methanol mixed solution (6 mL-2 mL) of methyl N-{[6-(4-chlorophenyl)imidazo[1,2-a]pyridin-2-yl]carbonyl}-O-(2-fluorobenzyl)tyrosinate (150 mg, 0.27 mmol), and stirred for 30 minutes at 60°C. The reaction solution was neutralized with 1 N hydrochloric acid (550 µL), and the precipitated white solid was filtered out and washed successively with water and diethyl ether to obtain the title compound (121 mg, 83%) as a white solid.
   LC-MS 544 [M+H]⁺
   ¹H-NMR (300MHz, DMSO-d₆); δ 3.14-3.16 (m, 2H), 4.65-4.72 (m, 1H), 5.06 (s, 2H), 6.91-6.94 (d, J=8.7Hz, 2H), 7.15-7.26 (m, 4H), 7.36-7.44 (m, 1H), 7.50-7.60 (m, 3H), 7.71-7.77 (m, 4H), 8.22-8.25 (d, J=8.1Hz, 1H), 8.35 (s, 1H), 8.97 (s, 1H), 12.90 (br s, 1H)
   Melting point: 273°C
   Elemental analysis: Calcd. for C₃₀H₂₃N₃O₄ClF (cont. 0.2 mol H₂O), : C 65.80, H 4.31, N 7.67; Found: C 65.88, H 4.30, N 7.71

### Example E7

### N-{[6-(4-Chlorophenyl)imidazo[1,2-a]pyridin-2-yl]carbonyl}-O-(4-fluorobenzyl)tyrosine

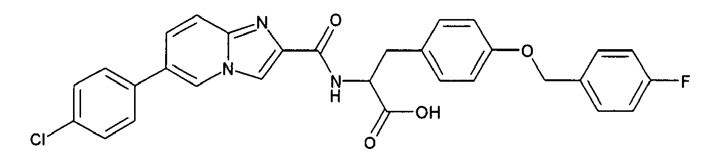

(1) A mixture of methyl N-(tert-butoxycarbonyl)tyrosinate (0.59 g, 2.0 mmol), 4-fluorobenzyl bromide (454 mg, 2.4 mmol), potassium carbonate (415 mg) and dimethyl formamide (3 mL) was stirred overnight at room temperature. Ethyl acetate and water were added to separate the solution, and the organic layer was washed twice with water and once with saturated sodium chloride solution, and dried over magnesium sulfate. This was concentrated under reduced pressure after removal of the drying agent by filtration. The residue was purified by silica gel column chromatography (eluate: ethyl acetate/hexane = 1/10-1/1) to obtain methyl N-(tert-butoxycarbonyl)-O-(4-fluorobenzyl)tyrosinate (720 mg, 93%) as a white solid.
   ¹H-NMR (300MHz, CDCl₃); δ 1.42 (s, 9H), 2.96-3.09 (m, 2H), 3.71 (s, 3H), 4.53-4.55 (m, 1H), 4.94-4.99 (m, 3H), 6.87-6.90 (d, J=8.7Hz, 2H), 7.03-7.11 (m, 4H), 7.37-7.41 (m, 2H)
(2) 4 N Hydrogen chloride-ethyl acetate solution (1 mL) was added to an ethyl acetate (3 mL) solution of methyl N-(tert-butoxycarbonyl)-O-(4-fluorobenzyl)tyrosinate (720 mg, 1.87 mmol), and stirred overnight at room temperature. The precipitated white solid was filtered out and washed successively with ethyl acetate and diethyl ether to obtain methyl O-(4-fluorobenzyl)tyrosinate hydrochloride (568 mg, 89%) as a white solid.
   LC-MS 304 [M+H]⁺
   ¹H-NMR (300MHz, DMSO-d₆); δ 2.99-3.12 (m, 2H), 3.68 (s, 3H), 4.21-4.26 (m, 1H), 5.00 (s, 2H), 6.96-6.99 (d, J=8.4Hz, 2H), 7.13-7.25 (m, 4H), 7.47-7.52 (m, 2H), 8.45 (s, 3H)
(3) A mixture of 6-(4-chlorophenyl)imidazo[1,2-a]pyridine-2-carboxylic acid (164 mg, 0.60 mmol), methyl O-(4-fluorobenzyl)tyrosinate hydrochloride (170 mg, 0.50 mmol), (3-dimethylaminopropyl)ethyl carbodiimide hydrochloride (192 mg, 1.00 mmol), 1-hydroxybenzotriazole (135 mg, 1.00 mmol), triethylamine (300 µL, 2.1 mmol), N,N-diemthylformamide (2 mL) and dichloromethane (6 mL) was stirred overnight at room temperature. Water was added to the reaction solution, which was then extracted with ethyl acetate. The extract was washed with water and saturated sodium chloride solution and then dried over anhydrous magnesium sulfate. The drying agent was removed by filtration, the solvent was evaporated under reduced pressure, and the residue was recrystallized from a chloroform-hexane mixed solvent to obtain methyl N-{[6-(4-chlorophenyl)imidazo[1,2-a]pyridin-2-yl]carbonyl}-O-(4-fluorobenzyl)tyrosinate (201 mg, 72%) as a white solid
   LC-MS 558 [M+H]⁺
   ¹H-NMR (300MHz, DMSO-d₆); δ 3.14-3.16 (d, J=6.9Hz, 2H), 3.66 (s, 3H), 4.71-4.79 (m, 1H), 5.01 (s, 2H), 6.89-6.92 (d, J=8.4Hz, 2H), 7.16-7.22 (m, 4H), 7.44-7.49 (m, 2H), 7.57-7.60 (d, J=8.1 Hz, 2H), 7.71-7.77 (m, 4H), 8.3 5 (s, 1 H), 8,46-8.49 (d, J=8.1Hz, 1 H), 8.98 (s, 1 H)
(4) 1 M Sodium hydroxide aqueous solution (538 µL) was added to a tetrahydrofuran-methanol mixed solution (6 mL-2 mL) of methyl N-{[6-(4-chlorophenyl)imidazo[1,2-a]pyridin-2-yl]carbonyl}-O-(4-fluorobenzyl)tyrosinate (150 mg, 0.27 mmol), and stirred for 30 minutes at 60°C. The reaction solution was neutralized with 1 N hydrochloric acid (550 µL), and the precipitated white solid was filtered out and washed successively with water and diethyl ether to obtain the title compound (119 mg, 82%) as a white solid.
   LC-MS 544 [M+H]⁺
   ¹H-NMR (300MHz, DMSO-d₆); δ 3.08-3.24 (m, 2H), 4.65-4.72 (m, 1H), 5.01 (s, 2H), 6.88-6.91 (d, J=8.4Hz, 2H), 7.14-7.22 (m, 4H), 7.44-7.49 (m, 2H), 7.57-7.60 (d, J=8.4Hz, 2H), 7.71-7.77 (m, 4H), 8.22-8.24 (d, J=8.1 Hz, 1H), 8.35 (s, 1H), 8.98 (s, 1H), 12.90 (br s, 1H)
   Melting point; 267°C
   Elemental analysis: Calcd. for C₃₀H₂₃N₃O₄ClF, : C 66.24, H 4.26, N 7.72; Found: C 66.08, H 4.20, N 7.79

### Example E8

### O-Benzyl-N-{[7-(4-chlorophenyl)imidazo[1,2-a]pyridin-2-yl]carbonyl}tyrosine

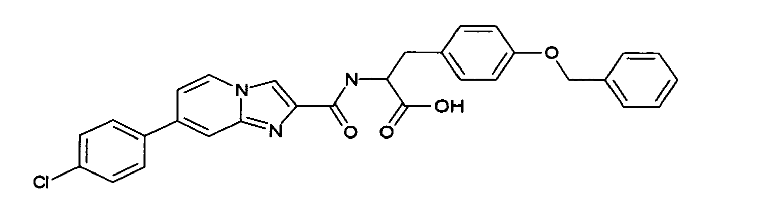

(1) A mixture of 7-(4-chlorophenyl)imidazo[1,2-a]pyridine-2-carboxylic acid (286 mg), methyl O-benzyltyrosinate hydrochloride (338 mg), (3-dimethylaminopropyl)ethyl carbodiimide hydrochloride (242 mg), 1-hydroxybenzotriazole (170 mg), triethylamine (0.52 mL) and N,N-dimethylformamide (8 mL) was stirred overnight at room temperature. Water was added to the reaction solution, which was then extracted with ethyl acetate. The extract was washed with hydrochloric acid and saturated sodium chloride solution and then dried over anhydrous magnesium sulfate. The solvent was evaporated under reduced pressure, and the residue was purified by silica gel column chromatography and recrystallized from ethyl acetate-hexane to obtain methyl O-benzyl-N-{[7-(4-chlorophenyl)imidazo[1,2-a]pyridin-2-yl]carbonyl}tyrosinate (380 mg) as colorless crystals.
   ¹H-NMR (300MHz, CDCl₃); δ 3.12-3.27 (2H, m), 3.73 (3H, s), 5.02 (2H, s), 5.00-5.11 (1H, m), 6.90 (2H, d, J=8.7Hz), 7.09 (1H, dd, J=1.8, 7.2Hz), 7.13 (2H, d, J=8.7Hz), 7.30-7.45 (5H, m), 7.47 (2H, d, J=8.7Hz), 7.58 (2H, d, J=8.7Hz), 7.74 (1H, s), 7.80 (1H, d, J=8.4Hz), 8.13 (1H, s), 8.18 (1H, dd, J=0.3, 7.2Hz)
   Elemental analysis: Calcd. for C₃₁H₂₆ClN₃O₄, : C 68.95, H 4.85, N 7.78; Found: C 68.88, H 4.76, N 7.78
(2) 1 N Sodium hydroxide aqueous solution (1 mL) and water (10 mL) were added at 60°C to a tetrahydrofuran-methanol mixed solution (7 mL-7 mL) of the compound (300 mg) obtained in (1), and stirred at that temperature for 15 minutes. The reaction solution was neutralized with 1 N hydrochloric acid, and the precipitated crystals were filtered out and washed with N,N-dimethylformamide-water to obtain the title compound (234 mg) as colorless crystals.
   Melting point: 255°C
   ¹H-NMR (300MHz, DMSO-d₆); δ 3.10-3.20 (2H, m), 4.65-4.75 (1H, m), 5.03 (2H, s), 6.90 (2H, d, J=8.7Hz), 7.15 (2H, d, J=8.7Hz), 7.27-7.45 (6H, m), 7.58 (2H, d, J=8.7Hz), 7.87 (2H, d, J=8.7Hz), 7.93 (1H, s), 8.17 (1H, d, J=7.8Hz), 8.39 (1H, s), 8.65 (1H, d, J=6.9Hz), 13.00 (1H, br s)
   Elemental analysis: Calcd. for C₃₀H₂₄ClN₃O₄, 68.50, H 4.60, N 7.99; Found: C 68.34, H 4.53, N 8.04

### Example E9

### O-Benzyl-N-{[5-(4-chlorophenyl)imidazo[1,2-a]pyridin-2-yl]carbonyl}tyrosine

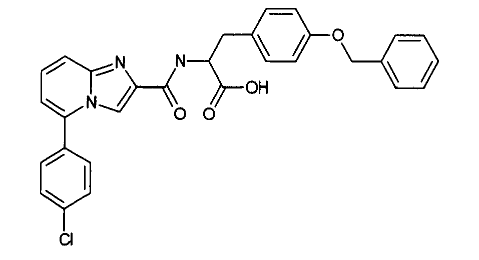

(1) A mixture of 5-(4-chlorophenyl)imidazo[1,2-a]pyridine-2-carboxylic acid (190 mg), methyl O-benzyltyrosinate hydrochloride (225 mg), (3-dimethylaminopropyl)ethyl carbodiimide hydrochloride (161 mg), 1-hydroxybenzotriazole (114 mg), triethylamine (0.29 mL) and N,N-dimethylformamide (8 mL) was stirred overnight at room temperature. Water was added to the reaction solution, which was then extracted with ethyl acetate. The extract was washed with hydrochloric acid and saturated sodium chloride solution and then dried over anhydrous magnesium sulfate. The solvent was evaporated under reduced pressure, and the residue was purified by silica gel column chromatography and recrystallized from ethyl acetate-hexane to obtain methyl O-benzyl-N-{[5-(4-chlorophenyl)imidazo[1,2-a]pyridin-2-yl]carbonyl}tyrosinate (286 mg) as colorless crystals.
   ¹H-NMR (300MHz, CDC1₃); δ 3.10-3.25 (2H, m), 3.72 (3H, s), 5.02 (2H, s), 5.02-5.10 (1H, m), 6.79 (1H, dd, J=0.9, 6.9Hz), 6.89 (2H, d, J=8.7Hz), 7.12 (2H, d, J=8.7Hz), 7.29-7.45 (6H, m), 7.50-7.62 (5H, m), 7.79 (1H, d, J=8.4Hz), 8.16 (1H, s)
   Elemental analysis: Calcd. for C₃₁H₂₆ClN₃O₄, : C 68.95, H 4.85, N 7.78; Found: C 69.05, H 5.11, N 7.63
(2) 1 N Sodium hydroxide aqueous solution (1 mL) and water (10 mL) were added at 60°C to a tetrahydrofuran-methanol mixed solution (7 mL-7 mL) of the compound (200 mg) obtained in (1), and stirred at that temperature for 15 minutes. The reaction solution was neutralized with 1 N hydrochloric acid, and extracted with diethyl ether. The extract was washed with saturated sodium chloride solution and then dried over anhydrous magnesium sulfate. The solvent was evaporated under reduced pressure, and the residue was recrystallized from methanol-diethyl ether to obtain the title compound (124 mg) as colorless crystals.
   Melting point: 218°C
   ¹H-NMR (300MHz, DMSO-d₆); δ 3.05-3.20 (2H, m), 4.60-4.75 (1H, m), 5.02 (2H, s), 6.89 (2H, d, J=8.4Hz), 7.03 (1H, d, J=6.6Hz), 7.14 (2H, d, J=8.4Hz), 7.27-7.53 (6H, m), 7.65-7.73 (3H, m), 7.78 (2H, d, J=8.7Hz), 8.01 (1H, s), 8.28 (1H, d, J=8.1Hz), 12.58 (1H, br s)
   Elemental analysis: Calcd. for C₃₀H₂₄ClN₃O₄, : C 68.50, H 4.60, N 7.99; Found: C 68.40, H 4.53, N 7.99

### Example E10

### N-{[6-(4-Chlorophenyl)imidazo[1,2-a]pyridin-2-yl]carbonyl}-O-(3-fluorobenzyl)tyrosine

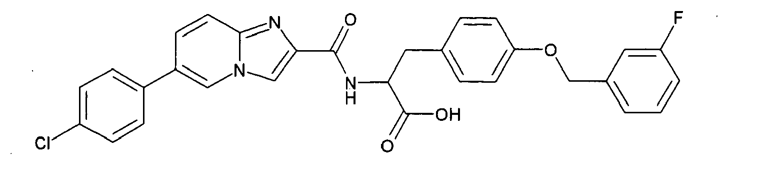

(1) A mixture of methyl N-(tert-butoxycarbonyl)tyrosinate (0.59 g, 2.0 mmol), 3-fluorobenzyl bromide (454 mg, 2.4 mmol), potassium carbonate (415 mg) and N,N-dimethylformamide (3 mL) was stirred overnight at room temperature. Ethyl acetate and water were added to separate the solution, and the organic layer was washed twice with water and once with saturated sodium chloride solution, and dried over magnesium sulfate. This was concentrated under reduced pressure after removal of the drying agent by filtration. The residue was purified by silica gel column chromatography (eluate: ethyl acetate/hexane = 1/10-1/1) to obtain methyl N-(tert-butoxycarbonyl)-O-(3-fluorobenzyl)tyrosinate (609 mg, 76%) as a white solid.
   ¹H-NMR (300MHz, CDCl₃); δ 1.42 (s, 9H), 2.95-3.09 (m, 2H), 3.71 (s, 3H), 4.53-4.56 (d, J=7.2Hz, 1H), 4.95-4.98 (d, J=8.4Hz, 1H), 5.03 (s, 2H), 6.87-6.90 (d, J=8.7Hz, 2H), 6.97-7.06 (m, 3H), 7.13-7.19 (m, 2H), 7.31-7.38 (m,1H)
(2) 4 N Hydrogen chloride-ethyl acetate solution (1 mL) was added to an ethyl acetate (3 mL) solution of methyl N-(tert-butoxycarbonyl)-O-(3-fluorobenzyl)tyrosinate (609 mg, 1.51 mmol), and stirred overnight at room temperature. The precipitated white solid was filtered out and washed successively with ethyl acetate and diethyl ether to obtain methyl O-(3-fluorobenzyl) tyrosinate hydrochloride (440 mg, 91%) as a white solid.
   LC-MS 304 [M+H]⁺
   ¹H-NMR (300MHz, DMSO-d₆); δ 2.98-3.11 (m, 2H), 3.68 (s, 3H), 4.22-4.26 (t, J=6.3Hz, 1H), 5.12 (s, 2H), 6.97-7.00 (d, J=8.7Hz, 2H), 7.14-7.19 (m, 3H), 7.25-7.29 (m, 2H), 7.41-7.48 (m, 1H), 8.42 (s, 3H)
(3) A mixture of 6-(4-chlorophenyl)imidazo[1,2-a]pyridine-2-carboxylic acid (164 mg, 0.60 mmol), methyl O-(3-fluorobenzyl)tyrosinate hydrochloride (170 mg, 0.50 mmol), (3-dimethylaminopropyl)ethyl carbodiimide hydrochloride (192 mg, 1.00 mmol), 1-hydroxybenzotriazole (135 mg, 1.00 mmol), triethylamine (300 µL, 2.1 mmol), N,N-diemthylformamide (2 mL) and dichloromethane (6 mL) was stirred overnight at room temperature. Water was added to the reaction solution, which was then extracted with ethyl acetate. The extract was washed with water and saturated sodium chloride solution and then dried over anhydrous magnesium sulfate. The drying agent was removed by filtration, the solvent was evaporated under reduced pressure, and the residue was recrystallized from a chloroform-hexane mixed solvent to obtain methyl N-{[6-(4-chlorophenyl)imidazo[1,2-a]pyridin-2-yl]carbonyl}-O-(3-fluorobenzyl)tyrosinate (246 mg, 88%) as a white solid.
   LC-MS 558 [M+H]⁺
   ¹H-NMR (300MHz, DMSO-d₆); δ 3.13-3.16 (m, 2H), 3.66 (s, 3H), 4.73-4.75 (m, 1H), 5.06 (s, 2H), 6.90-6.92 (d, J=8.4Hz, 2H), 7.14-7.27 (m, 5H), 7.38-7.42 (m, 1H), 7.57-7.60 (d, J=8.7Hz, 2H), 7.71-7.77 (m, 4H), 8.34 (s, 1H), 8.47-8.49 (d, J=8.1Hz, 1H), 8.98 (s, 1H)
(4) 1 M Sodium hydroxide aqueous solution (538 µL) was added to a tetrahydrofuran-methanol mixed solution (6 mL-2 mL) of methyl N-{[6-(4-chlorophenyl)imidazo[1,2-a]pyridin-2-yl]carbonyl}-O-(3-fluorobenzyl)tyrosinate (150 mg, 0.27 mmol), and stirred for 30 minutes at 60°C. The reaction solution was neutralized with 1 N hydrochloric acid (550 µL), and the precipitated white solid was filtered out and washed successively with water and diethyl ether to obtain the title compound (122 mg, 84%) as a white solid.
   LC-MS 544 [M+H]⁺
   ¹H-NMR (300MHz, DMSO-d₆); δ 3.13-3.15 (m, 2H), 4.64-4.70 (m, 1H), 5.06 (s, 2H), 6.89-6.92 (d, J=8.7Hz, 2H), 7.11-7.17 (m, 3H), 7.23-7.27 (m, 2H), 7.38-7.45 (m, 1H), 7.57-7.60 (d, J=8.4Hz, 2H), 7.71-7.77 (m, 4H), 8.22-8.25 (d, J=8.1Hz, 1H), 8.98 (s, 1H), 12.80 (br s, 1H)
   Melting point: 252°C
   Elemental analysis: Calcd. for C₃₀H₂₃N₃O₄ClF, : C 66.24, H 4.26, N 7.72; Found: C 66.01, H 4.25, N 7.69

### Example E11

### N-{[6-(4-Chlorophenyl)imidazo[1,2-a]pyridin-2-yl]carbonyl}-4-fluorophenylalanine

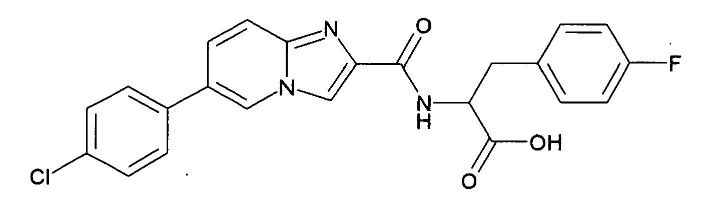

(1) Thionyl chloride (0.78 mL) was added dropwise with ice cooling into methanol (12 mL), and stirred for 30 minutes. 4-fluorophenylalanine (1.0 g, 5.46 mmol) was added, stirred overnight at room temperature, and then stirred for 3 hours at 40°C. The reaction solution was concentrated under reduced pressure to about 1/3, and diethyl ether was added. The precipitated white solid was filtered out and washed with diethyl ether to obtain methyl 4-fluorophenylalaninate hydrochloride (1.19 g, 94%) as a white solid.
   ¹H-NMR (300MHz, DMSO-d₆); δ 3.07-3.21 (m, 2H), 3.68 (s, 3H), 4.25-4.29 (t, J=6.6Hz, 1H), 7.14-7.20 (m, 2H), 7.27-7.32 (m, 2H), 8.64 (s, 3H)
(2) A mixture of 6-(4-chlorophenyl)imidazo[1,2-a]pyridine-2-carboxylic acid (164 mg, 0.60 mmol), methyl 4-fluorophenylalaninate hydrochloride (117 mg, 0.50 mmol), (3-dimethylaminopropyl)ethyl carbodiimide hydrochloride (192 mg, 1.00 mmol), 1-hydroxybenzotriazole (135 mg, 1.00 mmol), triethylamine (300 µL, 2.1 mmol), N,N-diemthylformamide (2 mL) and dichloromethane (6 mL) was stirred overnight at room temperature. Water was added to the reaction solution, which was then extracted with ethyl acetate. The extract was washed with water and saturated sodium chloride solution and then dried over anhydrous magnesium sulfate. The drying agent was removed by filtration, the solvent was evaporated under reduced pressure, and the residue was purified by basic silica gel column chromatography (eluate: chloroform/acetone = 20/1) and recrystallized from a chloroform-hexane mixed solvent to obtain methyl N-{[6-(4-chlorophenyl)imidazo[1,2-a]pyridin-2-yl]carbonyl}-4-fluorophenylalaninate (192 mg, 85%) as a white solid.
   LC-MS 452 [M+H]⁺
   ¹H-NMR (300MHz, DMSO-d₆); δ 3.19-3.22 (m, 2H), 3.66 (s, 3H), 4.73-4.81 (m, 1H), 7.05-7.11 (m, 2H), 7.27-7.32 (m, 2H), 7.57-7.60 (d, J=8.7Hz, 2H), 7.71-7.77 (m, 4H), 8.32 (s, 1H), 8.59-8.61 (d, J=8.1 Hz, 1H), 8.97 (s, 1H)
(3) 1 M Sodium hydroxide aqueous solution (600 µL) was added to a tetrahydrofuran-methanol mixed solution (6 mL-2 mL) of methyl N-{[6-(4-chlorophenyl)imidazo[1,2-a]pyridin-2-yl]carbonyl}-4-fluorophenylalaninate (135 mg, 0.30 mmol), and stirred for 30 minutes at 60°C. The reaction solution was neutralized with 1 N hydrochloric acid (610 µL), and the precipitated white solid was filtered out and washed successively with water and diethyl ether to obtain the title compound (106 mg, 81%) as a white solid.
   LC-MS 438 [M+H]⁺
   ¹H-NMR (300MHz, DMSO-d₆); δ 3.14-3.26 (m, 2H), 4.67-4.74 (m, 1H), 7.05-7.11 (t, J=8.7Hz, 2H), 7.25-7.30 (m, 2H), 7.57-7.60 (d, J=8.7Hz, 2H), 7.71-7.77 (m, 4H), 8.33-8.35 (m, 2H), 8.97 (s, 1H), 13.0 (br s, 1H)
   Melting point: 266°C
   Elemental analysis: Calcd. for C₂₃H₁₇N₃O₃ClF, : C 63.09, H 3.91, N 9.60; Found: C 63.18, H 3.97, N 9.68

### Example E12

### O-Benzyl-N-{[6-(4-chlorophenyl)imidazo[1,2-a]pyridin-2-yl]carbonyl}-N-methyltyrosine

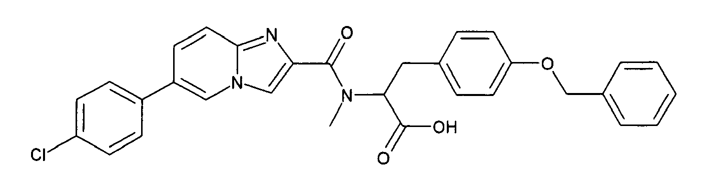

(1) A mixture of methyl O-benzyltyrosinate hydrochloride (4.79 mg, 15 mmol), 2-nitrobenzenesulfonyl chloride (3.99 g, 18 mmol), triethylamine (5.22 mL, 37.5 mmol) and tetrahydrofuran (150 mL) was stirred for 2 hours at room temperature. Water was added, and the mixture was stirred for 30 minutes and extracted twice with ethyl acetate. The organic layer was washed with sodium bicarbonate solution and saturated sodium chloride solution and dried over magnesium sulfate. The drying agent was removed by filtration, and the filtrate was concentrated under reduced pressure. The residue was purified by silica gel column chromatography (eluate: ethyl acetate/hexane = 1/10-1/1) to obtain methyl O-benzyl-N-[(2-nitrophenyl)sulfonyl]tyrosinate (7.05 g, 100%) as a yellow oil.
   LC-MS 471 [M+H]⁺
   ¹H-NMR (300MHz, CDCl₃); δ 2.98-3.14 (m, 2H), 3.53 (s, 3H), 4.38-4.45 (m, 1H), 5.01 (s, 2H), 5.95-5.98 (d, J=8.7Hz, 1H), 6.79-6.82 (d, J=8.7Hz, 2H), 7.00-7.03 (d, J=8.7Hz, 2H), 7.30-7.45 (m, 5H), 7.62-7.70 (m, 2H), 7.81-7.86 (m, 1H), 7.93-7.99 (m, 1H)
(2) A toluene solution (6.49 mL) of 40% diethyl azodicarboxylate was added dropwise with ice cooling into a tetrahydrofuran (50 mL) solution of methyl O-benzyl-N-[(2-nitrophenyl)sulfonyl]tyrosinate (3.5 g, 7.45 mmol), methanol (604 µL) and triphenylphosphine (3.91 g, 14.9 mmol). The reaction solution was stirred overnight at room temperature. The reaction solution was concentrated under reduced pressure, and the residue was purified by silica gel column chromatography (eluate: ethyl acetate/hexane = 1/10-1/1) to obtain methyl O-benzyl-N-methyl-N-[(2-nitrophenyl)sulfonyl]tyrosinate (3.61 g, 100%) as a yellow oil.
   LC-MS 485 [M+H]⁺
   ¹H-NMR (300MHz, CDCl₃); δ 2.86-2.94 (m, 1H), 3.03 (s, 3H), 3.18-3.31 (m, 1H), 3.64 (s, 3H), 4.83-4.89 (m, 1H), 5.01 (s, 2H), 6.81-6.84 (d, J=8.7Hz, 2H), 7.09-7.12 (d, J=8.7Hz, 2H), 7.31-7.45 (m, 5H), 7.50-7.75 (m, 3H), 7.75-7.78 (d, J=8.4Hz, 1H)
(3) Potassium carbonate (3.09 g, 22.35 mmol) and benzene thiol (918 µL, 8.94 mmol) were added to a dimethyl formamide (80 mL) solution of methyl O-benzyl-N-methyl-N-[(2-nitrophenyl)sulfonyl]tyrosinate (3.61 g, 7.45 mmol), and stirred for 2 hours at room temperature. This was extracted twice with ethyl acetate after addition of water. The organic layer was washed with water and saturated sodium chloride solution, and dried by addition of magnesium sulfate. The drying agent was removed by filtration, and the filtrate was concentrated under reduced pressure. The residue was purified by basic silica gel column chromatography (eluate: ethyl acetate/hexane = 1/10-1/1) to obtain methyl O-benzyl-N-methyltyrosinate (1.98 g, 88%) as a colorless oil.
   LC-MS 300 [M+H]⁺
   ¹H-NMR (300MHz, CDCl₃); δ 2.34 (s, 3H), 2.88-2.91 (m, 1H), 3.38-3.43 (m, 1H), 3.66 (s, 3H), 4.16-4.24 (m, 1H), 5.04 (s, 2H), 6.89-6.92 (d, J=8.7Hz, 2H), 7.06-7.11 (d, J=8.7Hz, 2H), 7.29-7.41 (m, 5H)
(4) A mixture of 6-(4-chlorophenyl)imidazo[1,2-a]pyridine-2-carboxylic acid (164 mg, 0.60 mmol), methyl O-benzyl-N-methyltyrosinate (150 mg, 0.50 mmol), (3-dimethylaminopropyl)ethyl carbodiimide hydrochloride (192 mg, 1.00 mmol), 1-hydroxybenzotriazole (135 mg, 1.00 mmol), triethylamine (300 µL, 2.1 mmol), N,N-dimethylformamide (2 mL) and dichloromethane (6 mL) was stirred overnight at room temperature. Water was added to the reaction solution, which was then extracted with ethyl acetate. The extract was washed with water and saturated sodium chloride solution and then dried over anhydrous magnesium sulfate. The drying agent was removed by filtration, the solvent was evaporated under reduced pressure, and the residue was purified by basic silica gel column chromatography (eluate: ethyl acetate/hexane = 1/10-1/1) and recrystallized from a chloroform-hexane mixed solvent to obtain methyl O-benzyl-N-{[6-(4-chlorophenyl)imidazo[1,2-a]pyridin-2-yl]carbonyl}-N-methyltyrosinate (200 mg, 72%) as a white solid.
   LC-MS 554 [M+H]⁺
   ¹H-NMR (300MHz, DMSO-d₆); δ 2.88 (s, 1.5H), 3.01-3.18 (m, 2H), 3.23 (s, 1.5H), 3.68-3.71 (d, J=10.2Hz, 3H), 4.92-5.12 (m, 3H), 6.73-6.76 (d, J=8.1Hz, 1H), 6.91-6.93 (d, J=8.1Hz, 1H), 7.07-7.10 (d, J=8.1Hz, 1H), 7.18-7.20 (d, J=8.1Hz, 1H), 7.30-7.43 (m, 5H), 7.57-7.59 (d, J=6.6Hz, 2H), 7.69-7.79 (m, 4H), 8.10 (s, 0.5H), 8.28 (s, 0.5H), 8.90-8.96 (d, J=15.OHz, 1H)
(5) 1 M Sodium hydroxide aqueous solution (600 µL) was added to a tetrahydrofuran-methanol mixed solution (6 mL-2 mL) of methyl O-benzyl-N-{[6-(4-chlorophenyl)imidazo[1,2-a]pyridin-2-yl)carbonyl}-N-methyltyrosinate (165 mg, 0.30 mmol), and stirred for 30 minutes at 60°C. The reaction solution was neutralized with 1 N hydrochloric acid (610 µL), and the precipitated white solid was filtered out and washed successively with water and diethyl ether to obtain the title compound (125 mg, 78%) as a white solid.
   LC-MS 540 [M+H]⁺
   ¹H-NMR (300MHz, DMSO-d₆); δ 2.88 (s, 1.5H), 2.99-3.27 (m, 2H), 3.31 (s, 1.5H), 4.98-5.04 (d, J=12.1Hz, 2H), 5.11-5.16 (m, 1H), 6.74-6.77 (d, J=8.7Hz, 2H), 6.90-6.93 (d, J=8.7Hz, 1H), 7.07-7.10 (d, J=8.4Hz, 1H), 7.18-7.21 (d, J=8.4Hz, 1H), 7.29-7.43 (m, 5H), 7.56-7.59 (d, J=8.4Hz, 2H), 7.64-7.76 (m, 4H), 8.09 (s, 0.5H), 8.26 (s, 0.5H), 8.92-8.96 (d, J=11.4Hz, 1H), 12.80 (br s, 1H)
   Melting point: 216°C
   Elemental analysis: Calcd. for C₃₁H₂₆N₃O₄Cl, : C 68.95, H 4.85, N 7.78; Found: C 68.78, H 4.79, N 7.80

### Example E13

### N-{[6-(4-Chlorophenyl)imidazo[1,2-a]pyridin-2-yl]carbonyl}-N-{4-[(4-fluorobenzyl)oxy]benzyl}glycine

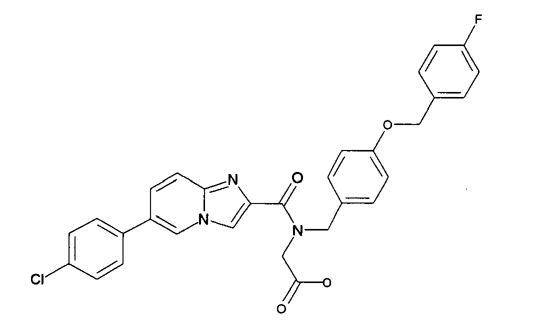

(1) Ethyl 1-bromoacetate (256 µL, 2.2 mmol) was added to a tetrahydrofuran (10 mL) solution of benzylamine (500 mg, 2.0 mmol) and triethylamine (699 µL, 5.0 mmol), and stirred overnight. Ethyl acetate and water were added to the reaction solution, and the organic layer was isolated. The organic layer was washed with water and saturated sodium chloride solution, and dried over magnesium sulfate. The drying agent was removed by filtration, and the filtrate was concentrated under reduced pressure. The residue was purified by silica gel column chromatography (eluate: ethyl acetate/hexane = 1/10-1/1) to obtain ethyl N-{4-[(4-fluorobenzyl)oxy]benzyl}glycinate (242 mg, 38%) as a colorless oil.
   ¹H-NMR (300MHz, CDCl₃); δ 1.25-1.30 (t, J=7.2Hz, 3H), 3.39 (s, 2H), 3.74 (s, 2H), 4.15-4.23 (q, J=7.2Hz, 2H), 5.01 (s, 2H), 6.91-6.94 (d, J=8.7Hz, 2H), 7.03-7.11 (m, 2H), 7.22-7.26 (m, 2H), 7.37-7.42 (m, 2H)
(2) A mixture of 6-(4-chlorophenyl)imidazo[1,2-a]pyridine-2-carboxylic acid (164 mg, 0.60 mmol), ethyl N-{4-[(4-fluorobenzyl)oxy]benzyl}glycinate (159 mg, 0.50 mmol), (3-dimethylaminopropyl)ethyl carbodiimide hydrochloride (192 mg, 1.00 mmol), 1-hydroxybenzotriazole (135 mg, 1.00 mmol), triethylamine (300 µL, 2.1 mmol), N,N-dimethylformamide (2 mL) and dichloromethane (6 mL) was stirred overnight at room temperature. Water was added to the reaction solution, which was then extracted with ethyl acetate. The extract was washed with water and saturated sodium chloride solution and then dried over anhydrous magnesium sulfate. The drying agent was removed by filtration, the solvent was evaporated under reduced pressure, and the residue was purified by basic silica gel column chromatography (eluate: ethyl acetate/hexane = 1/10-1/1) and recrystallized from a chloroform-hexane mixed solvent to obtain ethyl N-{[6-(4-chlorophenyl)imidazo[1,2-a]pyridin-2-yl]carbonyl}-N-{4-[(4-fluorobenzyl)oxy]benzyl}glycinate (240 mg, 84%) as a white solid.
   LC-MS 572 [M+H]⁺
   ¹H-NMR (300MHz, DMSO-d₆); δ 1.12-1.20 (m, 3H), 3.99 (s, 1H), 4.03-4.13 (m, 2H), 4.65 (s, 1H), 4.83 (s, 1H), 5.07 (s, 2H), 5.39 (s, 1H), 6.96-6.99 (d, J=8.7Hz, 2H), 7.18-7.24 (t, J=9.0Hz, 2H), 7.28-7.33 (m, 2H), 7.47-7.52 (m, 2H), 7.57-7.60 (d, J=8.4Hz, 2H), 7.64-7.74 (m, 4H), 8.43 (s, 1H), 8.99-9.01 (d, J=6.9Hz, 1H)
(3) 1 M Sodium hydroxide aqueous solution (700 µL) was added to a tetrahydrofuran-methanol mixed solution (6 mL-2 mL) of ethyl N-{[6-(4-chlorophenyl)imidazo[1,2-a]pyridin-2-yl]carbonyl}-N-{4-[(4-fluorobenzyl)oxy]benzyl}glycinate (200 mg, 0.35 mmol), and stirred for 1 hour at 60°C. The reaction solution was neutralized with 1 N hydrochloric acid (710 µL), and the precipitated white solid was filtered out and washed successively with water and diethyl ether to obtain the title compound (169 mg, 90%) as a white solid.
   LC-MS 544 [M+H]⁺
   ¹H-NMR (300MHz, DMSO-d₆); 83.91 (s, 1H), 4.64 (s, 1H), 4,80 (s, 1H), 5.07 (s, 2H), 5.36 (s, 1H), 6.97-7.00 (d, J=8.1Hz, 2H), 7.19-7.33 (m, 4H), 7.47-7.52 (m, 2H), 7.57-7.60 (d, J=8.4Hz, 2H), 7.64-7.77 (m, 4H), 8.42-8.43 (d, J=2.7Hz, 1H), 8.99-9.01 (d, J=5.7Hz, 1H), 12.6 (br s, 1H)
   Melting point: 206°C
   Elemental analysis: Calcd. for C₃₀H₂₃N₃O₄ClF, : C 66.24, H 4.26, N 7.72; Found: C 66.14, H 4.26, N 7.75

### Example E14

### N-[2-[4-(Benzyloxy)phenyl]-1-(1H-tetrazol-5-yl)ethyl]-6-(4-chlorophenyl)imidazo[1,2-a]pyridine-2-carboxamide

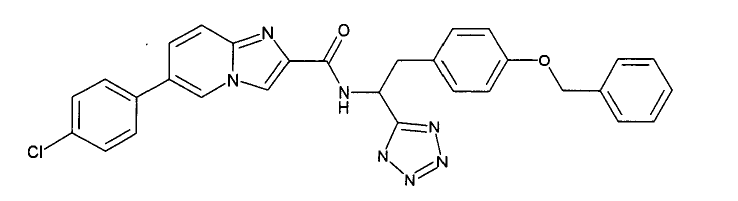

(1) 1 M Sodium hydroxide aqueous solution (16 mL) was added to a methanol (32 mL) solution of methyl O-benzyl-N-(tert-butoxycarbonyl)tyrosinate (3.09 g, 8.02 mmol), and stirred for 3 hours at room temperature. The reaction solution was concentrated under reduced pressure, and 1 N hydrochloric acid (18 mL) was added. The precipitated white solid was filtered out and washed with water and diethyl ether to obtain O-benzyl-N-(tert-butoxycarbonyl)tyrosine (2.8 g, 94%) as a white solid.
   ¹H-NMR (300MHz, CDCl₃); δ 1.43 (s, 9H), 3.00-3.17 (m, 2H), 4.54-4.56 (d, J=6.0Hz, 1H), 4.91-4.93 (d, J=5.7Hz, 1H), 5.04 (s, 2H), 6.91-6.93 (d, J=8.4Hz, 2H), 7.09-7.12 (d, J=8.4Hz, 2H), 7.30-7.61 (m, 5H)
(2) A mixture of O-benzyl-N-(tert-butoxycarbonyl)tyrosine (1.5 g, 4.0 mmol), 2-cyanoethylamine (360 µL, 4.8 mmol), (3-dimethylaminopropyl)ethyl carbodiimide hydrochloride (1.62 g, 4.8 mmol), 1-hydroxybenzotriazole (1.09 g, 4.8 mmol), triethylamine (2.56 mL) and N,N-dimethylformamide (10 mL) was stirred overnight at room temperature. Water was added and stirred for 30 minutes, and the mixture was extracted twice with ethyl acetate. The organic layer was washed with water and saturated sodium chloride solution, and dried by addition of magnesium sulfate. The drying agent was removed by filtration, and the filtrate was concentrated under reduced pressure. The residue was recrystallized from an ethyl acetate-hexane mixed solvent to obtain O-benzyl-Nα-(tert-butoxycarbonyl)-N-(2-cyanoethyl)tyrosinamide (1.57 g, 92%) as a white solid.
   LC-MS 446 [M+Na]⁺
   ¹H-NMR (300MHz, DMSO-d₆); δ 1.30 (s, 9H), 2.57-2.71 (m, 3H), 2.84-2.90 (m, 1H), 3.21-3.37 (m, 2H), 4.05-4.09 (m, 1H), 5.06 (s, 2H), 6.87-6.92 (m, 3H), 7.14-7.17 (d, J=8.4Hz, 2H), 7.29-7.45 (m, 5H), 8.27-8.29 (m, 1H)
(3) Diethyl azodicarboxylate (1.92 mL, 12.2 mmol) was added dropwise with ice cooling into a tetrahydrofuran (100 mL) solution of O-benzyl-Nα-(tert-butoxycarbonyl)-N-(2-cyanoethyl)tyrosinamide (1.57 g, 3.71 mmol), trimethyl silylazide (1.68 mL, 12.2 mmol) and triphenyl phosphine (3.2 g, 12.2 mmol), and stirred overnight at room temperature. 5% diammonium cerium (IV) sulfate aqueous solution was added to the reaction solution, which was then stirred for 1 hour and extracted twice with ethyl acetate. The organic layer was washed with water and saturated sodium chloride solution and then dried by addition of anhydrous magnesium sulfate. The drying agent was removed by filtration, and the filtrate was concentrated under reduced pressure. The residue was purified by silica gel column chromatography (eluate: ethyl acetate/hexane = 1/2-1/1) and recrystallized from an ethyl acetate-hexane mixed solvent to obtain tert-butyl {2-[4-(benzyloxy)phenyl]-1-[1-(2-cyanoethyl)-1H-tetrazol-5-yl]ethyl}carbamate (423 mg, 26%) as a white solid.
   LC-MS 471 [M+Na]⁺
   ¹H-NMR (300MHz, DMSO-d₆); δ 1.28 (s, 9H), 2.89-3.22 (m, 4H), 4.60-4.64 (t, J=6.6Hz, 2H), 5.06-5.13 (m, 3H), 6.88-6.91 (d, J=8.4Hz, 2H), 7.14-7.17 (d, J=8.7Hz, 2H), 7.29-7.43 (m, 5H), 7.83-7.86 (d, J=7.8Hz, 1H)
(4) 4 N Hydrogen chloride-ethyl acetate solution (1 mL) was added to an ethyl acetate (3 mL)-tetrahydrofuran (3 mL) solution of tert-butyl {2-[4-(benzyloxy)phenyl]-1-[1-(2-cyanoethyl)-1H-tetrazol-5-yl]ethyl}carbamate (423 mg, 0.94 mmol), and stirred overnight at room temperature. The precipitated white solid was filtered out and washed successively with ethyl acetate and diethyl ether to obtain 3-(5-{1-amino-2-[4-(benzyloxy)phenyl]ethyl}-1H-tetrazol-1-yl)propanenitrile hydrochloride (242 mg, 67%) as a white solid
   LC-MS 349 [M+H]⁺
   ¹H-NMR (300MHz, DMSO-d₆); δ 2.65-2.90 (m, 2H), 3.10-3.25 (m, 2H), 4.31-4.52 (m, 2H), 5.06 (s, 2H), 5.14-5.18 (m, 1H), 6.91-7.00 (m, 4H), 7.33-7.41 (m, 5H), 8.79 (s, 3H)
(5) A mixture of 6-(4-chlorophenyl)imidazo[1,2-a]pyridine-2-carboxylic acid (111 mg, 0.40 mmol), 3-(5-{1-amino-2-[4-(benzyloxy)phenyl]ethyl}-1H-tetrazol-1-yl)propanenitrile hydrochloride (121 mg, 0.31 mmol), (3-dimethylaminpropyl)ethyl carbodiimide hydrochloride (121 mg, 0.60 mmol), 1-hydroxybenzotriazole (81 mg, 0.60 mmol), triethylamine (300 µL, 2.1 mmol), N,N-dimethylformamide (2 mL) and dichloromethane (6 mL) was stirred overnight at room temperature. Water was added to the reaction solution, which was then extracted with ethyl acetate. The extract was washed with water and saturated sodium chloride solution, and dried over anhydrous magnesium sulfate. The drying agent was removed by filtration, the solvent was evaporated under reduced pressure, and the residue was purified by basic silica gel column chromatography (eluate: ethyl acetate/hexane = 1/10-1/1) to obtain N-{2-[4-(benzyloxy)phenyl]-1-[1-(2-cyanoethyl)-1H-tetrazol-5-yl]ethyl}-6-(4-chlorophenyl)imidazo[1,2-a]pyridine-2-carboxamide (177 mg, 94%) as a white solid.
   LC-MS 603 [M+H]⁺
   1H-NMR (300MHz, DMSO-d₆); δ 2.97-3.16 (m, 2H), 3.27-3.52 (m, 2H), 4.64-4.75 (m, 2H), 5.03 (s, 2H), 5.61-5.66 (m, 1H), 6.87-6.90 (d, J=8.4Hz, 2H), 7.20-7.23 (d, J=8.4Hz, 2H), 7.28-7.43 (m, 5H), 7.57-7.60 (d, J=8.4Hz, 2H), 7.71-7.76 (m, 4H), 8.34 (s, 1H), 8.96 (s, 1H), 9.21-9.24 (s, 1H)
(6) 1 M Sodium hydroxide aqueous solution (587 µL) was added to a tetrahydrofuran-methanol mixed solution (6 mL-2 mL) of N-{2-[4-(benzyloxy)phenyl]-1-[1-(2-cyanoethyl)-1H-tetrazol-5-yl]ethyl}-6-(4-chlorophenyl)imidazo[1,2-a]pyridine-2-carboxamide (177 mg, 0.29 mmol), and stirred for 1 hour at 60°C. The reaction solution was neutralized with 1 N hydrochloric acid (600 µL), and extracted twice with ethyl acetate. The organic layer was washed with saturated sodium chloride solution and dried over magnesium sulfate. The drying agent was removed by filtration, and the filtrate was concentrated under reduced pressure. The residue was recrystallized from an ethyl acetate-hexane mixed solvent to obtain the title compound (90.5 mg, 56%) as a white solid.
   LC-MS 550 [M+H]⁺
   ¹H-NMR (300MHz, DMSO-d₆); δ 3.32-3.36 (m, 2H), 5.01 (s, 2H), 5.57-5.65 (m, 1H), 6.86-6.89 (d, J=8.7Hz, 2H), 7.14-7.17 (d, J=8.7Hz, 2H), 7.30-7.42 (m, 5H), 7.57-7.60 (d, J=8.4Hz, 2H), 7.71-7.70 (m, 4H), 8.34 (s, 1H), 8.90-8.93 (d, J=8.4Hz, 1H), 8.98 (s, 1H)
   Melting point: 241°C
   Elemental analysis: Calcd. for C₃₀H₂₄N₇O₂Cl, : C 65.51, H 4.40, N 17.83; Found: C 65.47, H 4.54, N 17.97

### Example E15

### N-[2-[4-(Benzyloxy)phenyl]-1-(1H-tetrazol-5-yl)ethyl]-6-(4-chlorophenyl)imidazo[1,2-b]pyridazine-2-carboxamide

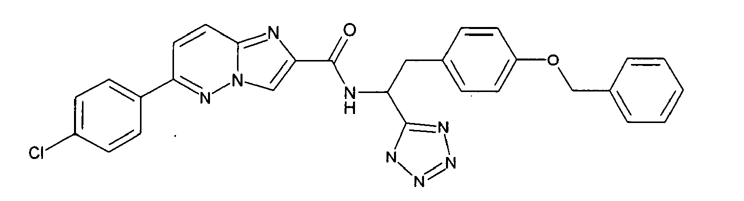

(1) A mixture of 6-(4-chlorophenyl)imidazo[1,2-b]pyridazine-2-carboxylic acid (111 mg, 0.40 mmol), 3-(5-{ 1-amino-2-[4-(benzyloxy)phenyl]ethyl}-1H-tetrazol-1-yl)propanenitrile hydrochloride (121 mg, 0.31 mmol), ), (3-dimethylaminpropyl)ethyl carbodiimide hydrochloride (121 mg, 0.60 mmol), 1-hydroxybenzotriazole (81 mg, 0.60 mmol), triethylamine (300 µL, 2.1 mmol), N,N-dimethylformamide (2 mL) and dichloromethane (6 mL) was stirred overnight at room temperature. Water was added to the reaction solution, which was then extracted with ethyl acetate. The extract was washed with water and saturated sodium chloride solution, and dried over anhydrous magnesium sulfate. The drying agent was removed by filtration, the solvent was evaporated under reduced pressure, and the residue was purified by basic silica gel column chromatography (eluate: ethyl acetate/hexane = 1/10-1/1) to obtain N-{2-[4-(benzyloxy)phenyl]-1-[1-(2-cyanoethyl)-1H-tetrazol-5-yl]ethyl}-6-(4-chlorophenyl)imidazo[1,2-b]pyridazine-2-carboxamide (189 mg, 100%) as a white solid.
   LC-MS 604 [M+H]⁺
   ¹H-NMR (300MHz, DMSO-d₆); δ 2.96-3.17 (m, 2H), 3.27-3.33 (m, 1H), 3.48-3.56 (m, 1H), 4.65-4.73 (m, 2H), 5.03 (s, 2H), 5.60-5.67 (m, 1H), 6.87-6.90 (d, J=8.7Hz, 2H), 7.21-7.24 (d, J=8.7Hz, 2H), 7.27-7.42 (m, 5H), 7.64-7.67 (d, J=8.7Hz, 2H), 7.93-7.96 (d, J=9.6Hz, 1H), 8.10-8.13 (d, J=8.7Hz, 2H), 8.27-8.30 (d, J=9.6Hz, 1H), 8.69 (s, 1H), 9.31-9.34 (d, J=7.8Hz, 1H)
(2) 1 M Sodium hydroxide aqueous solution (662 µL) was added to a tetrahydrofuran-methanol mixed solution (6 mL-2 mL) of N-{2-[4-(benzyloxy)phenyl]-1-[1-(2-cyanoethyl)-1H-tetrazol-5-yl]ethyl}-6-(4-chlorophenyl)imidazo[1,2-b]pyridazine-2-carboxamide (200 mg, 0.33 mmol), and stirred for 1 hour at 60°C. The reaction solution was neutralized with 1 N hydrochloric acid (680 µL), and extracted twice with ethyl acetate. The organic layer was washed with saturated sodium chloride solution and dried over magnesium sulfate. The drying agent was removed by filtration, and the filtrate was concentrated under reduced pressure. The residue was recrystallized from an ethyl acetate-hexane mixed solvent to obtain the title compound (116 mg, 64%) as a white solid
   LC-MS 551 [M+H]⁺
   ¹H-NMR (300MHz, DMSO-d₆); δ 3.33-3.38 (m, 2H), 5.01 (s, 2H), 5.60-5.68 (m, 1H), 6.87-6.90 (d, J=8.4Hz, 2H), 7.16-7.19 (d, J=8.7Hz, 2H), 7.29-7.42 (m, 5H), 7.64-7.67 (d, J=8.7Hz, 2H), 7.94-7.97 (d, J=9.9Hz, 1H), 8.11-8.14 (d, J=8.7Hz, 2H), 8.28-8.31 (d, J=9.6Hz, 1H), 8.69 (s, 1H), 9.03-9.06 (d, J=8.4Hz, 1H), 16.4 (br s)
   Melting point: 186°C
   Elemental analysis: Calcd. for C₂₉H₂₃N₈O₂Cl (cont. 0.2 mol H₂O), : C 62.80, H 4.25, N 20.20; Found: C 62.83, H 4.32, N 20.27

### Example E16

### O-Benzyl-N-{[7-(4-chlorophenyl)pyrazolo[1,5-a]pyrimidin-2-yl]carbonyl}tyrosine

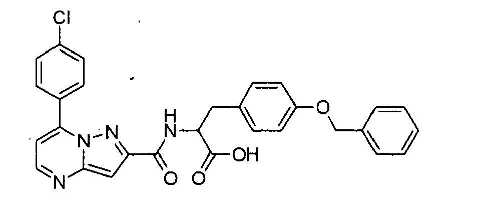

(1) A mixture of 7--(4-chlorophenyl)pyrazolo[1,5-a]pyrimidine-2-carboxylic acid (273 mg), methyl O-benzyltyrosinate hydrochloride (321 mg), (3-dimethylaminpropyl)ethyl carbodiimide hydrochloride (230 mg), 1-hydroxybenzotriazole (162 mg), triethylamine (0.42 mL) and N,N-dimethylformamide (7 mL) was stirred overnight at room temperature. Water was added to the reaction solution, which was then extracted with ethyl acetate. The extract was washed with hydrochloric acid and saturated sodium chloride solution, and dried over anhydrous magnesium sulfate. The solvent was evaporated under reduced pressure, and the residue was purified by silica gel column chromatography and recrystallized from ethyl acetate-hexane to obtain methyl O-benzyl-N-{[7-(4-chlorophenyl)pyrazolo[1,5-a]pyrimidin-2-yl]carbonyl}tyrosinate (394 mg) as colorless crystals.
   ¹H-NMR (300MHz, CDCl₃); δ 3.19 (2H, d, J=5.7Hz), 3.74 (3H, s), 5.00-5.10 (1H, m), 5.04 (2H, s), 6.88 (2H, d, J=8.7Hz), 6.99 (1H, d, J=4.2Hz), 7.08 (2H, d, J=8.7Hz), 7.31-7.46 (7H, m), 7.57 (2H, d, J=8.7Hz), 7.98 (2H, d, J=8.7Hz), 8.58 (1H, d, J=4.2Hz)
   Elemental analysis: Calcd. for C₃₀H₂₅ClN₄O₄, : C 66.60, H 4.66, N 10.36; Found: C 66.66, H 4.65, N 10.34
(2) 1 N Sodium hydroxide aqueous solution (1 mL) and water (5 mL) were added at 60°C to a tetrahydrofuran-methanol mixed solution (10 mL-5 mL) of the compound (300 mg) obtained in (1), and stirred at that temperature for 15 minutes. The reaction solution was neutralized with 1 N hydrochloric acid. The precipitated crystals were filtered out, washed with water, and recrystallized from methanol to obtain the title compound (113 mg) as colorless crystals.
   Melting point: 102-103°C
   ¹H-NMR (300MHz, DMSO-d₆); δ 3.05-3.20 (2H, m), 4.55-4.65 (1H, m), 5.02 (2H, s), 6.88 (2H, d, J=8.4Hz), 7.17 (1H, s), 7.21 (2H, d, J=8.4Hz), 7.26-7.45 (6H, m), 7.71 (2H, d, J=8.4Hz), 8.22 (2H, d, J=8.4Hz), 8.28 (1H, d, J=8.1 Hz), 8.70 (1H, d, J=4.2Hz), 12.80 (1H, br s)
   Elemental analysis: Calcd. for C₂₉H₂₃ClN₄O₄•0.5 H₂O, : C 64.99, H 4.51, N 10.45; Found: C 64.97, H 4.45, N 10.58

### Example E17

### N-{[6-(4-Chlorophenyl)imidazo[1,2-a]pyridin-2-yl]carbonyl }-O-(cyclopropylmethyl)tyrosine

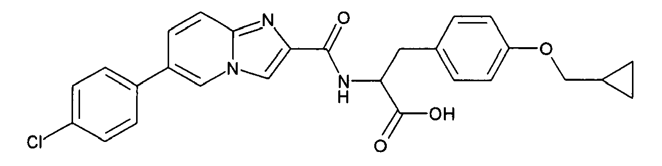

1) A mixture of methyl N-(tert-butoxycarbonyl)tyrosinate (0.59 g, 2 mmol), (bromomethyl)cyclopropane (233 µL, 2.4 mmol), potassium carbonate (415 mg) and dimethylformamide (3 mL) was stirred overnight at 60°C. Water and ethyl acetate were added to separate the solution, and the organic layer was washed twice with water and once with saturated sodium chloride solution and dried over magnesium sulfate. The drying agent was removed by filtration, and the filtrate was concentrated under reduced pressure. The residue was purified by silica gel column chromatography (eluate: ethyl acetate/hexane = 1/10-1/1) to obtain methyl N-(tert-butoxycarbonyl)-O-(cyclopropylmethyl)tyrosinate (333 mg, 48%) as a white solid. ¹H-NMR (300MHz, CDCl₃); δ 0.33-0.36 (m, 2H), 0.61-0.67 (m, 2H), 1.19-1.34 (m, 1H), 1.42 (s, 9H), 2.95-3.08 (m, 2H), 3.71 (s, 3H), 3.76-3.78 (d, J=6.9Hz, 2H), 4.52-4.55 (d, J=7.2Hz, 1H), 4.93-4.96 (d, J=7.8Hz, 1H), 6.81-6.84 (d, J=8.4Hz, 2H), 7.00-7.03 (d, J=8.4Hz, 2H)
(2) 4 N Hydrogen chloride-ethyl acetate solution (0.5 mL) was added to an ethyl acetate (2 mL) solution of methyl N-(tert-butoxycarbonyl)-O-(cyclopropylmethyl)tyrosinate (333 mg, 0.95 mmol), and stirred overnight at room temperature. The precipitated white solid was filtered out and washed successively with ethyl acetate and diethyl ether to obtain methyl O-(cyclopropylmethyl)tyrosinate hydrochloride (225 mg, 83%) as a white solid.
   LC-MS 250 [M+H]⁺
   ¹H-NMR (300MHz, DMSO-d₆); δ 0.28-0.33 (m, 2H), 0.53-0.60 (m, 2H), 1.14-1.24 (m, 1H), 2.97-3.11 (m, 2H), 3.68 (s, 3H), 3.77-3.80 (d, J=6.9Hz, 2H), 4.20-4.24 (m, 1H), 6.86-6.89 (d, J=8.7Hz, 2H), 7.10-7.13 (d, J=8.4Hz, 2H), 8.43 (s, 3H)
(3) A mixture of 6-(4-chlorophenyl)imidazo[1,2-a]pyridine-2-carboxylic acid (164 mg, 0.60 mmol), methyl O-(cyclopropylmethyl)tyrosinate hydrochloride (143 mg, 0.50 mmol), (3-dimethylaminopropyl)ethyl carbodiimide hydrochloride (192 mg, 1.00 mmol), 1-hydroxybenzotriazole (135 mg, 1.00 mmol), triethylamine (300 µL, 2.1 mmol), N,N-diemthylformamide (2 mL) and dichloromethane (6 mL) was stirred overnight at room temperature. Water was added to the reaction solution, which was then extracted with chloroform. The extract was washed with water and saturated sodium chloride solution and then dried over anhydrous magnesium sulfate. The drying agent was removed by filtration, the solvent was evaporated under reduced pressure, and the residue was purified by silica gel column chromatography (eluate: ethyl acetate/hexane = 1/4-1/1) and recrystallized from a chloroform-hexane mixed solvent to obtain methyl N-{[6-(4-chlorophenyl)imidazo[1,2-a]pyridin-2-yl]carbonyl}-O-(cyclopropylmethyl)tyrosinate (186 mg, 74%) as a white solid.
   LC-MS 504 [M+H]⁺
   ¹H-NMR (300MHz, DMSO-d₆); δ 0.25-0.30 (m, 2H), 0.50-0.56 (m, 2H), 1.13-1.19 (m, 1H), 3.12-3.14 (d, J=6.9Hz, 2H), 3.66 (s, 3H), 3.73-3.75 (d, J=6.9Hz, 2H), 4.70-4.77 (m, 1H), 6.79-6.82 (d, J=8.4Hz, 2H), 7.12-7.15 (d, J=8.7Hz, 2H), 7.57-7.60 (d, J=8.4Hz, 2H), 7.71-7.77 (m, 4H), 8.34 (s, 1H), 8.43-8.46 (d, J=8.1Hz, 1H), 8.98 (s, 1H)
(4) 1 M Sodium hydroxide aqueous solution (734 µL) was added to a tetrahydrofuran-methanol mixed solution (4 mL-2 mL) of methyl N-{[6-(4-chlorophenyl)imidazo[1,2-a]pyridin-2-yl]carbonyl}-O-(cyclopropylmethyl)tyrosinate (185 mg, 0.36 mmol), and stirred for 30 minutes at 60°C. The reaction solution was neutralized with 1 N hydrochloric acid (750 µL), and the precipitated white solid was filtered out and washed successively with water and diethyl ether to obtain the title compound (156 mg, 87%) as a white solid.
   LC-MS 490 [M+H]⁺
   ¹H-NMR (300MHz, DMSO-d₆); δ 0.28-0.30 (m, 2H), 0.50-0.56 (m, 2H), 1.13-1.21 (m, 1H), 3.12-3.17 (m, 2H), 3.72-3.74 (d, J=6.9Hz, 2H), 4.64-4.71 (m, 1H), 6.78-6.81 (d, J=8.7Hz, 2H),7.11-7.14 (d, J=8.4Hz, 2H), 7.57-7.60 (d, J=8.7Hz, 2H), 7.68-7.77 (m, 4H), 8.19-8.22 (d, J=8.1Hz, 1H), 8.34 (s, 1H), 8.98 (s, 1H), 12.80 (br s, 1H)
   Melting point: 276°C
   Elemental analysis: Calcd. for C₂₇H₂₄N₃O₄, : C 66.19, H 4.94, N 8.58; Found: C 66.10, H 4.96, N 8.59

### Example E18

### N-{[6-(4-Chlorophenyl)imidazo[1,2-a]pyridin-2-yl]carbonyl}-O-(cyclohexylmethyl)tyrosine

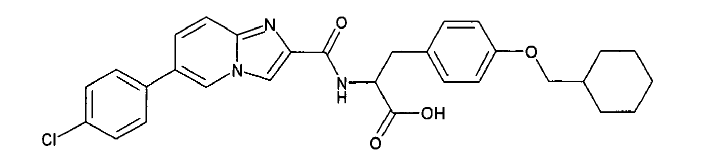

(1) A mixture of methyl N-(tert-butoxycarbonyl)tyrosinate (0.59 g, 2 mmol), (bromomethyl)cyclohexane (335 µL, 2.4 mmol), potassium carbonate (415 mg) and dimethyl formamide (3 mL) was stirred overnight at 60°C. Ethyl acetate and water were added to separate the solution, and the organic layer was washed twice with water and once with saturated sodium chloride solution, and dried over magnesium sulfate. The drying agent was removed by filtration, and the filtrate was concentrated under reduced pressure. The residue was purified by silica gel column chromatography (eluate: ethyl acetate/hexane = 1/10-1/1) to obtain methyl N-(tert-butoxycarbonyl)-O-(cyclohexylmethyl)tyrosinate (279 mg, 36%) as a white solid.
   ¹H-NMR (300MHz, CDCl₃); δ 0.98-1.14 (m, 2H), 1.16-1.33 (m, 3H), 1.42 (s, 9H), 1.63-1.88 (m, 6H), 2.95-3.08 (m, 2H), 3.73 (s, 3H), 4.50-4.56 (m, 1H), 4.93-4.96 (d, J=8.1Hz, 1H), 6.80-6.83 (d, J=8.7Hz, 2H), 6.99-7.02 (d, J=8.4Hz, 2H)
(2) 4 N Hydrogen chloride-ethyl acetate solution (0.4 mL) was added to an ethyl acetate (2 mL) solution of methyl N-(tert-butoxycarbonyl)-O-(cyclohexylmethyl)tyrosinate (279 mg, 0.71 mmol), and stirred overnight at room temperature. The precipitated white solid was filtered out and washed successively with ethyl acetate and diethyl ether to obtain methyl O-(cyclohexylmethyl)tyrosinate hydrochloride (153 mg, 65%) as a white solid.
   LC-MS 292 [M+H]⁺
   ¹H-NMR (300MHz, DMSO-d₆); δ 0.97-1.14 (m, 2H), 1:20-1.31 (m, 3H), 1.69-1.81 (m, 6H), 2.97-3.10 (m, 2H), 3.69 (s, 3H), 3.74-3.76 (d, J=6.0Hz, 2H), 4.20-4.25 (m, 1H), 6.86-6.89 (d, J=8.4Hz, 2H), 7.10-7.13 (d, J=8.4Hz, 2H), 8.41 (s, 3H)
(3) A mixture of 6-(4-chlorophenyl)imidazo[1,2-a]pyridine-2-carboxylic acid (164 mg, 0.60 mmol), methyl O-(cyclopropylmethyl)tyrosinate hydrochloride (143 mg, 0.50 mmol), (3-dimethylaminopropyl)ethyl carbodiimide hydrochloride (192 mg, 1.00 mmol), 1-hydroxybenzotriazole (135 mg, 1.00 mmol), triethylamine (300 µL, 2.1 mmol), N,N-diemthylformamide (2 mL) and dichloromethane (6 mL) was stirred overnight at room temperature. Ethyl acetate and water were added to separate the solution, and the organic layer was washed twice with water and once with saturated sodium chloride solution and then dried over magnesium sulfate. The drying agent was removed by filtration, and the filtrate was concentrated under reduced pressure. The residue was purified by silica gel column chromatography (eluate: ethyl acetate/hexane = 1/10-1/1) and recrystallized from a chloroform-hexane mixed solvent to obtain methyl N-{[6-(4-chlorophenyl)imidazo[1,2-a]pyridin-2-yl]carbonyl)-O-(cyclohexylmethyl)tyrosinate (220 mg, 84%) as a white solid.
   LC-MS 546 [M+H]⁺
   ¹H-NMR (300MHz, DMSO-d₆); δ 0.97-1.95 (m, 2H), 1.18-1.24 (m, 3H), 1.62-1.79 (m, 6H), 3.12-3.14 (m, 2H), 3.66-3.71 (m, 5H), 4.72-4.75 (m, 1H), 6.79-6.82 (d, J=8.4Hz, 2H), 7.12-7.15 (d, J=8.7Hz, 2H), 7.57-7.60 (d, J=8.7Hz, 2H), 7.71-7.77 (m, 4H), 8.34 (s, 1H), 8.44-8.46 (d, J=8.1Hz, 1H), 8.98 (s, 1H)
(4) 1 M Sodium hydroxide aqueous solution (806 µL) was added to a tetrahydrofuran-methanol mixed solution (8 mL-2 mL) of methyl N-{[6-(4-chlorophenyl)imidazo[1,2-a]pyridin-2-yl]carbonyl}-O-(cyclohexylmethyl)tyrosinate (220 mg, 0.40 mmol), and stirred for 30 minutes at 60°C. The reaction solution was neutralized with 1 N hydrochloric acid (810 µL), and the precipitated white solid was filtered out and washed successively with water and diethyl ether to obtain the title compound (151 mg, 71%) as a white solid.
   LC-MS 532 [M+H]⁺
   ¹H-NMR (300MHz, DMSO-d₆); δ 0.95-1.29 (m, 5H), 1.66-1.79 (m, 6H), 3.07-3.14 (m, 2H), 3.69-3.71 (d, J=6.3Hz, 2H), 4.64-4.70 (m, 1H), 6.78-6.81 (d, J=8.7Hz, 2H), 7.11-7.14 (d, J=8.7Hz, 2H), 7.57-7.60 (d, J=8.4Hz, 2H), 7.71-7.77 (m, 4H), 8.20-8.22 (d, J=8.1Hz, 1H), 8.34 (s, 1H), 8.98 (s, 1H), 12.80 (br s, 1H)
   Melting point: Decomposes at 277°C
   Elemental analysis: Calcd. for C₃₀H₃₀N₃O₄Cl, : C 67.73, H 5.68, N 7.90; Found: C 67.66, H 5.62, N 7.98

### Example E19

### O-Benzyl-N-{[6-(2-fluorophenyl)imidazo[1,2-a]pyridin-2-yl]carbonyl}tyrosine

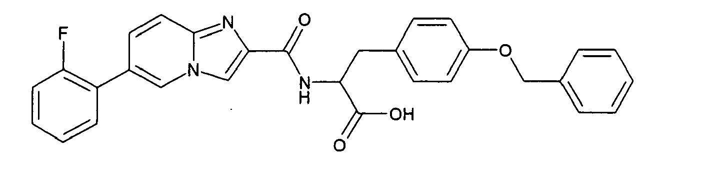

(1) 1 M Sodium hydroxide aqueous solution (18 mL) was added to a methanol (36 mL)-tetrahydrofuran (36 mL) solution of ethyl 6-bromoimidazo[1,2-a]pyridine-2-carboxylate (3.22 g, 12 mmol), and stirred for 1 hour at room temperature. The reaction solution was concentrated under reduced pressure and neutralized with 1 N hydrochloric acid (20 mL), and the precipitated white solid was filtered out and washed successively with water and diethyl ether to obtain 6-bromoimidazo[1,2-a]pyridine-2-carboxylic acid (2.51 g, 87%) as a white solid
   ¹H-NMR (300MHz, DMSO-d₆); δ 7.44-7.47 (d, J=9.9Hz, 1H), 7.60-7.63 (d, J=9.9Hz, 1H), 8.42 (s, 1H), 8.92 (s, 1H), 12.80 (br s, 1H)
(2) A mixture of 6-bromoimidazo[1,2-a]pyridine-2-carboxylic acid (2.51 g, 10.4 mmol), methyl O-benzyltyrosinate hydrochloride (2.80 mg, 8.68 mmol), (3-dimethylaminopropyl)ethyl carbodiimide hydrochloride (3.33 g, 17.3 mmol), 1-hydroxybenzotriazole (2.34 g, 17.3 mmol), triethylamine (5.2 mL), N,N-dimethylformamide (30 mL) and dichloromethane (60 mL) was stirred overnight at room temperature. Ethyl acetate and water were added to separate the solution, and the organic layer was washed twice with water and once with saturated sodium chloride solution, and dried over magnesium sulfate. The drying agent was removed by filtration, and the filtrate was concentrated under reduced pressure. The residue was recrystallized from an ethyl acetate-hexane mixed solvent to obtain methyl O-benzyl-N-[(6-bromoimidazo[1,2-a]pyridin-2-yl)carbonyl]tyrosinate (3.28 g, 74%) as a white solid.
   LC-MS 510 [M+2+H]⁺
   ¹H-NMR (300MHz, DMSO-d₆); δ 3.11-3.14 (m, 2H), 3.64 (s, 3H), 4.69-4.76 (m, 1H), 5.03 (s, 2H), 6.88-6.91 (d, J=8.7Hz, 2H), 7.14-7.17 (d. J=8.4Hz, 2H), 7.28-7.50 (m, 6H), 7.59-7.62 (d, J=9.6Hz, 1H), 8.30 (s, 1H), 8.49-8.51 (d, J=8.1 Hz, 1H), 8.93 (s, 1H)
(3) Methyl O-benzyl-N-[(6-bromoimidazo[1,2-a]pyridin-2-yl)carbonyl]tyrosinate (508 mg, 1.00 mmol), 2-fluorophenylboronic acid (168 mg, 1.20 mmol), tetrakis triphenylphosphine palladium (58 mg), 2 M sodium carbonate aqueous solution (1 mL) and 1,2-dimethoxyethane (6 mL) were stirred for 4 hours at 80°C in an argon gas atmosphere. The reaction mixture was filtered, and the filtrate was diluted with ethyl acetate and washed twice with saturated sodium bicarbonate solution and once with saturated sodium chloride solution. The organic layer was dried by addition of anhydrous magnesium sulfate. The drying agent was removed by filtration, and the filtrate was concentrated under reduced pressure. The residue was purified by silica gel chromatography (eluate: ethyl acetate/hexane = 2/98-1/1) and recrystallized from a chloroform-hexane mixed solvent to obtain methyl O-benzyl-N-{[6-(2-fluorophenyl)imidazo[1,2-a]pyridin-2-yl]carbonyl}tyrosinate (367 mg, 65%) as a white solid.
   LC-MS 524 [M+H]⁺
   ¹H-NMR (300MHz, DMSO-d₆); δ 3.13-3.16 (d, J=7.2Hz, 2H), 3.66 (s, 3H), 4.71-4.76 (m, 1H), 5.03 (s, 2H), 6.89-6.92 (d, J=8.7Hz, 2H), 7.15-7.18 (d, J=8.7Hz, 2H), 7.30-7.73 (m, 11H), 8.41 (s, 1H), 8.46-8.49 (d, J=8.1Hz, 1H); 8.86 (s, 1H)
(4) 1 M Sodium hydroxide aqueous solution (1.0 mL) was added to a tetrahydrofuran-methanol mixed solution (6 mL-3 mL) of methyl O-benzyl-N-{[6-(2-fluorophenyl)imidazo[1,2-a]pyridin-2-yl]carbonyl}tyrosinate (262 mg, 0.50 mmol), and stirred for 30 minutes at 60°C. The reaction solution was neutralized with 1 N hydrochloric acid (1.1 mL), and the precipitated white solid was filtered out and washed successively with water and diethyl ether to obtain the title compound (210 mg, 82%) as a white solid.
   LC-MS 510 [M+H]⁺
   ¹H-NMR (300MHz, DMSO-d₆); δ 3.08-3.19 (m, 2H), 4.65-4.72 (m, 1H), 5.03 (s, 2H), 6.89-6.92 (d, J=8.7Hz, 2H), 7.14-7.17 (d, J=8.7Hz, 2H), 7.28-7.73 (m, 11H), 8.23-8.26 (d, J=8.1Hz, 1H), 8.41 (s, 1H), 8.86 (s, 1H), 12.80 (br s, 1H)
   Melting point: 238°C
   Elemental analysis: Calcd. for C₃₀H₂₄N₃O₄, : C 70.72, H 4.75, N 8.25; Found: C 70.47, H 4.53, N 8.31

### Example E20

### O-Benzyl-N-[(6-(pyridin-3-yl)imidazo[1,2-a]pyridine-2-yl)carbonyl]tyrosine

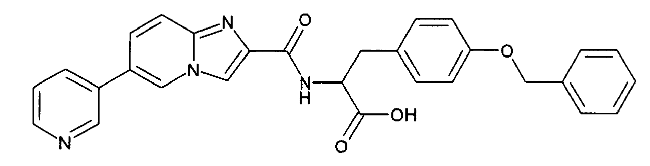

(1) Methyl O-benzyl-N-[(6-bromoimdazo[1,2-a]pyridin-2-yl)carbonyl]tyrosinate (508 mg, 1.00 mmol), pyridin-3-ylboronic acid (148 mg, 1.20 mmol), ), tetrakis(triphenylphosphine)palladium (58 mg), 2 M sodium carbonate aqueous solution (1 mL) and 1,2-dimethoxyethane (6 mL) were stirred for 4 hours at 80°C in an argon gas atmosphere. The reaction mixture was filtered, and the filtrate was diluted with ethyl acetate and washed twice with saturated sodium bicarbonate solution and once with saturated sodium chloride solution. The organic layer was dried by addition of anhydrous magnesium sulfate. The drying agent was removed by filtration, and the filtrate was concentrated under reduced pressure. The residue was purified by basic silica gel chromatography (eluate: ethyl acetate/hexane = 2/98-1/1) to obtain methyl O-benzyl-N-[(6-(pyridin-3-yl)imidazo[1,2-a]pyridin-2-yl)carbonyl]tyrosinate (124 mg, 25%) as a white solid.
   LC-MS 507 [M+H]⁺
   ¹H-NMR (300MHz, DMSO-d₆); δ 3.13-3.16 (d, J=7.2Hz, 2H), 3.66 (s, 3H), 4.71-4.78 (m, 1H), 5.03 (s, 2H), 6.89-6.92 (d, J=8,7Hz, 2H), 7.15-7.18 (d, J=8.7Hz, 2H), 7.28-7.43 (m, 5H), 7.53-7.57 (m, 1H), 7.72-7.80 (m, 2H), 8.12-8.16 (m, 1H), 8.35 (s, 1H), 8.47-8.50 (d, J=8.1Hz, 1H), 8.62-8.64 (d, J=6.0Hz, 1H), 8.95-8.96 (d, J=1.8Hz, 1H), 9.05 (s, 1H)
(2) 1 M Sodium hydroxide aqueous solution (490 µL) was added to a tetrahydrofuran-methanol mixed solution (2 mL-2 mL) of methyl O-benzyl-N-[(6-(pyridin-3-yl)imidazo[1,2-a]pyridin-2-yl)carbonyl]tyrosinate (124 mg, 0.24 mmol), and stirred for 30 minutes at 60°C. The reaction solution was neutralized with 1 N hydrochloric acid (500 µL), and the precipitated white solid was filtered out and washed successively with water and diethyl ether to obtain the title compound (102 mg, 85%) as a white solid.
   LC-MS 493 [M+H]⁺
   ¹H-NMR (300MHz, DMSO-d₆); δ 3.13-3.18 (m, 2H), 4.65-4.72 (m, 1H), 5.03 (s, 2H), 6.89-6.91 (d, J=8.7Hz, 2H), 7.15-7.17 (d, J=8.4Hz, 2H), 7.28-7.44 (m, 5H), 7.53-7.57 (m, 1H), 7.72-7.79 (m, 2H), 8.12-8.16 (m, 1H), 8.24-8.26 (d, J=8.1Hz, 1H), 8.36 (s, 1H), 8.62-8.64 (m, 1H), 8.95-8.96 (d, J=2.4Hz, 1H), 9.05 (s, 1H), 12.80 (br s, 1H)
   Melting point: 244°C
   Elemental analysis: Calcd. for C₂₉H₂₄N₄O₄ (cont. 0.3 mol H₂O), : C 69.95, H 4.98, N 11.25; Found: C 70.06, H 5.02, N 11.30

### Example E21

### O-Benzyl-N-[(6-(pyridine-4-yl)imidazo[1,2-a] pyridin-2-yl)carbonyl]tyrosine

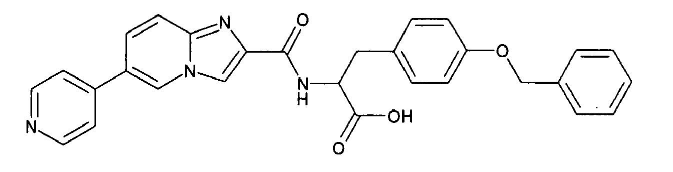

(1) Methyl O-benzyl-N-[(6-bromoimidazo[1,2-a]pyridin-2-yl)carbonyl]tyrosinate (508 mg, 1.00 mmol), pyridin-4-ylboronic acid (148 mg, 1.20 mmol), ), tetrakis(triphenylphosphine)palladium (58 mg), 2 M sodium carbonate aqueous solution (1 mL) and 1,2-dimethoxyethane (6 mL) were stirred for 4 hours at 80°C in an argon gas atmosphere. The reaction mixture was filtered, and the filtrate was diluted with ethyl acetate and washed twice with saturated sodium bicarbonate solution and once with saturated sodium chloride solution. The organic layer was dried by addition of anhydrous magnesium sulfate. The drying agent was removed by filtration, and the filtrate was concentrated under reduced pressure. The residue was purified by basic silica gel chromatography (eluate: ethyl acetate/hexane = 2/98-1/1) to obtain methyl O-benzyl-N-[(6-(pyridin-4-yl)imidazo[1,2-a]pyridin-2-yl)carbonyl]tyrosinate (71 mg, 14%) as a white solid.
   LC-MS 507 [M+H]⁺
   ¹H-NMR (300MHz, DMSO-d₆); δ 3.13-3.15 (m, 2H), 3.66 (s, 3H), 4.71-4.78 (m, 1H), 5.03 (s, 2H), 6.89-6.92 (d, J=8.7Hz, 2H), 7.15-7.18 (d, J=8.7Hz, 2H), 7.30-7.43 (m, 5H), 7.55-7.63 (m, 1H), 7.74-7.84 (m, 4H), 8.37 (s, 1H), 8.48-8.51 (d, J=8.1Hz, 1H), 8.68-8.70 (m, 2H), 9.17 (s, 1H)
(2) 1 M Sodium hydroxide aqueous solution (280 µL) was added to a tetrahydrofuran-methanol mixed solution (2 mL-2 mL) of methyl O-benzyl-N-[(6-(pyridin-4-yl)imidazo[1,2-a]pyridin-2-yl)carbonyl]tyrosinate (71 mg 0.14 mmol), and stirred for 30 minutes at 60°C. The reaction solution was neutralized with 1 N hydrochloric acid (290 µL), and the precipitated white solid was filtered out and washed successively with water and diethyl ether to obtain the title compound (102 mg, 85%) as a white solid.
   LC-MS 493 [M+H]⁺
   ¹H-NMR (300MHz, DMSO-d₆); δ 3.14-3.16 (m, 2H), 4.65-4.71 (m,1H), 5.03 (s, 2H), 6.89-6.91 (d, J=8.4Hz, 2H), 7.14-7.17 (d, J=8.4Hz, 2H), 7.28-7.43 (m, 5H), 7.74-7.84 (m, 4H), 8.25-8.27 (d, J=8.1Hz, 1H), 8.37 (s, 1H), 8.68-8.70 (m, 2H), 9.18 (s, 1H), 12.80 (br s, 1H).
   Melting point: 268°C
   Elemental analysis: Calcd. for C₂₉H₂₄N₄O₄ (cont. 0.1 mol H₂O), : C 70.46, H 4.93, N 11.33; Found: C 70.25, H 4.98, N 11.28

### Example E22

### O-Benzyl-N-{[6-(phenylethynyl)imidazo[1,2-b]pyridazin-2-yl]carbonyl}tyrosine

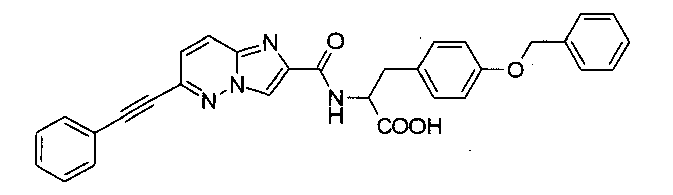

(1) A mixture of ethyl 6-chloroimidazo[1,2-b]pyridazine-2-carboxylate (0.30 g, 1.33 mmol), tetrakis(triphenylphosphine)palladium (0.15 g, 0.13 mmol), CuI (50 mg, 0.26 mmol), N,N-dimethylamide (5 mL) and triethylamine (0.60 mL) was heated for 4 hours at 120°C by a microwave synthesizer. The reaction solution was diluted with ethyl acetate, washed with water, and dried over anhydrous magnesium sulfate. The solvent was evaporated under reduced pressure, and the residue was purified by silica gel column chromatography (eluate: 10-50% ethyl acetate-hexane) to obtain ethyl 6-(phenylethynyl)imidazo[1,2-b]pyridazine-2-carboxylate (0.15 g, 38%) as a pale brown powder.
   ¹H-NMR (300MHz, CDCl₃)δ: 1.46 (3H, t, J=7.2Hz), 4.49 (2H, q, J=7.2Hz), 7.28 (1H, d, J=9.6Hz), 7.38-7.49 (3H, m), 7.61-7.66 (2H, m), 8.00 (1H, d, J=9.0Hz), 8.50 (1H, s)
   LC-MS 292 [M+H]⁺
(2) Methyl O-benzyl-N-{[6-(phenylethynyl)imidazo[1,2-b]pyridazin-2-yl]carbonyl}tyrosinate (0.15 g, 60%) was obtained by methods similar to those of Example C139-(3) from ethyl 6-(phenylethynyl)imidazo[1,2-b]pyridazine-2-carboxylate (0.14 g, 0.48 mmol).
   ¹H-NMR (300MHz, CDCl₃)δ: 3.17-3.23 (2H, m), 5.03-5.11 (3H, m), 7.29-7.46 (9H, m), 7.61-7.65 (2H, m), 7.79 (1H, d, J=8.1Hz), 7.90 (1H, d, J=9.3Hz), 8.47 (1H, s)
   LC-MS 531 [M+H]⁺
(3) The title compound (0.09 g, 69%) was obtained as colorless crystals by methods similar to those of Example C167-(6) from methyl O-benzyl-N-{[6-(phenylethynyl)imidazo[1,2-b]pyridazin-2-yl]carbonyl}tyrosinate (0.14 g, 0.26 mmol).
   ¹H-NMR (300MHz, DMSO-d₆)δ: 3.14-3.19 (2H, m), 4.67-4.74 (1H, m), 5.03 (2H, s), 6.90 (2H, d, J=8.7Hz), 7.17 (2H, d, J=8.7Hz), 7.28-7.43 (5H, m), 7.48-7.57 (4H, m), 7.69-7.72 (2H, m), 8.25 (1H, d, J=9.9Hz), 8.36 (1H, d, J=8.1Hz), 8.70 (1H, s), 8.70 (1H, s), 12.95 (1H, br s)
   LC-MS 517 [M+H]⁺
   Melting point: 224-227°C
   Elemental analysis: Calcd. for C₃₁N₂₄N₄O₄•0.1 H₂O, C 71.80, H 4.74, N 10.80; Found: C 71.70, H 4.52, N 10.80

### Example E23

### O-Benzyl-Nα-{[6-(4-chlorophenyl)imidazo[1,2-a]pyridin-2-yl]carbonyl}-N-(methylsulfonyl)tyrosinamide

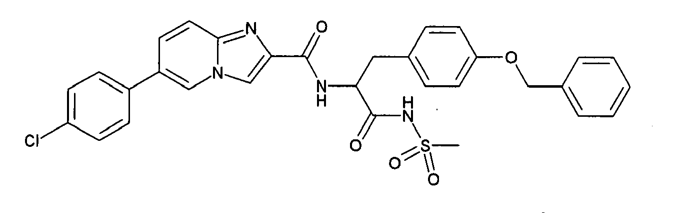

Carbodiimidazole (97 mg, 0.6 mmol) was added in an argon gas atmosphere to a tetrahydrofuran (3 mL)-N,N-dimethylformamide (3 mL) solution of O-benzyl-N-{[6-(4-chlorophenyl)imidazo[1,2-a]pyridin-2-yl]carbonyl}tyrosine (Example E1) (263 mg, 0.5 mmol), and stirred for 30 minutes and then heated and refluxed for 1 hours. Methanesulfonamide (57 mg, 0.6 mmol) was added to the reaction solution and stirred for 15 minutes at room temperature. 1,8-diazabicyclo[5.4.0]undeca-7-ene (90 µL, 0.6 mmol) was added, and stirred overnight. 1 N hydrochloric acid was added to the reaction solution and stirred for 15 minutes. This was extracted twice with chloroform, washed with saturated sodium chloride solution and dried by addition of magnesium sulfate. The drying agent was removed by filtration, and the filtrate was concentrated under reduced pressure. The residue was purified by preparative HPLC and recrystallized from a tetrahydrofuran-diisopropyl ether mixed solvent to obtain the title compound (144 mg, 48%) as a white solid.
LC-MS 603 [M+H]⁺
¹H-NMR (300MHz, DMSO-d₆); δ 3.08-3.10 (m, 2H), 3.25 (s, 3H), 4.68-4.80 (m, 1H), 5.03 (s, 2H), 6.90-6.93 (d, J=8.7Hz, 2H), 7.23-7.26 (d, J=8.7Hz, 2H), 7.29-7.43 (m, 5H), 7.57-7.60 (d, J=8.7Hz, 2H), 7.72-7.77 (m, 4H), 8.35-8.41 (m, 2H), 8.98 (s, 1H), 12.20 (br s, 1H)
Melting point: 232°C
Elemental analysis: Calcd. for C₃₁H₂₇N₄O₅SCl, : C 61.74, H 4.51, N 9.29; Found: C 61.78, H 4.53, N9.21

### Example E24

### O-Benzyl-N-{[6-(2-chlorophenyl)imidazo[1,2-a]pyridin-2-yl]carbonyl}tyrosine

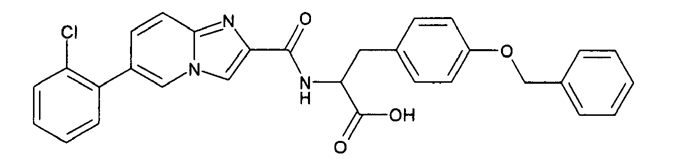

(1) Methyl O-benzyl-N-[(6-bromoimidazo[1,2-a]pyridin-2-yl)carbonyl]tyrosinate (508 mg, 1.00 mmol), 2-chlorophenylboronic acid (188 mg, 1.20 mmol), tetrakis(triphenylphosphine)palladium (58 mg), 2 M sodium carbonate aqueous solution (1 mL) and dimethoxyethane (6 mL) were stirred overnight at 80°C in an argon gas atmosphere. The reaction mixture was filtered, and the filtrate was diluted with ethyl acetate and washed twice with saturated sodium bicarbonate solution and once with saturated sodium chloride solution. The organic layer was dried by addition of anhydrous magnesium sulfate. The drying agent was removed by filtration, and the filtrate was concentrated under reduced pressure. The residue was purified by basic silica gel chromatography (eluate: ethyl acetate/hexane = 2/98-1/1) to obtain methyl O-benzyl-N-{[6-(2-chlorophenyl)imidazo[1,2-a]pyridin-2-yl]carbonyl}tyrosinate (123 mg, 23%) as a white solid.
   LC-MS 540 [M+H]⁺
   ¹H-NMR (300MHz, DMSO-d₆); δ 3.13-3.16 (m, 2H), 3.66 (s, 3H), 4.73-4.76 (m, 1H), 5.04 (s, 2H), 6.89-6.92 (d, J=8.7Hz, 2H), 7.15-7.18 (d, J=8.7Hz, 2H), 7.30-7.71 (m, 11H), 8.39 (s, 1H), 8.47-8.50 (d, J=8.1Hz, 1H), 8.71 (s, 1H)
(2) 1 M Sodium hydroxide aqueous solution (455 µL) was added to a tetrahydrofuran-methanol mixed solution (2 mL-2 mL) of methyl O-benzyl-N-{[6-(2-chlorophenyl)imidazo[1,2-a]pyridin-2-yl]carbonyl}tyrosinate (123 mg, 0.23 mmol), and stirred for 30 minutes at 60°C. The reaction solution was neutralized with 1 N hydrochloric acid (460 µL), and the precipitated white solid was filtered out and washed successively with water and diethyl ether to obtain the title compound (112 mg, 94%) as a white solid.
   LC-MS 526 [M+H]⁺
   ¹H-NMR (300MHz, DMSO-d₆);δ 3.08-3.15 (m, 2H), 4.65-4.72 (m, 1H), 5.03 (s, 2H), 6.89-6.92 (d, J=8.4Hz, 2H), 7.14-7.17 (d, J=8.7Hz, 2H), 7.28-7.70 (m, 11H), 8.22-8.25 (d, J=8.1Hz, 1H), 8.39 (s, 1H), 8.71 (s, 1H), 12.80 (br s, 1H)
   Melting point: 228°C
   Elemental analysis: Calcd. for C₃₀H₂₄N₃O₄Cl, : C 68.50, H 4.60, N 7.99; Found: C 68.38, H 4.59, N 8.05

### Example E25

### O-Benzyl-N-{[2-(4-chlorophenyl)imidazo[1,2-a]pyridin-6-yl]carbonyl}tyrosine

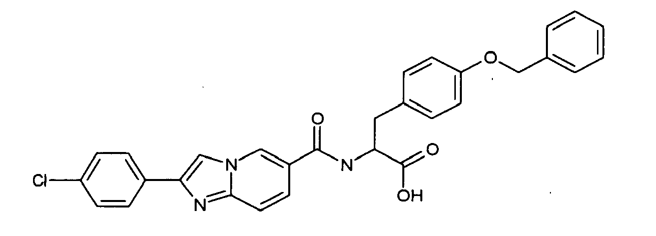

(1) A mixture of 2-(4-chlorophenyl)imidazo[1,2-a]pyridine-6-carboxylic acid (272 mg), methyl O-benzyltyrosinate hydrochloride (321 mg), (3-dimethylaminopropyl)ethyl carbodiimide hydrochloride (230 mg), 1-hydroxybenzotriazole (162 mg), triethylamine (0.42 mL) and N,N-dimethylformamide (7 mL) was stirred overnight at room temperature. Water was added to the reaction solution, which was then extracted with ethyl acetate. The extract was washed with hydrochloric acid and saturated sodium chloride solution, and dried over anhydrous magnesium sulfate. The solvent was evaporated under reduced pressure, and the residue was filtered and washed with methanol-diethyl ether to obtain methyl O-benzyl-N-{[2-(4-chlorophenyl)imidazo[1,2-a]pyridin-6-yl]carbonyl}tyrosinate (440 mg) as colorless crystals.
   ¹H-NMR (300MHz, CDCl₃); δ 3.15-3.30 (2H, m), 3.80 (3H, s), 5.03-5.10 (1H, m), 5.04 (2H, s), 6.52 (1H, d, J=7.5Hz), 6.92 (2H, d, J=8.7Hz), 7.04 (2H, d, J=8.7Hz), 7.30-7.45 (8H, m), 7.62 (1H, d, J=9.6Hz), 7.88-7.92 (3H, m), 8.74 (1H, s)
   Elemental analysis: Calcd. for C₃₁H₂₆ClN₃O₄, : C 68.95, H 4.85, N 7.78; Found: C 68.74, H 4.87, N 7.82
(2) 1 N Sodium hydroxide aqueous solution (2 mL) and water (10 mL) were added at 60°C to a tetrahydrofuran-methanol mixed solution (70 mL-10 mL) of the compound (380 mg) obtained in (1), and stirred for 10 minutes at that temperature. The reaction solution was neutralized with 1 N hydrochloric acid, and concentrated under reduced pressure. Water was added to the residue, and the precipitated crystals were filtered out and washed with water to obtain the title compound (185 mg) as colorless crystals.
   Melting point: 278-280°C
   ¹H-NMR (300MHz, DMSO-d₆); δ 3.00 (1H, dd, J=10.5, 13.8Hz), 3.15 (1H, dd, J=4.5, 13.8Hz), 4.55-4.65 (1H, m), 5.03 (2H, s), 6.92 (2H, d, J=8.7Hz), 7.22-7.44 (7H, m), 7.52 (2H, d, J=8.7Hz), 7.60-7.69 (2H, m), 8.01 (2H, d, J=8.7Hz), 8.57 (1H, s), 8.84 (1H, d, J=8.1Hz), 9.03 (1H, s), 12.85 (1H, br s)
   Elemental analysis: Calcd. for C₃₀H₂₄ClN₃O₄, : C 68.50, H 4.60, N 7.99; Found: C 68.20, H 4.67, N 7.92

### Example E26

### O-Benzyl-N-{[6-(4-chlorophenyl)-5-methylimidazo[1,2-a]pyridin-2-yl]carbonyl}tyrosine

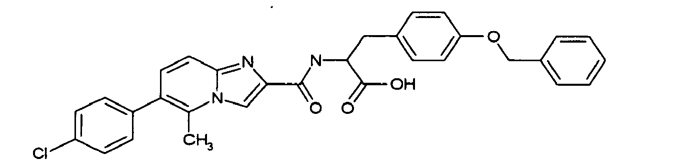

(1) A mixture of 6-(4-chlorophenyl)-5-methylimidazo[1,2-a]pyridine-2-carboxylic acid (229 mg), methyl O-benzyltyrosinate hydrochloride (258 mg), (3-dimethylaminpropyl)ethyl carbodiimide hydrochloride (184 mg), 1-hydroxybenzotriazole (130 mg), triethylamine (0.34 mL) and N,N-dimethylformamide (8 mL) was stirred overnight at room temperature. Water was added to the reaction solution, which was then extracted with ethyl acetate. The extract was washed successively with hydrochloric acid and saturated sodium chloride solution, and then dried over anhydrous magnesium sulfate. The solvent was evaporated under reduced pressure, and the residue was purified by silica gel column chromatography and recrystallized from ethyl acetate-hexane to obtain methyl O-benzyl-N-{[6-(4-chlorophenyl)-5-methylimidazo[1,2-a]pyridin-2-yl]carbonyl}tyrosinate (340 mg) as colorless crystals.
   ¹H-NMR (300MHz, CDCl₃); δ 2.54 (3H, s), 3.14-3.28 (2H, m), 3.73 (3H, s), 5.03 (2H, s), 5.04-5.12 (1H, m), 6.90 (2H, d, J=8.4Hz), 7.13 (2H, d, J=8.4Hz), 7.20-7.48 (10H, m), 7.53 (1H, d, J=9.3Hz), 7.82 (1H, d, J=8.4Hz), 8.13 (1H, s)
(2) 1 N Sodium hydroxide aqueous solution (1 mL) and water (10 mL) were added at 60°C to a tetrahydrofuran-methanol mixed solution (10 mL-5 mL) of the compound (300 mg) obtained in (1), and stirred for 20 minutes at that temperature. The reaction solution was neutralized with 1 N hydrochloric acid, and the precipitated crystals were filtered out and washed successively with water, methanol and diethyl ether to obtain the title compound (245 mg) as colorless crystals.
   Melting point: 263-264°C
   ¹H-NMR (300MHz, DMSO-d₆); δ 2.57 (3H, s), 3.09-3.22 (2H, m), 4.68-4.76 (1H, m), 5.03 (2H, s), 6.90 (2H, d, J=8.7Hz), 7.15 (2H, d, J=8.7Hz), 7.27-7.64 (11H, m), 8.24 (1H, d, J=8.1Hz), 8.34 (1H, s), 13.00 (1H, br s)
   Elemental analysis: Calcd. for C₃₁H₂₆ClN₃O₄, : C 68.95, H 4.85, N 7.78; Found: C 68.80, H 4.77, N 7.84

### Example E27

### O-Benzyl-N-{[6-(4-chlorophenyl)-8-methylimidazo[1,2-a]pyridin-2-yl]carbonyl}tyrosine

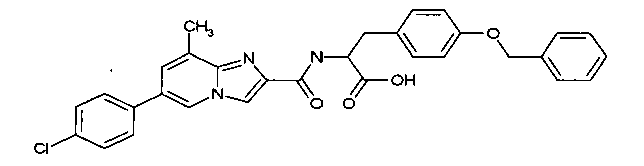

(1) A mixture of 6-(4-chlorophenyl)-8-methylimidazo[1,2-a]pyridine-2-carboxylic acid (229 mg), methyl O-benzyltyrosinate hydrochloride (258 mg), (3-dimethylaminpropyl)ethyl carbodiimide hydrochloride (184 mg), 1-hydroxybenzotriazole (130 mg), triethylamine (0.34 mL) and N,N-dimethylformamide (8 mL) was stirred overnight at room temperature. Water was added to the reaction solution, which was then extracted with ethyl acetate. The extract was washed successively with hydrochloric acid and saturated sodium chloride solution, and then dried over anhydrous magnesium sulfate. The solvent was evaporated under reduced pressure, and the residue was purified by silica gel column chromatography and recrystallized from ethyl acetate-hexane to obtain methyl O-benzyl-N-{[6-(4-chlorophenyl)-8-methylimidazo[1,2-a]pyridin-2-yl]carbonyl}tyrosinate (335 mg) as colorless crystals.
   ¹H-NMR (300MHz, CDCl₃); δ 2.63 (3H, s), 3.21 (2H, d, J=6.0Hz), 3.73 (3H, s), 5.03 (2H, s), 5.03-5.07 (1H, m), 6.91 (2H, d, J=8.7Hz), 7.15 (2H, d, J=8.7Hz), 7.20-7.52 (10H, m), 7.90 (1H, d, J=8.4Hz), 8.11-8.16 (2H, m)
(2) 1 N Sodium hydroxide aqueous solution (1 mL) and water (10 mL) were added at 60°C to a tetrahydrofuran-methanol mixed solution (10 mL-5 mL) of the compound (295 mg) obtained in (1), and stirred for 20 minutes at that temperature. The reaction solution was neutralized with 1 N hydrochloric acid, and the precipitated crystals were filtered out and washed successively with water, methanol and diethyl ether to obtain the title compound (256 mg) as colorless crystals.
   Melting point: 249-250°C
   ¹H-NMR (300MHz, DMSO-d₆); δ 2.58 (3H, s), 3.15 (2H, d, J=6.0Hz), 4.67-4.75 (1H, m), 5.03 (2H, s), 6.91 (2H, d, J=8.7Hz), 7.15 (2H, d, J=8.7Hz), 7.27-7.45 (5H, m), 7.54-7.60 (3H, m), 7. 75 (2H, d, J=8.7Hz), 8.09 (1H, d, J=8.1 Hz), 8.35 (1H, s), 8.82 (1H, s), 13.00 (1H, br s) Elemental analysis: Calcd. for C₃₁H₂₆ClN₃O₄, C 68.95, H 4.85, N 7.78; Found: C 68.76, H 4.78, N 7.88

### Example E28

### O-Benzyl-N-({8-[(4-chlorobenzyl)oxy]imidazo[1,2-a]pyridin-2-yl}carbonyl)tyrosine

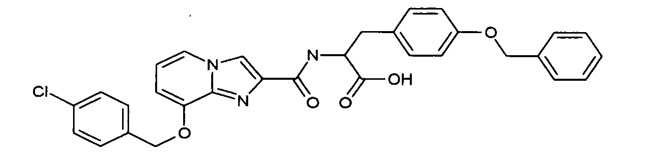

(1) A mixture of 8-[(4-chlorobenzyl)oxy]imidazo[1,2-a]pyridine-2-carboxylic acid (307 mg), methyl O-benzyltyrosinate hydrochloride (325 mg), (3-dimethylaminpropyl)ethyl carbodiimide hydrochloride (232 mg), 1-hydroxybenzotriazole (163 mg), triethylamine (0.42 mL) and N,N-dimethylformamide (10 mL) was stirred overnight at room temperature. Water was added to the reaction solution, which was then extracted with ethyl acetate. The extract was washed with hydrochloric acid and saturated sodium chloride solution, and then dried over anhydrous magnesium sulfate. The solvent was evaporated under reduced pressure, and the residue was purified by silica gel column chromatography to obtain methyl O-benzyl-N-({8-[(4-chlorobenzyl)oxy]imidazo[1,2-a]pyridin-2-yl}carbonyl)tyrosinate (400 mg) as a colorless oil. ¹H-NMR (300MHz, CDCl₃); δ 3.11-3.25 (2H, m), 3.70 (3H, s), 5.00 (2H, s), 5.00-5.10 (1H, m), 5.34 (2H, s), 6.50 (1H, d, J=7.2Hz), 6.68 (1H, dd, J=6.6, 7.2Hz), 6.88 (2H, d, J=8.7Hz), 7.14 (2H, d, J=8.7Hz), 7.28-7.47 (9H, m), 7.78 (1H, d, J=6.6Hz), 7.82 (1H, d, J=8.4Hz), 8.11 (1H, s)
(2) 1 N Sodium hydroxide aqueous solution (1 mL) and water (10 mL) were added at 60°C to a tetrahydrofuran-methanol mixed solution (10 mL-5 mL) of the compound (400 mg) obtained in (1), and stirred for 20 minutes at that temperature. The reaction solution was neutralized with 1 N hydrochloric acid, and extracted with ethyl acetate. The extract was washed with saturated sodium chloride solution, and dried over anhydrous magnesium sulfate. The solvent was evaporated under reduced pressure, and the precipitated crystals were filtered out and washed with methanol to obtain the title compound (207 mg) as colorless crystals.
   Melting point: 210°C
   ¹H-NMR (300MHz, DMSO-d₆); δ 3.13 (2H, d, J=6.6Hz), 4.64-4.72 (1H, m), 5.01 (2H, s), 5.32 (2H, s), 6.78-6.91 (4H, m), 7.14 (2H, d, J=8.7Hz), 7.27-7.44 (5H, m), 7.48 (2H, d, J=8.7Hz), 7.56 (2H, d, J=8 . 7Hz), 8.15-8.19 (2H, m), 8.35 (1H, s), 12. 90 (1H, s)
   Elemental analysis: Calcd. for C₃₁H₂₆C1N₃0₅, : C 66.97, H 4.71, N 7.56; Found: C 66.78, H 4.70, N 7.57

### Example E29

### O-Benzyl-N-({6-[(E)-2-(4-chlorophenyl)ethenyl]imidazo[1,2-b]pyridazin-2-yl}carbonyl)tyrosine

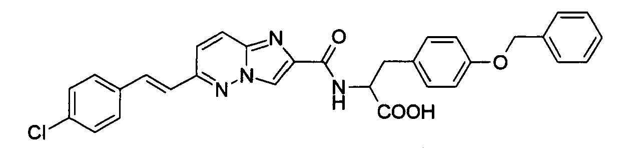

(1) Ethyl 6-[(E)-2-(4-chlorophenyl)ethenyl]imidazo[1,2-b]pyridazine-2-carboxylate (0.08 g, 19%) was obtained as a white powder by methods similar to those of Example C167-(1) from ethyl 6-chloroimidazo[1,2-b]pyridazine-2-carboxylate (0.30 g, 1.33 mmol) and (E)-2-(4-chlorophenyl)vinyl boronic acid (0.29 g, 1.60 mmol).
   ¹H-NMR (300MHz, CDCl₃)δ: 1.46 (3H, t, J=7.2Hz), 4.48 (2H, q, J=7.2Hz), 7.14 (1H, d, J=16Hz), 7.38-7.43 (4H, m), 7.48-7.54 (2H, m), 7.97 (1H, d, J=9.6Hz), 8.47 (1H, s)
   LC-MS 328 [M+H]⁺
(2) 6-[2-(4-Chlorophenyl)vinyl]imidazo[1,2-b]pyridazine-2-carboxylic acid (0.12 g, 99%) was obtained by methods similar to those of Example E28(2) from ethyl 6-[(E)-2-(4-chlorophenyl)ethenyl]imidazo[1,2-b]pyridazine-2-carboxylate (0.15 g, 0.46 mmol).
   ¹H-NMR (300MHz, DMSO-d₆)δ: 7.36 (1H, d, J=16.SHz), 7.51 (2H, d, J=8.7Hz), 7.75-7.82 (2H, 4m), 8.20 (1H, d, J=9.9Hz), 12.98 (1H, br s)
   LC-MS 300 [M+H]⁺
(3) The title compound (0.06 g, 68%) was obtained by methods similar to those of Example C139-(3) and (4) performed in sequence from 6-[(E)-2-(4-chlorophenyl)ethenyl]imidazo[1,2-b]pyridazine-2-carboxylic acid (0.11 g, 0.41 mmol).
   ¹H-NMR (300MHz, DMSO-d₆)δ: 3.13-3.16 (2H, m), 4.66-4.73 (1H, m), 5.03 (2H, s), 6.90 (2H, d, J=8.4Hz), 7.16 (2H, d, J=8.4Hz), 7.51 (2H, d, J=8.4Hz), 7.74-7.83 (4H, m), 8.19 (1H, d, J=9.6Hz), 8.27 (1H, d, J=8.1Hz), 8.58 (1H, s), 12.96 (1H, br s)
   LC-MS 554 [M+H]⁺
   Melting point: 256-257°C
   Elemental analysis: C₃₁H₂₅N₄O₄Cl, : C 67.73, H 4.56, N 10.13; Found: C 67.25, H 4.71, N 9.98

### Example E30

### N-[2-[4-(Benzyloxy)phenyl]-1-(5-oxo-4, 5-dihydro-1,2,4-oxadiazol-3-yl)ethyl]-6-(4-chlorophenyl)imidazo[1,2-a]pyridine-2-carboxamide

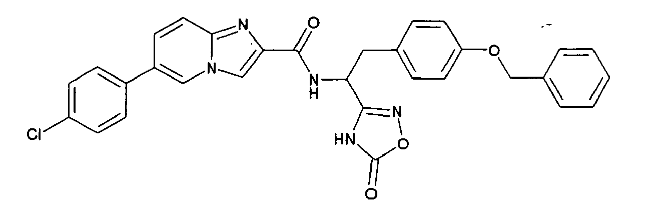

(1) Pyridine (0.9 mL, 12 mmol) was added dropwise with ice cooling into an acetonitrile (50 mL)-tetahydrofuran (50 mL) mixture of O-benzyl-N-(tert-butoxycarbonyl)tyrosine (4.45 g, 12 mmol), ammonium carbonate (9.5 g, 24 mmol) and di-tert-butyl dicarbonate (5.23 g, 24 mmol). The reaction mixture was stirred overnight at room temperature, and then stirred for 8 hours at 50°C. Water was added and stirred for 15 minutes, the reaction solution was concentrated under reduced pressure, and the precipitated white solid was filtered out and washed with water to obtain O-benzyl-Nα-(tert-butoxycarbonyl)tyrosinamide (2.87 g, 65%) as a white solid.
   ¹H-NMR (300MHz, DMSO-d₆); δ 1.32 (s, 9H), 2.71-2.79 (m, 1H), 2.90-2.97 (m, 1H), 3.99-4.04 (m, 1H), 5.06 (s, 2H), 6.12 (br s, 2H), 6.90-6.93 (d, J=8.4Hz, 2H), 7.00-7.03 (d, J=8.1Hz, 1H), 7.14-7.17 (d, J=8.4Hz, 2H), 7.32-7.45 (m, 5H)
(2) 2,4,6-Trichloro-1,3,5-triazine (435 mg, 2.36 mmol) was added with ice cooling to a dimethylformamide (6 mL) solution of O-benzyl-Nα-(tert-butoxycarbonyl)tyrosinamide (729 mg, 1.97 mmol), and the mixture was warmed to room temperature and stirred overnight. Water was added to complete the reaction, and the mixture was extracted twice with ethyl acetate. The organic layer was washed successively with water and saturated sodium chloride solution and dried by addition of magnesium sulfate. The drying agent was removed by filtration, and the filtrate was concentrated under reduced pressure. The residue was recrystallized from an ethyl acetate-hexane mixed solvent to obtain tert-butyl {2-[4-(benzyloxy)phenyl]-1-cyanoethyl}carbamate (602 mg, 87%) as a white solid.
   ¹H-NMR (300MHz, DMSO-d₆); δ 1.50 (s, 9H), 2.95-2.97 (m, 2H), 4.72-4.76 (m, 1H), 5.07 (s, 2H), 6.94-6.97 (d, J=8.7Hz, 2H), 7.07-7.11 (d, J=8.4Hz, 2H), 7.30-7.46 (m, 5H), 9.06 (s, 1H)
(3) Hydroxylamine hydrochloride (1.75 g, 25.2 mmol) was dissolved at 40°C in dimethylsulfoxide (15 mL), and sodium hydrogencarbonate (2.54 g, 30.3 mmol) was added in 4 additions. This was stirred for 30 minutes, and tert-butyl {2-[4-(benzyloxy)phenyl]-1-cyanoethyl}carbamate (1.07 g, 3.03 mmol) was added. The reaction solution was stirred for 19 hours at 90°C. After being left to cool, the reaction solution was poured into water and extracted twice with ethyl acetate. The organic layer was washed with water (twice) and saturated sodium chloride solution, and dried by addition of magnesium sulfate. The drying agent was removed by filtration, and the filtrate was concentrated under reduced pressure. The residue was dissolved in tetrahydrofuran (20 mL), and carbodiimidazole (409 mg, 2.52 mmol) and 1,8-diazobicyclo[5.4.0]unde-7-ene (377 µL) were added and stirred overnight at room temperature. The reaction solution was concentrated under reduced pressure, diluted with ethyl acetate, and washed with 0.1 N sodium hydroxide aqueous solution and saturated sodium chloride solution. The organic layer was dried over magnesium sulfate, the drying agent was removed by filtration, and the filtrate was concentrated under reduced pressure. The residue was purified by silica gel column chromatography (eluate: ethyl acetate/hexane = 1/10-1/1) to obtain tert-butyl -[2-[4-(benzyloxy)phenyl]-1-(5-oxo-4,5-dihydro-1,2,4-oxadiazol-3-yl)ethyl]carbamate (330 mg, 26%) as a white solid.
   ¹H-NMR (300MHz, DMSO-d₆); δ 1.42 (s, 9H), 3.19-3.21 (m, 2H), 4.55-4.62 (m, 2H), 4.92-4.94 (d, J=6.3Hz, 1H), 5.06 (s, 2H), 6.93 (d, J=8.7Hz, 2H), 7.12-7.15 (d, J=8.7Hz, 2H), 7.36-7.45 (m, 5H)
(4) 4 N Hydrogen chloride ethyl acetate solution (0.5 mL) was added to an ethyl acetate (2 mL)-tetrahydrofuran (2 mL) solution of tert-butyl -[2-[4-(benzyloxy)phenyl]-1-(5-oxo-4,5-dihydro-1,2,4-oxadiazole-3-yl)ethyl]carbamate (330 mg, 0.80 mmol), and stirred overnight at room temperature. The precipitated white solid was filtered out and washed successively with ethyl acetate and diethyl ether to obtain 3-{1-amino-2-[4-(benzyloxy)phenyl)ethyl}-1,2,4-oxadiazol-5(4H)-one hydrochloride (215 mg, 77%) as a white solid.
   ¹H-NMR (300MHz, DMSO-d₆); δ 3.16-3.18 (m, 2H), 4.56-4.60 (t, J=6.9Hz, 1H), 5.08 (s, 2H), 6.98-7.00 (d, J=8.7Hz, 2H), 7.15-7.17 (d, J=8.4Hz, 2H), 7.31-7.45 (m, 5H)
(5) Oxalyl chloride (347 µL, 0.40 mmol) and dimethylformamide (a few drops) were added to a dichloromethane suspension of 6-(4-chlorophenyl)imidazo[1,2-a]pyridine-2-carboxylic acid (94 mg, 0.34 mmol), and heated and refluxed for 1 hour. The reaction solution was concentrated under reduced pressure to obtain a yellow solid. This was dissolved in dimethylacetamide (3 mL), and the result was added to a dimethylacetamide (2 mL) solution of 3-{1-amino-2-[4-(benzyloxy)phenyl]ethyl}-1,2,4-oxadiazol-5(4H)-one hydrochloride (100 mg, 0.29 mmol) and triethylamine (41 µL), and stirred for 1 hour at room temperature. Water was added to the reaction solution and stirred for 15 minutes. This was extracted twice with ethyl acetate, and the organic layer was washed successively with water, saturated sodium bicarbonate solution, 1 N hydrochloric acid and saturated sodium chloride solution, and dried over magnesium sulfate. The drying agent was removed by filtration, and the filtrate was concentrated under reduced pressure. The residue was recrystallized from an ethyl acetate-diisopropyl ether mixed solvent to obtain the title compound (88.6 mg, 54%) as a white solid.
   LC-MS 566 [M+H]⁺
   ¹H-NMR (300MHz, DMSO-d₆); δ 3.18-3.20 (d, J=7.2Hz, 2H), 5.02 (s, 2H), 5.18-5.26 (m, 1H), 6.90-6.92 (d, J=8.4Hz, 2H), 7.19-7.22 (d, J=8.7Hz, 2H), 7.30-7.42 (m, 4H), 7.58-7.61 (d, J=8.4Hz, 2H), 7.70-7.77 (m, 4H), 8.37 (s, 1H), 8.83 (d, J=8.4Hz,1H), 9.00 (s,1H), 12.50 (s, 1H)
   Melting point: 167°C
   Elemental analysis: Calcd. for C₃₁H₂₄N₅O₄Cl (cont. 1 mol H₂O),: C 63.75, H 4.49, N 11.99; Found: C 64.65, H 4.44, N 12.07

### Example E31

### N-{[6-(4-Chlorophenyl)imidazo[1,2-a]pyridin-2-yl]carbonyl}-O-(3-furylmethyl)tyrosine

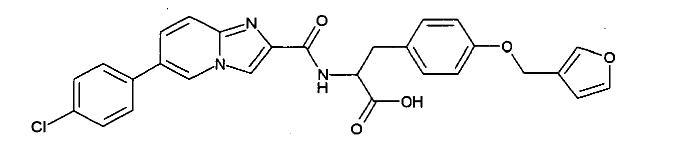

(1) 1,1'-(Azodicarbonyl)dipiperidine (6.56 g, 26 mmol) was added with ice cooling to a tetrahydrofuran (80 mL) solution of methyl N-(tert-butoxycarbonyl)tyrosinate (5.9 g, 20 mmol), 3-furyl methanol (2.24 mL, 26 mmol) and tributyl phosphine (6.9 mL, 26 mmol), and stirred overnight at room temperature. Tributyl phosphine (3.5 mL, 13 mmol) and 1,1'-(azodicarbonyl)dipiperidine (3.3 g, 13 mmol) were added and stirred for 10 hours. The reaction solution was filtered, and the filtrate was concentrated under reduced pressure. The residue was purified by silica gel column chromatography (eluate: ethyl acetate/hexane = 1/10-1/1) to obtain methyl N-(tert-butoxycarbonyl)-O-(3-furylmethyl)tyrosinate (2.52 g, 33%) as a white solid. ¹H-NMR (300MHz, CDCl₃); δ 1.42 (s, 9H), 2.96-3.09 (m, 2H), 3.71 (s, 3H), 4.53-4.56 (d, J=7.5Hz, 1H), 4.91-4.97 (m, 3H), 6.48 (s, 1H), 6.87-6.90 (d, J=8.4Hz, 2H), 7.03-7.06 (d, J=8.7Hz, 2H), 7.43 (s, 1H), 7.50 (s, 1H)
(2) 4 N Hydrogen chloride-ethyl acetate solution (3.35 mL) was added to an ethyl acetate (6 mL) solution of methyl N-(tert-butoxycarbonyl)-O-(3-furylmethyl)tyrosinate (2.52 g, 6.71 mmol), and stirred overnight at room temperature. The precipitated white solid was filtered out and washed successively with ethyl acetate and diethyl ether to obtain methyl O-(3-furylmethyl)tyrosinate hydrochloride (1.67 g, 80%) as a white solid.
   ¹H-NMR (300MHz, DMSO-d₆); δ 2.98-3.10 (m, 2H), 3.68 (s, 3H), 4.22-4.26 (t, J=6.6Hz, 1H), 4.94 (s, 2H), 6.56 (s, 1H), 6.95-6.98 (d, J=8.4Hz, 2H), 7.12-7.15 (d, J=8.7Hz, 2H), 6.95-6.98 (d, J=8.4Hz, 2H), 7.12-7.15 (d, J=8.7Hz, 2H), 7.67 (s, 1H), 7.78 (s, 1H), 8.38 (s, 3H)
(3) A mixture of 6-(4-chlorophenyl)imidazo[1,2-a]pyridine-2-carboxylic acid (164 mg, 0.60 mmol), methyl O-(3-furylmethyl)tyrosinate hydrochloride (156 mg, 0.50 mmol), (3-dimethylaminpropyl)ethyl carbodiimide hydrochloride (192 mg, 1.00 mmol), 1-hydroxybenzotriazole (135 mg, 1.00 mmol), triethylamine (300 µL, 2.1 mmol), N,N-dimethylformamide (2 mL) and dichloromethane (6 mL) was stirred overnight at room temperature. Ethyl acetate and water were added to separate the solution, and the organic layer was washed twice with water and once with saturated sodium chloride solution, and dried over magnesium sulfate. The drying agent was removed by filtration, and the filtrate was concentrate under reduced pressure. The residue was purified by silica gel column chromatography (eluate: ethyl acetate/hexane = 1/10-1/1) and recrystallized from a chloroform-hexane mixed solvent to obtain methyl N-{[6-(4-chlorophenyl)imidazo[1,2-a]pyridin-2-yl]carbonyl}-O-(3-furylmethyl)tyrosinate (255 mg, 96%) as a white solid.
   LC-MS 530 [M+H]⁺
   ¹H-NMR (300MHz, DMSO-d₆); δ 3.13-3.15 (d, J=7.2Hz, 2H), 3.66 (s, 3H), 4.71-4.78 (m, 1H), 4.89 (s, 2H), 6.53 (s, 1H), 6.88-6.90 (d, J=8.7Hz, 2H), 7.14-7.17 (d, J=8.7Hz, 2H), 7.57-7.64 (m, 3H), 7.71-7.77 (m, 5H), 8.34 (s, 1H), 8.44-8.46 (d, J=7.8Hz, 1H), 8.98 (s, 1H)
(4) 1 M Sodium hydroxide aqueous solution (962 µL) was added to a tetrahydrofuran-methanol mixed solution (6 mL-2 mL) of methyl N-{[6-(4-chlorophenyl)imidazo[1,2-a]pyridin-2-yl]carbonyl}-O-(3-furylmethyl)tyrosinate (255 mg, 0.48 mmol), and stirred for 30 minutes at 60°C. The reaction solution was neutralized with 1 N hydrochloric acid (1000 µL), and the precipitated white solid was filtered out and washed successively with water and diethyl ether and recrystallized from a dimethylformamide-water mixed solvent to obtain the title compound (167 mg, 67%) as a white solid.
   LC-MS 516 [M+H]⁺
   ¹H-NMR (300MHz, DMSO-d₆); δ 3.08-3.18 (m, 2H), 4.64-4.70 (m, 1H), 4.88 (s, 2H), 6.53-6.54 (m,1H), 6.87-6.90 (d, J=8.7Hz, 2H), 7.13-7.16 (d, J=8.7Hz, 2H), 7.56-7.60 (m, 2H), 7.63-7.64 (t, J=1.2Hz, 1H), 7.75-7.77 (m, 5H), 8.20-8.23 (d, J=8.1Hz, 1H), 8.34 (s, 1H), 8.97-8.98 (t, J=1.2Hz, 1H), 12.80 (br s, 1H)
   Melting point: 262°C
   Elemental analysis: Calcd. for C₂₈H₂₂N₃O₅Cl, : C 65.18, H 4.30, N 8.14; Found: C 65.07, H 4.16, N 8.15

### Example E32

### N-{[6-(4-Chlorophenyl)imidazo[1,2-b]pyridazin-2-yl]carbonyl}-O-(3-furylmethyl)tyrosine

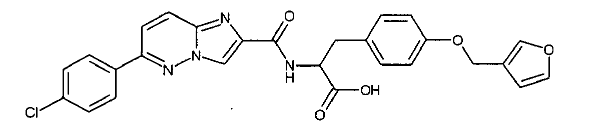

(1) A mixture of 6-(4-chlorophenyl)imidazo[1,2-b]pyridazine-2-carboxylic acid (164 mg, 0.60 mmol), methyl 0-(3-furylmethyl)tyrosinate hydrochloride (156 mg, 0.50 mmol), (3-dimethylaminpropyl)ethyl carbodiimide hydrochloride (192 mg, 1.00 mmol), 1-hydroxybenzotriazole (135 mg, 1.00 mmol), triethylamine (300 µL, 2.1 mmol), N,N-dimethylformamide (2 mL) and dichloromethane (6 mL) was stirred overnight at room temperature. Ethyl acetate and water were added to separate the solution, and the organic layer was washed twice with water and once with saturated sodium chloride solution, and dried over magnesium sulfate. The drying agent was removed by filtration, and the filtrate was concentrate under reduced pressure. The residue was purified by silica gel column chromatography (eluate: ethyl acetate/hexane = 1/10-1/1) and recrystallized from a chloroform-hexane mixed solution to obtain methyl N-{[6-(4-ch!orophenyl)imidazo[1,2-b]pyridazin-2-yl]carbonyl}-O-(3-furylmethyl)tyrosinate (235 mg, 89%) as a white solid.
   LC-MS 531 [M+H]⁺
   ¹H-NMR (300MHz, DMSO-d₆); δ 3.14-3.16 (d, J=6.9Hz, 2H), 3.66 (s, 3H), 4.73-4.80 (m, 1H), 4.88 (s, 2H), 6.53-6.54 (m, 1H), 6.87-6.90 (d, J=8.4Hz, 2H), 7.15-7.18 (d, J=8.7Hz, 2H), 7.63-7.67 (m, 3H), 7.74 (s, 1H), 7.93-7.97 (d, J=9.9Hz, 1H), 8.11-8.14 (dd, J=1.8, 5.1Hz, 2H), 8.27-8.31 (d, J=9.9Hz, 1H), 8.55-8.57 (d, J=8.4Hz, 1H), 8.69 (s, 1H)
(2) 1 M Sodium hydroxide aqueous solution (885 µL) was added to a tetrahydrofuran-methanol mixed solution (4 mL-2 mL) of methyl N-{[6-(4-chlorophenyl)imidazo[1,2-b]pyridazin-2-yl]carbonyl}-O-(3-furylmethyl)tyrosinate (235 mg, 0.44 mmol), and stirred for 30 minutes at 60°C. The reaction solution was neutralized with 1 N hydrochloric acid (900 µL), and the precipitated white solid was filtered out and washed successively with water and diethyl ether and recrystallized from a dimethylformamide-water mixed solvent to obtain the title compound (167 mg, 67%) as a white solid.
   LC-MS 517 [M+H]⁺
   ¹H-NMR (300MHz, DMSO-d₆); δ 3.09-3.16 (m, 2H), 4.67-4.73 (m, 1H), 4.88 (s, 2H), 6.53 (d, J=1.2Hz, 1H), 6.87-6.90 (d, J=8.4Hz, 2H), 7.14-7.17 (d, J=8.7Hz, 2H), 7.63-7.67 (m, 3H), 7.72, (s, 1H), 7.93-7.94 (d, J=9.9Hz, 1H), 8.11-8.14 (dd, J=1.8, 4.8Hz, 2H), 8.27-8.32 (m, 2H), 8.69 (s, 1H), 12.80 (br s, 1H)
   Melting point; 220°C
   Elemental analysis: Calcd. for C₂₇H₂₁N₄O₅, : C 62.73, H 4.09, N 10.84; Found: C 62.46, 4.08, N 11.08

### Example E33

### N-{[7-(4-Chlorophenyl)imidazo[1,2-a]pyridin-2-yl]carbonyl}-O-(3-furylmethyl)tyrosine

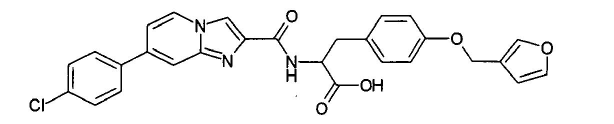

(1) A mixture of 7-(4-chlorophenyl)imidazo[1,2-a]pyridine-2-carboxylic acid (17 mg, 0.06 mmol), methyl O-(3-furylmethyl)tyrosinate hydrochloride (24 mg, 0.07 mmol), (3-dimethylaminopropyl)ethyl carbodiimide hydrochloride (20 mg, 0.10 mmol), 1-hydroxybenzotriazole (14 mg, 0.10 mmol), triethylamine (30 µL, 0.21 mmol), N,N-dimethylformamide (1 mL) and dichloromethane (1 mL) was stirred overnight at room temperature. Ethyl acetate and water were added to separate the solution, and the organic layer was washed twice with water and once with saturated sodium chloride solution, and dried over magnesium sulfate. The drying agent was removed by filtration, and the filtrate was concentrated under reduced pressure. The residue was purified by silica gel column chromatography (eluate: ethyl acetate/hexane = 1/10-1/1) to obtain a white solid. This was dissolved in tetrahydrofuran-methanol (4 mL-2 mL). 1 M sodium hydroxide aqueous solution (125 µL) was added, and stirred for 60 minutes at 60°C. The reaction solution was neutralized with 1 N hydrochloric acid (150 µL), and the precipitated white solid was filtered out and washed successively with water and diethyl ether to obtain the title compound (18 mg, 59%) as a white solid.
   LC-MS 516 [M+H]⁺
   ¹H-NMR (300MHz, DMSO-d₆); δ 3.15-3.18 (m, 2H), 4.67-4.70 (m, 1H), 4.88 (s, 2H), 6.53 (d, J=0.9Hz, 1H), 6.87-6.90 (d, J=8.7Hz, 2H), 7.13-7.15 (d, J=8.7Hz, 2H), 7.37-7.40 (m, 1H), 7.56-7.59 (d, J=8.4Hz, 2H), 7.63-7.64 (t, J=1.5Hz, 1H), 7.74 (s, 1H), 7.85-7.88 (d, J=8.7Hz, 2H), 7.94 (s, 1H), 8.14-8.17 (d, J=7.8Hz, 2H), 8.38 (s, 1H), 8.64-8.66 (d, J=7.2Hz, 1H), 12.80 (br s, 1H) Melting point: 243°C

### Example E34

### O-Benzyl-N-({6-[(E)-2-phenylethenyl]imidazo[1,2-a]pyridin-2-yl}carbonyl)tyrosine

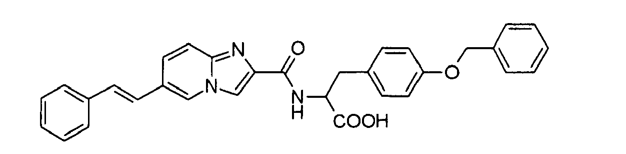

(1) Ethyl 6-[(E)-2-phenylethenyl]imidazo[1,2-a]pyridine-2-carboxylate (0.15 g, 56%) was obtained as a white powder by methods similar to those of Example C169(1) from the ethyl 6-bromoimidazo[1,2-a]pyridine-2-carboxylate (0.25 g, 0.93 mmol) obtained in Reference Example 30(1) and styrene (0.21 mL, 1.86 mmol).
   ¹H-NMR (300MHz, CDCl₃)δ: 1:45(3H, t, J=7.2Hz), 4.47 (2H, q, J=7.2Hz), 7.00 (1H, d, J=16.2Hz), 7.13 (1H, d, J=16.2Hz), 7.31-7.42 (3H, m), 7.51-7.60 (3H, m), 7.68 (1H, d, J=9.6Hz), 8.14-8.17 (2H, m)
   LC-MS 293 [M+H]⁺
(2) A mixture of ethyl 6-[(E)-2-phenylethenyl]imidazo[1,2-a]pyridine-2-carboxylate (0.33 g, 1.13 mmol), tetrahydrofuran (4 mL), ethanol (4 mL), water (2 mL) and lithium hydroxide monohydrate (95 mg, 2.26 mmol) was stirred for 30 minutes at 70°C. The reaction solution was neutralized with 1 N hydrochloric acid, and filtered to obtain 6-[(E)-2-phenylethenyl]imidazo[1,2-a]pyridine-2-carboxylic acid as a white powder. The title compound (0.13 g, 22%) was obtained from this as white crystals by methods similar to those of Example E1(1) and Example E1(2) in sequence.
   ¹H-NMR (300MHz, DMSO-d₆)δ: 3.12-3.15 (2H, m), 4.65-4.72 (1H, m), 5.03 (2H, s), 6.91 (2H, d, J=8.7Hz), 7.15 (2H, d, J=8.7Hz), 7.29-7.43 (10H, m), 7.61-7.65 (3H, m), 7.80 (1H, d, J=9.9Hz), 8.18 (1H, d, J=8.1Hz), 8.37 (1H, s), 8.70 (1H, s), 8.68 (1H, s), 12.92 (1H, br s)
   LC-MS 518 [M+H]⁺
   Melting point: 262-263°C
   Elemental analysis: Calcd. for C₃₂H₂₇N₃O₄, : C 74.26, H 5.26, N 8.12; Found: C 74.20, H 5.32, N 8.01

### Example E35

### O-Benzyl-N- 4{[6-(4-chlorophenyl)-7-methylimidazo[1,2-a]pyridin-2-yl]carbonyl}tyrosine

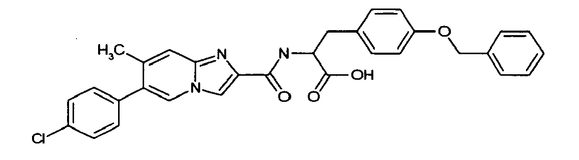

(1) A mixture of 6-(4-chlorophenyl)-7-methylimidazo[1,2-a]pyridine-2-carboxylic acid (200 mg), methyl O-benzyltyrosinate hydrochloride (225 mg), (3-dimethylaminopropyl)ethyl carbodiimide hydrochloride (161 mg), 1-hydroxybenzotriazole (114 mg), triethylamine (0.29 mL) and N,N-dimethylformamide (6 mL) was stirred overnight at room temperature. Water was added to the reaction solution, which was then extracted with diethyl ether. The extract was washed with hydrochloric acid and saturated sodium chloride solution and then dried over anhydrous magnesium sulfate. The solvent was evaporated under reduced pressure, and the residue was purified by silica gel column chromatography and recrystallized from ethyl acetate-hexane to obtain methyl O-benzyl-N-{[6-(4-chlorophenyl)-7-methylimidazo[1,2-a]pyridin-2-yl]carbonyl}tyrosinate (290 mg) as colorless crystals.
   ¹H-NMR (300MHz, CDCl₃); δ 2.25 (3H, s), 3.12-3.25 (2H, m), 3.72 (3H, s), 5.03 (2H, s), 5.03-5.10 (1H, m), 6.89 (2H, d, J=8.7Hz), 7.13 (2H, d, J=8.7Hz), 7.24-7.46 (10H, m), 7.78 (1H, d, J=8.4Hz), 7.95 (1H, s), 8.05 (1H, s)
(2) 1 N Sodium hydroxide aqueous solution (1 mL) and water (7 mL) were added at 60°C to a tetrahydrofuran-methanol mixed solution (6 mL-3 mL) of the compound (250 mg) obtained in (1), and stirred at that temperature for 20 minutes. The reaction solution was neutralized with 1 N hydrochloric acid, and the precipitated crystals were filtered out and washed successively with water, methanol and diethyl ether to obtain the title compound (203 mg) as colorless crystals. Melting point: 248-249°C
   ¹H-NMR (300MHz, DMSO-d₆); δ 2.24 (3H, s), 3.10-3.15 (2H, m), 4.63-4.73 (1H, m), 5.03 (2H, s), 6.90 (2H, d, J=8.7Hz), 7.15 (2H, d, J=8.7Hz), 7.27-7.5 7 (10H, m), 8.17 (1H, d, J=8.1Hz), 8.26 (1H, s), 8.47 (1H, s), 12.93 (1H, br s)
   Elemental analysis: Calcd. for C₃₁H₂₆ClN₃O₄, : C 68.95, H 4.85, N 7.78; Found: C 68.73, H 4.83, N 7.87

### Example E36

### O-Benzyl-N-{[6-(4-chlorophenyl)-5,7-dimethylimidazo[1,2-a]pyridin-2-yl]carbonyl tyrosine

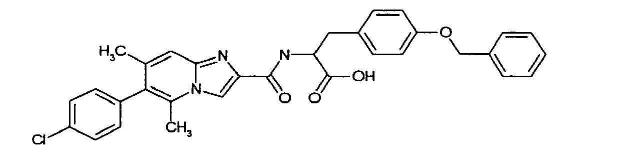

(1) A mixture of 6-(4-chlorophenyl)-5,7-dimethylimidazo[1,2-a]pyridine-2-carboxylic acid (210 mg), methyl O-benzyltyrosinate hydrochloride (225 mg), (3-dimethylaminopropyl)ethyl carbodiimide hydrochloride (161 mg), 1-hydroxybenzotriazole (114 mg), triethylamine (0.29 mL) and N,N-dimethylformamide (6 mL) was stirred overnight at room temperature. Water was added to the reaction solution, which was then extracted with diethyl ether. The extract was washed with hydrochloric acid and saturated sodium chloride solution and then dried over anhydrous magnesium sulfate. The solvent was evaporated under reduced pressure, and the residue was purified by silica gel column chromatography and recrystallized from ethyl acetate-hexane to obtain methyl O-benzyl-N-{[6-(4-chlorophenyl)-5,7-dimethylimidazo[1,2-a]pyridin-2-yl]carbonyl}tyrosinate (287 mg) as colorless crystals.
   ¹H-NMR (300MHz, CDCl₃); δ 2.07 (3H, s), 2.32 (3H, s), 3.11-3.29 (2H, m), 3.72 (3H, s), 5.03 (2H, s), 5.03-5.10 (1H, m), 6.89 (2H, d, J=8.7Hz), 7.10-7.18 (4H, m), 7.28-7.48 (8H, m), 7.80 (1H, d, J=8. 4Hz), 8.03 (1H, s)
(2) 1 N Sodium hydroxide aqueous solution (1 mL) and water (7 mL) were added at 60°C to a tetrahydrofuran-methanol mixed solution (6 mL-3 mL) of the compound (250 mg) obtained in (1), and stirred at that temperature for 20 minutes. The reaction solution was neutralized with 1 N hydrochloric acid, and the precipitated crystals were filtered out and washed successively with water, methanol and diethyl ether to obtain the title compound (180 mg) as colorless crystals. Melting point: 250-251°C
   ¹H-NMR (300MHz, DMSO-d₆); δ 2.03 (3H, s), 2.33 (3H, s), 3.10-3.20 (2H, m), 4.65-4,75 (1H, m), 5.03 (2H, s), 6.90 (2H, d, J=8.7Hz), 7.15 (2H, d, J=8.7Hz), 7.28-7.47 (8H, m), 7.57 (2H, d, J=8.7Hz), 8.18 (1H, d, J=8.4Hz), 8.21 (1H, s), 12.95 (1H, br s)
   Elemental analysis: Calcd. for C₃₂H₂₈ClN₃O₄, : C 69.37, H 5.09, N 7.58; Found: C 69.35, H 5.10, N 7.64

### Example E37

### O-Benzyl-N-{[3-chloro-6-(4-chlorophenyl)imidazo[1,2-a]pyridin-2-yl]carbonyl}tyrosine

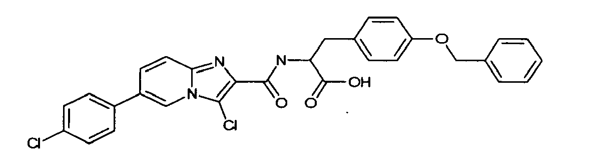

(1) A mixture of 3-chloro-6-(4-chlorophenyl)imidazo[1,2-a]pyridine-2-carboxylic acid (92 mg), methyl O-benzyltyrosinate hydrochloride (97 mg), (3-dimethylaminopropyl)ethyl carbodiimide hydrochloride (69 mg), 1-hydroxybenzotriazole (49 mg), triethylamine (0.12 mL) and N,N-dimethylformamide (5 mL) was stirred overnight at room temperature. Water was added to the reaction solution, which was then extracted with diethyl ether. The extract was washed with hydrochloric acid and saturated sodium chloride solution and then dried over anhydrous magnesium sulfate. The solvent was evaporated under reduced pressure, and the residue was purified by silica gel column chromatography to obtain methyl O-benzyl-N-{[3-chloro-6-(4-chlorophenyl)imidazo[1,2-a]pyridin-2-yl]carbonyl}tyrosinate (147 mg) as pale yellow crystals.
   ¹H-NMR (300MHz, CDCl₃); δ 3.13-3.28 (2H, m), 3.73 (3H, s), 5.03 (2H, s), 5.03-5.10 (1H, m), 6.90 (2H, d, J=8.7Hz), 7.13 (2H, d, J=8.7Hz), 7.30-7.56 (10H, m), 7.65 (1H, dd, J=0.6, 9.3Hz), 7.82 (1H, d, J=8.1 Hz), 8.24-8.26 (1H, m)
(2) 1 N Sodium hydroxide aqueous solution (1 mL) and water (5 mL) were added at 60°C to a tetrahydrofuran-methanol mixed solution (3 mL-3 mL) of the compound (135 mg) obtained in (1), and stirred at that temperature for 20 minutes. The reaction solution was neutralized with 1 N hydrochloric acid, and the precipitated crystals were filtered out and washed successively with water, methanol and diethyl ether to obtain the title compound (75 mg) as colorless crystals. Melting point: 242-244°C
   ¹H-NMR (300MHz, DMSO-d₆); δ 3.10-3.20 (2H, m), 4.60-4.70 (1H, m), 5.03 (2H, s), 6.91 (2H, d, J=8.4Hz), 7.16 (2H, d, J=8.4Hz), 7.26-7.44 (5H, m), 7.58 (2H, d, J=8.4Hz), 7.77-7.89 (4H, m), 8.31 (1H, d, J=8.1Hz), 8.60 (1H, s), 12.98 (1H,brs)
   Elemental analysis: Calcd. for C₃₀H₂₃Cl₂N₃O₄**•**0.25 H₂O), : C 63.78, H 4.19, N 7.44; Found: C 63.52, H 4.40, N 7.28

### Example E38

### 5-Bromo-2-({[6-(4-chlorophenyl)imidazo[1,2-a]pyridin-2-yl]carbonyl}amino)indan-2-carboxylic acid

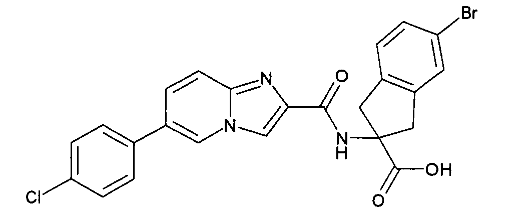

(1) A mixture of 6-(4-chlorophenyl)imidazo[1,2-b]pyridazine-2-carboxylic acid (164 mg, 0.60 mmol), methyl 2-amino-5-bromoindan-2-carboxylate hydrochloride (142 mg, 0.50 mmol), (3-dimethylaminopropyl)ethyl carbodiimide hydrochloride (192 mg, 1.00 mmol), 1-hydroxybenzotriazole (135 mg, 1.00 mmol), triethylamine (300 µL, 2.1 mmol), N,N-dimethylformamide (2 mL) and dichloromethane (6 mL) was stirred overnight at room temperature. Ethyl acetate and water were added to separate the solution, and the organic layer was washed twice with water and once with saturated sodium chloride solution, and dried over magnesium sulfate. The drying agent was removed by filtration, and the filtrate was concentrated under reduced pressure The residue was purified by silica gel column chromatography (eluate: ethyl acetate/hexane= 1/10-1/1) and recrystallized from a chloroform-hexane mixed solvent to obtain ethyl 5-bromo-2-({[6-(4-chlorophenyl)imidazo[1,2-a]pyridin-2-yl]carbonyl}amino)indan-2-carboxylate (242 mg, 92%) as a white solid.
   LC-MS 540 [M+H]⁺
   ¹H-NMR (300MHz, DMSO-d₆); δ 1.10-1.15 (t, J=7.2Hz, 3H), 3.43-3.63 (m, 4H), 4.06-4.14 (q, J=7.2Hz, 2H), 7.19-7.22 (d, J=8.1Hz, 1H), 7.35-7.37 (d, J=8.1Hz, 1H), 7.44 (s, 1H), 7.57-7.76 (m, 6H), 8.37 (s, 1H), 8.87 (s, 1H), 8.98 (s, 1H)
(2) 1 M Sodium hydroxide aqueous solution (900 µL) was added to a tetrahydrofuran-methanol mixed solution (4 mL-2 mL) of ethyl 5-bromo-2-({[6-(4-chlorophenyl)imidazo[1,2-a]pyridin-2-yl]carbonyl}amino)indan-2-carboxylate (243 mg, 0.45 mmol), and stirred for 30 minutes at 60°C. The reaction solution was neutralized with 1 N hydrochloric acid (950 µL), and the precipitated white solid was filtered out and washed successively with water and diethyl ether to obtain the title compound (198 mg, 86%) as a white solid.
   LC-MS 512 [M+H]⁺
   ¹H-NMR (300MHz, DMSO-d₆); δ 3.49-3.55 (m, 4H), 7.18-7.21 (d, J=8.1Hz, 1H), 7.34-7.36 (d, J=8.1Hz, 1H), 7.43 (s, 1H), 7.57-8.00 (d, J=8.7Hz, 2H), 7.64-7.69 (m, 2H), 7.74-7.77 (d, J=8.7Hz, 2H), 8.36 (s, 1H), 8.70 (s, 1H), 8.98-8.99 (d, J=1.2Hz, 1H)
   Melting point: 328°C
   Elemental analysis: Calcd. for C₂₄H₁₇N₃O₃BrCl, : C 56.44, H 3.35, N 8.23; Found: C 56.59, H 3.35, N 8.41

### Example E39

### N-[4-(Benzyloxy)benzyl]-N-{[6-(4-chlorophenyl)imidazo[1,2-a]pyridin-2-yl]carbonyl}glycine

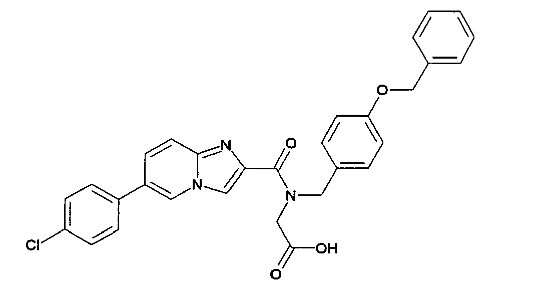

(1) A mixture of 1-(benzyloxy)-4-(chloromethyl)benzene (2.32 g, 10 mmol), methyl glycinate hydrochloride (1.26 g, 10 mmol), triethylamine (2.79 mL, 20 mmol) and dimethyl formamide (50 mL) was stirred for 3 hours at 60°C. Ethyl acetate and water were added to the reaction solution, and the organic layer was isolated. The organic layer was washed with water and saturated sodium chloride solution, and dried by addition of magnesium sulfate. The drying agent was removed by filtration, and the filtrate was concentrated under reduced pressure. The residue was purified by basic silica gel chromatography (eluate: ethyl acetate/hexane = 2/98-100/0) to obtain methyl N-[4-(benzyloxy)benzyl]glycinate (430 mg, 15%) as a colorless oil.
   LC-MS 286 [M+H]⁺
   ¹H-NMR (300MHz, CDCl₃); δ 3.41 (s, 2H), 3.72 (s, 3H), 3.74 (s, 2H), 5.06 (s, 2H), 6.92-6.95 (d, J=8.7Hz, 2H), 7.23-7.44 (m, 7H)
(2) A mixture of 6-(4-chlorophenyl)imidazo[1,2-b]pyridazine-2-carboxylic acid (164 mg, 0.60 mmol), methyl N-[4-(benzyloxy)benzyl]glycinate (143 mg, 0.50 mmol), (3-dimethylaminpropyl)ethyl carbodiimide hydrochloride (192 mg, 1.00 mmol), 1-hydroxybenzotriazole (135 mg, 1.00 mmol), triethylamine (300 µL, 2.1 mmol), N,N-dimethylformamide (2 mL) and dichloromethane (6 mL) was stirred overnight at room temperature. Ethyl acetate and water were added to separate the solution, and the organic layer was washed twice with water and once with saturated sodium chloride solution, and dried over magnesium sulfate. The drying agent was removed by filtration, and the filtrate was concentrate under reduced pressure. The residue was purified by silica gel column chromatography (eluate: ethyl acetate/hexane = 1/10-1/1) and recrystallized from an ethyl acetate-hexane mixed solvent to obtain methyl N-[4-(benzyloxy)benzyl]-N-{[6-(4-chlorophenyl)imidazo[1,2-a]pyridin-2-yl]carbonyl}glycinate (192 mg, 71%) as a white solid.
   LC-MS 540 [M+H]⁺
   ¹H-NMR (300MHz, DMSO-d₆); δ 3.61-3.63 (d, J=6.6Hz, 3H), 4.01 (s, 1H), 4.65 (s, 1H), 4.87 (s, 1H), 5.08-5.09 (d, J=2.4Hz, 2H), 5.39 (s, 1H), 6.97-7.00 (d, J=8.4Hz, 2H), 7.27-7.46 (m, 7H), 7.57-7.60 (d, J=8.7Hz, 2H), 7.66-7.78 (m, 4H), 8.43 (s, 1H), 8.98-9.00 (d, J=6.9Hz, 1H)
(3) 1 M Sodium hydroxide aqueous solution (711 µL) was added to a tetrahydrofuran-methanol mixed solution (6 mL-2 mL) of methyl N-[4-(benzyloxy)benzyl]-N-{[6-(4-chlorophenyl)imidazo[1,2-a]pyridin-2-yl]carbonyl}glycinate (192 mg, 0.36 mmol), and stirred for 60 minutes at 60°C. The reaction solution was neutralized with 1 N hydrochloric acid (730 µL), and the precipitated white solid was filtered out and washed successively with water and diethyl ether to obtain the title compound (165 mg, 89%) as a white solid.
   LC-MS 526 [M+H]⁺
   ¹H-NMR (300MHz, DMSO-d₆); δ 3.91 (s, 1H), 4.63 (s, 1H), 4.80 (s, 1H), 5.08 (s, 2H), 5.36 (s, 1H), 6.97-7.00 (d, J=8.7Hz, 2H), 7.27-7.46 (m, 7H), 7.57-7.60 (d, J=8.7Hz, 2H), 7.67-7.70 (d, J=7.8Hz, 2H), 7.74-7.77 (m, 2H), 8.41-8.42 (d, J=2.4Hz, 1H), 8.99-9.00 (d, J=5.4Hz, 1H), 12.60 (br s, 1H)
   Melting point: 213-214°C
   Elemental analysis: Calcd. for C₃₀H₂₄N₃O₄Cl (cont. 0.1 mol H₂O), : C 68.27, H 4.62, N 7.96; Found: C 68.19, H 4.78, N 8.18

### Example E40

### O-Benzyl-N-{[6-(2,4-dichlorophenyl)imidazo[1,2-a]pyridin-2-yl]carbonyl}tyrosine

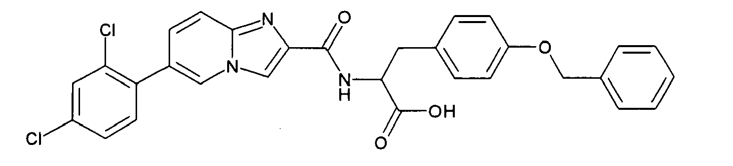

(1) A mixture of methyl O-benzyl-N-[(6-bromoimidazo[1,2-a]pyridin-2-yl)carbonyl]tyrosinate (210 mg, 0.5 mmol), 2,4-dichlorophenyl boronic acid (114 mg, 0.6 mmol), tetrakis(triphenylphosphine)palladium (36.6 mg, 0.04 mmol), dicyclohexyl(2'6'-diisopropoxybiphenyl-2-yl) phosphine (19 mg, 0.04 mmol), 2 M sodium carbonate aqueous solution (0.5 mL) and dimethoxyethane (5 mL) was heated using a microwave reactor (150°C, 5 bar, 6 min. x 2). The reaction solution was filtered, and the filtrate was diluted with ethyl acetate and washed with saturated sodium hydrogencarbonate aqueous solution and saturated sodium chloride solution and dried by addition of anhydrous magnesium sulfate. The drying agent was removed by filtration, and the filtrate was concentrated under reduced pressure. The residue was purified by basic silica gel column chromatography (eluate: ethyl acetate/hexane = 1/10-1/1) and recrystallized from ethyl acetate to obtain methyl O-benzyl-N-{[6-(2,4-dichlorophenyl)imidazo[1,2-a]pyridin-2-yl]carbonyl}tyrosinate (36.2 mg, 13%) as a white solid.
   LC-MS 574 [M+H]⁺
   ¹H-NMR (300MHz, DMSO-d₆); δ 2.88-3.00 (m, 2H), 3.66 (s, 3H), 4.71-4.78 (m, 1H), 5.03 (s, 2H), 6.89-6.92 (d, J=8.7Hz, 2H), 7.15-7.18 (d, J=8.4Hz, 2H), 7.30-7.46 (m, 6H), 7.59 (s, 2H), 7.68-7.71 (d, J=9.6Hz, 1H), 7.82 (s, 1H), 8.38 (s, 1H), 8.46-8.49 (d, J=8.1Hz, 1H), 8.72 (s, 1H)
(2) 1 M Sodium hydroxide aqueous solution (125 µL) was added to a tetrahydrofuran-methanol mixed solution (1 mL-1 mL) of methyl O-benzyl-N-{[6-(2,4-dichlorophenyl)imidazo[1,2-a]pyridin-2-yl]carbonyl}tyrosinate (36 mg, 0.06 mmol), and stirred for 60 minutes at 60°C. The reaction solution was neutralized with 1 N hydrochloric acid (150 µL), and the precipitated white solid was filtered out and washed successively with water and diethyl ether to obtain the title compound (27 mg, 77%) as a white solid.
   LC-MS 560 [M+H]⁺
   ¹H-NMR (300MHz, DMSO-d₆); δ 3.08-3.18 (m, 2H), 4.64-4.71 (m, 1H), 5.03 (s, 2H), 6.88-6.91 (d, J=8.7Hz, 2H), 7.13-7.16 (d, J=8.7Hz, 2H), 7.28-7.45 (m, 6H), 7.58 (s, 2H), 7.65-7.68 (d, J=8.7Hz, 1H), 7.81 (s, 1H), 8.22-8.25 (d, J=8.1Hz, 1H), 8.39 (s, 1H), 8.72 (s, 1H), 12.80 (br s, 1H)
   Melting point: 237-239°C

### Example E41

### O-Benzyl-N-({6-[(E)-2-(4-chlorophenyl)ethenyl]imidazo[1,2-a]pyridin-2-yl}carbonyl)tyrosine

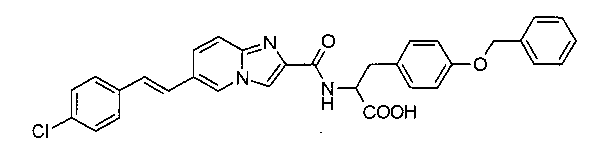

Ethyl 6-[(E)-2-(4-chlorophenyl)ethenyl]imidazo[1,2-a]pyridine-2-carboxylate (0.44 g, 72%) was obtained by methods similar to those of Example C169-(1) from the ethyl 6-bromoimidazo[1,2-a]pyridine-2-carboxylate (0.50 g, 1.87 mmol) obtained in Reference Example 30(1) and 4-chlorostyrene (0.48 mL, 3.74 mmol), and the title compound (0.11 g, 11%) was then obtained as colorless crystals by methods similar to those of Example E34-(2).
¹H-NMR (300MHz, DMSO-d₆)δ: 3.12-3.15 (2H, m), 4.15-4.29 (1H, m), 5.03 (2H, s), 6.91 (2H, d, J=8.7Hz), 7.16 (2H, d, J=8.7Hz), 7.31-7.48 (9H, m), 7.62-7.66 (3H, m), 7.79 (1H, dd, J=1.8, 9.9Hz), 8.18 (1H, d, J=8.1Hz), 8.3 (1H, s), 8.67 (1H, s), 12.94 (1H, br s)
LC-MS 553 [M+H]⁺
Melting point: 267-270°C
Elemental analysis: Calcd. for C₃₂H₂₆N₃O₄Cl, : C 69.63, H 4.75, N 7.61; Found: C 69.55, H 4.91, N 8.58

### Example E42

### O-Benzyl-N-({6-[(E)-2-(4-methylphenyl)ethenyl]imidazo[1,2-a]pyridin-2-yl}carbonyl) tyrosine

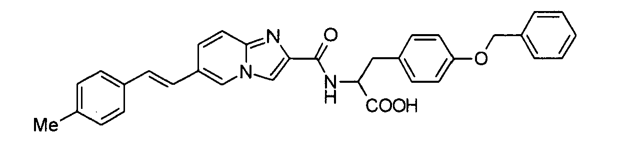

(1) Ethyl 6-[(E)-2-(4-methylphenyl)ethenyl]imidazo[1,2-a]pyridine-2-carboxylate (0.44 g, 72%) was obtained as a white powder by methods similar to those of Example C169-(1) from the ethyl 6-bromoimidazo[1,2-a]pyridine-2-carboxylate (0.50 g, 1.87 mmol) obtained in Reference Example 30(1) and 4-methylsturene (0.48 mL, 3.74 mmol).
   ¹H-NMR (300MHz, CDCl₃)δ: 1.45(3H, t, J=7.2Hz), 4.47 (2H, q, J=7.2Hz), 7.03 (1H, d, J=16.2Hz), 7.06 (1H, d, J=16.2Hz), 7.36 (2H, d, J=8.4Hz), 7.44 (2H, d, J=8.4Hz), 7.55 (1H, dd, J=1.8, 9.6Hz), 7.68 (1H, d, J=9.6Hz), 8.14 (1H, s), 8.16 (1H, s)
   LC-MS 307 [M+H]⁺
(2) A mixture of ethyl 6-[(E)-2-(4-methylphenyl)ethenyl]imidazo[1,2-a]pyridine-2-carboxylate (0.37 g, 1.13 mmol), tetrahydrofuran (4 mL), ethanol (1 mL), water (3 mL) and lithium hydroxide monohydrate (95 mg, 2.26 mmol) was stirred for 16 hours at room temperature. The reaction solution was neutralized with 1 N hydrochloric acid and filtered to obtain 6-[(E)-2-(4-methylphenyl)ethenyl]imidazo[1,2-a]pyridine-2-carboxylic acid as a white powder. From this, the title compound (0.11 g, 22%) was obtained as colorless crystals by methods similar to those of Example E1(1) and Example E1(2) in sequence.
   ¹H-NMR (300MHz, DMSO-d₆)δ:2.32 (3H, s), 3.12-3.15 (2H, m), 4.65-4.72 (1H, m), 5.03 (2H, s), 6.90 (2H, d, J=8.7Hz), 7.14-7.44 (1H, m), 7.50 (2H, d, J=8.1Hz), 7.62-7.66 (1H, d, J=9. 3 Hz), 7.79 (1H, d, J=9.6Hz), 8.17 (1H, d, J=8.4Hz), 8.35 (1H, s), 8.65 (1H, s), 12. 94 (1H, br s)
   LC-MS 532 [M+H]⁺
   Melting point: 266-267°C
   Elemental analysis: Calcd. for C₃₃H₂₉N₃O₄, : C 74.56, H 5.50, N 7.90; Found: C 74.48, H 5.56, N 7.72

### Example E43

### O-Benzyl-N-({6-[(E)-2-(4-methoxyphenyl)ethenyl]imidazo[1,2-a]pyridin-2-yl}carbonyl)tyrosine

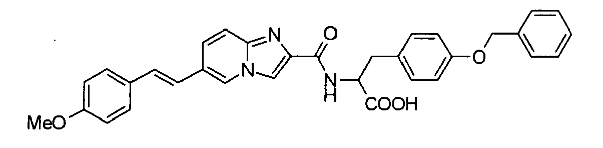

(1) Ethyl 6-[(E)-2-(4-methoxyphenyl)ethenyl]imidazo[1,2-a]pyridazine-2-carboxylate (0.27 g, 45%) was obtained as a white powder by methods similar to those of Example C167(1) from the ethyl 6-bromoimidazo[1,2-a]pyridine-2-carboxylate (0.50 g, 1.87 mmol) obtained in Reference Example 30(1) and 4-methoxystyrene (0.50 g, 3.74 mmol).
   ¹H-NMR (300MHz, CDCl₃)δ: 1.45 (3H, t, J=7.2Hz), 3.84 (3H, s), 4.46 (2H, q, J=7.2Hz), 6.84-6.94 (3H, m), 7.05 (1H, d, J=16.2Hz), 7.45 (2H, d, J=8.7Hz), 7.55 (1H, d, J=9.6Hz), 7.65 (1H, d, J=9.6Hz), 8.09 (1H, s), 8.15 (1H, s)
   LC-MS 323 [M+H]⁺
(2) A mixture of ethyl 6-[(E)-2-(4-methoxyphenyl)ethenyl]imidazo[1,2-a]pyridazine-2-carboxylate (0.24 g, 0.74 mmol), tetrahydrofuran (3 mL), ethanol (0.5 mL), water (2 mL) and lithium hydroxide monohydrate (62 mg, 1.48 mmol) was stirred for 1 hour at 60°C. The reaction solution was neutralized with 1 N hydrochloric acid and filtered to obtain 6-[(E)-2-(4-methoxyphenyl)ethenyl]imidazo[1,2-a]pyridazine-2-carboxylic acid as a white powder. From this, the title compound (0.18 g, 22%) was obtained as white crystals by methods similar to those of Example E1(1) and Example E1(2) in sequence.
   ¹H-NMR (300MHz, DMSO-d₆)δ: 3.12-3.15 (2H, m), 3.79 (3H, s), 4.65-4.72 (1H, m), 5.03 (2H, s), 6.90 (2H, d, J=8.7Hz), 6.98 (2H, d, J=8.7Hz), 7.07-7.44 (9H, m), 7.54-7.62 (3H, m), 7.77 (1H, d, J=9.9Hz), 8.16 (1R, d, J=8.1Hz), 8.35 (1H, s), 8.63 (1H, s), 12.94 (1H, br s)
   LC-MS 548 [M+H]⁺
   Melting point: 262-263°C
   Elemental analysis: Calcd. for C₃₃H₂₉N₃O₅, : C 72.38, H 5.34, N 7.67; Found: C 72.31, H 5.49, N 7.60

### Example E44

### O-Benzyl-N-({6-[4-(trifluoromethoxy)phenyl)]imidazo[1,2-a]pyridin-2-yl}carbonyl) tyrosine

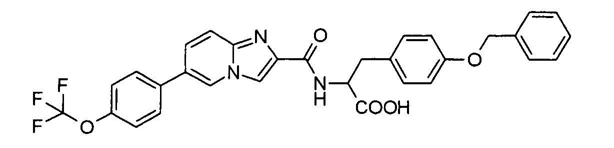

(1) A mixture of the ethyl 6-bromoimidazo[1,2-a]pyridine-2-carboxylate (0.50 g, 1.87 mmol) obtained in Reference Example 30(1), 4-trifluoromethoxyphenylboronic acid (0.42 g, 2.06 mmol), 1,2-dimethoxyethane (10 mL), 2 M sodium carbonate aqueous solution and tetrakis triphenylphosphine palladium (0) (69 mg, 0.06 mol) was heated for 6 minutes at 150°C by a microwave reactor. The reaction solution was diluted with ethyl acetate, washed with water, and dried over anhydrous magnesium sulfate. The solvent was evaporated, and the residue was purified by silica gel column chromatography (eluate: 5-50% ethyl acetate-hexane) to obtain ethyl 6-[4-(trifluoromethoxy)phenyl)]imidazo[1,2-a]pyridine-2-carboxylate (0.29 g, 46%).
   ¹H-NMR (300MHz, CDCl₃)δ: 1.45 (3H, t, J=7.2Hz), 4.90 (2H, q, J=7.2Hz), 7.35 (2H, d, J=8.1 Hz), 7.47 (1H, dd, J=1.5, 9.3Hz), 7.58 (2H, d, J=8.7Hz), 7.77 (1H, d, J=9.3Hz), 8.24 (1H, s), 8.28 (1H, s)
   LC-MS 339 [M+H]⁺
(2) A mixture of ethyl 6-[4-(trifluoromethoxy)phenyl]imidazo[1,2-a]pyridine-2-carboxylate (0.26 g, 0.77 mmol), tetrahydrofuran (5 mL), ethanol (1 mL), water (3 mL) and lithium hydroxide monohydrate (65 mg, 1.54 mmol) was stirred for 4 hours at room temperature. The reaction solution was neutralized with 1 N hydrochloric acid and filtered to obtain 6-[4-(trifluoromethoxy)phenyl)]imidazo[1,2-a]pyridine-2-carboxylic acid as a white powder. From this, the title compound (0.22 g, 50%) was obtained as colorless crystals by methods similar to those of Example E1(1) and Example E 1 (2) in sequence.
   ¹H-NMR (300MHz, DMSO-d₆)δ: 3.13-3.19 (2H, m), 4.66-4.73 (1H, m), 5.03 (2H, s), 6.90 (2H, d, J=8.7Hz), 7.15 (2H, d, J=8.7Hz), 7.28-7.44 (5H, m), 7.52 (2H, d, J=8.1Hz), 7.72 (2H, s), 7.85 (2H, d, J=9.0Hz), 8.23 (1H, d, J=8.1Hz), 8.36 (1H, s), 8.98 (1H, s), 12.95 (1H, br s)
   LC-MS 576 [M+H]⁺
   Melting point: 270-271°C
   Elemental analysis: Calcd. for C₃₁H₂₄N₃O₅F₃, : C 64.69, H 4.20, N 7.30; Found: C 64.57, H 4.28, N 7.25

### Example E45

### O-Benzyl-N-{[6-(4-isopropoxyphenyl)imidazo[1,2-a]pyridin-2-yl]carbonyl} tyrosine

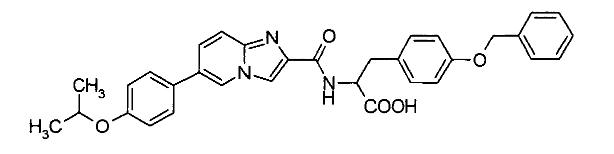

(1) Ethyl 6-(4-isopropoxy)phenyl]imidazo[1,2-a]pyridine-2-carboxylate (0.32 g, 52%) was obtained as a white powder by methods similar to those of Example E44(1) from the ethyl 6-bromoimidazo[1,2-a]pyridine-2-carboxylate (0.50 g, 1.87 mmol) obtained in Reference Example 30(1) (0.50 g, 1.87 mmol) and 4-isopropoxyphenylboronic acid (0.37 g, 2.06 mmol).
   ¹H-NQ4R (300MHz, CDCl₃)δ: 1.37 (6H, d, J=6.0Hz), 1.45 (3H, t, J=7.2Hz), 4.48 (2H, q, J=7.2Hz), 4.57-4.65 (1H, m), 6.98 (2H, d, J=8.7Hz), 7.31 (1H, dd, J=1.5, 9.3Hz), 7.46 (2H, d, J=8.7Hz), 7.59 (1H, d, J=9.6Hz), 8.21 (1H, s), 8.29 (1H, s)
   LC-MS 325 [M+H]⁺
(2) A mixture of ethyl 6-(4-isopropoxy)phenyl]imidazo[1,2-a]pyridine-2-carboxylate (0.28 g, 0.86 mmol), tetrahydrofuran (4 mL), ethanol (0.5 mL), water (3 mL) and lithium hydroxide monohydrate (72 mg, 1.72 mmol) was stirred for 2 hours at room temperature. The reaction solution was neutralized with 1 N hydrochloric acid and filtered to obtain 6-[4-(isopropoxy)phenyl]imidazo[1,2-a]pyridine-2-carboxylic acid as a white powder. From this, the title compound (0.11 g, 23%) was obtained as colorless crystals by methods similar to those of Example E1(1) and Example E1 (2) in sequence.
   ¹H-NMR (300MHz, DMSO-d₆)δ: 1.29 (6H, d, J=6.0Hz), 3.12-3.15 (2H, m), 4.64-4.72 (1H, m), 5.03 (2H, s), 6.90 (2H, d, J=8.4Hz), 7.05 (2H, d, J=8.4Hz), 7.15 (2H, d, J=8.4Hz), 7.28-7.43 (5H, m), 7.61-7.66 (4H, m), 8.19 (1H, d, J=8.4Hz), 8.32 (1H, s), 8.86 (1H, s), 12.93 (1H, br s)
   LC-MS 550 [M+H]⁺
   Melting point: 263°C (decomposes)
   Elemental analysis: Calcd. for C₃₃H₃₁N₃O₅, : C 72.12, H 5.68, N 7.65; Found: C 72.05, H 5.71, N 7.60

### Example E46

### O-Benzyl-N-{[6-(2,6-difluoro-4-methoxyphenyl)imidazo[1,2-a]pyridin-2-yl]carbonyl}tyrosine

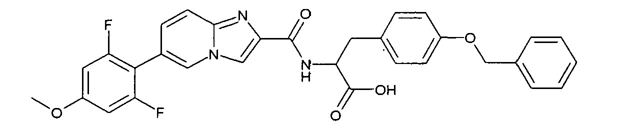

(1) A mixture of methyl O-benzyl-N-[(6-bromoimidazo[1,2-a]pyridin-2-yl)carbonyl]tyrosinate (210 mg, 0.5 mmol), (2,6-difluoro-4-methoxyphenyl)boronic acid (113 mg, 0.6 mmol), tetrakis(triphenylphosphine)palladium (36.6 mg, 0.04 mmol), dicyclohexyl(2',6'-diisopropoxybiphenyl-2-yl)phosphine (19 mg, 0.04 mmol), 2 M sodium carbonate aqueous solution (1 mL) and dimethoxyethane (10 mL) was heated with a microwave reactor (150 °C, 5 bar, 16 minutes). The reaction solution was filtered, and the filtrate was diluted with ethyl acetate, washed with saturated sodium hydrogencarbonate aqueous solution and saturated sodium chloride solution, and dried by addition of magnesium sulfate. The drying agent was removed by filtration, and the filtrate was concentrated under reduced pressure. The residue was purified by basic silica gel column chromatography (eluate: ethyl acetate/hexane = 1/10-1/1) and recrystallized from an ethyl acetate-diisopropyl ether mixed solvent to obtain methyl O-benzyl-N-{[6-(2,6-difluoro-4-methoxyphenyl)imidazo[1,2-a]pyridin-2-yl]carbonyl}tyrosinate (46 mg, 16%) as a white solid.
   LC-MS 572 [M+H]⁺
   ¹H-NMR (300MHz, DMSO-d₆); δ 3.13-3.15 (d, J=7.2Hz, 2H), 3.66 (s, 3H), 3.85 (s, 3H), 4.71-4.78 (m, 1H), 5.03 (s, 2H), 6.89-6.97 (m, 4H), 7.15-7.18 (d, J=8.4Hz, 2H), 7.30-7.43 (m, 6H), 7.69-7.72 (d, J=9.6Hz, 1H), 8.40 (s, 1H), 8.46-8.49 (d, J=8.1Hz, 1H), 8.74 (s, 1H)
(2) 1 M Sodium hydroxide aqueous solution (164 µL) was added to a tetrahydrofuran-methanol mixed solution (2 mL-1 mL) of methyl O-benzyl-N-{[6-(2,6-difluoro-4-methoxyphenyl)imidazo[1,2-a]pyridin-2-yl]carbonyl}tyrosinate (46 mg, 0.08 mmol), and stirred for 60 minutes at 60°C. The reaction solution was neutralized with 1 N hydrochloric acid (180 µL), and the precipitated white solid was filtered out and washed successively with water and diethyl ether to obtain the title compound (37 mg, 81%) as a white solid.
   LC-MS 558 [M+H]⁺
   ¹H-NMR (300MHz, DMSO-d₆); δ 3.05-3.15 (m, 2H), 3.85 (s, 3H), 4.62-4.69 (m, 1H), 5.03 (s, 2H), 6.88-6.96 (m, 4H), 7.13-7.16 (d, J=8.4Hz, 2H), 7.28-7.43 (m, 6H), 7.69-7.72 (d, J=9.6Hz, 1H), 8.22-8.25 (d, J=8.1Hz, 1H), 8.40 (s, 1H), 8.74 (s, 1H), 12.80 (s, 1H)
   Melting point: 219°C

### Example E47

### N-{[7-(4-Chlorophenyl)imidazo[1,2-a]pyridin-2-yl]carbonyl}-O-(2-fluorobenzyl)tyrosine

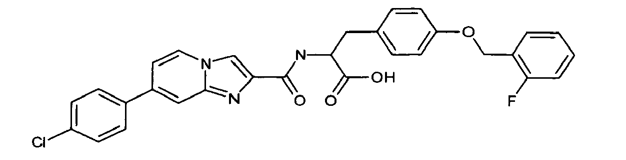

(1) A mixture of 7-(4-chlorophenyl)imidazo[1,2-a]pyridine-2-carboxylic acid (95 mg), methyl O-(2-fluorobenzyl)tyrosinate hydrochloride (119 mg), (3-dimethylaminopropyl)ethyl carbodiimide hydrochloride (81 mg), 1-hydroxybenzotriazole (57 mg), triethylamine (0.15 mL) and N,N-dimethylformamide (4 mL) was stirred overnight at room temperature. Water was added to the reaction solution, which was then extracted with ethyl acetate. The extract was washed with hydrochloric acid and saturated sodium chloride solution and dried over anhydrous magnesium sulfate. The solvent was evaporated under reduced pressure, and the residue was purified by silica gel column chromatography to obtain methyl N-{[7-(4-chlorophenyl)imidazo[1,2-a]pyridin-2-yl]carbonyl}-O-(2-fluorobenzyl)tyrosinate (150 mg) as pale yellow crystals.
   ¹H-NMR (300MHz, CDCl₃); δ 3.12-3.27 (2H, m), 3.73 (3H, s), 5.03-5.10 (1H, m), 5.10 (2H, s), 6.91 (2H, d, J=8.7Hz), 7.03-7.18 (5H, m), 7.26-7.35 (1H, m), 7.44-7.53 (3H, m), 7.58 (2H, d, J=8.7Hz), 7. 73-7. 75 (1H, m), 7.80 (1H, d, J=8,4Hz), 8.13 (1H, d, J=0.9Hz), 8.18 (1H, dd, J=0. 9, 7.2Hz)
(2) 1 N Sodium hydroxide aqueous solution (0.5 mL) and water (5 mL) were added at 60°C to a tetrahydrofuran-methanol mixed solution (5 mL-5 mL) of the compound (145 mg) obtained in (1), and stirred for 30 minutes at that temperature. The reaction solution was neutralized with 1 N hydrochloric acid, and the precipitated crystals were filtered out and washed successively with water, methanol and diethyl ether to obtain the title compound (116 mg) as pale yellow crystals.
   Melting point: 250-252°C
   ¹H-NMR (300MHz, DMSO-d₆); δ 3.08-3.20 (2H, m), 4.65-4.75 (1H, m), 5.07 (2H, s), 6.92 (2H, d, J=8.7Hz), 7.13-7.26 (4H, m), 7.35-7.45 (2H, m), 7.49-7.60 (3H, m), 7.86 (2H, d, J=8.7Hz), 7.93 (1H, s), 8.17 (1H, d, J=8.1Hz), 8.39 (1H, s), 8.65 (1H, d, J=7.2Hz), 12.98 (1H, br s) Elemental analysis: Calcd. for C₃₀H₂₃ClFN₃O₄, : C 66.24, H 4.26, N 7.72; Found: C 66.29, H 4.38, N 7.93

### Example E48

### N-{[7-(4-Chlorophenyl)imidazo[1,2-a]pyridin-2-yl]carbonyl)-O-(3-fluorobenzyl)tyrosine

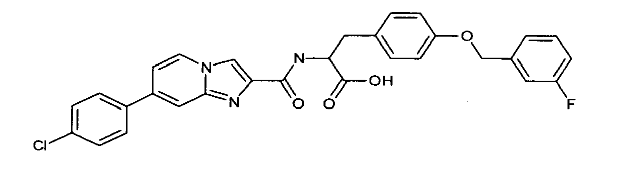

(1) A mixture of 7-(4-chlorophenyl)imidazo[1,2-a]pyridine-2-carboxylic acid (95 mg), methyl O-(4-fluorobenzyl)tyrosinate hydrochloride (119 mg), (3-dimethylaminopropyl)ethyl carbodiimide hydrochloride (81 mg), 1-hydroxybenzotriazole (57 mg), triethylamine (0.15 mL) and N,N-dimethylformamide (4 mL) was stirred overnight at room temperature. Water was added to the reaction solution, which was then extracted with ethyl acetate. The extract was washed with hydrochloric acid and saturated sodium chloride solution and dried over anhydrous magnesium sulfate. The solvent was evaporated under reduced pressure, and the residue was purified by silica gel column chromatography to obtain methyl N-{[7-(4-chlorophenyl)imidazo[1,2-a]pyridin-2-yl]carbonyl}-O-(3-fluorobenzyl)tyrosinate (164 mg) as pale yellow crystals.
   ¹H-NMR (300MHz, CDCl₃); δ 3.12-3.27 (2H, m), 3.73 (3H, s), 5.02 (2H, s), 5.03-5.10 (1H, m), 6.88 (2H, d, J=8.7Hz), 6.96-7.03 (1H, m), 7.07-7.20 (5H, m), 7.27-7.37 (1H, m), 7.47 (2H, d, J=8.7Hz), 7.58 (2H, d, J=8.7Hz), 7.72-7.75 (1H, m), 7.79 (1H, d, J=8.4Hz), 8.13 (1H, s), 8.18 (1H, d, J=7.2Hz)
(2) 1 N Sodium hydroxide aqueous solution (0.5 mL) and water (5 mL) were added at 60°C to a tetrahydrofuran-methanol mixed solution (5 mL-5 mL) of the compound (159 mg) obtained in (1), and stirred for 30 minutes at that temperature. The reaction solution was neutralized with 1 N hydrochloric acid, and the precipitated crystals were filtered out and washed successively with water, methanol and diethyl ether to obtain the title compound (127 mg) as pale yellow crystals.
   Melting point: 248-250°C
   ¹H-NMR (300MHz, DMSO-d₆); 5 3.08-3.20 (2H, m), 4.65-4.75 (1H, m), 5.01 (2H, s), 6.90 (2H, d, J=8.7Hz), 7.11-7.23 (4H, m), 7.35-7.50 (3H, m), 7.57 (2H, d, J=8.7Hz), 7.86 (2H, d, J=8.7Hz), 7.93 (1H, s), 8.16 (1H, d, J=8.1Hz), 8.39 (1H, s), 8.65 (1H, d, J=6.9Hz), 12.98 (1H, br s) Elemental analysis: Calcd. for C₃₀H₂₃CIFN₃O₄, : C 66.24, H 4.26, N 7.72; Found: C 66.16, H 4.28, N 7.78

### Example E49

### N-{[7-(4-Chlorophenyl)imidazo[1,2-a]pyridin-2-yl]carbonyl}-O-(4-fluorobenzyl)tyrosine

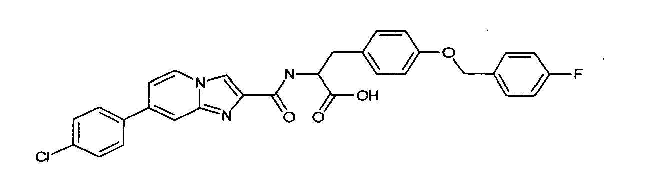

(1) A mixture of 7-(4-chlorophenyl)imidazo[1,2-a]pyridine-2-carboxylic acid (95 mg), methyl O-(4-fluorobenzyl)tyrosinate hydrochloride (119 mg), (3-dimethylaminopropyl)ethyl carbodiimide hydrochloride (81 mg), 1-hydroxybenzotriazole (57 mg), triethylamine (0.15 mL) and N,N-dimethylformamide (4 mL) was stirred overnight at room temperature. Water was added to the reaction solution, which was then extracted with ethyl acetate. The extract was washed with hydrochloric acid and saturated sodium chloride solution and dried over anhydrous magnesium sulfate. The solvent was evaporated under reduced pressure, and the residue was purified by silica gel column chromatography to obtain methyl N-{[7-(4-chlorophenyl)imidazo[1,2-a]pyridin-2-yl]carbonyl}-O-(4-fluorobenzyl)tyrosinate (160 mg) as pale yellow crystals.
   ¹H-NMR (300MHz, CDCl₃); δ 3.02-3.17 (2H, m), 3.73 (3H, s), 4.98 (2H, s), 5.03-5.10 (1H, m), 6.88 (2H, d, J=8.7Hz), 7.01-7.17 (5H, m), 7.35-7.42 (2H, m), 7.46 (2H, d, J=8.7Hz), 7.58 (2H, d, J=8.7Hz), 7.72-7.75 (1H, m), 7.80 (1H, d, J=8.4Hz), 8.13 (1H, d, J=0.6Hz), 8.18 (1H, dd, J=0.6, 7.2Hz)
(2) 1 N Sodium hydroxide aqueous solution (0.5 mL) and water (5 mL) were added at 60°C to a tetrahydrofuran-methanol mixed solution (5 mL-5 mL) of the compound (155 mg) obtained in (1), and stirred for 30 minutes at that temperature. The reaction solution was neutralized with 1 N hydrochloric acid, and the precipitated crystals were filtered out and washed successively with water, methanol and diethyl ether to obtain the title compound (126 mg) as pale yellow crystals.
   Melting point: 255-257°C
   H-NMR (300MHz, DMSO-d₆); δ 3.08-3.20 (2H, m), 4.65-4.75 (1H, m), 5.01 (2H, s), 6.90 (2H, d, J=8.7Hz), 7.11-7.23 (4H, m), 7.35-7.50 (3H, m), 7.57 (2H, d, J=8.7Hz), 7.86 (2H, d, J=8.7Hz), 7.93 (1H, s), 8.16 (1H, d, J=8.1Hz), 8.39 (1H, s), 8.65 (1H, d, J=6.9Hz), 12.98 (1H, br s). Elemental analysis: Calcd. for C₃₀H₂₃ClFN₃O₄, : C 66.24, H 4.26, N 7.72; Found: C 66.23, H 4.33, N 7.85

### Example E50

### O-Benzyl-N-{[3-chloro-2-(4-chlorophenyl)imidazo[1,2-a]pyridin-6-yl]carbonyl}tyrosine

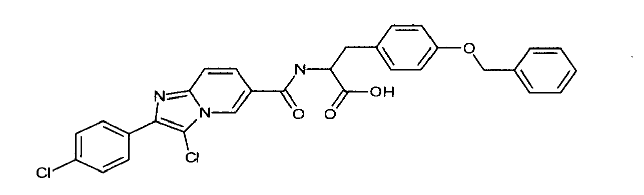

(1) A mixture of 3-chloro-2-(4-chlorophenyl)imidazo[1,2-a]pyridine-6-carboxylic acid (184 mg), methyl O-benzyltyrosinate hydrochloride (204 mg), (3-dimethylaminopropyl)ethyl carbodiimide hydrochloride (138 mg), 1-hydroxybenzotriazole (97 mg), triethylamine (0.25 mL) and N,N-dimethylformamide (6 mL) was stirred overnight at room temperature. Water was added to the reaction solution, which was then extracted with ethyl acetate. The extract was washed with hydrochloric acid and saturated sodium chloride solution and dried over anhydrous magnesium sulfate. The solvent was evaporated under reduced pressure, and the residue was purified by silica gel column chromatography to obtain methyl O-benzyl-N-{[3-chloro-2-(4-chlorophenyl)imidazo[1,2-a]pyridin-6-yl]carbonyl}tyrosinate (264 mg) as a colorless amorphous substance.
   ¹H-NMR (300MHz, CDCl₃); δ 3.16-3.31 (2H, m), 3.81 (3H, s), 5.04 (2H, s), 5.04-5.11 (1H, m), 6.61 (1H, d, J=7.5Hz), 6.92 (2H, d, J=8.7Hz), 7.06 (2H, d, J=8.7Hz), 7.28-7.50 (8H, m), 7.62 (1H, dd, J=0.6, 9.3Hz), 8.09 (2H, d, J=8.7Hz), 8.66-8.69 (1H, m)
(2) 1 N Sodium hydroxide aqueous solution (1 mL) and water (5 mL) were added at 60°C to a tetrahydrofuran-methanol mixed solution (5 mL-5 mL) of the compound (260 mg) obtained in (1), and stirred for 30 minutes at that temperature. The reaction solution was neutralized with 1 N hydrochloric acid, and the precipitated crystals were filtered out and washed successively with water, methanol and diethyl ether to obtain the title compound (126 mg) as colorless crystals. Melting point: 241-243°C
   ¹H-NMR (300MHz, DMSO-d₆); δ 3.01 (1H, dd, J=10.5, 13.8Hz), 3.17 (1H, dd, J=4.5, 13.8Hz), 4.58-4.68 (1H, m), 5.03 (2H, s), 6.92 (2H, d, J=8.4Hz), 7.22-7.42 (7H, m), 7.61 (2H, d, J=8.4Hz), 7.70-7.79 (2H, m), 8.14 (2H, d, J=8.7Hz), 8.84 (1H, s), 9.06 (1H, d, J=8.1Hz), 12.85 (1H, br s) Elemental analysis: Calcd. for C₃₀H₂₃Cl₂N₃O₄•0.25 H₂O, : C 63.78, H 4.19, N 7.44; Found: C 63.70, H 4.14, N 7.26

### Example E51

### O-Benzyl-N-{[7-(4-chlorophenyl)imidazo[1,2-a]pyridin-2-yl]carbonyl}-N-methyltyrosine

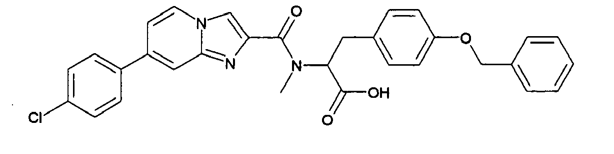

(1) A mixture of 7-(4-chlorophenyl)imidazo[1,2-a]pyridine-2-carboxylic acid (100 mg, 0.50 mmol), methyl O-benzyl-N-methyltyrosinate (82 mg, 0.25 mmol), (3-dimethylaminopropyl)ethyl carbodiimide hydrochloride (192 mg, 1.00 mmol), 1-hydroxybenzotriazole (135 mg, 1.00 mmol), triethylamine (300 µL, 2.1 mmol), N,N-dimethylformamide (2 mL) and dichloromethane (6 mL) was stirred overnight at room temperature. Water was added to the reaction solution, which was then extracted with ethyl acetate. The extract was washed with water and saturated sodium chloride solution and dried over anhydrous magnesium sulfate. The drying agent was removed by filtration, the solvent was evaporated under reduced pressure, and the residue was purified by basic silica gel column chromatography (eluate: ethyl acetate/hexane = 1/10-1/1) and recrystallized from a chloroform-hexane mixed solvent to obtain methyl O-benzyl-N-{[7-(4-chlorophenyl)lmidazo[1,2-a]pyridin-2-yl]carbonyl}-N-methyltyrosinate (80 mg, 58%) as a white solid.
   LC-MS 554 [M+H]⁺
   ¹H-NMR (300MHz, DMSO-d₆); δ 2.88 (s, 1.5H), 3.03-3.24 (m, 2H), 3.31 (s, 1.5H), 3.68-3.71 (d, J=9.0Hz, 3H), 4.98-5.04 (d, J=9.9Hz, 2H), 5.08-5.13 (m, 1H), 6.76-6.78 (d, J=8.4Hz, 1H), 6.90-6.93 (d, J=8.4Hz, 1H), 7.09-7.11 (d, J=8.4Hz, 1H), 7.18-7.21 (d, J=8.4Hz, 1H), 7.30-7.40 (m, 6H), 7.54-7.58 (dd, J=2.7, 5.7Hz, 2H), 7.86-7.98 (m, 3H), 8.13 (s, 0.5H), 8.31 (s, 0.5H), 8.57-8.63 (m, 1H)
(2) 1 M Sodium hydroxide aqueous solution (292 µL) was added to a tetrahydrofuran-methanol mixed solution (2 mL-2 mL) of methyl O-benzyl-N-{[7-(4-chlorophenyl)imidazo[1,2-a]pyridin-2-yl]carbonyl}-N-methyltyrosinate (80 mg, 0.15 mmol), and stirred for 30 minutes at 60°C. The reaction solution was neutralized with 1 N hydrochloric acid (300 µL), and the precipitated white solid was filtered out and washed successively with water and diethyl ether and then recrystallized from a tetrahydrofuran-diisopropyl ethyl ether mixed solvent to obtain the title compound (56 mg, 71%) as a white solid
   LC-MS 540 [M+H]⁺
   ¹H-NMR (300MHz, DMSO-d₆); δ 2.88 (s, 1.5H), 3.00-3.28 (m, 2H), 3.32 (s, 1.5H), 4.99-5.03 (d, J=13.5Hz, 2H), 5.11-5.16 (m, 1H), 6.76-6.79 (d, J=8.4Hz, 1H), 6.90-6.93 (d, J=8.4Hz, 1H), 7.08-7.11 (d, J=8.4Hz, 1H), 7.18-7.21 (d, J=8.7Hz, 1H), 7.25-7.39 (m, 6H), 7.54-7.58 (m, 2H), 7.86-8.00 (m, 3H), 8.12 (s, 0.5H), 8.29 (s, 0.5H), 8.58-8.63 (m, 1H)
   Melting point: 222°C
   Elemental analysis: Calcd. for C₃₁H₂₆N₃O₄Cl, : C 68.95, H 4.85, N 7.78; Found: C 68.68, H 4.86, N 7.70

### Example E52

### N-[4-(Benzyloxy)benzyl]-N-{[7-(4-chlorophenyl)imidazo[1,2-a]pyridin-2-yl]carbonyl}glycine

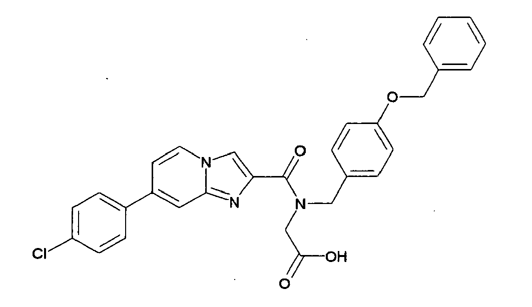

(1) A mixture of 7-(4-chlorophenyl)imidazo[1,2-b]pyridazine-2-carboxylic acid (136 mg, 0.50 mmol), methyl N-[4-(benzyloxy)benzyl]glycinate (143 mg, 0.50 mmol), (3-dimethylaminopropyl)ethyl carbodiimide hydrochloride (192 mg, 1.00 mmol), 1-hydroxybenzotriazole (135 mg, 1.00 mmol), triethylamine (300 µL, 2.1 mmol), N,N-dimethylformamide (2 mL) and dichloromethane (6 mL) was stirred overnight at room temperature. Ethyl acetate and water were added to separate the solution, and the organic layer was washed twice with water and once with saturated sodium chloride solution and dried over magnesium sulfate. The drying agent was removed by filtration, and the filtrate was concentrated under reduced pressure. The residue was purified by silica gel column chromatography (eluate: ethyl acetate/hexane = 1/10-1/1) and recrystallized from an ethyl acetate-hexane mixed solvent to obtain methyl N-[4-(benzyloxy)benzyl]-N-{[7-(4-chlorophenyl)imidazo[1,2-a]pyridin-2-yl]carbonyl}glycinate (217 mg, 80%) as a white solid.
   LC-MS 540 [M+H]⁺
   ¹H-NMR (300MHz, DMSO-d₆); δ 3.62-3.63 (d, J=4.8Hz, 3H), 4.01 (s, 1H), 4.65 (s, 1H), 4.90 (s, 1H), 5.09 (s, 2H), 5.41 (s, 1H), 6.97-7.00 (d, J=8.4Hz, 2H), 7.27-7.45 (m, 8H), 7.53-7.57 (m, 2H), 7.87-7.89 (d, J=6.6Hz, 3H), 8.46 (s, 1H), 8.64-8.68 (m, 1H)
(2) 1 M Sodium hydroxide aqueous solution (803 µL) was added to a tetrahydrofuran-methanol mixed solution (2 mL-2 mL) of methyl N-[4-(benzyloxy)benzyl]-N-{[7-(4-chlorophenyl)imidazo[1,2-a]pyridin-2-yl]carbonyl}glycinate (216 mg, 0.40 mmol), and stirred for 60 minutes at 60°C. The reaction solution was neutralized with 1 N hydrochloric acid (830 µL), and the precipitated white solid was filtered out and washed successively with water and diethyl ether and then recrystallized from a tetrahydrofuran-diisopropyl ethyl ether mixed solvent to obtain the title compound (167 mg, 80%) as a white solid.
   LC-MS 526 [M+H]⁺
   ¹H-NMR (300MHz, DMSO-d₆); δ 3.91 (s, 1H), 4.64 (s, 1H), 4.85 (s, 1H), 5.09 (s, 2H), 5.38 (s, 1H), 6.97-7.00 (d, J=8.4Hz, 2H), 7.27-7.46 (m, 8H), 7.53-7.57 (dd, J=2.7, 6.0Hz, 2H), 7.87-7.90 (d, J=8.7Hz, 3H), 8.45 (s, 1H), 8.65-8.69 (m, 1H), 12.60 (br s, 1H)
   Melting point: 186°C
   Elemental analysis: Calcd. for C₃₀H₂₄N₃O₄Cl (cont. 0.2 mmol H₂O), : C 68.04, H 4.64, N 7.93; Found: C 68.07, H 4.60, N 7.83

### Example E53

### N-{[7-(4-Chlorophenyl)imidazo[1,2-a]pyridin-2-yl]carbonyl}-N-[4-(3-thienylmethoxy)benzyl]glycine

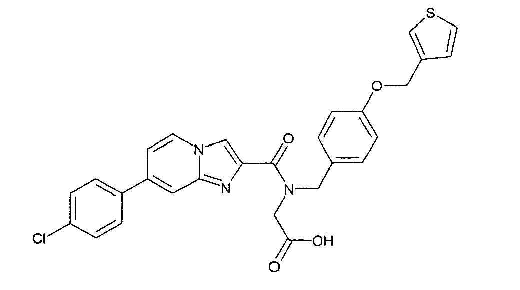

(1) Sodium methoxide (108 mg, 2 mmol) was added to a mixture of glycine methyl ester hydrochloride (252 mg, 2 mmol) and methanol (5 mL), and stirred for 30 minutes. 4-(3-thienylmethoxy)benzaldehyde (436 mg, 2 mmol) and acetic acid (a few drops) were added, and heated and refluxed for 2 hours. Sodium cyanoborohydride (251 mg, 4 mmol) was added and stirred overnight at room temperature. Water was added to the reaction solution, which was then stirred for 15 minutes and extracted twice with ethyl acetate. The organic layer was washed with water and saturated sodium chloride solution and dried by addition of magnesium sulfate. The reaction solution was filtered, and the filtrate was concentrated. The residue was dissolved in tetrahydrofuran (3 mL). Di-tert-butyl dicarbonate (437 mg, 2 mmol) and triethylamine (418 µL, 3 mmol) were added and stirred for 1 hour at room temperature. The reaction solution was concentrated under reduced pressure, and the residue was purified by basic silica gel column chromatography (eluate: ethyl acetate/hexane = 2/98-100/0) to obtain methyl N-(tert-butoxycarbonyl)-N-[4-(3-thienylmethoxy)benzyl]glycinate (150 mg, 19%) as a colorless oil. ¹H-NMR (300MHz, CDCl₃); δ 1.48 (s, 9H), 3.69 (s, 3H), 3.77 (s, 1H), 3.90 (s, 1H), 4.44-4.48 (d, J=10.2Hz, 2H), 5.06 (s, 2H), 6.91-6.93 (d, J=8.4Hz, 2H), 7.13-7.19 (m, 3H), 7.32-7.44 (m, 2H)
(2) 4 N Hydrogen chloride-ethyl acetate solution (2 mL) was added to an ethyl acetate (2 mL) solution of methyl N-(tert-butoxycarbonyl)-N-[4-(3-thienylmethoxy)benzyl]glycinate (150 mg, 0.38 mmol), and stirred for 2 hours at room temperature. The precipitated white solid was filtered out and washed successively with ethyl acetate and diethyl ether to obtain methyl N-[4-(3-thienylmethoxy)benzyl]glycinate hydrochloride (64 mg, 52%) as a white solid.
   ¹H-NMR (300MHz, DMSO-d₆); δ 3.73 (s, 3H), 3.92 (s, 2H), 4.08 (s, 2H), 5.12 (s, 2H), 7.05-7.08 (d, J=8.7Hz, 2H), 7.16-7.18 (m, 1H), 7.38-7.41 (d, J=9.0Hz, 2H), 7.55-7.57 (m, 2H), 9.24 (s, 3H)
(3) A mixture of 7-(4-chlorophenyl)imidazo[1,2-a]pyridine-2-carboxylic acid (64 mg, 0.23 mmol), methyl N-[4-(3-thienylmethoxy)benzyl]glycinate hydrdochloride (64 mg, 0.20 mmol), (3-dimethylaminopropyl)ethyl carbodiimide hydrochloride (96 mg, 0.50 mmol), 1-hydroxybenzotriazole (68 mg, 0.50 mmol), triethylamine (150 µL, 1.0 mmol), N,N-dimethylformamide (2 mL) and dichloromethane (4 mL) was stirred overnight at room temperature. Water was added to the reaction solution, which was then extracted with ethyl acetate. The extract was washed with water and saturated sodium chloride solution and dried over anhydrous magnesium sulfate. The drying agent was removed by filtration, the solvent was evaporated under reduced pressure, and the residue was purified by basic silica gel column chromatography (eluate: ethyl acetate/hexane = 1/10-1/1) and recrystallized from an ethyl acetate-hexane mixed solvent to obtain methyl N-{[7-(4-chlorophenyl)imidazo[1,2-a]pyridin-2-yl]carbonyl}-N-[4-(3-thienylmethoxy)benzyl]glycinate (90 mg, 85%) as a white solid.
   LC-MS 546 [M+H]⁺
   ¹H-NMR (300MHz, DMSO-d₆); δ 3.62-3.63 (d, J=4.5Hz, 3H), 4.01 (s, 1H), 4.65 (s, 1H), 4.90 (s, 1H), 5.07 (s, 2H), 5.41 (s, 1H), 6.96-6.99 (d, J=8.4Hz, 2H), 7.16-7.18 (d, J=4.8Hz, 1H), 7.27-7.33 (t, J=9.0Hz, 2H), 7.39-7.42 (t, J=5.1Hz, 1H), 7.53-7.56 (m, 4H), 7.88-7.90 (m, 3H), 8.47 (s, 1H), 8.64-8.69 (t, J=6.6Hz, 1H)
(4) 1 M Sodium hydroxide aqueous solution (327 µL) was added to a tetrahydrofuran-methanol mixed solution (2 mL-2 mL) of methyl N-{[7-(4-chlorophenyl)imidazo[1,2-a]pyridin-2-yl]carbonyl}-N-[4-(3-thienylmethoxy)benzyl]glycinate (90 mg, 0.17 mmol), and stirred for 30 minutes at 60°C. The reaction solution was neutralized with 1 N hydrochloric acid (350 µL), and the precipitated white solid was filtered out and washed successively with water and diethyl ether to obtain the title compound (73 mg, 83%) as a white solid.
   LC-MS 532 [M+H]⁺
   ¹H-NMR (300MHz, DMSO-d₆); δ 3.90 (s, 1H), 4.64 (s, 1H), 4.84 (s, 1H), 5.07 (s, 2H), 5.38 (s, 1H), 6.97-6.99 (d, J=8.4Hz, 2H), 7.16-7.18 (d, J=4.8Hz, 1H), 7.26-7.33 (m, 2H), 7.39-7.41 (d, J=7.2Hz, 1H), 7.54-7.56 (m, 4H), 7.87-7.90 (d, J=9.0Hz, 1H), 8.45-8.46 (d, J=2.1Hz, 1H), 8.65-8.69 (m, 1H), 12.60 (br s, 1H)
   Melting point: 187°C
   Elemental analysis: Calcd. for C₂₈H₂₂N₃O₄SCl (cont. 0.4 mol H₂O), : C 62.37, H 4.26, N 7.79; Found: C 62.36, H 4.30, N 7.76

### Example E54

### O-Benzyl-N-{[6-(benzylthio)imidazo[1,2-b]pyridazine-2-yl]carbonyl}tyrosine

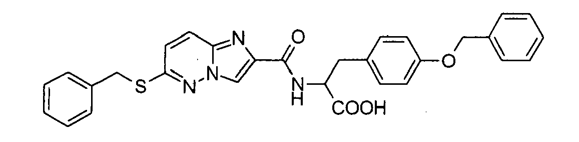

(1) 3-Amino-6-chloropyridazine (2.0 g, 15.4 mmol), ethanol (4 mL), tetrahydrofuran (20 mL), 4 N sodium hydroxide aqueous solution (5 mL) and benzylmercaptane (2.0 mL, 17.0 mmol) were heated for 10 minutes at 150°C by a microwave reactor. The reaction solution was diluted with ethyl acetate, washed with water, and dried over anhydrous magnesium sulfate. The solvent was evaporated to obtain 3-amino-6-(benzylthio)pyridazine (1.89 g, 56%) as a white powder.
   ¹H-NMR (300MHz, CDCl₃)δ: 4.49 (2H, s), 4.54 (2H, br s), 6.61 (1H, d, J=9.0Hz), 7.05 (1H, d, J=9.0Hz), 7.23-7.32 (3H, m), 7.40-7.43 (2H, m)
   LC-MS 218 [M+H]⁺
(2) 3-Amino-6-(benzylthio)pyridazine (1.00 g, 4.60 mmol), 1,2-dimethoxyethane (10 mL) and ethyl 3-bromopyruvate (0.94 g, 4.83 mmol) were stirred for 16 hours at room temperature. This was then heated for 10 minutes at 130°C by a microwave reactor. The reaction solution was diluted with ethyl acetate, washed with water, and dried over anhydrous magnesium sulfate, and the solvent was evaporated. The residue was purified by silica gel column chromatography (eluate: 10-40% ethyl acetate/hexane) to obtain ethyl 6-(benzylthio)imidazo[1,2-b]pyridazine-2-carboxylate (0.77 g, 53%) as a white powder.
   ¹H-NMR (300MHz, CDCl₃)δ: 1.44 (3H, t, J=7.2Hz), 4.43 (2H, s), 4.47 (2H, q, J=7.2Hz), 6.88 (1H, d, J=9.6Hz), 7.27-7.37 (3H, m), 7.43-7.46 (2H, m), 7.75 (1H, d, J=9.9Hz), 8.41 (1H, s)
   LC-MS 314 [M+H]⁺
(3) 6-(Benzylthio)imidazo[1,2-b]pyridazine-2-carboxylic acid was obtained by methods similar to those of Example C167-(6) from ethyl 6-(benzylthio)imidazo[1,2-b]pyridazine-2-carboxylate (0.25 g, 0.80 mmol), and the title compound (0.14 g, 33%) was then obtained as colorless crystals by methods similar to those of Example C139-(3) and Example C139-(4).
   ¹H-NMR (300MHz, DMSO-d₆)δ: 3.11-3.14 (2H, m), 4.46 (2H, s), 4.66-4.70 (1H, m), 5.02 (2H, s), 6.90 (2H, d, J=8.7Hz), 7.15 (2H, d, J=8.7Hz), 7.23-7.43 (9H, m), 7.50-7.53 (2H, m), 8.00 (1H, d, J=9.6Hz), 8.23 (1H, d, J=8.1Hz), 8.59 (1H, s), 12.95 (1H, br s)
   LC-MS 539 [M+H]⁺
   Melting point: 178-179°C
   Elemental analysis: Calcd. for C₃₀H₂₆N₄O₄S, : C 66.90, H 4.87, N 10.40; Found: C 66.85, H 4.91, N 10.33

### Example E55

### O-Benzyl-N-{[2-(4-chlorophenyl)-3-methylimidazo[1,2-a]pyridin-6-yl]carbonyl}tyrosine

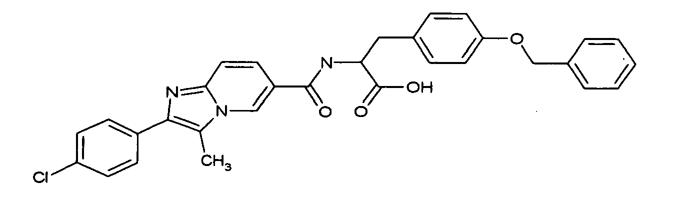

(1) A mixture of 2-(4-chlorophenyl)-3-methylimidazo[1,2-a]pyridine-6-carboxylic acid (143 mg), methyl O-benzyltyrosinate hydrochloride (161 mg), (3-dimethylaminopropyl)ethyl carbodiimide hydrochloride (115 mg), 1-hydroxybenzotriazole (81 mg), triethylamine (0.21 mL) and N,N-dimethylformamide (5 mL) was stirred overnight at room temperature. Water was added to the reaction solution, which was then extracted with ethyl acetate. The extract was washed with hydrochloric acid and saturated sodium chloride solution and dried over anhydrous magnesium sulfate. The solvent was evaporated under reduced pressure, and the residue was purified by silica gel column chromatography to obtain methyl O-benzyl-N-{ [2-(4-chlorophenyl)-3-methylimidazo[1,2-a]pyridin-6-yl]carbonyl}tyrosinate (230 mg) as a colorless amorphous substance.
   ¹H-NMR (300MHz, CDCl₃); δ 2.67 (3H, s), 3.16-3.29 (2H, m), 3.81 (3H, s), 5.04 (2H, s), 5.04-5.11 (1H, m), 6.60 (1H, d, J=7.5Hz), 6.92 (2H, d, J=8.7Hz), 7.06 (2H, d, J=8.7Hz), 7.28-7.48 (8H, m), 7.61 (1H, dd, J=0.6, 9.3Hz), 7.75 (2H, d, J=8.7Hz), 8.58 (1H, s)
(2) 1 N Sodium hydroxide aqueous solution (5 mL) was added to a tetrahydrofuran-methanol mixed solution (5 mL-5 mL) of the compound (230 mg) obtained in (1), and stirred for 1 hour at 60°C. The reaction solution was neutralized with 1 N hydrochloric acid, and the precipitated crystals were filtered out and washed successively with water, methanol and diethyl ether to obtain the title compound (152 mg) as colorless crystals.
   Melting point: 244-245°C
   ¹H-NMR (300MHz, DMSO-d₆);δ 2.70 (3H, s), 3.01 (1H, dd, J=10.2, 13.8Hz), 3.16 (1H, dd, J=4.8, 13.8Hz), 4.59-4.68 (1H, m), 5.03 (2H, s), 6.92 (2H, d, J=8.7Hz), 7.22-7.43 (7H, m), 7.56 (2H, d, J=8.7Hz), 7.59-7.67 (2H, m), 7.87 (2H, d, J=8.7Hz), 8.79 (1H, s), 8.85 (1H, d, J=8.1Hz), 12.84 (1H, br s)
   Elemental analysis: Calcd. for C₃₁H₂₆ClN₃O₄•0.75 H₂O, : C 67.27, H 5.01, N 7.59; Found: C 67.37, H 4.89, N 7.63

### Example E56

### O-Benzyl-N-{[7-(4-chlorophenyi)imidazo[1,2-a]pyridin-2-yl]carbonyl}-L-tyrosine

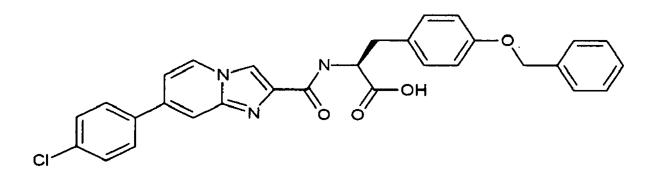

(1) A mixture of 7-(4-chlorophenyl)imidazo[1,2-a]pyridine-2-carboxylic acid (136 mg), methyl O-benzyl-L-tyrosinate hydrochloride (161 mg; synthesized from L-tyrosine by methods similar to those of Reference Example 19), (3-dimethylaminopropyl)ethyl carbodiimide hydrochloride (115 mg), 1-hydroxybenzotriazole (81 mg), triethylamine (0.21 mL) and N,N-dimethylformamide (5 mL) was stirred overnight at room temperature. Water was added to the reaction solution, which was then extracted with ethyl acetate. The extract was washed with hydrochloric acid and saturated sodium chloride solution and dried over anhydrous magnesium sulfate. The solvent was evaporated under reduced pressure, and the residue was purified by silica gel column chromatography to obtain methyl O-benzyl-N-{[7-(4-chlorophenyl)imidazo[1,2-a]pyridin-2-yl]carbonyl}-L-tyrosinate (244 mg) as pale yellow crystals.
   ¹H-NMR spectrum data is identical to that of the compound of Example E1(1).
(2) 1 N Sodium hydroxide aqueous solution (0.5 mL) and water (10 mL) were added to a tetrahydrofuran-methanol mixed solution (5 mL-5 mL) of the compound (153 mg) obtained in (1), and stirred for 2 hours at room temperature. The reaction solution was neutralized with 1 N hydrochloric acid, and the precipitated crystals were filtered out and washed successively with water, methanol and diethyl ether to obtain the title compound (130 mg) as pale yellow crystals. Melting point; 248-249°C
   ¹H-NMR spectrum data matches that of the compound of Example E1 (2). Elemental analysis: Calcd. for C₃₀H₂₄ClN₃O₄, : C 68.50, H 4.60, N 7.99; Found: C 68.41, H 4.83, N 8.07

### Example E57

### O-Benzyl-N-{[6-(2-phenethyl)imidazo[1,2-a]pyridin-2-yl]carbonyl}tyrosine

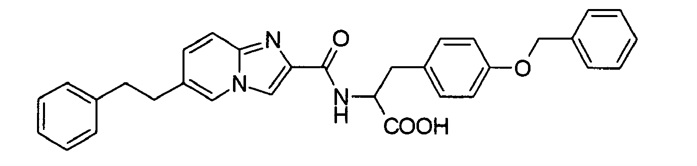

(1) A mixture of the ethyl 6-(2-phenylethenyl)imidazo[1,2-a]pyridine-2-carboxylate obtained in Example E34(1) (0.56 g, 1.92 mmol), tetrahydrofuran (5 mL), ethanol (10 mL) and Pd/C (0.1 g) was stirred for 18 hours at room temperature in a hydrogen atmosphere. The reaction solution was filtered, and the filtrate was concentrated under reduced pressure and separated with ethyl acetate and water. The organic layer was dried over anhydrous sodium sulfate, the solvent was evaporated, and the residue was purified by silica gel column chromatography (eluate: 10-40% ethyl acetate/hexane) to obtain ethyl 6-(2-phenethyl)imidazo[1,2-a]pyridine-2-carboxylate (0.14 g, 25%) as a white powder.
   ¹H-NMR (300MHz, CDCl₃)δ: 1.43 (3H, t, J=7.2Hz), 2.89-2.98 (4H, m), 4.45 (2H, q, J=7.2Hz), 7.09-7.14 (3H, m), 7.18-7.30 (3H, m), 7.60 (1H, d, J=9.3Hz), 7.77 (1H, s), 8.06(1H, s)
   LC-MS 295 [M+H]⁺
(2) 6-(2-Phenethyl)imidazo[1,2-a]pyridine-2-carboxylic acid was obtained by methods similar to those of Example C167(6) from 6-(2-phenethyl)imidazo[1,2-a]pyridine-2-carboxylate (0.11 g, 0.34 mmol), and the title compound (0.11 g, 61%) was then obtained as colorless crystals by methods similar to those of Example C139(3) and Example C139(4).
   ¹H-NMR (300MHz, DMSO-d₆)δ: 2.49-2.51 (4H, m), 2.86-2.95 (2H, m), 4.43-4.70 (1H, m), 5.03 (2H, s), 6.90 (2H, d, J=8.7Hz), 7.13-7.43 (13H, m), 7.53 (1H, d, J=9.3Hz), 8.13 (1H, d, J=8.4Hz), 8.25 (1H, s), 8.36 (1H, s), 12.91 (1H, br s)
   LC-MS 520 [M+H]⁺
   Melting point: 239°C (decomposes)
   Elemental analysis: Calcd. for C₃₂H₂₉N₃O₄, : C 73.97, H 5.63, N 8.09; Found: C 73.88, H 5.72, N 7.96

### Example E58

### O-Benzyl-N-{[7-(phenylethynyl)imidazo[1,2-a]pyridin-2-yl]carbonyl}tyrosine

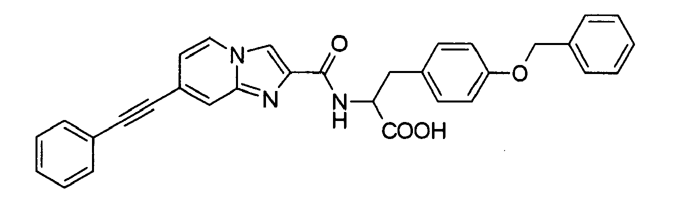

(1) A mixture of 2-chloro-4-iodopyridine (3.0 g, 12.5 mmol), acetonitrile (30 mL), ethynylbenzene (1.50 mL, 13.8 mmol), 1,4-diazobicyclo[2.2.2]octane (2.82 g, 25.1 mmol) di-µ-chlorobis[(η-aryl)palladium (II) (0.11 g, 0.30 mmol) and tri(n-butyl)phosphine (0.25 g, 126 mmol) was stirred for 20 minutes at room temperature. The reaction solution was filtered, and the filtrate was concentrated under reduced pressure and separated with ethyl acetate and water. The organic layer was dried over anhydrous sodium sulfate, the solvent was evaporated, and the residue was purified by silica gel column chromatography (eluate: 10-30% ethyl acetate/hexane) to obtain 2-chloro-4-(phenylethynyl)pyridine (2.41 g, 90%) as a pale brown powder.
   ¹H-NMR (300MHz, CDCl₃)δ: 7.30 (1H, dd, J=1.5, 5.1Hz), 7.37-7.44 (4H, m), 7.53-7.68 (2H, m), 8.37 (1H, d, J=5.4Hz)
   LC-MS 214 [M+H]⁺
(2) 2-Amino-4-(phenylethynyl)pyridine (0.62 g, 51%) was obtained as a white powder by methods similar to those of Reference Example 33 from 2-chloro-4-(phenylethynyl)pyridine (1.34 g, 6.27 mmol).
   ¹H-NMR (300MHz, CDCl₃)δ: 4.44 (2H, br s), 6.62 (1H, s), 6.76 (1H, dd, J=1.5, 5.4Hz), 7.35-7.38 (3H, m), 7.52-7.55 (2H, m), 8.06 (1H, d, J=5.1Hz)
   LC-MS 195 [M+H]⁺
(3) Ethyl 7-(phenylethynyl)imidazo[1,2-a]pyridine-2-carboxylate (0.20 g, 91%) was obtained as a white powder by methods similar to those of Example E54-(2) from 2-amino-4-(phenylethynyl)pyridine (0.1 g, 9.77 mmol).
   ¹H-NMR (300MHz, CDCl₃)δ: 1.34 (3H, t, J=7.2Hz), 4.36 (2H, q, J=7.2Hz),7.24(1H, dd, J=1.5, 7.2Hz), 7.46-7.51 1 (3H, m), 7.62-7.66 (2H, m), 7. 91 (1H, s), 8.66 (1H, d, J=7.2Hz), 8.71 (1H, s) LC-MS 291 [M+H]⁺
(4) 7-(Phenylethynyl)imidazo[1,2-a]pyridine-2-carboxylic acid was obtained by methods similar to those of Example C167-(6) from ethyl 7-(phenylethynyl)imidazo[1,2-a]pyridine-2-carboxylate (0.18 g, 0.62 mmol), and the title compound (0.11 g, 34%) was then obtained as colorless crystals by methods similar to those of Example C139-(3) and Example C139-(4).
   ¹H-NMR (300MHz, DMSO-d₆)δ: 3.14 (2H, d, J=6.6Hz), 4.65-4.73 (1H, m), 5.03 (2H, s), 6.90 (2H, d, J=8.7Hz), 7.09 (1H, dd, J=1.5, 6.9Hz), 7.16 (2H, d, J=8.7Hz), 7.28-7.49 (8H, m), 7.59-7.64 (2H, m), 7.84 (1H, s), 8.25 (1H, d, J=8.1Hz), 8.25 (1H, d, J=8.1Hz), 8.42 (1H, s), 8.59 (1H, d, J=7.2Hz), 12.94 (1H, br s)
   LC-MS 516 [M+H]⁺
   Melting point: 243°C (decomposes)
   Elemental analysis: Calcd. for C₃₂H₂₅N₃O₄, : C 74.55, H 4.89, N 8.15; Found: C 74.51, H 4.92, N 8.01

### Example E59

### N-{[7-(4-Chlorophenyl)imidazo[1,2-a]pyridin-2-yl]carbonyl}-O-(4-methylbenzyl)tyrosine

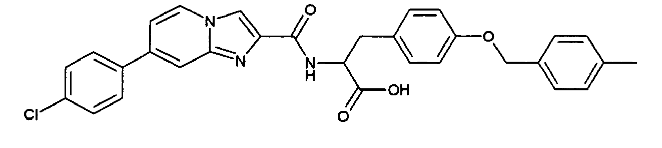

(1) A mixture of 7-(4-chlorophenyl)imidazo[1,2-a]pyridine-2-carboxylic acid (136 mg, 0.50 mmol), methyl O-(4-methylbenzyl)tyrosinate hydrochloride (151 mg, 0.45 mmol), (3-dimethylaminopropyl)ethyl carbodiimide hydrochloride (192 mg, 1.00 mmol), 1-hydroxybenzotriazole (135 mg, 1.00 mmol), triethylamine (300 µL, 2.1 mmol), N,N-dimethylformamide (2 mL) and dichloromethane (4 mL) was stirred overnight at room temperature. Water was added to the reaction solution, which was then extracted with chloroform. The extract was washed with water and saturated sodium chloride solution and dried over anhydrous magnesium sulfate. The drying agent was removed by filtration, the solvent was evaporated under reduced pressure, and the residue was purified by basic silica gel column chromatography (eluate: ethyl acetate/hexane = 1/10-1/1) and recrystallized from an ethyl acetate-hexane mixed solvent to obtain methyl N-{[7-(4-chlorophenyl)imidazo[1,2-a]pyridin-2-yl]carbonyl}-O-(4-methylbenzyl)tyrosinate (199 mg, 80%) as a white solid.
   LC-MS 554 [M+H]⁺
   ¹H-NMR (300MHz, DMSO-d₆); δ 2.28 (s, 3H), 3.13-3.15 (d, J=6.9Hz, 2H), 3.66 (s, 3H), 4.72-4.79 (m, 1H), 4.98 (s, 2H), 6.88-6.90 (d, J=8.7Hz, 2H), 7.13-7.17 (m, 4H), 7.28-7.31 (d, J=8.1Hz, 2H), 7.13-7.17 (m, 4H), 7.28-7.31 (d, J=8.1Hz, 2H), 7.37-7.40 (dd, J=1.8, 5.4Hz, 1H), 7.56-7.59 (d, J=8.4Hz, 2H), 7.85-7.91 (m, 3H), 8.36-8.38 (m, 2H), 8.64-8.66 (d, 7.2Hz, 1H)
(2) 1 M Sodium hydroxide aqueous solution (719 µL) was added to a tetrahydrofuran-methanol mixed solution (4 mL-2 mL) of methyl N-{[7-(4-chlorophenyl)imidazo[1,2-a]pyridin-2-yl]carbonyl}-O-(4-methylbenzyl)tyrosinate (199 mg, 0.36 mmol), and stirred for 30 minutes at 60°C. The reaction solution was neutralized with 1 N hydrochloric acid (800 µL), and the precipitated white solid was filtered out and washed successively with water and diethyl ether and then recrystallized from a dimethylformamide-water mixed solvent to obtain the title compound (167 mg, 86%) as a white solid.
   LC-MS 540 [M+H]⁺
   ¹H-NMR (300MHz, DMSO-d₆); δ 2.28 (s, 3H), 3.12-3.15 (m, 2H), 4.65-4.72 (m, 1H), 4.97 (s, 2H), 6.86-6.89 (d, J=8.7Hz, 2H), 7.12-7.17 (m, 4H), 7.28-7.31 (d, J=8.1Hz, 2H), 7.37-7.40 (dd, J=1.8, 5.7Hz, 1H), 7.56-7.59 (d, J=8.4Hz, 2H), 7.85-7.88 (d, J=8.4Hz, 2H), 7.93 (s, 1H), 8.14-8.16 (d, J=8.1Hz, 1H), 8.64-8.66 (d, J=7.2Hz, 1H), 12.80 (br s, 1H)
   Melting point: 262°C
   Elemental analysis: Calcd. for C₃₁H₂₆N₃O₄Cl, : C 68.95, H 4.85, N 7.78; Found: C 68.92, H 4.77, N 7.87

### Example E60

### N-{[7-(4-Chlorophenyl)imidazo[1,2-a]pyridin-2-yl]carbonyl}-O-(3-methylbenzyl)tyrosine

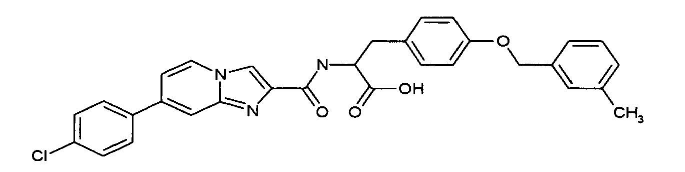

(1) A mixture of 7-(4-chlorophenyl)imidazo[1,2-a]pyridine-2-carboxylic acid (95 mg), methyl O-(3-methylbenzyl)tyrosinate hydrochloride (118 mg), (3-dimethylaminopropyl)ethyl carbodiimide hydrochloride (81 mg), 1-hydroxybenzotriazole (57 mg), triethylamine (0.15 mL) and N,N-dimethylformamide (4 mL) was stirred overnight at room temperature. Water was added to the reaction solution, which was then extracted with ethyl acetate. The extract was washed with hydrochloric acid and saturated sodium chloride solution and dried over anhydrous magnesium sulfate. The solvent was evaporated under reduced pressure, and the residue was purified by silica gel column chromatography to obtain methyl N-{[7-(4-chlorophenyl)imidazo[1,2-a]pyridin-2-yl]carbonyl}-O-(3-methylbenzyl)tyrosinate (139 mg) as pale yellow crystals.
   ¹H-NMR (300MHz, CDCl₃); δ 2.36 (3H, s), 3.12-3.26 (2H, m), 3.73 (3H, s), 4.98 (2H, s), 5.00-5:10 (1H, m), 6.90 (2H, d, J=8.4Hz), 7.07-7.29 (7H, m), 7.47 (2H, d, J=8.4Hz), 7.58 (2H, d, J=8.4Hz), 7.73-7.75 (1H, m), 7.80 (1H, d, J=8.4Hz), 8.13 (1H, d, J=0.9Hz), 8.18 (1H, dd, J=0.9, 7.2Hz)
(2) 1 N Sodium hydroxide aqueous solution (0.5 mL) and water (5 mL) were added at 60°C to a tetrahydrofuran-methanol mixed solution (5 mL-5 mL) of the compound (134 mg) obtained in (1), and stirred for 1 hour at that temperature. The reaction solution was neutralized with 1 N hydrochloric acid, and the precipitated crystals were filtered out and washed successively with water, methanol and diethyl ether to obtain the title compound (96 mg) as pale yellow crystals. Melting point: 245-246°C
   ¹H-NMR (300MHz, DMSO-d₆); δ 2.29 (3H, s), 3.08-3.18 (2H, m), 4.65-4.75 (1H, m), 4.98 (2H, s), 6.90 (2H, d, J=8.7Hz), 7.08-7.28 (6H, m), 7.39 (1H, dd, J=1.8, 7.5Hz), 7.57 (2H, d, J=8.7Hz), 7.87 (2H, d, J=8.7Hz), 7. 91-7.94 (1H, m), 8.16 (1H, d, J=8.1Hz), 8.39 (1H, s), 8.65 (1H, d, J=7.5Hz), 12.98 (1H, br s)
   Elemental analysis: Calcd. for C₃₁H₂₆CIN₃O₄, : C 68.95, H 4.85, N 7.78; Found: C 68.83, H 4.83, N 7.73

### Example E61

### N-{[7-(4-Chlorophenyl)imidazo[1,2-a]pyridin-2-yl]carbonyl}-O-(3-methoxybenzyl)tyrosine

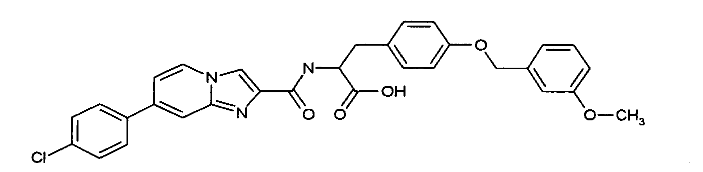

(1) A mixture of 7-(4-chlorophenyl)imidazo[1,2-a]pyridine-2-carboxylic acid (95 mg), methyl O-(3-methylbenzyl)tyrosinate hydrochloride (123 mg), (3-dimethoxyaminopropyl)ethyl carbodiimide hydrochloride (81 mg), 1-hydroxybenzotriazole (57 mg), triethylamine (0.15 mL) and N,N-dimethylformamide (4 mL) was stirred overnight at room temperature. Water was added to the reaction solution, which was then extracted with ethyl acetate. The extract was washed with hydrochloric acid and saturated sodium chloride solution and dried over anhydrous magnesium sulfate. The solvent was evaporated under reduced pressure, and the residue was purified by silica gel column chromatography to obtain methyl N-{[7-(4-chlorophenyl)imidazo[1,2-a]pyridin-2-yl]carbonyl}-O-(3-methoxybenzyl)tyrosinate (142 mg) as pale yellow crystals.
   ¹H-NMR (300MHz, CDCl₃); δ 3.13-3.27 (2H, m), 3.73 (3H, s), 3.80 (3H, s), 5.00 (2H, s), 5.00-5.10 (1H, m), 6.82-6.92 (3H, m), 6.95-7.01 (2H, m), 7.08 (1H, dd, J=1.8, 7.2Hz), 7.13 (2H, d, J=8.7Hz), 7.25-7.31 (1H, m), 7.47 (2H, d, J=8.7Hz), 7.57 (2H, d, J=8.7Hz), 7.72-7.75 (1H, m), 7.80 (1H, d, J=8.4Hz), 8.13 (1H, d, J=0.6Hz), 8.18 (1H, dd, J=0.6, 7.2Hz)
(2) 1 N Sodium hydroxide aqueous solution (0.5 mL) and water (5 mL) were added at 60°C to a tetrahydrofuran-methanol mixed solution (5 mL-5 mL) of the compound (137 mg) obtained in (1), and stirred for 1 hour at that temperature. The reaction solution was neutralized with 1 N hydrochloric acid, and the precipitated crystals were filtered out and washed successively with water, methanol and diethyl ether to obtain the title compound (101 mg) as pale yellow crystals. Melting point: 239-240°C
   ¹H-NMR (300MHz, DMSO-d₆); δ 3.05-3.20 (2H, m), 3.73 (3H, s), 4.63-4.73 (1H, m), 5.00 (2H, s), 6.83-6.93 (5H, m), 7.15 (2H, d, J=8.7Hz), 7.27 (1H, t, J=8.1Hz), 7.38 (1H, dd, J=1.8, 7.2), 7.57 (2H, d, J=8.7Hz), 7.86 (2H, d, J=8.7Hz), 7.93 (1H, s), 8.16 (1H, d, J=8.1Hz), 8.39 (1H, s), 8.65 (1H, d, J=7.2Hz), 12.98 (1H, br s)
   Elemental analysis: Calcd. for C₃₁H₂₆ClN₃Oₛ, : C 66.97, H 4.71, N 7.56; Found: C 66.89, H 4.70, N 7.50

### Example E62

### O-(4-Fluorobenzyl)-N-{[7-(phenylethynyl)imidazo[1,2-a]pyridin-2-yl]carbonyl }tyrosine

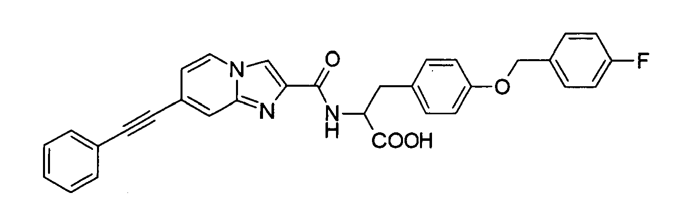

7-(Phenylethynyl)imidazo[1,2-a]pyridine-2-carboxylic acid was obtained by methods similar to those of Example C167-(6) from the ethyl 7-(phenylethynyl)imidazo[1,2-a]pyridine-2-carboxylate (0.19 g, 0.65 mmol) obtained in Example E58-(3), and the title compound (0.15 g, 43%) was then obtained as colorless crystals by methods similar to those of Example C139-(3) and Example C139-(4).
¹H-NMR (300MHz, DMSO-d₆)δ: 3.13-3.18 (2H, m), 4.64-4.71 (1H, m), 5.01 (2H, s), 6.89 (2H, d, J=8.7Hz), 7.08-7.23 (5H, m), 7.44-7.49 (5H, m), 7.59-7.63 (2H, m), 7.59-7.63 (2H, m), 7.84 (1H, s), 8.25 (1H, d, J=8.1Hz), 8.42 (1H, s), 8.59 (1H, d, J=6.9Hz), 12.94 (1H, br s)
LC-MS 534 [M+H]⁺
Melting point: 243°C (decomposes)
Elemental analysis: Calcd. for C₃₂H₂₄N₃O₄F, : C 72.04, H 4.53, N 7.88; Found: C 71.96, H 4.59, N 7.75

### Example E63

### O-(4-Chlorobenzyl)-N-{[7-(4-chlorophenyl)imidazo[1,2-a]pyridin-2-yl]carbonyl}tyrosine

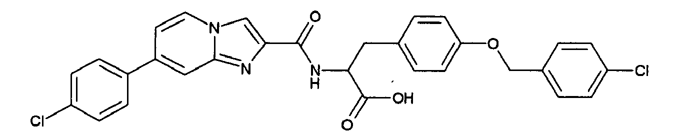

(1) A mixture of 7-(4-chlorophenyl)imidazo[1,2-a]pyridine-2-carboxylic acid (136 mg, 0.50 mmol), methyl O-(4-chlorobenzyl)tyrosinate hydrochloride (160 mg, 0.45 mmol), (3-dimethylaminopropyl)ethyl carbodiimide hydrochloride (192 mg, 1.00 mmol), 1-hydroxybenzotriazole (135 mg, 1.00 mmol), triethylamine (300 µL, 2.1 mmol), N,N-dimethylformamide (2 mL) and dichloromethane (4 mL) was stirred overnight at room temperature. Water was added to the reaction solution, which was then extracted with chloroform. The extract was washed with water and saturated sodium chloride solution and dried over anhydrous magnesium sulfate. The drying agent was removed by filtration, the solvent was evaporated under reduced pressure, and the residue was recrystallized from a dichloromethane-diisopropyl ether mixed solvent to obtain methyl O-(4-chlorobenzyl)-N-{[7-(4-chlorophenyl)imidazo[1,2-a]pyridin-2-yl]carbonyl}tyrosinate (229 mg, 89%) as a white solid.
   LC-MS 574 [M+H]⁺
   ¹H-NMR (300MHz, DMSO-d₆); δ 3.13-3.16 (d, J=6.6Hz, 2H), 3.66 (s, 3H), 4.72-4.79 (m, 1H), 5.04 (s, 2H), 6.89-6.92 (d, J=8.4Hz, 2H), 7.15-7.18 (d, J=8.4Hz, 2H), 7.37-7.46 (m, 5H), 7.56-7.59 (d, J=8.7Hz, 2H), 7.85-7.91 (m, 3H), 8.37-8.40 (m, 2H), 8.64-8.66 (d, J=7.2Hz, 1H)
(2) 1 M Sodium hydroxide aqueous solution (800 µL) was added to a tetrahydrofuran-methanol mixed solution (4 mL-2 mL) of methyl O-(4-chlorobenzyl)-N-{[7-(4-chlorophenyl)imidazo[1,2-a]pyridin-2-yl]carbonyl}tyrosinate (229 mg, 0.40 mmol), and stirred for 60 minutes at 60°C. The reaction solution was neutralized with 1 N hydrochloric acid (900 µL), and the precipitated white solid was filtered out and washed successively with water and diethyl ether and then recrystallized from a dimethylformamide-water mixed solvent to obtain the title compound (149 mg, 67%) as a white solid.
   LC-MS 560 [M+H]⁺
   ¹H-NMR (300MHz, DMSO-d₆); δ 3.08-3.21 (m, 2H), 4.69-4.74 (m, 1H), 5.03 (s, 2H), 6.88-6.91 (d, J=8.7Hz, 2H), 7.14-7.17 (d, J=8.4Hz, 2H), 7.37-7.46 (m, 5H), 7.56-7.59 (d, J=8.7Hz, 2H), 7.85-7.88 (d, J=8.7Hz, 2H), 7.93 (s, 1H), 8.15-8.17 (d, J=8.1Hz, 1H), 8.39 (s, 1H), 8.64-8.66 (d, J=7.2Hz, 1H), 12.80 (br s, 1H)
   Melting point: 261°C
   Elemental analysis: Calcd. for C₃₀H₂₃N₃O₄Cl₂ (cont. 0.1 mol H₂O), : C 64.09, H 4.16, N 7.47; Found: C 63.86, H 4.08, N 7.45

### Example E64

### N-{[7-(4-Chlorophenyl)imidazo[1,2-a]pyridin-2-yl]carbonyl}-O-(4-cyanobenzyl)tyrosine

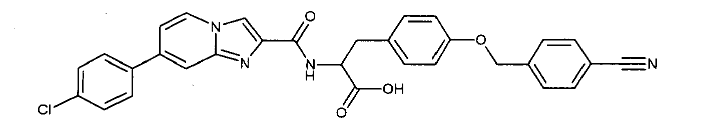

(1) A mixture of 7-(4-chlorophenyl)imidazo[1,2-a]pyridine-2-carboxylic acid (136 mg, 0.50 mmol), methyl O-(4-methylbenzyl)tyrosinate hydrochloride (156 mg, 0.45 mmol), (3-dimethylaminopropyl)ethyl carbodiimide hydrochloride (192 mg, 1.00 mmol), 1-hydroxybenzotriazole (135 mg, 1.00 mmol), triethylamine (300 µL, 2.1 mmol), N,N-dimethylformamide (2 mL) and dichloromethane (4 mL) was stirred overnight at room temperature. Water was added to the reaction solution, which was then extracted with chloroform. The extract was washed with water and saturated sodium chloride solution and dried over anhydrous magnesium sulfate. The drying agent was removed by filtration, the solvent was evaporated under reduced pressure, and the residue was recrystallized from a dichloromethane-diisopropyl ether mixed solvent to obtain methyl N-{[7-(4-chlorophenyl)imidazo[1,2-a]pyridine-2-yl]carbonyl}-O-(4-cyanobenzyl)tyrosinate (234 mg, 92%) as a white solid.
   LC-MS 565 [M+H]⁺
   ¹H-NMR (300MHz, DMSO-d₆); δ 3.14-3.16 (d, J=6.9Hz, 2H), 3.66 (s, 3H), 4.72-4.80 (m, 1H), 5.16 (s, 2H), 6.90-6.93 (d, J=8.7Hz, 2H), 7.16-7.19 (d, J=8.7Hz, 2H), 7.37-7.40 (dd, J=1.8, 5.4Hz, 1H), 7.56-7.62 (m, 4H), 7.82-7.91 (m, 5H), 8.38-8.41 (m, 2H), 8.63-8.66 (d, J=7.2Hz, 1 H)
(2) 1 M Sodium hydroxide aqueous solution (830 µL) was added to a tetrahydrofuran-methanol mixed solution (4 mL-2 mL) of methyl N-{[7-(4-chlorophenyl)imidazo[1,2-a]pyridin-2-yl]carbonyl}-O-(4-cyanobenzyl)tyrosinate (234 mg, 0.41 mmol), and stirred for 60 minutes at 60°C. The reaction solution was neutralized with 1 N hydrochloric acid (900 µL), and the precipitated white solid was filtered out and washed successively with water and diethyl ether and then recrystallized from an N,N-dimethylformamide-water mixed solvent to obtain the title compound (184 mg, 81%) as a white solid.
   LC-MS 551 [M+H]⁺
   ¹H-NMR (300MHz, DMSO-d₆); δ 3.08-3.21 (m, 2H), 4.66-4.73 (m, 1H), 5.15 (s, 2H), 6.90-6.93 (d, J=8.7Hz, 2H), 7.15-7.18 (d, J=8.4Hz, 2H), 7.37-7.40 (dd, J=1.8, 5.4Hz, 1H), 7.56-7.62 (m, 4H), 7.82-7.88 (m, 4H), 7.93 (s, 1H), 8.16-8.18 (d, J=8.1Hz, 1H), 8.38 (s, 1H), 8.64-8.66 (d, J=7.2Hz, 1H), 12.80 (br s, 1H)
   Melting point: 263°C
   Elemental analysis: Calcd. for C₃₁H₂₃N₄O₄Cl (cont. 0.2 mol H₂O), : C 67.14, H 4.25, N 10.10; Found: C 67.01, H 4.16, N 9.92

### Example E65

### N-{[7-(4-Chlorophenyl)imidazo[1,2-a]pyridin-2-yl]carbonyl}-O-(4-ethylbenzyl) tyrosine

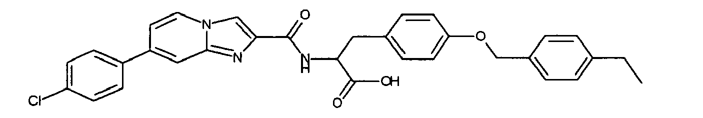

(1) A mixture of 7-(4-chlorophenyl)imidazo[1,2-a]pyridine-2-carboxylic acid (136 mg, 0.50 mmol), methyl O-(4-ethylbenzyl)tyrosinate hydrochloride (153 mg, 0.45 mmol), (3-dimethylaminopropyl)ethyl carbodiimide hydrochloride (192 mg, 1.00 mmol), 1-hydroxybenzotriazole (135 mg, 1.00 mmol), triethylamine (300 µL, 2.1 mmol), N,N-dimethylformamide (2 mL) and dichloromethane (4 mL) was stirred overnight at room temperature. Water was added to the reaction solution, which was then extracted with chloroform. The extract was washed with water and saturated sodium chloride solution and dried over anhydrous magnesium sulfate. The drying agent was removed by filtration, the solvent was evaporated under reduced pressure, and the residue was purified by basic silica gel column chromatography (eluate: ethyl acetate/hexane = 1/10-1/1) and recrystallized from an ethyl acetate-hexane mixed solvent to obtain methyl N-{[7-(4-chlorophenyl)imidazo[1,2-a]pyridin-2-yl]carbonyl}-O-(4-ethylbenzyl)tyrosinate (206 mg, 80%) as a white solid.
   LC-MS 568 [M+H]⁺
   ¹H-NMR (300MHz, DMSO-d₆); δ 1.13-1.18 (t, J=7.5Hz, 3H), 2.54-2.68 (m, 2H), 3.13-3.15 (d, J=6.6Hz, 2H), 3.66 (s, 3H), 4.72-4.80 (m ,1H), 4.98 (s, 2H), 6.88-6.91 (d, J=8.7Hz, 2H), 7.14-7.20 (m, 4H), 7.25-7.42 (m, 3H), 7.56-7.59 (d, J=8.7Hz, 2H), 7.86-7.91 (t, J=8.7Hz, 3H), 8.40 (s, 2H), 8.65-8.68 (d, J=7.2Hz, 1H)
(2) 1 M Sodium hydroxide aqueous solution (725 µL) was added to a tetrahydrofuran-methanol mixed solution (2 mL-2 mL) of methyl N-{[7-(4-chlorophenyl)imidazo[1,2-a]pyridin-2-yl]carbonyl}-O-(4-ethylbenzyl)tyrosinate (206 mg, 0.36 mmol), and stirred for 60 minutes at 60°C. The reaction solution was neutralized with 1 N hydrochloric acid (750 µL), and the precipitated white solid was filtered out and washed successively with water and diethyl ether and then recrystallized from a dimethylformamide-water mixed solvent to obtain the title compound (166 mg, 83%) as a white solid.
   LC-MS 554 [M+H]⁺
   ¹H-NMR (300MHz, DMSO-d₆);δ 1.13-1.18 (t, J=7.8Hz, 3H), 2.51-2.68 (m, 2H), 3.13-3.15 (m, 2H), 4.67-4.74 (m, 1H), 4.98 (s, 2H), 6.88-6.90 (d, J=8.4Hz, 2H), 7.13-7.40 (m, 7H), 7.56-7.59 (d, J=8.7Hz, 2H), 7.85-7.88 (d, J=8.7Hz, 2H), 7.93 (s, 1H), 8.15-8.17 (d, J=5.1Hz, 1H), 8.39 (s, 1H), 8.64-8.66 (d, J=7.2Hz, 1H), 12.80 (br s, 1H)
   Melting point: 245°C
   Elemental analysis: Calcd. for C₃₂H₂₈N₃O₄ (cont. 0.1 mol H₂O), : C 69.15, H 5.11, N 7.56; Found: C 69.02, H 4.98, N 7.55

### Example E66

### N-{[7-(4-Chlorophenyl)imidazo[1,2-a]pyridin-2-yl]carbonyl}-O-[4-(trifluoromethyl)benzyl]tyrosine

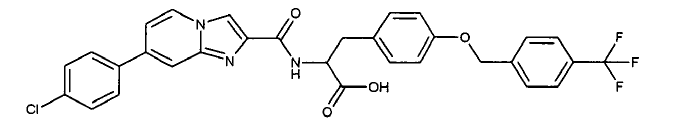

(1) A mixture of 7-(4-chlorophenyl)imidazo[1,2-a]pyridine-2-carboxylic acid (78 mg, 0.29 mmol), methyl O-[4-(trifluoromethyl)benzyl]tyrosinate hydrochloride (134 mg, 0.34 mmol), (3-dimethylaminopropyl)ethyl carbodiimide hydrochloride (192 mg, 1.00 mmol), 1-hydroxybenzotriazole (135 mg, 1.00 mmol), triethylamine (300 µL, 2.1 mmol), N,N-dimethylformamide (2 mL) and dichloromethane (4 mL) was stirred overnight at room temperature. Water was added to the reaction solution, which was then extracted with chloroform. The extract was washed with water and saturated sodium chloride solution and dried over anhydrous magnesium sulfate. The drying agent was removed by filtration, the solvent was evaporated under reduced pressure, and the residue was purified by basic silica gel column chromatography (eluate: ethyl acetate/hexane = 1/10-1/1) and recrystallized from a mixed ethyl acetate-hexane mixed solvent to obtain methyl N-{[7-(4-chlorophenyl)imidazo[1,2-a]pyridin-2-yl]carbonyl}-O-[4-(trifluoromethyl)benzyl]tyrosinate (122 mg, 83%) as a white solid.
   LC-MS 608 [M+H]⁺
   ¹H-NMR (300MHz, DMSO-d₆); δ 3.14-3.16 (d, J=6.9Hz, 2H), 3.66 (s, 3H), 4.72-4.80 (m, 2H), 5,16 (s, 2H), 6.91-6.94 (d, J=8.7Hz, 2H), 7.16-7.19 (d, J=8.7Hz, 2H), 7.37-7.40 (dd, J=1.8, 5.4Hz, 1H), 7.56-7.65 (m, 4H), 7.72-7.75 (d, J=8.1Hz, 2H), 7.85-7.91 (m, 3H), 8.38-8.41 (m, 2H), 8.64-8.66 (d, J=7.2Hz, 1H)
(2) 1 M Sodium hydroxide aqueous solution (403 µL) was added to a tetrahydrofuran-methanol mixed solution (2 mL-2 mL) of methyl N-{[7-(4-chlorophenyl)imidazo[1,2-a]pyridin-2-yl]carbonyl}-O-[4-(trifluoromethyl)benzyl]tyrosinate (122 mg, 0.20 mmol), and stirred for 60 minutes at 60°C. The reaction solution was neutralized with 1 N hydrochloric acid (450 µL), and the precipitated white solid was filtered out and washed successively with water and diethyl ether and then recrystallized from a dimethylformamide-water mixed solvent to obtain the title compound (109 mg, 92%) as a white solid.
   LC-MS 594 [M+H]⁺
   ¹H-NMR (300MHz, DMSO-d₆); δ 3.16 (m, 2H), 4.53-4.61 (m, 1H), 5.16 (s, 2H), 6.90-6.93 (d, J=8.7Hz, 2H), 7.15-7.18 (d, J=8.7Hz, 2H), 7.36-7.39 (m, 1H), 7.56-7.59 (d, J=8.4Hz, 2H), 7.62-7.65 (d, J=8.1Hz, 2H), 7.72-7.75 (d, J=8.1Hz, 2H), 7.85-7.88 (d, J=8.7Hz, 2H), 7.93 (s, 1H), 8.15-8.18 (m, 1H), 8.38 (s, 1H), 8.64-8.66 (d, J=7.2Hz, 1H), 12.80 (br s, 1H)
   Melting point: 263°C
   Elemental analysis: Calcd. for C₃₁H₂₃N₃O₄ClF₃, C 62.68, H 3.90, N 7.07; Found: C 62.80, H 4.03, N 7.08

### Example E67

### N-{[6-(4-Chlorophenyl)imidazo[1,2-a]pyridin-2-yl]carbonyl}glycine

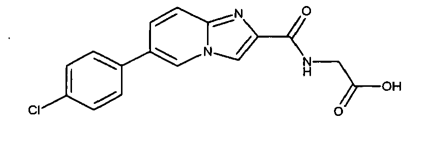

(1) A mixture of 6-(4-chlorophenyl)imidazo[1,2-a]pyridine-2-carboxylic acid (164 mg, 0.60 mmol), methyl glycinate hydrochloride (63 mg, 0.50 mmol), (3-dimethylaminopropyl)ethyl carbodiimide hydrochloride (192 mg, 1.00 mmol), 1-hydroxybenzotriazole (135 mg, 1.00 mmol), triethylamine (300 µL, 2.1 mmol), N,N-dimethylformamide (2 mL) and dichloromethane (6 mL) was stirred overnight at room temperature. Water was added to the reaction solution, which was then extracted with chloroform. The extract was washed with water and saturated sodium chloride solution and dried over anhydrous magnesium sulfate. The drying agent was removed by filtration, the solvent was evaporated under reduced pressure, and the residue was purified by basic silica gel column chromatography (eluate: ethyl acetate/hexane = 1/10-1/1) and recrystallized from an ethyl acetate-diisopropyl ether mixed solvent to obtain methyl N-{[6-(4-chlorophenyl)imidazo[1,2-a]pyridin-2-yl]carbonyl}glycinate (152 mg, 89%) as a white solid.
   LC-MS 344 [M+H]⁺
   ¹H-NMR (300MHz, DMSO-d₆); δ 3.66 (3H, s), 4.03-4.05 (2H, d, J=6.0Hz), 7.58-7.60 (2H, d, J=8.4Hz), 7.72-7.78 (4H, m), 8.38 (1H, s), 8.73-8.77 (1H, t, J=6.0Hz), 8.99 (1H, s)
(2) 1 M Sodium hydroxide aqueous solution (884 µL) was added to a tetrahydrofuran-methanol mixed solution (2 mL-2 mL) of methyl N-{[6-(4-chlorophenyl)imidazo[1,2-a]pyridin-2-yl]carbonyl}glycinate (152 mg, 0.44 mmol), and stirred for 60 minutes at 60°C. The reaction solution was neutralized with 1 N hydrochloric acid (900 µL), and the precipitated white solid was filtered out and washed successively with water and diethyl ether to obtain the title compound (105 mg, 72%) as a white solid.
   LC-MS 330 [M+H]⁺
   ¹H-NMR (300MHz, DMSO-d₆); δ 3.94-3.96 (2H, d, J=6.0Hz), 7.58-7.60 (2H, d, J=8.4Hz), 7.66-7.78 (4H, m), 8.38 (1H, s), 8.56-8.60 (1H, t, J=6.0Hz), 8.99 (1H, s), 12.60 (1H, br s)
   Elemental analysis: Calcd. for C₁₆H₁₂N₃O₃Cl, : C 58.28, H 3.67, N 12.74; Found: C 58.38, H 3.82, N 12.97

### Example E68

### N-{[6-(4-Chlorophenyl)imidazo[1,2-a]pyridin-2-yl]carbonyl}-β-alanine

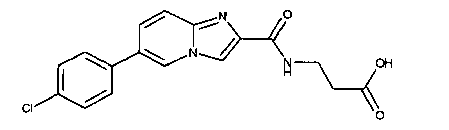

(1) A mixture of 6-(4-chlorophenyl)imidazo[1,2-a]pyridine-2-carboxylic acid (164 mg, 0.60 mmol), methyl β-alaninate hydrochloride (70 mg, 0.50 mmol), (3-dimethylaminopropyl)ethyl carbodiimide hydrochloride (192 mg, 1.00 mmol), 1-hydroxybenzotriazole (135 mg, 1.00 mmol), triethylamine (300 µL, 2.1 mmol), N,N-dimethylformamide (2 mL) and dichloromethane (6 mL) was stirred overnight at room temperature. Water was added to the reaction solution, which was then extracted with chloroform. The extract was washed with water and saturated sodium chloride solution and dried over anhydrous magnesium sulfate. The drying agent was removed by filtration, the solvent was evaporated under reduced pressure, and the residue was purified by basic silica gel column chromatography (eluate: ethyl acetate/hexane = 1/10-1/1) and recrystallized from an ethyl acetate-diisopropyl ether mixed solvent to obtain methyl N-{[6-(4-chlorophenyl)imidazo[1,2-a]pyridin-2-yl]carbonyl}-β-alaninate (160 mg, 89%) as a white solid.
   LC-MS 358 [M+H]⁺
   ¹H-NMR (300MHz, DMSO-d₆); δ 2.57-2.64 (2H, t, J=7.2Hz), 3.52-3.58 (2H, m), 3.61 (3H, s), 7.57-7.60 (2H, d, J=6.6Hz), 7.70 (2H, s), 7.73-7.77 (2H, d, J=8.7Hz), 8.34 (1H, s), 8.44-8.48 (1H, t, J=6.0Hz), 8.98 (1H, s)
(2) 1 M Sodium hydroxide aqueous solution (894 µL) was added to a tetrahydrofuran-methanol mixed solution (2 mL-2 mL) of methyl N-{[6-(4-chlorophenyl)imidazo[1,2-a]pyridin-2-yl]carbonyl}-β-alaninate (160 mg, 0.45 mmol), and stirred for 60 minutes at 60°C. The reaction solution was neutralized with 1 N hydrochloric acid (920 µL), and the precipitated white solid was filtered out and washed successively with water and diethyl ether to obtain the title compound (123 mg, 81%) as a white solid.
   LC-MS 344 [M+H]⁺
   ¹H-NMR (300MHz, DMSO-d₆); δ 2.54-2.56 (2H, m), 3.43-3.53 (2H, m), 7.57-7.60 (2H, d, J=8.7Hz), 7.66-7.77 (4H, m), 8.35-8.40 (2H, m), 8.99 (1H, s), 12.40 (1H, br s)
   Elemental analysis: Calcd. for C₁₇H₁₄N₃O₃Cl, : C 59.40, H 4.10, N 12.22; Found: C 69.27, H 4.07, N 12.20

**Preparation Example 1**

| | | |
|---|---|---|
| (1) | Compound of Example B134 | 50 mg |
| (2) | Lactose | 34 mg |
| (3) | Corn starch | 10.6 mg |
| (4) | Corn starch (paste) | 5 mg |
| (5) | Magnesium stearate | 0.4 mg |
| (6) | Carboxymethyl cellulose calcium | 20 mg |
| | Total | 120 mg |

(1)-(6) above are mixed by ordinary methods, and molded with a tablet machine to obtain tablets.

### Preparation Example 2

| | | |
|---|---|---|
| (1) | Compound of Example B134 | 10.0 mg |
| (2) | Lactose | 60.0 mg |
| (3) | Corn starch | 35.0 mg |
| (4) | Gelatin | 3.0 mg |
| (5) | Magnesium stearate | 2.0 mg |

A mixture of 10.0 mg of the compound of Example B134, 60.0 mg of lactose and 35.0 mg of corn starch is granulated by being passed through a 1 mm mesh sieve using a 10% gelatin aqueous solution (3.0 mg as gelatin), and then dried at 40°C and sieved again. The resulting granules are mixed with 2.0 mg of magnesium stearate and compressed. The resulting core tablet is sugar-coated using an aqueous suspension of sucrose, titanium dioxide, talc and gum arabic. The tablet with coating is polished with beeswax to obtain a coated tablet.

**Preparation Example 3**

| | | |
|---|---|---|
| (1) | Compound of Example B134 | 10.0 mg |
| (2) | Lactose | 70.0 mg |
| (3) | Corn starch | 50.0 mg |
| (4) | Soluble starch | 7.0 mg |
| (5) | Magnesium stearate | 3.0 mg |

10.0 mg of the compound of Example B134 and 3.0 mg of magnesium stearate are granulated with 0.07 ml of an aqueous solution of soluble starch (7.0 mg as soluble starch), dried, and mixed with 70.0 mg of lactose and 50.0 mg of corn starch. The mixture is compressed to obtain a tablet.

**Preparation Example 4**

| | | |
|---|---|---|
| (1) | Compound of Example C156 | 50 mg |
| (2) | Lactose | 34 mg |
| (3) | Corn starch | 10.6 mg |
| (4) | Corn starch (paste) | 5 mg |
| (5) | Magnesium stearate | 0.4 mg |
| (6) | Carboxymethyl cellulose calcium | 20 mg |
| | Total | 120 mg |

(1)-(6) are mixed by ordinary methods and molded with a tablet machine to obtain a tablet.

**Preparation Example 5**

| | | |
|---|---|---|
| (1) | Compound of Example C156 | 10.0 mg |
| (2) | Lactose | 60.0 mg |
| (3) | Corn starch | 35.0 mg |
| (4) | Gelatin | 3.0 mg |
| (5) | Magnesium stearate | 2.0 mg |

A mixture of 10.0 mg of the compound of Example C156, 10.0 mg of lactose and 35.0 mg of corn starch is granulated by being passed through a 1 mm mesh sieve using 0.03 ml of 10% gelatin aqueous solution (3.0 mg as gelatin), and then dried at 40°C and sieved again. The resulting granules are mixed with 2.0 mg of magnesium stearate and compressed. The resulting core tablet is sugar-coated with an aqueous suspension of sucrose, titanium dioxide, talc and gum arabic. The tablet with coating is polished with beeswax to obtain a coated tablet.

**Preparation Example 6**

| | | |
|---|---|---|
| (1) | Compound of Example C133 | 10.0 mg |
| (2) | Lactose | 70.0 mg |
| (3) | Corn starch | 50.0 mg |
| (4) | Soluble starch | 7.0 mg |
| (5) | Magnesium stearate | 3.0 mg |

10.0 mg of the compound of Example C 133 and 3.0 mg of magnesium stearate are granulated with 0.07 ml of an aqueous solution of soluble starch (7.0 mg as soluble starch), dried, and mixed with 70.0 mg of lactose and 50.0 mg of corn starch. The mixture is compressed to obtain a tablet.

**Preparation Example 7**

| | | |
|---|---|---|
| (1) | Compound of Example E51 | 50 mg |
| (2) | Lactose | 34 mg |
| (3) | Corn starch | 10.6 mg |
| (4) | Corn starch (paste) | 5 mg |
| (5) | Magnesium stearate | 0.4 mg |
| (6) | Carboxymethyl cellulose calcium | 20 mg |
| | Total | 120 mg |

(1)-(6) are mixed by ordinary methods and molded with a tablet machine to obtain a tablet.

**Preparation Example 8**

| | | |
|---|---|---|
| (1) | Compound of Example E51 | 10.0 mg |
| (2) | Lactose | 60.0 mg |
| (3) | Corn starch | 35.0 mg |
| (4) | Gelatin | 3.0 mg |
| (5) | Magnesium stearate | 2.0 mg |

A mixture of 10.0 mg of the compound of Example E51, 10.0 mg of lactose and 35.0 mg of corn starch is granulated by being passed through a 1 mm mesh sieve using 0.03 ml of 10% gelatin aqueous solution (3.0 mg as gelatin), and then dried at 40°C and sieved again. The resulting granules are mixed with 2.0 mg of magnesium stearate and compressed. The resulting core tablet is sugar-coated with an aqueous suspension of sucrose, titanium dioxide, talc and gum arabic. The tablet with coating is polished with beeswax to obtain a coated tablet.

**Preparation Example 9**

| | | |
|---|---|---|
| (1) | Compound of Example E63 | 10.0 mg |
| (2) | Lactose | 70.0 mg |
| (3) | Corn starch | 50.0 mg |
| (4) | Soluble starch | 7.0 mg |
| (5) | Magnesium stearate | 3.0 mg |

10.0 mg of the compound of Example E63 and 3.0 mg of magnesium stearate are granulated with 0.07 ml of an aqueous solution of soluble starch (7.0 mg as soluble starch), dried, and mixed with 70.0 mg of lactose and 50.0 mg of corn starch. The mixture is compressed to obtain a tablet.

**Preparation Example 10**

| | | |
|---|---|---|
| (1) | Compound of Example E59 | 50 mg |
| (2) | Lactose | 34 mg |
| (3) | Corn starch | 10.6 mg |
| (4) | Corn starch (paste) | 5 mg |
| (5) | Magnesium stearate | 0.4 mg |
| (6) | Carboxymethyl cellulose calcium | 20 mg |
| | Total | 120 mg |

(1)-(6) are mixed by ordinary methods and molded with a tablet machine to obtain a tablet.

### Test Example 1

### Preparation of human GPR34/Gα16-expressing CHO cells

The Gα16 gene was cloned by PCR from human lung cDNA (Clontech). The composition of the PCR reaction solution and the reaction conditions were as follows. The reaction solution consisted of 1 µl of human lung cDNA, 0.5 µM of synthetic DNA primer (SEQ ID NO:5), 0.5 µM of synthetic DNA primer (SEQ ID NO:6), 0.2 mM of dNTPs, 0.4 µl of Advantage cDNA polymerase (Clontech) and the buffer for the enzyme, for a total volume of 20 µl. The PCR reaction was performed using a thermal cycler (Applied Biosystems) under conditions of 120 seconds of heating at 94°C followed by 30 cycles of 30 seconds at 94°C, 30 seconds at 60°C and 90 seconds at 72°C, followed by 10 minutes at 72°C. The DNA in the reaction solution was cloned to pCR4-TOPO according to the protocols of the TOPO TA Cloning Kit (Invitrogen). This was introduced to transform *Escherichia coli* DH5α T1 phage resistant competent cells (Invitrogen), clones having the cDNA inserted fragment were selected with LB agar medium containing ampicillin and X-gal, and only those clones that appeared white were separated with a sterilized toothpick to obtain transformants. Each clone was cultured overnight in ampicillin-containing LB medium, and plasmid DNA was prepared using a QIAwell 8 Plasmid Kit (Qiagen). A BigDye Terminator Cycle Sequencing Ready Reaction Kit (Applied Biosystems) was used in the reaction to determine the nucleotide sequence, which was read with a fluorescent automatic sequencer to obtain the nucleotide sequence of SEQ ID NO:7. In comparison with the Gα16 gene registered at the Entrez Nucleotides database (Accession No. NM_002068, GeneID: 2769), this nucleotide sequence (SEQ ID NO:7) differed by one nucleotide not involving a mutation in the Gα16 protein amino acid sequence. This plasmid vector was called pCR4-TOPO human lung Gα16.

Next, Gα16 protein-coding DNA obtained by a PCR reaction using this plasmid vector as the template was cloned to the animal cell expressing vector pCR3.1 (Invitrogen). The PCR composition of the reaction solution and the reaction conditions were as follows. The reaction solution consisted of 0.5 ng of pCR4-TOPO human lung Gα16, 0.5 µM of synthetic DNA primer (SEQ ID NO:8), 0.5 µM of synthetic DNA primer (SEQ ID NO:6), 0.2 mM dNTPS, 0.4 µl of Advantage cDNA polymerase (Clontech) and the buffer for the enzyme, for a total volume of 20 µl. The PCR reaction was performed using a thermal cycler (Applied Biosystems) under conditions of 120 seconds of heating at 94°C followed by 20 cycles of 30 seconds at 94°C, 30 seconds at 60°C and 90 seconds at 72°C, followed by 10 minutes at 72°C. The amplified DNA was isolated by 1.5% agarose gel electrophoresis, gel containing DNA about 1100 nucleotides in length was excised with a razor, and the DNA was collected using a QIAquick Gel Extraction Kit (Qiagen). This DNA was cloned to pCR3.1 using a eukaryotic cell TA expression kit (Invitrogen) in accordance with the attached protocols. This was introduced to transform *Escherichia coli* TOP10F' competent cells (Invitrogen). Individual clones were cultured overnight in LB medium containing ampicillin, and plasmid DNA was prepared using a QIAwell 8 Plasmid Kit (Qiagen). A BigDye Terminator Cycle Sequencing Ready Reaction Kit (Applied Biosystems) was used in the reaction to determine the nucleotide sequence, which was read with a fluorescent automatic sequencer to obtain the nucleotide sequence represented by SEQ ID NO:9. This nucleotide sequence was confirmed to code for the amino acid sequence of the Gα16 protein, and the resulting plasmid vector was called pCR3.1 human lung Gα16.

pCR3.1 human lung Gα16 was transfected into human GPR34 CHO cells to obtain a human GPR34 CHO cell line stably expressing Gα16. pCR3.1 human lung Gα16 was introduced into a human GPR34 CHO cell line using Effectene Transfection Reagent (Qiagen). These cells were cultured in nucleic acid-free αMEM medium containing 3 mg/ml Geneticin (Invitrogen) and 10% dialyzed fetal bovine serum, and cells having the introduced pCR3.1 human lung Gα16 were selected. Cells that formed colonies and survived were removed and cultured in nucleic acid-free αMEM medium containing 3 mg/ml Geneticin (Invitrogen) and 10% dialyzed fetal bovine serum.

The number of expressed copies of the Gα16 gene in the human GPR34 CHO cell line with introduced Gα16 gene was counted by the TaqMan method, and a human GPR34 CHO cell line highly expressing Gα16 was selected. RNA of the human GPR34 CHO cell strain with introduced Gα16 gene was purified using ISOGEN (Nippon Gene). The solution for the cDNA synthesis reaction with this RNA as the template consisted of 0.5 µg of RNA, 25 pmol of random hexamer, 1 mM of dNTPs, 1 µl of ReverTraAce (Toyobo) and reaction buffers attached to the kit, for a total volume of 20 µl. The reverse transcription reaction was performed using a thermal cycler (Takara) under conditions of 10 minutes at 30°C followed by 60 minutes at 42°C followed by 5 minutes at 99°C. The TaqMan PCR reaction solution for measuring the number of expressed copies of the gene consisted of 1 µl of cDNA solution or assay Gα16 standard DNA solution, 0.2 µM of TaqMan forward primer (SEQ ID NO:10), 0.2 µM of TaqMan reverse primer (SEQ ID NO:11), 0.2 µM of Gα16 TaqMan probe (SEQ ID NO:12) and a TaqMan Universal PCR Master Mix (Applied Biosystems), for a total volume of 25 µl. The PCR reaction was performed using an ABI Prism 7700 Sequence Detector System (Applied Biosystems), and consisted of 2 minutes at 50°C and 10 minutes at 95°C, followed by 40 cycles of 15 seconds at 95°C and 60 seconds at 60°C. The number of expressed copies of the Gα16 gene was calculated using ABI Prism 7700 SDS software.

The reactivity of the human GPR34 CHO cell strain stably expressing Gα16 to lysophosphatidyl serine (lysoPS) was investigated by the FLIPR (Molecular Devices) assay method.

The fluorescent calcium indicator Fluo 3-AM (Wako Pure Chemical) was incorporated into the human GPR34 CHO cell strain stably expressing Gα16, and the cells were washed with assay buffer. Various concentrations of 50 µl lysophosphatidyl serine solution were added to cells immersed in 100 µl of assay buffer, and the intracellular fluorescent strength 3 minutes after addition was measured by FLIPR.

The results are shown in Table 1.

In the human GPR34 CHO cell strain stably expressing Gα16, lysophosphatidyl serine caused a rise in intracellular fluorescent strength reflecting a temporary rise in intracellular calcium concentration. The rise in intracellular fluorescent strength was also dependent on the lysophosphatidyl serine concentration.

### Test Example 2

### Measurement of human GPR34 agonist and antagonist activity of compound of invention as indicated by changes in intracellular calcium concentration

CHO cells expressing human GPR34/Gα were diluted to 3 x 10⁴/100 µl in cell culture medium [nucleic acid-free Minimum Essential Alpha Medium (Invitrogen) containing 10% dialyzed fetal bovine serum (Invitrogen) 0.5 mg/ml Geneticin (Invitrogen), penicillin and streptomycin (Invitrogen)], dispensed 100 µl per well into a Black walled 96-well plate (Costar), and cultured overnight in a CO₂ incubator. Changes in intracellular calcium concentration were measured with a FLIPR (Molecular Device) by the methods described below.

50 µg of Fluo-3AM (Dojindo) was dissolved in 21 µl of DMSO (Dojindo), an equal amount of 20% pluronic acid (Molecular Probes) was added and mixed, and this was added to 10.6 ml of assay buffer [prepared by adding 20 ml of 1 M HEPES (pH 7.4, Dojindo) to HBSS (Invitrogen), and then adding 10 ml of a solution obtained by dissolving 710 mg of Probenecid (Sigma) in 5 ml of 1 N NaOH and then adding and mixing 5 ml of the HBSS/HEPES solution)] with 105 µl of fetal bovine serum added thereto to prepare a fluorescent dye solution. The medium was removed from the cell plate, 100 µl per well of the fluorescent dye solution was immediately poured in, and the cells were cultured for 1 hour in a CO₂ incubator to incorporate the fluorescent dye into the cells. After culture, the cells were washed with the aforementioned assay buffer. The compound was dissolved in DMSO (Wako Pure Chemical), diluted with DMSO as necessary, added to the aforementioned assay buffer to the target concentration and poured into the plate. To measure antagonist activity, 8 µM lysophosphatidyl serine (lysoPS, Alexis) solution (2 µM final concentration during reaction) was poured into the plate, which was set in the FLIPR. Following these preliminaries, the agonist effect was measured in the FLIPR by measuring changes in intracellular calcium concentration up to 180 seconds after addition of the sample, and after 200 seconds lysoPS was added and the antagonist effect was measured. In the case of the agonist effect, the EC₅₀ value of the added compound was calculated from the dose reaction curve using changes in fluorescent strength from 0 to 180 seconds after the start of the reaction. In the case of the antagonist effect, the IC₅₀ was calculated from the dose reaction curve using changes in maximum fluorescent strength after 200 to 380 seconds of reaction time. The data was analyzed using "GraphPad PRISM4".

As a result, in terms of antagonist activity the compounds of Example B130, Example B134, Example B147, Example B155, Example B158, Example B174, Example C 115, Example C118 and Example C119 exhibited IC₅₀ values of 1 µM or less.

This shows that the compound of the present invention is an excellent antagonist against human GPR34.

### Test Example 3

### Measurement of human GPR34 antagonist activity of compounds of the present invention as indicated by cAMP

### (3-1) Cloning of cDNA coding for human GPR34, and preparation of animal cell expression vector

Using the human genome (Clontech) as the template, PCR was performed using primer 1 (SEQ ID NO:3) with an SalI recognition sequence added thereto and primer 2 (SEQ ID NO:4) with a SpeI recognition sequence added thereto. With 100 ng of the aforementioned genome as the template, the composition of the reaction solution in this reaction was 2.5 U of Pfu Turbo DNA Polymerase (Stratagene), 1.0 µM each of primer 1 (SEQ ID NO:3) and primer 2 (SEQ ID NO:4), 200 µM of dNTPs and 25 µl of 2XGC BufferI (Takara) added to the enzyme, for a liquid volume of 50 µl. The PCR reaction consisted of 1 minute at 94°C followed by 38 cycles of 30 seconds at 94°C, 15 seconds at 54°C and 1.5 minutes at 72°C. The PCR reaction product was purified with a PCR Purification Kit (Qiagen) and eluted with 30 µl of the Buffer EB attached to the kit, and 10 µl of this was cleaved with the restriction enzymes SalI and SpeI. The restriction enzyme reaction product was subjected to agarose gel electrophoresis, excised from the agarose gel and collected with a Gel Extraction Kit (Qiagen). This reaction product was added to the animal cell expression vector plasmid pAKKO-111H (identical to the pAKK01.11H described in Biochim. Biophys. Acta, Vol. 1219, pp. 251-259, 1994) cleaved with SalI and Spel, and subjected to a ligation reaction using a DNA Ligation Kit Ver. 2 (TaKaRa). This was introduced into *E*. *coli* TOP10 (Invitrogen), and clones having GPR34 cDNA were selected in LB agar medium containing ampicillin. The sequences of the individual clones were analyzed to obtain *E*. *coli* containing a plasmid (called pAKKO-GPR34) comprising the nucleotide sequence (SEQ ID NO:2) of cDNA coding for GPR34 (SEQ ID NO:1). This *E*. *coli* TOP10 transformed with pAKKO-GPR34 was cultured, and pAKKO-GPR34 plasmid DNA was prepared using a Plasmid Miniprep Kit (Biorad). The resulting GPR34 amino acid sequence was identical to the GPR34 amino acid sequence described in Genomics, Vol. 56, pp. 12-21, 1999, with Leu at No. 181 of the amino acid sequence. The GPR34 described in Biochim. Biophys. Acta, Vol. 1446, pp. 57-70, 1999 has Val at No. 181 in the amino acid sequence.

### (3-2) Preparation of GPR34-expressing CHO cell strain

1×10⁵ hamster CHO/dhfr cells were seeded on a falcon dish (dia. 3.5 cm) using α-MEM medium (Gibco, Cat. No. 12571) containing 10% fetal bovine serum, and cultured overnight at 37°C in a 5% CO₂ incubator. 2 µg of the expression plasmid pAKKO-GPR34 obtained in (1-1) above was transfected using a Transfection Reagent FuGENE6 (Roche) in accordance with the methods described in the manual, and new growth medium was substituted after 18 hours of culture. Culture was continued for 10 more hours, and the transfected cells were collected by trypsin-EDTA treatment and seeded in a flat-bottomed 96-well plate using selection medium (α-MEM medium (Gibco, Cat. No. 12561) containing 10% dialyzed fetal bovine serum). Culture was continued with the selection medium changed every 3-4 days, and 76 DHFR⁺ cell clones that proliferated as colonies were obtained after 2-3 weeks.

### (3-3) Measurement of human GPR34 antagonist activity as indicated by cAMP

Human GPR34-expressing CHO cells were maintained in nucleic acid-free MEM-α (Invitrogen) containing 10% dialyzed FBS (Invitrogen) and penicillin and streptomycin (BioWhittaker) (hereunder described as culture medium), and on the day before assay the cells were stripped with trypsin/EDTA (Invitrogen), centrifuged, re-suspended in culture medium and seeded 2 x 10⁵ per well, 1 ml/well on a 24-well plate (Nunc). The seeded cells were cultured overnight at 37°C in a CO₂ incubator, and subjected to cAMP assay the next day. The cAMP assay was performed by the following methods, with the indicator being the degree to which the compound restored cAMP levels depleted by lysoPS addition after intracellular cAMP had been elevated by addition of 2.5 5 µM Forskolin (Wako Pure Chemical) in human GPR34-expressing CHO cells.

The plate was washed twice, 500 µL/well with Hank's balanced salt solution (HBSS, Invitrogen) containing 20 mM HEPES (1 M HEPES solution, Dojindo) with 0.2 mM 3-isobutyl-1-methylxanthine (IBMX, Wako Pure Chemical) added thereto (hereunder described as assay buffer (HBSS+HEPES+IBMX)), and kept warm for 30 minutes in a water bath at 37°C. Next, 250 µL of assay buffer (HBSS+HEPES+IBMX) was substituted, and the plate was kept for a further 15 minutes in a water bath at 37°C. 2 µl of GPR34 antagonist compound solution at 250x the final concentration was added and gently stirred with a plate mixer, 2 µL of lysoPS (Sigma or Alexis) at 250x the final concentration and 5 µM (2x the final concentration) of Forskolin (Wako Pure Chemical) were added, and 250 µL of assay buffer (HBSS+HEPES+IBMX) was added and reacted for 30 minutes in a water bath at 37°C. In the case of the human GPR34-expressing CHO cells, 2 µL of 50 µM lysoPS was added so that the final concentration would be roughly the EC₅₀ value of 0.2 µM. After completion of the reaction, intracellular cAMP was detected by competitive EIA using a cAMP Screen System (Applied Biosystems). After completion of the assay, 200 µL of the Assay/lysis buffer for the cAMP Screen System was substituted, and intracellular cAMP was extracted by maintaining the cells for about 40 minutes at 37°C. EIA was then performed in accordance with the cAMP Screen Systems protocols to assay intracellular cAMP.

Relative percentage values were calculated by Microsoft Excel with the cAMP level with only assay buffer (HBSS+HEPES+IBMX) added ("None" in the table) given as 0%, and the cAMP level with the final concentration 2.5 µM Forskolin added given as 100%.

**The results are shown below.**

| Compound | Concentration (µM) | cAMP (%) |
|---|---|---|
| None | 0 | 0 |
| Forskolin | 2.5 | 100 |
| Forskolin + LysoPS | 2.5 + 0.2 | 43 |
| Forskolin + LysoPS + Example B134 | 2.5 + 0.2 + 10 | 299 |

These results show that at 10 µM, the compound of Example B134 restored cAMP levels to at or above the level before Forskolin stimulus.

This shows that the compound of the present invention is an excellent inverse agonist for human GPR34.

### Test Example 4

### Degranulation inhibiting activity of compound of invention in rat peritoneal mast cells

### (4-1) Preparation of rat peritoneal mast cells

Mast cells from rat peritoneum (hereunder RPMC) were obtained as follows.

Ice-cooled mast cell medium (hereunder called MCM: 150 mM NaCl, 3.7 mM KCI, 3.0 mM NaH₂PO₄, 3.5 mM KH₂PO₄, 5.6 mM (D)-Glucose, 0.1% bovine serum albumin (BSA), 0.1% gelatin, 10 U/ml heparin, pH 6.8) was injected intraperitoneally into rats, about 40 ml per rat, the abdomens were massaged for about 2 minutes and then opened along the median line, and ascites was collected. The collected ascites were filtered with a cell strainer (Becton Dickinson) and centrifuged for 7 minutes at 50 x g, and the precipitated cells were suspended in MCM and then centrifuged again for 7 minutes at 50 x g. The precipitated cells were suspended in 5 ml of MCM, layered over 2 ml of 32% (w/v) BSA solution (dissolved in 0.9% NaCL), and left for 20 minutes at room temperature. This was centrifuged for 15 minutes at room temperature at 300 x g, the cells between the MCM and BSA solution were discarded, and the precipitated cells were suspended in MCM and washed twice by being centrifuged for 5 minutes at 300 x g. The precipitated cells were suspended in assay buffer (150 mM NaCl, 3.7 mM KCl, 3.0 mM NaH₂PO₄, 3.5 mM KH₂PO₄, 0.9 mM CaCl₂, 5.6 mM (D)-Glucose, 0.01% BSA, pH 6.8), and these cells were used in the following test. When some of the resulting cells were taken and stained with alcian blue solution, 95% were shown to be RPMC cells.

### (4-2) Measurement of degranulation inhibiting activity

Rat peritoneal mast cells (RPMC) obtained by the methods given in (4-1) above were activated as follows.

RPMC cells suspended in assay buffer were passively sensitized by adding 10 µg/ml of monoclonal mouse anti-DNP-IgE (Seikagaku) and leaving them for 30 minutes on ice. After 5 minutes of centrifugation at 300 x g, the precipitated cells were washed 3 times with assay buffer. The RPMC cells suspended in assay buffer were dispensed on 96-well V-bottom plates, 2 x 10⁴ cells per well. The assay was performed with n = 3 under each set of reaction conditions. The plates were maintained for 5 minutes at 37°C, the test compound was added, and after 15 minutes lysoPS (Sigma) was added to a final concentration of 3 µM and DNP-BSA (Calbiochem) to a final concentration of 100 ng/ml for a final capacity of 200 µl, and left on a water bath at 37°C. After 20 minutes, the plate was ice cooled to stop RPMC activation, followed by 5 minutes of centrifugation at 300 x g, and β-hexosaminidase activity in the supernatant was measured using 4-methylumbelliferyl-2-acetamido-2-deoxy-β-D-glucopyranoside (Wako Pure Chemical) degradation as the indicator.

As a result, spontaneous degranulation was 3.1%. Without LysoPS, 100 ng/ml of DNP-BSA stimulus caused 4.4% RPMC degranulation. Degranulation due to 100 ng/ml of DNP-BSA stimulus rose with the concentration of lysoPS, to 8.4% when 0.1 µM of lysoPS was added simultaneously, 14.7% when 0.3 µM of lysoPS was added simultaneously, 26.0% when 1 µM of lysoPS was added simultaneously, 36.7% when 3 µM of lysoPS was added simultaneously and 45.2% when 10 µM of lysoPS was added simultaneously.

This shows that promotion of mast cell degranulation is increased lysoPS concentration-dependently.

When the degranulation inhibiting activity of test compounds was evaluated, with degranulation caused by co-stimulation with 3 µM of LysoPS and 100 ng/ml of DNP-BSA as 100% and spontaneous degranulation as 0%, degranulation of rat peritoneal mast cells due to LysoPS and DNP-BSA stimulation was significantly inhibited (t-test vs DNP-BSA/lysoPS p<0.01) by 10 µM of the compounds of Example B13, Example B174 and Example C 115, to 10%, 8% and 20%, respectively.

This shows that the compound of the present invention has mast cell degranulation inhibiting action.

### Test Example 5

### Measurement of human GPR34 agonist and antagonist activity of compound of invention as indicated by changes in intracellular calcium concentration

When alterations in intracellular calcium concentration by test compounds were measured as in Test Example 2, the antagonist activity of the compounds of Example B183, Example B184, Example B185, Example B186, Example B192, Example B194, Example C 119, Example C122, Example C123, Example C131, Example C133, Example C139, Example C142, Example C 143, Example C 144, Example C 145, Example C 146, Example C 147, Example C 148, Example C149, Example C153, Example C156, Example C157, Example C158, Example C163, Example C164, Example C165, Example C166, Example C167, Example C168, Example D1, Example D2, Example E1, Example E2, Example E3, Example E5, Example E6, Example E7, Example E8, Example E9, Example E10, Example E12, Example E13, Example E14, Example E15, Example E16, Example E19, Example E22, Example E23, Example E24, Example E25, Example E26, Example E27, Example E28, Example E29, Example E37, Example E41, Example E42, Example E45, Example E47, Example E48, Example E49, Example E51, Example E54, Example E56, Example E57, Example E59, Example E62, Example E63, Example E64 and Example E65 was measured at an IC₅₀ value of 1 µM or less.

This shows that the compound of the present invention is an excellent antagonist against human GPR34.

### Test Example 6

### Degranulation inhibiting activity of compound of invention in rat peritoneal mast cells

When the degranulation inhibiting activity of test compounds was evaluated as in Test Example 2, degranulation of rat peritoneal mast cells due to stimulus with lysoPS and DNP-BSA was significantly inhibited by 10 µM of the compounds of Example C131, Example C133, Example C157, Example E51, Example E57, Example E59, Example E63 and Example E65, to 10.2%, 12.4%, 4.8%, 5.0%, 8.3%, 5.1%, 5.7% and 8.8%, respectively.

This shows that the compound of the present invention has excellent mast cell degranulation inhibiting action.

### Industrial Applicability

Due to its unique chemical structure, Compound (I) of the present invention has excellent GPR34 function regulating activity such as GPR34 antagonist activity, mast cell degranulation-inhibiting action, histamine release-inhibiting action, leukotriene production-inhibiting action, prostaglandin production-inhibiting action, IL-13 production-inhibiting action, tryptase excretion-inhibiting action, antigen-antibody reaction-inhibiting action and the like, and has low toxicity and few side-effects. Consequently, Compound (I) is useful as a safe medicament, such as for example a GPR34 receptor antagonist (including inverse agonist or partial agonist), mast cell degranulation inhibitor, histamine release inhibitor, eicosanoid production inhibitor, mast cell proliferation inhibitor, IL-13 production inhibitor, tryptase secretion inhibitor or antigen-antibody reaction inhibitor; or a preventive/therapeutic agent for immune disease [for example, inflammatory disease (e.g., pituitary abscess, thyroiditis, peritonitis, Crohn's disease, ulcerative colitis, erythema nodosum, chronic rheumatoid arthritis, systemic lupus erythematosus and the like), allergies (e.g., allergic conjunctivitis, allergic rhinitis, hay fever, metal allergies and the like), asthma, exudative otitis media, Ménière's disease, contact dermatitis, anaphylaxis, hives, myasthenia gravis, glomerulonephritis, Sjögren's syndrome, Basedow's disease, insulin resistant diabetes, atopic dermatitis, leukocyte abnormalities and the like], respiratory disease [for example, chronic obstructive pulmonary disease (e.g., chronic bronchitis or pulmonary edema), diffuse panbronchiolitis, cystic fibrosis, hypersensitivity pneumonitis, idiopathic interstitial pneumonia, pulmonary fibrosis and the like], urinary tract disease (e.g., renal tubulointerstitial disease (fibrosis), interstitial cystitis, allergic cystitis and the like), cardiovascular disease (e.g., arteriosclerosis, acute coronary syndrome, atherosclerotic aortic aneurysm, cardiac anaphylaxis, heart failure, myocardial infarction, angina, arrhythmia, deep phlebothrombosis, post-PTCA restenosis and the like), ophthalmological disease (e.g., pterygium, vernal conjunctivitis, dry eye and the like), cancer (e.g., papillary thyroid carcinoma, non-small cell lung cancer, endometrial cancer, cervical cancer, stomach cancer, pancreatic cancer, lung cancer, kidney cancer, liver cancer, ovarian cancer, prostate cancer, bladder cancer, breast cancer, colon cancer, rectal cancer, Kaposi's sarcoma, mastocytoma and the like), digestive disease [including chronic liver disease, food allergies, allergic enteritis, milk protein-induced proctitis, digestive ulcers (e.g., stomach ulcer, duodenal ulcer, stomal ulcer, Zollinger-Ellison syndrome and the like), gastritis, reflux esophagitis, NUD (non-ulcer dyspepsia), gastric MALT lymphoma, ulcers caused by non-steroidal anti-inflammatory drugs, hyperacidity, ulcers and hyperacidity caused by post-surgical stress and the like], cerebral infarction, hyperlipidemia, acute renal failure, diabetes, obesity, edema, granuloma, atopic myelitis, neurofibroma, nasal mucosal hypersensitivity, Hodgkin's disease, endometrial hyperplasia, central disease [for example, neurodegenerative disease (e.g., Alzheimer's disease (familial Alzheimer's disease, early-onset Alzheimer's disease, sporadic Alzheimer's disease, etc.), Parkinson's disease, Down's syndrome, amyotrophic lateral sclerosis, prion disease (Creutzfeldt-Jakob disease), Huntington's chorea, diabetic neuropathy, multiple sclerosis and the like), psychological disease (e.g., schizophrenia, depression, bipolar disorder, anxiety disorder, attention deficit hyperactivity disorder, panic disorder and the like), and cerebrovascular disease (e.g., cerebral thrombosis, cerebral infarction, transient ischemic attack, etc.) and the like] and others.

## Claims

1. A GPR34 receptor function regulator comprising the compound represented by the formula: 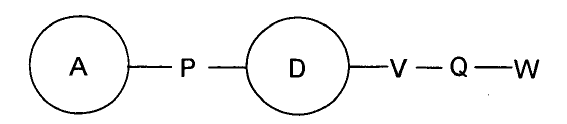 [wherein ring A is an optionally substituted isocyclic or heterocyclic ring,
P is a bond or spacer,
ring D is an optionally substituted monocyclic aromatic ring which may be condensed with a 5- to 7-membered ring,
V is a bond or the group represented by the formula -CR¹⁴=CR¹⁵- or -N=CR¹⁶- (wherein R¹⁴, R¹⁵ and R¹⁶ are each hydrogen atoms or optionally substituted hydrocarbon groups),
Q is a bond or spacer, and
W is a carboxyl or a group biologically equivalent to a carboxyl, and
the group represented by the formula -V-Q-W is substituted at any position on ring D, and
V is the group represented by the formula -CR¹⁴=CR¹⁵- or -N=CR¹⁶- when the group represented by -V-Q-W and the group represented by 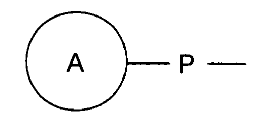 are substituted on the same ring], or its salt or a prodrug thereof

2. The regulator according to Claim 1, wherein ring D is an optionally substituted condensed ring formed from a 5- to 7-membered ring and a monocyclic aromatic ring.

3. The regulator according to Claim 1, containing the compound represented by the formula: 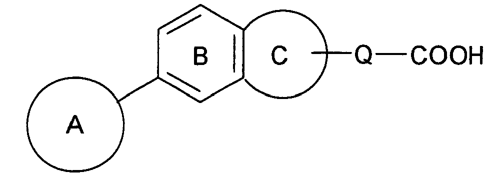 (wherein ring A and Q are as defined in Claim 1,
ring B is an optionally substituted benzene ring,
ring C is a 5- to 7-membered isocyclic or heterocyclic ring which may also include a substituent other than the group represented by the formula -Q-COOH, and
the group represented by the formula -Q-COOH is substituted at any position on ring C), or its salt or a prodrug thereof

4. The regulator according to Claim 1, which is a GPR34 receptor antagonist.

5. The regulator according to Claim 1, which is a mast cell degranulation inhibitor, histamine release inhibitor, eicosanoid production inhibitor, mast cell proliferation and differentiation inhibitor or IL-13 production inhibitor.

6. The regulator according to Claim 1, which is a preventive/therapeutic agent for immune disease, inflammatory disease, allergic disease, respiratory disease, urinary tract disease, central disease or cardiovascular disease.

7. A compound represented by the formula: 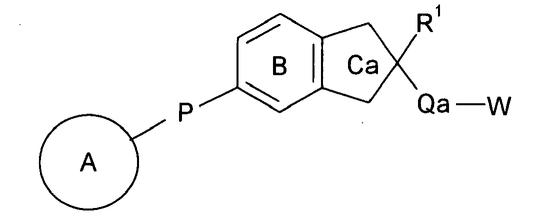 [wherein A is an optionally substituted isocyclic or heterocyclic ring,
ring B is an optionally substituted benzene ring,
ring Ca is a cyclopentene ring which may also include a substituent other than R¹ and the group represented by -Qa-W (wherein Qa is a bond or -CO-Qa'- (with Qa' being a spacer having a terminal carbon atom to which a carboxyl or a group biologically equivalent to a carboxyl is bound), and W is a carboxyl or a group biologically equivalent to a carboxyl),
P is a bond or spacer, and
R¹ is an optionally substituted amino], or a salt thereof.

8. The compound according to Claim 7, wherein P is a bond or a spacer having two atoms in the main chain.

9. The compound according to Claim 7, wherein P is a bond and W is a carboxyl.

10. The compound according to Claim 7, wherein ring A is an optionally substituted aromatic ring.

11. The compound according to Claim 7, wherein R¹ is a carbonylamino having a substituent or a sulfonylamino having a substituent.

12. The compound according to Claim 11, wherein the substituent is a group having an optionally substituted aromatic group.

13. The compound according to Claim 7, wherein R¹ is a group represented by the formula: 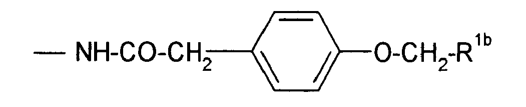 (wherein R^{1b} is an optionally substituted aromatic group).

14. The compound according to Claim 7, wherein either Qa is a bond or Qa'-W is an amino acid.

15. The compound according to Claim 7, represented by the formula: 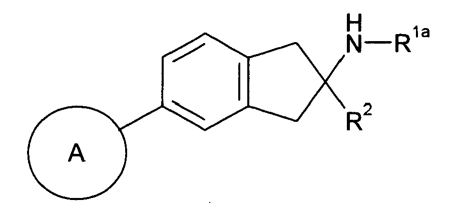 [wherein R^{1a} is either
(i) a carbonyl having a substituent selected from (1) alkyl which have optionally substituted aromatic group and the alkyl may be further substituted, (2) alkyl which have optionally substituted non-aromatic heterocyclic group and which may themselves be further substituted, (3) alkyl which have optionally substituted mercapto and the alkyl may be further substituted, (4) optionally substituted aromatic group, (5) amino having optionally substituted aromatic group, (6) optionally substituted cycloalkyl, (7) optionally substituted nitrogen-containing non-aromatic heterocyclic group, (8) optionally substituted alkyl and (9) optionally substituted alkenyl, or
(ii) an alkylsulfonyl which has an optionally substituted aromatic group and the alkylsulfonyl may be further substituted,
R² is a carboxyl, a group biological equivalent to a carboxyl or the group represented by the formula CO-Z-OH (wherein Z-OH is an amino acid), and
ring A is as defined in Claim 7].

16. The compound according to Claim 7, wherein
ring A is a 5- or 6-membered aromatic ring optionally substituted with 1 to 3 group selected from halogen atom, optionally halogenated alkyl and optionally halogenated alkoxy,
ring B is a benzene ring having 1 to 3 substituents selected from halogen atom, alkyl and alkoxy,
ring Ca is an unsubstituted cyclopentene ring,
P is a bond, ethylene, ethenylene or ethynylene,
R¹ is an acylamino having an aromatic group, and
Qa-W is a carboxyl or the group represented by CO-Z-OH (wherein Z-OH is an amino acid).

17. The compound according to Claim 15, wherein
ring A is a 5- or 6-membered aromatic ring which may have 1 to 3 substituents selected from (a) halogen atom, (b) optionally halogenated C₁₋₆ alkyl, (c) optionally halogenated C₁₋₆ alkoxy and (d) amino optionally substituted with 1 or 2 groups selected from C₁₋₆ alkyl-carbonyl and C₁₋₆ alkyl,
R^{1a} is:
(i) a carbonyl having a substituent selected from
(1) C₁₋₆ alkyl having 5- to 10-membered aromatic group which may have 1 to 3 substituents selected from (a) halogen atom, (b)C₁₋₆ alkylsulfonyl, (c) C₁₋₆ alkoxy optionally substituted with 5- or 6-membered aromatic heterocyclic group which may have C₁₋₆ alkyl, (d) C₇₋₁₃ aralkyloxy which may have 1 to 3 substituents selected from halogen atom, C₁₋₆ alkoxy and optionally halogenated C₁₋₆ alkyl, (e) 5- to 10-membered non-aromatic isocyclic ring-oxy, (f) optionally halogenated C₆₋₁₂ aryloxy, (g) 5- or 6-membered aromatic heterocyclic ring-oxy, (h) optionally halogenated C₆₋₁₂ aryl-carbonylamino, (i) optionally halogenated C₇₋₁₃ aralkylamino and (j) C₆₋₁₂ aryl, and optionally having 1 to 3 substituents selected from amino which may have hydroxyl and C₁₋₆ alkoxy-carbonyl,
(2)C ₁-₆alkyl having 6-membered nitrogen-containing non-aromatic heterocyclic group which may have 1 or 2 substituents selected from (a) C₁₋₆ alkyl which may have 1 or 2 optionally halogenated C₆₋₁₂ aryl (b) C₆₋₁₂ aryl, (c) optionally halogenated C₁₋₁₂ aryl-carbonyl, and (d) optionally halogenated C₇₋₁₃ aralkyl-carbonyl,
(3)C₁₋₆alkyl having 5- to 10-membered aromatic mercapto,
(4) 5- to 10-membered aromatic group which may have 1 to 3 substituents selected from halogen atom, the C₁₋₆ alkyl which may have 5- to 10-membered aromatic heterocyclic group optionally substituted with C₁₋₆ alkyl, and the optionally halogenated C₆₋₁₂ aryl,
(5) amino having optionally halogenated C₆₋₁₂ aryl,
(6) C₃₋₆ cycloalkyl,
(7) optionally oxonated 5-membered nitrogen-containing non-aromatic heterocyclic group,
(8)C₁₋₆alkyl which may have 1 or 2 substituents selected from optionally oxonated 5-membered nitrogen-containing non-aromatic heterocyclic group, C₇₋₁₃ aralkyloxy, C₁₋₆ alkoxy, carboxy, C₁₋₆ alkoxy-carbonyl and amino, and
(9) C₂₋₆ alkenyl, or
(ii) an optionally halogenated C₇₋₁₃ aralkylsulfonyl,
and R² is a carboxy-C₁₋₆ alkyl-carbamoyl, carboxyl, 5-tetrazolyl or 5-tetrazolylaminocarbonyl.

18. The compound according to Claim 7, which is 5-(4-chlorophenyl)-2-[({4-[(4-fluorobenzyl)oxy]phenyl}acetyl)amino]indan-2-carboxylic acid.

19. A prodrug of the compound according to Claim 7.

20. A pharmaceutical comprising the compound according to Claim 7 or its salt or a prodrug thereof.

21. The compound represented by the formula: 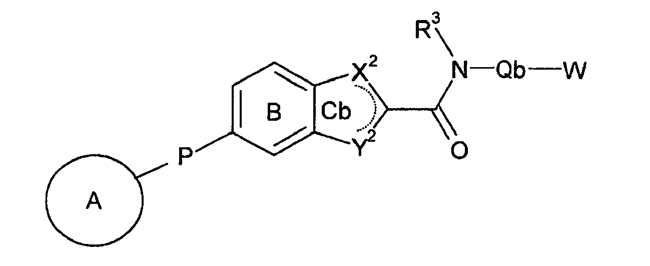 [wherein ring A is an optionally substituted isocyclic or heterocyclic ring,
ring B is an optionally substituted benzene ring,
ring Cb is an optionally substituted 5- or 6-membered ring,
one of X² and Y² is -O-, -S-, -NR⁴-, -O-CH₂-, -S-CH₂- or -NR⁴-CH₂-(wherein R⁴ is a hydrogen atom, optionally substituted hydrocarbon group or substituted sulfonyl) while the other is -N= or -CR⁵= (wherein R⁵ is a hydrogen atom or a substituent),
R³ is a hydrogen atom or optionally substituted hydrocarbon group, or R³ and Qb may be bound together to form a ring,
Qb is an optionally substituted chain spacer,
W is a carboxyl or a group biologically equivalent to a carboxyl,
P is a bond or spacer, and
any one of the broken lines represents a double bond], or a salt thereof.

22. The compound according to Claim 21, wherein ring A is an optionally substituted aromatic ring.

23. The compound according to Claim 21, wherein ring A is an optionally substituted 5- or 6-membered aromatic ring.

24. The compound according to Claim 21, wherein one of X² and Y² is -O-, -S-, - NR⁴- or -S-CH₂-, while the other is -N= or -CR⁵=

25. The compound according to Claim 21, wherein R³ is a hydrogen atom or C₁₋₆ alkyl.

26. The compound according to Claim 21, wherein Qb is an optionally substituted C₁₋₃ alkylene.

27. The compound according to Claim 26, wherein the substituent is a group having an optionally substituted aromatic group.

28. The compound according to Claim 26, wherein the optionally substituted C₁₋₃ alkylene is a methylene having an optionally substituted benzyl.

29. The compound according to Claim 21, wherein Qb is the group represented by the formula: 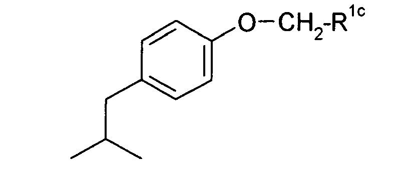 (wherein R^{1c} is an optionally substituted aromatic group).

30. The compound according to Claim 21, wherein P is a bond or a spacer having two atoms in the main chain.

31. The compound according to Claim 30, wherein the spacer having two atoms in the main chain is -CH₂CH₂-, -CH=CH-, -C≡C-, -CH₂O-, -CH₂S-, -CH₂SO- or -CH₂SO₂-.

32. The compound according to Claim 21, wherein W is a carboxyl, 5-tetrazolyl or 5-tetrazolylaminocarbonyl.

33. The compound according to Claim 21, represented by the formula: 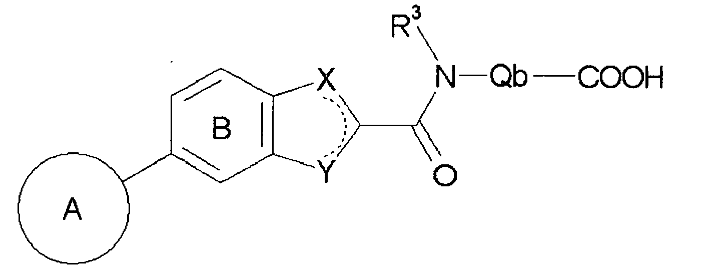 (wherein one of X and Y is -O-, -S- or -NR⁴- while the other is -N= or -CR⁵=,
ring A, ring B, R³, R⁴, R⁵ and Qb are as defined in Claim 21,
and any one of the broken lines represents a double bond).

34. The compound according to Claim 33, wherein one of X and Y is -O- or -S- and the other is -CR⁵=.

35. The compound according to Claim 34, wherein R⁵ is a hydrogen atom or hydrocarbon group.

36. The compound according to Claim 21, wherein:
ring A is a 5- or 6-membered aromatic ring optionally substituted with 1 to 3 groups selected from halogen atom, optionally halogenated C₁₋₆ alkyl and optionally halogenated C₁₋₆ alkoxy,
ring B is a benzene ring optionally substituted with 1 to 3 groups selected from halogen atom, C₁₋₆ alkyl and C₁₋₆ alkoxy,
one of X² and Y² is -O-, -S-, -NR^{4a}- or -S-CH₂-, while the other is -N= or -CR^{5a}=,
R^{4a} is a hydrogen atom or C₁₋₆ alkyl,
R^{5a} is hydrogen atom, halogen atom, amino, C₁₋₆ alkyl-carbonylamino, C₁₋₆ alkoxy-carbonylamino or C₁₋₆ alkyl,
R³ is a hydrogen atom or C₁₋₆ alkyl, or R³ and Qb may be bound together to form a 5- to 7-membered ring,
Qb is an optionally substituted C₁₋₃ alkylene or NR³-Qb-W is an amino acid,
P is a bond, ethylene, ethenylene or ethynylene, and
W is a carboxyl, 5-tetrazolyl or 5-tetrazolylaminocarbonyl.

37. The compound according to Claim 21, represented by the formula: 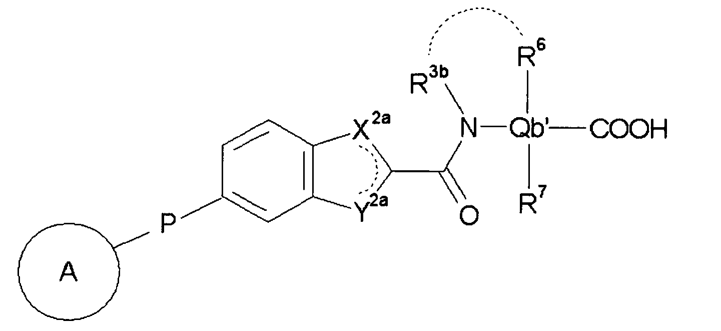 (wherein P is a bond, ethenylene or ethynylene,
ring A is a 5- or 6-membered aromatic ring optionally substituted with 1 to 3 groups selected from (1) halogen atom, (2) C₁₋₆ alkyl optionally substituted with 1 to 3 groups selected from (a) 5- or 6-membered aromatic heterocyclic group which may have C₁₋₆ alkyl and may be condensed with benzene rings and (b) halogens, and (3) amino optionally substituted with 1 or 2 groups selected from C₁₋₆ alkyl and C₁₋₆ alkyl-carbonyl,
one of X^{2a} and Y^{2a} is -O-, -S-, -NH-, -NH-CO- or -S-CH₂- while the other is -N= or - CR^{5b}= ,
R^{5b} is a hydrogen atom, amino or C₁₋₆ alkyl,
one of the broken lines in the ring represents a double bond,
R^{3b} is a hydrogen atom or C₁₋₆ alkyl,
Qb' is a C₁₋₃ alkylene,
R⁶ and R⁷ are located on the same or different carbon atoms and each represents a
(1) hydrogen atom,
(2) C₁₋₆ alkyl optionally substituted with 1 to 3 groups selected from C₁₋₆ alkoxy-carbonyl, C₇₋₁₃ aralkyloxy-carbonyl, hydroxyl, carboxyl and C₁₋₆ alkylthio group and the 5- or 6-membered aromatic heterocyclic group optionally condensed with benzene ring,
(3) C₇₋₁₃ aralkyl which may have 1 to 3 substituents selected from (a) the C₇₋₁₃ aralkyloxy optionally substituted with 1 or 2 groups selected from halogen atom, cyano, optionally halogenated C₁₋₆ alkyl and C₁₋₆ alkoxy and (b) the 5- or 6-membered aromatic heterocyclic ring-C₁₋₃alkoxy,
(4) C₆₋₁₂ aryl or
(5) 5- or 6- member aromatic heterocyclic group optionally condensed with benzene ring, or alternatively
R⁶ and R⁷ are bound together to form, together with an adjacent carbon atom, a C₃₋₇ cycloalkane optionally condensed with a C₆₋₁₂ aryl which may have 1 or 2 substituents selected from optionally halogenated C₆₋₁₂ aryl and halogens, or 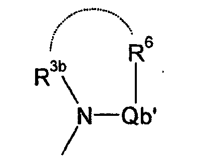 represents a 5- or 6-membered saturated cyclic amino).

38. The compound according to Claim 21, which is N-{[5-(4-chlorophenyl)-1-benzothiophen-2-yl]carbonyl}-O-(4-fluorobenzyl)tyrosine or (2S)-[4-(benzyloxy)phenyl]({[6-(4-chlorophenyl)-3-methyl-1-benzofuran-2-yl]carbonyl}amino)acetic acid.

39. A prodrug of the compound according to Claim 21.

40. A pharmaceutical comprising the compound according to Claim 21 or its salt or a prodrug thereof.

41. The compound represented by the formula: 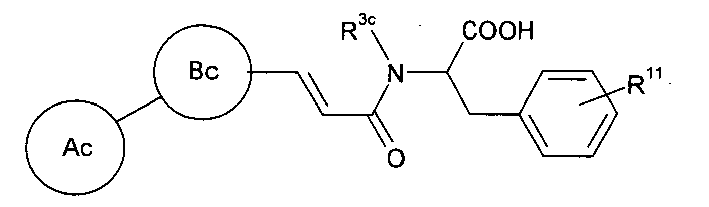 (wherein ring Ac is an optionally substituted 5- or 6-membered aromatic ring,
ring Bc is an optionally substituted 5- or 6-membered aromatic ring,
R^{3c} is a hydrogen atom or optionally substituted hydrocarbon group, and
R¹¹ is a substituent), or a salt thereof.

42. The compound according to Claim 41, wherein 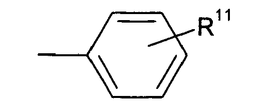 is the group represented by the formula: 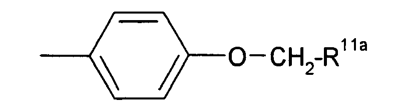 (wherein R^{11a} is an optionally substituted aromatic group).

43. The compound according to Claim 41, wherein ring Ac is an optionally substituted benzene ring.

44. The compound according to Claim 41, wherein ring Ac is an optionally halogenated benzene ring,
ring Bc is a 5- or 6-membered aromatic ring,
R^{3c} is a hydrogen atom, and
R¹¹ is an optionally halogenated C₇₋₁₃ aralkyloxy.

45. The compound according to Claim 41, which is O-benzyl-N-[(2E)-3-(4'-chlorobiphenyl-4-yl)propa-2-enoyl]tyrosine.

46. A prodrug of the compound according to Claim 41.

47. A pharmaceutical comprising the compound according to Claim 41 or its salt or a prodrug thereof.

48. The compound represented by the formula 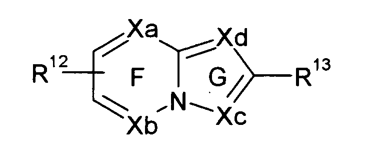 [wherein ring F and ring G form an aromatic condensed azole ring which may also have substituents other than R¹² and R¹³,
Xa, Xb, Xc and Xd are each CH or N,
one of R¹² and R¹³ is a group represented by the formula: 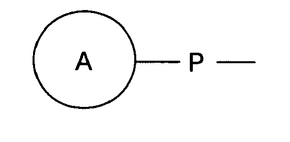 (wherein ring A is an optionally substituted isocyclic or heterocyclic ring and P is a bond or spacer) while the other is a group represented by the formula: 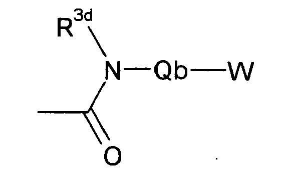 (wherein R^{3d} is a hydrogen atom or optionally substituted hydrocarbon group, Qb is an optionally substituted chain spacer and W is a carboxyl or a group biologically equivalent to a carboxyl)], or a salt thereof, with the proviso that R¹³ is not a phenyl having a substituent selected from NO₂, NH₂, CN, CSNH₂, C(=NH)S-C₁₋₆ alkyl, C(=NH)NHOH, C(=NH)-NH₂, CH₂-NH₂, CH₂NH-C(=NH)-NH₂, NH-C(=NH)-NH₂, CH₂NHCO-C₁₋₆ alkylene-NH₂, CH₂NHCO-phenylene-C(=NH)NH₂, CH₂NHCO-phenylene-CH₂-NH₂, 5-hydroxy-1,2,4-oxadiazole-3-yl, 5-C₁₋₆ alkyl-1,2,4-oxadiazole-3-yl and 5-phenyl-1,2,4-oxadiazole-3-yl, in the case where Xa, Xb and Xc are CH and Xd is N.

49. The compound according to Claim 48, wherein the aromatic condensed azole ring is

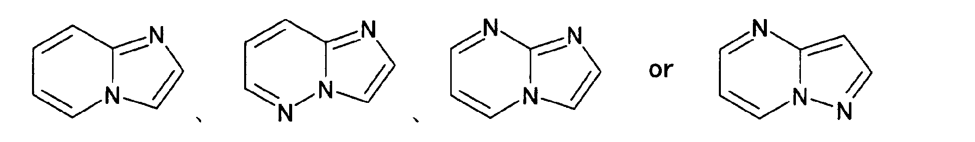

50. The compound according to Claim 48, wherein ring A is a 5- or 6-membered aromatic ring optionally substituted with 1 to 3 groups selected from halogen atom, optionally halogenated alkyl and optionally halogenated alkoxy.

51. The compound according to Claim 48, wherein P is a bond or a spacer having two atoms in the main chain.

52. The compound according to Claim 51, wherein the spacer having two atoms in the main chain is -CH₂CH₂-, -CH=CH-, -C≡C-, -CH₂O-, -CH₂S-, -CH₂SO- or -CH₂SO₂-.

53. The compound according to Claim 48, wherein NR^{3d}-Qb-W is an amino acid.

54. The compound according to Claim 48, wherein Qb is a methylene having a substituent having an aromatic.

55. The compound according to Claim 48, wherein Qb is the group represented by the formula: 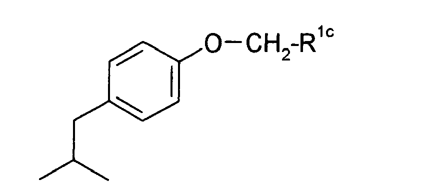 (wherein R^{1c} is an optionally substituted aromatic group).

56. The compound according to Claim 48, wherein W is a carboxyl, 5-tetrazolyl or 5-tetrazolylaminocarbonyl.

57. The compound according to Claim 48, represented by the formula: 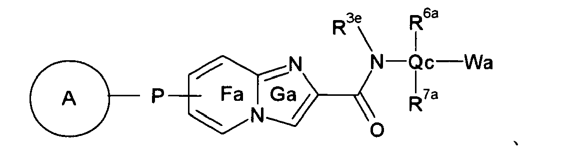 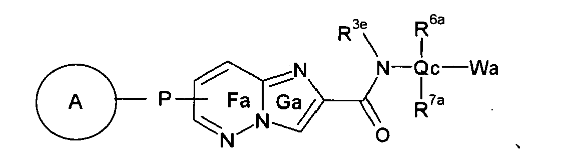 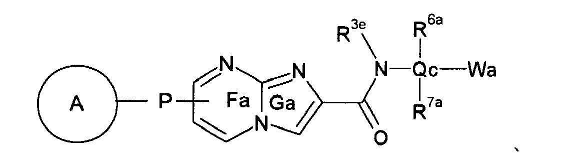 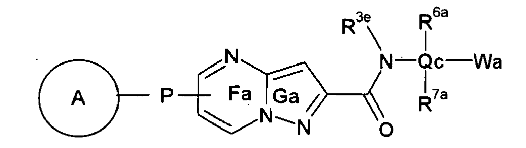 or 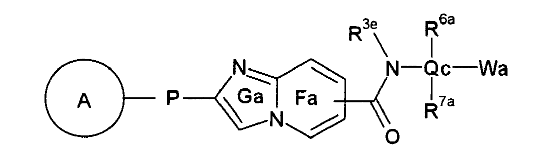 [wherein ring A is a 5- or 6-membered aromatic ring optionally substituted with 1 or 2 groups selected from halogen atom, optionally halogenated C₁₋₆ alkyl, optionally halogenated C₁₋₆ alkoxy and cyano,
P is a bond, ethylene, ethenylene, ethynylene, -CH₂-O- or -CH₂-S-,
ring Fa and ring Ga is a ring which may each have 1 or 2 substituents selected from halogen atom and optionally halogenated C₁₋₆ alkyl,
R^{3e} is a hydrogen atom or C₁₋₆ alkyl or a group represented by the formula: 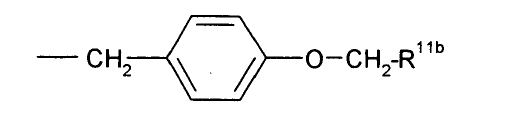 (wherein R^{11b} is a 5- or 6-membered aromatic group optionally substituted with 1 to 3 groups selected from halogen atom, optionally halogenated C₁₋₆ alkyl, optionally halogenated C₁₋₆ alkoxy and cyano)
Qc is a C₁₋₃ alkylene,
R^{6a} and R^{7a} are located on the same or different carbon atoms and are each hydrogen atom or a group represented by the formula: 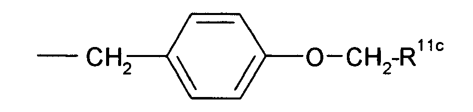 (wherein R^{11c} is a 5- or 6-membered aromatic group which may have 1 to 3 substituents selected from halogen atom, cyano, optionally halogenated C₁₋₆ alkyl and optionally halogenated C₁₋₆ alkoxy), or
R^{6a} and R^{7a} are bound together to form, together with an adjacent carbon atom, a C₃₋₇ cycloalkane optionally condensed with a C₆₋₁₂ aryl which may have 1 or 2 halogen atoms, and
Wa is a carboxyl, C₁₋₆ alkylsulfonylaminocarbonyl, 5-tetrazolyl, 5-tetrazolylaminocarbonyl or 5-oxo-1,2,4-oxadiazol-3-yl).

58. The compound according to Claim 57, wherein R^{3e} is the group represented by the formula: 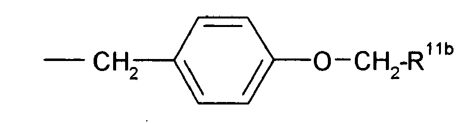 (wherein all symbols are as defined in Claim 57), and/or
at least one of R^{6a} and R^{7a} is the group represented by the formula: 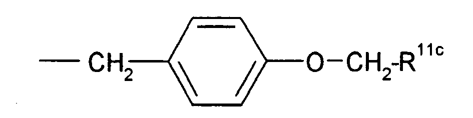 (wherein all symbols are as defined in Claim 57).

59. The compound according to Claim 48, which is
O-benzyl-N-{[6-(4-chlorophenyl)imidazo[1,2-b] pyridazin-2-yl]carbonyl}tyrosine,
O-benzyl-N-{[6-(phenylethynyl)imidazo[1,2-b] pyridazin-2-yl]carbonyl}tyrosine,
O-benzyl-N-{[6-[(E)-2-(4-chlorophenyl)ethenyl]imidazo[1,2-b] pyridazin-2-yl}carbonyl)tyrosine,
O-benzyl-N-{[7-(4-chlorophenyl)imidazo[1,2-a] pyridin-2-yl]carbonyl}-N-methyltyrosine,
N-{[7-(4-chlorophenyl)imidazo[1,2-a]pyridin-2-yl]carbonyl}-O-(4-methylbenzyl)tyrosine, or
O-(4-chlorobenzyl)-N-{[7-(4-chlorophenyl)imidazo[1,2-a] pyridin-2-yl]carbonyl}tyrosine.

60. A prodrug of the compound according to Claim 48.

61. A pharmaceutical comprising the compound according to Claim 48 or its salt or a prodrug thereof.
